(19) Europäisches Patentamt
European Patent Office
Office européen des brevets

(11) EP 1 727 065 A1

(12) **EUROPEAN PATENT APPLICATION**
published in accordance with Art. 158(3) EPC

(43) Date of publication:
**29.11.2006 Bulletin 2006/48**

(21) Application number: **05704204.6**

(22) Date of filing: **27.01.2005**

(51) Int Cl.:
***G06F 19/00*** *(2006.01)*

(86) International application number:
**PCT/JP2005/001151**

(87) International publication number:
**WO 2005/073890 (11.08.2005 Gazette 2005/32)**

(84) Designated Contracting States:
**AT BE BG CH CY CZ DE DK EE ES FI FR GB GR HU IE IS IT LI LT LU MC NL PL PT RO SE SI SK TR**

(30) Priority: **30.01.2004 JP 2004024923**

(71) Applicants:
- **National Institute of Advanced Industrial Science and Technology**
  **Tokyo 100-8921 (JP)**
- **Cytopathfinder, Inc.**
  **Tokyo 142-0084 (JP)**

(72) Inventors:
- **MIYAKE, Masato,**
  **AIST Kansai, Amagasaki Site**
  **Amagasaki-shi, Hyogo 6610974 (JP)**
- **YOSHIKAWA, Tomohiro,**
  **AIST Kansai, Amagasaki Site**
  **Amagasaki-shi, Hyogo 6610974 (JP)**
- **MIYAKE, Jun,**
  **AIST Kansai, Amagasaki Site**
  **Amagasaki-shi, Hyogo 6610974 (JP)**

(74) Representative: **Bates, Philip Ian**
**Reddie & Grose**
**16 Theobalds Road**
**London WC1X 8PL (GB)**

(54) **EVENT SEQUENCER**

(57) There is provided a tool for effectively performing a meaningful analysis of a system state by using a specific index. A part having unsual behavior is extracted as an event timing from time-series data on an index derived from a system. An event descriptor describing the state of the system by using the event timing is generated. A method for generating the event descriptor associated with at least one system includes: a step (A) for acquiring time-series data on at least one index derived from at least one system; a step (B) for providing at least one peculiar behavior associated with the index; and a step (C) for extracting a part having the peculiar behavior as an event timing in the time-series data and generating an event descriptor described by the event timing.

Fig. 43

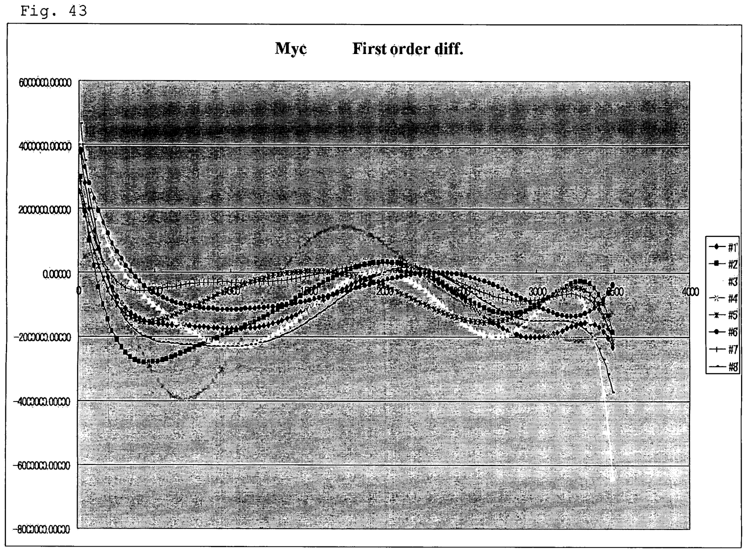


EP 1 727 065 A1

## Description

TECHNICAL FIELD

The present invention is related to a descriptor, a method for producing the same, a system using the same, a method of analysis, and program therefor, for describing an event relating to a system (for example, biological systems such as a cell, a biological organism, social systems such as a corporate organization, or economic systems such as a stock exchange quotation, and the like).

BACKGROUND ART

The following description comprises information useful for understanding the present invention. The information presented herein does not represent admitted prior art against the present invention. Further, any references referred to explicitly or implicitly herein are not admitted prior art against the present invention.

The description of a system (for example, a biological system, a economical system, and a social system), is presently conducted as a function using continuous, time-series or pseudo-continuous data, and usually is analyzed using simple arithmetic such as by processing the function thereof with simple mathematics.

However, a system is generally complex, and it is thus difficult to extract only significant descriptions as a description thereof, and thus presently global analysis, including insignificant descriptions, are conducted.

For example, a biological organism is a representative system. The principal of the biology targeting a biological organism of research interest, in particular, modern biology, is presently focused on the analysis of genetic information based on molecular biology. The minimal unit for observing life phenomena is usually a cell. In a cell, a variety of events occur (hereinafter generallu simply referred to as an "event") based on information incorporated in the genome, which controls the genetic information thereof. The entirety of such events means that a cell is "alive". Patterns in which particular subunits of cellular events are expressed in a particular cell during a particular period of time determines the phenotype thereof, and ultimately defines the type and state of a cell and a tissue.

Viruses, which are the simplest organisms in a genetic sense, usually have about 10-50 genes, and require components provided by another biological cell in order to replicate themselves. As such, a variety of events, which may be called "cellular events", substantially occur.

Biological organisms which live independently with minimal complexity in terms of genetics, which can live in a cellular unit (i.e., organisms have a genome encoding all the information necessary for survival and replication thereof, e.g., *Mycoplasma genitalium*) has about 400 genes or the like, and depending thereon, exponential number of events or combination thereof may occur.

Multicellular organisms with more complexity (e.g. mice, or humans) have a genome which is believed to consist of tens of thousands of genes (e.g., in human, it is said that there are thirty thousand or more, genes) encoding each and respective expression product. As such, the types of events that occur, depending thereon, will be enormous.

However, conventional biological research is only directed to separate observation and description of e.g., the expression of indiviudal genes, and it cannot be said that research on the entirety of the genome has been conducted. Furthermore, observation of events directly related to a gene does not directly correlate to the observation of cellular events which are not directly related to a gene, and thus it cannot be said that the entire cell is observed. Moreover, conventional methods are not suitable for analyzing cellular events in a comparative and relative manner.

Furthermore, only vague processing of time-series data will include significant data and insignificant data together, and it will be difficult to efficiently conduct significant analysis with respect to an index relating to a certain state.

Rosetta Inpharmatics has proposed cellular information as a profile in some patent applications (WO01/006013, WO01/005935 WO00/3933 WO00/39338, WO00/39337, WO00/24936, WO00/17402, WO99/66067, WO99/66024, WO99/58720, and WO99/59037). In such a profile, information from separate cells is processed as a group of separate pieces of information, but notas continuous information. Therefore, this technique is limited in that information analysis is not conducted on a single (the same) cell. Particularly, in this technique, analysis is conducted only at one specific time point before and after a certain change, and a series of temporal changes at a point (gene) are not analyzed.

Recent advances in profiling technique have led to accurate measurement of cellular components, and thus, profiling of cellular information (e.g., Schena et al., 1995, "Quantitative monitoring of gene expression patterns with a complementary DNA microarray", Science 270:467-470; Lockhart et al., 1996, "Expression monitoring by hybridization to high-density oligonucleotide arrays", Nature Biotechnology 14:1675-1680; Blanchard et al., 1996, "Sequence to array: Probing the genome's secrets", Nature Biotechnology 14:1649; and WO01/006013). For organisms whose genome has been entirely sequenced, it is possible to analyze the transcripts of all genes in a cell. In the case of other organisms for whom the amount of genomic information available is increasing, a number of genes in a cell can be simultaneously monitored.

As array technology advances, arrays also have been utilized in the field of drug discovery (e.g., Marton et al.,

"Drug target validation and identification of second-order drug target effects using Microarrays", Nat. Med., 1998 Nov, 4(11):1293-301; and Gray et al., 1998, "Exploiting chemical libraries, structure, and genomics in the search for kinase inhibitors", Science, 281:533-538). Analysis using profile (e.g., US Patent No. 5, 777, 888) and clustering of profiles provides information about conditions of cells, transplantation, target molecules and candidates of drugs, and/or the relevant functions, efficacy and toxicity of drugs. These techniques can be used to induce a common profile which represents ideal drug activity and disease conditions. Comparing profiles assists in detecting diseases in patients at early stages and provides prediction of improved clinical outcomes for patients who have been diagnosed with a disease.

With respect to time-series data, however, no means for efficiently performing significant analysis has been provided. Further, the above-mentioned technology does not allow presentation of data as an average of a heterologous cellular population, and significant analysis with respect to such time-series data. Therefore, there are deficiencies in which a variety of analyses and evaluation based on such data lacks accuracy. Accordingly, there is increasing demand for technologies enabling analysis of the state of a system with a significant format.

(patent literature 1)
Japanese PCT National Phase Laid-Open Publication No.: 2003-505038
(patent literature 2)
Japanese PCT National Phase Laid-Open Publication No.: 2003-505022
(patent literature 3)
Japanese PCT National Phase Laid-Open Publication No.: 2002-533701
(patent literature 4)
Japanese PCT National Phase Laid-Open Publication No.: 2002-533700
(patent literature 5)
Japanese PCT National Phase Laid-Open Publication No.: 2002-533699
(patent literature 6)
Japanese PCT National Phase Laid-Open Publication No.: 2002-528095
(patent literature 7)
Japanese PCT National Phase Laid-Open Publication No.: 2002-526757
(patent literature 8)
Japanese PCT National Phase Laid-Open Publication No.: 2002-518021
(patent literature 9)
Japanese PCT National Phase Laid-Open Publication No.: 2002-518003
(patent literature 10)
Japanese PCT National Phase Laid-Open Publication No.: 2002-514804
(patent literature 11)
Japanese PCT National Phase Laid-Open Publication No.: 2002-514773
(patent literature 12)
Japanese PCT National Phase Laid-Open Publication No.: 2002-514437
(patent literature 13)
United States Patent No. 5,569,588
(patent literature 14)
United States Patent No. 5,777,888
(non-patent literature 1)
Schena et al., 1995, Quantitative monitoring of gene expression patterns with a complementary DNA micro-array, Science 270:467-470
(non-patent literature 2)
Lockhart et al., 1996, Expression monitoring by hybridization to high-density oligonucleotide arrays, Nature Biotechnology 14:1675-1680
(non-patent literature 3)
Blanchard et al., 1996, Sequence to array: Probing the genome's secrets, Nature Biotechnology 14:1649
(non-patent literature 4)
Marton et al., 1998, Drug target validation and identification of second-order drug target effects using Microarrays,Nat Med. 1998 Nov;4(11):1293-301
(non-patent literature 5)
Gray et al., 1998, Exploiting chemical libralies, structure, and genomics in the search for kinase inhibitors, Science 281: 533-538

DISCLOSURE OF THE INVENTION

(Problems to be solved by the invention)

It is an object of the present invention to provide a tool for performing significant analysis of the state of a system in an efficient manner using a particular index. In particular, it is an object of the present invention to provide means for description so as to allow analysis of an event using an algorithm or the like in order to provide technology to describe characteristic events relating to a system as an "event.

(Means for solving the problem)

The above-mentioned objects have been solved by extracting a portion having a characteristic behaviour relating to a time-series data of an index derived from a system as an event timing and producing an event descriptor which describes the state of a system therefrom.

Regarding a biological system, for example, the above-mentioned problems have been solved by immobilizing a cell onto a support, monitoring an index relating to the cell in a time-lapse manner, and producing time-series data of a cell to describe the state of a cell thereby, thus allowing analysis of a variety of changes of a cell in an efficient manner.

Such a method is used to allow complex analysis regarding a variety of indexes in a variety of systems in a simple manner to simply find out an interrelationship. For example, a number of genes can now be described in an complete manner regarding the interrelationship therebetween.

Accordingly, the present invention provides the following:

1. A method for producing an event descriptor relating to at least one system, comprising the steps of:

   (A) obtaining time series data of at least one index derived from at least one system;
   (B) providing at least one characteristic behaviour relating to the index; and
   (C) extracting a portion having the characteristic behaviour in the times series data as an event timing to produce an event descriptor described by the event timing.

2. A method according to item 1, wherein the system is biological.

3. A method according to item 1, wherein the system is a portion of a biological entity selected from the group consisting of biological body, organ, tissue, cell population, cell and cellular organelle.

4. A method according to item 1, wherein the system is a cell.

5. A method according to item 1, wherein the system is a social organization.

6. A method according to item 1, wherein the system is an economic system.

7. A method according to item 1, wherein the index is selected from the group consisting of a natural scientific index, a technical index, a social scientific index, and a human scientific index.

8. A method according to item 1, wherein the index is related to at least one state selected from the group consisting of a differentiation state, a response to a external agent, a cellular cycle state, a proliferation state, apoptosis state, a response to a circumstantial change and an aging state.

9. A method according to item 1 wherein the index comprises at least one selected from the group consisting of a gene expression level, a gene transcription level, a gene post-translational modification level, a chemical level present intracellularly, an intracellular ion level, cell size, a biochemical process level, and a biophysical process level.

10. A method according to item 1, wherein the index comprises at least one selected from the group consisting of a gene expression level and a gene transcription level.

11. A method according to item 1 wherein the index comprises a gene transcription level.

12. A method according to item 1, wherein the characteristic behaviour comprises at least one selected from the group consisting of: coincidence of the time-series data and a predetermined value, or a specific variation or no change of the absolute value change rate thereof; coincidence of a first-order differentiation value of the time-series data and a predetermined value, or a specific variation or no change of the absolute value change rate thereof; coincidence of a second-order differentiation value of the time-series data and a predetermined value, or a specific variation or no change of the absolute value change rate thereof; change in sign (+/-) of the time-series data; change in sign (+/-) of the first-order differentiation value of the time-series data; change in sign (+/-) of the second-order differentiation value of the time-series data; coincidence of the time-series data and time-series data of another index; coincidence of the first-order differentiation of the time-series data and the first-order differentiation of time-series data of another index; coincidence of the second-order differentiation of the time-series data and the second-order differentiation of time-series data of another index; coincidence of sign (+/-) of the time-series data and the sign of time-series data of another index; coincidence of sign (+/-) of the first-order differentiation value of the time-series data and the sign of the first-order differentiation value of time-series data of another index; coincidence of sign (+/-) of the second-order differentiation value of the time-series data and the sign of the second-order differentiation value of time-series data of another index; coincidence of the time-series data and another time-series data of the index; coincidence of the first-order differentiation of the time-series data and the first-order differentiation of another time-series data of the index; and coincidence of the second-order differentiation of the time-series data and the second-order differentiation of another time-series data of the index.

13. A method according to Item 1, wherein the characteristic behaviour is the change of the sign of the first-order differentiation value of the time-series data.

14. A method according to item 1, wherein the time-series data is continuous or discontinuous.

15. A method according to item 1, wherein the time-series data is described in relative time or absolute time.

16. A method according to item 1, wherein the time series data is described in such a manner that the initiation time of observation is expressed as a reference (0).

17. A method according to item 1, wherein the time-series data is expressed as a relative or absolute level.

18. A method according to item 1, wherein the time-series data are those of a genetic expression level, and the genetic expression level is an expression level of a flucorescent protein.

19. A method according to item 1, wherein the time-series data are normalized data.

20. A method according to item 1 wherein the event timing is expressed as a time point or a time range.

21. A method according to item 1, wherein the event timing is within a shift or within a time range of 12 hours or less.

22. A method according to item 1, wherein the event timing is within a shift or within a time range of one hour or less.

23. A method according to item 1, further comprising the step of mathematically processing the time series data.

24. A method according to item 23, wherein the mathematical process is selected from the group consisting of normalization, first-order differentiation, second-order differentiation, third-order differentiation, linear approximation, non-linear approximation, moving average, noise filter, Fourier's transform, fast Fourier's transform and principal component analysis.

25. A method according to item 1, wherein the event timing is calculated based on raw data of the time series data.

26. A method according to item 1, wherein the event timing is calculated based on first-order differentiation of the time series data.

27. A method according to item 1, wherein the event timing is calculated based on second-order differentiation of the time series data.

28. A method according to item 1, wherein the event timing is calculated based on the coincidence of increase or decrease per unit time in a plurality of time series data.

29. A method according to item 28, wherein each of the unit times are identical or different.

30. A method according to item 1, wherein the event timing is represented in the increase, decrease or unchanged status of the index.

31. A method according to item 1, wherein the event timing is represented by the expression manner of (time t, the increase, decrease or unchangeness of the index <+, - or 0>).

32. A method according to item 31, wherein the time t is represented by a time point or time range.

33. A method according to item 1, wherein the event descriptor is represented by aligning characters or letters related to the event timing in an order of time points.

34. A method according to item 1, wherein the description relating to the event timing is represented by means of A, T, G or C, which are single letter designators of nucleic acids, in an order of time points.

35. A method according to item 1, wherein the increase or decrease in the index is characterized in that the point at which the sign of the first-order differentiation is changed, the sign of the second-order differentiation is changed, or the case where the value of raw data is significantly changed in an experiment, are indicative of the increase or decrease.

36. A method according to item 1, wherein the increase or decrease in the index is characterized in that the point at which the sign of the first-order differentiation is changed, the sign of the second-order differentiation is changed, or the case where the value of raw data is significantly changed in an experimental system, in a normalized form of the time-series data.

37. A method according to item 1, wherein at least two indices are used as the index, and, as the event timing, those at which the behaviours of increase or decrease coincide with respect to the increase/decrease of the index at at least one point in at least two types of indices.

38. A method according to item 1, wherein sign change in first-order differentiation and sign change in second-order differentiation are used as the characteristic behavior, and a first letter/character corresponding to the sign change of the first-order differentiation and a second letter corresponding to the sign change of the second-order differentiation are represented in the form of a character string according to the time order as the event descriptor.

39. A method according to item 1, wherein sign change in first-order differentiation and sign change in second-order differentiation are used as the characteristic behavior, and a first letter/character corresponding to the sign change of the first-order differentiation, a second letter corresponding to the sign change of the second-order differentiation and a third letter/character corresponding to another letter/character regarding the time without sign change are represented in a form of a character string according to the time order as the event descriptor.

40. A method according to item 1, wherein sign change in raw data is used as the characteristic behavior, and a first letter/character corresponding to the increase in the raw data, and a second letter/character corresponding to the decrease in the raw data, are represented in a form of a character string according to the time order as the event descriptor.

41. A method according to item 1, wherein sign change in raw data is used as the characteristic behavior, and a first letter/character corresponding to the increase in the raw data, a second letter/character corresponding to the decrease in the raw data, and a third letter/character corresponding to another character/letter regarding the time without increase or decrease are represented in a form of a character string according to the time order as the event descriptor.

42. A method according to item 1, wherein the event descriptor is described with the notation selected from the group consisting of an electric wave, a magnetic wave, a sound, light, color, image, number and character/letter.

43. A method according to item 1, wherein the event descriptor is notated by characters or letters.

44. A method according to item 1, further comprising the step of recording the event descriptor on a storage medium.

45. A method for analyzing at least one system using an event descriptor relating to the system, comprising the steps of:

(A) obtaining time-series data of at least one index derived from at least one system;
(B) providing at least one characteristic behavior;
(C) extracting a portion having the characteristic as an event timing in the time-series data; and
(D) analyzing the at least one event descriptor.

46. A method according to item 45, wherein the analysis uses an algorithm.

47. A method according to item 45, wherein the algorithm comprises one selected from the group consisting of self-organization mapping, cluster analysis, genetic algorithm, alignment analysis, and parsing in a natural language processing.

48. A method according to item 48, wherein the algorithm comprises a genetic algorithm.

49. A method according to Item 45, wherein the system is a biological system.

50. A method according to Item 45, wherein the system is a cell.

51. A method for analyzing the relationship between a first index and a second index in a system, comprising the steps of:

(A) producing a first event descriptor relating to a first index using a method according to item 1;
(B) producing a second event descriptor relating to a second index using a method according to item 1; and
(C) comparing the first and second event descriptors obtained in steps (A) and (B).

52. A method according to item 51, wherein the comparison in the step (c) is conducted by production of coincidence event timing whose behaviors coincide in the first and second event descriptors.

53. A method for analyzing the relationship between a first index from a first system and a second index from a second system, comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

54. A method for analyzing the relationship between indices at a first and second time points from a system, comprising the steps of:

(A) producing a first event descriptor relating to the first time point using a method according to item 1;
(B) producing a second event descriptor relating to the second time point using a method according to item 1; and
(C) comparing the first and second event descriptors obtained in the steps (A) and (B).

55. A method for analyzing an index from a system using an event descriptor obtained using first and second characteristic behaviors, comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

56. A method according to item 55, wherein the step of comparison comprises the step of extracting an event timing which coincides in a time point between the event timing in the first event descriptor and the event timing of the second event descriptor.

57. A production system for producing an event descriptor relating to a system, comprising:

i) monitoring means for monitoring at least one index relating to the system in a time-lapse manner; and

ii) descriptor production means for producing an event descriptor by producing a time-series data of the system from a signal obtained from the monitoring means, and calculating the time-series data; wherein the descriptor production means

(A) obtains time series data of at least one index derived from at least one system;
(B) provides at least one characteristic behaviour relating to the index; and
(C) extracts a portion having the characteristic behaviour in the time series data as an event timing to produce an event descriptor described by the event timing.

58. A production system according to item 57, wherein the system is a cell, and the production system further comprises a support capable of maintaining a certain environment around the cell.

59. A production system according to item 57, wherein the monitoring means is selected from the group consisting of an optical microscope, a fluorescent microscope, reading devices using a laser light source, surface plasmon resonance (SPR) imaging, reading devices of a signal derived from a means using electric signals, chemical or biochemical markers or a combination thereof, CCD camera, autoradiography, MRI and sensors.

60. A production system according to item 57, wherein the monitoring means comprises means for outputting a signal.

61. A production system according to item 57, wherein the descriptor production means comprises means for producing the time-series data, and means for producing the descriptor by conducting the calculation step.

62. A system according to item 57, wherein the descriptor production means comprises a computer implementing a program instructing performing the steps of (A) through (C).

63. A system according to item 57, wherein the descriptor further comprises display means for displaying the descriptor.

64. A system according to item 63, wherein the display means has functions displaying a notation selected from the group consisting of an electric wave, a magnetic wave, sound, light, color, image, number and character/letter

65. A system according to item 63, wherein the display means has a letter/character displaying function.

66. A system according to item 57, further comprising means for recording the event descriptor on a storage medium.

67. An event descriptor for describing a system, comprising a portion having at least one characteristic behavior as an event timing relating to at least index derived from at least one system.

68. An event descriptor produced by a method according to item 1.

69. An analysis system for analyzing a system using a descriptor relating thereto, comprising:

i) monitoring means for monitoring at least one index relating to the system in a time-lapse manner;
ii) descriptor production means for producing an event descriptor by producing a time-series data of the system from a signal obtained from the monitoring means, and calculating the time-series data; and
iii) analysis means for analyzing the descriptor, wherein the descriptor production means

(A) obtains time series data of at least one index behavior from at least one system;
(B) provides at least one characteristic behavior relating to the index; and
(C) extracts a portion having the characteristic behavior in the times series data as an event timing to produce an event descriptor described by the event timing.

70. An analysis system according to item 69, wherein the analysis means has a function of analyzing at least one event descriptor with an algorithm analysis.

71. A method for analyzing a system using a sequence of event descriptors relating to at least one system, comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

72. A method according to item 71, wherein the analysis of sequence uses a genetic algorithm.

73. An analysis system for analyzing a system using a sequence of event descriptors relating to at least one system, comprising:

i) monitoring means for monitoring at least one index relating to the system in a time-lapse manner;
ii) descriptor production means for producing an event descriptor by producing a time-series data of the system from a signal obtained from the monitoring means, and calculating the time-series data to produce an event descriptor describing the event timing as a sequence; and
iii) analysis means for analyzing the sequence,
wherein the descriptor production means

(A) obtains time series data of at least one index behavior from at least one system;
(B) provides at least one characteristic behavior relating to the index; and
(C) extracts a portion having the characteristic behavior in the times series data as an event timing to produce an event descriptor described by the event timing.

74. An analysis system according to item 73, wherein the analysis of the sequence uses a genetic algorithm.

75. A program for implementing in a computer a process for producing an event descriptor relating to at least one system, the process comprises the steps of:

(A) obtaining time series data of at least one index derived from at least one system;
(B) providing at least one characteristic behavior relating to the index; and
(C) extracting a portion having the characteristic behavior in the times series data as an event timing to produce an event descriptor described by the event timing.

76. A program for implementing in a computer a process for analyzing at least one system using an event descriptor relating to the system, the process comprising the steps of:

(A) obtaining time-series data of at least one index derived from at least one system;
(B) providing at least one characteristic behavior;
(C) extracting a portion having the characteristic behavior as an event timing in the time-series data; and
(D) analyzing the at least one event descriptor.

77. A program for implementing in a computer a process for analyzing the relationship between a first index and a second index in a system, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

78. A program for implementing a computer a process for analyzing the relationship between a first index from a first system and a second index from a second system, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

79. A program for implementing a computer a process for analyzing an index from a system using an event descriptor obtained using first and second characteristic behaviors, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

80. A program for implementing in a computer a process for analyzing a system using a sequence of event descriptors relating to at least one system, the process comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

81. A storage medium storing a program for implementing in a computer a process for producing an event descriptor relating to at least one system, the process comprises the steps of:

(A) obtaining time series data of at least one index derived from at least one system;
(B) providing at least one characteristic behavior relating to the index; and
(C) extracting a portion having the characteristic behavior in the times series data as an event timing to produce an event descriptor described by the event timing.

82. A storage medium storing a program for implementing in a computer a process for analyzing at least one system using an event descriptor relating to the system, the process comprising the steps of:

(A) obtaining time-series data of at least one index derived from at least one system;
(B) providing at least one characteristic behavior;
(C) extracting a portion having the characteristic behavior as an event timing in the time-series data; and
(D) analyzing the at least one event descriptor.

83. A storage medium storing a program for implementing in a computer a process for analyzing the relationship between a first index and a second index in a system, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

84. A storage medium storing a program for implementing in a computer a process for analyzing the relationship between a first index from a first system and a second index from a second system, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

85. A storage medium storing a program for implementing in a computer a process for analyzing an index from a system using an event descriptor obtained using first and second characteristic behaviors, the process comprising the steps of:

(A) producing a first event descriptor relating to a first index using a method according to item 1;
(B) producing a second event descriptor relating to a second index using a method according to item 1; and
(C) comparing the first and second event descriptors obtained in the steps (A) and (B).

86. A storage medium storing a program for implementing in a computer a process for analyzing a system using a sequence of event descriptors relating to at least one system, the process comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

Hereinafter, the present invention will be described by way of preferred embodiments. It will be understood by those skilled in the art that the embodiments of the present invention can be appropriately made or carried out based on the description of the present specification and the accompanying drawings, and commonly used techniques well known in the art. The function and effect of the present invention can be easily recognized by those skilled in the art.

EFFECTS OF INVENTION

According to the present invention, it is now possible to efficiently describe the state of a system (for example, biological systems such as a cell, a biological organism, social systems such as a corporate organization, or economic systems such as a stock exchange quotation, and the like). The present description method is used to allow the analysis of a variety of systems in a simple manner. Furthermore, the analytical results of the system appear to be suitable for signifying the interrelationships therebetween.
As such, the present invention enables determination, examination, research and the like of the state of a system using surprisingly less data analysis. Such determination has application in diagnosis, prevention, therapy of disease, and the like, and the application ranges not only within the medical field but also a variety of fields including food products, cosmetics, agriculture, the environment, economy (stock values, exchange and the like), apparatus control, computer, general society, organizations and the like.

BRIEF DESCRIPTION OF THE DRAWINGS

Figure **1** shows the results of experiments in which various actin-like substances and HEK293 cells were used, where gelatin was used as a control.

Figure **2** shows exemplary transfection efficiencies when fibronectin fragments were used.

Figure **3** shows exemplary transfection efficiencies when fibronectin fragments were used.

Figure **4** shows a summary of the results presented in Figures **2** and **3.**

Figure **5** shows the results of an example in which transfection efficiency was studied for various cells.

Figure **6** shows the results of transfection when various plates were used.

Figure **7** shows the results of transfection when various plates were used at a fibronectin concentration of 0, 0.27, 0.53, 0.8, 1.07, and 1.33 (μg/μL, respectively).

Figure **8** provides exemplary photographs showing cell adhesion profiles in the presence or absence of fibronectin.

Figure **9** shows exemplary cross-sectional photographs of cell adhesion profiles in the presence or absence of fibronectin.

Figure **10** shows the transition of nuclear surface area.

Figure **11** shows the results of an exemplary transfection experiment when a transfection array chip was constructed and used.

Figure **12** shows exemplary contamination between each spot on an array.

Figure **13** shows an experiment in which spatially-spaced DNA was taken into cells after the solid phase transfection of the present invention in Example 4.

Figure **13A** schematically shows a method for producing a solid phase transfection array (SPTA). This figure shows the methodology of a solid transfection.

Figure **13B** shows the results of a solid phase transfection. A HEK293 cell line was used to produce a SPTA. Green colored portions indicate transfected adherent cells. According to this result, the method of the present invention can be used to produce a group of cells separated spatially and transfected with different genes. Figure 13C shows the methodology of a solid transfection.

Figure **14A-B** shows the results of comparison of liquid phase transfection and SPTA.

Figure **14A** shows the results of experiments where 5 cell lines were measured with respect to GFP intensity/$mm^2$. Transfection efficiency was determined as fluorescence intensity per unit area.

Figure **14B** shows fluorescent images of cells expressing EGFP corresponding to the data presented in Figure **14A.** White circular regions therein were regions in which plasmid DNA was fixed. In other regions, cells were also fixed in solid phase, however, cells expressing EGFP were not observed. The white bar indicates 500 μm.

Figure **14C** shows an exemplary transfection method of the present invention.

Figure **14D** shows an exemplary transfection method of the present invention.

Figure **15** shows the results of coating a chip, whereby cross contamination was reduced.

Figure **15** shows the results of liquid phase transfection and SPTA using HEK293 cells, HeLa cells, NIT3T3 cells (also referred to as "3T3"), HepG2 cells, and hMSCs. Transfection efficiency was determined by GFP intensity.

Figure **16** shows cross contamination between each spot. A nucleic acid mixture containing fibronectin having a predetermined concentration was fixed to a chip coated with APS or PLL (poly-L-lysine). Cell transfection was performed on the chip. Substantially no cross contamination was observed (upper and middle rows). In contrast, significant cross contamination of fixed nucleic acids was observed on an uncoated chip (lower row).

Figure **16C** shows a correlation relationship between the types of substances contained in a mixture used for fixation of nucleic acids, and the cell adhesion rate. The graph presented in Figure **16** shows an increase in the proportion of adherent cells over time. A longer time is required for cell adhesion when the slope of the graph is shallow than when the slope of the graph is steep.

Figure **16D** is an enlarged graph which is presented in Figure **16C.**

Figure **17** shows an exemplary configuration of a computer which was used to perform the method of the present invention.

Figure **18** depicts an example of a mathematical analysis method of the present invention. Profiles of promoters shown in Figure **18A** (average of pNEFAT-d2EGFP/negative control) and Figure **18B** (average of pERE-d2EGFP/negative control) are obtained by measuring the fluorescent intensity thereof over time. These profiles have been normalized using the autologous fluorescence of either the cell or medium used. Thereafter, in order to compare the amplitude of the reporter expression fluctuation, an amplitude = 5 or more (TH>=5) was determined to show a change in expression fluctuation state. Further, differentiation induction was divided into the following sections: start of differentiation induction, early stage (0-17.5 hours), and late stage (17.5-31.5 hours) and total stages (0-31.5 hours); and those observed with a variation in expression of an amplitude of 5 or more (TH>=5) were defined as (+) and those with an amplitude of less than 5 were defined as (-). Based on these definitions, the profiles of A and B were evaluated as shown in the lower tables of Figures **18A** and **18B.** In the table, when extracting any number of reporters, (A+B+ ... n) have been integrated with respect to n types of wave forms and the sum is divided by n to form the average wave form and if variations beyond the threshold were observed, such variations were deemed as being "changed".

Figure **18B** depicts another example of a mathematical analysis according to the present invention. When a reporter is extracted (A+B+ ... n), n types of wave types are integrated, and the sum is divided by n to produce an average wave form, which was deemed as being a change of the variation above a threshold. The left hand panel of Figure **18B** depicts the integration of two reporter profiles and draws the average wave form in red or with solid squares. Those with 5 or more variations of the average profile were deemed to be expression variations for evaluation. As a result, evaluation can be conducted for variation of the two extracted reporters, as shown in the table herein.

Figure **19** depicts exemplary plasmids containing promoters used in the present invention and an analysis according to the present invention. Seventeen types of transcriptional factors shown in the left hand panel of Figure **19** were used as a reporter under the conditions of osteoblast differentiation and maintenance of an undifferentiated mesenchymal stem cell, and the expression profile thereof have been obtained over time (Figure **19,** right handed panel). From these seventeen types of profiles, any number of profiles have been extracted and evaluated by the method as previously described in Figure **18,** taking the change in amplitude of the response profile of each transcriptional factor as a standard.

Figure **20** depicts an example of mathematical analysis at the early stage of induction of differentiation. By changing the combination arbitrarily extracted in the early differentiation induction stage, results as shown in Figure **20** have been obtained. Any number of reporters were extracted from the reporter group consisting of seventeen species, and calculated for the average profile according to the method shown in Figure **18.** Those having five or more variation ranges are the results evaluated with the evaluation windows 0-31.5, 0-17.5 and 17.5-31.5. Each extraction condition has seventeen extraction patterns, except for where the seventeen extraction pattern have only one method of extraction. Amongst these combinations, Figure **20** shows the ratio in which variation is found therein, including the table and graph included therein. This analysis allows confirmation of differentiation after fifteen hours although it is not possible to understand the very early stages of differentiation. The number of extractions where a 100 % change is found for variation is eight or more in this instance.

Figure **21** depicts an example of a mathematical analytical result at the undifferentiation maintenance stage. As in Figure 20, similar results as shown in the graphs have been obtained when a combination arbitrarily extracted under conditions to maintain undifferentiation. Comparing the results with the stage of differentiation induction, as in Figure **20,** the results are dramatically different. Based on this comparison, it is believed that it is possible to determine whether a cell is moving into cell differentiation induction, or is instead maintaining an undifferentiated state.

Figure **22** schematically shows a cocktail party process.

Figure **23** shows an exemplary construct of a gene transcription switch reporter used in a transfection plasmid of the present invention.

Figure **24** shows exemplary construction of a set of transcription factor reporters.

Figure **25** shows the results of exemplary assays using transcription factor reporters.

Figure **26** shows an example of measurements of transcriptional activity in the bone differentiation process, taken in a time-series manner.

Figure **27** shows an example of the oscillation phenomenon and phase analyses of transcriptional activity.

Figure **28** shows a protocol of an siRNA experiment.

Figure **29** shows the results of the siRNA experiments. The upper panel shows the results of hMSC, and the lower panel shows the results of HeLa cells. The numerals show the concentrations ($\mu$g/$\mu$L) of the siRNA used. The results obtained with the anti-GFP siRNA are shown on the left hand side, and the right hand side shows the results with the scramble siRNAs.

Figure **30** shows changes in the profile when using tetracycline dependent promoters.

Figure **31** shows expression when using tetracycline dependent promoters and tetracycline independent promoters.

Figure **32** depicts an example of a system configuration.

Figure **33** shows an exemplary real time measuring device.

Figure **34** shows a schematic, enlarged view of the cell measuring device of Figure **33.**

Figure **35** shows a scheme of cell measurement.

Figure **36** shows the experimentata format of an exemplary grid array used in the present invention. Real time monitoring was started under serum-free conditions, two days after transfection. Images were obtained at intervals of 30 minutes. All the reporter vectors were used under control conditions for confirmation.

Figure **37** shows raw data obtained using a grid array of the present invention. Names of genes used are shown in the lower left portion of the figure. Raw data obtained by using the grid array is shown in the right portion of the figure.

Figure **38A** shows a graph of raw data obtained in Example 5. The vertical axis represents fluorescence intensity (Arbitrary Unit), while the horizontal axis represents time (unit: minute (min)). The following genes were used: pEGFP-N1, pAP1-EGFP, pAP1(PMA)-EGFP, pE2F-EGFP, pGAS-EGFP, pHSE-EGFP, pMyc-EGFP, pNFkB-EGFP, pRb-EGFP, pSRE-EGFP, pp53-EGFP, pCRE-EGFP, pERE-EGFP, pGRE-EGFP, pISRE-EGFP, pNFAT-EGFP, pRARE-EGFP, pSTAT3-EGFP, pTRE-EGFP, pCREB-EGFP, pIkB-EGFP, pp53-EGFP (Signaling probe), and pCaspase3-Sensor.

Figure 3**8B** shows the raw data obtained in Example 5.

Figures **38C** shows the results of calculation after polynominal approximation of the data obtained in Example 5.

Figures **38D** shows the results of first-order differentiation and second-order differentiation of the data obtained in Example 5.

Figures **39-1** to **39-55** show raw data obtained in Example 5 for each gene. Figure **39-1** shows time-lapse data of EGFP-N1.

Figure **39-2** shows time-lapse data of AP1.

Figure **39-3** shows time-lapse data of AP1(PMA).

Figure **39-4** shows time-lapse data of CRE.

Figure **39-5** shows time-lapse data of E2F.

Figure **39-6** shows time-lapse data of none.

Figure **39-7** shows time-lapse data of EGFP-N1.

Figure **39-8** shows further time-lapse data of AP1.

Figure **39-9** shows further time-lapse data of AP1(PMA).

Figure **39-10** shows further time-lapse data of CRE.

Figure **39-11** shows further time-lapse data of E2F.

Figure **39-12** shows time-lapse data of ERE.

Figure **39-13** shows time-lapse data of GAS.

Figure **39-14** shows time-lapse data of GRE.

Figure **39-15** shows time-lapse data of HSE.

Figure **39-16** shows time-lapse data of ISRE.

Figure **39-17** shows further time-lapse data of none.

Figure **39-18** shows further time-lapse data of ERE.

Figure **39-19** shows further time-lapse data of GAS.

Figure **39-20** shows further time-lapse data of GRE.

Figure **39-21** shows time-lapse data of HSE.

Figure **39-22** shows time-lapse data of ISRE.

Figure **39-23** shows time-lapse data of Myc.

Figure **39-24** shows time-lapse data of NFAT.

Figure **39-25** shows time-lapse data of NFκB.

Figure **39-26** shows time-lapse data of RARE.

Figure **39-27** shows time-lapse data of Rb.

Figure **39-28** shows further time-lapse data of none.

Figure **39-29** shows time-lapse data of Myc.

Figure **39-30** shows further time-lapse data of NFAT.

Figure **39-31** shows further time-lapse data of NFκB.

Figure **39-32** shows further time-lapse data of RARE.

Figure **39-33** shows further time-lapse data of Rb.

Figure **39-34** shows time-lapse data of STAT3.

Figure **39-35** shows time-lapse data of SRE.

Figure **39-36** shows time-lapse data of TRE.

Figure **39-37** shows time-lapse data of p53.

Figure **39-38** shows time-lapse data of Caspase3.

Figure **39-39** shows further time-lapse data of none.

Figure **39-40** shows time-lapse data of STAT3.

Figure **39-41** shows further time-lapse data of SRE.

Figure **39-42** shows further time-lapse data of TRE.

Figure **39-43** shows further time-lapse data of p53.

Figure **39-44** shows further time-lapse data of Caspase3.

Figure **39-45** shows time-lapse data of CREB-EGFP.

Figure **39-46** shows time-lapse data of IκB-EGFP.

Figure **39-47** shows time-lapse data of pp53-EGFP.

Figure **39-48** shows further time-lapse data of none.

Figure **39-49** shows further time-lapse data of none.

Figure **39-50** shows further time-lapse data of none.

Figure **39-51** shows further time-lapse data of CREB-EGFP.

Figure **39-52** shows further time-lapse data of IκB-EGFP.

Figure **39-53** shows further time-lapse data of pp53-EGFP.

Figure **39-54** shows further time-lapse data of none.

Figure **39-55** shows further time-lapse data of none.

Figure **40** shows the structure of pMyc-TA-Luc.

Figure **41** depicts examples of data extraction amongst homogenous genes (eight types of Myc genes).

Figure **42** depicts examples of data after polynomial approximation and smoothing amongst homogenous genes (eight types of Myc genes).

Figure **43** depicts results after first-order differential of the data shown in Figure **42.**

Figure **44** depicts results after second-order differential of the data shown in Figure **42.**

Figure **45** depicts the extraction results amongst heterogenous genes.

Figure **46** depicts the structures of pE2F-Luc and pRb-TA-Luc.

Figure **47** depicts an example of brain waves.

Figure **48** depicts an example of a graph showing a stock quotation change.

DESCRIPTION OF SEQUENCE LISTING

SEQ ID NO. : 1: a nucleic acid sequence encoding fibronectin (human)
SEQ ID NO.: 2: an amino acid sequence of fibronectin (human)
SEQ ID NO. : 3: a nucleic acid sequence encoding vitronectin (mouse)
SEQ ID NO.: 4: an amino acid sequence of vitronectin (mouse)
SEQ ID NO. : 5: a nucleic acid sequence encoding laminin (mouse α-chain)
SEQ ID NO.: 6: an amino acid sequence of laminin (mouse α-chain)
SEQ ID NO. : 7: a nucleic acid sequence encoding laminin (mouse β-chain)
SEQ ID NO.: 8: an amino acid sequence of laminin (mouse β-chain)
SEQ ID NO. : 9: a nucleic acid sequence encoding laminin (mouse γ-chain)
SEQ ID NO.: 10: an amino acid sequence of laminin (mouse γ-chain)
SEQ ID NO.: 11: an amino acid sequence of fibronectin (bovine)
SEQ ID NO.: 12: siRNA used in the Examples
SEQ ID NO.: 13: mouse olfactory receptor 17 (heptanal-sensitive) nucleic acid (Genbank Accession No. AF106007)
SEQ ID NO.: 14: amino acid sequence of the protein encoded by the nucleic acid set forth in SEQ ID NO.: 13
SEQ ID NO: 15: the nucleic acid encoding the murine olfactory receptor S1 (mc9/bc9-equi-sensitive) (Genbank Accession Number AF121972)
SEQ ID NO: 16: the amino acid sequence of the protein encoded by the nucleic acid set forth in SEQ ID NO: 15
SEQ ID NO: 17: the nucleic acid encoding the murine olfactory receptor S50 (cc9-sensitive) (Genbank Accession Number AF121980)

SEQ ID NO: 18: the amino acid sequence of the protein encoded by the nucleic acid set forth in SEQ ID NO: 17
SEQ ID NO: 19: the nucleic acid encoding the murine olfactory receptor S19 (mc9/mh9/bc9-equi-sensitive) (Genbank Accession Number AF121976)
SEQ ID NO: 20: the amino acid sequence of the protein encoded by the nucleic acid set forth in SEQ ID NO: 19
SEQ ID NO: 21: the nucleic acid encoding the murine OR23 (lyral-sensitive) (only coding region of Genbank Accession Number X92969)
SEQ ID NO: 22: the amino acid sequence of the protein encoded by the nucleic acid set forth in SEQ ID NO: 21
SEQ ID NO: 23: nthe ucleic acid encoding the murine olfactory receptor mOR-EV (vanillin-sensitive) (Genbank Accession Number AB061229)
SEQ ID NO: 24: the amino acid sequence of the protein encoded by the nucleic acid set forth in SEQ ID NO: 23
SEQ ID NO: 25: the nucleic acid encoding the murine olfactory receptor or37a (Genbank Accession Number AJ133424)
SEQ ID NO: 26: the amino acid sequence of the protein encoded by the nucleic acid set forth in SEQ ID NO: 25
SEQ ID NO: 27: the nucleic acid encoding the murine olfactory receptor C6 (Genbank Accession Number AF102523)
SEQ ID NO: 28: the amino acid sequence of the protein encoded by the nucleic acid set forth in SEQ ID NO: 27
SEQ ID NO: 29: the nucleic acid encoding the murine olfactory receptor F5 (Genbank Accession Number AF102531)
SEQ ID NO: 30: the amino acid sequence of the protein encoded by the nucleic acid set forth in SEQ ID NO: 29
SEQ ID NO: 31: the nucleic acid encoding the murine olfactory receptor S6 (Genbank Accession Number AF121974)
SEQ ID NO: 32: the amino acid sequence of the protein encoded by the nucleic acid set forth in SEQ ID NO: 31
SEQ ID NO: 33: the nucleic acid encoding the murine olfactory receptor S18 (Genbank Accession Number AF121975)
SEQ ID NO: 34: the amino acid sequence of the protein encoded by the nucleic acid set forth in SEQ ID NO: 33
SEQ ID NO: 35: the nucleic acid encoding the murine olfactory receptor S25 (Genbank Accession Number AF121977)
SEQ ID NO: 36: the amino acid sequence of the protein encoded by the nucleic acid set forth in SEQ ID NO: 35
SEQ ID NO: 37: the nucleic acid encoding the murine olfactory receptor S46 (Genbank Accession Number AF121979)
SEQ ID NO: 38: the amino acid sequence of the protein encoded by the nucleic acid set forth in SEQ ID NO: 37
SEQ ID NO: 39: the nucleic acid encoding the $\alpha$ subunit of murine G-coupled protein (Genbank Accession Number M36778)
SEQ ID NO: 40: athe mino acid sequence of the protein encoded by the nucleic acid set forth in SEQ ID NO: 39
SEQ ID NO: 41: the nucleic acid encoding the $\beta$ subunit of murine G-coupled protein (Genbank Accession Number M87286)
SEQ ID NO: 42: the amino acid sequence of the protein encoded by the nucleic acid set forth in SEQ ID NO: 41
SEQ ID NO: 43: the nucleic acid encoding the y subunit of murine G-coupled protein (Genbank Accession Number U37527)
SEQ ID NO: 44: the amino acid sequence of the protein encoded by the nucleic acid set forth in SEQ ID NO: 43
SEQ ID NO: 45: the nucleic acid encoding the epidermal growth factor receptor (Genbank Accession Number BC023729)
SEQ ID NO: 46: amino acid sequence of the protein encoded by the nucleic acid set forth in SEQ ID NO: 45

BEST MODE FOR CARRYING OUT THE INVENTION

Hereinafter, the present invention will be described. It should be understood throughout the present specification that articles for a singular form (e.g., "a", "an", "the", etc. in English) include the concept of their plurality unless otherwise stated. It should be also understood that the terms as used herein have definitions as typically used in the art unless otherwise stated. Accordingly, unless otherwise defined, all technical and scientific terms used herein shall have the same meaning as that generally understood by those skilled in the art to which the present invention pertains. If there is any inconsistency, the present specification precedes, including definitions.

(Definition of terms)

Hereinafter, terms specifically used herein will be defined.

(System)

As used herein the term "system" refers to a collection of parts having functional association, for example, an existence separated and extracted from the circumstances as a target of analysis and discussion. Systems include, but are not limited to: for example, scientific systems (for example, physical systems, chemical systems, biological systems (for example, cells, tissues, organs, organisms and the like), geophysical systems, astronomical systems, and the like), social scientific systems (for example, company organization and the like), human scientific systems (for example, history, geography and the like), economic systems (for example, stock price, exchange and the like), machinery systems (for example, computers, apparatus and the like) and the like.

As used herein the term "scientific system" is interchangeably used with "natural scientific system" to refer to any system relating to science and technology (natural science and the like). Scientific systems include, but are not limited to: for example, physical systems, chemical systems, biological systems, geophysical systems, astronomical systems, and the like.

As used herein the term "biological system" refers to any system relating to biology. Accordingly, biological systems include, but are not limited to: for example, biological organisms (bodies), organs, tissues (biological tissues), cells, cellular organelles (for example, chloroplasts, mitochondria, and the like), intracellular fractions, chromosomes, genomes, genetic clusters, and the like.

As used herein the term "social scientific system" refers to any systems relating to social science (for example, politics, law, economy, history, ethnology, and the like). Such social scientific systems include, but are not limited to, for example, company organizations, government organizations, family and the like.

As used herein the term "economic system" refers to any systems relating to economy. Such economic systems include, but are not limited to, for example, stock price, exchange, other economic indices (for example, GNP, GDP and the like) and the like.

As used herein the term "human scientific system" refers to any system relating to human science (for example, philosophy, linguistics, literature, history and the like, and is also called as "culture science"). Such human scientific systems include, but are not limited to, for example, systems capable of being described by means of history, geography (state or country), ethics and the like.

As described hereinbelow in detail, the present invention is mainly described using ta ypical example of biological systems, but it is to be understood that the present invention is not limited to such biological systems.

(Biology)

The term "cell" is herein used in its broadest sense in the art, referring to a structural unit of the tissue of a multicellular organism, which is capable of self replicating, has genetic information and a mechanism for expressing it, and is surrounded by a membrane structure which isolates the cell from the outside. Cells used herein may be either naturally-occurring cells or artificially modified cells (e.g., fusion cells, genetically modified cells, etc.), as long as the cell has a chemical receptor or is capable of having such a chemical receptor introduced therein. Examples of cell sources include, but are not limited to, a single-cell culture; the embryo, blood, or body tissue of normally-grown transgenic animals; a mixture of cells derived from normally-grown cell lines; and the like.

As used herein, the term "digital cell" refers to a collection of at least one experimental data on a cell of experimental interest. These experimental data correlate the experimental conditions and the experimental results of an example conducted upon an actual cell. The digital cell is constituted such that once an experimental condition is given, the experimental result related to said experimental condition will be reproduced. The digital cell contemplated by the present invention comprises any cell which is amenable to an experiment. It should be understood that the description with respect to all the (living) cells described herein can be applied to a digital cell according to the present invention, as long as such description is applicable to the digital cell.

Using digital cells of the present invention allows reproduction of an experimental result of an experiment conducted using an actual cell, in a computer system. As such, the present invention allow research institutes, educational organizations and individuals having no experimental facilities, to conduct education and advanced research relating to a cell. As a result, business entities in different fields will be able to start business in this field, which has not been possible to date. It is understood that the digital cell is appropriate as a system of the target of the present invention.

Cells used herein may be derived from any organism (e.g., any unicellular organism (e.g., bacteria and yeast) or any multicellular organisms(e.g., animals (e.g., vertebrates and invertebrates), plants (e.g., monocotyledons and dicotyledons, etc.)). For example, cells used herein are derived from a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). In one embodiment, cells derived from primates (e.g., chimpanzee, Japanese monkey, human) are used. Particularly, without limitation, cells derived from a human are used. The above-described cells may be either stem cells or somatic cells. Also, the cells may be adherent cells, suspended cells, tissue forming cells, and mixtures thereof. The cells may be used for transplantation.

Any organ may be targeted by the present invention. A tissue or cell targeted by the present invention may be derived from any organ. As used herein, the term "organ" refers to a morphologically independent structure, localized to a particular portion of an individual organism, in which a certain function is performed. In multicellular organisms (e.g., animals, plants), an organ consists of several tissues spatially arranged in a particular manner, each tissue being composed of a number of cells. An example of such an organ includes an organ relating to the vascular system. In one embodiment, organs targeted by the present invention include, but are not limited to, skin, blood vessels, cornea, kidney, heart, liver, umbilical cord, intestine, nerve, lung, placenta, pancreas, brain, peripheral limbs, retina, and the like. As used herein, cells differentiated from a pluripotent cell of the present invention include, but are not limited to: epidermal cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells, and the like.

As used herein, the term "tissue" refers to an aggregate of cells having substantially the same function and/or form in a multicellular organism. "Tissue" is typically an aggregate of cells of the same origin, but may be an aggregate of cells of different origins as long as the cells have the same function and/or form. Therefore, when stem cells of the present invention are used to regenerate tissue, the tissue may be composed of an aggregate of cells of two or more different origins. Typically, a tissue constitutes a part of an organ. Animal tissues are separated into epithelial tissue, connective tissue, muscular tissue, nervous tissue, and the like, on a morphological, functional, or developmental basis. Plant tissues are roughly separated into meristematic tissue and permanent tissue, according to the developmental stage of the cells constituting the tissue. Alternatively, tissues may be separated into single tissues and composite tissues according to the type of cells constituting the tissue. Thus, tissues are separated into various categories.

As used herein the terms "(biological) organism" and "biological body" are interchangeably used in the broadest sense as usually used in the art, and refer to an individual biological entity which execute biological phenomena.

As used herein, the term "isolated" means that naturally accompanying material is at least reduced, or preferably substantially completely eliminated, in normal circumstances. Therefore, the term "isolated cell" refers to a cell substantially free from other accompanying substances (e.g., other cells, proteins, nucleic acids, etc.) in natural circumstances. The term "isolated" in relation to nucleic acids or polypeptides means that, for example, the nucleic acids or the polypeptides are substantially free from cellular substances or culture media when they are produced by recombinant DNA techniques; or precursory chemical substances or other chemical substances when they are chemically synthesized. Isolated nucleic acids are preferably free from sequences naturally flanking the nucleic acid within an organism from which the nucleic acid is derived (i.e., sequences positioned at the 5' terminus and the 3' terminus of the nucleic acid). Therefore, the system of the present invention is preferably such an isolated system.

As used herein, the term "established" in relation to cells refers to a state of a cell in which a particular property (pluripotency) of the cell is maintained and the cell undergoes stable proliferation under culture conditions.

As used herein, the term "state" refers to a condition concerning various parameters of a cell (e.g., cell cycle, response to an external agent, signal transduction, gene expression, gene transcription, etc.). Examples of such a state include, but are not limited to, differentiated states, undifferentiated states, responses to external agents, cell cycles, growth states, and the like. As used herein, the term "gene state" refers to any state associated with a gene (e.g., an expression state, a transcription state, etc.).

As used herein, the terms "differentiation" or "cell differentiation" refers to a phenomenon where two or more types of cells having qualitative differences in form and/or function occur in a daughter cell population derived from the division of a single cell. Therefore, "differentiation" includes a process during which a population (family tree) of cells, which do not originally have a specific detectable feature, acquire a feature, such as production of a specific protein, or the like. At present, cell differentiation is generally considered to be a state of a cell in which a specific group of genes in the genome are expressed. Cell differentiation can be identified by searching for intracellular or extracellular agents or conditions which elicit the above-described state of gene expression. Differentiated cells are stable in principle. Particularly, animal cells which have been once differentiated are rarely differentiated into other types of cells.

(Biochemistry and Molecular Biology)

As used herein, the term "agent" may refer to any substance or element as long as an intended object can be achieved (e.g., energy, such as ionizing radiation, radiation, light, acoustic waves, and the like). Examples of such a substance include, but are not limited to, proteins, polypeptides, oligopeptides, peptides, polynucleotides, oligonucleotides, nucleotides, nucleic acids (e.g., DNA such as cDNA, genomic DNA and the like, or RNA such as mRNA, RNAi and the like), polysaccharides, oligosaccharides, lipids, low molecular weight organic molecules (e.g., hormones, ligands, information transduction substances, low molecular weight organic molecules, molecules synthesized by combinatorial chemistry, low molecular weight molecules usable as medicaments (e.g., low molecular weight molecule ligands, etc.), etc.), and composite molecules thereof. External agents may be used singly or in combination. Examples of an agent specific to a polynucleotide include, but are not limited to, representatively, a polynucleotide having complementarity to the sequence of the polynucleotide with a predetermined sequence homology (e.g., 70% or more sequence identity), a polypeptide such as a transcriptional agent binding to a promoter region, and the like. Examples of an agent specific to a polypeptide include, but are not limited to, representatively, an antibody specifically directed to the polypeptide or derivatives or analogs thereof (e.g., single chain antibody), a specific ligand or receptor when the polypeptide is a receptor or ligand, a substrate when the polypeptide is an enzyme, and the like.

As used herein the term "biological agent" refers to an agent relating to a biological organism (for example, a cell). Preferably, an agent present in a cell in a normal state is referred to as a biological agent. Such biological agents include, but are not limited to, for example: nucleic acid molecules, proteins, sugars, lipids, metabolites, low molecular weight molecules, and complexes thereof, and agents including time elements and the like. Alternatively, it should be understood that such biological agents include electric current, electric potential (such as membrane potential), pH, osmotic pressure and the like, in the present invention. Useful biological agents as used herein include, for example, transcriptional controlling sequence (for example, promoters and the like), structural genes, and nucleic acids encoding the same. As used herein a "collection" of "biological agents" refer to a plurality of biological agents (of the same or different types). Preferably, the collection refers to biological agents which cooperate with each other.

As used herein, the term "gene" refers to an element defining a genetic trait. A gene is typically arranged in a given sequence on a chromosome. A gene which defines the first-order structure of a protein is called a structural gene. A gene which regulates the expression of a structural gene is called a regulatory gene (e.g., promoter). Genes herein include structural genes and regulatory genes unless otherwise specified. Therefore, the term "cyclin gene" typically includes the structural gene of cyclin and the promoter of cyclin. As used herein, "gene" may refer to "polynucleotide", "oligonucleotide", "nucleic acid", and "nucleic acid molecule" and/or "protein", "polypeptide", "oligopeptide" and "peptide". As used herein, "gene product" includes "polynucleotide", "oligonucleotide", "nucleic acid" and "nucleic acid molecule" and/or "protein", "polypeptide", "oligopeptide" and "peptide", which are expressed by a gene. Those skilled in the art understand what a gene product is, according to the context.

As used herein, the term "homology" in relation to a sequence (e.g., a nucleic acid sequence, an amino acid sequence, etc.) refers to the level of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other. As used herein, the term "similarity" in relation to a sequence (e.g., a nucleic acid sequence, an amino acid sequence, or the like) refers to the level of identity between two or more sequences when conservative substitution is regarded as positive (identical) in the above-described homology. Therefore, homology and similarity differ from each other in the presence of conservative substitutions. If no conservative substitutions are present, homology and similarity have the same value.

As used herein, the comparison of similarity, identity and homology of an amino acid sequence and a nuleotide sequence is calculated with FASTA, a tool for sequence analysis using default parameters.

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or nonnaturally-occurring amino acid, or a variant amino acid. The term may include those assembled into a composite of a plurality of polypeptide chains. The term also includes a naturally-occurring or artificially modified amino acid polymers. Such modification includes, for example, disulfide bond formation, glycosylation, lipidation, acetylation, phosphorylation, or any other manipulation or modification (e.g., conjugation with a labeling moiety). This definition encompasses a polypeptide containing at least one amino acid analog (e.g., nonnaturally-occurring amino acid, etc.), a peptide-like compound (e.g., peptoid), and other variants known in the art, for example. Gene products, such as extracellular matrix proteins (e.g., fibronectin, etc.), are usually in the form of a polypeptide.

The terms "polynucleotide", "oligonucleotide", "nucleic acid molecule" and "nucleic acid" as used herein have the same meaning and refer to a nucleotide polymer having any length. This term also includes an "oligonucleotide derivative" or a "polynucleotide derivative". An "oligonucleotide derivative" or a "polynucleotide derivative" includes a nucleotide derivative, or refers to an oligonucleotide or a polynucleotide having different linkages between nucleotides from typical linkages, which are interchangeably used. Examples of such an oligonucleotide specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2'-methoxyethoxy ribose. Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)). A gene encoding an extracellular matrix protein (e.g., fibronectin, etc.) or the like is usually in the form of polynucleotide. A molecule to be transfected is in the form of polynucleotide.

As used herein, the term "corresponding" amino acid or nucleic acid refers to an amino acid or nucleotide in a given polypeptide or polynucleotide molecule, which has, or is anticipated to have, a function similar to that of a predetermined amino acid or nucleotide in a polypeptide or polynucleotide as a reference for comparison. Particularly, in the case of enzyme molecules, the term refers to an amino acid which is present at a similar position in an active site and similarly contributes to catalytic activity. For example, in the case of antisense molecules for a certain polynucleotide, the term refers to a similar portion in an ortholog corresponding to a particular portion of the antisense molecule.

As used herein, the term "corresponding" gene (e.g., a polypeptide or polynucleotide molecule) refers to a gene in a given species, which has, or is anticipated to have, a function similar to that of a predetermined gene in a species as a reference for comparison. When there are a plurality of genes having such a function, the term refers to a gene having the same evolutionary origin. Therefore, a gene corresponding to a given gene may be an ortholog of the given gene. Therefore, genes corresponding to mouse cyclin genes can be found in other animals. Such a corresponding gene can be identified by techniques well known in the art. Therefore, for example, a corresponding gene in a given animal can be found by searching a sequence database of the animal (e.g., human, rat) using the sequence of a reference gene (e.g., mouse cyclin gene, etc.) as a query sequence.

As used herein, the term "fragment" with respect to a polypeptide or polynucleotide refer to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. As used herein, the length of polypeptides or polynucleotides can be represented by the number of amino acids or nucleic acids, respectively. However, the above-described numbers are not absolute. The above-described numbers as the upper or lower limit are intended to include some greater or smaller numbers (e.g., ±10%), as long as the same function is maintained. For this purpose, "about" may be herein put ahead of the numbers. However, it should be understood that the interpretation of numbers is not affected by the presence or absence of "about" in the present specification.

As used herein, the term "biological activity" refers to activity possessed by an agent (e.g., a polynucleotide, a protein, etc.) within an organism, including activities exhibiting various functions (e.g., transcription promoting activity, etc.). For example, when a certain factor is an enzyme, the biological activity thereof includes its enzyme activity. In another example, when a certain factor is a ligand, the biological activity thereof includes the binding of the ligand to a receptor corresponding thereto. The above-described biological activity can be measured by techniques well-known in the art.

As used herein, the term "polynucleotides hybridizing under stringent conditions" refers to conditions commonly used and well known in the art. Such a polynucleotide can be obtained by conducting colony hybridization, plaque hybridization, Southern blot hybridization, or the like using a polynucleotide selected from the polynucleotides of the present invention. Specifically, a filter on which DNA derived from a colony or plaque is immobilized is used to conduct hybridization at 65˚C in the presence of 0.7 to 1.0 M NaCl. Thereafter, a 0.1 to 2-fold concentration SSC (saline-sodium citrate) solution (1-fold concentration SSC solution is composed of 150 mM sodium chloride and 15 mM sodium citrate) is used to wash the filter at 65˚C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A or T. "Hybridizable polynucleotide" refers to a polynucleotide which can hybridize other polynucleotides under the above-described hybridization conditions. Specifically, the hybridizable polynucleotide includes at least a polynucleotide having a homology of at least 60% to the base sequence of DNA encoding a polypeptide having an amino acid sequence specifically herein disclosed, preferably a polynucleotide having a homology of at least 80%, and more preferably a polynucleotide having a homology of at least 95%.

As used herein, the term "salt" has the same meaning as that commonly understood by those skilled in the art, including both inorganic and organic salts. Salts are typically generated by neutralizing reactions between acids and bases. Salts include NaCl, $K_2SO_4$, and the like, which are generated by neutralization, and in addition, $PbSO_4$ $ZnCl_2$, and the like, which are generated by reactions between metals and acids. The latter salts may not be generated directly by neutralizing reactions, but may be regarded as a product of neutralizing reactions between acids and bases. Salts may be divided into the following categories: normal salts (salts without any H of acids or without any OH of bases, including, for example, NaCl, $NH_4Cl$, $CH_3COONa$, and $Na_2CO_3$), acid salts (salts with remaining H of acids, including, for example, $NaHCO_3$, $KHSO_4$, and $CaHPO_4$), and basic salts (salts with remaining OH of bases, including, for example, MgCl(OH) and CuCl(OH)). This classification is not very important in the present invention. Examples of preferable salts include salts constituting medium (e.g., calcium chloride, sodium hydrogen phosphate, sodium hydrogen carbonate, sodium pyruvate, HEPES, sodium chloride, potassium chloride, magnesium sulfide, iron nitrate, amino acids, vitamins, etc.), salts constituting buffer (e.g., calcium chloride, magnesium chloride, sodium hydrogen phosphate, sodium chloride, etc.), and the like. These salts are preferable as they have a high affinity for cells and thus are better able to maintain cells in culture. These salts may be used singly or in combination. Preferably, these salts may be used in combination. This is because a combination of salts tends to have a higher affinity for cells. Therefore, a plurality of salts (e.g., calcium chloride, magnesium chloride, sodium hydrogen phosphate, and sodium chloride) are preferably contained in a medium, rather than only NaCl or the like. More preferably, all salts for cell culture medium may be added to the medium. In another preferred embodiment, glucose may be added to medium.

As used herein the term "material" or "substance" is used in the broadest meaning as used in the art to refer to any thing that is positively or negatively charged.

As used herein, the term "positively charged substance" encompasses all substances having a positive charge. Such substances include cationic substances such as cationic polymers, cationic lipids and the like, but are not limited to these. Advantageously, such positively charged substances can form a complex. Such positively charged substances which can form a complex include, for example, substances having a certain molecular weight (for example, cationic polymers) and substances which can remain insoluble, that is, without being dissolved to a certain extent in a specific solvent such as water, an aqueous solution or the like (for example, cationic lipids), but are not limited to these. Preferable positively charged substances include, for example, polyethylene imine, poly-L-lysine, synthetic polypeptides, or derivatives thereof, but are not limited to these. Positively charged substances include, for example, biological molecules such as histone and synthetic polypeptides, but are not limited to these. The type of preferable positively charged substances changes in accordance with the type of negatively charged substances, which act as a complex partner to form complexes with the positively charged substances. It requires no specific creativity for those skilled in the art to select a preferable complex partner using technology well known in the art. For selecting a preferable complex partner, various parameters are considered including, but not limited to, charge, molecular weight, hydrophobicity, hydrophilicity, properties of substituents, pH, temperature, salt concentration, pressure, and other physical and chemical parameters.

As used herein, the term "cationic polymer" refers to a polymer having a cationic functional group, and encompasses, for example, polyethylene imine, poly-L-lysine, synthetic polypeptides, and derivatives thereof, but is not limited to these.

As used herein, the term "cationic lipid" refers to a lipid having a cationic functional group, and encompasses, for example, phosphatidyl choline, phosphatidyl ethanol amine, phosphatidyl serine, and derivatives thereof, but is not limited to these.

Cationic functional groups include, for example, first-order amines, second-order amines, and tertiary amines, but are not limited to these.

As used herein, the term "negatively charged substance" encompasses all substances having a negative charge. Such substances include biological molecular polymers, anionic substances such as anionic lipids, and the like, but are not limited to these. Advantageously, such negatively charged substances can form a complex. Such negatively charged substances which can form a complex include, for example, substances having a certain molecular weight (for example, anionic polymers such as DNA) and substances which can remain insoluble, that is, without being dissolved to a certain extent in a specific solvent such as water, an aqueous solutions or the like (for example, anionic lipids), but are not limited to these. Preferable negatively charged substances include, for example, DNA, RNA, PNA, polypeptides, chemical compounds, and complexes thereof, but are not limited to these. Negatively charged substances include, for example, DNA, RNA, PNA, polypeptides, chemical compounds, and complexes thereof, but are not limited to these. The type of preferable negatively charged substances changes in accordance with the type of positively charged substances, which act as a complex partner to form complexes with the negatively charged substances. It requires no specific creativity for those skilled in the art to select a preferable complex partner using technology well known in the art. For selecting a preferable complex partner, various parameters are considered as described above with regard to negatively charged substances.

As used herein, the term "anionic polymer" encompasses polymers having an anionic functional group, and includes, for example, DNA, RNA, PNA, polypeptides, chemical compounds, and complexes thereof, but is not limited to these.

As used herein, the term "anionic lipid" encompasses lipids having an anionic functional group, and include, for example, phosphatidic acid, phosphatidyl serine, but is not limited to these.

Anionic functional groups include, for example, carboxylic groups and phosphoric acid groups, but are not limited to these.

The type of charge of a target substance can be converted by adding a part of a substituent or the like having a positive charge or a negative charge to the target substance. In the case where a preferable complex partner has the same type of charge as that of the target substance, formation of a complex can be promoted by converting the type of charge of either the complex partner or the target substance.

As used herein, the term "complex" refers to two or more substances which directly or indirectly interact with each other and as a result, act as if they were one substance as a whole.

As used herein, the term "complex partner" used for a certain member forming a complex refers to another member interacting with the certain member directly or indirectly.

As used herein, the condition for forming a complex changes in accordance with the type of complex partner. Such a condition can be easily understood by those skilled in the art. Those skilled in the art can easily form a complex from any complex partners (for example, a positively charged substance and a negatively charged substance) using a technique well known in the art.

As used herein, when a complex of positively and negatively charged substances is used, either or both thereof may be identical to a biological agent.

As used herein, the term "immobilization" used for a solid-phase support refers to a state in which a substance as a subject of immobilization (e. g., a biological molecule) is held on the support for at least a certain time period, or an act-of placing the substance into such a state. As such, in the case where the condition is changed after the substance is immobilized on the solid-phase support (for example, the substance is immersed in another solvent), the substance may be released from the immobilization state.

As used herein, the term "cell affinity" refers to a property of a substance that when the substance is placed in an interactable state with a cell (e. g. germ cell, animal cell, yeast, plant cell) or an object containing a cell (e. g., tissue, organs, biological organisms), the substance does not have any adverse influence on the cell or the object containing the cell. Preferably, substances having cell affinity may be substances with which a cell interacts as a priority, but are not limited to these. According to the present invention, the substance to be immobilized (e. g., positively charged substances and/or negatively charged substances) preferably have cell affinity, but cell affinity is not absolutely necessary. It was unexpectedly found that when the substance to be immobilized has cell affinity, the cell affinity of the substance is maintained or improved when the substance is immobilized according to the present invention. In light of the past situation where a substance having cell affinity does not necessarily maintain its cell affinity when immobilized on a solid-phase support, the effect of the present invention is enormous.

As used herein, the term "probe" refers to a substance for use in searching, which is used in a biological experiment, such as *in vitro* and/or *in vivo* screening or the like, including, but not limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90% or at least 95%.

As used herein, the term "search" indicates that a given nucleic acid sequence is utilized to find other nucleic acid base sequences having a specific function and/or property either electronically or biologically, or using other methods. Examples of an electronic search include, but are not limited to, BLAST (Altschul et al., J. Mol. Biol. 215:403-410 (1990) ), FASTA (Pearson & Lipman, Proc. Natl. Acad. Sci., USA 85:2444-2448 (1988)), the Smith and Waterman method (Smith and Waterman, J. Mol. Biol. 147:195-197 (1981)), and the Needleman and Wunsch method (Needleman and Wunsch, J. Mol. Biol. 48:443-453 (1970)), and the like. Examples of a biological search include, but are not limited to, a macroarray in which genomic DNA is attached to a nylon membrane or the like or a microarray (microassay) in which genomic DNA is attached to a glass plate under stringent hybridization conditions, PCR, in situ hybridization, and the like.

As used herein, the term "primer" refers to a substance required for the initiation of a reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and most preferably at least 95%. An appropriate sequence as a primer may vary depending on the property of the sequence to be synthesized (amplified). Those skilled in the art can design an appropriate primer depending on the sequence of interest. Such primer design is well known in the art and may be performed manually or using a computer program (e.g., LASERGENE, Primer Select, DNAStar).

As used herein, the term "epitope" refers to an antigenic determinant. Therefore, the term "epitope" includes a set of amino acid residues which are involved in recognition by a particular immunoglobulin, or in the context of T cells, those residues necessary for recognition by the T cell receptor proteins and/or Major Histocompatibility Complex (MHC) receptors. This term is also used interchangeably with "antigenic determinant" or "antigenic determinant site". In the field of immunology, *in vivo* or *in vitro*, an epitope is the features of a molecule (e.g., first-order, second-order and tertiary peptide structure, and charge) that form a site recognized by an immunoglobulin, T cell receptor or HLA molecule. An epitope including a peptide comprises 3 or more amino acids in a spatial conformation which is unique to the epitope. Generally, an epitope consists of at least 5 such amino acids, and more ordinarily, consists of at least 6, 7, 8, 9 or 10 such amino acids. The greater the length of an epitope, the more the similarity of the epitope to the original peptide, i.e., longer epitopes are generally preferable. This is not necessarily the case when the conformation is taken into account. Methods of determining the spatial conformation of amino acids are known in the art, and include, for example, X-ray crystallography and 2-dimensional nuclear magnetic resonance spectroscopy. Furthermore, the identification of epitopes in a given protein is readily accomplished using techniques well known in the art. See, also, Geysen et al., Proc. Natl. Acad. Sci. USA (1984) 81: 3998 (general method of rapidly synthesizing peptides to determine the location of immunogenic epitopes in a given antigen); U. S. Patent No. 4,708,871 (procedures for identifying and chemically synthesizing epitopes of antigens); and Geysen et al., Molecular immunology (1986) 23: 709 (technique for identifying peptides with high affinity for a given antibody). Antibodies that recognize the same epitope can be identified in a simple immunoassay. Thus, methods for determining epitopes including a peptide are well known in the art. Such an epitope can be determined using a well-known, common technique by those skilled in the art if the first-order nucleic acid or amino acid sequence of the epitope is provided.

Therefore, an epitope including a peptide requires a sequence having a length of at least 3 amino acids, preferably at least 4 amino acids, more preferably at least 5 amino acids, at least 6 amino acids, at least 7 amino acids, at least 8 amino acids, at least 9 amino acids, at least 10 amino acids, at least 15 amino acids, at least 20 amino acids, and 25 amino acids.

As used herein, the term "agent binding specifically to" a certain nucleic acid molecule or polypeptide refers to an agent which has a level of binding to the nucleic acid molecule or polypeptide equal to or higher than a level of binding to other nucleic acid molecules or polypeptides. Examples of such an agent include, but are not limited to, when a target is a nucleic acid molecule, a nucleic acid molecule having a complementary sequence of a nucleic acid molecule of interest, a polypeptide capable of binding to a nucleic acid sequence of interest (e.g., a transcription agent, etc.), and the like, and when a target is a polypeptide, an antibody, a single chain antibody, either of a pair of a receptor and a ligand, either of a pair of an enzyme and a substrate, and the like.

As used herein, the term "antibody" encompasses polyclonal antibodies, monoclonal antibodies, human antibodies, humanized antibodies, polyfunctional antibodies, chimeric antibodies, and anti-idiotype antibodies, and fragments thereof (e.g., F(ab')2 and Fab fragments), and other recombinant conjugates. These antibodies may be fused with an enzyme (e.g., alkaline phosphatase, horseradish peroxidase, $\alpha$-galactosidase, and the like) via a covalent bond or by recombination.

As used herein, the term "monoclonal antibody" refers to an antibody composition having a group of homologous antibodies. This term is not limited by the production manner thereof. This term encompasses all immunoglobulin molecules and Fab molecules, F(ab')2 fragments, Fv fragments, and other molecules having an immunological binding property of the original monoclonal antibody molecule. Methods for producing polyclonal antibodies and monoclonal antibodies are well known in the art, and will be more sufficiently described below.

Monoclonal antibodies are prepared by using the standard technique well known in the art (e.g., Kohler and Milstein, Nature (1975) 256:495) or a modification thereof (e.g., Buck et al. (1982) In Vitro 18:377). Representatively, a mouse or rat is immunized with a protein bound to a protein carrier, and boosted. Subsequently, the spleen (and optionally several large lymph nodes) is removed and dissociated into a single cell suspension. If desired, the spleen cells may be screened (after removal of nonspecifically adherent cells) by applying the cell suspension to a plate or well coated with a protein antigen. B-cells that express membrane-bound immunoglobulin specific for the antigen bind to the plate, and are not rinsed away with the rest of the suspension. Resulting B-cells, or all dissociated spleen cells, are then induced to fuse with myeloma cells to form hybridomas. The hybridomas are used to produce monoclonal antibodies.

As used herein, the term "antigen" refers to any substrate to which an antibody molecule may specifically bind. As used herein, the term "immunogen" refers to an antigen capable of initiating activation of the antigen-specific immune response of a lymphocyte.

In a given protein molecule, a given amino acid may be substituted with another amino acid in a structurally important region, such as a cationic region or a substrate molecule binding site, without a clear reduction or loss of interactive binding ability. A given biological function of a protein is defined by the interactive ability or other property of the protein. Therefore, a particular amino acid substitution may be performed in an amino acid sequence, or at the DNA sequence level, to produce a protein which maintains the original property after the substitution. Therefore, various modifications of peptides as disclosed herein and DNA encoding such peptides may be performed without clear losses of biological activity.

When the above-described modifications are designed, the hydrophobicity indices of amino acids may be taken into consideration. The hydrophobic amino acid indices play an important role in providing a protein with an interactive biological function, which is generally recognized in the art (Kyte, J. and Doolittle, R.F., J. Mol. Biol. 157(1):105-132, 1982). The hydrophobic property of an amino acid contributes to the second-order structure of a protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0.5. It is understood in the art that such an amino acid substitution based on hydrophobicity is efficient.

Hydrophilicity may also be considered for conservative substitution. As described in US Patent No. 4, 554, 101, amino acid residues are given the following hydrophilicity indices: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ±1, and even more preferably ±0.5.

(Information of a system, profile, event and the relevant technologies thereof)

As used herein the term "information" of a system, refers to any element describing the system.

As used herein, the term "state" with respect to a system (e.g., a cell, a biological organism and the like) refers to any state relating to the system (e.g., a cell, a biological organism and the like), including, in the case of cells, a differentiation state, an an undifferentiation state, a cellular response to a external agent, displacement over time, cellular cycle, an aging state, a proliferation state and the like; in the case of biological organisms, brain waves, electrocardiogram, pulse, body temperature, blood pressure, MRI image, body weight, body height, blood composition (cellular composition, component composition) and the like; in the case of economic systems, stock quotation, other economic indices and the like.

Thus, as used herein, the term "index" relating to a state, refers to a function which is a hallmark to express the state. As used herein, for example, in the case of biological organisms or cells, the following can be used as such an "index" relating to the state: responses or resistance to: a variety of physical indices of the organism or cells (potential, in vivo temperature, migration rate, migration distance, localization ratio, ellipticity, elongationrate, rotation rate and the like), chemical indices (genome amount, transcription product of a particular gene (for example, mRNA), translated proteins, post-translationally modified proteins, ionic concentration, values of pH and the like, amount of metabolites, amount of ions and the like), biological indices (for example, individual differences, evolution rate, drug reponses and the like), and the like, and alternatively, the environment of the organisms or cells, for example, temperature, humidity (for example, absolute humidity, relative humidity, and the like), pH, salt concentration (for example, concentratino of entire salts or a particular salt), nutrition (for example, amount of vitamins, lipid, proteins, carbohydrates, metal ion concentration and the like), metal (for example, the entire amount of metals, or particular metals (for example, heavy metals, light metals, and the like), gas (for example, entire amount of gas, or a particular gas (for example, oxygen, carbon dioxide, hydrogen and the like) and the like), organic solvents (for example, the entire amount of organic solvent or a particular organic solvent (for example, ethanol and the like), DMAO, amount of methanol), pressure (for example, local pressure or the entire pressure (for example, air pressure, water pressure) and the like), viscosity, flow rate (for example, flow rate of a medium when the organism is present in the medium, membrane flow and the like, light intensity (for example, light intensity of a wave at a particular wave length (for example, ultaviolet ray, infrared ray, and the like in addition to visible light), electromagnetic wave, radiation, gravity, tensile, sonic wave, a biological organism which is different from the organism of interest (for example, parasites, pathogenic bacteria, bacteria, viruses and the like), chemicals (for example, pharmaceutical products, food additives, agricultrual chemicals, fertilizer, environmental hormones, and the like), antibiotics, natural products, phychological stress, physical stress, and the like.

As used herein, the term "profile" in relation to a system refers to a set of measurements of the biological state of the system. Particularly, the term "profile of a cell" refers to a set of discrete or continuous values obtained by quantitatively measuring a level or index of the cell. Such a level or index includes the expression level of a gene, the transcription level of a gene (the activity level of a transcription control sequence), the amount of mRNA encoding a specific gene, and the expression level of a protein in biological systems. The level of each cellular component, such as the expression level of mRNA and/or protein, is known to alter in response to treatment with drugs or cellular biological perturbation or modulation. Therefore, the measurement of a plurality of "cellular components" generates a large amount of information about the effects of stimuli on the biological state of a cell. Therefore, the profile is more and more important in the analysis of cells. Mammalian cells contain about 30,000 or more cellular components. Therefore, the profile of an individual cell is usually complicated. A profile in a predetermined state of a biological system may often be measured after stimulating the biological system. Such stimulation is performed under experimental or environmental conditions associated with the biological system. Examples of a stimulus include exposure of a biological system to a drug candidate, introduction of an exogenous gene, passage of time, deletion of a gene from the system, alteration of culture conditions, and the like. $\alpha$ wide range of measurements of cellular components (i.e., profiles of gene replication or transcription, protein expression, and response to stimuli) has a high level of utility including comparison and investigation of the effects of drugs, diagnosis of diseases, and optimization of drug administration to patients, as well as investigation of cells. Further, profiles are useful for basic life science research. Such profile data may be produced and presented as data in a variety of formats. Such formats include, but are not limited to: a function between a numerical value and a period of time, a graphic format, a image format and the like. Accordingly, data relating to a profile may also be called "profile data" as used herein. Such data production may readily be carried out using a computer. Coding an appropriate program may also be carried out by using well technology in the art. Of course, such profiles may also be described for the other systems (e.g., economic systems, social scientific systems and the like) in the same manner as the biological systems.

As used herein, the term "time-lapse profile" in relation to a certain cell refers to a profile which indicates time-lapse changes in a parameter relating to the cell. Examples of a time-lapse profile include, but are not limited to, a time-lapse profile of transcription levels, a time-lapse profile of expression levels (translation levels), a time-lapse profile of signal transduction, a time-lapse profile of neural potential, and the like. A time-lapse profile may be produced by continuously recording a certain parameter (e.g., a signal caused by a label associated with a transcription level). Time-lapse measurement may mean continuous measurement. Therefore, the term "time-lapse profile" as used herein may also be referred to as "continuous profile".

As used herein the term "time-series data" refers to a representation of data relating to a certain index which is represented in a time-series manner. Accordingly, the time-series data may overlap with the time lapse data in terms of concept. Time-series data may be discontinuous or continuous data due to the method of production, obtaining or recording of data used. AS such, in the present invention, time-series data may be discontinuous or continuous.

As used herein the term "profile" and "data" may be used in an overlapping manner, and profile refers to, as described above, a collection of measurements, and thus is encompassed by the concept of data.

As used herein, the term "characteristic behaviour" refers to a particular pattern of data, which may be arbitrarily determined by an observer, and includes, but is not limited to, for example, the inflection point of a first-order differentiation. As such, characteristic behaviour may be expressed in a two-dimensional manner, or one-dimensional manner.

As used herein the term "event" of a system refers to an event of any change of a state and those events relating thereto. Accordingly, the event may be clearly distinguished from the data (time-series data) of the system *per* se. Conventionally, there has been no such an example which describes a system noticing such an event. Such an event includes but is not limited to, when a cell is targeted, for example, a change in differentiation state of a cell, a response to a foreign agent in a cell, a change in cellular cycle in a cell, a change in the apoptotic state in a cell, a response to an environmental change in a cell, a change of aging state in a cell, and the like. Alternatively, when a biological organism is targeted, it includes, but is not limited to, a change in brain wave, a change in mouth odor, a change in psychology, inspiration, myocardial infarct, a state of life or death, birth of a life, emotion, memory, and the like. In an economic system, for example, it includes, but is not limited to, a sharp rise or drop in stock price, a sharp change in currency exchange, and the like. It is also understood that a change in historical fact, a transision in a state of a country, self-organization and the like are also an appropriate target of description and analysis.

As used herein the term "index" of a system refers to any indices describing the state of a system. In the case of a cell, for example, the term includes, but is not limited to: gene expression level, gene transcription level, post-transcriptional modification level of a gene, the level of a chemical substance present in a cell, intracellular ion level, cell size, biochemical process level, and biophysiological process level, and the like. In the cases of targeting a biological organism, the indices include, but are not limited to: brain wave level, electrocardiodiagram level, pulse, blood pressure, blood glucose level, cholesterol value, neutral lipid level, vigilance level, alpha wave, beta wave, and the like. In the cases of targeting an economic system, the indices include, but are not limited to: stock price, exchange, other economic indices (for example, GDP and the like), and the like.

As used herein the term "event timing" refers to a timing at which an event occurs. As used herein, the term "timing" is a manner of describing a time, and may be described by means of a time point or a time range (herein called "time range"). The timing may be described by means of an absolute or relative time. As used herein, the term "time point" refers to a manner of describing a time, which can substantially be described with a single point. When the time point is usually referred to, it has no time range, but in terms of the problems associated with a system of measurement, it may be described by means of a time range of a minimal unit which can be detected. As used herein, the term "time range" refers to, when describing a state of the aspect of a time, a manner of description of a certain period of time. Such a time range may include, for example, an order of seconds, minutes to hours, or days, months, years, or the like, depending on the system used. Those skilled in the art can appropriately select such a time range. As usehd herein, amongst event descriptors, in particular, those which coincide in the behaviour thereof (for example, identity in characteristic behaviour, time point, time range, pattern, and the like) are called coincidence event timing, and coincidence event timing may be individual event timing or a group of event timings or a pattern thereof.

As used herein, the term "event descriptor" refers to a descriptor for describing an event. As used herein, the term "descriptor" refers to a method of describing particular information or a representation described thereby. As used herein, the term "descriptor" may refer to "event descriptor". Event descriptors may be represented by an electric wave, a magnetic wave, sound, light, color, image, number, letter/character and the like and combinations thereof.

In the analysis according to the present invention, any of a variety of detection methods and means may be used, as long as such methods and means may be used to detect information due to a system or an agent interacting thereto. Such a method and means for detection includes but is not limited to, when a biological organism or cell is targeted, for example, methods and means using gross inspection, an optical microscope, a fluorescence microscope, a reading apparatus using a laser light source, a surface plasmon resonance (SPR), an imaging apparatus, an electric signal, a chemical or biochemical marker or a plurality thereof, and the like.

As used herein, the term "environment" (or "Umgebung" in German) in relation to an entity refers to a circumstance which surrounds the entity. In an environment, various components and quantities of state are recognized, which are called environmental factors. Examples of environmental factors include the above-described parameters. Environmental factors are typically roughly divided into non-biological environmental factors and biological environmental factors. Non-biological environmental factors (inorganic environment factors) may be divided into physical factors and chemical factors, or alternatively, climatic factors and soil factors. Various environmental factors do not always act on organisms independently, but may be associated with one another. Therefore, environmental factors may be herein observed one by one or as a entirety of various parameters. It has been believed that it was difficult to maintain such an environment in a consistent state. This is particularly the case since it has been difficult to maintain cells and to immobilize cells, and to introduce substances such as nucleic acids into a cell. The present invention has also solved at least one of these problems. As used herein the term "consistent environment" refers to substantially all of the circumstances surrounding a cell of interest. Accordingly, as long as a cell can grow or differentiate in a similar manner, such environments are deemed to be consistent environments. As used herein, a consistent environment refers to an environment where the parameters are the same except for a specific stimulus (for example, an external stimulus).

Examples of factors considering such an environment e.g. with respect to scientific systems such as biological systems, as a parameter, includes at least one factor as an index, selected from the group consisting of temperature, humidity, pH, salt concentration, nutrients, metal, gas, organic solvent, pressure, atmospheric pressure, viscosity, flow rate, light intensity, light wavelength, electromagnetic waves, radiation, gravity, tension, acoustic waves, organisms (e.g., parasites, etc.) other than the organism, chemical agents, antibiotics, natural substances, mental stress, and physical stress, and any combination thereof. In the case of social scientific systems, e.g., factors such as population, capital and the like may be used as an index, but is not limited thereto.

Examples of temperature include, but are not limited to, high temperature, low temperature, very high temperature (e.g., 95˚C, etc.), very low temperature (e.g., -80˚C, etc.), a wide range of temperature (e.g., 150 to -270˚C, etc.), and the like.

Examples of humidity include, but are not limited to, a relative humidity of 100%, a relative humidity of 0%, an arbitrary point from 0% to 100%, and the like.

Examples of pH include, but are not limited to, an arbitrary point from 0 to 14, and the like.

Examples of salt concentration include, but are not limited to, a NaCl concentration (e.g., 3%, etc.), an arbitrary point of other salt concentrations from 0 to 100%, and the like.

Examples of nutrients include, but are not limited to, proteins, glucose, lipids, vitamins, inorganic salts, and the like.

Examples of metals include, but are not limited to, heavy metals (e.g., mercury, cadmium, etc.), lead, gold, uranium, silver, and the like.

Examples of gas include, but are not limited to, oxygen, nitrogen, carbon dioxide, carbon monoxide, and a mixture thereof, and the like.

Examples of organic solvents include, but are not limited to, ethanol, methanol, xylene, propanol, and the like.

Examples of pressure include, but are not limited to, an arbitrary point from 0 to 10 ton/$cm^2$, and the like.

Examples of atmospheric pressure include, but are not limited to, an arbitrary point from 0 to 100 atmospheric pressure, and the like.

Examples of viscosity include, but are not limited to the viscosity of any fluid (e. g. water, glycerol, etc.) or a mixture thereof, and the like.

Examples of flow rate include, but are not limited to an arbitrary point from 0 to the velocity of light.

Examples of light intensity include, but are not limited to, a point between darkness and the level of sunlight.

Examples of light wavelength include, but are not limited to visible light, ultraviolet light (UV-A, UV-B, UV-C, etc.), infrared light (far infrared light, near infrared light, etc.), and the like.

Examples of electromagnetic waves include ones having an arbitrary wavelength.

Examples of radiation include ones having an arbitrary intensity.

Examples of gravity include, but are not limited to, an arbitrary gravity on the Earth or an arbitrary point from zero gravity to the gravity on the Earth, or an arbitrary gravity greater than or equal to a gravity on the Earth.

Examples of tension include ones having an arbitrary strength.

Examples of acoustic waves include ones having an arbitrary intensity and wavelength.

Examples of organisms other than an organism of interest include, but are not limited to, parasites, pathogenic bacteria, insects, nematodes, and the like.

Examples of chemicals include, but are not limited to hydrochloric acid, sulfuric acid, sodium hydroxide, and the like.

Examples of antibiotics include, but are not limited to, penicillin, kanamycin, streptomycin, quinoline, and the like.

Examples of naturally-occurring substances include, but are not limited to, puffer-fish toxin, snake venom, alkaloid, and the like.

Examples of physical stress include, but are not limited to vibration, noise, electricity, impact, and the like.

As used herein when referring to a digital cell of the present invention, the environment is presented as an "environment parameter". Such environment parameters include, but are not limited to, medium (type, composition), pH, temperature, moisture, $CO_2$ concentration, $O_2$ concentration, the presence or absence of an antibiotic, the presence or absence of a particular nutrient and the like.

As used herein the term "stimulant" refers to an active agent which causes or induces expression or enhancement of a specific living activity, given to a cell from outside. Stimuli include, but are not limited to: a physical stimulus, a chemical stimulus, a biological stimulus, a biochemical stimulus, and the like. Physical stimuli include, but are not limited to: for example, light, electric waves, electric current, pressure, sound (vibration) and the like. Chemical stimuli include but are not limited to: for example, stimuli from chemicals such as antibiotics, nutrients, vitamins, metals, ions, acids, alkalis, salts, buffers and the like. Biological stimuli include, but are not limited to: for example, the existence of another organism such as the existence of a parasitic organism or the density of a cell population and the like. Biochemical stimuli include, but are not limited to the existence of cell signaling transduction agents, and the like. A social scientific stimulus includes, but is not limited to, restructuring of an organization, war, law amendment and the like.

As used herein, a stimulus may be presented as a "stimulus index". Any index corresponding to the stimulus as described above, may be used as a stimulus index. As used herein, it should be understood that the stimulus index includes an agent (for example, a reporter) for transducing a stimulus. Such a reporter includes, when a cell is targeted, for example, on-off against an antibiotic, transcription controlling sequence, radioactivity, fluorescent substances, and the like.

As used herein the term "response" to a stimulus refers to any response of a cell to a stimulus such as a change in cell morphology, change in metabolism, change in other cellular behaviors, change in signal transduction and the like. Therefore, for example, results of experiments using the digital cell of the present invention may be recorded as cell dynamics data. Alternatively, when using the above reporter, the result of such a response to the stimulus may be raw data of the reporter, or data transformed from the data of the reporter.

As used herein, the term "transcription control sequence" refers to a sequence which can regulate the transcription level of a gene. Such a sequence is at least two nucleotides in length. Examples of such a sequence include, but are not limited to, promoters, enhancers, silencers, terminators, sequences flanking other genomic structural genes, genomic sequences other than exons, sequences within exons, and the like. A transcription control sequence used herein is not related to a particular type. Rather, important information about a transcription control sequence is thr time-lapse fluctuation thereof. Such fluctuation is referred to as a process (changes in a state of a cell). Therefore, such a transcription control sequence may be herein arbitrarily selected. Such a transcription control sequence may include those which are not conventionally used as markers. Preferably, a transcription control sequence has the ability to bind to a transcription factor.

As used herein, the term "transcription factor" refers to a factor which regulates the process of transcription of a gene. The term "transcription factor" mainly indicates a factor which regulates a transcription initiation reaction. Transcription factors are roughly divided into the following groups: basic transcription factors required for placing an RNA polymerase into a promoter region on DNA; and transcription regulatory factors which bind to cis-acting elements present upstream or downstream of a transcription region to regulate the synthesis initiation frequency of RNA.

Basic transcription factors are prepared depending on the type of RNA polymerase. A TATA-binding protein is believed to be common to all transcription systems. Although there are a number of types of transcription factors, a typical transcription factor consists of a portion structurally required for binding to DNA and a portion required for activating or suppressing transcription. Factors which have a DNA-binding portion and can bind to cis-acting elements are collectively referred to as trans-acting factors.

A portion required for activating or suppressing transcription is involved in interaction with other transcription factors or basic transcription factors. Such a portion is believed to play a role in regulating transcription via a structural change in DNA or a transcription initiating complex. Transcription regulatory factors are divided into several groups or families according to the structural properties of these portions, including many factors which play an important role in the development or differentiation of a cell.

Examples of such a transcription factor include, but are not limited to, STAT1, STAT2, STAT3, GAS, NFAT, Myc, AP1, CREB, NFκB, E2F, Rb, p53, RUNX1, RUNX2, RUNX3, Nkx-2, CF2-II, Skn-1, SRY, HFH-2, Oct-1, Oct-3, Sox-5, HNF-3b, PPARγ, and the like.

As used herein, the term "terminator" refers to a sequence which is located downstream of a protein-encoding region of a gene and which is involved in the termination of transcription when DNA is transcribed into mRNA, and the addition of a poly-A sequence. It is known that a terminator contributes to the stability of mRNA, and has an influence on the level of gene expression.

As used herein, the term "promoter" refers to a base sequence which determines the initiation site of transcription of a gene and is a DNA region which directly regulates the frequency of transcription. Transcription is started by RNA polymerase binding to a promoter. A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but is dependent on the structural gene, i.e., a putative promoter region may be located downstream of a structural gene. Preferably, a putative promoter region is located within about 2 kbp upstream of the translation initiation site of the first exon. Such promoters include, but are not limited to constitutive promoters, specific promoters and inductive promoters and the like.

As used herein, the term "enhancer" refers to a sequence which is used so as to enhance the expression efficiency of a gene of interest. One or more enhancers may be used, or no enhancer may be used.

As used herein, the term "silencer" refers to a sequence which has a function of suppressing and arresting the expression of a gene. Any silencer which has such a function may be herein used. No silencer may be used.

As used herein, the term "operably linked" indicates that a desired sequence is located such that expression (operation) thereof is under control of a transcription and translation regulatory sequence (e.g., a promoter, an enhancer, and the like) or a translation regulatory sequence. In order for a promoter to be operably linked to a gene, typically, the promoter is located immediately upstream of the gene. A promoter is not necessarily adjacent to a structural gene.

Sequences flanking other genome structural genes, genomic sequences other than exons, and sequences within exons may also be herein used. For example, in addition to the above-described sequences having specific names, structural gene-flanking sequences are thought to be involved in the control of transcription in terms of "processes". Therefore, such flanking sequences are also included in transcription control sequences. Genomic sequences other than exons and sequences within exons are also expected to be involved in the control of transcription in terms of "processes". Therefore, genomic sequences other than exons and sequences within exons are also included in transcription control sequences.

As used herein, the term "RNAi" is an abbreviation of RNA interference and refers to a phenomenon where an agent for causing RNAi, such as double-stranded RNA (also called dsRNA), is introduced into cells and mRNA homologous thereto is specifically degraded, so that the synthesis of gene products is suppressed, and techniques using the phenomenon. As used herein, RNAi may have the same meaning as that of an agent which causes RNAi.

As used herein, the term "an agent causing RNAi" refers to any agent capable of causing RNAi. As used herein, "an agent causing RNAi of a gene" indicates that the agent causes RNAi relating to the gene and that the effect of RNAi is achieved (e.g., suppression of expression of the gene, and the like). Examples of such an agent causing RNAi include, but are not limited to, a sequence having at least about 70% homology to the nucleic acid sequence of a target gene or a sequence hybridizable thereto under stringent conditions, RNA containing a double-stranded portion having a length of at least 10 nucleotides or variants thereof. Here, this agent may be preferably DNA containing a 3' protruding end, and more preferably the 3' protruding end has a length of 2 or more nucleotides (e.g., 2-4 nucleotides in length).

Though not wishing to be bound by any theory, a mechanism which causes RNAi is considered to be as follows. When a molecule which causes RNAi, such as dsRNA, is introduced into a cell, an RNaseIII-like nuclease having a helicase domain (called dicer) cleaves the molecule at about 20 base pair intervals from the 3' terminus in the presence of ATP in the case where the RNA is relatively long (e.g., 40 or more base pairs). As used herein, the term "siRNA" is an abbreviation of short interfering RNA and refers to short double-stranded RNA of 10 or more base pairs which are artificially chemically synthesized or biochemically synthesized, synthesized by an organism, or produced by double-stranded RNA of about 40 or more base pairs being degraded within the organism. siRNA typically has a structure comprising 5'-phosphate and 3'-OH, where the 3' terminus projects by about 2 bases. A specific protein is bound to siRNA to form RISC (RNA-induced-silencing-complex). This complex recognizes and binds to mRNA having the same sequence as that of siRNA and cleaves mRNA at the middle of siRNA due to RNaseIII-like enzymatic activity. It is preferable that the relationship between the sequence of siRNA and the sequence of mRNA to be cleaved as a target is a 100% match. However, base mutations at a site away from the middle of siRNA do not completely remove the cleavage activity by RNAi, leaving partial activity, while base mutations in the middle of siRNA have a large influence and the mRNA cleavage activity by RNAi is considerably lowered. By utilizing such a nature, only mRNA having a mutation can be specifically degraded. Specifically, siRNA in which the mutation is provided in the middle thereof is synthesized and is introduced into a cell. Therefore, in the present invention, siRNA per se, as well as an agent capable of producing siRNA (e.g., representatively dsRNA of about 40 or more base pairs) can be used as an agent capable of eliciting RNAi.

Also, though not wishing to be bound by any theory, apart from the above-described pathway, the antisense strand of siRNA binds to mRNA and siRNA functions as a primer for RNA-dependent RNA polymerase (RdRP), so that dsRNA is synthesized. This dsRNA is a substrate for a dicer again, leading to production of new siRNA. It is intended that such a reaction is amplified. Therefore, in the present invention, siRNA per se, as well as an agent capable of producing siRNA are useful. In fact, in insects and the like, for example, 35 dsRNA molecules can substantially completely degrade 1,000 or more copies of intracellular mRNA, and therefore, it will be understood that siRNA per se, as well as an agent capable of producing siRNA, is useful.

In the present invention, double-stranded RNA having a length of about 20 bases (e.g., representatively about 21 to 23 bases) or less than about 20 bases, called siRNA, can be used. Expression of siRNA in cells can suppress expression of a pathogenic gene targeted by the siRNA. Therefore, siRNA can be used for the treatment, prophylaxis, prognosis, and the like of diseases.

The siRNA of the present invention may be in any form as long as it can elicit RNAi.

In another embodiment, an agent capable of causing RNAi may have a short hairpin structure having a sticky portion at the 3' terminus (shRNA; short hairpin RNA). As used herein, the term "shRNA" refers to a molecule of about 20 or more base pairs in which a single-stranded RNA partially contains a palindromic base sequence and forms a double-strand structure therein (i.e., a hairpin structure). shRNA can be artificially chemically synthesized. Alternatively, shRNA can be produced by linking sense and antisense strands of a DNA sequence in reverse directions and synthesizing RNA *in vitro* with T7 RNA polymerase using the DNA as a template. Though not wishing to be bound by any theory, it should be understood that after shRNA is introduced into a cell, the shRNA is degraded in the cell to a length of about 20 bases (e.g., representatively 21, 22, 23 bases), and causes RNAi as with siRNA, leading to the treatment effects of the present invention. It should be understood that such an effect is exhibited in a wide range of organisms, such as insects, plants, animals (including mammals), and the like. Thus, shRNA elicits RNAi as with siRNA and therefore can be used as an effective component of the present invention. shRNA may preferably have a 3' protruding end. The length of the double-stranded portion is not particularly limited, but is preferably about 10 or more nucleotides, and more preferably about 20 or more nucleotides. Here, the 3' protruding end may be preferably DNA, more preferably DNA of at least 2 nucleotides in length, and even more preferably DNA of 2-4 nucleotides in length.

An agent capable of causing RNAi used in the present invention may be artificially synthesized (chemically or biochemically) or naturally occurring. There is substantially no difference there between in terms of the effect of the present invention. A chemically synthesized agent is preferably purified by liquid chromatography or the like.

An agent capable of causing RNAi used in the present invention can be produced *in vitro.* In this synthesis system, T7 RNA polymerase and T7 promoter are used to synthesize antisense and sense RNAs from template DNA. These RNAs are annealed and thereafter introduced into a cell. In this case, RNAi is caused via the above-described mechanism, thereby achieving the effect of the present invention. Here, for example, the introduction of RNA into cell can be carried out using a calcium phosphate method.

Another example of an agent capable of causing RNAi according to the present invention is a single-stranded nucleic acid hybridizable to mRNA, or all nucleic acid analogs thereof. Such agents are useful for the method and composition of the present invention.

As used herein, the term "time-lapse" and "time-series" are interchangeably used to mean any action or phenomenon that is related to the passage of time.

As used herein, the term "monitor" refers to the measurement of a state of a cell using at least one parameter as a measure (e.g., a labeling signal attributed to transcription, etc.). Preferably, monitoring is performed using a device, such as a detector, a measuring instrument, or the like. More preferably, such a device is connected to a computer for recording and/or processing data. Monitoring may comprise the step of obtaining image data of a solid phase support (e.g., an array, a plate, etc.).

As used herein, the term "real time" means that a certain state is substantially simultaneously displayed in another form (e.g., as an image on a display or a graph with processed data). In such a case, the "real time" lags behind an actual event by the time required for data processing. Such a time lag is included in the scope of "real time" if it is substantially negligible. Such a time lag may be typically within 10 seconds, and preferably within 1 second, without limitation. A time lag exceeding 10 seconds may be included in the scope of "real time".

As used herein, the determination of a state of a cell can be performed using various methods. Examples of such methods include, but are not limited to, mathematical processing (e.g., signal processing, multivariate analysis, etc.), empirical processing, phase changes, and the like.

As used herein, the term "difference" refers to a result of mathematical processing in which a value of a control profile (e.g., without a stimulus) is subtracted from a certain profile.

As used herein, the term "phase" in relation to a time-lapse profile refers to a result of a determination of whether the profile is positive or negative with respect to a reference point (typically 0), which is expressed with + or -, and also refers to analysis based on such a result.

As used herein, the term "inflection point" refers to a point at which sign (+ or -) of curvature of a curve is changed, and convex to concave on one side. Specifically, it refers to a point at which a second-order derivative becomes zero in a graph of a function. As used herein, the point of inflection may have significant meaning as an event timing.

As used herein, the term "correlate" or "correlation" in relation to a profile (e.g., a time-lapse profile, etc.) and a state of a time-lapse data, event descriptor such as event timing and the like, refers to an act of associating the profile (e.g. time-lapse data) or particular information about changes, with the state of the system. A relationship between them is referred to as "correlation" or a "correlation relationship". Conventionally, it was substantially impossible to associate a profile (e.g., a time-lapse profile, etc.) with a state of a system. No relationship between them was known. The present invention has an advantageous effect of performing such a correlation, and further, the present invention achieved the correlation in a simple manner.

As used herein, correlation may be performed by correlating at least one event descriptor, and a change (for example, affinity, drug resistance and the like) in a state of a system (for example, a cell, a tissue, an organ, or a biological organism and the like), for example, corresponding an event descriptor to at least one index of a state of a system in a quantitative or qualitative manner. The number of the at least one event descriptor used in the correlation may be small as possible as long as the correlation may be conducted, and usually at least one, preferably at least two, more preferably at least three, but is not limited thereto. In the present invention, it has been elucidated that specifying at least two, preferably at least three of the at least one event descriptor, may be sufficient to extract and analyze specific information of a certain system. Such an effect was not expectable using conventional profiling or assay swhich perform observation using a point. As such, it can be said that the effect is a significant effect, which has been provided for the first time by the present invention. In such a case, when corresponding at least one event descriptor with a state of a system, a matrix may be used to conduct mathematical processing. Alternatively, an algorithm such as genetic algorithm may be used. The event descriptor of the present invention may be described as a sequence of a letter/character string. As such, it is possible to use any general analytical method relating to a letter/character string. However, by using the technology according to the present invention, it is possible to understand the state of the system to a substantive extent simply by selecting any one of indexes and obtaining an event descriptor.

Examples of a specific method for correlation include, but are not limited to, signal processing (e.g., wavelet analysis, etc.), multivariate analysis (e.g., cluster analysis, etc.), and the like.

As used herein, the term "genetic algorithm (GA)" refers to an algorithm for optimization, in which adaptation to an environment, which is a major challenge in evolution, is viewed as processing of a genetic information, and which is a molecular process in overall evolutionary theory. Specifically, a genetic algorithm is an algorithm for adaptation, which is based on learning called self-organization resulting from the complexed combination of recognition of a target, interaction with the environment, and memory storing properties observed in organisms, and the basis of the information is heredity

The genetic algorithm utilizes two processes, sexual reproduction and natural selection, which are used by organisms. In the sexual reproduction of organisms, homologous chromosomes pair as represented by fertilization of a sperm and an egg. Thereafter, crossover occurs any site in a chromosome, causing gene exchange, i.e., gene recombination. Gene recombination achieves diversification of information more effectively and efficiently than mutation. In natural selection, in which individuals diversified by sexual reproduction or the like are caused to remain and become next-generation surviving organisms, i.e., adaptive organisms, are determined. Unlike conventional algorithms, the genetic algorithm is characterized in that the risk of a solution falling into a local optimum is significantly reduced.

The basic scheme of the genetic algorithm will be described. The entire genetic algorithm is roughly divided into the following eight processes:

(1) Determination of genotype;
(2) Generation of population;
(3) Evaluation of individuals;
(4) Selection (deletion);
(5) Reproduction;
(6) crossover (recombination);
(7) Mutation; and
(8) Evaluation of groups.

A population generated in (2) is subjected to selection in (3) and (4), and diversified in (5) to (7). The resulting solutions are evaluated in (8). Depending on the results, (3) to (7) (herein referred to as one "generation") are repeated. The above-described generation of new individuals and change of generation are the basic scheme of the genetic algorithm. In this manner, in the genetic algorithm, a population of events to be solved (optimum solution region: a region having a plurality of solutions, but not a sole solution) are artificially evolved (i.e., optimum adaptation) so that the solution approaches a true optimum value for the entire population. Here, at least one of processes (4) to (7) may be omitted.

Next, each process in the genetic algorithm will be described.

(1) Determination of genotype

In this process, a genotype is determined. An event or system is modeled (i.e., division of the event into components, definition thereof, and definition between each component) and the model is represented by symbols. Therefore, the event can be described by DNAs and amino acids. Representatively, the event is represented by, but is not limited to, binary digits (bit), numerical values, characters, or the like. If the modeling of an event is not appropriate for the above-described symbolic representation, the event is not adapted to GA.

(2) Generation of population

Diversity is generated. In principle, a number of slightly different individuals are generated. A random method and a rule method may be used. In the random method, an initial value is based on random number generation. In the rule method, an initial value is based on a predetermined criterion.

In this process, individuals are ranked in terms of fitness to an environment from high to low. Examples of evaluation parameters for proteins include, but are not limited to, empirical molecular mechanics potential, semi-empirical quantum mechanics potential, non-empirical quantum mechanics potential, electromagnetic potential, solvation potential, structural entropy, pI (isoelectric point), and the like. These evaluation parameters may be directly or indirectly related to the biochemical properties of protein.

(4) Selection (deletion)

Selection is a process for selecting individuals which remain in the next generation based on the evaluation values resulting from an evaluation function in (3). Therefore, some individuals are deleted depending on the evaluation by the evaluation function. Selection is roughly divided into three categories, depending on the manner of deletion.

(a) Random method (roulette method):

individuals are first rejected that have numerical values of fitness less than a predetermined value, and the remaining individuals are randomly screened.

(b) fitness ranking method (ranking method):

individuals are not rejected depending on the numerical values of fitness. Instead, individual members are ranked in the terms of fitness and are each given selection probabilities depending on their rank. The individuals are selected based on their probabilities.

(c) High fitness choice method (elite conservation method) : the individual which has the greatest fitness in a group to which the individual belongs is unconditionally selected.

(5) Reproduction

In this process, the reduced number of individuals in (4) are subjected to reproduction. Reproduction is conducted in a predetermined manner so that a predetermined proportion of individuals are extracted from the overall individuals after the selection and are then subjected to reproduction. This process leads to an increase in the average value of fitness in the entire population. Examples of the reproduction include causing individuals having high evaluation values to reproduce preferentially, causing individuals to reproduce in proportion to the proportion of remaining individuals.

(6) Crossover (recombination)

Crossover mimics a crossover event in gene recombination. In this process, particular symbols in one individual are replaced with corresponding symbols in another individual. When only selection is performed, no individual having an evaluation value exceeding the highest evaluation value in the population is newly generated. With this process, it is possible to generate an individual having a still higher evaluation value.

Crossover is roughly divided into one-point crossover, multi-point crossover, uniform crossover, order crossover, cycle crossover, and partially matched crossover.

(7) Mutation

Mutation is a process in which particular sites of individuals are changed with a predetermined probability. Species to be changed may be all naturally occurring amino acids (20 types), or a group of particular amino acids. Alternatively, non-naturally occurring amino acids or modified amino acids may be changed. In selection or crossover, the resultant highest value is constrained by the initial values. With mutation, individuals having high fitness values can be generated without depending on the initial values. Mutation is divided into translocation, overlapping, inversion, insertion, deletion, and the like.

(8) Evaluation of organism population

In this process, the individual population obtained by the above-described processes is evaluated using predetermined characteristic parameters. In this case, a termination condition, i.e., whether or not the above-described processes are to be repeated is judged.

The above-described processes are repeated over a certain number of generations, thereby achieving the genetic algorithm.

Correlation may be performed in advance or may be performed at the time of determination of cells using a control.

As used herein, the term "external agent" in relation to a system refers to a factor or agent which is not usually present in the system (e.g., a substance, a social factor, a stress, energy, a legal factor etc.). As used herein, the term "factor" may refer to any substance or element as long as an intended object can be achieved (e.g., energy, such as ionizing radiation, radiation, light, acoustic waves, and the like). Examples of such a substance include, but are not limited to, proteins, polypeptides, oligopeptides, peptides, polynucleotides, oligonucleotides, nucleotides, nucleic acids (e.g., DNA such as cDNA, genomic DNA and the like, or RNA such as mRNA, RNAi and the like), polysaccharides, oligosaccharides, lipids, low molecular weight organic molecules (e.g., hormones, ligands, information transduction substances, low molecular weight organic molecules, molecules synthesized by combinatorial chemistry, low molecular weight molecules usable as medicaments (e.g., low molecular weight molecule ligands, etc.), etc.), and composite molecules thereof. External agents may be used singly or in combination. Examples of an external agent as used herein include, but are not limited to, temperature changes, humidity changes, electromagnetic wave, potential difference, visible light, infrared light, ultraviolet light, X-rays, chemical substances, pressure, gravity changes, gas partial pressure, osmotic pressure, and the like. In one embodiment, an external agent may be a biological molecule or a chemically synthesized substance.

(BIOLOGICAL MOLECULE AND SUBSTANCES)

As used herein, the term "biological molecule" refers to molecules relating to an organism and aggregations thereof. As used herein, the term "biological" or "organism" refers to a biological organism, including, but being not limited to, an animal, a plant, a fungus, a virus, and the like. Biological molecules include molecules extracted from an organism and aggregations thereof, though the present invention is not limited to this. Any molecule capable of affecting an organism and aggregations thereof fall within the definition of a biological molecule. Therefore, low molecular weight molecules (e.g., low molecular weight molecule ligands, etc.) capable of being used as medicaments fall within the definition of a biological molecule as long as an effect on an organism is intended. Examples of such a biological molecule include, but are not limited to, proteins, polypeptides, oligopeptides, peptides, polynucleotides, oligonucleotides, nucleotides, nucleic acids (e.g., DNA such as cDNA and genomic DNA; RNA such as mRNA), polysaccharides, oligosaccharides, lipids, low molecular weight molecules (e.g., hormones, ligands, information transmitting substances, low molecular weight organic molecules, etc.), and composite molecules thereof and aggregations thereof (e.g., glycolipids, glycoproteins, lipoproteins, etc.), and the like. A biological molecule may include a cell itself or a portion of tissue as long as it is intended to be introduced into a cell. Typically, a biological molecule may be a nucleic acid, a protein, a lipid, a sugar, a proteolipid, a lipoprotein, a glycoprotein, a proteoglycan, or the like. Preferably, a biological molecule may include a nucleic acid (DNA or RNA) or a protein. In another preferred embodiment, a biological molecule is a nucleic acid (e.g., genomic DNA or cDNA, or DNA synthesized by PCR or the like). In another preferred embodiment, a biological molecule may be a protein. Preferably, such a biological molecule may be a hormone or a cytokine.

As used herein, the term "compound" refers to any substance which may be synthesized by using typical chemical techniques. Such synthesizing techniques are well known in the art. Those skilled in the art can produce compounds by combining such techniques as appropriate.

The term "cytokine" is used herein in the broadest sense in the art and refers to a physiologically active substance which is produced by a cell and acts on the same or different cell. Cytokines are generally proteins or polypeptides having a function of controlling an immune response, regulating the endocrine system, regulating the nervous system, acting against a tumor, acting against a virus, regulating cell growth, regulating cell differentiation, or the like. Cytokines are used herein in the form of a protein or a nucleic acid or in other forms. In actual practice, cytokines are typically proteins. The terms "growth factor" refers to a substance which promotes or controls cell growth. Growth factors are also called "proliferation factors" or "development factors". Growth factors may be added to cell or tissue culture medium, substituting for serum macromolecules. It has been revealed that a number of growth factors have a function of controlling differentiation in addition to a function of promoting cell growth. Examples of cytokines representatively include, but are not limited to, interleukins, chemokines, hematopoietic factors (e.g., colony stimulating factors), tumor necrosis factor, and interferons. Representative examples of growth factors include, but are not limited to, platelet-derived growth factor (PDGF), epidermal growth factor (EGF), fibroblast growth factor (FGF), hepatocyte growth factor (HGF), endothelial cell growth factor (VEGF), cardiotrophin, and the like, which have proliferative activity.

The term "hormone" is herein used in its broadest sense in the art, referring to a physiological organic compound which is produced in a particular organ or cell of an animal or plant, and has a physiological effect on an organ apart from the site producing the compound. Examples of such a hormone include, but are not limited to, growth hormones, sex hormones, thyroid hormones, and the like. The scope of hormones may overlap partially with that of cytokines.

As used herein, the term "actin acting substance" refers to a substance which interacts directly or indirectly with actin within cells to alter the form or state of actin. Examples of such a substance include, but are not limited to, extracellular matrix proteins (e.g., fibronectin, vitronectin, laminin, etc.), and the like. Such actin acting substances include substances identified by the following assays. As used herein, interaction with actin is evaluated by visualizing actin with an actin staining reagent (Molecular Probes, Texas Red-X phalloidin) or the like, followed by microscopic inspection to observe and determine actin aggregation, actin reconstruction or an improvement in cellular outgrowth rate. Such evaluation may be performed quantitatively or qualitatively. Actin acting substances are herein utilized so as to increase transfection efficiency. An actin acting substance used herein is derived from any organism, including, for example, mammals, such as human, mouse, bovine, and the like.

As used herein, the terms "cell adhesion agent", "cell adhesion molecule", "adhesion agent" and "adhesion molecule" are used interchangeably to refer to a molecule capable of mediating the joining of two or more cells (cell adhesion) or adhesion between a substrate and a cell. In general, cell adhesion molecules are divided into two groups: molecules involved in cell-cell adhesion (intercellular adhesion) (cell-cell adhesion molecules) and molecules involved in cell-extracellular matrix adhesion (cell-substrate adhesion) (cell-substrate adhesion molecules). For a method of the present invention, either type of molecule is useful and can be effectively used. Therefore, cell adhesion molecules herein include a substrate protein and a cellular protein (e.g., integrin, etc.) involved in cell-substrate adhesion. A molecule other than a protein can fall within the concept of cell adhesion molecule as long as it can mediate cell adhesion.

For cell-cell adhesion, cadherin, a number of molecules belonging in an immunoglobulin superfamily (NCAM, L1, ICAM, fasciclin II, III, etc.), selectin, and the like are known, each of which is known to connect cell membranes via a specific molecular reaction.

On the other hand, a major cell adhesion molecule functioning for cell-substrate adhesion is integrin, which recognizes and binds to various proteins contained in extracellular matrices. These cell adhesion molecules are all located on cell membranes and can be regarded as a type of receptor (cell adhesion receptor). Therefore, receptors present on cell membranes can also be used in a method of the present invention. Examples of such a receptor include, but are not limited to, α-integrin, β-integrin, CD44, syndecan, aggrecan, and the like. Techniques for cell adhesion are well known as described above and as described in, for example, "Saibogaimatorikkusu -Rinsho heno Oyo- [Extracellular matrix -Clinical Applications-], Medical Review.

It can be determined whether or not a certain molecule is a cell adhesion molecule, by an assay, such as biochemical quantification (an SDS-PAGE method, a labeled-collagen method, etc.), immunological quantification (an enzyme antibody method, a fluorescent antibody method, an immunohistological study, etc.), a PDR method, a hybridization method, or the like, in which a positive reaction is detected. Examples of such a cell adhesion molecule include, but are not limited to, collagen, integrin, fibronectin, laminin, vitronectin, fibrinogen, immunoglobulin superfamily members (e.g., CD2, CD4, CD8, ICM1, ICAM2, VCAM1), selectin, cadherin, and the like. Most of these cell adhesion molecules transmit an auxiliary signal for cell activation into a cell due to intercellular interaction as well as cell adhesion. It can be determined whether or not such an auxiliary signal can be transmitted into a cell, by an assay, such as biochemical quantification (an SDS-PAGE method, a labeled-collagen method, etc.), immunological quantification (an enzyme antibody method, a fluorescent antibody method, an immunohistological study, etc.), a PDR method, a hybridization method, or the like, in which a positive reaction is detected.

Examples of cell adhesion molecules include, but are not limited to, immunoglobulin superfamily molecules (LFA-3, ICAM-1, CD2, CD4, CD8, ICM1, ICAM2, VCAM1, etc.); integrin family molecules (LFA-1, Mac-1, gpIIbIIIa, p150, p95, VLA1, VLA2, VLA3, VLA4, VLA5, VLA6, etc.); selectin family molecules (L-selectin, E-selectin, P-selectin, etc.), and the like.

As used herein, the term "extracellular matrix protein" refers to a protein constituting an "extracellular matrix". As used herein, the term "extracellular matrix" (ECM) is also called "extracellular substrate" and has the same meaning as commonly used in the art, and refers to a substance existing between somatic cells no matter whether the cells are epithelial cells or non-epithelial cells. Extracellular matrices are involved in supporting tissue as well as in internal environmental structures essential for survival of all somatic cells. Extracellular matrices are generally produced from connective tissue cells. Some extracellular matrices are secreted from cells possessing basal membrane, such as epithelial cells or endothelial cells. Extracellular matrices are roughly divided into fibrous components and matrices filling there between. Fibrous components include collagen fibers and elastic fibers. A basic component of matrices is glycosaminoglycan (acidic mucopolysaccharide), most of which is bound to non-collagenous protein to form a polymer of a proteoglycan (acidic mucopolysaccharide-protein complex). In addition, matrices include glycoproteins, such as laminin of basal membrane, microfibrils around elastic fibers, fibers, fibronectins on cell surfaces, and the like. Particularly differentiated tissue has the same basic structure. For example, in hyaline cartilage, chondroblasts characteristically produce a large amount of cartilage matrices including proteoglycans. In bones, osteoblasts produce bone matrices which cause calcification. Examples of extracellular matrices for use in the present invention include, but are not limited to, collagen, elastin, proteoglycan, glycosaminoglycan, fibronectin, laminin, elastic fiber, collagen fiber, and the like.

As used herein, the term "receptor" refers to a molecule which is present on cells, within nuclei, or the like, and is capable of binding to an extracellular or intracellular agent where the binding mediates signal transduction. Receptors are typically in the form of proteins. The binding partner of a receptor is usually referred to as a ligand.

As used herein, the term "agonist" refers to an agent which binds to the receptor of a certain biologically acting substance (e.g., ligand, etc.), and has the same or similar function as the function of the substance.

As used herein, the term "antagonist" refers to a factor which competitively binds to the receptor of a certain biologically acting substance (ligand), and does not produce a physiological action via the receptor. Antagonists include antagonist drugs, blockers, inhibitors, and the like.

(Devices and solid phase supports)

As used herein, the term "device" refers to a part which can constitute the entire or a portion of an apparatus, and comprises a support (preferably, a solid phase support) and a target substance carried thereon. Examples of such a device include, but are not limited to, chips, arrays, microtiter plates, cell culture plates, Petri dishes, films, beads, and the like.

As used herein, the term "support" refers to a material which can fix a substance, such as a biological molecule. Such a support may be made from any fixing material which has a capability of binding to a biological molecule as used herein via covalent or noncovalent bond, or which may be induced to have such a capability.

Examples of materials used for supports include any material capable of forming a solid surface, such as, without limitation, glass, silica, silicon, ceramics, silicon dioxide, plastics, metals (including alloys), naturally-occurring and synthetic polymers (e.g., polystyrene, cellulose, chitosan, dextran, and nylon), and the like. A support may be formed of layers made of a plurality of materials. For example, a support may be made of an inorganic insulating material, such as glass, quartz glass, alumina, sapphire, forsterite, silicon oxide, silicon carbide, silicon nitride, or the like. A support may be made of an organic material, such as polyethylene, ethylene, polypropylene, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, polyphenylene oxide, polysulfone, and the like. Also in the present invention, nitrocellulose film, nylon film, PVDF film, or the like, which are used in blotting, may be used as a material for a support. When a material constituting a support is in the solid phase, such as a support is herein particularly referred to as a "solid phase support". A solid phase support may be herein in the form of a plate, a microwell plate, a chip, a glass slide, a film, beads, a metal (surface), or the like. A support may not be coated or may be coated.

As used herein, the term "liquid phase" has the same meaning as commonly understood by those skilled in the art, typically referring a state in solution.

As used herein, the term "solid phase" has the same meaning as commonly understood by those skilled in the art, typically referring to a solid state. As used herein, liquid and solid may be collectively referred to as a "fluid".

As used herein, the term "substrate" refers to a material (preferably, solid) which is used to construct a chip or array according to the present invention. Therefore, substrates are included in the concept of plates. Such a substrate may be made from any solid material which has a capability of binding to a biological molecule as used herein via covalent or noncovalent bonds, or which may be induced to have such a capability.

Examples of materials used for plates and substrates include any material capable of forming a solid surface, such as, without limitation, glass, silica, silicon, ceramics, silicon dioxide, plastics, metals (including alloys), naturally-occurring and synthetic polymers (e.g., polystyrene, cellulose, chitosan, dextran, and nylon), and the like. A support may be formed of layers made of a plurality of materials. For example, a support may be made of an inorganic insulating material, such as glass, quartz glass, alumina, sapphire, forsterite, silicon oxide, silicon carbide, silicon nitride, or the like. A support may be made of an organic material, such as polyethylene, ethylene, polypropylene, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, polyphenylene oxide, polysulfone, and the like. A material preferable as a substrate varies depending on various parameters such as a measuring device, and can be selected from the above-described various materials as appropriate by those skilled in the art. For transfection arrays, glass slides are preferable. Preferably, such a substrate may have a coating.

As used herein, the term "coating" in relation to a solid phase support or substrate refers to an act of forming a film of a material on a surface of the solid phase support or substrate, and also refers to a film itself. Coating is performed for various purposes, such as, for example, improvement in the quality of a solid phase support and substrate (e.g., elongation of life span, improvement in resistance to hostile environment, such as resistance to acids, etc.), an improvement in affinity to a substance integrated with a solid phase support or substrate, and the like. Various materials may be used for such coating, including, without limitation, biological substances (e.g., DNA, RNA, protein, lipid, etc.), polymers (e.g., poly-L-lysine, MAS (available from Matsunami Glass, Kishiwada, Japan), and hydrophobic fluorine resin), silane (APS (e.g., γ-aminopropyl silane, etc.)), metals (e.g., gold, etc.), in addition to the above-described solid phase support and substrate. The selection of such materials is within the technical scope of those skilled in the art and thus can be performed using techniques well known in the art. In one preferred embodiment, such a coating may be advantageously made of poly-L-lysine, silane (e.g., epoxy silane or mercaptosilane, APS (γ-aminopropyl silane), etc.), MAS, hydrophobic fluorine resin, a metal (e.g., gold, etc.). Such a material may be preferably a substance suitable for cells or objects containing cells (e.g., organisms, organs, etc.).

As used herein, the terms "chip" or "microchip" are used interchangeably to refer to a micro integrated circuit which has versatile functions and constitutes a portion of a system. Examples of a chip include, but are not limited to, DNA chips, protein chips, and the like.

As used herein, the term "array" refers to a substrate (e.g., a chip, etc.) which has a pattern of a composition containing at least one (e.g., 1000 or more, etc.) target substances (e.g., DNA, proteins, transfection mixtures, etc.), which are arrayed. Among arrays, patterned substrates having a small size (e.g., 10x10 mm, etc.) are particularly referred to as microarrays. The terms "microarray" and "array" are used interchangeably. Therefore, a patterned substrate having a larger size than that which is described above may be referred to as a microarray. For example, an array comprises a set of desired transfection mixtures fixed to a solid phase surface or a film thereof. An array preferably comprises at least $10^2$ antibodies of the same or different types, more preferably at least $10^3$, even more preferablyat least $10^4$, and still even more preferably at least $10^5$. These antibodies are placed on a surface of up to 125×80 mm, more preferably 10×10 mm. An array includes, but is not limited to, a 96-well microtiter plate, a 384-well microtiter plate, a microtiter plate the size of a glass slide, and the like. A composition to be fixed may contain one or a plurality of types of target substances. Such a number of target substance types may be in the range of from one to the number of spots, including, without limitation, about 10, about 100, about 500, and about 1,000.

As described above, any number of target substances (e.g., proteins, such as antibodies) may be provided on a solid phase surface or film, typically including no more than $10^8$ biological molecules per substrate, in another embodiment no more than $10^7$ biological molecules, no more than $10^6$ biological molecules, no more than $10^5$ biological molecules, no more than $10^4$ biological molecules, no more than $10^3$ biological molecules, or no more than $10^2$ biological molecules. A composition containing more than $10^8$ biological molecule target substances may be provided on a substrate. In these cases, the size of a substrate is preferably small. Particularly, the size of a spot of a composition containing target substances (e.g., proteins such as antibodies) may be as small as the size of a single biological molecule (e.g., 1 to 2 nm order). In some cases, the minimum area of a substrate may be determined based on the number of biological molecules on a substrate. A composition containing target substances, which are intended to be introduced into cells, are herein typically arrayed on and fixed via covalent bonds or physical interaction to a substrate in the form of spots having a size of 0.01 mm to 10 mm.

"Spots" of biological molecules may be provided on an array. As used herein, the term "spot" refers to a certain set of compositions containing target substances. As used herein, the term "spotting" refers to an act of preparing a spot of a composition containing a certain target substance on a substrate or plate. Spotting may be performed by any method, for example, pipetting or the like, or alternatively, using an automatic device. These methods are well known in the art.

As used herein, the term "address" refers to a unique position on a substrate, which may be distinguished from other unique positions. Addresses are appropriately associated with spots. Addresses can have any distinguishable shape such that substances at each address may be distinguished from substances at other addresses (e.g., optically). A shape defining an address may be, for example, without limitation, a circle, an ellipse, a square, a rectangle, or an irregular shape. Therefore, the term "address" is used to indicate an abstract concept, while the term "spot" is used to indicate a specific concept. Unless it is necessary to distinguish them from each other, the terms "address" and "spot" may be herein used interchangeably.

The size of each address particularly depends on the size of the substrate, the number of addresses on the substrate, the amount of a composition containing target substances and/or available reagents, the size of microparticles, and the level of resolution required for any method used for the array. The size of each address may be, for example, in the range of from 1-2 nm to several centimeters, though the address may have any size suited to an array.

The spatial arrangement and shape which define an address are designed so that the microarray is suited to a particular application. Addresses may be densely arranged or sparsely distributed, or subgrouped into a desired pattern appropriate for a particular type of material to be analyzed.

Microarrays are widely reviewed in, for example, "Genomu Kino Kenkyu Purotokoru [Genomic Function Research Protocol] (Jikken Igaku Bessatsu [Special Issue of Experimental Medicine], Posuto Genomu Jidai no Jikken Koza 1 [Lecture 1 on Experimentation in Post-genome Era), "Genomu Ikagaku to korekarano Genomu Iryo [Genome Medical Science and Futuristic Genome Therapy (Jikken Igaku Zokan [Special Issue of Experimental Medicine]), and the like.

A vast amount of data can be obtained from a microarray. Therefore, data analyzsis software is important for administration of correspondence between clones and spots, data analysis, and the like. Such software may be attached to various detection systems (e.g., Ermolaeva O. et al., (1998) Nat. Genet., 20: 19-23). The format of database includes, for example, GATC (genetic analysis technology consortium) proposed by Affymetrix.

Micromachining for arrays is described in, for example, Campbell, S.A. (1996), "The Science and Engineering of Microelectronic Fabrication", Oxford University Press; Zaut, P.V. (1996), "Micromicroarray Fabrication: a Practical Guide to Semiconductor Processing", Semiconductor Services; Madou, M.J. (1997), "Fundamentals of Microfabrication", CRC1 5 Press; Rai-Choudhury, P. (1997), "Handbook of Microlithography, Micromachining, & Microfabrication: Microlithography"; and the like, portions related thereto of which are herein incorporated by reference.

(Detection)

In cell analysis or determination in the present invention, various detection methods and means can be used as long as they can be used to detect information attributed to a cell or a substance interacting therewith. Examples of such detection methods and means include, but are not limited to, visual inspection, optical microscopes, confocal microscopes, reading devices using a laser light source, surface plasmon resonance (SPR) imaging, electric signals, chemical or biochemical markers, which may be used singly or in combination. Examples of such a detecting device include, but are not limited to, fluorescence analyzing devices, spectrophotometers, scintillation counters, CCD, luminometers, and the like. Any means capable of detecting a biological molecule may be used.

As used herein, the term "marker" refers to a biological agent for indicating a level or frequency of a substance or state of interest. Examples of such a marker include, but are not limited to, nucleic acids encoding a gene, gene products, metabolic products, receptors, ligands, antibodies, and the like.

Therefore, as used herein, the term "marker" in relation to a state of a cell refers to an agent (e.g., ligands, antibodies, complementary nucleic acids, etc.) interacting with intracellular factors indicating the state of the cell (e.g.,

nucleic acids encoding a gene, gene products (e.g., mRNA, proteins, posttranscriptionally modified proteins, etc.), metabolic products, receptors, etc.) in addition to transcription control factors. In the present invention, such a marker may be used to produce a time-lapse profile which is in turn analyzed. Such a marker may preferably interact with a factor of interest. As used herein, the term "specificity" in relation to a marker refers to a property of the marker which interacts with a molecule of interest to a significantly higher extent than with similar molecules. Such a marker is herein preferably present within cells or may be present outside cells.

As used herein, the term "label" refers to a factor which distinguishes a molecule or substance of interest from others (e.g., substances, energy, electromagnetic waves, etc.). Examples of labeling methods include, but are not limited to, RI (radioisotope) methods, fluorescence methods, biotinylation methods, chemoluminance methods, and the like. When the above-described nucleic acid fragments and complementary oligonucleotides are labeled by fluorescence methods, fluorescent substances having different fluorescence emission maximum wavelengths are used for labeling. The difference between each fluorescence emission maximum wavelength may be preferably 10 nm or more. Any fluorescent substance which can bind to a base portion of a nucleic acid may be used, preferably including a cyanine dye (e.g., Cy3 and Cy5 in the Cy Dye™ series, etc.), a rhodamine 6G reagent, N-acetoxy-N2-acetyl amino fluorene (AAF), AAIF (iodine derivative of AAF), and the like. Examples of fluorescent substances having a difference in fluorescence emission maximum wavelength of 10 nm or more include a combination of Cy5 and a rhodamine 6G reagent, a combination of Cy3 and fluorescein, a combination of a rhodamine 6G reagent and fluorescein, and the like. In the present invention, such a label can be used to alter a sample of interest so that the sample can be detected by detecting means. Such alteration is known in the art. Those skilled in the art can perform such alteration using a method appropriate for a label and a sample of interest.

As used herein, the term "interaction" refers to, without limitation, hydrophobic interactions, hydrophilic interactions, hydrogen bonds, Van der Waals forces, ionic interactions, nonionic interactions, electrostatic interactions, and the like.

As used herein, the term "interaction level" in relation to interaction between two substances (e.g., cells, etc.) refers to the extent or frequency of interaction between the two substances. Such an interaction level can be measured by methods well known in the art. For example, the number of cells which are fixed and actually perform interaction is counted directly or indirectly (e.g., the intensity of reflected light), for example, without limitation, by using an optical microscope, a fluorescence microscope, a phase-contrast microscope, or the like, or alternatively by staining cells with a marker, an antibody, a fluorescent label or the like specific thereto and measuring the intensity thereof. Such a level can be displayed directly from a marker or indirectly via a label. Based on the measured value of such a level, the number or frequency of genes, which are actually transcribed or expressed in a certain spot, can be calculated.

(Presentation and display)

As used herein, the terms "display" and "presentation" are used interchangeably to refer to an act of providing a profile obtained by a method of the present invention or information derived therefrom directly or indirectly, or in an information-processed form. Examples of such displayed forms include, but are not limited to, various methods, such as graphs, photographs, tables, animations, and the like. Such techniques are described in, for example, METHODS IN CELL BIOLOGY, VOL. 56, ed. 1998, pp: 185-215, A High-Resolution Multimode Digital Microscope System (Sluder & Wolf, Salmon), which discusses application software for automating a microscope and controlling a camera and the design of a hardware device comprising an automated optical microscope, a camera, and a Z-axis focusing device, which can be used herein. Image acquisition by a camera is described in detail in, for example, Inoue and Spring, Video Microscopy, 2d. Edition, 1997, which is herein incorporated by reference.

Real time display can also be performed using techniques well known in the art. For example, after all images are obtained and stored in a semi-permanent memory, or substantially at the same time as when an image is obtained, the image can be processed with appropriate application software to obtain processed data. For example, data may be processed by a method for playing back a sequence of images without interruption, a method for displaying images in real time, or a method for displaying images as a "movie" showing irradiating light as changes or continuation on a focal plane.

In another embodiment, application software for measurement and presentation typically includes software for setting conditions for applying stimuli or conditions for recording detected signals. With such a measurement and presentation application, a computer can have a means for applying a stimulus to cells and a means for processing signals detected from cells, and in addition, can control an optically observing means (a SIT camera and an image filing device) and/or a cell culturing means.

By inputting conditions for stimulation on a parameter setting screen using a keyboard, a touch panel, a mouse, or the like, it is possible to set desired complicated conditions for stimulation. In addition, various conditions, such as a temperature for cell culture, pH, and the like, can be set using a keyboard, a mouse, or the like. A display screen displays a time-lapse profile detected from a cell or information derived therefrom in real time or after recording. In addition, another recorded profile or information derived therefrom of a cell can be displayed while being superimposed with a microscopic image of the cell. In addition to recorded information, measurement parameters in recording (stimulation conditions, recording conditions, display conditions, process conditions, various conditions for cells, temperature, pH, etc.) can be displayed in real time. The present invention may be equipped with a function of issuing an alarm when a temperature or pH departs from the tolerable range.

On a data analysis screen, it is possible to set conditions for various mathematical analyses, such as Fourier transformation, cluster analysis, FFT analysis, coherence analysis, correlation analysis, and the like. The present invention may be equipped with a function of temporarily displaying a profile, a function of displaying topography, or the like. The results of these analyses can be displayed while being superimposed with microscopic images stored in a recording medium.

(Gene introduction)

Any technique may be used herein for introduction of a nucleic acid molecule into cells, including, for example, transformation, transduction, transfection, and the like. In the present invention, transfection is preferable.

As used herein, the term "transfection" refers to an act of performing gene introduction or transfection by culturing cells with gene DNA, plasmid DNA, viral DNA, viral RNA or the like in a substantially naked form (excluding viral particles), or adding such a genetic material into a cell suspension to allow the cells to take in the genetic material. A gene introduced by transfection is typically expressed within cells in a temporary manner or may be incorporated into cells in a permanent manner.

Such a nucleic acid molecule introduction technique is well known in the art and commonly used, and is described in, for example, Ausubel F.A. et al., editors, (1988), Current Protocols in Molecular Biology, Wiley, New York, NY; Sambrook J. et al. (1987) Molecular Cloning: A Laboratory Manual, 2nd Ed. and its 3rd Ed., Cold Spring Harbor Laboratory Press, Cold Spring Harbor, NY; Special issue, Jikken Igaku [Experimental Medicine] "Experimental Methods for Gene introduction & Expression Analysis", Yodo-sha, 1997; and the like. Gene introduction can be confirmed by method as described herein, such as Northern blotting analysis and Western blotting analysis, or other well-known, common techniques.

When a gene is mentioned herein, the term "vector" or "recombinant vector" refers to a vector transferring a polynucleotide sequence of interest to a target cell. Such a vector is capable of self-replication or incorporation into a chromosome in a host cell (e.g., a prokaryotic cell, yeast, an animal cell, a plant cell, an insect cell, an individual animal, and an individual plant, etc.), and contains a promoter at a site suitable for transcription of a polynucleotide of the present invention. A vector suitable for performing cloning is referred to as a "cloning vector". Such a cloning vector ordinarily contains a multiple cloning site containing a plurality of restriction sites. Restriction enzyme sites and multiple cloning sites as described above are well known in the art and can be used as appropriate by those skilled in the art depending on the purpose in accordance with publications described herein (e.g., Sambrook et al., *supra).*

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes, and enhancers. It is well known in the art that types of expression vectors of a biological organism (for example, animal) and a regulatory element used in may vary depending on the host organism used.

Examples of "recombinant vectors" for prokaryotic cells include, but are not limited to, pcDNA3 (+), pBluescript=SK(+/-), pGEM-T, pEF-BOS, pEGFP, pHAT, pUC18, pFT-DEST™42GATEWAY (Invitrogen), and the like.

Examples of "recombinant vectors" for animal cells include, but are not limited to, pcDNAI/Amp, pcDNAI, pCDM8 (all commercially available from Funakoshi), pAGE107 [Japanese Laid-Open Publication No. 3-229 (Invitrogen), pAGE103 [J. Biochem., 101, 1307 (1987)], pAMo, pAMoA [J. Biol. Chem., 268, 22782-22787(1993)], a retrovirus expression vector based on a murine stem cell virus (MSCV), pEF-BOS, pEGFP, and the like.

Examples of recombinant vectors for plant cells include, but are not limited to, pPCVICEn4HPT, pCGN1548, pCGN1549, pBI221, pBI121, and the like.

Any of the above-described methods for introducing DNA into cells can be used as a vector introduction method, including, for example, transfection, transduction, transformation, and the like (e.g., a calcium phosphate method, a liposome method, a DEAE dextran method, an electroporation method, a particle gun (gene gun) method, and the like), a lipofection method, a spheroplast method (Proc. Natl. Acad. Sci. USA, 84, 1929(1978)), a lithium acetate method (J. Bacteriol., 153, 163(1983); and Proc. Natl. Acad. Sci. USA, 75, 1929(1978)), and the like.

As used herein, the term "operably linked" indicates that a desired sequence is located such that expression (operation) thereof is under control of a transcription and translation regulatory sequence (e.g., a promoter, an enhancer, and the like) or a translation regulatory sequence. In order for a promoter to be operably linked to a gene, typically, the promoter is located immediately upstream of the gene. A promoter is not necessarily adjacent to a structural gene.

As used herein, the term "gene introduction reagent" refers to a reagent which is used in a gene introduction method so as to enhance introduction efficiency. Examples of such a gene introduction reagent include, but are not limited to, cationic polymers, cationic lipids, polyamine-based reagents, polyimine-based reagents, calcium phosphate, and the like. Specific examples of a reagent used in transfection include reagents available from various sources, such as, without limitation, Effectene Transfection Reagent (cat. no. 301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France) and ExGen 500 (R0511, Fermentas Inc., MD), and the like.

Gene expression (e.g., mRNA expression, polypeptide expression) may be "detected" or "quantified" by an appropriate method, including mRNA measurement and immunological measurement method. Examples of molecular biological measurement methods include Northern blotting methods, dot blotting methods, PCR methods, and the like. Examples of immunological measurement method include ELISA methods, RIA methods, fluorescent antibody methods, Western blotting methods, immunohistological staining methods, and the like, where a microtiter plate may be used. Examples of quantification methods include ELISA methods, RIA methods, and the like. A gene analysis method using an array (e.g., a DNA array, a protein array, etc.) may be used. The DNA array is widely reviewed in Saibo-Kogaku [Cell Engineering], special issue, "DNA Microarray and Up-to-date PCR Method", edited by Shujun-sha. The protein array is described in detail in Nat Genet. 2002 Dec; 32 Suppl:526-32. Examples of methods for analyzing gene expression include, but are not limited to, RT-PCR methods, RACE methods, SSCP methods, immunoprecipitation methods, two-hybrid systems, *in vitro* translation methods, and the like in addition to the above-described techniques. Other analysis methods are described in, for example, "Genome Analysis Experimental Method, Yusuke Nakamura's Lab-Manual, edited by Yusuke Nakamura, Yodo-sha (2002), and the like. All of the above-described publications are herein incorporated by reference.

As used herein, the term "expression level" refers to the amount of a polypeptide or mRNA expressed in a subject cell. The term "expression level" includes the level of protein expression of a polypeptide evaluated by any appropriate method using an antibody, including immunological measurement methods (e.g., an ELISA method, an RIA method, a fluorescent antibody method, a Western blotting method, an immunohistological staining method, and the like, or the mRNA level of expression of a polypeptide evaluated by any appropriate method, including molecular biological measurement methods (e.g., a Northern blotting method, a dot blotting method, a PCR method, and the like) . The term "change in expression level" indicates that an increase or decrease in the protein or mRNA level of expression of a polypeptide evaluated by an appropriate method including the above-described immunological measurement method or molecular biological measurement method.

(Screening)

As used herein, the term "screening" refers to selection of a target, such as an organism, a substance, or the like, a given specific property of interest from a population containing a number of elements using a specific operation/evaluation method. For screening, an agent (e.g., an antibody), a polypeptide or a nucleic acid molecule of the present invention can be used.

As used herein, screening by utilizing an immunological reaction is also referred to as "immunophenotyping". In this case, an antibody or a single chain antibody may be used for immunophenotyping a cell line and a biological sample. A transcription or translation product of a gene may be useful as a cell specific marker, or more particularly, a cell marker which is distinctively expressed in various stages in differentiation and/or maturation of a specific cell type. A monoclonal antibody directed to a specific epitope, or a combination of epitopes allows for screening of a cell population expressing a marker. Various techniques employ monoclonal antibodies to screen for a cell population expressing a marker. Examples of such techniques include, but are not limited to, magnetic separation using magnetic beads coated with antibodies, "panning" using antibodies attached to a solid matrix (i.e., a plate), flow cytometry, and the like (e.g., US Patent No. 5,985,660; and Morrison et al., Cell, 96:737-49 (1999)).

These techniques may be used to screen cell populations containing undifferentiated cells, which can grow and/or differentiate as seen in human umbilical cord blood or which are treated and modified into an undifferentiated state (e.g., embryonic stem cells, tissue stem cells, etc.).

(Diagnosis)

As used herein, the term "diagnosis" refers to an act of identifying various parameters associated with a disease, a disorder, a condition, or the like of a subject and determining a current state of the disease, the disorder, the condition, or the like. A method, device, or system of the present invention can be used to analyze a sugar chain structure, a drug resistance level, or the like. Such information can be used to select parameters, such as a disease, a disorder, a condition, and a prescription or method for treatment or prevention of a subject.

A diagnosis method of the present invention can use, in principle, a sample which is derived from the body of a subject. Therefore, it is possible for some one which is not a medical practitioner, such as a medical doctor, to deal with such a sample. The present invention is industrially useful.

(Therapy)

As used herein, the term "therapy" refers to an act of preventing progression of a disease or a disorder, preferably maintaining the current state of a disease or a disorder, more preferably alleviating a disease or a disorder, and more preferably extinguishing a disease or a disorder.

As used herein, the term "subject" refers to an organism which is subjected to the treatment of the present invention. A subject is also referred to as a "patient". A patient or subject may preferably be a human. Although the subject is often used in the contect of a therapy, as used herein, it is used to describe any system.

As used herein, the term "cause" or "pathogen" in relation to a disease, a disorder or a condition of a subject refers to an agent associated with the disease, the disorder or the condition (also collectively referred to as a "lesion", or "disease damage" in plants), including, without limitation, a causative or pathogenic substance (pathogenic agent), a disease agent, a disease cell, a pathogenic virus, and the like.

A disease targeted by the present invention may be any disease associated with a pathogenic gene. Examples of such a disease include, but are not limited to, cancer, infectious diseases due to viruses or bacteria, allergy, hypertension, hyperlipemia, diabetes, cardiac diseases, cerebral infarction, dementia, obesity, arteriosclerosis, infertility, mental and nervous diseases, cataract, progeria, hypersensitivity to ultraviolet radiation, and the like.

A disorder targeted by the present invention may be any disorder associated with a pathogenic gene.

Examples of such a disease, disorder or condition include, but are not limited to, circulatory diseases (anemia (e.g., aplastic anemia (particularly, severe aplastic anemia), renal anemia, cancerous anemia, second-order anemia, refractory anemia, etc.), cancer or tumors (e.g., leukemia, multiple myeloma), etc.); neurological diseases (dementia, cerebral stroke and sequela thereof, cerebral tumor, spinal injury, etc.); immunological diseases (T-cell deficiency syndrome, leukemia, etc.); motor organ and the skeletal system diseases (fracture, osteoporosis, luxation of joints, subluxation, sprain, ligament injury, osteoarthritis, osteosarcoma, Ewing's sarcoma, osteogenesis imperfecta, osteochondrodysplasia, etc.); dermatologic diseases (atrichia, melanoma, cutis malignant lympoma, hemangiosarcoma, histiocytosis, hydroa, pustulosis, dermatitis, eczema, etc.); endocrinologic diseases (hypothalamus/hypophysis diseases, thyroid gland diseases, accessory thyroid gland (parathyroid) diseases, adrenal cortex/medulla diseases, saccharometabolism abnormality, lipid metabolism abnormality, protein metabolism abnormality, nucleic acid metabolism abnormality, inborn error of metabolism (phenylketonuria, galactosemia, homocystinuria, maple syrup urine disease), analbuminemia, lack of ascorbic acid synthetic ability, hyperbilirubinemia, hyperbilirubinuria, kallikrein deficiency, mast cell deficiency, diabetes insipidus, vasopressin secretion abnormality, dwarfism, Wolman's disease (acid lipase deficiency)), mucopolysaccharidosis VI, etc.); respiratory diseases (pulmonary diseases (e.g., pneumonia, lung cancer, etc.), bronchial diseases, lung cancer, bronchial cancer, etc.); alimentary diseases (esophagial diseases (e.g., esophagial cancer, etc.), stomach/duodenum diseases (e.g., stomach cancer, duodenum cancer, etc.), small intestine diseases/large intestine diseases (e.g., polyps of the colon, colon cancer, rectal cancer, etc.), bile duct diseases, liver diseases (e.g., liver cirrhosis, hepatitis (A, B, C, D, E, etc.), fulminant hepatitis, chronic hepatitis, first-order liver cancer, alcoholic liver disorders, drug induced liver disorders, etc.), pancreatic diseases (acute pancreatitis, chronic pancreatitis, pancreas cancer, cystic pancreas diseases, etc.), peritoneum/abdominal wall/diaphragm diseases (hernia, etc.), Hirschsprung's disease, etc.); urinary diseases (kidney diseases (e.g., renal failure, first-order glomerulus diseases, renovascular disorders, tubular function abnormality, interstitial kidney diseases, kidney disorders due to systemic diseases, kidney cancer, etc.), bladder diseases (e.g., cystitis, bladder cancer, etc.); genital diseases (male genital organ diseases (e.g., male sterility, prostatomegaly, prostate cancer, testicular cancer, etc.), female genital organ diseases (e.g., female sterility, ovary function disorders, hysteromyoma, adenomyosis uteri, uterine cancer, endometriosis, ovarian cancer, villosity diseases, etc.), etc); circulatory diseases (heart failure, angina pectoris, myocardial infarct, arrhythmia, valvulitis, cardiac muscle/pericardium diseases, congenital heart diseases (e.g., atrial septal defect, arterial canal patency, tetralogy of Fallot, etc.), artery diseases (e.g., arteriosclerosis, aneurysm), vein diseases (e.g., phlebeurysm, etc.), lymphoduct diseases (e.g., lymphedema, etc.), etc.); and the like.

As used herein, the term "cancer" refers to a malignant tumor which has a high level of atypism, grows faster than normal cells, tends to disruptively invade surrounding tissue or metastasize to new body sites or a condition characterized by the presence of such a malignant tumor. In the present invention, cancer includes, without limitation, solid cancer and hematological cancer.

As used herein, the term "solid cancer" refers to a cancer having a solid shape in contrast to hematological cancer, such as leukemia and the like. Examples of such a solid cancer include, but are not limited to, breast cancer, liver cancer, stomach cancer, lung cancer, head and neck cancer, uterocervical cancer, prostate cancer, retinoblastoma, malignant lymphoma, esophagus cancer, brain tumor, osteoncus, and the like.

As used herein, the term "cancer therapy" encompasses administration of an anticancer agent (e.g., a chemotherapeutic agent, radiation therapy, etc.) or surgical therapy, such as surgical excision and the like.

Chemotherapeutic agents used herein are well known in the art and are described in, for example, Shigeru Tsukagoshi et al. editors, "Kogan zai Manuaru [Manual of Anticancer agents]", 2nd ed., ChugaiIgaku sha; Pharmacology; and Lippincott Williams & Wilkins, Inc. Examples of such chemotherapeutic agents are described below: 1) alkylating agents which alkylate cell components, such as DNA, protein, and the like, to produce cytotoxicity (e.g., cyclophosphamide, busulfan, thiotepa, dacarbazine, etc.); 2) antimetabolites which mainly inhibit synthesis of nucleic acids (e.g., antifolics (methotrexate, etc.), antipurines (6-mercaptopurine, etc.), antipyrimidines (fluorourasil (5-FU), etc.); 3) DNA topoisomerase inhibitors (e.g., camptothecin and etoposide, each of which inhibits topoisomerases I and II)); 4) tubulin agents which inhibit formation of microtubules and suppress cell division (vinblastine, vincristine, etc.); 5) platinum compounds which bind to DNA and proteins to exhibit cytotoxicity (cisplatin, carboplatin, etc.); 6) anticancer antibiotics which bind to DNA to inhibit synthesis of DNA and RNA (adriamycin, dactinomycin, mitomycin C, bleomycin, etc.); 7) hormone agents which are applicable to hormone-dependent cancer, such as breast cancer, uterus cancer, prostate cancer, and the like (e.g., tamoxifen, leuprorelin (LH-RH), etc.); 8) biological formulations (asparaginase effective for asparagine requiring blood malignant tumor, interferon exhibiting direct antitumor action and indirect action by immunopotentiation, etc.); 9) immunostimulants which exhibit capability of immune response, indirectly leading to antitumor activity (e.g., rentinan which is a polysaccharide derived from shiitake mushroom, bestatin which is a peptide derived from a microorganism, etc.).

An "anticancer agent" used herein selectively suppresses the growth of cancerous (tumor) cells, and includes both pharmaceutical agents and radiation therapy. Such an anticancer agent is well known in the art and described in, for example, Shigeru Tsukagoshi et al. editors, "Kogan zai Manuaru [Manual of Anticancer agents]", 2nd ed. , ChugaiIgaku sha; Pharmacology; and Lippincott Williams & Wilkins, Inc.

As used herein, the term "radiation therapy" refers to a therapy for diseases using ionizing radiation or radioactive substances. Representative examples of radiation therapy include, but are not limited to, X-ray therapy, $\gamma$-ray therapy, electron beam therapy, proton beam therapy, heavy particle beam therapy, neutron capture therapy, and the like. For example, heavy particle beam therapy is preferable. However, heavy particle beam therapy requires a large-size device and is not generally used. The above-described radiation therapies are well known in the art and are described in, for example, Sho Kei Zen, "Hoshasenkensa to Chiryo no Kiso: Hoshasen Chiryo to Shugakuteki Chiryo [Basics of Radiation Examination and Therapies: Radiation Therapy and Incentive Therapy]", (Shiga Medical School, Radiation): Total digestive system care, Vol. 6, No. 6, Pages 79-89, 6-7 (2002.02). For drug resistance to be identified in the present invention, chemotherapies are typically considered. However, resistance to radiation therapy is also associated with time-lapse profiles. Therefore, radiation therapy is herein encompassed by the concept of pharmaceutical agents.

As used herein, the term "pharmaceutically acceptable carrier" refers to a material for use in production of a medicament, an animal drug or an agricultural chemical, which does not have an adverse effect on an effective component. Examples of such a pharmaceutically acceptable carrier include, but are not limited to, antioxidants, preservatives, colorants, flavoring agents, diluents, emulsifiers, suspending agents, solvents, fillers, bulking agents, buffers, delivery vehicles, excipients, agricultural or pharmaceutical adjuvants, and the like.

As used herein, the term "pharmaceutically acceptable carrier" refers to a material for use in production of a medicament, an animal drug or an agricultural chemical, which does not have an adverse effect on an effective component. Examples of such a pharmaceutically acceptable carrier include, but are not limited to, antioxidants, preservatives, colorants, flavoring agents, diluents, emulsifiers, suspending agents, solvents, fillers, bulking agents, buffers, delivery vehicles, excipients, agricultural or pharmaceutical adjuvants, and the like.

The type and amount of a pharmaceutical agent used in a treatment method of the present invention can be easily determined by those skilled in the art based on information obtained by a method of the present invention (e.g., information about the level of drug resistance, etc.) and with reference to the purpose of use, a target disease (type, severity, and the like), the patient's age, weight, sex, and case history, the form or type of the cell, and the like. The frequency of the treatment method of the present invention applied to a subject (or patient) is also determined by those skilled in the art with respect to the purpose of use, target disease (type, severity, and the like), the patient's age, weight, sex, and case history, the progression of the therapy, and the like. Examples of the frequency include once per day to several months (e.g., once per week to once per month). Preferably, administration is performed once per week to month with reference to the progression.

As used herein, the term "instructions" refers to a description of a tailor made therapy of the present invention for a person who performs administration, such as a medical doctor, a patient, or the like. Instructions state when to administer a medicament of the present invention, such as immediately after or before radiation therapy (e.g., within 24 hours, etc.). The instructions are prepared in accordance with a format defined by an authority of a country in which the present invention is practiced (e.g., Health, Labor and Welfare Ministry in Japan, Food and Drug Administration (FDA) in the U.S., and the like), explicitly describing that the instructions are approved by the authority. The instructions are so-called package insert and are typically provided in paper media. The instructions are not so limited and may be provided in the form of electronic media (e.g., web sites, electronic mails, and the like provided on the internet).

In a therapy of the present invention, two or more pharmaceutical agents may be used as required. When two or more pharmaceutical agents are used, these agents may have similar properties or may be derived from similar origins, or alternatively, may have different properties or may be derived from different origins. A method of the present invention can be used to obtain information about the drug resistance level of a method of administering two or more pharmaceutical agents.

Also, in the present invention, gene therapy can be performed based on the resultant information about drug resistance. As used herein, the term "gene therapy" refers to a therapy in which a nucleic acid, which has been expressed or can be expressed, is administered into a subject. In such an embodiment of the present invention, a protein encoded by a nucleic acid is produced to mediate a therapeutic effect.

In the present invention, it will be understood by those skilled in the art that if the result of analysis of a certain specific time-lapse profile is correlated with a state of a cell in a similar organism (e.g., mouse with respect to human, etc.), the result of analysis of a corresponding time-lapse profile can be correlated with a state of a cell. This feature is supported by, for example, Dobutsu Baiyo Saibo Manuaru [Animal Culture Cell Manual], Seno, ed., Kyoritsu Shuppan, 1993, which is herein incorporated by reference.

The present invention may be applied to gene therapies. As used herein, the term "gene therapy" refers to a therapy in which a nucleic acid, which has been expressed or can be expressed, is administered into a subject. In such an embodiment of the present invention, a protein encoded by a nucleic acid is produced to mediate a therapeutic effect.

Any methods for gene therapy available in the art may be used in accordance with the present invention. Illustrative methods will be described below.

Methods for gene therapy are generally reviewed in, for example, Goldspiel et al., Clinical Pharmacy 12: 488-505(1993); Wu and Wu, Biotherapy 3: 87-95(1991); Tolstoshev, Ann. Rev. Pharmacol. Toxicol., 32: 573-596(1993) ; Mulligan, Science 260: 926-932(1993); and Anderson, Ann. Rev. Biochem., 62: 191-217(1993); and May, TIBTECH 11(5): 155-215(1993). Commonly known recombinant DNA techniques used in gene therapy are described in, for example, Ausubel et al. (ed.), Current Protocols in Molecular Biology, John Wiley & Sons, NY (1993) ; and Kriegler, Gene Transfer and Expression, A Laboratory Manual, Stockton Press, NY (1990).

(Basic biological techniques)

Techniques used herein are within the technical scope of the present invention unless otherwise specified. These techniques are commonly used in the fields of fluidics, micromachining, organic chemistry, biochemistry, genetic engineering, molecular biology, microbiology, genetics, and their relevant fields. The techniques are well described in documents described below and the documents mentioned herein elsewhere.

Microfabrication is described in, for example, Campbell, S.A. (1996), "The Science and Engineering of Microelectronic Fabrication", Oxford University Press; Zaut, P.V. (1996), "Micromicroarray Fabrication: a Practical Guide to Semiconductor Processing",Semiconductor Services; Madou, M.J. (1997), "Fundamentals of Microfabrication", CRC1 5 Press; Rai-Choudhury, P. (1997), "Handbook of Microlithography, Micromachining, & Microfabrication: Microlithography". Relevant portions (or possibly the entirety) of each of these publications are herein incorporated by reference.

Molecular biology techniques, biochemistry techniques, and microbiology techniques used herein are well known and commonly used in the art, and are described in, for example, Sambrook J. et al. (1989), "Molecular Cloning: A Laboratory Manual", Cold Spring Harbor and its 3rd Ed. (2001); Ausubel, F.M. (1987), "Current Protocols in Molecular Biology", Greene Pub. Associates and Wiley-Interscience; Ausubel, F.M. (1989), "Short Protocols in Molecular Biology: A Compendium of Methods from Current Protocols in Molecular Biology", Greene Pub. Associates and Wiley-Interscience; Innis, M.A. (1990), "PCR Protocols: A Guide to Methods and Applications", Academic Press; Ausubel, F.M. (1992), "Short Protocols in Molecular Biology: A Compendium of Methods from Current Protocols in Molecular Biology", Greene Pub. Associates; Ausubel, F.M. (1995), "Short Protocols in Molecular Biology: A Compendium of Methods from Current Protocols in Molecular Biology", Greene Pub. Associates; Innis, M.A. et al.(1995), "PCR Strategies", Academic Press; Ausubel, F.M. (1999), "Short Protocols in Molecular Biology: A Compendium of Methods from Current Protocols in Molecular Biology", Wiley, and annual updates; Sninsky, J.J. et al. (1999), "PCR Applications: Protocols for Functional Genomics", Academic Press; Special issue, Jikken Igaku [Experimental Medicine] "Idenshi Donyu & Hatsugenkaiseki Jikkenho [Experimental Method for Gene introduction & Expression Analysis]", Yodo-sha, 1997; and the like. Relevant portions (or possibly the entirety) of each of these publications are herein incorporated by reference.

DNA synthesis techniques and nucleic acid chemistry for producing artificially synthesized genes are described in, for example, Gait, M.J. (1985), "Oligonucleotide Synthesis: A Practical Approach", IRL Press; Gait, M.J. (1990), "Oligonucleotide Synthesis: A Practical Approach", IRL Press; Eckstein, F. (1991), "Oligonucleotides and Analogues: A Practical Approach", IRL Press; Adams, R.L. et al. (1992), "The Biochemistry of the Nucleic Acids", Chapman & Hall; Shabarova, Z. et al. (1994), "Advanced Organic Chemistry of Nucleic Acids", Weinheim; Blackburn, G.M. et al. (1996), "Nucleic Acids in Chemistry and Biology", Oxford University Press; Hermanson, G.T. (I996), "Bioconjugate Techniques", Academic Press; and the like. Relevant portions (or possibly the entirety) of each of these publications are herein incorporated by reference.

(Mathematical Analyses)

Mathematical processes used herein can be performed by using well-known techniques described in, for example, Kazuyuki Shimizu, "Seimei Sisutemu Kaiseki no tameno Sugaku [Mathematics for Analyzing Biological Systems]", Corona sha, 1999; and the like. Among these techniques, representative analysis techniques will be described below.

In one embodiment, such a mathematical process may be regression analysis. Examples of regression analysis include, but are not limited to, linear regression (e.g., simple regression analysis, multiple regression analysis, robust estimation, etc.), nonlinear estimation, and the like.

In simple regression analysis, n sets of data ($x_1$, $y_1$) to ($x_n$, $y_n$) are fitted to $y_i=ax_i+b+e_i$ (i=1, 2, ..., n) where a and b are model parameters, and $e_i$ represents a deviation or an error from the straight line. The parameters a and b are typically determined so that the mean of a sum of squares of the distance between a data point and the straight line is minimal. In this case, the rms of the distance is partially differentiated to produce simultaneous linear equations. These equations are solved for a and b which minimize the square errors. Such values are called least square estimates.

Next, a regression line is calculated based on the value obtained by subtracting the mean of all data values from each data value. A regression line represented by:

$$A\Sigma_i X_i + B = \Sigma Y_i$$

is assumed. Further, it is assumed that B=0. The mean ($x_{ave}$, $y_{ave}$) of ($x_i$, $y_i$) (i=1, 2, ..., n) is calculated, and the variance of x ($s_{xx}$) and the covariance of x and y ($s_{xy}$) are calculated. The above-described regression line can be represented by:

$$y - y_{ave} = (s_{xy}/s_{xx})(x - x_{ave}).$$

The correlation coefficient $r_{xy}$ is represented by:

$$r_{xy} = s_{xy}/\sqrt{(s_{xy}s_{yy})}.$$

In this case, the relationship $\Sigma e_i^2/n = S_{yy}(1 - r_{xy}^2)$ is satisfied. Therefore, as $|r_{xy}|$ approaches 1, the error is decreased, which means that data can be satisfactorily represented by the regression line.

In another embodiment, multiple regression analysis is used. In this technique, y is not a single independent variable, and is considered to be a function of two or more variables, e.g., is represented by:

$$y = a_0 + a_1x_1 + a_2x_2 + \cdots + a_nx_n.$$

This equation is called a multiple regression equation. $a_0$ and the like are called (partial) regression coefficients. In multiple regression analysis, a least square method is used and normal equations are solved to obtain least square estimates. Evaluation can be performed as with single regression analysis.

In another embodiment, robust estimation is used. The least square method is based on the premise that measurement values are not biased and measurement errors have a normal distribution, and models have no approximation error. In actual situations, however, there may be errors in measurement. In robust estimation, unreliable data is detected and separated as outliers from the great majority of data which are reliable, or is subjected to a statistical process. Such a robust estimation may be utilized herein.

Nonlinear estimation may also be used herein. With nonlinear estimation, it is possible to represent a nonlinear model as vector equations which are in turn solved.

Other mathematical processes used herein include principal component analysis, which utilizes two-dimensional data principal component analysis, multi-dimensional data principal component analysis, singular value decomposition, and generalized inverse matrix. Alternatively, canonical correlation analysis, factor analysis, discrimination analysis, cluster analysis, and the like may be used herein.

(Gene set classification by cluster analysis)

For a number of applications, it may be desirable to obtain a set of reference transcription control sequences which are cooperatively controlled under a wide range of conditions. An embodiment of identifying such a set of reference transcription control sequences is, for example, a clustering algorithm, which is reviewed in, for example, Fukunaga, 1990, "Statistical Pattern Recognition", 2nd ed., Academic Press, San Diego; Anderberg, 1973, "Cluster Analysis for Applications", Academic Press: New York; Everitt, 1974, "Cluster Analysis", London: Heinemann Educ. Books; Hartigan, 1975, "Clustering Algorithms", New York: Wiley; and Sneath and Sokal, 1973, "Numerical Taxonomy", Freeman.

For ease of understanding, transcriptional controlling sequences are used for exemplary description as follows. A set of transcription control sequences can also be defined based on a transcription control mechanism. Transcription control sequences having a transcription factor binding site for the same or similar sequences in a regulatory region are likely to be cooperatively regulated. In a certain embodiment, the regulatory regions of transcription control sequences of interest are compared with one another using multiple alignment analysis, so that a possible common transcription factor binding site can be determined (Stormo and Hartzell, 1989, "Identifying protein binding sites from unaligned DNA fragments", Proc. Natl. Acad. Sci., 86: 1183-1187; Hertz and Stormo, 1995, "Identification of consensus patterns in unaligned DNA and protein sequences: a large-deviation statistical basis for penalizing gaps", Proc. of 3rd Intl. Conf. on Bioinformatics and Genome Research, Lim and Cantor, ed., World Scientific Publishing Co., Ltd. Singapore, pp. 201-216).

It may be desirable to obtain a set of basic transcription control sequences which are cooperatively regulated under various conditions. With such a set, a method of the present invention can satisfactorily and efficiently carry out determination based on profiles. A preferable embodiment for identifying such a set of basic transcription control sequences includes a clustering algorithm.

In an embodiment using cluster analysis, the transcription levels of a number of transcription control sequences can be monitored while applying various stimuli to biological samples. A table of data containing measurements of the transcription levels of transcription control sequences is used in cluster analysis. In order to obtain a set of basic transcription control sequences containing transcription control sequences which simultaneously vary under various conditions, typically at least two, preferably at least 3, more preferably at least 10, even more preferably more than 50, and most preferably more than 100 stimuli or conditions are used. Cluster analysis is performed for a table of data having mxk dimensions where m is the total number of conditions or stimuli and k is the number of transcription control sequences to be measured.

A number of clustering algorithms are useful for clustering analysis. In clustering algorithms, differences or distances between samples are used to form clusters. In a certain embodiment, a distance used is a Euclidean distance in multi-dimensional space:

$$I(x,y) = \left\{\sum_i \left(X_i - Y_i\right)^2\right\}^{1/2} \quad (1)$$

where (x, y) represents a distance between gene X and gene Y (or any other cellular components X and Y (e.g., transcription control sequences)); $X_i$ and $Y_i$ represent gene expression in response to i stimuli. Euclidean distances may be squared and then multiplied with weighting which are increased with an increase in the distance. Alternatively, a distance reference may be, for example, a distance between transcription control sequences X and Y, or a Manhattan distance represented by:

$$I(x,y) = \sum_i \left|X_i - Y_i\right| \quad (2)$$

where $X_i$ and $Y_i$ represent responses of transcription control sequences or gene expression when i stimuli are applied. Several other definitions of distance include Chebyshev distance, power distance, and mismatch rate. When dimensional data can be categorized without modification, a mismatch rate defined as I (x, y) = (the number of $X_i \neq Y_i$)/i may be used in a method of the present invention. Such a method is particularly useful in terms of cellular responses. Another useful definition of distance is I=1-r where r is a correlation coefficient of response vectors X and Y, e.g., a normalized inner product X·Y/|X||Y|. Specifically, an inner product X·Y is defined by:

$$X \cdot Y = \sum_i X_i \times Y_i \qquad (3).$$

Also, $|X|=(X \cdot X)^{1/2}$ and $|Y|=(Y \cdot Y)^{1/2}$.

Most preferably, a distance reference is suited to a biological problem in order to identify cellular components (e.g., transcription control sequences, etc.) which are simultaneously changed and/or simultaneously regulated. For example, in a particularly preferred embodiment, a distance reference is I=1-r having a correlation coefficient containing a weighted inner product of genes X and Y. Specifically, in such a preferred embodiment, $r_\eta$ is defined by:

$$r = \frac{\sum_i \frac{X_i Y_i}{\sigma_i^{(X)} \sigma_i^{(Y)}}}{\left[\sum_i \left(\frac{X_i}{\sigma_i^{(X)}}\right)^2 \left(\frac{Y_i}{\sigma_i^{(Y)}}\right)^2\right]^{1/2}} \qquad (4)$$

where $\sigma_i^{(X)}$ and $\sigma_i^{(Y)}$ represent standard errors in measurement of genes X and Y in experiment i.

The above-described normalized and weighted inner products (correlation coefficients) are constrained between values +1 (two response vectors are completely correlated, i.e., the two vectors are essentially the same) and -1 (two response vectors are not correlated or do not have the same orientation (i.e., opposing orientations)). These correlation coefficients are particularly preferable in an embodiment of the present invention which tries to detect a set or cluster of components (e.g., biological agents, transcription control sequences, etc.) having the same sign or response.

In another embodiment, it is preferable to identify a set or cluster of cellular components (e.g., transcription control sequences, etc.) which simultaneously regulate the same biological response or pathway or are involved in such regulation, or have similar or non-correlated responses. In such a embodiment, it is preferable to use the absolute value of either the above-described normalized or weighted inner product, i.e., |r| as a correlation coefficient.

In still another embodiment, the relationship between cellular components (e.g., transcription control sequences, etc.), which are simultaneously regulated and/or simultaneously changed, are more complicated, e.g., a number of biological pathways (e.g., signal transduction pathways, etc.) are involved with the same cellular component (e.g., a transcription control sequence, etc.) so that different results may be obtained. In such an embodiment, it is preferable to use a correlation coefficient $r=r^{(change)}$ which can identify cellular components (other transcription control sequences as controls which are not involved in change) which are simultaneously changed and/or simultaneously regulated. A correlation coefficient represented by expression (5) is particularly useful for the above-described embodiment:

$$r = \frac{\sum_i \left|\frac{X_i}{\sigma_i^{(X)}}\right| \left|\frac{Y_i}{\sigma_i^{(Y)}}\right|}{\left[\sum_i \left(\frac{X_i}{\sigma_i^{(X)}}\right)^2 \left(\frac{Y_i}{\sigma_i^{(Y)}}\right)^2\right]^{1/2}} \qquad (5).$$

Various cluster linkage methods are useful in a method of the present invention.

Examples of such a technique include a simple linkage method, a nearest neighbor method, and the like. In these techniques, a distance between the two closest samples is measured. Alternatively, in a complete linkage method, which may be herein used, a maximum distance between two samples in different clusters is measured. This technique is particularly useful when genes or other cellular components naturally form separate "clumps".

Alternatively, the mean of non-weighted pairs is used to define the mean distance of all sample pairs in two different clusters. This technique is also useful in clustering genes or other cellular components which naturally form separate "clumps". Finally, a weighted pair mean technique is also available. This technique is the same as a non-weighted pair mean technique, except that in the former, the size of each cluster is used as a weight. This technique is particularly useful in an embodiment in which it is suspected that the size of a cluster of transcription control sequences or the like varies considerably (Sneath and Sokal, 1973, "Numerical taxonomy", San Francisco: W.H. Freeman & Co.). Other cluster linkage methods, such as, for example, non-weighted and weighted pair group centroid and Ward's method, are also useful in several embodiments of the present invention. See, for example, Ward, 1963, J. Am. Stat. Assn., 58: 236; and Hartigan, 1975, "Clustering algorithms", New York: Wiley.

In a certain preferred embodiment, cluster analysis can be performed using a well-known hclust technique (e.g., see a well-known procedure in "hclust" available from Program S-Plus, MathSoft, Inc., Cambridge, MA).

According to the present invention, it was found that even if the versatility of stimuli to a clustering set is increased, a state of a cell can be substantially elucidated by analyzing typically at least two, preferably at least 3, profiles using a method of the present invention. Such stimulation conditions include, for example with respect to biological systems treatment with a pharmaceutical agent in different concentrations, different measurement times after treatment, response to genetic mutations in various genes, a combination of treatment of a pharmaceutical agent and mutation, and changes in growth conditions (temperature, density, calcium concentration, etc.).

As used herein, the term "significantly different" in relation to two statistics means that the two statistics are different from each other with a statistical significance. In an embodiment of the present invention, data of a set of experiments assessing the responses of cellular components can be randomized by a Monte Carlo method to define an objective test.

In a certain embodiment, an objective test can be defined by the following technique. $p_{ki}$ represents a response of a component k in experiment i. $\Pi_{(i)}$ represents a random permutation of the indices of experiments. Next, $p_{k\Pi(i)}$ is calculated for a number of different random permutations (about 100 to 1,000). For each branch of the original tree and each permutation:

(1) hierarchical clustering is performed using the same algorithm as that which has been used for the original data which is not permutated (in this case, "hclust"); and

(2) an improvement f in classification in total variance about the center of clusters when transition is made from one cluster to two clusters;

$$f = 1 - \Sigma D_k^{(1)} / \Sigma D_k^{(2)} \qquad (6)$$

where $D_k$ is the square of the distance reference (mean) of component k with respect to the center of a cluster to which component k belongs. Superscript 1 or 2 indicates the center of all branches or the center of the more preferable cluster of the two subclusters. The distance function D used in this clustering technique has a considerable degree of freedom. In these examples, D=1-r, where r is a correlation coefficient of one response with respect to another response of a component appearing in a set of experiments (or of the mean cluster response).

Specifically, an objective statistical test can be preferably used to determine the statistical reliability of grouping any clustering methods or algorithms. Preferably, similar tests can be applied to both hierarchical and nonhierarchical clustering methods. The compactness of a cluster is quantitatively defined as, for example, the mean of squares of the distances of elements in the cluster from the "mean of the cluster", or more preferably, the inverse of the mean of squares of the distances of elements from the mean of the cluster. The mean of a specific cluster is generally defined as the mean of response vectors of all elements in the cluster. However, in a specific embodiment (e.g., the definition of the mean of the cluster is doubtful), for example, the absolute values of normalized or weighted inner products are used to evaluate the distance function of a clustering algorithm (i.e., I=1-|r|). Typically, the above-described definition of the mean may raise a problem in an embodiment in which response vectors have opposing directions so that the mean of the cluster as defined above is zero. Therefore, in such an embodiment, a different definition is preferably selected for the compactness of a cluster, for example, without limitation, the mean of squares of the distances of all pairs of elements in a cluster. Alternatively; the compactness of a cluster may be defined as the mean of distances between each element (e.g., a cellular component) of a cluster and another element of the cluster (or more preferably the inverse of the mean distance).

Other definitions, which may be used in statistical techniques used in the present invention, are obvious to those skilled in the art.

In another embodiment, a profile of the present invention can be analyzed using signal processing techniques. In these signal processing techniques, a correlation function is defined, a correlation coefficient is calculated, an autocorrelation function and a cross-correlation function are defined, and these functions are weighted, where the sum of the weights is equal to 1. Thereby, moving averages can be obtained.

In signal processing, it is important to consider a time domain and a frequency domain. Rhythm often plays an important role in dynamic characteristic analysis for natural phenomena, particularly life and organisms. If a certain time function f(t) satisfies the following condition, the function is called a periodic function:

$$f(t) = f(t+T).$$

At time 0, the function takes a value of f(0). The function takes a value of f(0) at time T again after taking various values after time 0. Such a function is called a periodic function. Such a function includes a sine wave. T is called a period. The function has one cycle per time T. Alternatively, this feature may be represented by 1/T which means the number of cycles per unit time (cycles/time) without loss of the information. The concept represented by the number of cycles per unit time is called frequency. If the frequency is represented by f, f is represented by:

$$f=1/T.$$

Thus, the frequency is an inverse of the time. The time is dealt in a time domain, while the frequency is dealt in a frequency domain. The frequency may be represented in an electrical engineering manner. For example, the frequency is represented by angular measure where one period corresponds to 360° or $2\pi$ radians. In this case, f (cycles/sec) is converted to $2\pi f$ (radians/sec), which is generally represented by $\omega$ ($=2\pi f$) and is called angular frequency.

Now, a sine wave is compared with a cosine wave. The cosine wave is obtained by translating the sine wave by 90° or $\pi/2$ radians. The sine wave may be represented by the delayed cosine wave. This time delay is called phase. For example, when a pure cosine wave has a phase of 0, a sine wave has a phase of 90°. When a sine wave is added to a cosine wave, the amplitude of the resultant wave is increased by a factor of $\sqrt{2}$ and the phase is $\pi/4$.

In such analysis, Fourier series and frequency analysis may be available. In addition, Fourier transformation, discrete Fourier transformation, and power spectrum may be available. In Fourier expansion, techniques, such as wavelet transformation and the like, may be available. These techniques are well known in the art and are described in, for example, Yukio Shimizu, "Seimei Sisutemu Kaiseki notameno Sugaku [Mathematics for analyzing life systems]", Corona sha, (1999); and Yasuhiro Ishikawa, "Rinsho Igaku notameno Ueburetto Kaiseki [Wavelet analysis for clinical medicine]", Igaku Shuppan.

(Description of elemental technologies)

Hereinafter, embodiments of elemental technologies for practicing the present invention will be described by way of embodiments. The embodiments described below are provided only for illustrative purposes. Accordingly, the scope of the present invention is not limited by the embodiments except as by the appended claims.

(A method for presenting a state of a biological system (e.g. a cell, a biological entity). The method comprises the steps of: a) obtaining a time-lapse profile of the biological system (e.g. a cell or a biological entity) by time-lapse monitoring of a gene state (e.g., the expression of a gene (transcription, translation, etc.), etc.) associated with at least one gene selected from genes derived from the biological system (e.g. a cell or a biological entity); and b) presenting the time-lapse profile. For example, the profile of the intensity of a signal obtained by monitoring is subjected to interval differentiation, thereby obtaining a function of changes which can be in turn displayed. In this case, preferably, for example a biological factor such as a constitutive promoter or the like, which is assumed to be changed, can be used as a reference to obtain a difference, thereby obtaining a time-lapse profile. The present invention is not limited to this.

Time-lapse profiles may be displayed using any method, for example, they may be visually displayed using a display device (e.g., an x axis showing time while the y axis shows signal intensity), or alternatively, may be displayed as a table of numerical values. Alternatively, signal intensity may be displayed as optical intensity. Furthermore, profiles may be presented by means of sound.

Preferably, biological systems (e.g. a cell or a biological entity) are fixed to a solid phase support (e.g., an array, a plate, a microtiter plate, etc.) when they are monitored. Such fixation can be carried out using techniques known in the art or techniques as described herein. Fixation or immobilization of the biological system (e.g. a cell or a biological entity) allows systematic investigation thereof.

In a preferred embodiment, such a time-lapse profile may be presented in real time. The real time presentation may contain a time lag to some extent if it is performed substantially in real time. A tolerable time lag is, for example, 10 seconds at maximum, and more preferably 1 second at maximum, though the tolerable time lag depends on the required level of real time (simultaneity).

In another aspect, the present invention provides a method for determining a state of a biological system (e.g. a cell or a biological organism). Such determination of the state of the biological system (e.g. a cell or a biological organism) is achieved by monitoring changes in a transcriptional state of a transcription control factor, which are not conventionally observed. Therefore, the method of the present invention for determining the state of the biological system (e.g. a cell or a biological organism) allows determination of various states which cannot be conventionally observed. Such a method comprises the steps of: a) obtaining a time-lapse profile of the biological system (e.g. a cell or a biological organism) by time-lapse monitoring of a transcriptional state associated with at least one biological agent selected from a biological agent group derived from the biological system (e.g. a cell or a biological organism); and b) determining the state of the cell based on the time-lapse profile of the transcription level.

Preferably, a biological system (e.g. a cell or a biological organism) is fixed to a solid phase support (e.g., an array, a plate, a microtiter plate, etc.) when they are monitored. Such fixation can be carried out using techniques known in the art or techniques as described herein.

In a preferred embodiment, advantageously, the state determination method for a biological system (e.g. a cell or a biological organism) of the present invention may further comprise correlating the time-lapse profile with the state of the biological system (e.g. a cell or a biological organism) before obtaining the time-lapse profile. Alternatively, such correlation information may be provided from known information. Such a correlating step may be performed at every determining step or correlation information may be stored in a database and used as required.

In a preferred embodiment, the biological agent used in the present invention may be a transcription control sequence. Such a transcription control sequence may be, without limitation, a promoter, an enhancer, a silencer, another flanking sequence of a structural gene in a genome, and a genomic sequence other than exons. A promoter is preferable. This is because a transcription level can be directly measured, and the state of transcription directly reflects the state of a biological system (e.g. a cell or a biological organism). In a particular embodiment, the transcription control sequences may include constitutive promoters, specific promoters, inducible promoters, and the like.

In certain embodiments, any biological agent, such as a promoter, may be used. The present invention is characterized in that any type of biological agent can be used. According to the method of the present invention, profiles can be analyzed from a viewpoint of "procession". Therefore, it is possible to determine a state of a biological system (e.g. a cell or a biological organism) using any biological agent such as a promoter, structual gene, or any set or combination thereof. Such determination cannot be achieved by conventional techniques. The present invention is highly useful since the present invention achieves what cannot be achieved by conventional techniques.

In a preferred embodiment, at least two biological agents (for example, transcriptional control sequence) are monitored. By observing at least two biological agents, 80% of the states of a biological system (e.g. a cell or a biological organism) can be typically identified. More preferably, at least 3 biological agents are monitored. By observing at least three biological agents, at least 90% of the states of a cell can be typically identified. In a most preferred embodiment, at least 8 biological agents are monitored. By observing at least 8 biological agents, substantially all of the states of a biological system (e.g. a cell or a biological organism) can be typically identified. Thus, although any biological agents are selected, substantially all of the states of a biological system (e.g. a cell or a biological organism) can be determined by selecting and monitoring a small number of biological agents, as described above. This feature has not been conventionally expected. The method of the present invention is simpler, more precise and more accurate than conventional determination methods in which observation is made at time points and resultant data is statistically processed as a heterologous group. Such provision of time-lapse information allows further processing of the information to obtain or extract particular information such as event timing.

Therefore, in the casese where a biological system is used, the determination method of the present invention preferably further comprises arbitrarily selecting at least one biological agent from a biological agent group before monitoring. An important feature of the present invention is such that a biological agent, which does not exhibit specificity when investigated from point to point, can be used. Further, the present invention allows accurate reflection of the resultant data to the state of a biological system (e.g. a cell or a biological organism) of interest, since linearly measured data under a consistent environment can be used. Such accurate data cannot be obtained conventionally.

In a preferred embodiment, such a profile obtained in the present invention may be presented in real time. Alternatively, in the present invention, data may be obtained in a real time manner. The real time presentation may contain a time lag to some extent if it is performed substantially in real time. A tolerable time lag is, for example, 10 seconds at maximum, and more preferably 1 second at maximum, though the tolerable time lag depends on the required level of real time (simultaneity). As used herein, the term "real time" means that the real time presentation may contain a time lag to some extent if it is performed substantially in real time. A tolerable time lag is, for example, 10 seconds at maximum, and more preferably 1 second at maximum, though the tolerable time lag depends on the required level of real time (simultaneity). For example, the level of real time may be preferably 30 seconds at maximum, or even longer in the case of, for example, therapies required for real time diagnosis.

In a particular preferable embodiment, states determined by the state of a biological system (e.g. a cell or a biological organism) the determination method of the present invention includes, for example, differentiated states, undifferentiated states, responses of a biological system (e.g. a cell or a biological organism) to external factors, cell cycles, growth states, and the like. More specifically, such a state includes, for example, without limitation, a response of a cancer cell to an anticancer agent, drug resistance, a response to a biological clock, a differentiated state of a stem cell (e.g., a mesenchymal stem cell, a neural stem cell, etc.), an undifferentiated state of a purified stem cell (e.g., an embryonic stem cell, etc.), a change in morphology of a biological system (e.g. a cell or a biological organism), a state of migration of a biological system (e.g. a cell or a biological organism), intracellular localization of a molecule, production of a secreted substance, and the like.

Therefore, in a preferred embodiment, a biological system (e.g. a cell or a biological organism) assessed by the determination method for the state of a biological system (e.g. a cell or a biological organism) of the present invention includes, for example, without limitation, a stem cell or a somatic cell, or a mixture thereof. Alternatively, such a cell includes an adherent cell, a suspended cell, a tissue forming cell, and a mixture thereof.

In a preferred embodiment, the state of a biological system (e.g. a cell or a biological organism) determination method of the present invention may be performed upon a biological system (e.g. a cell or a biological organism) fixed on a substrate which is a solid phase support. In such a case, the solid phase support is called a chip. When biological systems (e.g. cells or biological organisms) are arrayed on the substrate, the substrate is also called an array.

In a particularly preferred embodiment of the state determination method for a biological system (e.g. a cell or a biological organism) of the present invention, advantageously, when a biological agent (for example, a transcription control sequence) used for determination is a nucleic acid molecule, such a nucleic acid molecule may be operably linked to a reporter gene sequence and may be transfected into a cell. In this case, the transcription level of the transcription control sequence can be measured as a signal from the reporter gene.

Such transfection may be performed in a solid phase or in a liquid phase. For transfection, a technique for increasing the efficiency of introduction of a target substance into a cell may be used. In the present invention, a target substance (e.g., DNA, RNA, a polypeptide, a sugar chain, or a composite substance thereof, etc.), which cannot be substantially introduced into cells under typical conditions, is presented (preferably, contacted) along with an actin acting substance, such as fibronectin, to a cell, thereby making it possible to efficiency introduce the target substance into cells. Therefore, the transfection method comprises the steps of: A) providing a target substance (i.e., DNA comprising a transcription control sequence) and B) providing an actin acting substance (e.g., fibronectin), wherein the order of steps of A) and B) is not particularly limited, and C) contacting the target substance and the actin acting substance with the cell. The target substance and the actin acting substance may be provided together or separately. The actin acting substance may be used as described in detail above for the composition of the present invention for increasing the efficiency of introduction of a target substance into a cell. Such a technique can be carried out as appropriate based on the present specification by those skilled in the art. Therefore, the actin acting substance may be used in a manner which is described in detail above for the composition of the present invention for increasing the efficiency of introduction of a target substance into a cell. Preferably, the actin acting substance may be an extracellular matrix protein (e.g., fibronectin, vitronectin, laminin, etc.) or a variant thereof. More preferably, fibronectin or a variant or fragment thereof may be used.

In one embodiment, in the case where a biological agent used in the present invention is a transcription control sequence, the transcription control sequence used in the present invention may be capable of binding to a transcription factor. Examples of such a transcription factor include, but are not limited to, ISRE, RARE, STAT3, GAS, NFAT, MIC, AP1, SRE, GRE, CRE, NFκB, ERE, TRE, E2F, Rb, p53, and the like. These transcription factors are commercially available from BD Biosciences Clonetech, CA, USA. ISRE is related to STAT1/2. RARE is related to retinoic acid. STAT3 is related to the control of differentiation. GRE is related to the metabolism of sugar. CRE is related to cAMP. TRE is related to thyroid hormone. E2F is related to cell cycle. p53 is related to G1 check point. Therefore, such information can be used to determine a state of a cell.

In a preferred embodiment, the determination step of the present invention comprises comparing the phases of the time-lapse profiles. Phases can be calculated by those skilled in the art using general techniques as described herein above and techniques described in the examples below.

In another preferred embodiment, the determination step of the present invention comprises calculating a difference between the time-lapse profile of the cell and a control profile. The difference can be calculated by those skilled in the art using general techniques as described herein above and techniques described in Examples below.

In another preferred embodiment, the determination step of the present invention comprises a mathematical process selected from the group consisting of signal processing and multivariate analysis. Such a mathematical process can be easily carried out by those skilled in the art based on the description of the present specification.

(Description of preferred embodiments)

Hereinafter, the present invention will be described by way of embodiments. The embodiments described below are provided only for illustrative purposes. Accordingly, the scope of the present invention is not limited by the embodiments.

(Event descriptor production method)

In one embodiment, the present invention provides a method for producing an event descriptor relating to at least one system. The present method comprises the steps of: (A) obtaining time series data of at least one index derivied from at least one system; (B) providing at least one characteristic behaviour relating to the index; and (C) extracting a portion having the characteristic behaviour in the times series data as an event timing to produce an event descriptor described by the event timing. As used herein, time-series data may be obtained by means of any appropriate methods depending on the system and index used. For example, as an index of a biological system, biological means, biochemical means, chemical means (for example, using chemical reactions), physical means (for example, absorbance and the like), may be used herein. As an index of an economic system, raw numerical values may be used. Characteristic behaviours may be appropriately determined depending on the system and/or index to be targeted by the analysis. Such a characteristic behaviour includes, but is not limited to, for example, change in sign (+/-) of a first-order differentiation, coincidence of the time-series data and a predetermined value, or a specific variation or no change of the absolute value change rate thereof; coincidence of a first-order differentiation value of the time-series data and a predetermined value, or a specific variation or no change of the absolute value change rate thereof; coincidence of a second-order differentiation value of the time-series data and a predetermined value, or a specific variation or no change of the absolute value change rate thereof; change in sign (+/-) of the time-series data; change in sign (+/-) of the first-order differentiation value of the time-series data; change in sign (+/-) of the second-order differentiation value of the time-series data; coincidence of the time-series data and time-series data of another index; coincidence of the first-order differentiation of the time-series data and the first-order differentiation of time-series data of another index; coincidence of the second-order differentiation of the time-series data and the second-order differentiation of time-series data of another index; coincidence of sign (+/-) of the time-series data and the sign of time-series data of another index; coincidence of sign (+/-) of the first-order differentiation value of the time-series data and the sign of the first-order differentiation value of time-series data of another index; coincidence of sign (+/-) of the second-order differentiation value of the time-series data and the sign of the second-order differentiation value of time-series data of another index; coincidence of the time-series data and another time-series data of the index; coincidence of the first-order differentiation of the time-series data and the first-order differentiation of another time-series data of the index; and coincidence of the second-order differentiation of the time-series data and the second-order differentiation of another time-series data of the index. Those skilled in the art may appropriately select any appropriate means. The production of the descriptor may be performed using any means from a signal obtained by monitoring. Such a method includes, but is not limited to a method for replacing a particular electric signal with a particular letter/character string, a method for altering an electric signal to a light signal, and the like, and those methods may be performed using any method well known in the art. For example, it may be a method for mathmaticallyprocessing an electric signal to replace the result thereof with another signal.

In an embodiment, a system includes but is not limited to, for example a scientific system (for example, a physical system, chemical system, biological system (for example, a cell, tissue, organ, organism and the like), a geophysical system, an astronomical system and the like), a social scientific system (for example, a company organization), a human scientific system (for example, history, geography, and the like), an economic system (for example, stock exchange, currency exchange and the like), a mechanical system (for example, a computer, apparatus and the like), and the like.

In a preferable embodiment, a biological system (for example, a cell, tissue, organ, organism and the like) may be targeted. The analysis of a biological system (for example, a cell, tissue, organ, organism and the like) was impossible subject for detailed analysis using time-series data. Accordingly, the present invention provides a significant effect in efficiency and simplicity in the analysis of such a biological system (for example, a cell, tissue, organ, organism and the like).

In another embodiment, a biological system (for example, a cell, tissue, organ, organism and the like) may be a biological organism *per se,* or an organ, tissue, group of cells, cell and cellular organelle and the like. Alternatively, in another preferable embodiment, the system may be a cell. The time-series data of a cell can only obtained by means of the methods described herein in detail for the first time, developed by the present inventors. As such, the present invention is said to be achieved by the present disclosure of the present invention.

In another embodiment, the system may be a social scientific organization. It was unexpectedly elucidated that the present invention may be used to produce descriptors for use in analysis of an organization (for example, the state of an organization).

In another embodiment, the system may be an economic system. It was unexpectedly elucidated that the present invention may be used to correlated information behind the simple analysis in an economical system, such as stock prices.

Indices used in the present invention may vary depending on the system used, and it is understood that those skilled in the art can appropriately select an index depending on the system used. The system which may be used in the present invention, includes but is not limited to, for example, indices such as natural scientific indices, technical indices, social scientific indices and human scientific indices, for example, physical indices, chemical indices, biochemical indices, and biological indices and the like.

In a preferable embodiment, indices used in the present invention include a differentiation state, responses to an external agent, cellular cycle, proliferation state, an apoptosis state, response to to an environmental change, an aging state, intracellular interaction, chemostasis, elongation rate, morphology, volume change and the like.

In another embodiment, the indices used in the present invention includes gene expression level, gene trancriptional level, gene posttranslational modification level, chemical substance level present inside a cell, intracellular ionic level, cellular volume, biochemical process level, and biophysical process level (for example, including those expressed as biological macromolecule, study of the physical structure or property of a structure constituted by the macromolecule, study for elucidation at a molecular level of a variety of biological mechanisms, study to elucidate using simulation using physical data and computer to model biological mechanisms, and the like).

In a preferable embodiment, the indices used in the present invention may be selected from the group consisting of gene expression level and gene trancriptional level. More preferably, the indices used in the present invention include gene transcriptional level. Analysis of transcriptional level allows analysis of behaviours inside a cell in a detailed manner.

The characteristic behaviour used in the present invention may be any pattern depending on the system and index used, and the characteristic behaviour according to the present invention includes, but is not limited to, for example, coincidence of the time-series data and a predetermined value, or a specific variation or no change of the absolute value change rate thereof; coincidence of a first-order differentiation value of the time-series data and a predetermined value, or a specific variation or no change of the absolute value change rate thereof; coincidence of a second-order differentiation value of the time-series data and a predetermined value, or a specific variation or no change of the absolute value change rate thereof; change in sign (+/-) of the time-series data; change in sign (+/-) of the first-order differentiation value of the time-series data; change in sign (+/-) of the second-order differentiation value of the time-series data; coincidence of the time-series data and time-series data of another index; coincidence of the first-order differentiation of the time-series data and the first-order differentiation of time-series data of another index; coincidence of the second-order differentiation of the time-series data and the second-order differentiation of time-series data of another index; coincidence of sign (+/-) of the time-series data and the sign of time-series data of another index; coincidence of sign (+/-) of the first-order differentiation value of the time-series data and the sign of the first-order differentiation value of time-series data of another index; coincidence of sign (+/-) of the second-order differentiation value of the time-series data and the sign of the second-order differentiation value of time-series data of another index; coincidence of the time-series data and another time-series data of the index; coincidence of the first-order differentiation of the time-series data and the first-order differentiation of another time-series data of the index; and coincidence of the second-order differentiation of the time-series data and the second-order differentiation of another time-series data of the index, and the like.

In a preferable embodiment, the characteristc behavious is a change in the sign of the first-order differentiation of a time-series data. This change is also called an inflection point, and when this is used in terms of gene control, it is believed that it shows the on-off state of the means of controlling the gene regulation.

In one embodiment, the time-series data used in the present invention may be in any format. For example, time-series data may be continuous or discontinuous. Usually, discontinuous data is used. Description of continuous data is impossible when using digital recording apparatus. It should be understood that continuous data may be used in the present invention.

In one embodiment, the time-series data used in the present invention may be described in a relative or absolute time. An absolute time is preferable, but may be relative. When using an absolute time, the time range of the timing may be arbitrarily determined.

In a preferable embodiment, the time-series data used in the present invention may be described in such a manner that the initiation time of observation is expressed as a reference (0). This is becaused the data at the initiation time of obseration is assumed to be observed under conditions with some fixed reference. However, the data is not necessarily employed as a reference. When it is possible to identify or assume an event as a different reference, such an event may be empolyed as a reference (0) .

In one embodiment, the time-series data used in the present invention may be described in a relative level or an absolute level. There are some cases where relative level is preferable. In some cases, absolute level is preferable. Alternatively, data after normalization may be preferably used. Such processing may be appropriately determined depending on the property of the system and indices used.

In a preferable embodiment, the time-series data used in the present invention is gene expression data, and the gene expression level may be the expression level of a fluorescent protein. The gene expression used herein includes transcription and translation. The behaviour of the "change" of such genes may be observed using a fluorescent protein. In particular, when referring to transcription level, the behaviour of the promoter may be visualaized using means of a fluorescent protein. Methods for linking a fluorescent protein encoding sequence to a promoter are concisely described herein and well known in the art.

In one embodiment, the event timing used in the present invention maybe expressed as a time point or a time range. Such a time range or shift between the time point may be contemplated to any period of time, and includes, but is not limited to, for example, within one or more seconds, one or more minutes, one or more hours, one or more days, one or more months, one or more years, and the like. Such a period of time may be altered and selected depending on the system and indices of interest. In some embodiments, it includes, but is not limited to within twelve hours, within ten hours, within eight hours, within six hours, within three hours, within two hours, within one hour, within thirty minutes, within fifteen minutes, within five minutes, within one minute and the like.

In another embodiment, the present invention further comprises the step of mathematically processing the time series data. Such mathematical processing includes, but is not limited to, for example, normalization, first-order differentiation, second-order differntiation, third-order differentiation, linear approximation, non-linear approximation, moving agerage, noise filter (Kalman filter and the like), Fourier's transform, fast Fourier's transform and principal component analysis and the like.

In one embodiment, the event timing used in the present invention may be calculated based on the raw data of the time-series data. Raw data may be directly used, or alternatively, can be normalized. Alternatively, with respect to raw data, the lowest value is assumed to be zero (0), and the maximum value is assumed to be 100, and the value may be expressed as a relative value.

Alternatively, in a preferable embodiment, the event timing usedin the present invention, may be calculated based on the first- or second-order differentiation of the time-series data. These mathmatical processing may be used in combination of two or more thereof.

In another embodiment, the event timing used in the present invention may be calculated based on the coincidence of the increase or descrease per unit time in a plurality of time-series data. As used herein, the unit time may be identical or different to each other.

In another embodiment, the event timing used in the present invention may be represented in the increase, decrease or unchangeness of the index. The representation of the unchangeness may be omitted.

In another embodiment, the event timing used in the present invention may be represented by the expression manner of (time t, the increase, decrease or unchangeness of the index <+, - or 0>). Such an expression manner may be arbitrarily altered. As used herein, the time t used herein may be expressed as a time point or time range.

In one embodiment, the event descriptor used in the present invention may be described by aligning characters or letters related to the event timing in an order of time points. When altering the same into characters or letters, it is possible to use any technology used in word-processing technology, and the following technologies may be used: alignment analysis in space sequence, alignment analysis allowing characters/letters expressing a null state, parsing methods in a natural language processing, sequence analysis algorithm used in gene sequence alignment, and the like.

In another embodiment, the event descriptor used in the present invention may be represented by means of A, T, G or C, which are single letter designations of nucleic acids in an order of time points. The use of such a designation method allows analysis of a system (for example, a cell, a biological organism and the like) using a genetic algorithm.

In another embodiment, the increase or decrease in index usedin the present invention includes, but is not limited to cases where it is recognizable that the value or raw data in an experimental system is statistically significant compared to the control for comparison, and the cases where the value after the first-order differentiation is increased by 10% or the like.

In another embodiment, the increase or decrease in index used in the present invention may use the following but is not limited thereto: statistically significant increase in the normalized time-series data, 1% increase over the previous time point (timing), 2% increase over the previous time point (timing), 5% increase over the previous time point (timing), 10% increase over the previous time point (timing), 20% increase over the previous time point (timing), exceeding or underperforming a reference value, the point at which the sign of a first-order differentiation value is changed, the point at which the sign of a second-order differentiation value is changed, the cases where the value of raw data is significantly altered in an experimental system. Such a reference may be appropriately determined and selected by those skilled in the art depending on the nature of a system.

In another embodiment, at least two indices are used as an index used in the present invention, and it is preferable to select those which coincide in at least two types of indices in terms of the behaviour of increase or decrease of the index at least one point, may be used as an event timing used in the present invention. By selecting the coincidence of the timing may allow selection of a collection of inflection points. When using the same in a cell, an important inflection point may be extracted. Such an inflection point is referred as "big event" amongst cell events, and is an important event in the activities of a cell. As used herein, such big event may be called or referred to as, for example, "private event", "marked event", "individual event", "turning event", "characteristic event", "peculiar event", "typical event", "special event", and the like. Such an event had not been able to be selected without the use of the technology according to the present invention, and thus can be recognised as a significant effect which has been first attained by the present invention.

In another embodiment, when a change in a first-order differentiation and a sign change in a second-order differentiation are used as a characteristic behaviour used in the present invention, the event descriptor may be expressed as a letter/character string in an order of the first letter corresponding to the first-order differentiation value, and the second letter/character corresponding to the sign change in the second-order differentiation value. Such an expression system allows global analysis relating to the rate and acceleration. A variety of tendency of indices may be analyzed as a sequence.

In another embodiment, when a sign change in first-order differentiation and sign change in second-order differentiation are used as the characteristic behaviour are used as a characteristic behaviour used in the present invention, a first letter/character corresponding to the sign change of the first-order differentiation, a second letter corresponding to the sign change of the second-order differentiation and a third letter/character corresponding to another letter/character regarding the time without sign change may be represented in a form of a character string according to the time order as the event descriptor. Such a description allows global analysis relating to the rate and acceleration. In such a case, analysis may be possible referring to an absolute time. A variety of tendency of indices may be analyzed as a sequence.

In another embodiment, when a sign change in raw data is used as a characteristic behaviour used in the present invention, a first letter/character corresponding to the increase in the raw data, and a second letter/character corresponding to the decrease in the raw data, may be represented in a form of a character string according to the time order as the event descriptor. In such a case, for example, the tendency of a rise in stock price may be analyzed as a sequence.

In another embodiment, when a sign change in raw data is used as a characteristic behaviour used in the present invention, a first letter/character corresponding to the increase in the raw data, a second letter/character corresponding to the decrease in the raw data, and a third letter/character corresponding to another character/letter regarding the time without increase or decrease may be represented in a form of a character string according to the time order as the event descriptor. A variety of tendency of indices may be analyzed as a sequence.

In another embodiment, the event descriptor used in the present invention may be described with the notation such as electric wave, magnetic wave, sound, light, color, image, number and character/letter, and the like. When used in information processing, letter/character, or numbers are preferably used as the notation. There are some cases where it is efficient to use means such as electric waves, sound, magnetic wavse, and the like, and thus the present invention is not necessarily limited to the use of letters/characters or numbers.

In a preferable embodiment, the present invention further comprises the step of recording an event descriptor on a storage medium. Such a storage medium may be, for example, any types of flexible disc, MO, CD-ROM, CD-R, DVD-ROM, and the like.

(Analysis method)

In another aspect, the present invention provides a method for analyzing at least one system using an event descriptor relating to the system. The present method comprises the steps of: (A) obtaining time-series data of at least one index derived from at least one system; (B) providing at least one characteristic behaviour; (C) extracting a portion having the characteristic behaviour as an event timing in the time-series data; and (D) analyzing the at least one event descriptor. Amongst the steps involved, steps (A), (B) and (C) may make use of any embodiment described in the above-described event descriptor production method described herein. Event descriptor analysis may be performed by means of any appropriate means depending on the expression method of the descriptor. For example, when using letters/characters, algorithms such as an alignment analysis for letter/character processing may be used. It should be understood that such an algorithm includes, but is not limited to, for example, alignment analysis, self-organization mapping, cluster analysis, genetic algorithm, alignment analysis, and parsing in a natural language processing, and the like.

In another embodiment, systems targeted by the method of analysis according to the present invention, includes, but are not limited to: for example, scientific systems (for example, physical systems, chemical systems, biological systems (for example, cells, tissues, organs, organisms and the like), geophysical systems, astronomic systems, and the like), social scientific systems (for example, company organisations and the like), human scientific systems (for example, history, geography and the like), economic systems (for example, stock price, exchange and the like), machinery systems (for example, computer, apparatus and the like) and the like. In a particular embodiment, the system targeted by the method of analysis according to the present invention may be a biological system. In particular, the system targeted by the method of analysis according to the present invention may be a cell.

In one preferable embodiment, an analysis method according to the present invention analyzes the relationship between first and second indices in a system. The present particular method comprises the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). The event descriptor production method used herein may be of any embodiment described in detail hereinabove in the above-desribed descriptor production method. As used herein the event descriptor analysis may be performed using any appropriate means depending on the method of displaying the descriptor. For example, when using letters/characters, genetic algorithm may be used as such an algorithm.

In a preferable embodiment, the comparison in step (C) in the above-mentioned analysis method according to the present invention may be performed by means of production of coinciding event timing at which behaviours coincide in the first and second event descriptors.

In a preferable embodiment, the analysis method according to the present invention analyzes the relationship between a first index from a first system and a second index from a second system. The present particular analysis method comprises: the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). The event descriptor production method used herein may be of any embodiment described in detail hereinabove in the above-desribed descriptor production method. As used herein the event descriptor analysis may be performed using any appropriate means depending on the method of displaying the descriptor. For example, when using letters/characters, genetic algorithm may be used as such an algorithm.

In another particular embodiment, the method for analysis according to the present invention analyzes the relationship between indices at a first and second time points from a system. The present particular analysis method comprises: the steps of: (A) producing a first event descriptor relating to the first time point using a method according to the present invention; (B) producing a second event descriptor relating to the second time point using a method according to the present invention; and (C) comparing the first and second event descriptors obtained in the steps (A) and (B). The event descriptor production method used herein may be of any embodiment described in detail hereinabove in the above-desribed descriptor production method. As used herein the event descriptor analysis may be performed using any appropriate means depending on the method of displaying the descriptor. For example, when using letters/characters, genetic algorithm may be used as such an algorithm.

In another particular embodiment, the method for analysis according to the present invention analyzes an index from a system using an event descriptor obtained using first and second characteristic behaviours. The present particular analysis method comprises: the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). The event descriptor production method used herein may be of any embodiment described in detail hereinabove in the above-desribed descriptor production method. As used herein the event descriptor analysis may be performed using any appropriate means depending on the method of displaying the descriptor. For example, when using letters/characters, genetic algorithm may be used as such an algorithm.

In a particular embodiment, the step of comparison comprises the step of extracting an event timing which coincide a time point between the event timing in the first event descriptor and the event timing of the second event descriptor.

(Descriptor production system)

In another aspect, the present invention provides a production system for producing an event descriptor relating to a system. The production system comprises i) monitoring means for monitoring at least one index relating to the system in a time-lapse manner; and ii) descriptor production means for producing an event descriptor by producing a time-series data of the system from a signal obtained from the monitoring means, and calculating the time-series data. As used herein the monitoring means may be of any means as long as the means can monitor at least one index relating to a system targeted by the measurement in a time-lapse manner. Such means varies depending on the system and indices used, and it is understood that those skilled in the art can appropriately select such means. Such monitoring means includes, but is not limited to, for example, calculation (for example, of reflection light intensity) in a direct or indirect manner by the use of an optical microscope, a fluorescent microscope, reading devices or the like, means for measuring the intensity of a marker, antibody, fluorescence label specific to a cell by staining, a reading apparatus using a laser light source, surface plasmon resonance (SPR) imaging, reading devices of a signal derived from a means using electric signals, chemical or biochemical markers or a combination thereof, CCD camera, autoradiography, MRI and a variety of sensors (temperature sensors, oxygen electrodes, and the like), and the like.

As used herein the descriptor production means used in the production system according to the present invention has a function of (A) obtaining time series data of at least one index derivied from at least one system; (B) providing at least one characteristic behaviour relating to the index; and (C) extracting a portion having the characteristic behaviour in the times series data as an event timing to produce an event descriptor described by the event timing. Such a function is described in the section "Event descriptor production method" hereinabove, and those skilled in the art can produce an appropriate means having such a function. Examples of such a means includes, but is not limited to, for example, a computer allowing processing of a signal, and the like.

A system of interest targeted by the production system according to the present invention includes, but is not limited to: scientific systems (for example, physical systems, chemical systems, biological systems (for example, cells, tissues, organs, organisms and the like), geophysical systems, astronomic systems, and the like), social scientific systems (for example, company organisations and the like), human scientific systems (for example, history, geography and the like), economic systems (for example, stock prices, exchange and the like), machinery systems (for example, computer, apparatus and the like) and the like. In a particular embodiment, the system targeted by the analysis method is a biological system. In particular, the system targeted by the analysis method according to the present invention may be a cell.

In a particular embodiment according to the present invention which targets a cell, the analysis system according to the present invention may further comprise a support allowing the environment surrounding the cell to be maintained consistently. Such a support is described herein elsewhere in a detailed manner. Such a support was also deveploed by the present inventors, and conventionally, such a support for maintaining the consistent environment around a cell cannot be provided. In particular, in the case of gene introduction (for example, transfection) and the like, the provision of such an environment was not possible. Thus, in a particular preferable embodiment, the present invention provides a significant descriptor production system in which a time-series data of a cell can be obtained and processed.

In a preferable embodiment, monitoring means used in the analysis system according to the present invention comprises a means for outputting a signal. Such a means for outputting may make use of any apparatus well known in the art, and those skilled in the art may appropriately select such a means depending on the type of the signal used. For example, the output of the monitor is an electric signal, it may be a terminal allowing output of such an electric signal, but is not limited thereto.

In a preferable embodiment, the descriptor production means used in the present invention separately comprises means for producing time-series data and a means for perfroming calculation process to produce the descriptor. Alternatively, means for producing these time-series data and means for performing calculation process to produce the descriptor may be the same means.

In an embodiment, the descriptor production means used in the present invention comprises a computer implementing a program ordering implementation of the steps (A) through (C) as described above. Such an implementation method may be achieved by implementing a computer-readable storage medium (for example, when CD-R is used, CD-R drive is used) with a program stored thereon on a computer via means of reading the storage medium.

In a preferable embodiment, the system according to the present invention further comprises a display means for displaying the descriptor. Use of such a display means allows visual description and analysis by a user. Display means may be any type as long as it can describe the descriptor, and may make use of for example, electric wave, magnetic wave, sound, light, color, image, number and character/letter and the like. Such a display means may have a function of performing the display method selected. Preferably, the display means has a function of displaying the letter/character displaying function. In such a case, a computer display used therein may be used, but is not limited thereto. In the case of a sound, a speaker may be used.

In one embodiment, the system according to the present invention may further comprise a storage medium for storing the event descriptor on a storage medium. Such a storage meands may be selected by those skilled in the art depending on the storage medium used, and includes, for example, when CD-R is used as a storage medium, any drives allowing writing on the CD-R or a hard drive disk, and the like.

(Event descriptor)

In another aspecet, the present invention provides an event descriptor for describing a system. The present event descriptor comprises a portion having at least one characteristic behaviour as an event timing relating to at least index derived from at least one sytstem. Such an event descriptor may preferably produced by means of a method according to the present invention described herein, but is not limited thereto, and methods other ther those described herein may be used. Such a descriptor may be expressed as a string (sequence) of letters/characters, for example. In such a case, in particular, it is also called event sequence. When using a single letter of a nucleic acid notation as a letter/character string, four letters of ATGC or less number of letters may be used. The present invention also provides a storage medium or transimitting medium for storing the event descriptor according to the present invention (for example, the internet, an intranet, LAN and the like). It is understood that such a storage medium and transmitting mdium may also be within the scope of the present invention.

(Analysis system of descriptor)

In another aspect, the present invention provides an analysis system for analyzing a system using a descriptor relating to the system. The analysis system comprises: i) monitoring means for monitoring at least one index relating to the system in a time-lapse manner; ii) descriptor production means for producing an event descriptor by producing a time-series data of the system from a signal obtained from the monitoring means, and calculating the time-series data; and iii) analysis means for analyzing the descriptor, wherein the descriptor production means (A) obtains time series data of at least one index derivied from at least one system; (B) provides at least one characteristic behaviour relating to the index; and (C) extracts a portion having the characteristic behaviour in the times series data as an event timing to produce an event descriptor described by the event timing. Such a function is described herein the section "Method for producing an event descriptor", and thus those skilled in the art can appropriately produce any means having such a function. Such a means includes, but is not limited to a computer allowing processing of a signal and the like. Means for analysis may be appropriately selected depending on the description method of descriptor produced.

A system of interest targeted by the analysis system according to the present invention includes, but is not limited to: scientific systems (for example, physical systems, chemical systems, biological systems (for example, cells, tissues, organs, organisms and the like), geophysical systems, astronomic systems, and the like), social scientific systems (for example, company organisations and the like), human scientific systems (for example, history, geography and the like), economic systems (for example, stock price, exchange and the like), machinery systems (for example, computer, apparatus and the like) and the like. In a particular embodiment, the system targeted by the analysis method is a biological system. In particular, the system targeted by the analysis method according to the present invention may be a cell.

In a particular embodiment according to the present invention targeting a cell, the analysis system according to the present invention may further comprise a support allowing the environment surrounding the cell to be maintained consistently. Such a support is described herein elsewhere in a detailed manner. Such a support was also deveploed by the present inventors, and conventionally, such a support maintaining the consistent environment against a cell cannot be provided. In particular, during gene introduction (for example, transfection) and the like, the provision of such an environment was not possible. Thus, in a particular preferable embodiment, the present invention provides a significant descriptor production system wherein time-series data of a cell can be obtained and processed.

In a preferable embodiment, the monitoring means used in the analysis system of the present invention comprises a means for outputting a signal. Such an outputting means may make use of any apparatus well known in the art, and those skilled in the art can appropriately select such a means depending on the type of a signal. For example, when the output of the monitoring is an electric signal, it may be a terminal which allows output of an electric signal and the like, and is not limited thereto.

In a preferable embodiment, the descriptor production means used in the present invention separately comprises means for producing time-series data and a means for performing calculation processes to produce the descriptor. Alternatively, means for producing these time-series data and means for performing calculation processes to produce the descriptor may be the same means.

In an embodiment, the descriptor production means used in the present invention comprises a computer implementing a program ordering implementation of the steps (A) through (C) as described above. Such an implementation method may be achieved by implementing a computer-readable storage medium (for example, when CD-R is used, CD-R drive is used) with a program stored thereon on a computer via means of reading the storage medium.

In a preferable embodiment, the system according to the present invention further comprises a display means for displaying the descriptor. Use of such a display means allows visual description and analysis by a user. The display means may be of any type as long as it can describe the descriptor, and may make use of for example, electric wave, magnetic wave, sound, light, color, image, number and character/letter and the like. Such a display means may have a function performing the display method selected. Preferably, the display means has a function of displaying the letter/character displaying function. In such a case, a computer display used therein may be used, but is not limited thereto. In the case of a sound, a speaker may be used.

In one embodiment, the system according to the present invention may further comprise a storage medium for storing the event descriptor on a storage medium. Such a storage means may be selected by those skilled in the art depending on the storage medium used, and includes, for example, when CD-R is used as a storage medium, any drives allowing writing on the CD-R or a hard drive disk, and the like.

In one embodiment, the analysis means used in the present invention has a function of performing algorithm analysis of at least one event descriptor. Any embodiment described hereinabove in the section (Analysis method) may be used as such an algorithm. A method for implementing such an algorithm is also well known in the art, and for example, includes means for performing the same by implementing a program for perfoming the same on a computer.

(Event sequence analysis)

In another aspect, the present invention provides a method for analyzing at least one system using an event descriptor relating to the system. The present method comprises the steps of: (A) obtaining time-series data of at least one index derived from at least one system; (B) providing at least one characteristic behaviour; (C) extracting a portion having the characteristic behaviour as an event timing in the time-series data; and (D) analyzing the at least one event descriptor. As used herein, the obtainment of a time-series data, provision of the characteristic behaviours may make use of any embodiment described in detailed in the sectino of "Event descriptor production method" described herein. Extraction of an event timing may also make use of any embodiment described in detail in the sectino "Event descriptor production method" described herein as well. Sequence production may be performed by using any display means and describing the same as a string of the means (for example, letters/characters). Typically, it may be expressed as a string of letters/characters or arithmetic sequence.

In the present method according to the present invention, it is understood that the analysis of a sequence may be performed by means of a genetic algorithm, but it is not limited thereto, and thus any algorithm may be used.

In a preferable embodiment, the present invention provides an analysis system for analyzing a system using a sequence of event descriptors relating to at least one system. The present analysis system comprises: i) monitoring means for monitoring at least one index relating to the system in a time-lapse manner; ii) descriptor production means for producing an event descriptor by producing a time-series data of the system from a signal obtained from the monitoring means, and calculating the time-series data to produce an event descriptor describing the event timing as a sequence; and iii) analysis means for analyzing the sequence. Specifically, the descriptor production means (A) obtains time series data of at least one index derivied from at least one system; (B) provides at least one characteristic behaviour relating to the index; and (C) extracts a portion having the characteristic behaviour in the times series data as an event timing to produce an event descriptor described by the event timing. As used herein, the monitoring means and descriptor means may make use of any embodiment described hereinabove.

In the present method according to the present invention, it is understood that the analysis of a sequence may be performed by means of a genetic algorithm, but it is not limited thereto, and thus any algorigthm may be used.

(Programs)

Hereinbefore, the programs provided according to the present invention are described. The description of a program includes but is not limited to the use of any language, for exampel, C+, Perl, Basic, html, XML,. Pascal, FORTRAN, and the like. As used herein, unless otherwise specified, it is understood that program refers to a computer program.

In another aspect, the present invention provides a program for implementing a computer process for producing an event descriptor relating to at least one system. The method to be included in the program comprises the steps of: (A) obtaining time series data of at least one index derivied from at least one system; (B) providing at least one characteristic behaviour relating to the index; and (C) extracting a portion having the characteristic behaviour in the times series data as an event timing to produce an event descriptor described by the event timing. Any embodiment described in detail hereinabove in the section (Event Descriptor Production Method) may be used as such a method.

In another aspect, the present invention provides a program for implementing a computer a process for analyzing at least one system using an event descriptor relating to the system. Such a process used in the program comprises the steps of: (A) obtaining time-series data of at least one index derived from at least one system; (B) providing at least one characteristic behaviour; (C) extracting a portion having the characteristic behaviour as an event timing in the time-series data; and (D) analyzing the at least one event descriptor. Any embodiment described in detail hereinabove in the sections (Event Descriptor Production Method) and (Analysis Method) may be used as such a method.

In another aspect, the present invention provides a program for implementing in a computer a process for analyzing the relationship between a first index and a second index in a system. Such a process comprises: (A) producing a first event descriptor relating to a first index using a method according to the present invention; (B) producing a second event descriptor relating to a second index using a method according to the present invention; and (C) comparing the first and second event descriptors obtained in the steps (A) and (B). Any embodiment described in detail hereinabove in the sections (Event Descriptor Production Method) and (Analysis Method) may be used as such a method.

In another aspect the present invention provides a program for implementing in a computer a process for analyzing the relationship between a first index from a first system and a second index from a second system. Such a process employed herein comprises the steps of: (A) producing a first event descriptor relating to a first index using a method according to claim 1; (B) producing a second event descriptor relating to a second index using a method according to claim 1; and (C) comparing the first and second event descriptors obtained in the steps (A) and (B). Any embodiment described in detail hereinabove in the sections (Event Descriptor Production Method) and (Analysis Method) may be used as such a method.

In another aspect, the present invention provides a program for implementing in a computer a process for analyzing an index from a system using an event descriptor obtained using first and second characteristic behaviours. The process used in the present invention comprises the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). Any embodiment described in detail hereinabove in the sections (Event Descriptor Production Method) and (Analysis Method) may be used as such a method.

In another aspect, the present invention provides a program for implementing in a computer a process for analyzing a system using a sequence of event descriptors relating to at least one system. The present process used herein comprises the steps of: (A) obtaining time-series data of at least one index derived from at least one system; (B) providing at least one characteristic behaviour; (C) extracting a portion having the characteristic behaviour as an event timing in the time-series data, and producing an event descriptor describing the event timing as a sequence; and (D) analyzing the sequence. Any embodiment described in detail hereinabove in the sections (Event Descriptor Production Method) and (Analysis Method) may be used as such a method.

(Storage Medium)

Hereinbelow, a storage medium provided by the present inevntion is described. It should be understood that any type of storage medium may be used such as for example, any type of flexible disk, MO, CD-ROM, CD-R, DVD-ROM and the like, as long as a program can be recorded thereon.

In one aspect, the present invention provides a storage medium for storing a program for implementing in in a computer a process for producing an event descriptor relating to at least one system. The process used herein comprises the steps of: (A) obtaining time series data of at least one index derivied from at least one system; (B) providing at least one characteristic behaviour relating to the index; and (C) extracting a portion having the characteristic behaviour in the times series data as an event timing to produce an event descriptor described by the event timing. Such a process may be used in any embodiment described in detail hereinabove in (Event descriptor production method).

In another aspect, the present invention provides a storage medium for storing a program for implementing in a computer a process for analyzing at least one system using an event descriptor relating to the system. The process used herein comprises the steps of: (A) obtaining time-series data of at least one index derived from at least one system; (B) providing at least one characteristic behaviour; (C) extracting a portion having the characteristic behaviour as an event timing in the time-series data; and (D) analyzing the at least one event descriptor. Any embodiment described in detail hereinabove in the sections (Event Descriptor Production Method) and (Analysis Method) may be used as such a method.

In another aspect, the present invention provides a storage medium for storing a program for implementing in a computer a process for analyzing the relationship between a first index and a second index in a system. The process used herein comprises the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). Any embodiment described in detail hereinabove in the sections (Event Descriptor Production Method) and (Analysis Method) may be used as such a method.

In another aspect, the present invention provides a storage medium for storing a program for implementing in a computer a process for analyzing the relationship between a first index from a first system and a second index from a second system. The process used herein comprises the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). Any embodiment described in detail hereinabove in the sections (Event Descriptor Production Method) and (Analysis Method) may be used as such a method.

In another aspect, the present invention provides a storage medium for storing a program for implementing in a computer a process for analyzing an index from a system using an event descriptor obtained using first and second characteristic behaviours. The process used herein comprises the steps of: (A) producing a first event descriptor relating to a first index using a method according to the present invention; (B) producing a second event descriptor relating to a second index using a method according to the present invention; and (C) comparing the first and second event descriptors obtained in the steps (A) and (B). Any embodiment described in detail hereinabove in the sections (Event Descriptor Production Method) and (Analysis Method) may be used as such a method.

In another aspect, the present invention provides a storage medium for storing a program for implementing in a computer a process for analyzing a system using a sequence of event descriptors relating to at least one system. The process used herein comprises the steps of: (A) obtaining time-series data of at least one index derived from at least one system; (B) providing at least one characteristic behaviour; (C) extracting a portion having the characteristic behaviour as an event timing in the time-series data, and producing an event descriptor describing the event timing as a sequence; and (D) analyzing the sequence. Any embodiment described in detail hereinabove in the sections (Event Descriptor Production Method) and (Analysis Method) may be used as such a method.

(Correlation with an external agent)

In another aspect, the present invention provides a method for correlating an external factor with a response of a system such as a biological system (for example, a cell, a biological organism) or an economic system, to an external or foreign factor. The method comprises the steps of: a) exposing a system such as a system such as a biological system (for example, a cell, a biological organism) or an economic system, to an external factor on a support capable of retaining the system such as a biological system (for example, a cell, a biological organism) or an economic system, in a consistent environment; b) monitoring a the state of the system such as a biological system (for example, a cell, a biological organism) or an economic system over time to generate descriptor data for the system such as a biological system (for example, a cell, a biological organism) or an economic system; and c) correlating the external factor with the descriptor.

An external or foreign agent to be correlated in the present invention may be of any type. Such an external factor is preferably directly or indirectly applicable to a system such as a biological system (for example, a cell, a biological organism) or an economic system. A method for applying such an external factor is well known in the art, depending on the type of the external factor used. When a substance is used, the substance is dissolved into a solvent, and the resultant solution is added to a medium containing a system such as a biological system (for example, a cell, a biological organism).

The correlation method of the present invention may utilize the descriptor production method as described hereinabove.

A variety of methods can be provided for correlating a foreign agent and a descriptor in the method of correlation of the present invention. In brief, profiles obtained when a foreign agent is applied to a system such as a biological system, are patternized, and if there is little difference between the patternized descriptors, it can be inferred that the particular foreign agent has been applied to the system.

Preferably, a biological system (for example, a cell, a biological organism) may be monitored in an immobilized state to a solid support such as an array, a plate, a microtiterplate and the like. Such a method for immobilization can be conducted based on any known methodology in the art or the methods described herein.

In a preferable embodiment, the correlation method according to the present invention may comprise the step of using at least two foreign agents to obtain descriptors corresponding to each of the foreign agents. In certain embodiments, at least three, or at least four, more preferably at least ten such foreign agents may be used but the present invention is not limited thereto.

More preferably, the correlation step may further comprise dividing at least two descriptors into categories and classifying the external factors corresponding to the respective descriptors into the categories. Such categorization may be readily conducted by those skilled in the art based on the description of the present specification. Such categorization or classification allows correlation and identification of an unknown foreign agent by means of the method of the present invention.

In a preferred embodiment, when a transcription control sequence is used as a biological agent,a transcription control sequence used in the present invention may be, without limitation, a promoter, an enhancer, a silencer, other flanking sequences of structural genes in genomes, and genomic sequences other than exons. A promoter is preferable, since the transcription level can be directly measured.

In a particular embodiment, transcription control sequences used in the present invention may be constitutive promoters, specific promoters, inducible promoters, and the like. The present invention is characterized in that any type of promoter can be used. According to the method of the present invention, descriptors can be analyzed from a viewpoint of "process" or "procession". Therefore, it is possible to determine a state of a cell using any promoter or any set of promoters. Such determination cannot be achieved by conventional techniques. The present invention is highly useful since the present invention achieves what cannot be achieved by conventional techniques.

In a preferred embodiment, at least two agents (for example, biological agents such as transcription control sequences) are monitored. By observing at least two agents, at least 80% of the states of a system such as a biological system (for example, a cell, a biological organism) or an economic system can be typically identified. More preferably, at least 3 agents are monitored. By observing at least three agents, at least 90% of the states of a system such as a biological system (for example, a cell, a biological organism) or an economic system can be typically identified. In a most preferred embodiment, at least 8 agents are monitored. By observing at least 8 agents, substantially all of the states of a system such as a biological system (for example, a cell, a biological organism) or an economic system can be typically identified. Thus, although any agents are selected, substantially all of the states of a system such as a biological system (for example, a cell, a biological organism) or an economic system can be determined by selecting and monitoring a small number of agents as described above. This feature has not been conventionally expected. The method of the present invention is simpler, more precise and more accurate than conventional determination methods in which observation is made at time points and resultant data is statistically processed as heterologous groups.

Therefore, the determination method of the present invention preferably further comprises arbitrarily selecting at least one agent from a group of agents before monitoring. An important feature of the present invention is that an agent, which does not exhibit specificity when investigated from point to point, can be used.

In a preferred embodiment, such a time-lapse descriptor may be presented in real time. The real time presentation may contain a time lag to some extent if it is performed substantially in real time. A tolerable time lag is, for example, 10 seconds at maximum, and more preferably 1 second at maximum, though the tolerable time lag depends on the required level of real time (simultaneity). For example, in the case of environment measurement requiring real time identification of external factors, the tolerable time lag may be, for example, 1 sec at maximum, 0.1 sec at maximum, or the like. Alternatively, after data is stored on a storage medium at real time, descriptors may be presented corresponding to the data based on the stored data, with some time lag.

In a preferred embodiment, in the correlation step of c) of the present invention, the phase of the time-lapse descriptor may be used as information about the time-lapse descriptor in order to correlate the external factor with the time-lapse descriptor. The phase is represented by plus or minus depending on the signal intensity at a certain time. Even using such a simplified method, a system such as a biological system (for example, a cell, a biological organism) or an economic system or an external factor can be identified, thus demonstrating the precision of the method of the present invention.

Preferably, in the method of the present invention, a biological system (for example, a cell, a biological organism) is advantageously cultured on an array. This is because a number of biological systems (for example, a cell, a biological organism) can be simultaneously observed. Preferably, when a biological system (for example, a cell, a biological organism) is immobilized on a solid support such as an array, a salt may be used.

In a preferred embodiment, the step of monitoring the state of a biological system (for example, a cell, a biological organism) over time may comprise obtaining image data from the array. This is because image data can be subjected to visual inspection and a human (particularly, a person skilled in the art, such as a medical practitioner or the like) can easily examine image data with his/her eyes.

In a preferred embodiment of the present invention, the step of correlating the external factor with the time-lapse descriptor may comprise distinguishing the phases of the time-lapse descriptors. As described above, phase is a simple parameter, and its information processing is simple. Thus, a biological system (for example, a cell, a biological organism) can be well identified by such simple information processing.

In a preferred embodiment, examples of an external factor to be identified by the method of the present invention include, but are not limited to, a temperature change, a humidity change, an electromagnetic wave, a potential difference, visible light, infrared light, ultraviolet light, X-ray, a chemical substance, a pressure, a gravity change, a gas partial pressure, an osmotic pressure, and the like. These factors cannot be satisfactorily identified by conventional methods. By using the biological system (for example, a cell, a biological organism) and a determination method of the present invention which places an importance on "procession", an influence of a factor on a biological system (for example, a cell, a biological organism) can be well examined.

In a particularly preferred embodiment, an external factor to be identified by the method of the present invention may be a chemical substance. Examples of such a chemical substance include, but are not limited to, biological molecules, chemical compounds, media, and the like.

Examples of biological molecules include, but are not limited to, nucleic acids, proteins, lipids, sugars, proteolipids, lipoproteins, glycoproteins, proteoglycans, and the like. These biological molecules are known to have an influence on organisms. Unknown biological molecules are also highly likely to have an influence on organisms and are considered to be important targets for study.

Particularly preferably, hormones, cytokine, cell adhesion factors, extracellular matrices, receptor agonists, receptor antagonists, and the like, which are expected to have an influence on a biological system (for example, a cell, a biological organism), are used as biological molecules to be investigated.

(Identification of an external agent)

In another aspect, the present invention provides a method for inferring an unidentified external factor given to a system such as a biological system (for example, a cell, a biological organism), or an economic system, based on a descriptor of the system such as biological system (for example, a cell, a biological organism) or an economic system. The method comprises the steps of: a) exposing the cell to a plurality of known external factors; b) obtaining a descriptor of the system such as biological system (for example, a cell, a biological organism) or an economic system, for each known external factor by time-lapse monitoring of a state associated with at least one agent selected from the group consisting of agents derived from the system such as biological system (for example, a cell, a biological organism) or an economic system; c) correlating the known external factors with the respective time-lapse descriptors; d) exposing the system such as biological system (for example, a cell, a biological organism), or an economic system to the unidentified external factor; e) obtaining a descriptor of the unidentified external factor by time-lapse monitoring of the state of the selected agent; f) determining a descriptor corresponding to the time-lapse descriptor obtained in the step of e) from the descriptors obtained in the step of b) ; and g) determining that the unidentified external factor is the known external factor corresponding to the descriptor determined in the step of f).

In the method of the present invention, the step of exposing a system to external factors can be performed as described above herein or as illustrated in the examples described below. The step of obtaining a time-lapse descriptor can be performed as described above herein or as illustrated in the examples described below. The correlation step can be performed as described above herein or as illustrated in the examples described below. After information about all known external factors has been obtained, an unidentified external factor is similarly monitored. These pieces of information are compared to determine whether or not the unidentified external factor is a known one. If the descriptor of an unidentified factor fully matches the descriptor of a known factor, these two factors can be determined as being identical. Also, if the descriptor of an unidentified factor substantially matches the descriptor of a known factor, these two factors can be determined to be identical. Such determination depends on the information quantity and quality of the known external factor. Such determination can be easily carried out by those skilled in the art considering various elements.

(Methods for predicting a foreign agent)

The present invention may use a method for predicting an unidentified foreign agent which has given in a system such as a biological system (for example, a cell, a biological organism) or an economic system, from a descriptor of the system such as a biological system (for example, a cell, a biological organism) or an economic system. Such a method comprises the steps of: a) providing data relating to a correlation between a known foreign agent relating to at least one agent present in a system such as the biological system (for example, a cell, biological organism), an economic system or the like, and a descriptor of a system such as the biological system (for example, a cell, biological organism), economic system or the like, which corresponds to the known foreign agent; b) subjecting the system such as the biological system (for example, a cell, biological organism), economic system or the like to an unidentified foreign agent; c) monitoring the state of the system such as the biological system (for example, a cell, biological organism), economic system or the like in a time-lapse manner to obtain a description of the system such as the the biological system (for example, a cell, biological organism), economic system or the like; d) determining the descriptor corresponding to the descriptor obtained in step c) amongst the descriptors provided in step a); and e) determining that the unidentified foreign agent is the known foreign agent corresponding to the determined descriptor.

With respect to the exposure to a foreign agent, production of descriptors, correlations and the like, technologies described hereinabove and exemplified in the Examples may be used to practice the same.

(System for presenting state)

The present invention may make use of a presentation system for presenting the state of a system such as the biological system (for example, a cell, biological organism), economic system or the like. Such a presentation system comprises a) means for monitoring the state of at least one agent derived from the biological system (for example, a cell, biological organism), economic system or the like, in a time-lapse manner; and b) means for presenting the descriptor. Examples of such presentation systems are shown in Figure **32.**

A configuration of a computer or system for implementing a method for presenting the state of a system such as a biological system (for example, a cell, biological organism), economic system or the like, according to the present invention is shown in Figure **17.** Figure **17** shows an exemplary configuration of a computer **500** for executing a method for presenting the state of a system such as a biological system (for example, a cell, biological organism), economic system or the like, according to the present invention. An exemplary system configuration is presented in Figure **32.**

The computer **500** comprises an input section **501,** a CPU **502,** an output section **503,** a memory **504,** and a bus **505.** The input section **501,** the CPU **502,** the output section **503,** and the memory **504** are connected via a bus **505.** The input section **501** and the output section **503** are connected to an I/O device **506.**

An outline of a process for presenting the state of a system such as a biological system (for example, a cell, biological organism), economic system or the like, according to the present invention, which is executed by the computer **500,** will be described below.

A program for executing a process for presenting the state of a system such as a biological system (for example, a cell, biological organism), economic system or the like, according to the present invention (hereinafter referred to as a "program for presenting the state of a system such as a biological system (for example, a cell, biological organism), economic system or the like, according to the present invention") is stored in, for example, the memory **502.** Alternatively, each component of the cellular state presenting program may be stored in any type of recording medium, such as a floppy disk, MO, CD-ROM, CD-R, DVD-ROM, or the like separately or together. Alternatively, the program for presenting the state of a system such as a biological system (for example, a cell, biological organism), economic system or the like, according to the present invention may be stored in an application server. The program for presenting the state of a system such as a biological system (for example, a cell, biological organism), economic system or the like, according to the present invention, which has been stored in such a recording medium is loaded via the I/O device **506** (e.g., a disk drive, a network (e.g., the Internet)) to the memory **504** of the computer **500.** The CPU **502** executes a program for presenting the state of a system such as a biological system (for example, a cell, biological organism), economic system or the like, so that the computer **500** functions as a device for performing a method for presenting the state of a system such as a biological system (for example, a cell, biological organism), economic system or the like, according to the present invention according to the present invention.

Information about a system such as a biological system (for example, a cell, biological organism), economic system or the like is input via the input section **501** as well as descriptor data measured obtained. Known information may be input as appropriate.

The CPU **502** generates display data based on the information about descriptor data and a system such as a biological system (for example, a cell, biological organism), economic system or the like, through the input section **501,** and stores the display data into the memory **504.** Thereafter, the CPU **502** may store the information in the memory **504.** Thereafter, the output section **503** outputs the state of a system such as a biological system (for example, a cell, biological organism), economic system or the like, selected by the CPU **502** as display data. The output data is output through the I/O device **506.**

(System for determining state)

In another aspect, the present invention provides a determination system for determining the state of a system such as a biological system (for example, a cell, biological organism), economic system or the like. The determination system comprises: the steps of presenting the state of a system such as the biological system (for example, a cell, biological organism), economic system or the like. Such a presentation system comprises a) means for monitoring the state of at least one agent derived from the biological system (for example, a cell, biological organism), economic system or the like, in a time-lapse manner; and b) means for determing the state of the system such as the biological system (for example, a cell, biological organism), economic system or the like from the desrciptor. Examples of such presentation systems are shown in Figure **32.**

A configuration of a computer or system for implementing a method for determining the state of a system such as a biological system (for example, a cell, biological organism), economic system or the like, according to the present invention is shown in Figure **17.** Figure **17** shows an exemplary configuration of a computer **500** for executing a method for determining the state of a system such as a biological system (for example, a cell, biological organism), economic system or the like, according to the present invention. An exemplary system configuration is presented in Figure **32.**

The computer **500** comprises an input section **501,** a CPU **502, an** output section **503,** a memory **504,** and a bus **505.** The input section **501,** the CPU **502,** the output section **503,** and the memory **504** are connected via a bus **505.** The input section **501** and the output section **503** are connected to an I/O device **506.**

An outline of a process for determining the state of a system such as a biological system (for example, a cell, biological organism), economic system or the like, according to the present invention, which is executed by the computer **500,** will be described below.

A program for executing a process for determining the state of a system such as a biological system (for example, a cell, biological organism), economic system or the like, according to the present invention (hereinafter referred to as a "program for determining the state of a system such as a biological system (for example, a cell, biological organism), economic system or the like, according to the present invention") is stored in, for example, the memory **502.** Alternatively, each component of the cellular state determining program may be stored in any type of recording medium, such as a floppy disk, MO, CD-ROM, CD-R, DVD-ROM, or the like separately or together. Alternatively, the program for determining the state of a system such as a biological system (for example, a cell, biological organism), economic system or the like, according to the present invention may be stored in an application server. The program for determining the state of a system such as a biological system (for example, a cell, biological organism), economic system or the like, according to the present invention, which has been stored in such a recording medium is loaded via the I/O device **506** (e.g., a disk drive, a network (e.g., the Internet)) to the memory **504** of the computer **500.** The CPU **502** executes a program for determining the state of a system such as a biological system (for example, a cell, biological organism), economic system or the like, so that the computer **500** functions as a device for performing a method for determining the state of a system such as a biological system (for example, a cell, biological organism), economic system or the like, according to the present invention.

Information about a system such as a biological system (for example, a cell, biological organism), economic system or the like is input via the input section **501** as well as descriptor data obtained. Known information may be input as appropriate.

The CPU **502** generates display data based on the information on the relationship between the descriptor data and a system such as a biological system (for example, a cell, biological organism), economic system or the like, to produce determination result data through the input section **501,** and storesthe determination result data into the memory **504.** Thereafter, the CPU **502** may store the information in the memory **504.** Thereafter, the output section **503** outputs the state of a system such as a biological system (for example, a cell, biological organism), economic system or the like, selected by the CPU **502** as determination result data. The output data is output through the I/O device **506.**

In another aspect, the present invention provides a system for correlating an external factor with a response of a system such as a biological system (for example, a cell, biological organism), economic system or the like, to the external factor. The system comprises: a) means for exposing the system such as a biological system (for example, a cell, biological organism), economic system or the like to the external factor; b) means for obtaining a descriptor of the cell by time-lapse monitoring of a state associated with at least one agent selected from the group consisting of agents derived from the system such as a biological system (for example, a cell, biological organism), economic system or the like; and c) means for correlating the external factor with the descriptor. Such a system can be implemented using a computer as with the above-described systems. An exemplary system configuration is presented in Figure **32.**

(External agent inferring system)

The present invention provides a system for inferring an unidentified external factor given to a system such as a biological system (for example, a cell, biological organism), economic system or the like, based on a descriptor. The system comprising: a) means for exposing the cell to a plurality of known external factors; b) means for obtaining a descriptor of the cell for each known external factor by time-lapse monitoring of a state associated with at least one agent selected from the group consisting of agents derived from the system such as a biological system (for example, a cell, biological organism), economic system or the like; c) means for correlating the known external factors with the respective descriptors; d) means for exposing the system such as a biological system (for example, a cell, biological organism), economic system or the like, to the unidentified external factor; e) means for obtaining a descriptor of the unidentified external factor by time-lapse monitoring of the state of the selected agent; f) means for determining a descriptor corresponding to the descriptor obtained in the means of e) from the descriptors obtained in the means of b); and g), means for determining that the unidentified external factor is the known external factor corresponding to the descriptor determined in the means of f). Such a system can be implemented using a computer as with the above-described systems. An exemplary system configuration is presented in Figure **32.**

(External agent inferring system)

The present invention may make use of a system for inferring an unidentified external factor given to a system such as a biological system (for example, a cell, biological organism), economic system or the like, based on a descriptor. The present system comprises: a) means for providing data relating to a correlation relationship between known external factors and time-lapse profiles of the system such as a biological system (for example, a cell, biological organism), economic system or the like, in response to the known external factors, in relation to at least one agent selected from agents present in the cell; b) means for exposing the system such as a biological system (for example, a cell, biological organism), economic system or the like, to the unidentified external factor; c) means for obtaining a time-lapse profile of the system such as a biological system (for example, a cell, biological organism), economic system or the like, by time-lapse monitoring of a state associated with the selected agent; d) means for determining a descriptor corresponding to the descriptors obtained in the means of c) from the descriptor obtained in the means of a); and e) determining that the unidentified external factor is the known external factor corresponding to the profile determined in the means of d). Such a system can be implemented using a computer as with the above-described systems. An exemplary system configuration is presented in Figure **32.**

When the present invention is provided in the form of a system as described above, each constituent element thereof can be implemented as with the detailed or preferred embodiments of the method of the present invention. Preferred embodiments of such a system can be easily selected by those skilled in the art and can be made or carried out by those skilled in the art based on the present specification. An exemplary system configuration is presented in Figure **32.**

(State presentation program)

The present invention may make use of a computer-readable storage medium with a program stored thereon to implement a process presenting the state of a system such as a biological system (for example, a cell, biological organism), economic system or the like. As used herein, the recording medium records at least a program for executing the procedures of: a) obtaining a descriptor of the system such as a biological system (for example, a cell, biological organism), economic system or the like by time-lapse monitoring of a state associated with at least one agent selected from the group consisting of agents derived from the system such as a biological system (for example, a cell, biological organism), economic system or the like; and b) presenting the descriptor.

In another aspect, the present invention provides a computer recordable recording medium recording a program for executing a process for determining a state of a system such as a biological system (for example, a cell, biological organism), economic system or the like, to a computer. The recording medium records at least a program for executing the procedures of: a) obtaining a descriptor of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; and b) determining the state of the system such as a biological system (for example, a cell, biological organism), economic system or the like, based on the descriptor of the state.

In another aspect, the present invention provides a computer recordable recording medium recording a program for executing a process for correlating an external factor with a response of a system such as a biological system (for example, a cell, biological organism), economic system or the like, to the external factor. The recording medium records at least a program for executing the procedures of: a) exposing the system such as a biological system (for example, a cell, biological organism), economic system or the like to the external factor; b) obtaining a time-lapse profile of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; and c) correlating the external factor with the descriptor.

In another aspect, the present invention provides a computer recordable recording medium recording a program for executing a process for inferring an unidentified external factor given to a system such as a biological system (for example, a cell, biological organism), economic system or the like, based on a descriptor of the system such as a biological system (for example, a cell, biological organism), economic system or the like. The recording medium records at least a program for executing the procedures of: a) exposing the system such as a biological system (for example, a cell, biological organism), economic system or the like, to a plurality of known external factors; b) obtaining a descriptor of the system such as a biological system (for example, a cell, biological organism), economic system or the like, for each known external factor by time-lapse monitoring of a state associated with at least one agent selected from the group consisting of agents derived from the system such as a biological system (for example, a cell, biological organism), economic system or the like; c) correlating the known external factors with the respective descriptors; d) exposing the system such as a biological system (for example, a cell, biological organism), economic system or the like, to the unidentified external factor; e) obtaining a descriptor of the unidentified external factor by time-lapse monitoring of the state of the selected agent; f) determining a descriptor corresponding to the descriptor obtained in the procedure of e) from the descriptor obtained in the procedure of b); and g) determining that the unidentified external factor is the known external factor corresponding to the descriptor determined in the procedure of f).

In another aspect, the present invention provides a computer recordable recording medium recording a program for executing a process for inferring an unidentified external factor given to a system such as a biological system (for example, a cell, biological organism), economic system or the like, based on a descriptor of the system such as a biological system (for example, a cell, biological organism), economic system or the like. The recording medium records at least a program for executing the procedures of: a) providing data relating to a correlation relationship between known external factors and time-lapse profiles of the system such as a biological system (for example, a cell, biological organism), economic system or the like, in response to the known external factors, in relation to at least one agent selected from agents present in the system such as a biological system (for example, a cell, biological organism), economic system or the like; b) exposing the system such as a biological system (for example, a cell, biological organism), economic system or the like to the unidentified external factor; c) obtaining a descriptor of the system such as a biological system (for example, a cell, biological organism), economic system or the like by time-lapse monitoring of a state associated with the selected agent; d) determining a descriptor corresponding to the descriptor obtained in the procedure of c) from the descriptor obtained in the procedure of a) ; and e) determining that the unidentified external factor is the known external factor corresponding to the descriptor determined in the procedure of d).

When the present invention is provided in the form of a recording medium as described above, each constituent element thereof can be implemented as with the detailed or preferred embodiments of the method of the present invention. Preferred embodiments of such a recording medium can be easily selected by those skilled in the art and can be made or carried out by those skilled in the art based on the present specification.

In another aspect, the present invention provides a program for executing a process for presenting a state of a system such as a biological system (for example, a cell, biological organism), economic system or the like to a computer. The program executes the procedures of: a) obtaining a descriptor of the system such as a biological system (for example, a cell, biological organism), economic system or the like, by time-lapse monitoring of a state associated with at least one agent selected from the group consisting of biological agents derived from the system such as a biological system (for example, a cell, biological organism), economic system or the like; and b) presenting the descriptor.

In another aspect, the present invention provides a program for executing a process for determining a state of a system such as a biological system (for example, a cell, biological organism), economic system or the like in a computer. The program executes the procedures of: a) obtaining a descriptor of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; and b) determining the state of the system such as a biological system (for example, a cell, biological organism), economic system or the like, based on the descriptor of the state.

In another aspect, the present invention provides a program for executing a process for correlating an external factor with a response of a system such as a biological system (for example, a cell, biological organism), economic system or the like,to the external factor. The program executes the procedures of: a) exposing the system such as a biological system (for example, a cell, biological organism), economic system or the like, to the external factor; b) obtaining a descriptor of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; and c) correlating the external factor with the descriptor. Such a technology for implementing these procedues are well knonw in the art, and those skilled in the art may produce an appropriate program depending on the purpose thereof.

In another aspect, the present invention provides a program for executing a process for inferring an unidentified external factor given to a system such as a biological system (for example, a cell, biological organism), economic system or the like, based on a descriptor. The program executes the procedures of: a) exposing the system such as a biological system (for example, a cell, biological organism), economic system or the like, to a plurality of known external factors; b) obtaining a descriptor of the system such as a biological system (for example, a cell, biological organism), economic system or the like, for each known external factor by time-lapse monitoring of a state associated with at least one agent selected from the group consisting of agents derived from the cell; c) correlating the known external factors with the respective descriptors; d) exposing the system such as a biological system (for example, a cell, biological organism), economic system or the like, to the unidentified external factor; e) obtaining a descriptor of the unidentified external factor by time-lapse monitoring of the state of the selected agent; f) determining a profile corresponding to the descriptor obtained in the procedure of e) from the descriptors obtained in the procedure of b) ; and g) determining that the unidentified external factor is the known external factor corresponding to the descriptor determined in the procedure of f).

In another aspect, the present invention provides a program for executing a process for inferring an unidentified external factor given to a system such as a biological system (for example, a cell, biological organism), economic system or the like, based on a descriptor. The program executes the procedures of: a) providing data relating to a correlation relationship between known external factors and descriptors of the system such as a biological system (for example, a cell, biological organism), economic system or the like, in response to the known external factors, in relation to at least one agent selected from agents present in the system such as a biological system (for example, a cell, biological organism), economic system or the like; b) exposing the system such as a biological system (for example, a cell, biological organism), economic system or the like, to the unidentified external factor; c) obtaining a descriptor of the system such as a biological system (for example, a cell, biological organism), economic system or the like, by time-lapse monitoring of a state associated with the selected agent; d) determining a descriptor corresponding to the descriptor obtained in the procedure of c) from the descriptors obtained in the procedure of a); and e) determining that the unidentified external factor is the known external factor corresponding to the descriptor determined in the procedure of d).

When the present invention is provided in the form of a program as described above, each constituent element thereof can be implemented as with the detailed or preferred embodiments of the method of the present invention. Preferred embodiments of such a program can be easily selected by those skilled in the art and can be made or carried out by those skilled in the art based on the present specification. Description formats of such a program are well known to those skilled in the art and include, for example, the C+ language, and the like.

In another aspect, the present invention provides a method and system for diagnosing a subject. The diagnosis method comprises the steps of: a) obtaining a descriptor of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; b) determining the state of the system such as a biological system (for example, a cell, biological organism), economic system or the like, based on the descriptor of the state; and c) determining a condition, disorder or disease of a subject based on the state of the system such as a biological system (for example, a cell, biological organism), economic system or the like. The diagnosis method is provided in the form of a system, the system of the present invention comprises: a) means for obtaining a descriptor of the system such as a biological system (for example, a cell, biological organism), economic system or the like, by time-lapse monitoring of a state associated with at least one agent selected from the group consisting of agents derived from the system such as a biological system (for example, a cell, biological organism), economic system or the like; b) means for determining the state of the system such as a biological system (for example, a cell, biological organism), economic system or the like, based on the descriptor of the state; and c) means for determining a condition, disorder or disease of a subject based on the state of the system such as a biological system (for example, a cell, biological organism), economic system or the like. The present invention is applicable to tailor-made diagnoses and therapies, such as drug resistance, selection of appropriate anticancer agents, selection of appropriate transplant system such as a biological system (for example, a cell, biological organism), economic system, and the like. Preferably, the diagnosis method of the present invention may be provided as a therapeutic or preventative method comprising the step of treating a subject with a therapy or preventative method selected based on the result of diagnosis. In another preferred embodiment, the diagnosis system of the present invention may be provided as a therapeutic or preventative system comprising means for treating a subject with a therapy or preventative method, selected based on the result of diagnosis. An exemplary system configuration is shown in Figure **32.**

A configuration of a computer or system for implementing the diagnosis method and system of the present invention is shown in Figure **17.** Figure **17** shows an exemplary configuration of a computer **500** for executing the cellular state determining method of the present invention. An exemplary system configuration is shown in Figure **32.**

The computer **500** comprises an input section **501,** a CPU **502**, an output section **503**, a memory **504,** and a bus **505.** The input section **501,** the CPU **502,** the output section **503,** and the memory **504** are connected via a bus **505.** The input section **501** and the output section **503** are connected to an I/O device **506.**

An outline of a correlation process, which is executed by the computer **500,** will be described below.

A program for executing the correlation method and/or selection of treatment or preventative method (hereinafter referred to as a "correlation program" and a "selection program", respectively) is stored in, for example, the memory **502.** Alternatively, the correlation program and the selection program may be stored in any type of recording medium, such as a floppy disk, MO, CD-ROM, CD-R, DVD-ROM, or the like, separately or together. Alternatively, the programs may be stored in an application server. The correlation program and the selection program stored in such a recording medium are loaded via the I/O device **506** (e.g., a disk drive, a network (e.g., the Internet)) to the memory **504** of the computer **500.** The CPU **502** executes the correlation program and the selection program, so that the computer **500** functions as a device for performing the correlation method and the selection method of the present invention.

The result of analysis of a descriptor (e.g., phase, etc.) and information about a system such as a biological system (for example, a cell, biological organism), economic system or the like are input via the input section **501.** Secondary information about a condition, disorder or disease to be correlated with a descriptor and information about treatment and/or preventative methods may be input as required.

The CPU **502** correlates information about a desrciptor with a state of a system such as a biological system (for example, a cell, biological organism), economic system or the likeor a condition, disorder or disease of a subject and a preventative or therapeutic method as required, based on the information input through the input section **501,** and stores correlation data into the memory **504.** Thereafter, the CPU **502** may store the information in the memory **504.** Thereafter, the output section **503** outputs information about a state of a system such as a biological system (for example, a cell, biological organism), economic system or the like or a condition, disorder or disease of a subject and a preventative or therapeutic method as required, which has been selected by the CPU **502** as diagnostic information. The output data is output through the I/O device **506.**

(Generation of data)

In one embodiment, the present invention provides a method for generating descriptor data of information of a biological system (for example, a cell, biological organism). The method comprises the steps of: a) providing and fixing the biological system (for example, a cell, biological organism) to a support; and b) monitoring a biological agent or an aggregation of biological agents on or within the biological system (for example, a cell, biological organism) over time to generate data on the descriptor of the biological system (for example, a cell, biological organism). In this aspect, the present invention is characterized in that the biological system (for example, a cell, biological organism) is fixed to substantially the same site of the support so that information can be continuously (e.g., in a time-lapse manner, etc.) obtained from the same biological system (for example, a cell, biological organism). Thereby, it is possible to monitor a biological agent and an aggregation of biological agents over time. The time-lapse monitoring makes it possible to obtain a descriptor of a biological system (for example, a cell, biological organism) and construct a digital biological system (for example, a cell, biological organism). To fix a biological system (for example, a cell, biological organism) to a support, a fixing agent, such as a salt or the like, may be used for the support in the present invention. A combination of a salt, a complex of a positively charged substance and a negatively charged substance, and a biological system (for example, a cell, biological organism) may fix the biological system (for example, a cell, biological organism) to the support. Any salt may be used in the present invention. Examples of such a salt include, but are not limited to, calcium chloride, sodium hydrogen phosphate, sodium hydrogen carbonate, sodium pyruvate, HEPES, sodium chloride, potassium chloride, magnesium sulfide, iron nitrate, amino acids, vitamins, and the like. Examples of the above-described combination of a positively charged substance and a negatively charged substance include, but are not limited to, complexes of a negatively charged substance selected from the group consisting of DNA, RNA, PNA, a polypeptide, a chemical compound, and a complex thereof and a positively charged substance selected from the group consisting of a cationic polymer, a cationic lipid, a cationic polyamino acid, and a complex thereof. In a preferred embodiment of the present invention, a biological agent of interest may be a nucleic acid molecule or a molecule derived from such a nucleic acid molecule. This is because most nucleic acid molecules carry genetic information, from which information of the biological system (for example, a cell, biological organism) can be obtained.

(Data)

In another aspect, the present invention relates to data obtained by a method comprising the steps of: a) providing and fixing the system such as a biological system (for example, a cell, biological organism), economic system or the like to a support; and b) monitoring a agent or an aggregation of agents on or within the system such as a biological system (for example, a cell, biological organism), economic system or the like, over time to generate data of the descriptor of the system such as a biological system (for example, a cell, biological organism), economic system or the like. Such data is obtained by a method which is not conventionally available, and is thus novel. Therefore, the present invention provides a recording medium storing such data.

(Method for generating descriptor data of information of a plurality of biological systems in a consistent environment)

In another aspect, the present invention relates to a method for generating descriptor of information about a plurality of biological systems (for example, a cell, biological organism)in a consistent environment. The method comprises the steps of: a) providing a plurality of biological systems (for example, a cell, biological organism) on a support which can maintain a consistent environment; and b) monitoring a biological agent or an aggregation of biological agents on or within the biological systems (for example, a cell, biological organism) over time to generate descriptor for the biological systems (for example, a cell, biological organism). In this aspect, the present invention is characterized in that descriptor of information for a plurality of biological systems (for example, a cell, biological organism) in a consistent environment can be obtained. Techniques for providing such an environment is also within the scope of the present invention. To provide a consistent environment for a plurality of biological systems (for example, a cell, biological organism), a fixing agent, such as a salt or the like, may be used for the support in the present invention. A combination of a salt, a complex of a positively charged substance and a negatively charged substance, and cells may fix the biological systems (for example, a cell, biological organism) to the support. Any salt may be used in the present invention. Examples of such a salt include, but are not limited to, calcium chloride, sodium hydrogen phosphate, sodium hydrogen carbonate, sodium pyruvate, HEPES, sodium chloride, potassium chloride, magnesium sulfide, iron nitrate, amino acids, vitamins, and the like. Examples of the above-described combination of a positively charged substance and a negatively charged substance include, but are not limited to, complexes of a negatively charged substance selected from the group consisting of DNA, RNA, PNA, a polypeptide, a chemical compound, and a complex thereof and a positively charged substance selected from the group consisting of a cationic polymer, a cationic lipid, a cationic polyamino acid and a complex thereof. In a preferred embodiment of the present invention, a biological agent of interest may be a nucleic acid molecule or a molecule derived from such a nucleic acid molecule. This is because most nucleic acid molecules carry genetic information, from which information of the biological system (for example, a cell, biological organism) can be obtained.

In a preferred embodiment, an actin-like acting substance is preferably provided to the biological systems (for example, a cell, biological organism) in the method of the present invention. The actin-like acting substance acts on actin within the biological systems (for example, a cell, biological organism) to deform the internal cytoskeleton to facilitate introduction of an external factor into the biological systems (for example, a cell, biological organism). The presence of such an actin-like acting substance makes it possible to investigate an influence of an external factor of interest on the biological systems (for example, a cell, biological organism).

In one embodiment, a biological agent targeted by the present invention is at least one factor selected from the group consisting of nucleic acids, proteins, sugar chains, lipids, low molecular weight molecules, and composite molecules thereof.

In a preferred embodiment, biological systems (for example, a cell, biological organism) targeted by the present invention are preferably cultured for a certain period of time without stimulation before monitoring. This procedure is performed for the purpose of synchronizing the target biological systems (for example, a cell, biological organism). The period of time required for synchronization is, for example, advantageously at least one day, more preferably at least two days, even more preferably at least 3 days, and still even more preferably at least 5 days. It should be noted that as the period of time for culture is increased, the necessity of maintaining the culture conditions increases. In the synchronization procedure, the same medium is preferably supplied to biological systems (for example, a cell, biological organism). Therefore, the culture medium is preferably consistent or at least changed in a consistent manner. To achieve this, a means for causing convection in the medium may be preferably provided and used.

In a more preferred embodiment, a biological agent provided to a biological system (for example, a cell, biological organism) in the present invention may comprise a nucleic acid molecule encoding a gene. The nucleic acid molecule encoding a gene is preferably transfected into a biological system (for example, a cell, biological organism). Preferably, such a biological agent may be provided along with a transfection reagent (gene introduction reagent). More preferably, the nucleic acid molecule encoding a gene may be provided to a biological system (for example, a cell, biological organism) along with a gene introduction reagent and an actin-like acting substance. In this case, the biological system (for example, a cell, biological organism) is preferably provided with a complex of a salt, a positively charged substance,

and a negatively charged substance (in this case, a nucleic acid molecule and a gene introduction reagent). Thus, the biological system (for example, a cell, biological organism) and the target molecule are fixed on a support. In addition, this technique makes it possible to allow separate biological agents (e.g., nucleic acid molecules) to be separately introduced into biological systems (for example, a cell, biological organism) without a partition. As substantially no partition is used, a plurality of biological systems (for example, a cell, biological organism) can be monitored in substantially a consistent environment. Further, different biological agents can be introduced into a biological system (for example, a cell, biological organism), thereby making it possible to obtain a descriptor of a state of the cell affected by the biological agents. Such a descriptor can be stored as data. Such data may be stored in a certain standard format, and therefore, can be reproduced and compared. Thus, the present invention has an effect which is not achieved by conventional biological assays. Such data, once obtained and stored in such a standard format, can be extracted and used for various purposes and a number of times. For example, researchers can perform "virtual experiments" to conduct various analyses under the same conditions while taking into consideration differences in a substantially infinite number of parameters. In addition, since virtual experiments and the results thereof are stored in a raw data format, undergraduate and graduate students, who otherwise spend most of their school life doing laboratory work, can receive education in data analysis in the true sense. The above-described cellular descriptor data can be easily standardized, thereby making it possible to do research based on data which may have been obtained by experiments under the same conditions over the world. Such data may be distributed in a standardized form. Such a standardized form may be readable to typical computers (e.g., computers having a commonly available OS, such as Windows, Mac, UNIX, LINUX, or the like). Data produced in the present invention may include generated descriptor data, information about experimental conditions used in data generation, information about biological systems (for example, a cell, biological organism), information about environments, and the like.

In a preferred embodiment, a descriptor targeted by the present invention may include a descriptor of gene expression, a descriptor of an apoptotic signal, a descriptor of a stress signal, a descriptor of the localization of a molecule (preferably, the molecule is labeled with a fluorescent, phosphorescent, or radioactive substance, or a combination thereof), a descriptor of changes in morphology of a biological system (for example, a cell, biological organism), a descriptor of a promoter, a descriptor of a promoter dependent on a specific pharmaceutical agent (e.g., antibiotics, ligands, toxins, nutrients, vitamins, hormones, cytokines, etc.), a descriptor of an intermolecular interaction, and the like. In an embodiment in which the present invention targets a descriptor of a promoter dependent on a specific pharmaceutical agent, it is preferable that the present invention may further comprise administering the specific pharmaceutical agent.

In a preferred embodiment, the present invention may further comprise providing an external stimulus to the biological system (for example, a cell, biological organism). Such an external stimulus may or may not be a biological agent. The external factor may be any factor and includes, without limitation, substances or other elements (e.g., energy, such as ionizing radiation, radiation, light, acoustic waves, and the like).

In one embodiment, an external factor used in the present invention may be RNAi. RNAi can be used to substantially suppress an arbitrary gene. It is possible to produce RNAi for all existing genes and investigate the effect of RNAi on the genes. RNAi can be created by techniques well known in the art.

In another embodiment, an external factor of the present invention may comprise a chemical substance which does not exist in organisms. By providing a biological system (for example, a cell, biological organism) with such a chemical substance which does not exist in organisms, it is possible to collect a variety of information. Once collected, such data can be reused. Therefore, assuming that a chemical substance which does not exist in organisms is not substantially available, if data can be obtained once for such a chemical substance in accordance with the present invention, research can continue without worrying about the availability of such a chemical substance.

In one embodiment, an external factor targeted by the present invention may comprise a ligand to a receptor of a biological system (for example, a cell, biological organism). By analyzing a ligand, it is possible to study various signal transduction pathways. Therefore, in such a case, a descriptor obtained according to the present invention may be a descriptor of receptor-ligand interactions.

In a preferred embodiment of the present invention, a descriptor of morphology of a biological system (for example, a cell, biological organism) may be obtained. In this case, a method of the present invention may further comprise applying a stimulus to a biological system (for example, a cell, biological organism) which may be selected from the group consisting of overexpression of a gene, underexpression of a gene, knock down of a gene, addition of an external factor, and a change in an environment.

In a preferred embodiment, a descriptor obtained according to the present invention may be a descriptor of interactions between molecules present within a biological system (for example, a cell, biological organism). Such descriptors relating to an intermolecular interaction includes, but is not limited to, descriptors of interaction between molecules present in a signal transduction pathway, interaction between a receptor and a ligand, interaction between a transcription factor and a transcription factor sequence, and the like.

In another preferred embodiment, a descriptor obtained according to the present invention may be a descriptor of interaction between molecules present in a biological system (for example, a cell, biological organism). In this case, a method of the present invention may further comprise observing a biological system (for example, a cell, biological organism) using a technique selected from the group consisting of a two-hybrid method, FRET, and BRET. The two-hybrid method detects intermolecular interaction within a biological system (for example, a cell, biological organism). Specifically, this technique is described in, for example, Protein-Protein Interactions, A MOLECULAR CLONING MANUAL, Edited by Erica Golemis, Cold Spring Habor Laboratory Press, Cold Spring Harbor, New York (this document also describes FRET). FRET is a technique for detecting inter- or intra-molecular resonance energy shift as a fluorescent wavelength, and is described in, for example, Protein-Protein Interactions (supra); and Miyawaki A., Visualization of the spatial and temporal dynamics of intracellular signaling, Dev. Cell, 2003 Mar; 4(3):295-305. BRET is an intermolecular interaction assay system and is described, for example, Boute N., The use of resonance energy transfer in high-throughput screening: BRET versus FRET, Trends Pharmacol Sci., 2002 Aug; 23(8):351-4.

In a preferred embodiment, biological systems (for example, a cell, biological organism) targeted by the present invention are preferably arranged on a support in a pattern of an array. In this case, preferably, a plurality of biological systems (for example, a cell, biological organism) targeted by the present invention may be spaced at intervals of 10 cm at maximum, more preferably 1 cm at maximum, even more preferably 1 mm at maximum, and most preferably 0.1 mm at maximum. The biological systems (for example, a cell, biological organism) need to be spaced at minimum intervals. Such intervals may be preferably set so that substantially no interaction occurs.

In one embodiment, a descriptor obtained according to the present invention may or may not be obtained in real time. A real time descriptor may be advantageous. When simultaneity is important, it is important to obtain a descriptor in real time. Alternatively, when a descriptor is intended to be stored, the descriptor is not necessarily obtained in real time.

In an additional embodiment, the present invention further comprises fixing a biological system (for example, a cell, biological organism) to a solid phase support. In this case, the biological systems (for example, a cell, biological organism) is fixed to the solid phase support along with a salt, a complex, an actin-like acting substance, or the like.

In one embodiment, data generated according to the present invention may contain information about a descriptor. In a preferred embodiment, data generated according to the present invention may contain information about conditions for monitoring, information about a state of a biological system (for example, a cell, biological organism), information about an external factor, information about an environment, and the like.

In a preferred embodiment, at least two biological agents may be preferably monitored in the present invention, more preferably at least 3 biological agents, and even more preferably at least 8 biological agents. Alternatively, all biological agents in a certain specific category (e.g., all olfactory receptors, all gustatory receptors, etc.) may be preferably monitored.

Alternatively, in another preferred embodiment, the present invention may further comprise arbitrarily selecting the above-described biological agents.

In a preferred embodiment, a biological system (for example, a cell, biological organism) targeted by the present invention may be selected from the group consisting of biological systems (for example, a cell, biological organism) derived from stem cells and somatic cells.

In one embodiment, a support used in the present invention is preferably a solid phase support. This is because cells are easily fixed to such a support. Such a solid phase support may be made of any material known in the art. The support may be in the form of a substrate.

In one embodiment of the present invention, the above-described biological agent may be a nucleic acid and the above-described biological system (for example, a cell, biological organism) may be transfected with the nucleic acid. By transfecting the biological system (for example, a cell, biological organism) with the nucleic acid, an influence of the nucleic acid on the cell can be collected in real time or in a standardized storable format into data or a descriptor. This cannot be achieved by conventional techniques. In a preferred embodiment, transfection may be performed in a solid phase or in a liquid phase. More preferably, transfection may be advantageously performed in a solid phase. This is because data collection and standardization or normalization can be more easily carried out.

In a preferred embodiment of the present invention, a descriptor may be subjected to a process selected from the group consisting of phase comparison, calculation of a difference from a control descriptor, signal processing, and multivariate analysis. Data processed in such a manner may fall within the scope of the present invention.

(Presentation of descriptors of a biological system in a consistent environment)

In another aspect, the present invention provides a method for presenting descriptor of information about a plurality of cells in a consistent environment. The method comprises the steps of: a) providing a plurality of biological systems (for example, a cell, biological organism) on a support capable of retaining the biological systems (for example, a cell, biological organism) in a consistent environment; b) monitoring a biological agent or an aggregation of biological agents on or within the biological systems (for example, a cell, biological organism) over time to generate descriptor data for the biological systems (for example, a cell, biological organism); and c) presenting the data.

The above-described support capable of retaining a plurality of biological systems (for example, a cell, biological organism) in a consistent environment can be achieved as described elsewhere herein. The step of generating data can be performed as described elsewhere herein. The step of presenting data can be performed as described elsewhere herein. Examples of a method of performing such presentation include, but are not limited to, techniques of using various sensory means, such as visual means, auditory means, olfactory means, tactile means, gustatory means, and the like. Preferably, a visually presentation means may be used. Such visual means include, without limitation, a computer display and the like.

Preferably, in the presentation method of the present invention, presentation may be performed in real time. Alternatively, stored data may be stored and presentation may be delayed. When presentation should be performed in real time, data signals may be transferred directly to, for example, a display.

(Method for determining a state of a biological system in a consistent environment)

In another aspect, the present invention provides a method for determining states of biological systems (for example, a cell, biological organism) in a consistent environment. The method comprises the steps of: a) providing a plurality of biological systems (for example, a cell, biological organism) on a support capable of retaining the cells in a consistent environment; b) monitoring a biological agent or an aggregation of biological agents on or within the biological systems (for example, a cell, biological organism) over time to generate descriptor data for the cells; and c) determining the states of the biological systems (for example, a cell, biological organism) based on the data.

The above-described support capable of retaining a plurality of biological systems (for example, a cell, biological organism) in a consistent environment can be achieved as described elsewhere herein. The step of generating data can be performed as described elsewhere herein. The step of determining the states of the biological systems (for example, a cell, biological organism) may be performed by correlating the generated data with information about the biological systems (for example, a cell, biological organism), or comparing the generated data with standard data. In this case, the data may be statistically processed.

Therefore, in a certain embodiment, the present invention may further comprise correlating a descriptor obtained according to the present invention with a state of a biological system (for example, a cell, biological organism) before obtaining the time-lapse descriptor. To perform determination smoothly, the biological systems (for example, a cell, biological organism) targeted by the present invention may advantageously include biological systems (for example, a cell, biological organism) whose states are known. It is possible to store data of biological systems (for example, a cell, biological organism) whose states are known, determination can thus be quickly performed by comparing data between the known biological system (for example, a cell, biological organism) and unknown biological systems (for example, a cell, biological organism).

During determination, at least two biological agents are preferably present. In this case, the plurality of biological agents may belong to heterologous categories (e.g., proteins and nucleic acids, etc.) or homologous categories.

Preferably, the present invention may further comprise arbitrarily selecting a biological agent. Any biological agent can be selected and used to characterize a state of a biological system (for example, a cell, biological organism) to some extent, and in some cases, identification is possible. Thus, the present invention has an effect which cannot be expected from conventional techniques.

In the determination method of the present invention, data may be preferably generated in real time. When data is generated in real time, an unknown substance or state of an unknown biological system (for example, a cell, biological organism) may be determined in real time.

In the determination method of the present invention, examples of a state of a target biological system (for example, a cell, biological organism) include, but are not limited to, differentiated states, undifferentiated states, responses of a biological system (for example, a cell, biological organism) to external factors, cycles of a biological system (for example, a cell, biological organism), growth states, and the like.

A cell targeted by the present invention may be either a biological system (for example, a cell, biological organism) derived from a stem cell or a somatic cell. Any biological system (for example, a cell, biological organism) derived from a somatic cell may be used. A biological system (for example, a cell, biological organism) may be selected by those skilled in the art, depending on the purpose of use of the biological system (for example, a cell, biological organism).

A solid phase support used in the determination method of the present invention may comprise a substrate. In the present invention, such a substrate can be used as a part of a computer system, so that determination can be automated. An exemplary configuration of such a system is shown in Figure 32.

In a preferred embodiment, in the determination method of the present invention, the biological agent may be a nucleic acid molecule, and the biological system (for example, a cell, biological organism) is transfected with the nucleic acid molecule. Transfection may be performed on a solid phase support using any material, but preferably a gene introduction agent, more preferably a salt, an actin-like acting substance, or the like. Transfection may be performed in solid phase or in liquid phase, and preferably in solid phase.

In a determination method of the present invention, a target biological agent may be capable of binding to another biological agent. By investigating a biological agent having such a property, a network mechanism in a biological system (for example, a cell, biological organism) may be elucidated.
In a determination method of the present invention, the determination step may comprise a mathematical process selected from the group consisting of comparison of phases of descriptors, collection of differences from a control descriptor, signal processing, and multivariate analysis. Such processing techniques are well known in the art and described in detail herein.

(Correlation amongst a biological system and an external agent in a consistent environment)

In another aspect, the present invention provides a method for correlating an external factor with a response of a biological system (for example, a cell, biological organism) to the external factor. The method comprises the steps of: a) exposing a plurality of biological systems (for example, a cell, biological organism) to an external factor on a support capable of retaining the biological systems (for example, a cell, biological organism) in a consistent environment; b) monitoring a biological agent or an aggregation of biological agents on or within the biological systems (for example, a cell, biological organism) over time to generate descriptor data for the biological systems (for example, a cell, biological organism) ; and c) correlating the external factor with the descriptor. Exposure of the biological systems (for example, a cell, biological organism) to the external factor may be achieved by placing the biological systems (for example, a cell, biological organism) and the external factor into an environment in which the biological systems (for example, a cell, biological organism) are contacted with the external factor. For example, when the biological systems (for example, a cell, biological organism) are fixed on the support, the external factor is added to the support to achieve exposure. Techniques for generating and correlating data are also well known in the art, and may be used singly or in combination. Preferably, statistical processes are performed to generate statistically significant data and information.
In a preferred embodiment, in the correlation method of the present invention, the biological systems (for example, a cell, biological organism) may be fixed on the support. Since the biological systems (for example, a cell, biological organism) are fixed, data can be easily standardized, so that data can be significantly efficiently processed.
In a preferred embodiment, a correlation method of the present invention may further comprise using at least two external factors to obtain a descriptor for each external factor. Techniques for obtaining such a descriptor are well described herein.
More preferably, the correlation step may further comprise dividing at least two descriptors into categories and classifying the external factors corresponding to the respective descriptors into the categories. By categorization, data can be processed in a more standardized manner.
In a preferred embodiment, a descriptor obtained by the present invention may be presented in real time. When data is intended to be stored, data may not be particularly presented in real time.
In a preferred embodiment, a biological system (for example, a cell, biological organism) used in the present invention may be cultured on an array. In such a case, therefore, the biological system (for example, a cell, biological organism) is preferably covered with medium. Any medium which is commonly used for biological systems (for example, a cell, biological organism) may be used.
In a preferred embodiment of the present invention, the step of monitoring a descriptor may comprise obtaining image data from the array. Particularly, when a descriptor contains visual information (e.g., emission of fluorescence due to gene expression), the descriptor can be obtained by capturing image data.
In a correlation method of the present invention, the step of correlating an external factor with a descriptor may comprise distinguishing between phases of the descriptor. Distinguishing phases of the descriptor can be achieved only after the present invention provides time-lapse descriptors obtained in a consistent environment.
An external factor targeted by the present invention may be selected from the group consisting of a temperature change, a humidity change, an electromagnetic wave, a potential difference, visible light, infrared light, ultraviolet light, X-rays, a chemical substance, a pressure, a gravity change, a gas partial pressure, and an osmotic pressure. Preferably, the chemical substance may be a biological molecule, a chemical compound, or a medium. Examples of such a biological molecule include, but are not limited to, nucleic acid molecules, proteins, lipids, sugars, proteolipids, lipoproteins, glycoproteins, proteoglycans, and the like. Such a biological molecule may also be, for example, a hormone, a cytokine, a cell adhesion factor, an extracellular matrix, or the like. Alternatively, the chemical substance may be either a receptor agonist or antagonist.
In another aspect, the present invention relates to a method for identifying an unidentified external factor given to a cell from a descriptor of the biological system (for example, a cell, biological organism). The method comprises the steps of: a) exposing a biological system (for example, a cell, biological organism) to a plurality of known external factors on a support capable of retaining the cell in a consistent environment; b) monitoring a biological agent or an aggregation of biological agents on or within the biological system (for example, a cell, biological organism) over time to generate a descriptor of the biological system (for example, a cell, biological organism) to each of the known external factors and to generate descriptor data for the biological system (for example, a cell, biological organism); c) correlating each of the known external factors with each of the descriptors; d) exposing the biological system (for example, a cell, biological organism) to an unidentified external factor; e) monitoring a biological agent or an aggregation of biological agents on or within the biological system (for example, a cell, biological organism) exposed to the external factors over time to obtain a descriptor of the biological system (for example, a cell, biological organism) with respect to the unidentified external factor; f) determining, from the descriptors obtained in step b), a descriptor corresponding to the descriptor obtained the step of e); and g) determining that the unidentified external factor is the known external factor corresponding to the descriptor determined in the step of f). Techniques for exposure to external factors, data generation, correlation, exposure to unidentified external factors, and the like are described elsewhere herein and can be selected as appropriate depending on the purpose by those skilled in the art taking such descriptions into consideration.

In another aspect, the present invention provides a method for identifying an unidentified external factor given to a cell from a descriptor of the biological system (for example, a cell, biological organism). The method comprises the steps of: a) providing data relating to a correlation relationship between known external factors and descriptors of the biological system (for example, a cell, biological organism) in response to the known external factors, in relation to a biological agent or an aggregation of biological agents on or within the biological system (for example, a cell, biological organism); b) exposing the biological system (for example, a cell, biological organism) to the unidentified external factor; c) monitoring the biological agent or the aggregation of the biological agents on or within the biological system (for example, a cell, biological organism) to obtain a descriptor of the biological system (for example, a cell, biological organism) ; d) determining, from the descriptors provided in the step of a), a descriptor corresponding to the descriptor obtained in the step of c) ; and e) determining that the unidentified external factor is the known external factor corresponding to the descriptor determined in the step of d). Techniques for exposure to external factors, data generation, correlation, exposure to unidentified external factors, and the like are described elsewhere herein and can be selected as appropriate depending on the purpose by those skilled in the art taking such descriptions into consideration.

In another aspect, the present invention provides a method for obtaining a descriptor relating to information for a plurality of biological systems (for example, a cell, biological organism) in a consistent environment. The method comprises the steps of: a) providing a plurality of biological systems (for example, a cell, biological organism) on a support capable of retaining the biological systems (for example, a cell, biological organism) in a consistent environment; and b) monitoring a biological agent or an aggregation of biological agents on or within the cell over time to generate a descriptor of the biological systems (for example, a cell, biological organism). Techniques for exposure to external factors, data generation, correlation, exposure to unidentified external factors, and the like are described elsewhere herein and can be selected as appropriate, depending on the purpose by those skilled in the art taking such descriptions into consideration.

In another aspect, the present invention relates to a recording medium in which data generated by a method for generating descriptor data of a biological system (for example, a cell, biological organism) of the present invention is stored. Data may be stored in any format. Any recording medium may be used. Examples of such a recording medium include, but are not limited to, CD-ROMs, flexible disks, CD-Rs, CD-RWs, MOs, mini disks, DVD-ROMs, DVD-Rs, memory sticks, hard disks, and the like. The present invention also relates to a transmission medium in which data generated by a method for generating descriptor data of biological systems (for example, a cell, biological organism) of the present invention is stored. Examples of such a transmission medium include, but are not limited to, networks, such as intranets, the Internet, and the like.

A recording medium or transmission medium of the present invention may further contain data relating to at least one piece of information selected from the group consisting of information about conditions for the monitoring step, information about the descriptor, information about the state of a biological system (for example, a cell, biological organism), and information about the biological agent. Data relating to such information may be stored while being linked to one another. Preferably, the data may be advantageously standardized. Standardized data can be distributed on general distribution pathways. The above-described linkage may be constructed for each biological system (for example, a cell, biological organism) or for each biological agent, or for both.

In another aspect, the present invention relates to data generated by a method for generating descriptor data of a biological system (for example, a cell, biological organism) of the present invention. Such data cannot be generated by conventional techniques and is thus novel.

In another aspect, the present invention provides a system for generating descriptor data of information for a plurality of biological systems (for example, a cell, biological organism) in a consistent environment. The system comprises: a) a support capable of retaining a plurality of biological systems (for example, a cell, biological organism) in a consistent environment; b) means for monitoring a biological factor or an aggregation of biological factors on or within the biological systems (for example, a cell, biological organism) over time; and c) means for generating descriptor data for the biological systems (for example, a cell, biological organism) from a signal obtained from the monitoring means. The support capable of retaining biological systems (for example, a cell, biological organism) in a consistent environment can be made by those skilled in the art using a technique first provided by the present invention. Such a technique is attributed to the finding that biological systems (for example, a cell, biological organism) are fixed and arrayed without a partition. Examples of the monitoring means include, but are not limited to, microscopes (e.g., optical microscopes, fluorescence microscopes, phase-contrast microscopes, etc.), electron microscopes, scanners, naked eyes, infrared cameras, confocal/nonconfocal microscopes, CCD cameras, and the like. An exemplary configuration of such a system is shown in Figure **32.**

In a system of the present invention, the system may not necessarily contain biological systems (for example, a cell, biological organism) from the start, but preferably may contain biological systems (for example, a cell, biological organism) which are advantageously fixed on a support. In such a case, fixation is preferably standardized. In addition, the biological systems (for example, a cell, biological organism) are fixed and spaced, for example, without limitation, at intervals of 1 mm or the like.

In a preferred embodiment, at least one substance selected from the group consisting of salts and actin-like acting substances may be preferably adhered to the support. By adhering cells to the support with a salt or an actin-like acting substance, or preferably with both, fixation of the biological systems (for example, a cell, biological organism) and/or introduction of a substance into the biological systems (for example, a cell, biological organism) can be enhanced.

Examples of the monitoring means used in the system of the present invention include, but are not limited to, optical microscopes, fluorescence microscopes, phase-contrast microscopes, reading devices using a laser source, means using surface plasmon resonance (SPR) imaging, electric signals, chemical or biochemical markers singly or in combination, radiation, confocal microscopes, nonconfocal microscopes, differential interference microscopes, stereoscopic microscopes, video monitors, infrared cameras, and the like. Preferably, a scanner (e.g., a scanner for scanning a surface of a substrate using a white light source or laser) may be used. The reason a scanner is preferable is that fluorescence can efficiently transmit excited energy and microscopic technology can be easily applied. Further, measurement can be advantageously performed without significant damage to biological systems (for example, a cell, biological organism). An exemplary configuration of such a system is shown in Figure **32.**

In another aspect, the present invention provides a system for presenting a descriptor of information for a plurality of biological systems (for example, a cell, biological organism) in a consistent environment. The system comprises: a) a support capable of retaining a plurality of biological systems (for example, a cell, biological organism) in a consistent environment; b) means for monitoring a biological factor or an aggregation of biological factors on or within the biological systems (for example, a cell, biological organism) over time; c) means for generating descriptor data for the biological systems (for example, a cell, biological organism) from a signal obtained from the monitoring means; and d) means for presenting the data. The support, the monitoring means, and the data generating means can be made as described elsewhere herein. The means for presenting data can be achieved by techniques well known in the art. Examples of such a data presenting means include, but are not limited to, computer displays, loudspeakers, and the like. An exemplary configuration of such a system is shown in Figure **32.**

A presentation system of the present invention may further comprise a plurality of biological systems (for example, a cell, biological organism), in which the biological systems (for example, a cell, biological organism) are preferably fixed to the support. In such a case, at least one substance selected from the group consisting of salts and actin-like acting substances may be preferably adhered to the support. By adhering cells to the support with a salt or an actin-like acting substance, or preferably with both, fixation of the biological systems (for example, a cell, biological organism) and/or introduction of a substance into the biological systems (for example, a cell, biological organism) can be enhanced.

Any monitoring means may be used. Examples of the monitoring means include, but are not limited to, optical microscopes; fluorescence microscopes; phase microscopes; reading devices using a laser source; means using surface plasmon resonance (SPR)imaging, electric signals, chemical or biochemical markers singly or in combination; and the like.

Any data presenting means may be used, including, without limitation, displays, loudspeakers, and the like.

In another aspect, the present invention provides a system for determining a state of a biological system (for example, a cell, biological organism). The system comprises: a) a support capable of retaining a plurality of biological systems (for example, a cell, biological organism) in a consistent environment; b) means for monitoring a biological factor or an aggregation of biological factors on or within the biological systems (for example, a cell, biological organism) over time; c) means for generating data from a signal obtained by the monitoring means; and d) means for extrapolating the state of the biological system (for example, a cell, biological organism) from the data. The support, the monitoring means, and the data generating means can be made by those skilled in the art as described elsewhere herein. The means for extrapolating a state of a biological system (for example, a cell, biological organism) from data may be produced and used by techniques well known in the art. For example, measured data can be compared with standard data for known biological systems (for example, a cell, biological organism) to achieve extrapolation. A device storing a program for such extrapolation or a computer capable of executing such a program may be used as the extrapolation means. An exemplary configuration of such a system is shown in Figure **32.**

In another aspect, the present invention provides a system for correlating an external factor with the responses of biological systems (for example, a cell, biological organism) to the external factor. The system comprises: a) a support capable of retaining a plurality of cells in a consistent environment; b) means for exposing the biological system (for example, a cell, biological organism) to the external factor; c) means for monitoring a biological factor or an aggregation of biological factors on or within the biological systems (for example, a cell, biological organism) over time; d) generating descriptor data for the biological systems (for example, a cell, biological organism) from a signal from the monitoring means; and e) means for correlating the external factor with the descriptor. The support, the monitoring means, and the data generating means can be made by those skilled in the art as described elsewhere herein. The means for exposing the biological systems (for example, a cell, biological organism) to the external factor can be designed and carried out as appropriate by those skilled in the art depending on the properties of the external factor. The correlation means can employ a recording medium storing a program for correlation or a computer capable of executing such a program. Preferably, a system of the present invention comprises a plurality of biological systems (for example, a cell, biological organism). An exemplary configuration of such a system is shown in Figure **32.**

In another aspect, the present invention provides a system for identifying an unidentified external factor given to a biological system (for example, a cell, biological organism) based on a descriptor of the cell. The system comprises: a) a support capable of retaining a plurality of biological systems (for example, a cell, biological organism) in a consistent environment; b) means for exposing the biological system (for example, a cell, biological organism) to one or more known external factors; c) means for monitoring a biological factor or an aggregation of biological factors on or within the biological systems (for example, a cell, biological organism) over time; d) means for obtaining a descriptor of the biological system (for example, a cell, biological organism) with respect to each of the known external factors to generate descriptor data for the cell; e) means for correlating each of the known external factors with each descriptor; f) means for exposing the biological system (for example, a cell, biological organism) to the unidentified external factor; g) means for comparing the descriptors of the known external factors obtained by the means of d) with the descriptor of the unidentified external factor to determine a descriptor of the unidentified external factor from the descriptors of the known external factors, wherein the determined unidentified external factor is the known external factor corresponding to the determined descriptor. The support, the exposure means, the monitoring means, the data generating means, and the correlation means, and the other exposure means can be made and carried out as appropriate by those skilled in the art as described elsewhere herein. The means for determining a corresponding descriptor can also be made and carried out by utilizing a recording medium storing a program capable of executing such a determination process and a computer capable of executing such a program. Preferably, a system of the present invention comprises a plurality of cells. An exemplary configuration of such a system is shown in Figure **32.**

In another aspect, the present invention provides a system for identifying an unidentified external factor given to a biological system (for example, a cell, biological organism) based on a descriptor of the biological system (for example, a cell, biological organism). The system comprises: a) a recording medium storing providing data relating to a correlation relationship between known external factors and descriptors of the biological system (for example, a cell, biological organism) in response to the known external factors, in relation to a biological factor or an aggregation of biological factors on or within the biological system (for example, a cell, biological organism) ; b) means for exposing the biological system (for example, a cell, biological organism) to the unidentified external factor; c) a support capable of retaining a plurality of biological systems (for example, a cell, biological organism) in a consistent environment ; d) means for monitoring a biological factor or an aggregation of biological factors on or within the biological systems (for example, a cell, biological organism) over time; e) means for obtaining a descriptor of the biological system (for example, a cell, biological organism) from a signal obtained by the monitoring means; f) means for determining, from the descriptors stored in the recording medium of a), a descriptor corresponding to the descriptor obtained with respect to the unidentified external factor, wherein the determined unidentified external factor is the known external factor corresponding to the determined descriptor. The support, the exposure means, the monitoring means, the data generating means, and the correlation means, and the other exposure means can be made and carried out as appropriate by those skilled in the art as described elsewhere herein. The means for determining a corresponding descriptor can also be made and carried out by utilizing a recording medium storing a program capable of executing such a determination process and a computer capable of executing such a program. Preferably, a system of the present invention comprises a plurality of biological systems (for example, a cell, biological organism). An exemplary configuration of such a system is shown in Figure **32.**

In another aspect, the present invention relates to a support capable of maintaining a consistent environment for a plurality of biological systems (for example, a cell, biological organism). Such a support was first provided by the present invention. By utilizing such a support, a plurality of biological systems (for example, a cell, biological organism) can be analyzed in a consistent environment.

Preferably, biological systems (for example, a cell, biological organism) are arranged on a support in the form of an array. This is because standardized analysis can be achieved thereby. In this case, the support may preferably comprise a salt or an actin-like acting substance. More preferably, the support may advantageously comprise a complex of a positively charged substance and a negatively charged substance. This is because biological systems (for example, a cell, biological organism) can be easily fixed to the support using such a complex. Actin-like acting substances are preferable when the interior of biological systems (for example, a cell, biological organism) is analyzed, since the actin-like acting substances increase the efficiency of introduction of external factors into biological systems (for example, a cell, biological organism). Therefore, in a preferred embodiment of the present invention, the support may comprise a salt and an actin-like acting substance, and more preferably may comprise a complex of a positively charged substance and a negatively charged substance.

A support of the present invention is characterized in that biological systems (for example, a cell, biological organism) may be provided and spaced at intervals of 1 mm. In the case of such intervals, it is not conventionally possible to provide an environment without a partition. Therefore, the present invention has a remarkable effect, as well as practicability, applicability and utility.

In a preferred embodiment, a support of the present invention may comprise a biological system (for example, a cell, biological organism) fixed thereto. In a more preferred embodiment, a support of the present invention may comprise a biological factor fixed thereto.

In a preferred embodiment, at least two biological factors may be fixed to the support. Such biological factors may be factors selected from the group consisting of nucleic acid molecules, proteins, sugars, lipids, metabolites, low molecular weight molecules, and complexes thereof, and factors containing physical elements and/or temporal elements.

In a more preferred embodiment, a biological system (for example, a cell, biological organism) and a biological factor may be fixed to a support of the present invention in a mixed manner. The biological factor and the biological system (for example, a cell, biological organism) may be provided so that they can interact with each other. Such interaction may vary depending on the biological factor. According to the properties of the biological factor, those skilled in the art can understand how the biological factor interacts with the biological system (for example, a cell, biological organism) and where the biological factor is positioned so as to interact with the biological system (for example, a cell, biological organism).

In a preferred embodiment, a salt, a complex of a positively charged substance and a negatively charged substance, and an actin-like acting substance are fixed along with a biological system (for example, a cell, biological organism) and a biological factor to a support of the present invention.

In a more preferred embodiment, a salt, a complex of a positively charged substance and a negatively charged substance, and an actin-like acting substance are fixed along with a biological system (for example, a cell, biological organism) and a biological factor to a support of the present invention in the form of an array. With such a structure, a chip of a biological system (for example, a cell, biological organism) capable of generating the descriptor data of a biological system (for example, a cell, biological organism) can be provided. The support has a structure in which a salt, a complex of a positively charged substance and a negatively charged substance, and an actin-like acting substance are fixed, along with a biological system (for example, a cell, biological organism) and a biological factor in the form of an array. Such a support is also called a "transfection array".

Examples of a salt used in the support of the present invention include, but are not limited to, calcium chloride, sodium hydrogen phosphate, sodium hydrogen carbonate, sodium pyruvate, HEPES, sodium chloride, potassium chloride, magnesium sulfide, iron nitrate, amino acids, vitamins, and the like. A preferable salt is, for example, without limitation, sodium chloride or the like.

Examples of a gene introduction agent used in the support of the present invention include, but are not limited to, cationic polymers, cationic lipids, polyamine-based reagents, polyimine-based reagents, calcium phosphate, oligofectamin, and oligofectors and the like. Preferably the gene introduction reagents used may be preferably, but are not limited to, lipofectamines, oligofectamines and oligofectors.

Examples of an actin-like acting substance used in the support of the present invention include, but are not limited to, fibronectin, laminin, vitronectin, and the like. A preferable actin-like acting substance is, for example, without limitation, fibronectin.

Examples of a nucleic acid molecule used in the support of the present invention include, but are not limited to, nucleic acid molecules comprising transcription control sequences (e.g., promoters, enhancers, etc.), gene coding sequences, genomic sequences containing nontranslation regions, nucleic acid sequences encoded by the genome of a host (a fluorescent protein gene, E. coli/yeast self-replication origins, a GAL4 domain, etc.), and the like. Preferable nucleic acid molecules include, but are not limited to, transcription control sequences (e.g., promoters, enhancers, etc.), gene coding sequences, genomic sequences containing nontranslation regions, and the like.

Examples of a biological system (for example, a cell, biological organism) used in the support of the present invention include, but are not limited to, a biological system (for example, a cell, biological organism) derived from stem cells, established biological systems (for example, a cell, a biological organism), a primary cultured biological system (for example, a cell, biological organism), an insect biological system (for example, a cell, biological organism), a bacterial biological system (for example, a cell, biological organism), and the like. Preferable biological systems (for example, a cell, biological organism) include, but are not limited to, stem cells, established cell lines, primary culture cells, and the like.

Examples of a material for a support of the present invention include, but are not limited to, glass, silica, plastics, and the like. Preferable materials include, but are not limited to, the above-described materials with a coating.

In another aspect, the present invention provides a method for producing a support comprising a plurality of biological systems (for example, a cell, biological organism) fixed thereto and capable of maintaining a consistent environment for the biological systems (for example, a cell, biological organism). The method comprises the steps of: A) providing the support; and B) fixing the biological systems (for example, a cell, biological organism) via a salt and a complex of a positively charged substance and a negatively charged substance onto the support. The step of providing a support may be achieved by obtaining a commercially available support or molding a support material. A support material may be prepared by mixing starting materials for the material as required. The fixing step can be carried out by using techniques known in the art. Examples of such fixing techniques include, but are not limited to, an ink jet printing technique, a pin array technique, a stamping technique, and the like. These techniques are well known and can be performed as appropriate by those skilled in the art.

In a preferred embodiment, the fixing step in the present invention may comprise fixing a mixture of the salt, the complex of a gene introduction agent and an actin-like acting substance (positively charged substances) and a nucleic acid molecule (a negatively charged substance), and the biological system (for example, a cell, biological organism) in the form of an array. Such a fixing step may be achieved by printing techniques.

In another aspect, the present invention provides a device for producing a support comprising a plurality of biological systems (for example, a cell, biological organism) fixed thereto and capable of maintaining a consistent environment for the biological systems (for example, a cell, biological organism). The device comprises: A) means for providing the support; and B) means for fixing the biological systems (for example, a cell, biological organism) via a salt and a complex of a positively charged substance and a negatively charged substance onto the support. The support may be obtained using means which can perform the above-described methods. Examples of such means include, but are not limited to, a support molding means, a material formulating means (e.g., a mixing means), and the like. The molding means can employ techniques well known in the art. The fixing means may comprise a printing means. As such a printing means, commercially available ink jet printers can be used.

It should be understood that the above desrcibed embodiments in a consisten environment has been described as an example in which the event sequence production method and an analysis method using the same can be sufficiently applied, but, the present invention is not limited to such embodiments.

All patents, published patent applications and publications cited herein are incorporated by reference as if set forth fully herein.

The preferred embodiments of the present invention have been heretofore described for a better understanding of the present invention. Hereinafter, the present invention will be described by way of examples. The above described detailed description and the following examples are provided by means of illustrative purposes and not for the purpose of limitation. Accordingly, the scope of the present invention is not limited by means of embodiments or examples specifically described herein, and the scope of the present invention is not limited except as by the appended claims. The examples described below are provided only for illustrative purposes with respect to the examples using a cell, a stock price, and a brain wave. According to the examples below, it will be understood that those skilled in the art can select cells, supports, biological agents, salts, positively charged substances, negatively charged substances, actin-like acting substances, and the like, as appropriate, and can make or carry out the present invention. Alternatively, it is understood that stock prices may also be used, as long as similar mathematical processing can be conduced, and similar event descriptors may be used and produced in other fields.

## EXAMPLES

Hereinafter, the present invention will be described in greater detail by way of examples, though the present invention is not limited to the examples below. Reagents, supports, and the like are commercially available from Sigma (St. Louis, USA), Wako Pure Chemical Industries (Osaka, Japan), Matsunami Glass (Kishiwada, Japan) unless otherwise specified.

### (Example 1: Reagents)

Formulations below were prepared in Example 1.

As candidates for an actin-like acting substance, various extracellular matrix proteins and variants or fragments thereof were prepared in Example 1, as listed below. Fibronectin and the like were commercially available. Fragments and variants were obtained by genetic engineering techniques:

1) fibronectin (SEQ ID NO.: 11);
2) fibronectin 29 kDa fragment;
3) fibronectin 43 kDa fragment;
4) fibronectin 72 kDa fragment;

5) fibronectin variant (SEQ ID NO.: 11, an alanine at postion 152 was substituted with leucine);
6) ProNectin F (Sanyo Chemical Industries, Kyoto, Japan);
7) ProNectin L (Sanyo Chemical Industries);
8) ProNectin Plus (Sanyo Chemical Industries);
9) laminin (SEQ ID NO.: 6);
10) RGD peptide (tripeptide);
11) RGD-containing 30kDa peptide;
12) 5 amino acids of laminin (IKVAV); and
13) gelatin.

Plasmids were prepared as DNA for transfection. Plasmids, pEGFP-N1 and pDsRed2-N1 (both from BD Biosciences, Clontech, CA, USA) were used. In these plasmids, gene expression was under the control of cytomegalovirus (CMV) promoter. The plasmid DNA was amplified in E. coli (XL1 blue, Stratgene, TX, USA) and the amplified plasmid DNA was used as a complex partner. The DNA was dissolved in distilled water free from DNase and RNase.

The following transfection reagents were used: Effectene Transfection Reagent (cat. no. 301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France), and ExGen 500 (R0511, Fermentas Inc., MD). These transfection reagents were added to the above-described DNA and actin-like acting substances in advance, or complexes thereof with the DNA were produced in advance.

The thus-obtained solutions were used in assays using the transfection arrays described below.

(Example 2: Transfection array - Demonstration using mesenchymal stem cells)

In Example 2, an improvement in the transfection efficiency in solid phase was observed. The protocol used in Example 2 will be described below.

(Protocol)

The final concentration of DNA was adjusted to 1 μg/μL. An actin-like acting substance was stored as a stock having a concentration of 10 μg/μL, in $ddH_2O$. All dilutions were made using PBS, $ddH_2O$, or Dulbecco' s MEM. A series of dilutions, for example, 0.2 μg/μL, 0.27 μg/μL, 0.4 μg/μL, 0.53 μg/μL, 0.6 μg/μL, 0.8 μg/μL, 1.0 μg/μL, 1.07 μg/μL, 1.33 μg/μL, and the like, were formulated.

Transfection reagents were used in accordance with instructions provided by each manufacturer.

Plasmid DNA was removed from a glycerol stock and amplified in 100 mL L-amp overnight. Qiaprep Miniprep or Qiagen Plasmid Purification Maxi was used to purify DNA in accordance with a standard protocol provided by the manufacturer.

In Example 2, the following 5 cells were used to confirm an effect: human mesenchymal stem cell (hMSCs, PT-2501, Cambrex BioScience Walkersville, Inc., MD); human embryonic renal cell (HEK293, RCB1637, RIKEN Cell Bank, JPN); NIH3T3-3 cell (RCB0150, RIKEN Cell Bank, JPN); HeLa cell (RCB0007, RIKEN Cell Bank,JPN); and HepG2 (RCB1648, RIKEN Cell Bank, JPN) . These cells were cultured in DMEM/10% IFS containing L-glutamine and penicillin/streptomycin.

(Dilution and DNA spots)

Transfection reagents and DNA were mixed to form a DNA-transfection reagent complex. Formation of the complex requires a certain period of time. Therefore, the mixture was spotted onto a solid phase support (e.g., a poly-L-lysine slide) using an arrayer. In Example 2, as a solid phase support, an APS slide, a MAS slide, and an uncoated slide were used, as well as a poly-L-lysine slide. These slides are available from Matsunami Glass (Kishiwada, Japan), or the like.

For complex formation and spot fixation, the slides were dried overnight in a vacuum dryer. Drying was performed for a duration in the range of 2 hours to 1 week.

Although the actin-like acting substance might be used during complex formation, it was also used immediately before spotting in the present Example.

(Formulation of mixed solution and application to solid phase supports)

300 μL of DNA concentrated buffer (EC buffer) + 16 μL of an enhancer were mixed in an Eppendorf tube. The mixture was mixed with a Vortex, followed by incubation for 5 minutes. 50 μL of a transfection reagent (Effectene, etc.) was added to the mixture, followed by mixing by pipetting. To apply a transfection reagent, an annular wax barrier was formed around the spots on the slide. 366 μL of the mixture was added to the spot region surrounded by the wax, followed by incubation at room temperature for 10 to 20 minutes. Thereby, the fixation to the support was manually achieved.

(Distribution of cells)

Next, a protocol for adding cells will be described. Cells were distributed for transfection. The distribution was typically performed by reduced-pressure suction in a hood. A slide was placed on a dish, and a solution containing cells was added to the dish for transfection. The cells were distributed as follows.

The growing cells were adjusted to a concentration of $10^7$ cells/25 mL. The cells were plated on the slide in a 100×100×15 mm squared Petri dish or a 100 mm (radius) × 15 mm circular dish. Transfection was conducted for about 40 hours. This period of time corresponded to about 2 cell cycles. The slide was treated for immunofluorescence.

(Evaluation of gene introduction)

Gene introduction was evaluated by detection using, for example, immunofluorescence, fluorescence microscope examination, laser scanning, radioactive labels, and sensitive films, or emulsion.

When an expressed protein to be visualized is a fluorescent protein, such a protein can be observed with a fluorescence microscope and a photograph thereof can be taken. For large-sized expression arrays, slides may be scanned using a laser scanner for storage of data. If an expressed protein can be detected using fluorescent antibodies, an immunofluorescence protocol can be successively performed. If detection is based on radioactivity, the slide may be adhered as described above, and autoradiography using film or emulsion can be performed to detect radioactivity.

(Laser scanning and Quantification of fluorescence intensity)

To quantify transfection efficiency, the present inventors use a DNA microarray scanner (GeneTAC UC4x4, Genomic Solutions Inc., MI). Total fluorescence intensity (arbitrary unit) was measured, and thereafter, fluorescence intensity per unit surface area was calculated.

(Cross-sectional observation by confocal scanning microscope)

Cells were seeded on tissue culture dishes at a final concentration of $1\times10^5$ cells/well and cultured in appropriate medium (Human Mesenchymal Cell Basal Medium (MSCGM BulletKit PT-3001, Cambrex BioScience Walkersville, Inc., MD). After fixation of the cell layer with 4% paraformaldehyde solution, SYTO and Texas Red-X phalloidin (Molecular Probes Inc., OR, USA) was added to the cell layer for observation of nuclei and F-actin. The samples emitting light due to gene products and the stained samples were observed with a confocal laser microscope (LSM510: Carl ZeissCo., Ltd., pin holesize=Ch1=123 μm, Ch2=108 μm, image interval = 0.4) to obtain cross sectional views.

(Results)

Figure **1** shows the results of experiments in which various actin-like acting substances and HEK293 cells were used where gelatin was used as a control.

As can be seen from the results, whereas transfection was not very successful in a system using gelatin, transfection took place to a significant level in systems using fibronectin, ProNectin (ProNectin F, ProNectin L, ProNectin Plus) which is a variant of fibronectin, and laminin. Therefore, it was demonstrated that these molecules significantly increased transfection efficiency. Use of the RGD peptide alone exhibited substantially no effect.

Figures **2** and **3** show transfection efficiency when fibronectin fragments were used. Figure **4** shows the summary of the results. 29 kDa and 72 kDa fragments exhibited a significant level of transfection activity, while a 43 kDa fragment had activity but its level was low. Therefore, it was suggested that an amino acid sequence contained in the 29 kDa fragment played a role in an increase in transfection efficiency. Substantially no contamination was found in the case of the 29 kDa fragment, while contamination was observed in the case of the other two fragments (43 kDa and 72 kDa). Therefore, only the 29 kDa domain may be preferably used as an actin-like acting substance. When only the RGD peptide was used, increased transfection efficiency was not exhibited. The 29-kDa peptide therefore exhibited activity with respect to enhancing transfection efficiency. Such a system with an additional 6 amino acids of laminin (higher molecular weight) exhibited transfection activity. Therefore, these peptide sequences may also play an important role in increased transfection efficiency, without limitation. In such a case, a molecular weight of at least 5 kDa, preferably at least 10 kDa, and more preferably at least 15 kDa may be required for an increase in transfection efficiency.

Next, Figure **5** shows the result of studies on the transfection efficiency of cells. In Figure **5**, HEK293 cells, HeLa cells, and 3T3 cells, which were conventionally transfectable, and HepG2 cells and mesenchymal stem cells (MSC) which were conventionally believed to be substantially impossible to transfect, were used to show the effect of the transfection method of the present invention. The vertical axis represents the intensity of GFP.

In Figure **5,** the transfection method of the present invention using a solid phase support was compared with a conventional liquid phase transfection method. The conventional liquid phase transfection method was conducted in accordance with a protocol recommended by the kit manufacturer.

As can be seen from Figure **5,** transfection efficiency comparable to HeLa and 3T3 was achieved in HepG2 cells and mesenchymal stem cells (MSC) which were conventionally believed to be substantially impossible to transfect, as well as HEK293 cells, HeLa cells, and 3T3 cells, which were conventionally transfectable. Such an effect was not achieved by conventional transfection systems. The present invention was the first to provide a system which can increase transfection efficiency for substantially all cells and can provide practicable transfection to all cells. By using solid phase conditions, cross contamination was significantly reduced. Therefore, it was demonstrated that the present invention using a solid phase support is appropriate for production of an integrated bioarray.

Next, Figure **6** shows the results of transfection when various plates were used. As can be seen from the results of Figure **6,** when coating was provided, contamination was reduced as compared with when coating was not provided and transfection efficiency was increased.

Next, Figure **7** shows the results of transfection where the concentration of fibronectin was 0, 0.27, 0.53, 0.8, 1.07, and 1.33 (μg/μL for each). In Figure **7,** slides coated with PLL (poly-L-lysine), APS and uncoated slides are shown.

As can be seen from the results of Figure **7,** transfection efficiency was increased with an increase in fibronectin concentration. Note that in the case of PLL coating and the absence of coating, the transfection efficiency reached a plateau at a fibronectin concentration of more than 0.53 μg/μL. In the case of APS, it was found that the effect was further increased at a fibronectin concentration of more than of 1.07 μg/μL.

Next, Figure **8** shows photographs indicating cell adhesion profiles in the presence or absence of fibronectin. Figure **9** shows cross-sectional photographs. It was revealed that the morphology of adherent cells was significantly different (Figure **8).** The full extension of cells was found for the initial 3 hours of culture in the presence of fibronectin, while extension was limited in the absence of fibronectin (Figure **9).** Considering the behavior of filaments (Figure **9)** and the results of the time-lapse observation, it was considered that an actin-like acting substance, such as fibronectin, attached to a solid phase support had an influence on the shape and orientation of actin filaments, and the efficiency of introduction of a substance into a cell, such as transfection efficiency or the like, was thus increased. Specifically, actin filaments quickly change their location in the presence of fibronectin, and disappear from the cytoplasmic space under the nucleus as the cell extends. It is considered that actin depletion in the perinuclear space, which is induced by an actin-like acting substance, such as fibronectin, allows the transport of a target substance, such as DNA or the like, into cells or nuclei. Though not wishing to be bound by any theory, the reason is considered to be that the viscosity of cytoplasm is reduced and positively charged DNA particles are prevented from being trapped by negatively charged actin filaments. Additionally, it is considered that the surface area of the nucleus is significantly increased in the presence of fibronectin (Figure **10),** possibly facilitating the transfer of a target substance, such as DNA or the like, into nuclei.

(Example 3: Application to bioarrays)

Next, larger-scale experiments were conducted to determine whether or not the above-described effect was demonstrated when arrays were used.

(Experimental protocols)

(Cell sources, culture media, and culture conditions)

In this example, five different cell lines were used: human mesenchymal stem cells (hMSCs, PT-2501, Cambrex BioScience Walkersville, Inc., MD), human embryonic kidney cell HEK293 (RCB1637, RIKEN Cell Bank, JPN), NIH3T3-3 (RCB0150, RIKEN Cell Bank, JPN), HeLa (RCB0007, RIKEN Cell Bank, JPN), and HepG2 (RCB1648, RIKEN Cell Bank, JPN). In the case of human MSCs, cells were maintained in commercialized Human Mesenchymal Cell Basal Medium (MSCGM BulletKit PT-3001, Cambrex BioScience Walkersville, Inc., MD). In case of HEK293, NIH3T3-3, HeLa and HepG2, cells were maintained in Dulbecco's Modified Eagle's Medium (DMEM, high glucose 4.5 g/L with L-Glutamine and sodium pyruvate; 14246-25, Nakalai Tesque, JPN) with 10% fetal bovine serum (FBS, 29-167-54, Lot No. 2025F, Dainippon Pharmaceutical CO., LTD., JPN). All cells were cultivated in a controlled incubator at 37°C in 5% $CO_2$. In experiments involving hMSCs, we used hMSCs of less than five passages, in order to avoid phenotypic changes.

(Plasmids and Transfection reagents)

To evaluate the efficiency of transfection, the pEGFP-N1 and pDsRed2-N1 vectors (cat. no. 6085-1, 6973-1, BD Biosciences Clontech, CA) were used. Both genes' expressions were under the control of cytomegalovirus (CMV) promoter. Transfected cells continuously expressed EGFP or DsRed2, respectively. Plasmid DNAs were amplified using Escherichia coli, XL1-blue strain (200249, Stratagene, TX), and purified by EndoFree Plasmid Kit (EndoFree Plasmid Maxi Kit 12362, QIAGEN, CA). In all cases, plasmid DNA was dissolved in DNase and RNase free water. Transfection reagents were obtained as below: Effectene Transfection Reagent (cat. no.301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France), and ExGen 500 (R0511, Fermentas Inc., MD).

(Solid-Phase Transfection Array (SPTA) production)

The detail of protocols for 'reverse transfection' are described in the web site, 'Reverse Transfection Homepage' (http://staffa.wi.mit.edu/sabatini_public/reverse_trans fection.htm) or J. Ziauddin, D. M. Sabatini, Nature, 411, 2001, 107; and R.W. Zu, S.N. Bailey, D.M. Sabatini, Trends in Cell Biology, Vol. 12, No. 10, 485. In our solid phase transfection (SPTAmethod), three types of glass slides were studied (silanized glass slides; APS slides, and poly-L-lysine coated glass slides; PLL slides, and MAS coated slides; Matsunami Glass, JPN) with a 48 square pattern (3 mm $\times$ 3 mm) separated by a hydrophobic fluoride resin coating.

(Plasmid DNA printing solution preparation)

Two different ways to produce a SPTA were developed. The main differences reside in the preparation of the plasmid DNA printing solution.

(Method A)

In the case of using Effectene Transfection Reagent, the printing solution contained plasmid DNA and cell adhesion molecules (bovine plasma fibronectin (cat. no. 16042-41, Nakalai Tesque, JPN), dissolved in ultra-pure water at a concentration of 4 mg/mL). The above solution was applied on the surface of the slide using an inkjet printer (synQUAD™, Cartesian Technologies, Inc., CA) or manually, using a 0.5 to 10 $\mu$L tip. This printed slide was dried over 15 minutes at room temperature in a safety-cabinet. Before transfection, total Effectene reagent was gently poured on the DNA-printed glass slide and incubated for 15 minutes at room temperature. The excess Effectene solution was removed from the glass slide using a vacuum aspirator and dried at room temperature for 15 minutes in a safety-cabinet. The DNA-printed glass slide obtained was set in the bottom of a 100-mm culture dish and approximately 25 mL of cell suspension (2 to $4\times10^4$ cells/mL) was gently poured into the dish. Then, the dish was transferred to the incubator at 37°C in 5% $CO_2$ and incubated for 2 to 3 days.

(Method B)

In case of other transfection reagents (TransFast™, Tfx™-20, SuperFect, PolyFect, LipofectAMINE 2000, JetPEI ($\times$4) conc., orExGen), plasmid DNA, fibronectin, and the transfection reagent were mixed homogeneously in a 1.5-mL micro-tube according to the ratios indicated in the manufacturer's instructions and incubated at room temperature for 15 minutes before printing on a chip. The printing solution was applied onto the surface of the glass-slide using an inkjet printer or a 0.5- to 10-$\mu$L tip. The printed glass-slide was completely dried at room temperature over 10 minutes in a safety-cabinet. The printed glass-slide was placed in the bottom of a 100-mm culture dish and approximately 3 mL of cell suspension (2 to $4\times10^4$ cells/mL) was added and incubated at room temperature over 15 minutes in a safety-cabinet. After incubation, fresh medium was poured gently into the dish. Then, the dish was transferred to an incubator at 37°C in 5% $CO_2$ and incubated for 2 to 3 days. After incubation, using fluorescence microscopy (IX-71, Olympus PROMARKETING, INC., JPN), we observed the transfectants, based on their expression of enhanced fluorescent proteins (EFP, EGFP and DsRed2). Phase contrast images were taken with the same microscope. In both protocols, cells were fixed using a paraformaldehyde (PFA) fixation method (4% PFA in PBS, treatment time was 10 minutes at room temperature).

(Laser scanning and fluorescence intensity quantification)

In order to quantify the transfection efficiency, we used a DNA micro-array scanner (GeneTAC UC4×4, Genomic Solutions Inc., MI). The total fluorescence intensity (arbitrary units) was measured, and thereafter, the fluorescence intensity per surface area was calculated.

(Results)

(Fibronectin-supported localized transfection)

A transfection array chip was constructed as shown in Figure **11**. The transfection array chip was constructed by microprinting a cell cultivation medium solution containing fibronectin and DNA/transfection reagent onto a poly L lysine (PLL) coated glass slide.

Various cells were used for this example. The cells were cultivated under typical cell cultivation conditions. As they adhered to the glass slide, the cells efficiently incorporated and expressed the genes corresponding to the DNA printed at a given position on the array. As compared to conventional transfection methods (e.g., cationic lipid or cationic polymer-mediated transfection), the efficiency of transfection using the method of the present invention was high in all the cells tested. Importantly, it was found that tissue stem cells, such as HepG2 and hMSC, which were conventionally believed to resist transfection, were efficiently transfected. hMSC was transfected at an efficiency 40 or more times higher than that of conventional techniques. In addition, high spatial localization, which is required for high-density arrays, was achieved (low cross contamination between adjacent spots on the array). This was confirmed by production of a checkered pattern array of EGFP and Ds-Red. hMSC cultivated on this array expressed the corresponding fluorescent proteins with virtually total space resolution. The result is shown in Figure **12.** As can be seen from Figure **12,** it was found that there was little cross contamination. Based on the study of the role of the individual components of the printed mixture, transfection efficiency can be optimized.

(Efficiency improvement in local transfection by means of fibronectin)

In summarizing data as described above by the inventors, proteins collectively known as adhesion factors or extracellular matrix proteins such as fibronectin has been elucidated to have activities other than cell adhesion activity. Such activities vary depending on the type of various cells, and has been turned out that these activities are involved in enhancement of transfection efficiency. This is because according to the results (Figure **8)** in which changes of adhesion in the presence or absence of fibronectin were investigated, no difference was found in the state of adhesion *per se.*

(Solid-phase transfection array of human mesenchymal stem cells)

The capacity of human Mesenchymal Stem Cells (hMSC) to differentiate into various kinds of cells is particularly intriguing in studies which target tissue regeneration and renewal. In particular, the genetic analysis of transformation of these cells has attracted attention with expectation of understanding of a factor that controls the pluripotency of hMSC. In conventional hMSC studies, it is not possible to perform transfection with desired genetic materials.

To achieve this, conventional methods include either a viral vector technique or electroporation. The present inventors developed a complex-salt system, which could be used to achieve solid phase transfection which makes it possible to obtain high transfection efficiency to various cell lines (including hMSC) and special localization in high-density arrays. An outline of solid phase transfection is shown in Figure **13A.**

It was demonstrated that solid phase transfection can be used to achieve a "transfection patch" capable of being used for in vivo gene delivery and a solid phase transfection array (SPTA) for high-throughput genetic function research on hMSC.

Although a number of standard techniques are available for transfecting mammalian cells, it is known that it is inconvenient and difficult to introduce genetic material into hMSC as compared with cell lines, such as HEK293, HeLa, and the like. Conventional viral vector delivery and electroporation techniques are both important. However, these techniques have the following inconveniences: potential toxicity (for the virus technique); difficulty in high-throughput analysis at the genomic scale; and limited applications in in vivo studies (for electroporation).

The present inventors developed solid phase support fixed system which can be easily fixed to a solid phase support and has sustained-release capability and cell affinity, whereby most of the above-described drawbacks could be overcome.

An example of the results of the above-described experiment is shown in Figure **13B.** The present inventors used our microprinting technique to fix a mixture of a selected genetic material, a transfection reagent, an appropriate cell adhesion molecule, and a salt onto a solid support. By culturing cells on a support having such a mixture fixed thereonto, the gene contained in the mixture was allowed to be taken in by the cultured cells. As a result, it became possible to allow support-adherent cells to take in DNA spatially separated therefrom (Figure **13B).**

As a result of this example, several important effects were achieved: high transfection efficiency (thereby making it possible to study a group of cells having a statistically significant scale); low cross contamination between regions having different DNA molecules (thereby making it possible to study the effects of different genes separately); the extended survival of transfected cells; high-throughput, compatible and simple detecting procedure. SPTA having these features serves as an appropriate basis for further studies.

To achieve the above-described objects, the present inventors studied five different cell lines (HEK293, HeLa, NIH3T3, HepG2 and hMSC) as described above with both our methodology (transfection in a solid phase system) (see Figures **13A** and **13C)** and conventional liquid-phase transfection under a series of transfection conditions. Cross contamination was evaluated for both systems as follows. In the case of SPTA, we printed DNA's encoding a red fluorescent protein (RFP) and a green fluorescent protein (GFP) on glass supports in a checked pattern. In the case of experiments including conventional liquid phase transfection (where cells to be transfected cannot be spatially separated from one another spontaneously), a DNA encoding GFP was used. Several transfection reagents were evaluated: four liquid transfection reagents (Effectene, TransFast™, Tfx™-20, LopofectAMINE 2000), two polyamine (SuperFect, PolyFect), and two polyimine (JetPEI (x4) and ExGen 500).

Transfection efficiency: transfection efficiency was determined as total fluorescence intensity per unit area (Figure **14A** and Figure **14B** (images)). The results of liquid phase optimal for cell lines used were obtained using different transfection reagents (see Figures **14C** to **14D).** Next, these efficient transfection reagents were used to optimize a solid phase protocol. Several tendencies were observed. For cell lines which are readily transfectable (e.g., HEK293, HeLa, NIH3T3, etc.), the transfection efficiency observed in the solid phase protocol was slightly superior to, but essentially similar to, that of the standard liquid phase protocol (Figure **14A** to **14D).**

However, for cells which are difficult to transfect (e.g., hMSC, HepG2, etc.), we observed that transfection efficiency was increased up to 40 fold while the features of the cells were retained under conditions optimized to the SPTA methodology (see the above-described protocol and Figures **14C** and **14D).** In the case of hMSC (Figures **15A** and **15B),** the best conditions included use of a polyethylene imine (PEI) transfection reagent. As expected, important factors for achieving high transfection efficiency are the charge balance (N/P ratio) between the number of nitrogen atoms (N) in the polymer and the number of phosphate residues (P) in plasmid DNA, and DNA concentration. Generally, increases in the N/P ratio and the concentration lead to an increase in transfection efficiency. We also observed a significant reduction in the survival rate of hMSC cells in liquid phase transfection experiments where the DNA concentration was high and the N/P ratio was high. Because of these two opposing factors, the liquid phase transfection of hMSC had a relatively low cell survival rate (N/P ratio >10). In the case of the SPTA protocol, however, a considerably high N/P ratio (fixed to the solid support) and DNA concentration were tolerable (probably attributable to the effect of the solid support stabilizing cell membranes) while the cell survival rate and the cellular state were not significantly affected. Therefore, this is probably responsible for the dramatic improvement in transfection efficiency. It was found that the N/P ratio of 10 was optimal for SPTA, and a sufficient transfection level was provided while minimizing cytotoxicity. Another reason for the increase in transfection efficiency observed in the case of the SPTA protocol is that a high local ratio of the DNA concentration to the transfection reagent concentration was achieved (this leads to cell death in liquid phase transfection experiments).

The coating agent used is crucial to achieving high transfection efficiency on chips. It was found that when a glass chip is used, PLL provided best results both for transfection efficiency and cross contamination (described below). When fibronectin coating was not used, few transfectants were observed (all the other experimental conditions were retained unchanged). Although its function is not completely established, fibronectin probably plays a role in accelerating the cell adhesion process (data not shown), and thus limits the time which permits the diffusion of DNA released from the surface.

Low cross contamination: apart from the higher transfection efficiency observed in the SPTA protocol, an important advantage of the technique of present invention is the provision of an array of separated cells, in which selected genes are expressed in the separate positions. The present inventors printed JetPEI (see the "Experimental protocols" section) and two different reporter genes (RFP and GFP) mixed with fibronectin on glass surface coated with fibronectin. The resultant transfection chip was subjected to appropriate cell culture. Expressed GFP and RFP were localized in regions in which corresponding cDNA had been spotted, under experimental conditions which had been found to be best. Substantially no cross contamination was observed (Figures **16A** to **16D).** In the absence of fibronectin or PLL, however, cross contamination which hinders solid phase transfection was observed, and the transfection efficiency was significantly lower (see Figure **6).** This result demonstrated the hypothesis that the relative proportion of plasmid DNA, which was released from the cell adhesion and the support surface, is an important factor in high transfection efficiency and high cross contamination.

Another cause of cross contamination may be the mobility of transfected cells on a solid support. The present inventors measured both the rate of cell adhesion (Figure **16C)** and the diffusion rate of plasmid DNA on several supports. As a result, substantially no DNA diffusion occurred under optimum conditions. However, a considerable amount of plasmid DNA diffused under high cross contamination conditions until cell adhesion was completed, so that plasmid DNA was depleted from the solid phase surface.

This established technique is of particular importance in the context of cost-effective high-throughput gene function screening. Indeed, the small amounts of transfection reagent and DNA required, as well as the possible automatization of the entire process (from plasmid isolation to detection) increase the utility of the above presented method.

In conclusion, the present invention has successfully realized a hMSC transfection array in a system using complex-salt. With this technique, it will be possible to achieve high-throughput studies using solid phase transfection, such as the elucidation of the genetic mechanism underpinning the differentiation of pluripotent stem cells. The detailed mechanism of the solid phase transfection as well as methodologies for the use of this technology for high throughput, real time gene expression monitoring can be applied for various purposes.

(Example 4: Mathematical analysis)

Next, time-lapse profiles were produced based on data obtained using the techniques described in Examples 2 and 3.

(Induction of differentiation)

Each reporter was fixed to a solid phase support and cultured in undifferentiated mesenchymal stem cell maintenance medium (MSCGM, PT-3001, PT-3238, PT-4105, Cambrex, BioWhittaker, USA) for two days. Thereafter, the medium was replaced with differentiation-inducing medium (hMSC Differentiation, PT-3002, PT-4120, Cambrex, BioWhittaker, USA). The response profile of each reporter was measured.

(Mathematical analysis technique)

A mathematical analysis technique used herein is shown in Figures **18A** and **18B (18-1** to **18-2).**

(Transcription factors used herein)

As shown in Figures **19** and **24,** plasmids (commercially available from Clontech), in which 17 transcription factors (ISRE, RARE, STAT3, GAS, NFAT, MIC, AP1, SRE,GRE,CRE, NFκB, ERE, TRE, E2F, Rb, p53) were operably linked to GFP, were used to observe the differentiation of mesenchymal stem cells into osteoblasts. The resultant time-lapse profiles are shown in Figure **19.** Reporters for the transcription factors were constructed as shown in Figure **23.**

An assay was conducted using the reporters for the transcription factors under control conditions (cells, supplemental factors, culture conditions, etc.) published by Clontech.

The results are shown in Figure **25.** It was demonstrated that when compared to DNA only in this manner, most of the transcription factors were induced when inducing agents were added.

Next, the activity of the transcription factors was measured over time in the course of induction of differentiation into bone. In this case, time-lapse profiles, which were obtained during the induction of differentiation under the above-described conditions, were compared with each other. The time-lapse profiles were obtained as follows. Each reporter gene was introduced into mesenchymal stem cells by a solid phase transfection method. The cells were cultured in undifferentiated state maintenance medium for two days. Thereafter, the medium was replaced with osteoblast differentiation medium. This time point was referred to as the osteoblast differentiation start time. Supplement factors were added at concentrations recommended for the osteoblast differentiation medium. The other culture conditions were in accordance with Cambrex's instructions.

The results are shown in Figure **26.** The profile pattern on the left of Figure **26** was obtained 10 hours to 30 hours after replacement of the medium. The profile pattern on the right of Figure **26** was obtained 5 to 6 days after replacement of the medium. Thus, it was demonstrated that the pattern significantly changed over time. The phases of the profiles were calculated using a formula shown in Figure **27** and the results were summarized in a table to the right of Figure **27.** As can be seen, the inversion of the phase of the profile was closely associated with differentiation for ISRE, RARE, STAT3, GRE, CRE, TRE, E2F, and p53. Therefore, it was demonstrated that by examining the phase, changes in process, i.e., the occurrence of transcription control, could be detected.

(Arbitrary combination of reporters)

Next, it was demonstrated that differentiation could be identified using an arbitrary combination of promoters for which data was extracted at the initial stage of induction of differentiation. Briefly, the analysis was conducted as shown in Figure **20.**

The results are shown in Figure **20.** This analysis revealed that although differentiation could not be detected at its very initial stage (potentially due to noise), but could be confirmed about 15 hours after induction of differentiation. In this example, when data was extracted for 8 or more promoters, differentiation could be detected at a detection rate of 100%. When data was extracted for 3 promoters, differentiation could be detected at a detection rate of more than 90%. When data was extracted for two promoters, differentiation could be detected at a detection rate of 88%. When data was extracted for one promoter, differentiation could be detected at a detection rate of 82%. Thus, it was revealed that one, two or at least three promoters are sufficient for determination or identification of the state of cells.

The results are shown in Figure **20.** This analysis revealed that although differentiation could not be detected at its very initial stage (potentially due to noise), but could be confirmed about 15 hours after induction of differentiation. In this example, when data was extracted for 8 or more promoters, differentiation could be detected at a detection rate of 100%. When data was extracted for 3 promoters, differentiation could be detected at a detection rate of more than 90%. When data was extracted for two promoters, differentiation could be detected at a detection rate of 88%. When data was extracted for one promoter, differentiation could be detected at a detection rate of 82%. Thus, it was revealed that one, two or at least three promoters are sufficient for determination or identification of the state of cells.

(Maintenance of undifferentiated state)

Next, the maintenance of undifferentiated state was analyzed using an arbitrary combination of transcription control sequences for which data was extracted. Analysis was conducted as described in Figure **20.**

The results are shown in Figure **21.** As is seen from the results of induction of differentiation, by comparing the profiles of the transcription control sequences with one another, it could be determined whether or not stem cells were induced into differentiation or remained undifferentiated. Such a determination could be achieved using at least one transcription control sequence. The determination of the state of cells using such a small number of transcription control sequences cannot be achieved by conventional techniques. It can be said that the present invention achieved an excellent effect in this regard.

By analyzing a cellular process in such a fashion, the formation of cellular functions can be described as a cocktail party process as shown in Figure **22.** With such a process description, the present invention made it possible to analyze the progression of a cellular response to drugs and the progression of the induction of differentiation.

(Example 5: Real time measurement of a plurality of genes using cells)

Next, a device for measuring signals from cells in real time was used to obtain time-lapse data and a descriptor was produced from the data.

HeLa cells (available from RIKEN or the like) and Nakalai DMEM high Glucose supplemented with serum (10% FBS, Dainippon Pharmaceutical Co., Ltd.) were used. Transfection arrays were constructed as described in the above-described examples. 24 reporters for gene expression and signal transduction were introduced into the HeLa cells. The cells were cultured for 48 hours. A culture unit was installed and time-lapse observation was performed. A measuring device as shown in Figures **33** and **34** was used to detect the expression of the reporters via the intensity of fluorescence. Measurement was conducted in accordance with a procedure as shown in Figure **35.**

In this example, 570-grid arrays having a format as shown in Figure **36** were used. Real time monitoring was performed in serum-free medium two days after transfection for illustrative purposes. Images were taken every 30 minutes. The 24 genes (reporter vectors) were confirmed to have activity under control conditions. An exemplary image acquisition is shown in Figure **37.**

Time-lapse data obtained from the acquired image is shown for each gene. Figure **38A** is a graph showing data from all of the genes. Figures **38B** to **38E** show raw data. Figures **38F** to **38I** show the results of calculation after polynominal approximation. Figures **38J** to **38U** show data after first order differentiation and second order differentiation. Figures **39-1** to **39-55** show the genes separately. Figures **39-1** to **39-55** include data obtained from the same gene but at different points. The vertical axis represents the intensity of fluorescence (arbitrary unit = unit used in the device used herein), while the horizontal axis represents time (unit: hour (hr)).

Figure **39-1** shows time-lapse data of EGFP-N1.
Figure **39-2** shows time-lapse data of AP1.
Figure **39-3** shows time-lapse data of AP1(PMA).
Figure **39-4** shows time-lapse data of CRE.
Figure **39-5** shows time-lapse data of E2F.
Figure **39-6** shows time-lapse data of none.

Figure **39-7** shows time-lapse data of EGFP-N1.
Figure **39-8** shows further time-lapse data of AP1.
Figure **39-9** shows further time-lapse data of AP1(PMA).
Figure **39-10** shows further time-lapse data of CRE.
Figure **39-11** shows further time-lapse data of E2F.
Figure **39-12** shows time-lapse data of ERE.
Figure **39-13** shows time-lapse data of GAS.
Figure **39-14** shows time-lapse data of GRE.
Figure **39-15** shows time-lapse data of HSE.
Figure **39-16** shows time-lapse data of ISRE.
Figure **39-17** shows further time-lapse data of none.
Figure **39-18** shows further time-lapse data of ERE.
Figure **39-19** shows further time-lapse data of GAS.
Figure **39-20** shows further time-lapse data of GRE.
Figure **39-21** shows time-lapse data of HSE.
Figure **39-22** shows time-lapse data of ISRE.
Figure **39-23** shows time-lapse data of Myc.
Figure **39-24** shows time-lapse data of NFAT.
Figure **39-25** shows time-lapse data of NFκB.
Figure **39-26** shows time-lapse data of RARE.
Figure **39-27** shows time-lapse data of Rb.
Figure **39-28** shows further time-lapse data of none.
Figure **39-29** shows time-lapse data of Myc.
Figure **39-30** shows further time-lapse data of NFAT.
Figure **39-31** shows further time-lapse data of NFκB.
Figure **39-32** shows further time-lapse data of RARE.
Figure **39-33** shows further time-lapse data of Rb.
Figure **39-39** shows time-lapse data of STAT3.
Figure **39-35** shows time-lapse data of SRE.
Figure **39-36** shows time-lapse data of TRE.
Figure **39-37** shows time-lapse data of p53.
Figure **39-38** shows time-lapse data of Caspase3.
Figure **39-39** shows further time-lapse data of none.
Figure **39-40** shows time-lapse data of STAT3.
Figure **39-41** shows further time-lapse data of SRE.
Figure **39-42** shows further time-lapse data of TRE.
Figure **39-43** shows further time-lapse data of p53.
Figure **39-44** shows further time-lapse data of Caspase3.
Figure **39-45** shows time-lapse data of CREB-EGFP.
Figure **39-46** shows time-lapse data of IκB-EGFP.
Figure **39-47** shows time-lapse data of pp53-EGFP.
Figure **39-48** shows further time-lapse data of none.
Figure **39-49** shows further time-lapse data of none.
Figure **39-50** shows further time-lapse data of none.
Figure **39-51** shows further time-lapse data of CREB-EGFP.
Figure **39-52** shows further time-lapse data of IκB-EGFP.
Figure **39-53** shows further time-lapse data of pp53-EGFP.
Figure **39-54** shows further time-lapse data of none.
Figure **39-55** shows further time-lapse data of none.
Note that in the above-described listing, "none" represents a negative control.

Thus, time-lapse data was simultaneously obtained for various genes.

(Example 6: Biological systems: production of a plurality of descriptors)

Amongst the real-time data obtained in Example 5, data is extracted from eight series of data relating to Myc reporter. Myc vector (pMyc-TA-Luc; available from Clonetech PT3510-5) is shown in Figure **40.** pMyc-TA-Luc is designed to allow monitoring c-Myc and activation of a signal transduction pathway via c-Myc. Overexpression of Myc protein causes cellular transformation by activating necessary genes for cellular proliferation. Myc protein forms a heterodimer conjugate with a Max protein, thereby E-box DNA binding element (Locker (1996) Transcription Factors: Essential Data (Wiley&Sons, NY)). By this bindin eventg, transcription of a gene responsible for cellular proliferation starts (Bouchard, C. et al., (1988) Gene 66:1-10). pLyc-TA-Luc vector possesses six repeated copies of E-box consesus sequences, located upstream of TATA box of herpes simplex virus thymidine promoter (PTA), a minimul T promoter. Afirefly luciferase gene (luc) is located downstream of PTA. c-Myc protein binds to E-box, and thereafter transcription is induced to activate a reporter gene.

Such a Myc reporter was used as a reporter, and eight different coordinates were calculated, and measured in time-lapse manner under consistent culture conditions. The reporters used are pMyc-EGFP. Figure **41** shows the summary thereof.

These eight data sets were subjected to a polynomial approximate process, and a function in which the determinant coefficient after correction of degree of freedom was maximum, was used as an approximation function. Figure **42** shows data of the function notation after approximation smoothing.

With respect to the approximation function, a sectional differentiation:

$$dx/dt=(F_2-F_1)/(t_2-t_1)$$

was used to conduct first-order and second-order differentiation.

When calculating, the value of the original data was divided by 1000. This is because the process of the present Example has importance with respect to the change in the sign, and the value *per se* has no influence on the results of the analysis. The data was subjected to the following process:

(1) Differentiation operation was performed upon the original data. Values which were obtained by dividing measured values at two time points by measured time interval, variation per unit time [minute] was calculated (this is called "first-order differentiation").

(2) Differentiation operation was performed upon the first-order differentiation value. The result thereof is called the "second-order differentiation value".

(3) With respect to a time-series data relating to behaviour of the two genes, letter A is assigned to an event at which the sign of the first-order differentiation value is changed, and letter B is assigned to an event at which the second-order differentiation value is changed. Furthermore, "_" (underbar) per unit of measured space is assigned to the time course therebetween. At this time, the following event sequence is obtained.

Figures 43 and 44 show the graph after differentiation. Futhermore, the following shows the results of the assignment of the sign (+/-) in step (3):

#1: ABABABBB
#2: ABABABBB
#3: ABABABBB
#4: BABABABB
#5: ABABABBB
#6: ABABABB
#7: ABBBABABBB
#8: ABABABBB

With respect to the eight event sequences, an operation was conducted to calculate the longest common subsequence (LCS). Thus, the following sequences were obtained. Those contained in common in a plurality of letter strings amongst the subsequences in the letter strings are called common subsequences, and the longest amongst those are called the longest common subsequence. Those are not necessarily continuous. However, this search is only conducted for letter strings appearing the same elements, in the same order. Therefore, it is necessary to review whether the event occurs before or after the timepoint, and its correspondence.

LCS: ABABABB
#1: ABABABBB
#2: ABABABBB

#3: ABABABBB
#4: BABABABB
#5: ABABABBB
#6: ABABABB
#7: ABBBABABBB
#8: ABABABBB

This list contains examples of "cases where only the sequence of the event has important significance" and "cases where the connection/context of event occurrence has an important significance".

Once the longest common subsequence is calculated, the condition of "continuous letter/character strings" can be added for review. The addition of the condition gives an example of "cases where only the sequences of event has an important signficance".

The above-mentioned examples have the reaction pattern of the Myc gene in common. Therefore, the present Example shows the following: if the range is limited to the continuous portion, string ABABB is obtained. Further, when the circumferential context is included, pattern of string AB*ABABB (where x is arbitral) is extracted.

As such, the Myc gene has been elucidated to have a common event relating to the on-off of the switch of gene expression and the acceleration.

(Example 7: Biological systems: Production of a plurality of descriptors of genes - extraction examples of relationship between heterologous genes)

Next, amongst the data produced in Example 5, polynomial approximation was performed on pE2F and pRb (plasmids used are pE2F-Luc and pRb-TA-Luc, which are shown in Figure **46**), and similar to Example 6, the value of the original data was divided by 1000. This figure is shown as **M**. Relating to this data, the following process was conducted.

Trancsription factors Rb and E2F perform regulation of cellular cycle by direct interaction with the cellular cycle. Rb binds to E2F, and E2F is negatively regulated. However, when Rb is phosphorylated, binding is dissolved, and free E2F will induce the expression of each of proliferaltion related genes, which are targets thereof. When this is observed with the Mercury Signal Transduction Vector, the two reporters will show isophase response profiles due to the reporter capability of the Vectors.

(1) Differentiation operation is performed upon the original data. Values which were obtained by dividing measured values at two time points by measured time interval, variation per unit time [minute] is calculated (this is called "first-order differentiation").

(2) Differentiation operation is performed upon the first-order differentiation value. The result thereof is called "second-order differentiation value".

(3) With respect to a time-series data relating to behaviour of the two genes, letter A is assigned to an event at which the sign of the first-order differentiation value is changed, and letter B is assigned to an event at which the second-order differentiation value is changed. Furthermore, "_" (underbar) per unit of measured space is assigned to the time course therebetween. At this time, the following event sequence is obtained.

```
pE2F#1 :
      ________A____B______________B_____________________
__B____________________B_________B___________________B_
________________B_

pRb#1 :
      _______A_____B____________B____________________B__
________________________B______B____________________B__
__________B_______A
```

(4) The above-mentioned two event sequences are directly compared, allowing error within five units before and after (strictly speaking, although there is no coincidence) an event occurring at a time point, and is fouind that there is coincidence except for the last pRB#1.
As such, it can readily be confirmed that the heterologous gene reactions occur at substantially the same time.

(5) Furthermore, against the two event sequences, an operation is conducted to calculate the longest common subsequence. As such, the following event sequences are obtained.

LCS: ABBBBBBB
#1: ABBBBBBB
#2: ABBBBBBBA

As such, it was elucidated that the two gene event sequences have high similarity. Thus, it was elucidated that the descriptor of the present invention is useful for reviewing the relationship between heterologous genes.
The above Example demonstrates "the cases where time interval has an important significance".

(Example 8: Anticancer agent)

In this example, cisplatin was used as an exemplary anticancer agent and mixed into medium contacting cells. The concentration of the anticancer agent was selected as appropriate, such as 1 μM, 5 μM, 10 μM, and the like, to observe the reaction of the cells. Cisplatin was applied to cells resistant or sensitive to the anticancer agent. Time-lapse observation was conducted to produce profiles as in the above-described examples. As a result, it was revealed that time-lapse profiles varied depending on the difference in cisplatin concentration and resistance/sensitivity.

(Example 9: RNAi)

The present Example demonstrated that it was possible to obtain a profile relating toa gene knockdown effect using a cell was immobilized as described in Example 1, RNAi was used as a biological agent. The following was used as RNAi for experimentation. Gene expression inhibition methods using ribozymes and siRNA and the like allows profiles of response reactions in a cell for which gene expression inhibition is conducted, to be obtained, using the same.
RNAi: those sequences available at the URL: http://www.nippongene.jp/pages/products/sirna/review/ were used (for example, Control siRNA duplex).

(RNAi transfection)

First, it was confirmed whether the siRNA could achieve knockdown effects. Synthesis of siRNA 5'-AAGCAGCAGGACUUCUUCAAG-3' (SEQ ID NO: 2) corresponding to EGFP was performed to prepare an array substrate as described herein above in the Examples. The preparation of the array substrate using siRNA instead of nucleic acid molecules including promoter sequences was performed. Transfection using these array substrates confirmed effective inhibition of expression of a target gene. The protocols thereof are presented in Figure **28**.

(Results)

Figure **29A** shows the effects of target gene inhibition by siRNA. Expression of the target gene has actually been inhibited. The results using this gel may be stored as a profile in any data format.
Next, results of siRNA are stored as a profile data (image data of TIFF format having resolution at the level of 5 μm/pixel or less). As such, the results of siRNA may be stored as a profile data. Such a format is not limited to those specifically presented in this Example, but those skilled in the art may employ any type of formats. Furthermore, based on the profile data, it is possible to produce event descriptors using processes used in the above-described Examples.

(Example 10: Regulation of gene expression using a tetracycline-dependent promoter)

As described in the above-described Examples 1-6, it was demonstrated that a tetracycline-dependent promoter could be used to produce a profile showing how gene expression is regulated. It was then demonstrated that descriptors can be produced based on the profiles. The sequences described below were used.
As the tetracycline-dependent promoter (and its gene vector construct), pTet-Off and pTet-On vectors (BD Biosciences) were used (see http://www.clontech.com/techinfo/vectors/cattet.shtml). As a vector, pTRE-d2EGFP was used (see http://www.clontech.com/techinfo/vectors/vectorsT-Z/pTR E-d2EGFP.shtml).

(Protocol)

Tetracyclin dependent- and tetracyclin-independent promoters were printed on array substrates, and real time measurement was performed on the array substrates to determine whether or not tetracycline regulates gene expression. The results are shown in Figure **30.** As shown in Figure **30,** a change in gene expression was detected only for the tetracycline-dependent promoter. Figure **31** is a photograph showing the actual states of expression for the tetracycline-dependent promoter and the tetracycline-independent promoter. As can be seen, the difference between them is measurable by the naked eye.

(Measurement of profile data)

Images are taken in real time. Changes in intensity per cell or area are plotted on a graph. The resultant data may be subjected to linear transformation, such as noise reduction, and then multivariate analysis, signal processing, or the like, to obtain profile data. The resultant data is compared between phenomena or cells, thereby making it possible to obtain a response or identity specific to cells. Further, based on the profile data, it is possible to produce event descriptors using the processes used in the above-described Examples.

(Example 11: Gene expression)

Next, nucleic acid molecules encoding structural genes were used to produce cellular profiles. In this example, an olfactory receptor 17 (SEQ ID NOS: 13, 14) was used as a structural gene. The protocol used in the above-described Examples 1-6, was used.

As a result, as with the promoters, it was demonstrated that cellular profiles and descriptors could be produced by measuring the amount of gene products or the like.

(Example 12: Apoptotic signals)

Next, it was investigated that cellular profiles and descriptors could be produced by monitoring the activation of caspase 3 present within cells. Transfection and array preparation were performed as in the above-described Examples.

pCaspase3-Sensor Vector (BD Biosciences Clontech, 1020 East Meadow Circle, Palo Alto, CA 94303; cat. No. 8185-1) was used to monitor an apoptotic signal from caspase 3.

As a result, as with the promoters, it was demonstrated that cellular profiles could be produced by measuring apoptotic signals or the like. Furthermore, based on the profile data, it is possible to produce event descriptors using the processes used in the above-described Examples.

(Example 13: Stress signal)

Next, it was investigated whether cellular profiles and descriptors concerning stress signals from JNK, ERK, p38 or the like could be produced using transcription factor reporters. Transfection and array preparation were performed as in the above-described examples.

pAP1-EGFP, pCRE-EGFP, and pSRE-EGFP available from BD Bioscience Clontech were used to monitor stress signals from JNK, ERK, and p38.

As a result, as in the above-described examples, it was demonstrated that cellular profiles could be produced by measuring stress signals. Furthermore, based on the profile data, it is possible to produce event descriptors using the processes used in the above-described Examples.

(Example 14: Localization of molecules)

Next, it was demonstrated that a gene of interest could be fused with a fluorescent protein so that the expression profile and descriptors of the gene and the localization within cells of the gene could be visualized.

GFP, RFP, CFP and BFP, were used as fluorescent proteins and cloned KIAA cDNA libraries or the like were used as genes of interest to produce gene constructs. These materials are specifically described below:

cloned KIAA cDNA (KIAA=Kazusa DNA Research Institute, Kazusa, Chiba, Japan); and
cDNA libraries commercially available from Invitrogen.

Transfection and array preparation were performed as in the above-described examples.

The expression of cloned KIAA, KIAA1474, was monitored to produce a profile of the expression and investigate the localization of the expression.

As a result, as in the above-described examples, it was demonstrated that intentionally constructed gene constructs could be used to produce cellular profiles for target characteristics. Furthermore, based on the profile data, it is possible to produce event descriptors using the processes used in the above-described Examples.

(Example 16: Changes in cellular morphology)

Next, it was demonstrated that cellular profiles and descriptors concerning cellular morphology could be produced by expressing or knocking out genes or adding substances (glycerophosphate as a chemical substance and dexamethasone as a cytokine). Cellular morphology, such as multinucleated cells, cellular outgrowth, outgrowth projections, and the like, was measured and analyzed as three-dimensional data.

The specific sequences of the nucleic acid molecules that were introduced are described below:

Cloned KIAA (supra); and
RNAi for transcription factors (CBFA-1, AP1).

Transfection and array preparation were performed as in the above-described examples.

Mesenchymal stem cells as used in the above-described examples were used to monitor the morphology of cells which were induced to differentiate into osteoblasts.

As a result, as in the above-described examples, it was demonstrated that intentionally constructed gene constructs could be used to produce cellular profiles for target characteristics. Event descriptors can be produced based on the profile data using the process as used in the above-described examples.

(Example 17: Intermolecular interaction)

Next, it was demonstrated that cellular profiles and descriptors could be produced by using a technique such as a two-hybrid system, FRET, BRET, or the like.

The specific sequences of the introduced nucleic acid molecules are described below:

olfactory receptors (SEQ ID NOS: 13 to 38) ; and
G proteins (SEQ ID NOS: 39 to 44).

Transfection and array preparation were performed as in the above-described examples.

The dissociation of the olfactory receptor and G protein was monitored through induction of a scented substance, which was captured as changes in fluorescent wavelength. In this manner, cells were monitored.

The two-hybrid system, FRET, and BRET were specifically performed as follows.

The two-hybrid system was available from Clontech (http://www.clontech.co.jp/product/catalog/007003006.sh tml). FRET and BRET were performed using devices available from Berthold Japan.

As a result, as in the above-described examples, it was demonstrated that intentionally constructed gene constructs could be used in a two-hybrid system, in conjunction with FRET, BRET, or the like, to produce cellular profiles. Furthermore, based on the profile data, it is possible to produce event descriptors using the processes used in the above-described Examples.

(EXAMPLE 18: Receptor-Ligand)

Next, it was demonstrated that a cellular profile and descriptors can be produced by employing the interaction between a receptor and its ligand as an indicator. It is useful for network formation in a cell, to obtain information about interaction between a receptor protein present in the cell membrane or nuclear membrane, or the like, and a ligand thereto.

In the present Example, the following was prepared:

(Cell adhesion molecules)

A variety of extracellular matrix protein and variants and fragments thereof were prepared as candidates for cell adhesion molecules. What was prepared in the present Example is as follows. Cell adhesion molecules were commercially available.

1) ProNectin F (Sanyo Chemical Industries, Kyoto, Japan);
2) ProNectin L (Sanyo Chemical Industries);
3) ProNectin Plus (Sanyo Chemical Industries);
4) fibronectin (SEQ ID NO.: 2);
5) gelatin.

Plasmids were prepared as DNA for transfection. Plasmids, pEGFP-N1 and pDsRed2-N1 (both from BD Biosciences, Clontech, CA, USA) were used. In these plasmids, gene expression was under the control of cytomegalovirus (CMV) promoter. The plasmid DNA was amplified in E. coli (XL1 blue, Stratgene, TX, USA) and the amplified plasmid DNA was used as a complex partner. The DNA was dissolved in distilled water free from DNase and RNase.
The following transfection reagents were used: Effectene Transfection Reagent (cat. no. 301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France), and ExGen 500 (R0511, Fermentas Inc., MD). These transfection reagents were added to the above-described DNA and actin-like acting substance in advance or complexes thereof with the DNA were produced in advance.
The thus-obtained solution was used in assays using transfection arrays described below. Next, transfection effects on a solid phase were observed. The protocols therefor are described below:

(Protocol)

The final concentration of DNA was adjusted to 1 μg/μL. A cell adhesion molecule was preserved as a stock having a concentration of 10 μg/μL in $ddH_2O$. All dilutions were made using PBS, $ddH_2O$, or Dulbecco's MEM. A series of dilutions, for example, 0.2 μg/μL, 0.27 μg/μL, 0.4 μg/μL, 0.53 μg/μL, 0.6 μg/μL, 0.8 μg/μL, 1.0 μg/μL, 1.07 μg/μL, 1.33 μg/μL, and the like, were formulated.
Transfection reagents were used in accordance with instructions provided by each manufacturer.
Plasmid DNA was removed from a glycerol stock and amplified in 100 mL L-amp overnight. Qiaprep Miniprep or Qiagen Plasmid Purification Maxi was used to purify DNA in accordance with a standard protocol provided by the manufacturer.
In the present Example, the following five cells were used to confirm an effect: human mesenchymal stem cell (hMSCs, PT-2501, Cambrex BioScience Walkersville, Inc., MD) ; human embryonic renal cell (HEK293, RCB1637, RIKEN Cell Bank, JPN); NIH3T3-3 cell (RCB0150, RIKEN Cell Bank, JPN); HeLa cell (RCB0007, RIKEN Cell Bank, JPN); and HepG2 (RCB1648, RIKEN Cell Bank, JPN). These cells were cultured in DMEM/10% IFS containing L-glut and penicillin/streptomycin.

(Dilution and DNA spots)

Transfection reagents and DNA were mixed to form a DNA-transfection reagent complex. The complex formation requires a certain period of time. Therefore, the mixture was spotted onto a solid phase support (e. g. , a poly-L-lysine slide) using an arrayer. In the present Example, as a solid phase support, an APS slide, a MAS slide, and an uncoated slide were used, as well as a poly-L-lysine slide. These slides are available from Matsunami Glass (Kishiwada, Japan) or the like.
For complex formation and spot fixation, the slides were dried overnight in a vacuum dryer. Drying was performed for a duration in the range of 2 hours to 1 week.
Although the cell adhesion molecule might be used during the complex formation, it was also used immediately before spotting in the present Example.

(Formulation of mixed solution and application to solid phase supports)

300 μL of DNA concentrated buffer (EC buffer) + 16 μL of an enhancer were mixed in an Eppendorf tube. The mixture was mixed with a Vortex, followed by incubation for 5 minutes. 50 μL of a transfection reagent (Effectene, etc.) was added to the mixture, followed by mixing by pipetting. To apply a transfection reagent, an annular wax barrier was formed around the spots on the slide. 366 μL of the mixture was added to the spot region surrounded by the wax, followed by incubation at room temperature for 10 to 20 minutes. Thereby, the fixation to the support was manually achieved.

(Distribution of cells)

Next, a protocol for adding cells will be described. Cells were distributed for transfection. The distribution was typically performed by reduced-pressure suction in a hood. A slide was placed on a dish, and a solution containing cells was added to the dish for transfection. The cells were distributed as follows.

The growing cells were seeded at a concentration of $10^7$ cells/25 mL. The cells were plated on the slide in a 100x100x15 mm squared Petri dish or a 100 mm (radius) $\times$ 15 mm circular dish. Transfection was conducted for about 40 hours. This period of time corresponded to about 2 cell cycles. The slide was treated for immunofluorescence.

(Evaluation of gene introduction)

Gene introduction was evaluated by detection using, for example, immunofluorescence, fluorescence microscope examination, laser scanning, radioactive labels, and sensitive films, or emulsion.

When an expressed protein to be visualized is a fluorescent protein, such a protein can be observed with a fluorescence microscope and a photograph thereof can be taken. For large-sized expression arrays, slides may be scanned using a laser scanner for storage of data. If an expressed protein can be detected using specific fluorescence in the case of calcium, a protocol specific for detection of a specific fluorescence can be successively performed to detect signals. If an expressed protein can be detected using fluorescent antibodies, an immunofluorescence protocol can be successively performed.

(Laser scanning and Quantification of fluorescence intensity)

To quantify transfection efficiency, the present inventors used a DNA microarray scanner (GeneTAC UC4x4, Genomic Solutions Inc., MI). Total fluorescence intensity (arbitrary units) was measured, and thereafter, fluorescence intensity per unit surface area was calculated.

(Cross-sectional observation by confocal scanning microscope)

Cells were seeded on tissue culture dishes at a final concentration of $1\times10^5$ cells/well and cultured in appropriate medium (Human Mesenchymal Cell Basal Medium (MSCGM BulletKit PT-3001, Cambrex BioScience Walkersville, Inc., MD). After fixation of the cell layer with 4% paraformaldehyde solution, SYTO and Texas Red-X phalloidin (Molecular Probes Inc., OR, USA) was added to the cell layer for observation of nuclei and F-actin. The samples emitting light due to gene products and the stained samples were observed with a confocal laser microscope (LSM510: Carl Zeiss Co. , Ltd., pin hole size=Ch1=123 $\mu$m, Ch2=108 $\mu$m, image interval = 0.4) to obtain cross sectional views.

Next, an Example, to which the present invention is directed to, is described wherein an olfactory receptor is selected as a typical example of a chemical substance receptor. When a preliminary example was implemented, it was proved that transfection arrays can also be used for an olfactory receptor.

The olfactory receptor expression vector group was spotted per every kind of receptor, on a cover glass, which was made like an array, secured with screws and the like in a chamber for signal measurement, and cells having an almost homogeneous nature, were cultured thereon. Regarding a chamber for signal measurement, sample gas was introduced into a known structure (Proc. Natl. Acad. Sci. USA, 96(1999): 4040-4045 and the like). Other devised chambers are also intended. During response measurement, culture medium was passed through the chamber at a constant speed. Culture media was supplied to the chamber for measurement from the opening of a culture medium supply tube, and a sample gas supplying tube was secured at a position preferably near the liquid level, which is the upper portion of an interval whose boundary is defined by a wall which prevents the approach of culture media over a cover -slip that forms the ceiling of the measurement member, so that sample gas can be supplied to culture medium flowing across the interval. This sample gas supplying tube is preferably made of materials to which lipophilic odor substances and dust are not readily adsorbed, such as Teflon. A greater effect was obtained in the situation wherein, at times other than introducing sample gas, sample gas remaining in the tube was removed, and to preferably keep the interior clean, the tube (preferably with a broad opening) could be purged with odorless air by setting a three-way valve in the mid course, or by setting a check valve at a joint of an odorless air supplying tube. However, it was not necessary to do so. The example could also be implemented in the situation wherein, at a time other than when introducing sample gas from outside for an appropriate time such as 0.5-4 seconds, odorless air was introduced from mid course of a sample gas supplying tube near a opening for collecting gas from outside, the interior of the tube was washed therewith, and at the same time, odorless gas was supplied to the culture medium as sample gas in order to promote the removal of remaining gas in a measurement chamber. A supporting base for the upper-glass cover slip is made of a water repellent opaque plastic such as Teflon. A breadth of flow channel, where culture medium flows, is about 2-fold of a breadth of an array, and the array is disposed in the center of the flow channel. Regarding a culture medium supplying tube and an overflow culture medium sucking tube, a portion several millimeters from the opening at the side of the measurement chamber is made using materials which have high hydrophilicity and are difficult to deform, such as stainless steel. The upper portion of the supporting base of the upper glass cover-slip where culture medium flows from the openings of both tubes to an array, was coated, or covered with a pieces of lens paper and the like in order to provide sufficient hydrophilicity. Negative pressure for suction was adjusted at the level such that measurements were not affected by vibration from sound generated by aspiration of culture medium.

Generally, response measurement could be implemented 2 days after the gene introduced by the vector was expressed. Since an upper glass cover-slip was required only at the time of measurement, it was not required to install it during culture until the gene was expressed. Therefore, the Example could be implemented, adding an upper glass cover slip integrated with a wall which prevents leakage of culture medium, and a supporting base for the upper glass cover slip, to a chamber for measurement, when setting a chamber for measurement of a change in fluorescence measured by an apparatus after the gene was expressed. The Example could also be implemented in the situation wherein culture medium was exchanged without using a culture medium supply tube and an overflow culture suction line tube during culture until the gene was expressed. An amount of about 10ml of culture medium was supplied and exchanged at the frequency of about once per several hours to one day, only during the time that tissue culture was performed.

The size of odor response could be optically measured using a two-dimensional image sensor such as a sensitive video camera, with a calcium ion sensitive fluorescent dye, such as fura-2 and the like absorbed into the cell. The measurement interval preferably has a time resolution which can evaluate time constants of build-up and recovery of response of about 1/3-1 second. However, if average response time curve or its theoretical formula had been obtained, actual change was estimated from measurement results at 5 points with 5-second-intervals, 5, 10, 15, 20, and 25 seconds after stimulation. The obtained estimates of the time constant of the response starting time, response build-up time, and response recovery time was set as an index, and an evaluation could be made as to whether a signal was induced by odor, or generated by spontaneous activity of a cell, or other abnormalities. All of such evaluations could also be obtained as cellular profiles.

In this Example, the response of an expressed olfactory receptor in olfactory receptor neuron was studied by measuring the change in fluorescence intensity of a calcium sensitive fluorescent dye. Decrease of fluorescence intensity (downward change) corresponds to the response of an olfactory receptor. Odor molecules were added to the culture at the concentration indicated above them as stimulation source, and administered to a cell during the time indicated by a bar (4 or 2 seconds). As understood from this example, responses measured simultaneously in a simultaneously adjusted cell have high interconnectedness in response time characteristics, response threshold concentration corresponding to different stimulation per cell, and relative value of response amplitude. However, cells adjusted at a different times show some differences. These results show that the highest measurement reliability can be obtained by measuring odor response using a sensor arrayed to a size that allows a homogeneous administration of sample gas, providing the same adjustment conditions.

Accordingly, it was also demonstrated that an olfactory receptor-ligand (olfactory substance) may be used to obtain profiles of a cell. Furthermore, based on the profile data, it is possible to produce event descriptors using the processes used in the above-described Examples.

(Example 19: MicroRNA)

Next, nucleic acid molecules encoding microRNA (miRNA) were used to produce cellular profiles. As miRNA, miRNA-23 was used. A protocol as used in the above-described Examples 1-6 was used.

MicroRNA is a non-coding RNA of 18 to 25 bases (not translated into protein), which was first found in nematodes and then revealed to be conserved widely throughout animals and plants. It has been reported that miRNA is involved in the development and differentiation of nematodes and plants. It has been suggested that animals have a similar process. To date 200 or more miRNAs have been reported.

Nature 423, 838-842(2003) reported that the target of miRNA-23 is the Hes1 gene (Hes1 is a repressor transcription factor which suppress the differentiation of stem cells into neurons). miRNA-23 is present in the vicinity of the translation terminating codon for this gene, and forms incomplete complementary base pairing (77%). Such incomplete complementary base pairing is important for the function of miRNA. Indeed, it has already been found that synthetic miRNA-23, which when introduced into NT2 human embryonic tumor cells, can suppress the expression of Hes1. This activity can be knocked out by using siRNA or the like.

It can be demonstrated that such a system can be used to produce a profile and descriptors concerning the behavior of miRNA, and to measure the amount of relevant genetic material, thereby making it possible to produce cellular profiles and descriptors of a cell.

(Example 20: Biological system-ribozyme)

Next, a ribozyme was used to produce cellular profiles and descriptors. A ribozyme as described in 305 YAKUGAKU ZASSHI [Journal of Phamacology] 123(5) 305-313 (2003) is herein used. A protocol as described in Examples 1-6 is used.

Ribozymes were discovered by observing that the group I intron of tetrahymena catalyzes site specific cleavage and binding reactions of RNA chains. A ribozyme refers to RNA having such an enzymatic activity. Examples of ribozymes include hammerhead ribozymes, hairpin ribozymes, and the like.

It can be demonstrated that such a system can be used to produce a profile concerning the behavior of a ribozyme and measure the transcription level of relevant genes, the amount of relevant genetic materials, or the like, thereby making it possible to produce cellular profiles and descriptors.

(Example 21: A biological system: a biological organism - brain wave analysis)

Brain waves are also referred to as electroencephalogram (EEG, and can be measured at the scalp. Brain wave is a change in potential (voltage) which emits from the scalp and gradually changes w. A normal adult emits changes of several tens of microvolts (1microvolt = 1/1000000 volt), and thus potential change of several to several tens cahgens per second can be recorded as a signal.

In the present Example, it was demonstrated that descriptor production and analysis mehtods according to the present invention may be used for a biological organism per se, rather than simply a cell.

There are generally several types of brain waves such as waves activated in a significant manner to keep the brain awake (beta wave), and while it keeps awake, waves that do not activate the brain (alpha wave), while in dormant state, as well as waves asscoated with light sleep (theta wave and sleep spindle), and deep sleeping state (delta and theta waves).

Brain waves are classified into the four groups: referred to in Figure **47,** 14-25 Hz: beta wave, significantly appeared in 1 in the Figure; 8-13 Hz, alpha wave: significantly appeared in 2 in the figure; 4-7Hz, theta wave, appeared in 3 in the figure; delta wave: appeared in 4 in the figure.

Such waves are subjected to polynomial approximation similar to Example 6, to obtain first-order and second-order differentiations. Alternatively, a certain threshold is set, and times at which the threshold is exceeded, are recorded as an event timing, to produce an event descriptor. Analysis of such a descriptor will allow correlation between beta and alpha waves with the state of the brain. Conversely, mere simple analysis of the event descriptor allows determination of the brain activity.

(Example 22: Biological system: a biological organism - electrocardiodiagram analysis)

Next, as a biological organism, an electrocardiodiagram was used to perform the descriptor production and analysis method according to the present invention. As electrocardiodgrams, normal and extrasystolic diagrams were used. In the case of normal electrocardiodiagram, the electrocardiodiagram and the wave of the blood pressure are constant. Extrasystolic electrocardiodiagrams cause turbulance in the wave due to "unexpected systole". Strokes caused due to the unexpected systoles are weaker than usual, and thus are felt as intermittent pulses. Continuous occurrence of such extrasystoles does not sufficiently raise the blood pressure, and thus cannot deliver sufficient blood to the whole body. Therefore, conditions such as dizziness may occur. When the pulses are delayed, there may be intermittent pulses. One in two or one in three, are examples of regularly intermittent pulses, at which time extrasystole will occur.

In order to analyze such cases, electrocardiodiagram are obtained from normal subjects and subjects which appear to experience extrasystole. As described in Example 6, these electrocardiodiagrams are subjected to polynomial approximation, to obtain first-order and second-order differentiations. Alternatively, a certain threshold is set, and times at which the threshold is exceeded, are recorded as an event timing, to produce an event descriptor. Analysis of such a descriptor will allow simple analysis of a variety of diagnosis by solving the event sequences of electrocardiodiagrams.

(Example 23: Economic system: stock prices)

Examples relating to extractions related to a company group directed to stock price variation are demonstrated. Amongst the most recent stock prices of five companies at the date of deal, it is now demonstrated that the property of the change thereof is extracted to obtain useful information.

First, the stock prices of thirty deal days before for each company (Figure 48) are indexed as 100, and a smoothing operation is conducted against the indexed stock price data. The five-day moving average centering to the date of deal was calculated.

$$y(t) = \frac{1}{5}\{x(t-2) + x(t-1) + x(t) + x(t+1) + x(t+2)\}$$

The actual names are not shown herein, but A1-A3 are companies with capital relation, and B1-B2 also have capital relation to each other.

With respect to the stock price date after smoothing, letter U is assigned to the event at which the closing price at the date of deal is increased compared with the closing price of the previous date, and letter D is assigned to the event where the closing price is decreased compared with the previous date. Furthermore, "_" (underbar) is assigned to the time interval therebetween per unit of measured interval. Then, the following event sequences are obtained.

```
A1:   UD______U_D____U__DUD___
A2:   ________U__D____U_D_____
A3:   UDUD____U__________D____
B1:   ___U____D________U___D__
B2:   _D__U______D_____U___D__
```

The above-mentioned event sequences are compared to read out the relationship as shown below. A1 through A3 have similar sequences and B1-B2 also have similar sequeneces. However, the group of A1-A3 have a different tendency to the group of B1-B2. In fact, Group A1-A3 and Group B1-B2 belong to different company groups.

It was demonstrated that application of the method of extracting event sequences from such time-series data allows extraction of characteristics beteween the lineages and grouping (clustering).

TABLE 1

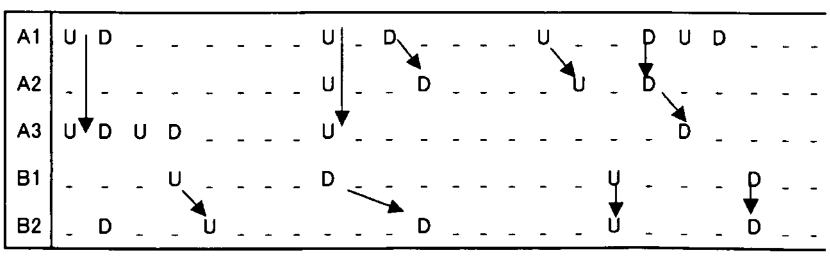


These measures may be applicable not only to stock prices but also a variety of economic indices (for example, currency exchange), social scientific indices and the like.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

## INDUSTRIAL APPLICABILITY

The present invention allows analysis of a variety of systems using an algorithm or the like, in a more efficient and/or more accurate manner. The descriptor and a method for analyzing using the same according to the present invention has been proved to be applied not only to a biological system but also to an economic or social scientific system or the like. As such, it is possible that analysis may be performed in any field of industry. Therefore, such determination allows application in diagnosis, prevention, therapy or the like, and thus the application extends not only to medicine but also to a variety of fields such as food, cosmetics, agriculture, environment and the like.

## Claims

**1.** A method for producing an event descriptor relating to at least one system, comprising the steps of:

(A) obtaining time series data of at least one index derived from at least one system;
(B) providing at least one characteristic behaviour relating to the index; and
(C) extracting a portion having the characteristic behaviour in the times series data as an event timing to produce an event descriptor described by the event timing.

**2.** A method according to claim 1, wherein the system is biological.

**3.** A method according to claim 1, wherein the system is a portion of a biological entity selected from the group consisting of biological body, organ, tissue, cell population, cell and cellular organelle.

**4.** A method according to claim 1, wherein the system is a cell.

**5.** A method according to claim 1, wherein the system is a social organization.

**6.** A method according to claim 1, wherein the system is an economic system.

**7.** A method according to claim 1, wherein the index is selected from the group consisting of a natural scientific index, a technical index, a social scientific index, and a human scientific index.

**8.** A method according to claim 1, wherein the index is related to at least one state selected from the group consisting of a differentiation state, a response to a external agent, a cellular cycle state, a proliferation state, an apoptosis state, a response to a circumstantial change and an aging state.

**9.** A method according to claim 1 wherein the index comprises at least one selected from the group consisting of a gene expression level, a gene transcription level, gene a post-translational modification level, chemical level present intracellularly, an intracellular ion level, cell size, a biochemical process level, and a biophysical process level.

**10.** A method according to claim 1, wherein the index comprises at least one selected from the group consisting of a gene expression level and a gene transcription level.

**11.** A method according to claim 1 wherein the index comprises a gene transcription level.

**12.** A method according to claim 1, wherein the characteristic behaviour comprises at least on selected from the group consisting of: coincidence of the time-series data and a predetermined value, or a specific variation or no change of the absolute value change rate thereof; coincidence of a first-order differentiation value of the time-series data and a predetermined value, or a specific variation or no change of the absolute value change rate thereof; coincidence of a second-order differentiation value of the time-series data and a predetermined value, or a specific variation or no change of the absolute value change rate thereof; change in sign (+/-) of the time-series data; change in sign (+/-) of the first-order differentiation value of the time-series data; change in sign (+/-) of the second-order differentiation value of the time-series data; coincidence of the time-series data and time-series data of another index; coincidence of the first-order differentiation of the time-series data and the first-order differentiation of time-series data of another index; coincidence of the second-order differentiation of the time-series data and the second-order differentiation of time-series data of another index; coincidence of sign (+/-) of the time-series data and the sign of time-series data of another index; coincidence of sign (+/-) of the first-order differentiation value of the time-series data and the sign of the first-order differentiation value of time-series data of another index; coincidence of sign (+/-) of the second-order differentiation value of the time-series data and the sign of the second-order differentiation value of time-series data of another index; coincidence of the time-series data and another time-series data of the index; coincidence of the first-order differentiation of the time-series data and the first-order differentiation of another time-series data of the index; and coincidence of the second-order differentiation of the time-series data and the second-order differentiation of another time-series data of the index.

**13.** A method according to Claim 1, wherein the characteristic behaviour is the change of the sign of the first-order differentiation value of the time-series data.

**14.** A method according to claim 1, wherein the time-series data is continuous or discontinuous.

**15.** A method according to claim 1, wherein the time-series data is described in relative time or absolute time.

**16.** A method according to claim 1, wherein the time series data is described in such a manner that the initiation time of observation is expressed as a reference (0).

**17.** A method according to claim 1, wherein the time-series data is expressed as a relative or absolute level.

**18.** A method according to claim 1, wherein the time-series data are those of a genetic expression level, and the genetic expression level is an expression level of a flucorescent protein.

**19.** A method according to claim 1, wherein the time-series data are normalized data.

**20.** A method according to claim 1 wherein the event timing is expressed as a time point or a time range.

**21.** A method according to claim 1, wherein the event timing is within a shift or within a time range of 12 hours or less.

**22.** A method according to claim 1, wherein the event timing is within a shift or within a time range of one hour or less.

**23.** A method according to claim 1, further comprising the step of mathematically processing the time series data.

**24.** A method according to claim 23, wherein the mathematical process is selected from the group consisting of normalization, first-order differentiation, second-order differentiation, third-order differentiation, linear approximation, non-linear approximation, moving average, noise filter, Fourier's transform, fast Fourier's transform and principal component analysis.

**25.** A method according to claim 1, wherein the event timing is calculated based on raw data of the time series data.

**26.** A method according to claim 1, wherein the event timing is calculated based on the first-order differentiation of the time series data.

**27.** A method according to claim 1, wherein the event timing is calculated based on the second-order differentiation of the time series data.

**28.** A method according to claim 1, wherein the event timing is calculated based on the coincidence of increase or decrease per unit time in a plurality of time series data.

**29.** A method according to claim 28, wherein each of the unit time are identical or different.

**30.** A method according to claim 1, wherein the event timing is represented in the increase, decrease or unchanged status of the index.

**31.** A method according to claim 1, wherein the event timing is represented by the expression manner of (time t, the increase, decrease or unchangeness of the index <+, - or 0>).

**32.** A method according to claim 31, wherein the time t is represented by a time point or time range.

**33.** A method according to claim 1, wherein the event descriptor is represented by aligning characters or letters related to the event timing in an order of time points.

**34.** A method according to claim 1, wherein the description relating to the event timing is represented by means of A, T, G or C, which are single letter designators of nucleic acids in an order of time points.

**35.** A method according to claim 1, wherein the increase or decrease in the index is **characterized in that** the point at which the sign of the first-order differentiation is changed, the sign of the second-order differentiation is changed, or the case where the value of raw data is significantly changed in an experiment, are indicative of the increase or decrease.

**36.** A method according to claim 1, wherein the increase or decrease in the index is **characterized in that** the point at which the sign of the first-order differentiation is changed, the sign of the second-order differentiation is changed, or the case where the value of raw data is significantly changed in an experimental system, in a normalized form of the time-series data.

**37.** A method according to claim 1, wherein at least two indices are used as the index, and, as the event timing, those at which the behaviors of increase or decrease coincide with respect to the increase/decrease of the index at at least one point in at least two types of indices.

**38.** A method according to claim 1, wherein sign change in first-order differentiation and sign change in second-order differentiation are used as the characteristic behavior, and a first letter/character corresponding to the sign change of the first-order differentiation and a second letter corresponding to the sign change of the second-order differentiation are represented in a form of a character string according to the time order as the event descriptor.

**39.** A method according to claim 1, wherein sign change in first-order differentiation and sign change in second-order differentiation are used as the characteristic behavior, and a first letter/character corresponding to the sign change of the first-order differentiation, a second letter corresponding to the sign change of the second-order differentiation and a third letter/character corresponding to another letter/character regarding the time without sign change are represented in the form of a character string according to the time order as the event descriptor.

**40.** A method according to claim 1, wherein sign change in raw data is used as the characteristic behavior, and a first letter/character corresponding to the increase in the raw data, and a second letter/character corresponding to the decrease in the raw data, are represented in a form of a character string according to the time order as the event descriptor.

**41.** A method according to claim 1, wherein sign change in raw data is used as the characteristic behavior, and a first letter/character corresponding to the increase in the raw data, a second letter/character corresponding to the decrease in the raw data, and a third letter/character corresponding to another character/letter regarding the time without increase or decrease are represented in a form of a character string according to the time order as the event descriptor.

**42.** A method according to claim 1, wherein the event descriptor is described with the notation selected from the group consisting of electric wave, magnetic wave, sound, light, color, image, number and character/letter.

**43.** A method according to claim 1, wherein the event descriptor is notated by characters or letters.

**44.** A method according to claim 1, further comprising the step of recording the event descriptor on a storage medium.

**45.** A method for analyzing at least one system using an event descriptor relating to the system, comprising the steps of:

(A) obtaining time-series data of at least one index derived from at least one system;
(B) providing at least one characteristic behavior;
(C) extracting a portion having the characteristic behaviour as an event timing in the time-series data; and
(D) analyzing the at least one event descriptor.

**46.** A method according to claim 45, wherein the analysis uses an algorithm.

**47.** A method according to claim 45, wherein the algorithm comprises one selected from the group consisting of self-organization mapping, cluster analysis, genetic algorithm, alignment analysis, and parsing in a natural language processing.

**48.** A method according to claim 48, wherein the algorithm comprises a genetic algorithm.

**49.** A method according to Claim 45, wherein the system is a biological system.

**50.** A method according to Claim 45, wherein the system is a cell.

**51.** A method for analyzing the relationship between a first index and a second index in a system, comprising the steps of:

(A) producing a first event descriptor relating to a first index using a method according to claim 1;
(B) producing a second event descriptor relating to a second index using a method according to claim 1; and
(C) comparing the first and second event descriptors obtained in steps (A) and (B).

**52.** A method according to claim 51, wherein the comparison in the step (c) is conducted by production of coincidence event timing whose behaviors coincide in the first and second event descriptors.

**53.** A method for analyzing the relationship between a first index from a first system and a second index from a second system, comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**54.** A method for analyzing the relationship between indices at a first and second time points from a system, comprising the steps of:

(A) producing a first event descriptor relating to the first time point using a method according to claim 1;
(B) producing a second event descriptor relating to the second time point using a method according to claim 1; and
(C) comparing the first and second event descriptors obtained in the steps (A) and (B).

**55.** A method for analyzing an index from a system using an event descriptor obtained using first and second characteristic behaviors, comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**56.** A method according to claim 55, wherein the step of comparison comprises the step of extracting an event timing which coincides at a time point between the event timing in the first event descriptor and the event timing of the second event descriptor.

**57.** A production system for producing an event descriptor relating to a system, comprising:

i) monitoring means for monitoring at least one index relating to the system in a time-lapse manner; and
ii) descriptor production means for producing an event descriptor by producing a time-series data of the system from a signal obtained from the monitoring means, and calculating the time-series data; wherein the descriptor production means

(A) obtains time series data of at least one index derived from at least one system;
(B) provides at least one characteristic behaviour relating to the index; and
(C) extracts a portion having the characteristic behaviour in the time series data as an event timing to produce an event descriptor described by the event timing.

**58.** A production system according to claim 57, wherein the system is a cell, and the production system further comprises a support capable of maintaining a certain environment around the cell.

**59.** A production system according to claim 57, wherein the monitoring means is selected from the group consisting of an optical microscope,a fluorescent microscope, reading devices using a laser light source, surface plasmon resonance (SPR) imaging, reading devices of a signal derived from a means using electric signals, chemical or biochemical markers or a combination thereof, CCD camera, autoradiography, MRI and sensors.

**60.** A production system according to claim 57, wherein the monitoring means comprises means for outputting a signal.

**61.** A production system according to claim 57, wherein the descriptor production means comprises means for producing the time-series data, and means for producing the descriptor by conducting the calculation step.

**62.** A production system according to claim 57, wherein the descriptor production means comprises a computer implementing a program instructing performing the steps of (A) through (C).

**63.** A production system according to claim 57, wherein the descriptor further comprises display means for displaying the descriptor.

**64.** A production system according to claim 63, wherein the display means has functions displaying a notation selected from the group consisting of an electric wave,a magnetic wave, sound, light, color, image, number and character/letter

**65.** A production system according to claim 63, wherein the display means has a letter/character displaying function.

**66.** A production system according to claim 57, further comprising means for recording the event descriptor on a storage medium.

**67.** An event descriptor for describing a system, comprising a portion having at least one characteristic behavior as an event timing relating to at least index derived from at least one system.

**68.** An event descriptor produced by a method according to claim 1.

**69.** An analysis system for analyzing a system using a descriptor relating thereto, comprising:

i) monitoring means for monitoring at least one index relating to the system in a time-lapse manner;
ii) descriptor production means for producing an event descriptor by producing a time-series data of the system from a signal obtained from the monitoring means, and calculating the time-series data; and
iii) analysis means for analyzing the descriptor,
wherein the descriptor production means

(A) obtains time series data of at least one index behavior derivied from at least one system;
(B) provides at least one characteristic behavior relating to the index; and
(C) extracts a portion having the characteristic behavior in the times series data as an event timing to produce an event descriptor described by the event timing.

**70.** An analysis system according to claim 69, wherein the analysis means has a function of analyzing at least one event descriptor with an algorithm analysis.

**71.** A method for analyzing a system using a sequence of event descriptors relating to at least one system, comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

**72.** A method according to claim 71, wherein the analysis of sequence uses genetic algorithm.

**73.** An analysis system for analyzing a system using a sequence of event descriptors relating to at least one system, comprising:

i) monitoring means for monitoring at least one index relating to the system in a time-lapse manner;
ii) descriptor production means for producing an event descriptor by producing a time-series data of the system from a signal obtained from the monitoring means, and calculating the time-series data to produce an event descriptor describing the event timing as a sequence; and
iii) analysis means for analyzing the sequence,
wherein the descriptor production means

(A) obtains time series data of at least one index derived from at least one system;
(B) provides at least one characteristic behavior relating to the index; and
(C) extracts a portion having the characteristic behavior in the times series data as an event timing to produce an event descriptor described by the event timing.

**74.** An analysis system according to claim 73, wherein the analysis of the sequence uses genetic algorithm.

**75.** A program for implementing a computer a process for producing an event descriptor relating to at least one system, the process comprises the steps of:

(A) obtaining time series data of at least one index derived from at least one system;
(B) providing at least one characteristic behavior relating to the index; and
(C) extracting a portion having the characteristic behavior in the times series data as an event timing to produce an event descriptor described by the event timing.

**76.** A program for implementing a computer a process for analyzing at least one system using an event descriptor relating to the system, the process comprising the steps of:

(A) obtaining time-series data of at least one index derived from at least one system;
(B) providing at least one characteristic behavior;
(C) extracting a portion having the characteristic behavior as an event timing in the time-series data; and
(D) analyzing the at least one event descriptor.

**77.** A program for implementing a computer a process for analyzing the relationship between a first index and a second index in a system, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**78.** A program for implementing a computer a process for analyzing the relationship between a first index from a first system and a second index from a second system, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**79.** A program for implementing a computer a process for analyzing an index from a system using an event descriptor obtained using first and second characteristic behaviors, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**80.** A program for implementing in a computer a process for analyzing a system using a sequence of event descriptors relating to at least one system, the process comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

**81.** A storage medium storing a program for implementing in a computer a process for producing an event descriptor relating to at least one system, the process comprises the steps of:

(A) obtaining time series data of at least one index derived from at least one system;
(B) providing at least one characteristic behavior relating to the index; and

(C) extracting a portion having the characteristic behavior in the times series data as an event timing to produce an event descriptor described by the event timing.

**82.** A storage medium storing a program for implementing in a computer a process for analyzing at least one system using an event descriptor relating to the system, the process comprising the steps of:

(A) obtaining time-series data of at least one index derived from at least one system;
(B) providing at least one characteristic behavior;
(C) extracting a portion having the characteristic behavior as an event timing in the time-series data; and
(D) analyzing the at least one event descriptor.

**83.** A storage medium storing a program for implementing in a computer a process for analyzing the relationship between a first index and a second index in a system, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**84.** A storage medium storing a program for implementing in a computer a process for analyzing the relationship between a first index from a first system and a second index from a second system, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**85.** A storage medium storing a program for implementing in a computer a process for analyzing an index from a system using an event descriptor obtained using first and second characteristic behaviors, the process comprising the steps of:

(A) producing a first event descriptor relating to a first index using a method according to claim 1;
(B) producing a second event descriptor relating to a second index using a method according to claim 1; and
(C) comparing the first and second event descriptors obtained in the steps (A) and (B).

**86.** A storage medium storing a program for implementing in a computer a process for analyzing a system using a sequence of event descriptors relating to at least one system, the process comprising the steps of:

(A) obtaining time-series data of at least one index derived from at least one system;
(B) providing at least one characteristic behavior;
(C) extracting a portion having the characteristic behavior as an event timing in the time-series data, and producing an event descriptor describing the event timing as a sequence; and
(D) analyzing the sequence.

SEQUENCE LISTING

<110> National Institute of Advanced Industrial Science and Technology, Masato MIYAKE, Tomohiro YOSHIKAWA, Jun MIYAKE

<120> Event Sequencer

<130> AI012PCT

<150> JP 2004-24923
<151> 2004-01-30

<160> 46

<170> PatentIn version 3.1

<210> 1
<211> 1929
<212> DNA
<213> Homo sapiens

<220>
<221> CDS
<222> (1)..(1929)
<223> fibronectin 1

<400> 1
```
atg ctt agg ggt ccg ggg ccc ggg ctg ctg ctg ctg gcc gtc cag tgc      48
Met Leu Arg Gly Pro Gly Pro Gly Leu Leu Leu Leu Ala Val Gln Cys
1               5                   10                  15

ctg ggg aca gcg gtg ccc tcc acg gga gcc tcg aag agc aag agg cag      96
Leu Gly Thr Ala Val Pro Ser Thr Gly Ala Ser Lys Ser Lys Arg Gln
            20                  25                  30

gct cag caa atg gtt cag ccc cag tcc ccg gtg gct gtc agt caa agc      144
Ala Gln Gln Met Val Gln Pro Gln Ser Pro Val Ala Val Ser Gln Ser
        35                  40                  45

aag ccc ggt tgt tat gac aat gga aaa cac tat cag ata aat caa cag      192
Lys Pro Gly Cys Tyr Asp Asn Gly Lys His Tyr Gln Ile Asn Gln Gln
    50                  55                  60

tgg gag cgg acc tac cta ggc aat gcg ttg gtt tgt act tgt tat gga      240
Trp Glu Arg Thr Tyr Leu Gly Asn Ala Leu Val Cys Thr Cys Tyr Gly
65                  70                  75                  80

gga agc cga ggt ttt aac tgc gag agt aaa cct gaa gct gaa gag act      288
Gly Ser Arg Gly Phe Asn Cys Glu Ser Lys Pro Glu Ala Glu Glu Thr
                85                  90                  95

tgc ttt gac aag tac act ggg aac act tac cga gtg ggt gac act tat      336
Cys Phe Asp Lys Tyr Thr Gly Asn Thr Tyr Arg Val Gly Asp Thr Tyr
            100                 105                 110

gag cgt cct aaa gac tcc atg atc tgg gac tgt acc tgc atc ggg gct      384
Glu Arg Pro Lys Asp Ser Met Ile Trp Asp Cys Thr Cys Ile Gly Ala
        115                 120                 125

ggg cga ggg aga ata agc tgt acc atc gca aac cgc tgc cat gaa ggg      432
Gly Arg Gly Arg Ile Ser Cys Thr Ile Ala Asn Arg Cys His Glu Gly
    130                 135                 140

ggt cag tcc tac aag att ggt gac acc tgg agg aga cca cat gag act      480
Gly Gln Ser Tyr Lys Ile Gly Asp Thr Trp Arg Arg Pro His Glu Thr
145                 150                 155                 160
```

```
ggt ggt tac atg tta gag tgt gtg tgt ctt ggt aat gga aaa gga gaa        528
Gly Gly Tyr Met Leu Glu Cys Val Cys Leu Gly Asn Gly Lys Gly Glu
                165                 170                 175

tgg acc tgc aag ccc ata gct gag aag tgt ttt gat cat gct gct ggg        576
Trp Thr Cys Lys Pro Ile Ala Glu Lys Cys Phe Asp His Ala Ala Gly
            180                 185                 190

act tcc tat gtg gtc gga gaa acg tgg gag aag ccc tac caa ggc tgg        624
Thr Ser Tyr Val Val Gly Glu Thr Trp Glu Lys Pro Tyr Gln Gly Trp
        195                 200                 205

atg atg gta gat tgt act tgc ctg gga gaa ggc agc gga cgc atc act        672
Met Met Val Asp Cys Thr Cys Leu Gly Glu Gly Ser Gly Arg Ile Thr
    210                 215                 220

tgc act tct aga aat aga tgc aac gat cag gac aca agg aca tcc tat        720
Cys Thr Ser Arg Asn Arg Cys Asn Asp Gln Asp Thr Arg Thr Ser Tyr
225                 230                 235                 240

aga att gga gac acc tgg agc aag aag gat aat cga gga aac ctg ctc        768
Arg Ile Gly Asp Thr Trp Ser Lys Lys Asp Asn Arg Gly Asn Leu Leu
                245                 250                 255

cag tgc atc tgc aca ggc aac ggc cga gga gag tgg aag tgt gag agg        816
Gln Cys Ile Cys Thr Gly Asn Gly Arg Gly Glu Trp Lys Cys Glu Arg
            260                 265                 270

cac acc tct gtg cag acc aca tcg agc gga tct ggc ccc ttc acc gat        864
His Thr Ser Val Gln Thr Thr Ser Ser Gly Ser Gly Pro Phe Thr Asp
        275                 280                 285

gtt cgt gca gct gtt tac caa ccg cag cct cac ccc cag cct cct ccc        912
Val Arg Ala Ala Val Tyr Gln Pro Gln Pro His Pro Gln Pro Pro Pro
    290                 295                 300

tat ggc cac tgt gtc aca gac agt ggt gtg gtc tac tct gtg ggg atg        960
Tyr Gly His Cys Val Thr Asp Ser Gly Val Val Tyr Ser Val Gly Met
305                 310                 315                 320

cag tgg ctg aag aca caa gga aat aag caa atg ctt tgc acg tgc ctg       1008
Gln Trp Leu Lys Thr Gln Gly Asn Lys Gln Met Leu Cys Thr Cys Leu
                325                 330                 335

ggc aac gga gtc agc tgc caa gag aca gct gta acc cag act tac ggt       1056
Gly Asn Gly Val Ser Cys Gln Glu Thr Ala Val Thr Gln Thr Tyr Gly
            340                 345                 350

ggc aac tca aat gga gag cca tgt gtc tta cca ttc acc tac aat ggc       1104
Gly Asn Ser Asn Gly Glu Pro Cys Val Leu Pro Phe Thr Tyr Asn Gly
        355                 360                 365

agg acg gac agc aca act tcg aat tat gag cag gac cag aaa tac tct       1152
Arg Thr Asp Ser Thr Thr Ser Asn Tyr Glu Gln Asp Gln Lys Tyr Ser
    370                 375                 380

ttc tgc aca gac cac act gtt ttg gtt cag act cga gga gga aat tcc       1200
Phe Cys Thr Asp His Thr Val Leu Val Gln Thr Arg Gly Gly Asn Ser
385                 390                 395                 400

aat ggt gcc ttg tgc cac ttc ccc ttc cta tac aac aac cac aat tac       1248
Asn Gly Ala Leu Cys His Phe Pro Phe Leu Tyr Asn Asn His Asn Tyr
                405                 410                 415

act gat tgc act tct gag ggc aga aga gac aac atg aag tgg tgt ggg       1296
Thr Asp Cys Thr Ser Glu Gly Arg Arg Asp Asn Met Lys Trp Cys Gly
            420                 425                 430
```

```
acc aca cag aac tat gat gcc gac cag aag ttt ggg ttc tgc ccc atg      1344
Thr Thr Gln Asn Tyr Asp Ala Asp Gln Lys Phe Gly Phe Cys Pro Met
        435                 440                 445

gct gcc cac gag gaa atc tgc aca acc aat gaa ggg gtc atg tac cgc      1392
Ala Ala His Glu Glu Ile Cys Thr Thr Asn Glu Gly Val Met Tyr Arg
    450                 455                 460

att gga gat cag tgg gat aag cag cat gac atg ggt cac atg atg agg      1440
Ile Gly Asp Gln Trp Asp Lys Gln His Asp Met Gly His Met Met Arg
465                 470                 475                 480

tgc acg tgt gtt ggg aat ggt cgt ggg gaa tgg aca tgc att gcc tac      1488
Cys Thr Cys Val Gly Asn Gly Arg Gly Glu Trp Thr Cys Ile Ala Tyr
                485                 490                 495

tcg cag ctt cga gat cag tgc att gtt gat gac atc act tac aat gtg      1536
Ser Gln Leu Arg Asp Gln Cys Ile Val Asp Asp Ile Thr Tyr Asn Val
            500                 505                 510

aac gac aca ttc cac aag cgt cat gaa gag ggg cac atg ctg aac tgt      1584
Asn Asp Thr Phe His Lys Arg His Glu Glu Gly His Met Leu Asn Cys
        515                 520                 525

aca tgc ttc ggt cag ggt cgg ggc agg tgg aag tgt gat ccc gtc gac      1632
Thr Cys Phe Gly Gln Gly Arg Gly Arg Trp Lys Cys Asp Pro Val Asp
    530                 535                 540

caa tgc cag gat tca gag act ggg acg ttt tat caa att gga gat tca      1680
Gln Cys Gln Asp Ser Glu Thr Gly Thr Phe Tyr Gln Ile Gly Asp Ser
545                 550                 555                 560

tgg gag aag tat gtg cat ggt gtc aga tac cag tgc tac tgc tat ggc      1728
Trp Glu Lys Tyr Val His Gly Val Arg Tyr Gln Cys Tyr Cys Tyr Gly
                565                 570                 575

cgt ggc att ggg gag tgg cat tgc caa cct tta cag acc tat cca agc      1776
Arg Gly Ile Gly Glu Trp His Cys Gln Pro Leu Gln Thr Tyr Pro Ser
            580                 585                 590

tca agt ggt cct gtc gaa gta ttt atc act gag act ccg agt cag ccc      1824
Ser Ser Gly Pro Val Glu Val Phe Ile Thr Glu Thr Pro Ser Gln Pro
        595                 600                 605

aac tcc cac ccc atc cag tgg aat gca cca cag cca tct cac att tcc      1872
Asn Ser His Pro Ile Gln Trp Asn Ala Pro Gln Pro Ser His Ile Ser
    610                 615                 620

aag tac att ctc agg tgg aga cct gtg agt atc cca ccc aga aac ctt      1920
Lys Tyr Ile Leu Arg Trp Arg Pro Val Ser Ile Pro Pro Arg Asn Leu
625                 630                 635                 640

gga tac tga                                                          1929
Gly Tyr
```

```
<210>  2
<211>  642
<212>  PRT
<213>  Homo sapiens

<400>  2

Met Leu Arg Gly Pro Gly Pro Gly Leu Leu Leu Leu Ala Val Gln Cys
1               5                   10                  15
```

Leu Gly Thr Ala Val Pro Ser Thr Gly Ala Ser Lys Ser Lys Arg Gln
20 25 30

Ala Gln Gln Met Val Gln Pro Gln Ser Pro Val Ala Val Ser Gln Ser
35 40 45

Lys Pro Gly Cys Tyr Asp Asn Gly Lys His Tyr Gln Ile Asn Gln Gln
50 55 60

Trp Glu Arg Thr Tyr Leu Gly Asn Ala Leu Val Cys Thr Cys Tyr Gly
65 70 75 80

Gly Ser Arg Gly Phe Asn Cys Glu Ser Lys Pro Glu Ala Glu Glu Thr
85 90 95

Cys Phe Asp Lys Tyr Thr Gly Asn Thr Tyr Arg Val Gly Asp Thr Tyr
100 105 110

Glu Arg Pro Lys Asp Ser Met Ile Trp Asp Cys Thr Cys Ile Gly Ala
115 120 125

Gly Arg Gly Arg Ile Ser Cys Thr Ile Ala Asn Arg Cys His Glu Gly
130 135 140

Gly Gln Ser Tyr Lys Ile Gly Asp Thr Trp Arg Arg Pro His Glu Thr
145 150 155 160

Gly Gly Tyr Met Leu Glu Cys Val Cys Leu Gly Asn Gly Lys Gly Glu
165 170 175

Trp Thr Cys Lys Pro Ile Ala Glu Lys Cys Phe Asp His Ala Ala Gly
180 185 190

Thr Ser Tyr Val Val Gly Glu Thr Trp Glu Lys Pro Tyr Gln Gly Trp
195 200 205

Met Met Val Asp Cys Thr Cys Leu Gly Glu Gly Ser Gly Arg Ile Thr
210 215 220

Cys Thr Ser Arg Asn Arg Cys Asn Asp Gln Asp Thr Arg Thr Ser Tyr
225 230 235 240

Arg Ile Gly Asp Thr Trp Ser Lys Lys Asp Asn Arg Gly Asn Leu Leu
245 250 255

Gln Cys Ile Cys Thr Gly Asn Gly Arg Gly Glu Trp Lys Cys Glu Arg
260 265 270

His Thr Ser Val Gln Thr Thr Ser Ser Gly Ser Gly Pro Phe Thr Asp
275 280 285

Val Arg Ala Ala Val Tyr Gln Pro Gln Pro His Pro Gln Pro Pro Pro
290 295 300

Tyr Gly His Cys Val Thr Asp Ser Gly Val Val Tyr Ser Val Gly Met
305 310 315 320

Gln Trp Leu Lys Thr Gln Gly Asn Lys Gln Met Leu Cys Thr Cys Leu
325 330 335

Gly Asn Gly Val Ser Cys Gln Glu Thr Ala Val Thr Gln Thr Tyr Gly
340 345 350

Gly Asn Ser Asn Gly Glu Pro Cys Val Leu Pro Phe Thr Tyr Asn Gly
355 360 365

Arg Thr Asp Ser Thr Thr Ser Asn Tyr Glu Gln Asp Gln Lys Tyr Ser
370 375 380

Phe Cys Thr Asp His Thr Val Leu Val Gln Thr Arg Gly Gly Asn Ser
385 390 395 400

Asn Gly Ala Leu Cys His Phe Pro Phe Leu Tyr Asn Asn His Asn Tyr
405 410 415

Thr Asp Cys Thr Ser Glu Gly Arg Arg Asp Asn Met Lys Trp Cys Gly
420 425 430

Thr Thr Gln Asn Tyr Asp Ala Asp Gln Lys Phe Gly Phe Cys Pro Met
435 440 445

Ala Ala His Glu Glu Ile Cys Thr Thr Asn Glu Gly Val Met Tyr Arg
450 455 460

Ile Gly Asp Gln Trp Asp Lys Gln His Asp Met Gly His Met Met Arg
465 470 475 480

Cys Thr Cys Val Gly Asn Gly Arg Gly Glu Trp Thr Cys Ile Ala Tyr
485 490 495

Ser Gln Leu Arg Asp Gln Cys Ile Val Asp Asp Ile Thr Tyr Asn Val
500 505 510

Asn Asp Thr Phe His Lys Arg His Glu Glu Gly His Met Leu Asn Cys
515 520 525

Thr Cys Phe Gly Gln Gly Arg Gly Arg Trp Lys Cys Asp Pro Val Asp
530 535 540

Gln Cys Gln Asp Ser Glu Thr Gly Thr Phe Tyr Gln Ile Gly Asp Ser
545 550 555 560

```
Trp Glu Lys Tyr Val His Gly Val Arg Tyr Gln Cys Tyr Cys Tyr Gly
                565                 570                 575

Arg Gly Ile Gly Glu Trp His Cys Gln Pro Leu Gln Thr Tyr Pro Ser
            580                 585                 590

Ser Ser Gly Pro Val Glu Val Phe Ile Thr Glu Thr Pro Ser Gln Pro
        595                 600                 605

Asn Ser His Pro Ile Gln Trp Asn Ala Pro Gln Pro Ser His Ile Ser
    610                 615                 620

Lys Tyr Ile Leu Arg Trp Arg Pro Val Ser Ile Pro Pro Arg Asn Leu
625                 630                 635                 640

Gly Tyr


<210>  3
<211>  1437
<212>  DNA
<213>  Mus musculus

<220>
<221>  CDS
<222>  (1)..(1437)
<223>  vitronectin

<400>  3
atg gca ccc ctg agg ccc ttt ttc ata cta gcc ctg gtg gca tgg gtt        48
Met Ala Pro Leu Arg Pro Phe Phe Ile Leu Ala Leu Val Ala Trp Val
1               5                   10                  15

tct ctg gct gac caa gag tca tgc aag ggc cgc tgc act cag ggt ttc        96
Ser Leu Ala Asp Gln Glu Ser Cys Lys Gly Arg Cys Thr Gln Gly Phe
            20                  25                  30

atg gcc agc aag aag tgt cag tgt gac gag ctt tgc act tac tat cag       144
Met Ala Ser Lys Lys Cys Gln Cys Asp Glu Leu Cys Thr Tyr Tyr Gln
        35                  40                  45

agc tgc tgt gcc gac tac atg gag cag tgc aag ccc caa gta acg cgg       192
Ser Cys Cys Ala Asp Tyr Met Glu Gln Cys Lys Pro Gln Val Thr Arg
    50                  55                  60

ggg gac gtg ttc act atg cca gag gat gat tat tgg agc tat gac tac       240
Gly Asp Val Phe Thr Met Pro Glu Asp Asp Tyr Trp Ser Tyr Asp Tyr
65                  70                  75                  80

gtg gag gag ccc aag aac aat acc aac acc ggt gtg caa ccc gag aac       288
Val Glu Glu Pro Lys Asn Asn Thr Asn Thr Gly Val Gln Pro Glu Asn
                85                  90                  95

acc tct cca ccc ggt gac cta aat cct cgg acg gac ggc act cta aag       336
Thr Ser Pro Pro Gly Asp Leu Asn Pro Arg Thr Asp Gly Thr Leu Lys
            100                 105                 110

ccg aca gcc ttc cta gat cct gag gaa cag cca agc acc cca gcg cct       384
Pro Thr Ala Phe Leu Asp Pro Glu Glu Gln Pro Ser Thr Pro Ala Pro
        115                 120                 125
```

```
aaa gtg gag caa cag gag gag atc cta agg ccc gac acc act gat caa      432
Lys Val Glu Gln Gln Glu Glu Ile Leu Arg Pro Asp Thr Thr Asp Gln
    130                 135                 140

ggg acc cct gag ttt cca gag gaa gaa ctg tgc agt gga aag ccc ttt      480
Gly Thr Pro Glu Phe Pro Glu Glu Glu Leu Cys Ser Gly Lys Pro Phe
145                 150                 155                 160

gac gcc ttc acg gat ctc aag aat ggg tcc ctc ttt gcc ttc cga ggg      528
Asp Ala Phe Thr Asp Leu Lys Asn Gly Ser Leu Phe Ala Phe Arg Gly
                165                 170                 175

cag tac cgc tgt gag cta gat gag acg gca gtg agg cct ggg tac ccc      576
Gln Tyr Arg Cys Glu Leu Asp Glu Thr Ala Val Arg Pro Gly Tyr Pro
            180                 185                 190

aaa ctt atc caa gat gtc tgg ggc att gag ggc ccc atc gat gct gcc      624
Lys Leu Ile Gln Asp Val Trp Gly Ile Glu Gly Pro Ile Asp Ala Ala
        195                 200                 205

ttc act cgc atc aac tgt cag ggg aag acc tac ttg ttc aag ggt agt      672
Phe Thr Arg Ile Asn Cys Gln Gly Lys Thr Tyr Leu Phe Lys Gly Ser
    210                 215                 220

cag tac tgg cgc ttt gag gat ggg gtc ctg gac cct ggt tat ccc cga      720
Gln Tyr Trp Arg Phe Glu Asp Gly Val Leu Asp Pro Gly Tyr Pro Arg
225                 230                 235                 240

aac atc tcc gaa ggc ttc agt ggc ata cca gac aat gtt gat gca gcg      768
Asn Ile Ser Glu Gly Phe Ser Gly Ile Pro Asp Asn Val Asp Ala Ala
                245                 250                 255

ttc gcc ctt cct gcc cac cgt tac agt ggc cgg gaa agg gtc tac ttc      816
Phe Ala Leu Pro Ala His Arg Tyr Ser Gly Arg Glu Arg Val Tyr Phe
            260                 265                 270

ttc aag ggg aag cag tac tgg gag cac gaa ttt cag cag caa ccc agc      864
Phe Lys Gly Lys Gln Tyr Trp Glu His Glu Phe Gln Gln Gln Pro Ser
        275                 280                 285

cag gag gag tgc gaa ggc agc tct ctg tca gcc gtg ttt gag cac ttt      912
Gln Glu Glu Cys Glu Gly Ser Ser Leu Ser Ala Val Phe Glu His Phe
    290                 295                 300

gcc ttg ctt cag cgg gac agc tgg gag aac att ttc gaa ctc ctc ttc      960
Ala Leu Leu Gln Arg Asp Ser Trp Glu Asn Ile Phe Glu Leu Leu Phe
305                 310                 315                 320

tgg ggc aga tcc tct gat gga gcc aga gaa ccc caa ttc atc agc cgg     1008
Trp Gly Arg Ser Ser Asp Gly Ala Arg Glu Pro Gln Phe Ile Ser Arg
                325                 330                 335

aac tgg cat ggt gtg cca ggg aaa gtg gac gct gct atg gcc ggc cgc     1056
Asn Trp His Gly Val Pro Gly Lys Val Asp Ala Ala Met Ala Gly Arg
            340                 345                 350

atc tac gtc act ggc tcc tta tcc cac tct gcc caa gcc aaa aaa cag     1104
Ile Tyr Val Thr Gly Ser Leu Ser His Ser Ala Gln Ala Lys Lys Gln
        355                 360                 365

ccg tct aag cgt aga agc cga aag cgc tat cgt tca cgc cga ggg cgt     1152
Pro Ser Lys Arg Arg Ser Arg Lys Arg Tyr Arg Ser Arg Arg Gly Arg
    370                 375                 380

ggc cac aga cgc agc cag agc tcg aac tcc cgt cgt tca tca cgt tca     1200
Gly His Arg Arg Ser Gln Ser Ser Asn Ser Arg Arg Ser Ser Arg Ser
385                 390                 395                 400
```

```
atc tgg ttc tct ttg ttc tcc agc gag gag agt ggg cta gga acc tac      1248
Ile Trp Phe Ser Leu Phe Ser Ser Glu Glu Ser Gly Leu Gly Thr Tyr
                405                 410                 415

aac aac tat gat tat gat atg gac tgg ctt gta cct gcc acc tgc gag      1296
Asn Asn Tyr Asp Tyr Asp Met Asp Trp Leu Val Pro Ala Thr Cys Glu
            420                 425                 430

ccc att cag agc gtc tat ttc ttc tct gga gac aaa tac tac cga gtc      1344
Pro Ile Gln Ser Val Tyr Phe Phe Ser Gly Asp Lys Tyr Tyr Arg Val
        435                 440                 445

aac ctt aga acc cgg cga gtg gac tct gtg aat cct ccc tac cca cgc      1392
Asn Leu Arg Thr Arg Arg Val Asp Ser Val Asn Pro Pro Tyr Pro Arg
    450                 455                 460

tcc att gct cag tat tgg ctg ggc tgc ccg acc tct gag aag tag          1437
Ser Ile Ala Gln Tyr Trp Leu Gly Cys Pro Thr Ser Glu Lys
465                 470                 475


<210>  4
<211>  478
<212>  PRT
<213>  Mus musculus

<400>  4

Met Ala Pro Leu Arg Pro Phe Phe Ile Leu Ala Leu Val Ala Trp Val
1               5                   10                  15


Ser Leu Ala Asp Gln Glu Ser Cys Lys Gly Arg Cys Thr Gln Gly Phe
            20                  25                  30


Met Ala Ser Lys Lys Cys Gln Cys Asp Glu Leu Cys Thr Tyr Tyr Gln
        35                  40                  45


Ser Cys Cys Ala Asp Tyr Met Glu Gln Cys Lys Pro Gln Val Thr Arg
    50                  55                  60


Gly Asp Val Phe Thr Met Pro Glu Asp Asp Tyr Trp Ser Tyr Asp Tyr
65                  70                  75                  80


Val Glu Glu Pro Lys Asn Asn Thr Asn Thr Gly Val Gln Pro Glu Asn

                85                  90                  95


Thr Ser Pro Pro Gly Asp Leu Asn Pro Arg Thr Asp Gly Thr Leu Lys
            100                 105                 110


Pro Thr Ala Phe Leu Asp Pro Glu Glu Gln Pro Ser Thr Pro Ala Pro
        115                 120                 125


Lys Val Glu Gln Gln Glu Glu Ile Leu Arg Pro Asp Thr Thr Asp Gln
    130                 135                 140


Gly Thr Pro Glu Phe Pro Glu Glu Glu Leu Cys Ser Gly Lys Pro Phe
145                 150                 155                 160
```

Asp Ala Phe Thr Asp Leu Lys Asn Gly Ser Leu Phe Ala Phe Arg Gly
165 170 175

Gln Tyr Arg Cys Glu Leu Asp Glu Thr Ala Val Arg Pro Gly Tyr Pro
180 185 190

Lys Leu Ile Gln Asp Val Trp Gly Ile Glu Gly Pro Ile Asp Ala Ala
195 200 205

Phe Thr Arg Ile Asn Cys Gln Gly Lys Thr Tyr Leu Phe Lys Gly Ser
210 215 220

Gln Tyr Trp Arg Phe Glu Asp Gly Val Leu Asp Pro Gly Tyr Pro Arg
225 230 235 240

Asn Ile Ser Glu Gly Phe Ser Gly Ile Pro Asp Asn Val Asp Ala Ala
245 250 255

Phe Ala Leu Pro Ala His Arg Tyr Ser Gly Arg Glu Arg Val Tyr Phe
260 265 270

Phe Lys Gly Lys Gln Tyr Trp Glu His Glu Phe Gln Gln Gln Pro Ser
275 280 285

Gln Glu Glu Cys Glu Gly Ser Ser Leu Ser Ala Val Phe Glu His Phe
290 295 300

Ala Leu Leu Gln Arg Asp Ser Trp Glu Asn Ile Phe Glu Leu Leu Phe
305 310 315 320

Trp Gly Arg Ser Ser Asp Gly Ala Arg Glu Pro Gln Phe Ile Ser Arg
325 330 335

Asn Trp His Gly Val Pro Gly Lys Val Asp Ala Ala Met Ala Gly Arg
340 345 350

Ile Tyr Val Thr Gly Ser Leu Ser His Ser Ala Gln Ala Lys Lys Gln
355 360 365

Pro Ser Lys Arg Arg Ser Arg Lys Arg Tyr Arg Ser Arg Arg Gly Arg
370 375 380

Gly His Arg Arg Ser Gln Ser Ser Asn Ser Arg Arg Ser Ser Arg Ser
385 390 395 400

Ile Trp Phe Ser Leu Phe Ser Ser Glu Glu Ser Gly Leu Gly Thr Tyr
405 410 415

Asn Asn Tyr Asp Tyr Asp Met Asp Trp Leu Val Pro Ala Thr Cys Glu
420 425 430

Pro Ile Gln Ser Val Tyr Phe Phe Ser Gly Asp Lys Tyr Tyr Arg Val
435 440 445

Asn Leu Arg Thr Arg Arg Val Asp Ser Val Asn Pro Pro Tyr Pro Arg
450 455 460

Ser Ile Ala Gln Tyr Trp Leu Gly Cys Pro Thr Ser Glu Lys
465 470 475

<210> 5

<211> 9511
<212> DNA
<213> Mus musculus

<220>
<221> CDS
<222> (121)..(9372)
<223> laminin-2 alpha chain

<400> 5
ggcacgagct gcaactccgt gggctccggg aggagtggat ctgctccggc caggatgcct 60

gcggccaccg ccgggatcct cttgctcctg ctcttgggga cgctcgaagg ctcccagact 120

cag cgg cga cag tcc caa gcg cat caa cag aga ggt tta ttt cct gct 168
Gln Arg Arg Gln Ser Gln Ala His Gln Gln Arg Gly Leu Phe Pro Ala
1 5 10 15

gtc ctg aat ctt gct tcg aat gca ctc atc aca acc aat gct aca tgt 216
Val Leu Asn Leu Ala Ser Asn Ala Leu Ile Thr Thr Asn Ala Thr Cys
20 25 30

ggg gaa aaa gga ccc gag atg tac tgc aag ttg gtg gaa cat gtc ccc 264
Gly Glu Lys Gly Pro Glu Met Tyr Cys Lys Leu Val Glu His Val Pro
35 40 45

ggg cag cct gtg agg aac cct cag tgc cga atc tgc aat cag aac agc 312
Gly Gln Pro Val Arg Asn Pro Gln Cys Arg Ile Cys Asn Gln Asn Ser
50 55 60

agc aat cca tac cag agg cac ccg att acg aat gct att gat ggc aag 360
Ser Asn Pro Tyr Gln Arg His Pro Ile Thr Asn Ala Ile Asp Gly Lys
65 70 75 80

aac aca tgg tgg cag agt ccc agt atc aag aat gga gtg gaa tac cat 408
Asn Thr Trp Trp Gln Ser Pro Ser Ile Lys Asn Gly Val Glu Tyr His
85 90 95

tat gtg aca att act ctg gat tta cag cag gtg ttc cag att gcc tac 456
Tyr Val Thr Ile Thr Leu Asp Leu Gln Gln Val Phe Gln Ile Ala Tyr
100 105 110

gta att gtg aag gca gcc aat tcc cct cgg cct gga aac tgg att ttg 504
Val Ile Val Lys Ala Ala Asn Ser Pro Arg Pro Gly Asn Trp Ile Leu
115 120 125

gaa cgt tcc ctg gat gac gtg gag tac aaa ccc tgg cag tat cat gcg 552
Glu Arg Ser Leu Asp Asp Val Glu Tyr Lys Pro Trp Gln Tyr His Ala
130 135 140

gtg aca gac acg gag tgc ctg acc ctc tac aat atc tat ccc cgc act 600

Val Thr Asp Thr Glu Cys Leu Thr Leu Tyr Asn Ile Tyr Pro Arg Thr
145 150 155 160

gga cca cca tcc tac gcc aaa gat gat gag gtc atc tgc act tca ttt 648
Gly Pro Pro Ser Tyr Ala Lys Asp Asp Glu Val Ile Cys Thr Ser Phe
165 170 175

tat tcg aag atc cac cct tta gaa aat gga gag att cac att tct ttg 696
Tyr Ser Lys Ile His Pro Leu Glu Asn Gly Glu Ile His Ile Ser Leu
180 185 190

atc aat ggg aga cca agt gct gat gac ccc tcc cct gaa ctc ctg gaa 744
Ile Asn Gly Arg Pro Ser Ala Asp Asp Pro Ser Pro Glu Leu Leu Glu
195 200 205

ttc acc tct gct cgc tac att cgc ctg aga ttt cag agg atc cgc acc 792
Phe Thr Ser Ala Arg Tyr Ile Arg Leu Arg Phe Gln Arg Ile Arg Thr
210 215 220

ttg aat gca gac ttg atg atg ttt gct cac aaa gac ccc aga gaa atc 840
Leu Asn Ala Asp Leu Met Met Phe Ala His Lys Asp Pro Arg Glu Ile
225 230 235 240

gat ccc att gtc aca cga aga tat tac tat tct gtc aag gat att tca 888
Asp Pro Ile Val Thr Arg Arg Tyr Tyr Tyr Ser Val Lys Asp Ile Ser
245 250 255

gtt ggc ggg atg tgc atc tgt tat ggt cat gcc cgg gct tgt cca ctt 936
Val Gly Gly Met Cys Ile Cys Tyr Gly His Ala Arg Ala Cys Pro Leu
260 265 270

gac cct gca aca aat aaa tca cgc tgt gag tgt gaa cat aac acc tgt 984
Asp Pro Ala Thr Asn Lys Ser Arg Cys Glu Cys Glu His Asn Thr Cys
275 280 285

ggg gaa agc tgt gac agg tgc tgt cca gga ttc cat cag aag cct tgg 1032
Gly Glu Ser Cys Asp Arg Cys Cys Pro Gly Phe His Gln Lys Pro Trp
290 295 300

aga gct gga acc ttc ctc acc aag tct gag tgt gaa gca tgc aat tgt 1080
Arg Ala Gly Thr Phe Leu Thr Lys Ser Glu Cys Glu Ala Cys Asn Cys
305 310 315 320

cac gga aaa gct gag gaa tgc tat tat gat gaa act gtt gct agc aga 1128
His Gly Lys Ala Glu Glu Cys Tyr Tyr Asp Glu Thr Val Ala Ser Arg
325 330 335

aat cta agt tta aat ata cat ggg aag tac atc gga ggg ggt gtg tgc 1176
Asn Leu Ser Leu Asn Ile His Gly Lys Tyr Ile Gly Gly Gly Val Cys
340 345 350

atc aac tgc aca cat aac acg gct ggg ata aat tgt gag aca tgt gtt 1224
Ile Asn Cys Thr His Asn Thr Ala Gly Ile Asn Cys Glu Thr Cys Val
355 360 365

gat gga ttc ttc aga ccc aaa ggg gtg tca cca aat tat cca aga cca 1272
Asp Gly Phe Phe Arg Pro Lys Gly Val Ser Pro Asn Tyr Pro Arg Pro
370 375 380

tgc cag cca tgt cac tgt gat cca act ggc tcc ctt agt gaa gtc tgt 1320
Cys Gln Pro Cys His Cys Asp Pro Thr Gly Ser Leu Ser Glu Val Cys
385 390 395 400

gtc aaa gat gag aaa tac gcc cag cga ggg ttg aaa cct gga tcc tgt 1368
Val Lys Asp Glu Lys Tyr Ala Gln Arg Gly Leu Lys Pro Gly Ser Cys
405 410 415

cac tgc aaa act ggc ttt gga ggc gtg aac tgt gat cgc tgt gtc agg 1416

```
His Cys Lys Thr Gly Phe Gly Gly Val Asn Cys Asp Arg Cys Val Arg
            420                 425                 430

ggt tac cat ggt tac cca gac tgc caa ccc tgt aac tgt agt ggc ttg      1464
Gly Tyr His Gly Tyr Pro Asp Cys Gln Pro Cys Asn Cys Ser Gly Leu
        435                 440                 445

ggg agc aca aat gag gac cct tgc gtt ggg ccc tgt agc tgt aag gag      1512
Gly Ser Thr Asn Glu Asp Pro Cys Val Gly Pro Cys Ser Cys Lys Glu
    450                 455                 460

aat gtt gaa ggt gaa gac tgt agt cgt tgc aaa tct ggt ttc ttc aac      1560
Asn Val Glu Gly Glu Asp Cys Ser Arg Cys Lys Ser Gly Phe Phe Asn
465                 470                 475                 480

ttg caa gaa gat aat cag aaa ggc tgt gag gag tgt ttc tgt tca gga      1608
Leu Gln Glu Asp Asn Gln Lys Gly Cys Glu Glu Cys Phe Cys Ser Gly
                485                 490                 495

gta tca aac aga tgt cag agt tcc tac tgg acc tat ggg aat att caa      1656
Val Ser Asn Arg Cys Gln Ser Ser Tyr Trp Thr Tyr Gly Asn Ile Gln
            500                 505                 510

gac atg cgt ggt tgg tat ctc aca gac ctc tct ggc cgc att cgg atg      1704
Asp Met Arg Gly Trp Tyr Leu Thr Asp Leu Ser Gly Arg Ile Arg Met
        515                 520                 525

gct ccc cag ctt gat aac cct gac tca cct cag cag atc agc atc agt      1752
Ala Pro Gln Leu Asp Asn Pro Asp Ser Pro Gln Gln Ile Ser Ile Ser
    530                 535                 540

aac tct gag gcc cgg aaa tcc ctg ctt gat ggt tac tac tgg agt gca      1800
Asn Ser Glu Ala Arg Lys Ser Leu Leu Asp Gly Tyr Tyr Trp Ser Ala
545                 550                 555                 560

ccg cct cca tat ctg gga aac aga ctt cca gct gtt ggg gga cag ttg      1848
Pro Pro Pro Tyr Leu Gly Asn Arg Leu Pro Ala Val Gly Gly Gln Leu
                565                 570                 575

tca ttt acc atc tca tat gac ctc gaa gaa gag gaa gac gat aca gaa      1896
Ser Phe Thr Ile Ser Tyr Asp Leu Glu Glu Glu Glu Asp Asp Thr Glu
            580                 585                 590

aaa ctc ctt cag ctg atg att atc ttt gag gga aat gac tta aga atc      1944
Lys Leu Leu Gln Leu Met Ile Ile Phe Glu Gly Asn Asp Leu Arg Ile
        595                 600                 605

agc aca gcg tat aag gag gtg tac tta gag cca tct gaa gaa cac gtt      1992
Ser Thr Ala Tyr Lys Glu Val Tyr Leu Glu Pro Ser Glu Glu His Val
    610                 615                 620

gag gag gtg tca ctc aaa gaa gag gcc ttt act ata cat gga aca aat      2040
Glu Glu Val Ser Leu Lys Glu Glu Ala Phe Thr Ile His Gly Thr Asn
625                 630                 635                 640

ttg cca gtc act aga aaa gat ttc atg att gtt ctc aca aat ttg gga      2088
Leu Pro Val Thr Arg Lys Asp Phe Met Ile Val Leu Thr Asn Leu Gly
                645                 650                 655

gag atc ctt atc caa atc aca tac aac tta ggg atg gac gcc atc ttc      2136
Glu Ile Leu Ile Gln Ile Thr Tyr Asn Leu Gly Met Asp Ala Ile Phe
            660                 665                 670

agg ctg agt tct gtc aat ctt gaa tct cct gtc cct tat cct act gat      2184
Arg Leu Ser Ser Val Asn Leu Glu Ser Pro Val Pro Tyr Pro Thr Asp
        675                 680                 685

aga cgt att gca act gat gtg gaa gtt tgc cag tgt cca cct ggg tac      2232
```

Arg Arg Ile Ala Thr Asp Val Glu Val Cys Gln Cys Pro Pro Gly Tyr
690 695 700

agt ggc agc tct tgt gaa aca tgt tgg cct agg cac cga aga gtt aac 2280
Ser Gly Ser Ser Cys Glu Thr Cys Trp Pro Arg His Arg Arg Val Asn
705 710 715 720

ggc acc att ttt ggt ggc att tgt gaa cca tgt cag tgc ttt gct cat 2328
Gly Thr Ile Phe Gly Gly Ile Cys Glu Pro Cys Gln Cys Phe Ala His
725 730 735

gca gaa gcc tgt gat gac atc aca gga gaa tgt ctg aac tgt aag gat 2376
Ala Glu Ala Cys Asp Asp Ile Thr Gly Glu Cys Leu Asn Cys Lys Asp
740 745 750

cac aca ggt ggg ccg tac tgc aat gaa tgt ctc cct gga ttc tat ggt 2424
His Thr Gly Gly Pro Tyr Cys Asn Glu Cys Leu Pro Gly Phe Tyr Gly
755 760 765

gat cct act cga gga agc cct gaa gac tgt cag ccc tgt gcc tgt cca 2472
Asp Pro Thr Arg Gly Ser Pro Glu Asp Cys Gln Pro Cys Ala Cys Pro
770 775 780

ctc aat atc cca tca aat aac ttt agt cca aca tgc cat tta gac cgg 2520
Leu Asn Ile Pro Ser Asn Asn Phe Ser Pro Thr Cys His Leu Asp Arg
785 790 795 800

agt ctg gga ttg atc tgt gac gag tgt cct att ggg tac aca gga ccg 2568
Ser Leu Gly Leu Ile Cys Asp Glu Cys Pro Ile Gly Tyr Thr Gly Pro
805 810 815

cgc tgt gag agg tgt gca gaa ggc tat ttt gga caa cct tcc gta cct 2616
Arg Cys Glu Arg Cys Ala Glu Gly Tyr Phe Gly Gln Pro Ser Val Pro
820 825 830

gga gga tca tgt cag cca tgc caa tgc aat gac aac ctt gac tac tcc 2664
Gly Gly Ser Cys Gln Pro Cys Gln Cys Asn Asp Asn Leu Asp Tyr Ser
835 840 845

atc cct ggc agc tgt gac agc ctg tct ggc tcc tgt ctg att tgt aag 2712
Ile Pro Gly Ser Cys Asp Ser Leu Ser Gly Ser Cys Leu Ile Cys Lys
850 855 860

cca ggt aca aca ggc cgg tac tgt gag ctc tgt gct gat ggg tat ttt 2760
Pro Gly Thr Thr Gly Arg Tyr Cys Glu Leu Cys Ala Asp Gly Tyr Phe
865 870 875 880

gga gac gcg gtt aat aca aag aac tgt caa cca tgc cgt tgt gat atc 2808
Gly Asp Ala Val Asn Thr Lys Asn Cys Gln Pro Cys Arg Cys Asp Ile
885 890 895

aat ggc tcc ttc tca gag gat tgt cac aca aga act ggg caa tgt gag 2856
Asn Gly Ser Phe Ser Glu Asp Cys His Thr Arg Thr Gly Gln Cys Glu
900 905 910

tgc aga ccc aat gtt cag ggg cgg cac tgt gac gag tgt aag cct gaa 2904
Cys Arg Pro Asn Val Gln Gly Arg His Cys Asp Glu Cys Lys Pro Glu
915 920 925

acc ttt ggc ctg caa ctg gga agg ggt tgt ctg ccc tgc aac tgc aat 2952
Thr Phe Gly Leu Gln Leu Gly Arg Gly Cys Leu Pro Cys Asn Cys Asn
930 935 940

tct ttt ggg tct aag tcc ttt gac tgt gaa gca agt ggg cag tgc tgg 3000
Ser Phe Gly Ser Lys Ser Phe Asp Cys Glu Ala Ser Gly Gln Cys Trp
945 950 955 960

tgc cag cct gga gta gca ggg aag aaa tgt gac cgt tgt gcc cat ggc 3048

```
Cys Gln Pro Gly Val Ala Gly Lys Lys Cys Asp Arg Cys Ala His Gly
                965                 970                 975

tac ttc aac ttc caa gaa gga ggc tgc ata gct tgt gac tgt tct cat      3096
Tyr Phe Asn Phe Gln Glu Gly Gly Cys Ile Ala Cys Asp Cys Ser His
            980                 985                 990

ctg ggc aac aac tgt gac cca aaa  act ggc caa tgc att  tgc cca ccc    3144
Leu Gly Asn Asn Cys Asp Pro Lys  Thr Gly Gln Cys Ile  Cys Pro Pro
        995                 1000                 1005

aat acc  act gga gaa aag tgt  tct gag tgt ctt ccc  aac acc tgg      3189
Asn Thr  Thr Gly Glu Lys Cys  Ser Glu Cys Leu Pro  Asn Thr Trp
    1010                 1015                 1020

ggt cac  agc att gtc acc ggc  tgt aag gtt tgt aac  tgc agc act      3234
Gly His  Ser Ile Val Thr Gly  Cys Lys Val Cys Asn  Cys Ser Thr
    1025                 1030                 1035

gtg ggg  tcc ttg gct tct cag  tgc aat gta aac acg  ggc cag tgc      3279
Val Gly  Ser Leu Ala Ser Gln  Cys Asn Val Asn Thr  Gly Gln Cys
    1040                 1045                 1050

agc tgt  cat cca aaa ttc tct  ggt atg aaa tgc tca  gag tgc agc      3324
Ser Cys  His Pro Lys Phe Ser  Gly Met Lys Cys Ser  Glu Cys Ser
    1055                 1060                 1065

cga ggt  cac tgg aac tat cct  ctc tgc act cta tgt  gac tgc ttc      3369
Arg Gly  His Trp Asn Tyr Pro  Leu Cys Thr Leu Cys  Asp Cys Phe
    1070                 1075                 1080

ctt cca  ggc aca gat gcc acg  act tgt gat ctg gag  act agg aaa      3414
Leu Pro  Gly Thr Asp Ala Thr  Thr Cys Asp Leu Glu  Thr Arg Lys
    1085                 1090                 1095

tgc tcc  tgt agt gat caa act  gga cag tgc agc tgt  aag gtg aat      3459
Cys Ser  Cys Ser Asp Gln Thr  Gly Gln Cys Ser Cys  Lys Val Asn
    1100                 1105                 1110

gtg gaa  ggc gtc cac tgt gac  agg tgc cgg cct ggc  aaa ttt gga      3504
Val Glu  Gly Val His Cys Asp  Arg Cys Arg Pro Gly  Lys Phe Gly
    1115                 1120                 1125

cta gat  gcc aag aac cca ctt  ggc tgc agc agc tgc  tac tgc ttt      3549
Leu Asp  Ala Lys Asn Pro Leu  Gly Cys Ser Ser Cys  Tyr Cys Phe
    1130                 1135                 1140

gga gtt  act agt caa tgc tct  gaa gca aag ggg ctg  atc cgt acg      3594
Gly Val  Thr Ser Gln Cys Ser  Glu Ala Lys Gly Leu  Ile Arg Thr
    1145                 1150                 1155

tgg gtg  act ttg agt gat gaa  cag acc att cta cct  ctg gtg gat      3639
Trp Val  Thr Leu Ser Asp Glu  Gln Thr Ile Leu Pro  Leu Val Asp
    1160                 1165                 1170

gag gcc  ctg cag cac acg act  acc aaa ggc att gct  ttc cag aaa      3684
Glu Ala  Leu Gln His Thr Thr  Thr Lys Gly Ile Ala  Phe Gln Lys
    1175                 1180                 1185

cca gag  att gtt gca aag atg  gat gaa gtc agg caa  gag ctc cat      3729
Pro Glu  Ile Val Ala Lys Met  Asp Glu Val Arg Gln  Glu Leu His
    1190                 1195                 1200

ttg gaa  cct ttt tac tgg aaa  ctc cca caa caa ttt  gaa ggg aaa      3774
Leu Glu  Pro Phe Tyr Trp Lys  Leu Pro Gln Gln Phe  Glu Gly Lys
    1205                 1210                 1215

aag ttg  atg gct tat ggt ggc  aaa ctc aag tat gcc  atc tat ttt      3819
```

```
Lys Leu Met Ala Tyr Gly Gly Lys Leu Lys Tyr Ala Ile Tyr Phe
    1220                1225                1230

gag gct cgg gat gag aca ggc ttt gcc aca tat aaa cct caa gtt      3864
Glu Ala Arg Asp Glu Thr Gly Phe Ala Thr Tyr Lys Pro Gln Val
    1235                1240                1245

atc att cga ggt gga act cct act cat gct aga att att acc aga      3909
Ile Ile Arg Gly Gly Thr Pro Thr His Ala Arg Ile Ile Thr Arg
    1250                1255                1260

cac atg gct gcc cct ctc att ggc cag ttg aca cgg cat gaa ata      3954
His Met Ala Ala Pro Leu Ile Gly Gln Leu Thr Arg His Glu Ile
    1265                1270                1275

gaa atg aca gag aaa gaa tgg aaa tat tat ggt gat gat cct cga      3999
Glu Met Thr Glu Lys Glu Trp Lys Tyr Tyr Gly Asp Asp Pro Arg
    1280                1285                1290

atc agt aga act gtg acc cgt gaa gac ttc ttg gat ata cta tat      4044
Ile Ser Arg Thr Val Thr Arg Glu Asp Phe Leu Asp Ile Leu Tyr
    1295                1300                1305

gat att cac tat atc ctt atc aag gct act tat gga aac gtt gtg      4089
Asp Ile His Tyr Ile Leu Ile Lys Ala Thr Tyr Gly Asn Val Val
    1310                1315                1320

aga caa agc cgc att tct gaa atc tcc atg gaa gta gct gaa cca      4134
Arg Gln Ser Arg Ile Ser Glu Ile Ser Met Glu Val Ala Glu Pro
    1325                1330                1335

gga cat gta tta gca ggg agc cca cca gca cac ttg ata gaa aga      4179
Gly His Val Leu Ala Gly Ser Pro Pro Ala His Leu Ile Glu Arg
    1340                1345                1350

tgc gat tgc cct cct ggc tat tct ggc ttg tct tgt gag acg tgt      4224
Cys Asp Cys Pro Pro Gly Tyr Ser Gly Leu Ser Cys Glu Thr Cys
    1355                1360                1365

gca cca gga ttt tac cga ctt cgt tct gaa cca ggt ggg cgg act      4269
Ala Pro Gly Phe Tyr Arg Leu Arg Ser Glu Pro Gly Gly Arg Thr
    1370                1375                1380

cct gga cca acc tta ggg acc tgt gtt ccc tgc caa tgt aat gga      4314
Pro Gly Pro Thr Leu Gly Thr Cys Val Pro Cys Gln Cys Asn Gly
    1385                1390                1395

cac agc agt cag tgt gat cct gag acc tca gta tgc cag aat tgt      4359
His Ser Ser Gln Cys Asp Pro Glu Thr Ser Val Cys Gln Asn Cys
    1400                1405                1410

cag cat cac act gct ggt gac ttc tgt gag cgc tgt gcc ctt ggc      4404
Gln His His Thr Ala Gly Asp Phe Cys Glu Arg Cys Ala Leu Gly
    1415                1420                1425

tac tat gga atc gtc agg gga ttg cca aat gac tgc caa cca tgt      4449
Tyr Tyr Gly Ile Val Arg Gly Leu Pro Asn Asp Cys Gln Pro Cys
    1430                1435                1440

gct tgt cct ctg att tcg ccc agc aac aat ttc agc ccc tct tgt      4494
Ala Cys Pro Leu Ile Ser Pro Ser Asn Asn Phe Ser Pro Ser Cys
    1445                1450                1455

gta ttg gaa ggt ctg gaa gat tac cgt tgc acc gcc tgc cca agg      4539
Val Leu Glu Gly Leu Glu Asp Tyr Arg Cys Thr Ala Cys Pro Arg
    1460                1465                1470

ggc tat gaa gga cag tac tgt gaa agg tgt gcc cca ggc tat act      4584
```

```
Gly Tyr Glu Gly Gln Tyr Cys Glu Arg Cys Ala Pro Gly Tyr Thr
    1475                1480                1485

ggc agc cca agc agc ccc gga ggc tcc tgc caa gaa tgt gag tgt      4629
Gly Ser Pro Ser Ser Pro Gly Gly Ser Cys Gln Glu Cys Glu Cys
    1490                1495                1500

gac cct tat ggc tcc cta ccg gtt ccc tgt gac cgg gtc aca gga      4674
Asp Pro Tyr Gly Ser Leu Pro Val Pro Cys Asp Arg Val Thr Gly
    1505                1510                1515

ctc tgc acg tgc cgc cct gga gcc aca gga agg aag tgt gat ggc      4719
Leu Cys Thr Cys Arg Pro Gly Ala Thr Gly Arg Lys Cys Asp Gly
    1520                1525                1530

tgc gag cac tgg cat gca cgc gag ggt gca gag tgt gtc ttt tgt      4764
Cys Glu His Trp His Ala Arg Glu Gly Ala Glu Cys Val Phe Cys
    1535                1540                1545

gga gac gag tgt aca ggc ctt ctt ctt ggt gac ctg gct cgt cta      4809
Gly Asp Glu Cys Thr Gly Leu Leu Leu Gly Asp Leu Ala Arg Leu
    1550                1555                1560

gag cag atg acc atg aac atc aac ctc acg ggc cca ctg cct gct      4854
Glu Gln Met Thr Met Asn Ile Asn Leu Thr Gly Pro Leu Pro Ala
    1565                1570                1575

cca tat aaa att ctg tat ggt ctt gaa aat aca act cag gaa ctc      4899
Pro Tyr Lys Ile Leu Tyr Gly Leu Glu Asn Thr Thr Gln Glu Leu
    1580                1585                1590

aag cac ctg cta tca ccg caa cgg gca cca gag agg ctc att cag      4944
Lys His Leu Leu Ser Pro Gln Arg Ala Pro Glu Arg Leu Ile Gln
    1595                1600                1605

ttg gca gag ggc aac gtg aac aca ctt gtg atg gaa aca aat gag      4989
Leu Ala Glu Gly Asn Val Asn Thr Leu Val Met Glu Thr Asn Glu
    1610                1615                1620

ctg cta acc aga gca acc aaa gtg aca gca gat ggt gag caa aca      5034
Leu Leu Thr Arg Ala Thr Lys Val Thr Ala Asp Gly Glu Gln Thr
    1625                1630                1635

gga caa gat gct gag agg acc aac tcc aga gca gaa tcc ttg gaa      5079
Gly Gln Asp Ala Glu Arg Thr Asn Ser Arg Ala Glu Ser Leu Glu
    1640                1645                1650

gaa ttc att aaa ggg ctt gtc cag gat gct gaa gcc ata aat gaa      5124
Glu Phe Ile Lys Gly Leu Val Gln Asp Ala Glu Ala Ile Asn Glu
    1655                1660                1665

aaa gct gta aaa cta aat gaa acc tta gga aat caa gat aag aca      5169
Lys Ala Val Lys Leu Asn Glu Thr Leu Gly Asn Gln Asp Lys Thr
    1670                1675                1680

gca gag aga aac ttg gag gag ctt caa aag gaa atc gac cgg atg      5214
Ala Glu Arg Asn Leu Glu Glu Leu Gln Lys Glu Ile Asp Arg Met
    1685                1690                1695

ctg aag gaa ctg aga agt aaa gat ctt caa aca cag aag gaa gtt      5259
Leu Lys Glu Leu Arg Ser Lys Asp Leu Gln Thr Gln Lys Glu Val
    1700                1705                1710

gct gag gat gag ctc gtg gca gca gaa ggc ctt ctg aag aga gta      5304
Ala Glu Asp Glu Leu Val Ala Ala Glu Gly Leu Leu Lys Arg Val
    1715                1720                1725

aac aag ctg ttt gga gag ccc aga gcc cag aat gaa gat atg gaa      5349
```

```
Asn Lys  Leu Phe Gly Glu Pro  Arg Ala Gln Asn Glu  Asp Met Glu
    1730                 1735                 1740

aag gat  ctc cag cag aaa ctg  gca gag tac aag aac  aaa ctt gat     5394
Lys Asp  Leu Gln Gln Lys Leu  Ala Glu Tyr Lys Asn  Lys Leu Asp
    1745                 1750                 1755

gat gct  tgg gat cta ttg aga  gaa gcc act gat aaa  acc cga gat     5439
Asp Ala  Trp Asp Leu Leu Arg  Glu Ala Thr Asp Lys  Thr Arg Asp
    1760                 1765                 1770

gct aat  cgt ttg tct gct gcc  aat caa aaa aac atg  acc ata ctg     5484
Ala Asn  Arg Leu Ser Ala Ala  Asn Gln Lys Asn Met  Thr Ile Leu
    1775                 1780                 1785

gag aca  aag aag gag gct att  gaa ggt agc aaa cga  caa ata gag     5529
Glu Thr  Lys Lys Glu Ala Ile  Glu Gly Ser Lys Arg  Gln Ile Glu
    1790                 1795                 1800

aac act  tta aag gaa ggc aat  gac atc ctt gat gaa  gcc aat caa     5574
Asn Thr  Leu Lys Glu Gly Asn  Asp Ile Leu Asp Glu  Ala Asn Gln
    1805                 1810                 1815

ctc tta  ggt gaa atc aac tca  gtc ata gat tat gtc  gac gac att     5619
Leu Leu  Gly Glu Ile Asn Ser  Val Ile Asp Tyr Val  Asp Asp Ile
    1820                 1825                 1830

aaa act  aag ttg cca cca atg  tcc gag gag ctg agt  gac aaa ata     5664
Lys Thr  Lys Leu Pro Pro Met  Ser Glu Glu Leu Ser  Asp Lys Ile
    1835                 1840                 1845

gat gac  ctc gcc cag gaa ata  aag gac aga agg ctt  gct gag aag     5709
Asp Asp  Leu Ala Gln Glu Ile  Lys Asp Arg Arg Leu  Ala Glu Lys
    1850                 1855                 1860

gtg ttc  cag gct gag agc cat  gct gct cag ctg aac  gac tcg tct     5754
Val Phe  Gln Ala Glu Ser His  Ala Ala Gln Leu Asn  Asp Ser Ser
    1865                 1870                 1875

gct gta  ctt gat gga atc ctg  gat gag gct aag aac  atc tct ttc     5799
Ala Val  Leu Asp Gly Ile Leu  Asp Glu Ala Lys Asn  Ile Ser Phe
    1880                 1885                 1890

aat gcc  acg gca gcc ttc aga  gct tac agt aat att  aaa gac tac     5844
Asn Ala  Thr Ala Ala Phe Arg  Ala Tyr Ser Asn Ile  Lys Asp Tyr
    1895                 1900                 1905

att gat  gaa gct gag aaa gtg  gcc aga gaa gcc aaa  gag ctt gcc     5889
Ile Asp  Glu Ala Glu Lys Val  Ala Arg Glu Ala Lys  Glu Leu Ala
    1910                 1915                 1920

caa ggg  gct aca aaa ctg gca  aca agt cct cag ggc  tta tta aaa     5934
Gln Gly  Ala Thr Lys Leu Ala  Thr Ser Pro Gln Gly  Leu Leu Lys
    1925                 1930                 1935

gaa gat  gcc aaa ggc tcc ctt  cag aaa agc ttc agg  atc ctc aat     5979
Glu Asp  Ala Lys Gly Ser Leu  Gln Lys Ser Phe Arg  Ile Leu Asn
    1940                 1945                 1950

gaa gcc  aag aag cta gca aac  gat gtg aaa gga aat  cac aat gat     6024
Glu Ala  Lys Lys Leu Ala Asn  Asp Val Lys Gly Asn  His Asn Asp
    1955                 1960                 1965

cta aat  gac ctg aaa acc agg  tta gaa act gct gac  ctt aga aac     6069
Leu Asn  Asp Leu Lys Thr Arg  Leu Glu Thr Ala Asp  Leu Arg Asn
    1970                 1975                 1980

agt gga  ctt cta gga gct cta  aat gac acc atg gac  aag tta tca     6114
```

```
Ser Gly Leu Leu Gly Ala Leu Asn Asp Thr Met Asp Lys Leu Ser
    1985                1990                1995

gcc att aca aat gac acg gct gct aaa ctg cag gct gtc aaa gag      6159
Ala Ile Thr Asn Asp Thr Ala Ala Lys Leu Gln Ala Val Lys Glu
    2000                2005                2010

aaa gcc aga gaa gcc aat gac aca gca aaa gct gtc ctg gcc cag      6204
Lys Ala Arg Glu Ala Asn Asp Thr Ala Lys Ala Val Leu Ala Gln
    2015                2020                2025

gtt aag gac ctg cat cag aac cta gat ggc ctg aag caa aac tac      6249
Val Lys Asp Leu His Gln Asn Leu Asp Gly Leu Lys Gln Asn Tyr
    2030                2035                2040

aat aaa ctg gca gac agc gtg gcc aaa acg aac gct gtg gtg aaa      6294
Asn Lys Leu Ala Asp Ser Val Ala Lys Thr Asn Ala Val Val Lys
    2045                2050                2055

gat cct tcc aaa aac aaa atc att gca gat gca ggc act tcc gtg      6339
Asp Pro Ser Lys Asn Lys Ile Ile Ala Asp Ala Gly Thr Ser Val
    2060                2065                2070

aga aat cta gaa cag gaa gct gac cgg cta atc gac aaa ctc aag      6384
Arg Asn Leu Glu Gln Glu Ala Asp Arg Leu Ile Asp Lys Leu Lys
    2075                2080                2085

ccc atc aag gag ctt gag gac aac cta aag aaa aac att tct gaa      6429
Pro Ile Lys Glu Leu Glu Asp Asn Leu Lys Lys Asn Ile Ser Glu
    2090                2095                2100

ata aag gaa ctg atc aac caa gct cgg aaa caa gct aac tct atc      6474
Ile Lys Glu Leu Ile Asn Gln Ala Arg Lys Gln Ala Asn Ser Ile
    2105                2110                2115

aaa gta tct gtt tct tcg gga ggt gac tgt gtt cgg aca tac agg      6519
Lys Val Ser Val Ser Ser Gly Gly Asp Cys Val Arg Thr Tyr Arg
    2120                2125                2130

cca gaa atc aag aaa gga agc tac aat aac atc gtt gtc cat gtc      6564
Pro Glu Ile Lys Lys Gly Ser Tyr Asn Asn Ile Val Val His Val
    2135                2140                2145

aag acc gct gtt gcc gac aac ctc ctt ttt tat ctt gga agt gcc      6609
Lys Thr Ala Val Ala Asp Asn Leu Leu Phe Tyr Leu Gly Ser Ala
    2150                2155                2160

aaa ttt att gac ttt ctt gct ata gaa atg cgc aaa ggc aaa gtc      6654
Lys Phe Ile Asp Phe Leu Ala Ile Glu Met Arg Lys Gly Lys Val
    2165                2170                2175

agc ttc ctc tgg att gtt ggc tct gga gtt ggc cga gta ggg ttt      6699
Ser Phe Leu Trp Ile Val Gly Ser Gly Val Gly Arg Val Gly Phe
    2180                2185                2190

cca gac ttg acc atc gac gac tcc tat tgg tac cgt att gaa gca      6744
Pro Asp Leu Thr Ile Asp Asp Ser Tyr Trp Tyr Arg Ile Glu Ala
    2195                2200                2205

tca aga acg gga aga aat gga tct att tct gtg aga gct tta gat      6789
Ser Arg Thr Gly Arg Asn Gly Ser Ile Ser Val Arg Ala Leu Asp
    2210                2215                2220

gga ccc aaa gcc agt atg gta ccc agc acc tac cat tca gtg tct      6834
Gly Pro Lys Ala Ser Met Val Pro Ser Thr Tyr His Ser Val Ser
    2225                2230                2235

cct ccc ggg tat act atc cta gat gtg gat gca aat gca atg ctg      6879
```

```
Pro Pro  Gly Tyr Thr Ile Leu  Asp Val Asp Ala Asn  Ala Met Leu
    2240                 2245                 2250

ttt gtt  ggt ggc ctg acc gga  aaa ata aag aag gcc  gat gct gta      6924
Phe Val  Gly Gly Leu Thr Gly  Lys Ile Lys Lys Ala  Asp Ala Val
    2255                 2260                 2265

cgt gtg  atc acc ttc acc ggc  tgt atg gga gaa aca  tac ttt gac      6969
Arg Val  Ile Thr Phe Thr Gly  Cys Met Gly Glu Thr  Tyr Phe Asp
    2270                 2275                 2280

aac aaa  cct ata ggt tta tgg  aac ttc cgg gag aaa  gaa ggc gac      7014
Asn Lys  Pro Ile Gly Leu Trp  Asn Phe Arg Glu Lys  Glu Gly Asp
    2285                 2290                 2295

tgt aag  gga tgt act gtc agc  cca caa gtg gaa gat  agt gag ggg      7059
Cys Lys  Gly Cys Thr Val Ser  Pro Gln Val Glu Asp  Ser Glu Gly
    2300                 2305                 2310

act att  cag ttt gat ggt gaa  ggc tat gca tta gtg  agc cgg ccc      7104
Thr Ile  Gln Phe Asp Gly Glu  Gly Tyr Ala Leu Val  Ser Arg Pro
    2315                 2320                 2325

atc cgc  tgg tac ccc aac atc  tcc aca gtc atg ttc  aag ttc cgg      7149
Ile Arg  Trp Tyr Pro Asn Ile  Ser Thr Val Met Phe  Lys Phe Arg
    2330                 2335                 2340

aca ttt  tca tca agt gct ctc  ctg atg tat ctt gcc  aca cga gac      7194
Thr Phe  Ser Ser Ser Ala Leu  Leu Met Tyr Leu Ala  Thr Arg Asp
    2345                 2350                 2355

ctg aaa  gat ttc atg agt gta  gag ctc agt gat gga  cat gtg aaa      7239
Leu Lys  Asp Phe Met Ser Val  Glu Leu Ser Asp Gly  His Val Lys
    2360                 2365                 2370

gtc agc  tat gac ctg ggc tca  gga atg act tcc gtt  gtc agc aat      7284
Val Ser  Tyr Asp Leu Gly Ser  Gly Met Thr Ser Val  Val Ser Asn
    2375                 2380                 2385

caa aac  cat aat gat ggg aaa  tgg aaa gca ttc acg  ctg tcg cgg      7329
Gln Asn  His Asn Asp Gly Lys  Trp Lys Ala Phe Thr  Leu Ser Arg
    2390                 2395                 2400

att cag  aaa caa gcc aac ata  tcg att gtc gac atc  gat tct aac      7374
Ile Gln  Lys Gln Ala Asn Ile  Ser Ile Val Asp Ile  Asp Ser Asn
    2405                 2410                 2415

cag gag  gag aat gta gct act  tca tct tct gga aac  aac ttt ggt      7419
Gln Glu  Glu Asn Val Ala Thr  Ser Ser Ser Gly Asn  Asn Phe Gly
    2420                 2425                 2430

ctt gac  ttg aaa gca gat gac  aaa ata tat ttt ggt  ggc ctg cca      7464
Leu Asp  Leu Lys Ala Asp Asp  Lys Ile Tyr Phe Gly  Gly Leu Pro
    2435                 2440                 2445

act ctg  aga aac ttg agt atg  aaa gca agg cca gaa  gtc aat gtg      7509
Thr Leu  Arg Asn Leu Ser Met  Lys Ala Arg Pro Glu  Val Asn Val
    2450                 2455                 2460

aag aaa  tac tcc ggc tgc ctc  aaa gat att gaa att  tca aga aca      7554
Lys Lys  Tyr Ser Gly Cys Leu  Lys Asp Ile Glu Ile  Ser Arg Thr
    2465                 2470                 2475

cct tac  aat ata ctc agc agc  cct gat tat gtt ggt  gtg acc aaa      7599
Pro Tyr  Asn Ile Leu Ser Ser  Pro Asp Tyr Val Gly  Val Thr Lys
    2480                 2485                 2490

ggc tgt  tca ctg gag aat gtt  aat aca gtt agt ttc  ccc aag cct      7644
```

```
Gly Cys Ser Leu Glu Asn Val Asn Thr Val Ser Phe Pro Lys Pro
    2495                2500                2505

ggt ttt gtg gag ctt gcc gct gtg tct att gat gtt gga aca gaa      7689
Gly Phe Val Glu Leu Ala Ala Val Ser Ile Asp Val Gly Thr Glu
    2510                2515                2520

atc aat ctg tcc ttt agt acc agg aac gag tct ggg atc att ctc      7734
Ile Asn Leu Ser Phe Ser Thr Arg Asn Glu Ser Gly Ile Ile Leu
    2525                2530                2535

ttg gga agt gga ggg aca ctc aca cca ccc agg aga aaa cgg aga      7779
Leu Gly Ser Gly Gly Thr Leu Thr Pro Pro Arg Arg Lys Arg Arg
    2540                2545                2550

caa acc aca cag gct tat tat gcc ata ttc ctc aac aag ggc cgc      7824
Gln Thr Thr Gln Ala Tyr Tyr Ala Ile Phe Leu Asn Lys Gly Arg
    2555                2560                2565

ttg gaa gtg cat ctc tcc tcg ggg aca cgg aca atg agg aaa att      7869
Leu Glu Val His Leu Ser Ser Gly Thr Arg Thr Met Arg Lys Ile
    2570                2575                2580

gtc atc aaa ccg gag cca aat ttg ttt cat gat ggg aga gaa cat      7914
Val Ile Lys Pro Glu Pro Asn Leu Phe His Asp Gly Arg Glu His
    2585                2590                2595

tct gtc cac gta gaa aga acc aga ggc atc ttc act gtt caa att      7959
Ser Val His Val Glu Arg Thr Arg Gly Ile Phe Thr Val Gln Ile
    2600                2605                2610

gat gaa gac aga aga cat atc caa aac ctg aca gag gaa cag ccc      8004
Asp Glu Asp Arg Arg His Ile Gln Asn Leu Thr Glu Glu Gln Pro
    2615                2620                2625

atc gaa gtg aaa aag ctc ttt gtc ggg ggt gct cct cct gaa ttt      8049
Ile Glu Val Lys Lys Leu Phe Val Gly Gly Ala Pro Pro Glu Phe
    2630                2635                2640

cag ccc tcc cca ctc agg aat att ccg gcc ttt caa ggc tgt gtg      8094
Gln Pro Ser Pro Leu Arg Asn Ile Pro Ala Phe Gln Gly Cys Val
    2645                2650                2655

tgg aac ctt gtt att aac tcc atc ccc atg gac ttt gcg cag cct      8139
Trp Asn Leu Val Ile Asn Ser Ile Pro Met Asp Phe Ala Gln Pro
    2660                2665                2670

ata gcc ttc aaa aat gcc gac att ggt cgc tgt acc tat caa aag      8184
Ile Ala Phe Lys Asn Ala Asp Ile Gly Arg Cys Thr Tyr Gln Lys
    2675                2680                2685

ccc cgg gaa gat gag agt gaa gca gtt cca gct gaa gtt att gtc      8229
Pro Arg Glu Asp Glu Ser Glu Ala Val Pro Ala Glu Val Ile Val
    2690                2695                2700

cag cct cag tcg gtg ccc acc cct gcc ttc cct ttc cca gtc ccc      8274
Gln Pro Gln Ser Val Pro Thr Pro Ala Phe Pro Phe Pro Val Pro
    2705                2710                2715

acc atg gtg cat ggc cct tgt gtt gca gaa tca gaa cca gct ctt      8319
Thr Met Val His Gly Pro Cys Val Ala Glu Ser Glu Pro Ala Leu
    2720                2725                2730

ctg aca ggg agc aag cag ttt ggg ctt tcc aga aac agc cac att      8364
Leu Thr Gly Ser Lys Gln Phe Gly Leu Ser Arg Asn Ser His Ile
    2735                2740                2745

gca att gtc ttt gat gac acc aaa gtt aaa aac cgc ctc acc att      8409
```

```
Ala Ile  Val Phe Asp Asp Thr  Lys Val Lys Asn Arg  Leu Thr Ile
    2750                 2755                 2760

gag ctg  gag gta cga act gaa  gct gaa tca ggc ttg  ctc ttc tac      8454
Glu Leu  Glu Val Arg Thr Glu  Ala Glu Ser Gly Leu  Leu Phe Tyr
    2765                 2770                 2775

atg ggt  cgg atc aat cat gct  gat ttt ggt act gtt  cag ctg agg      8499
Met Gly  Arg Ile Asn His Ala  Asp Phe Gly Thr Val  Gln Leu Arg
    2780                 2785                 2790

aat ggg  ttc ccg ttc ttc agt  tat gat ttg ggg agt  ggg agc acc      8544
Asn Gly  Phe Pro Phe Phe Ser  Tyr Asp Leu Gly Ser  Gly Ser Thr
    2795                 2800                 2805

aga acc  atg atc ccc aca aaa  atc aac gat ggt cag  tgg cac aag      8589
Arg Thr  Met Ile Pro Thr Lys  Ile Asn Asp Gly Gln  Trp His Lys
    2810                 2815                 2820

att aag  att gtg aga gtg aag  cag gag gga att ctt  tat gtg gat      8634
Ile Lys  Ile Val Arg Val Lys  Gln Glu Gly Ile Leu  Tyr Val Asp
    2825                 2830                 2835

gat gcc  tcc agc caa acc atc  agt ccc aag aaa gcc  gac atc ctg      8679
Asp Ala  Ser Ser Gln Thr Ile  Ser Pro Lys Lys Ala  Asp Ile Leu
    2840                 2845                 2850

gat gtc  ggg ggg att ctg tat  gtc ggt gga ttg ccg  atc aac tat      8724
Asp Val  Gly Gly Ile Leu Tyr  Val Gly Gly Leu Pro  Ile Asn Tyr
    2855                 2860                 2865

acc aca  cgc aga att ggt cca  gtg act tac agc ctg  gat ggc tgt      8769
Thr Thr  Arg Arg Ile Gly Pro  Val Thr Tyr Ser Leu  Asp Gly Cys
    2870                 2875                 2880

gtt agg  aat ctt cac atg gaa  caa gcc cct gtt gat  ctg gac cag      8814
Val Arg  Asn Leu His Met Glu  Gln Ala Pro Val Asp  Leu Asp Gln
    2885                 2890                 2895

cct acc  tcc agc ttt cac gtt  ggg aca tgc ttt gcg  aat gca gag      8859
Pro Thr  Ser Ser Phe His Val  Gly Thr Cys Phe Ala  Asn Ala Glu
    2900                 2905                 2910

agt ggg  act tac ttt gat gga  acc ggt ttt ggt aaa  gca gtt ggt      8904
Ser Gly  Thr Tyr Phe Asp Gly  Thr Gly Phe Gly Lys  Ala Val Gly
    2915                 2920                 2925

ggg ttc  atc gtt gga ttg gac  ctt ctt gtg gaa ttt  gaa ttc cgt      8949
Gly Phe  Ile Val Gly Leu Asp  Leu Leu Val Glu Phe  Glu Phe Arg
    2930                 2935                 2940

acc aca  aga ccc act ggg gtc  ctc ctg ggg atc agc  agt cag aag      8994
Thr Thr  Arg Pro Thr Gly Val  Leu Leu Gly Ile Ser  Ser Gln Lys
    2945                 2950                 2955

atg gat  gga atg ggt att gaa  atg atc gac gag aag  ctt atg ttc      9039
Met Asp  Gly Met Gly Ile Glu  Met Ile Asp Glu Lys  Leu Met Phe
    2960                 2965                 2970

cac gtg  gat aat ggc gct ggc  cga ttc act gca att  tat gat gct      9084
His Val  Asp Asn Gly Ala Gly  Arg Phe Thr Ala Ile  Tyr Asp Ala
    2975                 2980                 2985

gag atc  cca ggc cac atg tgc  aat gga cag tgg tat  aaa gtc act      9129
Glu Ile  Pro Gly His Met Cys  Asn Gly Gln Trp Tyr  Lys Val Thr
    2990                 2995                 3000

gcc aag  aag atc aaa aac cgt  ctt gag ctg gtg gta  gat ggg aac      9174
```

```
Ala Lys  Lys Ile Lys Asn Arg  Leu Glu Leu Val Val  Asp Gly Asn
    3005                3010                3015

cag gtg  gat gcc cag agc cca  aac tca gca tcg aca  tca gct gat      9219
Gln Val  Asp Ala Gln Ser Pro  Asn Ser Ala Ser Thr  Ser Ala Asp
    3020                3025                3030

aca aac  gac cct gtt ttc gtt  ggc ggt ttc cca ggt  ggc ctc aat      9264
Thr Asn  Asp Pro Val Phe Val  Gly Gly Phe Pro Gly  Gly Leu Asn
    3035                3040                3045

cag ttt  ggc ctg acc acc aac  att agg ttc cga ggc  tgc atc cga      9309
Gln Phe  Gly Leu Thr Thr Asn  Ile Arg Phe Arg Gly  Cys Ile Arg
    3050                3055                3060

tct ctg  aag ctc acc aaa ggc  act gca aac cgc tgg  agg tta att      9354
Ser Leu  Lys Leu Thr Lys Gly  Thr Ala Asn Arg Trp  Arg Leu Ile
    3065                3070                3075

ttg cca  agg ccc tgg aac tgaggggtgt tcaacctgta tcatgcccga          9402
Leu Pro  Arg Pro Trp Asn
    3080

ctacctaata aagatagttc aatcctgagg agaattcatc aaaacaagta tatcaagtta   9462

aacaatatac actcctatca tattaataaa actaatgtgc agcggccgc              9511


<210>  6
<211>  3084
<212>  PRT
<213>  Mus musculus

<400>  6

Gln Arg Arg Gln Ser Gln Ala His Gln Gln Arg Gly Leu Phe Pro Ala
1               5                   10                  15

Val Leu Asn Leu Ala Ser Asn Ala Leu Ile Thr Thr Asn Ala Thr Cys
            20                  25                  30

Gly Glu Lys Gly Pro Glu Met Tyr Cys Lys Leu Val Glu His Val Pro
        35                  40                  45

Gly Gln Pro Val Arg Asn Pro Gln Cys Arg Ile Cys Asn Gln Asn Ser
    50                  55                  60

Ser Asn Pro Tyr Gln Arg His Pro Ile Thr Asn Ala Ile Asp Gly Lys
65                  70                  75                  80

Asn Thr Trp Trp Gln Ser Pro Ser Ile Lys Asn Gly Val Glu Tyr His
                85                  90                  95

Tyr Val Thr Ile Thr Leu Asp Leu Gln Gln Val Phe Gln Ile Ala Tyr
            100                 105                 110

Val Ile Val Lys Ala Ala Asn Ser Pro Arg Pro Gly Asn Trp Ile Leu
        115                 120                 125

Glu Arg Ser Leu Asp Asp Val Glu Tyr Lys Pro Trp Gln Tyr His Ala
```

130 135 140

Val Thr Asp Thr Glu Cys Leu Thr Leu Tyr Asn Ile Tyr Pro Arg Thr
145 150 155 160

Gly Pro Pro Ser Tyr Ala Lys Asp Asp Glu Val Ile Cys Thr Ser Phe
165 170 175

Tyr Ser Lys Ile His Pro Leu Glu Asn Gly Glu Ile His Ile Ser Leu
180 185 190

Ile Asn Gly Arg Pro Ser Ala Asp Asp Pro Ser Pro Glu Leu Leu Glu
195 200 205

Phe Thr Ser Ala Arg Tyr Ile Arg Leu Arg Phe Gln Arg Ile Arg Thr
210 215 220

Leu Asn Ala Asp Leu Met Met Phe Ala His Lys Asp Pro Arg Glu Ile
225 230 235 240

Asp Pro Ile Val Thr Arg Arg Tyr Tyr Tyr Ser Val Lys Asp Ile Ser
245 250 255

Val Gly Gly Met Cys Ile Cys Tyr Gly His Ala Arg Ala Cys Pro Leu
260 265 270

Asp Pro Ala Thr Asn Lys Ser Arg Cys Glu Cys Glu His Asn Thr Cys
275 280 285

Gly Glu Ser Cys Asp Arg Cys Cys Pro Gly Phe His Gln Lys Pro Trp
290 295 300

Arg Ala Gly Thr Phe Leu Thr Lys Ser Glu Cys Glu Ala Cys Asn Cys
305 310 315 320

His Gly Lys Ala Glu Glu Cys Tyr Tyr Asp Glu Thr Val Ala Ser Arg
325 330 335

Asn Leu Ser Leu Asn Ile His Gly Lys Tyr Ile Gly Gly Gly Val Cys
340 345 350

Ile Asn Cys Thr His Asn Thr Ala Gly Ile Asn Cys Glu Thr Cys Val
355 360 365

Asp Gly Phe Phe Arg Pro Lys Gly Val Ser Pro Asn Tyr Pro Arg Pro
370 375 380

Cys Gln Pro Cys His Cys Asp Pro Thr Gly Ser Leu Ser Glu Val Cys
385 390 395 400

Val Lys Asp Glu Lys Tyr Ala Gln Arg Gly Leu Lys Pro Gly Ser Cys

405 410 415

His Cys Lys Thr Gly Phe Gly Gly Val Asn Cys Asp Arg Cys Val Arg
420 425 430

Gly Tyr His Gly Tyr Pro Asp Cys Gln Pro Cys Asn Cys Ser Gly Leu
435 440 445

Gly Ser Thr Asn Glu Asp Pro Cys Val Gly Pro Cys Ser Cys Lys Glu
450 455 460

Asn Val Glu Gly Glu Asp Cys Ser Arg Cys Lys Ser Gly Phe Phe Asn
465 470 475 480

Leu Gln Glu Asp Asn Gln Lys Gly Cys Glu Glu Cys Phe Cys Ser Gly
485 490 495

Val Ser Asn Arg Cys Gln Ser Ser Tyr Trp Thr Tyr Gly Asn Ile Gln
500 505 510

Asp Met Arg Gly Trp Tyr Leu Thr Asp Leu Ser Gly Arg Ile Arg Met
515 520 525

Ala Pro Gln Leu Asp Asn Pro Asp Ser Pro Gln Gln Ile Ser Ile Ser
530 535 540

Asn Ser Glu Ala Arg Lys Ser Leu Leu Asp Gly Tyr Tyr Trp Ser Ala
545 550 555 560

Pro Pro Pro Tyr Leu Gly Asn Arg Leu Pro Ala Val Gly Gly Gln Leu
565 570 575

Ser Phe Thr Ile Ser Tyr Asp Leu Glu Glu Glu Glu Asp Asp Thr Glu
580 585 590

Lys Leu Leu Gln Leu Met Ile Ile Phe Glu Gly Asn Asp Leu Arg Ile
595 600 605

Ser Thr Ala Tyr Lys Glu Val Tyr Leu Glu Pro Ser Glu Glu His Val
610 615 620

Glu Glu Val Ser Leu Lys Glu Glu Ala Phe Thr Ile His Gly Thr Asn
625 630 635 640

Leu Pro Val Thr Arg Lys Asp Phe Met Ile Val Leu Thr Asn Leu Gly
645 650 655

Glu Ile Leu Ile Gln Ile Thr Tyr Asn Leu Gly Met Asp Ala Ile Phe
660 665 670

Arg Leu Ser Ser Val Asn Leu Glu Ser Pro Val Pro Tyr Pro Thr Asp

675 680 685

Arg Arg Ile Ala Thr Asp Val Glu Val Cys Gln Cys Pro Pro Gly Tyr
690 695 700

Ser Gly Ser Ser Cys Glu Thr Cys Trp Pro Arg His Arg Arg Val Asn
705 710 715 720

Gly Thr Ile Phe Gly Gly Ile Cys Glu Pro Cys Gln Cys Phe Ala His
725 730 735

Ala Glu Ala Cys Asp Asp Ile Thr Gly Glu Cys Leu Asn Cys Lys Asp
740 745 750

His Thr Gly Gly Pro Tyr Cys Asn Glu Cys Leu Pro Gly Phe Tyr Gly
755 760 765

Asp Pro Thr Arg Gly Ser Pro Glu Asp Cys Gln Pro Cys Ala Cys Pro
770 775 780

Leu Asn Ile Pro Ser Asn Asn Phe Ser Pro Thr Cys His Leu Asp Arg
785 790 795 800

Ser Leu Gly Leu Ile Cys Asp Glu Cys Pro Ile Gly Tyr Thr Gly Pro
805 810 815

Arg Cys Glu Arg Cys Ala Glu Gly Tyr Phe Gly Gln Pro Ser Val Pro
820 825 830

Gly Gly Ser Cys Gln Pro Cys Gln Cys Asn Asp Asn Leu Asp Tyr Ser
835 840 845

Ile Pro Gly Ser Cys Asp Ser Leu Ser Gly Ser Cys Leu Ile Cys Lys
850 855 860

Pro Gly Thr Thr Gly Arg Tyr Cys Glu Leu Cys Ala Asp Gly Tyr Phe
865 870 875 880

Gly Asp Ala Val Asn Thr Lys Asn Cys Gln Pro Cys Arg Cys Asp Ile
885 890 895

Asn Gly Ser Phe Ser Glu Asp Cys His Thr Arg Thr Gly Gln Cys Glu
900 905 910

Cys Arg Pro Asn Val Gln Gly Arg His Cys Asp Glu Cys Lys Pro Glu
915 920 925

Thr Phe Gly Leu Gln Leu Gly Arg Gly Cys Leu Pro Cys Asn Cys Asn
930 935 940

Ser Phe Gly Ser Lys Ser Phe Asp Cys Glu Ala Ser Gly Gln Cys Trp

945 950 955 960

Cys Gln Pro Gly Val Ala Gly Lys Lys Cys Asp Arg Cys Ala His Gly
965 970 975

Tyr Phe Asn Phe Gln Glu Gly Gly Cys Ile Ala Cys Asp Cys Ser His
980 985 990

Leu Gly Asn Asn Cys Asp Pro Lys Thr Gly Gln Cys Ile Cys Pro Pro
995 1000 1005

Asn Thr Thr Gly Glu Lys Cys Ser Glu Cys Leu Pro Asn Thr Trp
1010 1015 1020

Gly His Ser Ile Val Thr Gly Cys Lys Val Cys Asn Cys Ser Thr
1025 1030 1035

Val Gly Ser Leu Ala Ser Gln Cys Asn Val Asn Thr Gly Gln Cys
1040 1045 1050

Ser Cys His Pro Lys Phe Ser Gly Met Lys Cys Ser Glu Cys Ser
1055 1060 1065

Arg Gly His Trp Asn Tyr Pro Leu Cys Thr Leu Cys Asp Cys Phe
1070 1075 1080

Leu Pro Gly Thr Asp Ala Thr Thr Cys Asp Leu Glu Thr Arg Lys
1085 1090 1095

Cys Ser Cys Ser Asp Gln Thr Gly Gln Cys Ser Cys Lys Val Asn
1100 1105 1110

Val Glu Gly Val His Cys Asp Arg Cys Arg Pro Gly Lys Phe Gly
1115 1120 1125

Leu Asp Ala Lys Asn Pro Leu Gly Cys Ser Ser Cys Tyr Cys Phe
1130 1135 1140

Gly Val Thr Ser Gln Cys Ser Glu Ala Lys Gly Leu Ile Arg Thr
1145 1150 1155

Trp Val Thr Leu Ser Asp Glu Gln Thr Ile Leu Pro Leu Val Asp
1160 1165 1170

Glu Ala Leu Gln His Thr Thr Thr Lys Gly Ile Ala Phe Gln Lys
1175 1180 1185

Pro Glu Ile Val Ala Lys Met Asp Glu Val Arg Gln Glu Leu His
1190 1195 1200

Leu Glu Pro Phe Tyr Trp Lys Leu Pro Gln Gln Phe Glu Gly Lys
1205 1210 1215

Lys Leu Met Ala Tyr Gly Gly Lys Leu Lys Tyr Ala Ile Tyr Phe
1220 1225 1230

Glu Ala Arg Asp Glu Thr Gly Phe Ala Thr Tyr Lys Pro Gln Val
1235 1240 1245

Ile Ile Arg Gly Gly Thr Pro Thr His Ala Arg Ile Ile Thr Arg
1250 1255 1260

His Met Ala Ala Pro Leu Ile Gly Gln Leu Thr Arg His Glu Ile
1265 1270 1275

Glu Met Thr Glu Lys Glu Trp Lys Tyr Tyr Gly Asp Asp Pro Arg
1280 1285 1290

Ile Ser Arg Thr Val Thr Arg Glu Asp Phe Leu Asp Ile Leu Tyr
1295 1300 1305

Asp Ile His Tyr Ile Leu Ile Lys Ala Thr Tyr Gly Asn Val Val
1310 1315 1320

Arg Gln Ser Arg Ile Ser Glu Ile Ser Met Glu Val Ala Glu Pro
1325 1330 1335

Gly His Val Leu Ala Gly Ser Pro Pro Ala His Leu Ile Glu Arg
1340 1345 1350

Cys Asp Cys Pro Pro Gly Tyr Ser Gly Leu Ser Cys Glu Thr Cys
1355 1360 1365

Ala Pro Gly Phe Tyr Arg Leu Arg Ser Glu Pro Gly Gly Arg Thr
1370 1375 1380

Pro Gly Pro Thr Leu Gly Thr Cys Val Pro Cys Gln Cys Asn Gly
1385 1390 1395

His Ser Ser Gln Cys Asp Pro Glu Thr Ser Val Cys Gln Asn Cys
1400 1405 1410

Gln His His Thr Ala Gly Asp Phe Cys Glu Arg Cys Ala Leu Gly
1415 1420 1425

Tyr Tyr Gly Ile Val Arg Gly Leu Pro Asn Asp Cys Gln Pro Cys
1430 1435 1440

Ala Cys Pro Leu Ile Ser Pro Ser Asn Asn Phe Ser Pro Ser Cys
1445 1450 1455

Val Leu Glu Gly Leu Glu Asp Tyr Arg Cys Thr Ala Cys Pro Arg
1460 1465 1470

Gly Tyr Glu Gly Gln Tyr Cys Glu Arg Cys Ala Pro Gly Tyr Thr
1475 1480 1485

Gly Ser Pro Ser Ser Pro Gly Gly Ser Cys Gln Glu Cys Glu Cys
1490 1495 1500

Asp Pro Tyr Gly Ser Leu Pro Val Pro Cys Asp Arg Val Thr Gly
1505 1510 1515

Leu Cys Thr Cys Arg Pro Gly Ala Thr Gly Arg Lys Cys Asp Gly
1520 1525 1530

Cys Glu His Trp His Ala Arg Glu Gly Ala Glu Cys Val Phe Cys
1535 1540 1545

Gly Asp Glu Cys Thr Gly Leu Leu Leu Gly Asp Leu Ala Arg Leu
1550 1555 1560

Glu Gln Met Thr Met Asn Ile Asn Leu Thr Gly Pro Leu Pro Ala
1565 1570 1575

Pro Tyr Lys Ile Leu Tyr Gly Leu Glu Asn Thr Thr Gln Glu Leu
1580 1585 1590

Lys His Leu Leu Ser Pro Gln Arg Ala Pro Glu Arg Leu Ile Gln
1595 1600 1605

Leu Ala Glu Gly Asn Val Asn Thr Leu Val Met Glu Thr Asn Glu
1610 1615 1620

Leu Leu Thr Arg Ala Thr Lys Val Thr Ala Asp Gly Glu Gln Thr
1625 1630 1635

Gly Gln Asp Ala Glu Arg Thr Asn Ser Arg Ala Glu Ser Leu Glu
1640 1645 1650

Glu Phe Ile Lys Gly Leu Val Gln Asp Ala Glu Ala Ile Asn Glu
1655 1660 1665

Lys Ala Val Lys Leu Asn Glu Thr Leu Gly Asn Gln Asp Lys Thr
1670 1675 1680

Ala Glu Arg Asn Leu Glu Glu Leu Gln Lys Glu Ile Asp Arg Met
1685 1690 1695

Leu Lys Glu Leu Arg Ser Lys Asp Leu Gln Thr Gln Lys Glu Val
1700 1705 1710

Ala Glu Asp Glu Leu Val Ala Ala Glu Gly Leu Leu Lys Arg Val
1715 1720 1725

Asn Lys Leu Phe Gly Glu Pro Arg Ala Gln Asn Glu Asp Met Glu
1730 1735 1740

Lys Asp Leu Gln Gln Lys Leu Ala Glu Tyr Lys Asn Lys Leu Asp
1745 1750 1755

Asp Ala Trp Asp Leu Leu Arg Glu Ala Thr Asp Lys Thr Arg Asp
1760 1765 1770

Ala Asn Arg Leu Ser Ala Ala Asn Gln Lys Asn Met Thr Ile Leu
1775 1780 1785

Glu Thr Lys Lys Glu Ala Ile Glu Gly Ser Lys Arg Gln Ile Glu
1790 1795 1800

Asn Thr Leu Lys Glu Gly Asn Asp Ile Leu Asp Glu Ala Asn Gln
1805 1810 1815

Leu Leu Gly Glu Ile Asn Ser Val Ile Asp Tyr Val Asp Asp Ile
1820 1825 1830

Lys Thr Lys Leu Pro Pro Met Ser Glu Glu Leu Ser Asp Lys Ile
1835 1840 1845

Asp Asp Leu Ala Gln Glu Ile Lys Asp Arg Arg Leu Ala Glu Lys
1850 1855 1860

Val Phe Gln Ala Glu Ser His Ala Ala Gln Leu Asn Asp Ser Ser
1865 1870 1875

Ala Val Leu Asp Gly Ile Leu Asp Glu Ala Lys Asn Ile Ser Phe
1880 1885 1890

Asn Ala Thr Ala Ala Phe Arg Ala Tyr Ser Asn Ile Lys Asp Tyr
1895 1900 1905

Ile Asp Glu Ala Glu Lys Val Ala Arg Glu Ala Lys Glu Leu Ala
1910 1915 1920

Gln Gly Ala Thr Lys Leu Ala Thr Ser Pro Gln Gly Leu Leu Lys
1925 1930 1935

Glu Asp Ala Lys Gly Ser Leu Gln Lys Ser Phe Arg Ile Leu Asn
1940 1945 1950

Glu Ala Lys Lys Leu Ala Asn Asp Val Lys Gly Asn His Asn Asp
1955 1960 1965

Leu Asn Asp Leu Lys Thr Arg Leu Glu Thr Ala Asp Leu Arg Asn
    1970                1975                1980

Ser Gly Leu Leu Gly Ala Leu Asn Asp Thr Met Asp Lys Leu Ser
    1985                1990                1995

Ala Ile Thr Asn Asp Thr Ala Ala Lys Leu Gln Ala Val Lys Glu
    2000                2005                2010

Lys Ala Arg Glu Ala Asn Asp Thr Ala Lys Ala Val Leu Ala Gln
    2015                2020                2025

Val Lys Asp Leu His Gln Asn Leu Asp Gly Leu Lys Gln Asn Tyr
    2030                2035                2040

Asn Lys Leu Ala Asp Ser Val Ala Lys Thr Asn Ala Val Val Lys
    2045                2050                2055

Asp Pro Ser Lys Asn Lys Ile Ile Ala Asp Ala Gly Thr Ser Val
    2060                2065                2070

Arg Asn Leu Glu Gln Glu Ala Asp Arg Leu Ile Asp Lys Leu Lys
    2075                2080                2085

Pro Ile Lys Glu Leu Glu Asp Asn Leu Lys Lys Asn Ile Ser Glu
    2090                2095                2100

Ile Lys Glu Leu Ile Asn Gln Ala Arg Lys Gln Ala Asn Ser Ile
    2105                2110                2115

Lys Val Ser Val Ser Ser Gly Gly Asp Cys Val Arg Thr Tyr Arg
    2120                2125                2130

Pro Glu Ile Lys Lys Gly Ser Tyr Asn Asn Ile Val Val His Val
    2135                2140                2145

Lys Thr Ala Val Ala Asp Asn Leu Leu Phe Tyr Leu Gly Ser Ala
    2150                2155                2160

Lys Phe Ile Asp Phe Leu Ala Ile Glu Met Arg Lys Gly Lys Val
    2165                2170                2175

Ser Phe Leu Trp Ile Val Gly Ser Gly Val Gly Arg Val Gly Phe
    2180                2185                2190

Pro Asp Leu Thr Ile Asp Asp Ser Tyr Trp Tyr Arg Ile Glu Ala
    2195                2200                2205

Ser Arg Thr Gly Arg Asn Gly Ser Ile Ser Val Arg Ala Leu Asp
    2210                2215                2220

Gly Pro Lys Ala Ser Met Val Pro Ser Thr Tyr His Ser Val Ser
2225 2230 2235

Pro Pro Gly Tyr Thr Ile Leu Asp Val Asp Ala Asn Ala Met Leu
2240 2245 2250

Phe Val Gly Gly Leu Thr Gly Lys Ile Lys Lys Ala Asp Ala Val
2255 2260 2265

Arg Val Ile Thr Phe Thr Gly Cys Met Gly Glu Thr Tyr Phe Asp
2270 2275 2280

Asn Lys Pro Ile Gly Leu Trp Asn Phe Arg Glu Lys Glu Gly Asp
2285 2290 2295

Cys Lys Gly Cys Thr Val Ser Pro Gln Val Glu Asp Ser Glu Gly
2300 2305 2310

Thr Ile Gln Phe Asp Gly Glu Gly Tyr Ala Leu Val Ser Arg Pro
2315 2320 2325

Ile Arg Trp Tyr Pro Asn Ile Ser Thr Val Met Phe Lys Phe Arg
2330 2335 2340

Thr Phe Ser Ser Ser Ala Leu Leu Met Tyr Leu Ala Thr Arg Asp
2345 2350 2355

Leu Lys Asp Phe Met Ser Val Glu Leu Ser Asp Gly His Val Lys
2360 2365 2370

Val Ser Tyr Asp Leu Gly Ser Gly Met Thr Ser Val Val Ser Asn
2375 2380 2385

Gln Asn His Asn Asp Gly Lys Trp Lys Ala Phe Thr Leu Ser Arg
2390 2395 2400

Ile Gln Lys Gln Ala Asn Ile Ser Ile Val Asp Ile Asp Ser Asn
2405 2410 2415

Gln Glu Glu Asn Val Ala Thr Ser Ser Ser Gly Asn Asn Phe Gly
2420 2425 2430

Leu Asp Leu Lys Ala Asp Asp Lys Ile Tyr Phe Gly Gly Leu Pro
2435 2440 2445

Thr Leu Arg Asn Leu Ser Met Lys Ala Arg Pro Glu Val Asn Val
2450 2455 2460

Lys Lys Tyr Ser Gly Cys Leu Lys Asp Ile Glu Ile Ser Arg Thr
2465 2470 2475

Pro Tyr Asn Ile Leu Ser Ser Pro Asp Tyr Val Gly Val Thr Lys
2480 2485 2490

Gly Cys Ser Leu Glu Asn Val Asn Thr Val Ser Phe Pro Lys Pro
2495 2500 2505

Gly Phe Val Glu Leu Ala Ala Val Ser Ile Asp Val Gly Thr Glu
2510 2515 2520

Ile Asn Leu Ser Phe Ser Thr Arg Asn Glu Ser Gly Ile Ile Leu
2525 2530 2535

Leu Gly Ser Gly Gly Thr Leu Thr Pro Pro Arg Arg Lys Arg Arg
2540 2545 2550

Gln Thr Thr Gln Ala Tyr Tyr Ala Ile Phe Leu Asn Lys Gly Arg
2555 2560 2565

Leu Glu Val His Leu Ser Ser Gly Thr Arg Thr Met Arg Lys Ile
2570 2575 2580

Val Ile Lys Pro Glu Pro Asn Leu Phe His Asp Gly Arg Glu His
2585 2590 2595

Ser Val His Val Glu Arg Thr Arg Gly Ile Phe Thr Val Gln Ile
2600 2605 2610

Asp Glu Asp Arg Arg His Ile Gln Asn Leu Thr Glu Glu Gln Pro
2615 2620 2625

Ile Glu Val Lys Lys Leu Phe Val Gly Gly Ala Pro Pro Glu Phe
2630 2635 2640

Gln Pro Ser Pro Leu Arg Asn Ile Pro Ala Phe Gln Gly Cys Val
2645 2650 2655

Trp Asn Leu Val Ile Asn Ser Ile Pro Met Asp Phe Ala Gln Pro
2660 2665 2670

Ile Ala Phe Lys Asn Ala Asp Ile Gly Arg Cys Thr Tyr Gln Lys
2675 2680 2685

Pro Arg Glu Asp Glu Ser Glu Ala Val Pro Ala Glu Val Ile Val
2690 2695 2700

Gln Pro Gln Ser Val Pro Thr Pro Ala Phe Pro Phe Pro Val Pro
2705 2710 2715

Thr Met Val His Gly Pro Cys Val Ala Glu Ser Glu Pro Ala Leu
2720 2725 2730

Leu Thr Gly Ser Lys Gln Phe Gly Leu Ser Arg Asn Ser His Ile
2735 2740 2745

Ala Ile Val Phe Asp Asp Thr Lys Val Lys Asn Arg Leu Thr Ile
2750 2755 2760

Glu Leu Glu Val Arg Thr Glu Ala Glu Ser Gly Leu Leu Phe Tyr
2765 2770 2775

Met Gly Arg Ile Asn His Ala Asp Phe Gly Thr Val Gln Leu Arg
2780 2785 2790

Asn Gly Phe Pro Phe Phe Ser Tyr Asp Leu Gly Ser Gly Ser Thr
2795 2800 2805

Arg Thr Met Ile Pro Thr Lys Ile Asn Asp Gly Gln Trp His Lys
2810 2815 2820

Ile Lys Ile Val Arg Val Lys Gln Glu Gly Ile Leu Tyr Val Asp
2825 2830 2835

Asp Ala Ser Ser Gln Thr Ile Ser Pro Lys Lys Ala Asp Ile Leu
2840 2845 2850

Asp Val Gly Gly Ile Leu Tyr Val Gly Gly Leu Pro Ile Asn Tyr
2855 2860 2865

Thr Thr Arg Arg Ile Gly Pro Val Thr Tyr Ser Leu Asp Gly Cys
2870 2875 2880

Val Arg Asn Leu His Met Glu Gln Ala Pro Val Asp Leu Asp Gln
2885 2890 2895

Pro Thr Ser Ser Phe His Val Gly Thr Cys Phe Ala Asn Ala Glu
2900 2905 2910

Ser Gly Thr Tyr Phe Asp Gly Thr Gly Phe Gly Lys Ala Val Gly
2915 2920 2925

Gly Phe Ile Val Gly Leu Asp Leu Leu Val Glu Phe Glu Phe Arg
2930 2935 2940

Thr Thr Arg Pro Thr Gly Val Leu Leu Gly Ile Ser Ser Gln Lys

2945 2950 2955

Met Asp Gly Met Gly Ile Glu Met Ile Asp Glu Lys Leu Met Phe
2960 2965 2970

His Val Asp Asn Gly Ala Gly Arg Phe Thr Ala Ile Tyr Asp Ala
2975 2980 2985

Glu Ile Pro Gly His Met Cys Asn Gly Gln Trp Tyr Lys Val Thr
2990 2995 3000

Ala Lys Lys Ile Lys Asn Arg Leu Glu Leu Val Val Asp Gly Asn
3005 3010 3015

Gln Val Asp Ala Gln Ser Pro Asn Ser Ala Ser Thr Ser Ala Asp
3020 3025 3030

Thr Asn Asp Pro Val Phe Val Gly Gly Phe Pro Gly Gly Leu Asn
3035 3040 3045

Gln Phe Gly Leu Thr Thr Asn Ile Arg Phe Arg Gly Cys Ile Arg
3050 3055 3060

Ser Leu Lys Leu Thr Lys Gly Thr Ala Asn Arg Trp Arg Leu Ile
3065 3070 3075

Leu Pro Arg Pro Trp Asn
3080

<210> 7
<211> 5583
<212> DNA
<213> Mus musculus

<220>
<221> CDS
<222> (42)..(5441)
<223> laminin, beta 2

<400> 7
ccacgcgtcc gggacaccag cccagtaccc acacggtcgg g atg gag tgg gcc tca 56
Met Glu Trp Ala Ser
1 5

gga gaa cca ggg agg ggc agg cag gga cag cct ttg cca tgg gaa ctt 104
Gly Glu Pro Gly Arg Gly Arg Gln Gly Gln Pro Leu Pro Trp Glu Leu
10 15 20

cgc ttg ggc cta ctt cta agt gtg ctg gct gcc aca ttg gcc cag gcc 152
Arg Leu Gly Leu Leu Leu Ser Val Leu Ala Ala Thr Leu Ala Gln Ala
25 30 35

ccg tcc ttg gat gta cct ggc tgt tct cga gga agc tgc tat cca gcc 200
Pro Ser Leu Asp Val Pro Gly Cys Ser Arg Gly Ser Cys Tyr Pro Ala
40 45 50

acc ggt gac ctg ttg gtg ggc cgt gcg gac aga ctg acg gcc tca tcc 248
Thr Gly Asp Leu Leu Val Gly Arg Ala Asp Arg Leu Thr Ala Ser Ser
55 60 65

acg tgt ggc ttg cat agc cct caa ccc tac tgt att gtc agt cac ctg 296
Thr Cys Gly Leu His Ser Pro Gln Pro Tyr Cys Ile Val Ser His Leu
70 75 80 85

cag gac gaa aag aag tgt ttc ctg tgt gac tcc cga cgt ccc ttc tct 344
Gln Asp Glu Lys Lys Cys Phe Leu Cys Asp Ser Arg Arg Pro Phe Ser
90 95 100

```
gct cga gac aac cca aat agt cat cgg atc cag aat gta gtc acc agc      392
Ala Arg Asp Asn Pro Asn Ser His Arg Ile Gln Asn Val Val Thr Ser
            105                 110                 115

ttt gcg cca caa cgc cgg acg gcc tgg tgg caa tcg gag aac ggg gtt      440
Phe Ala Pro Gln Arg Arg Thr Ala Trp Trp Gln Ser Glu Asn Gly Val
        120                 125                 130

cca atg gtc acc atc caa ctg gac ctg gaa gct gag ttt cat ttc acc      488
Pro Met Val Thr Ile Gln Leu Asp Leu Glu Ala Glu Phe His Phe Thr
    135                 140                 145

cac ctc att atg acg ttc aag acg ttc cgg cct gct gct atg ctg gtg      536
His Leu Ile Met Thr Phe Lys Thr Phe Arg Pro Ala Ala Met Leu Val
150                 155                 160                 165

gag cgt tct gca gac ttt ggc cgc acc tgg cac gtg tac cga tat ttt      584
Glu Arg Ser Ala Asp Phe Gly Arg Thr Trp His Val Tyr Arg Tyr Phe
                170                 175                 180

tcc tat gac tgc ggg gct gac ttc ccg gga atc cca ctg gcc ccg cca      632
Ser Tyr Asp Cys Gly Ala Asp Phe Pro Gly Ile Pro Leu Ala Pro Pro
            185                 190                 195

cgt cgc tgg gat gat gta gtg tgt gag tcc cgc tac tca gaa atc gag      680
Arg Arg Trp Asp Asp Val Val Cys Glu Ser Arg Tyr Ser Glu Ile Glu
        200                 205                 210

ccg tct acg gaa ggc gag gtc atc tat cgt gtg ctg gac cct gct att      728
Pro Ser Thr Glu Gly Glu Val Ile Tyr Arg Val Leu Asp Pro Ala Ile
    215                 220                 225

cct atc cca gac ccc tac agc tca cgg att cag aac ctg ttg aag atc      776
Pro Ile Pro Asp Pro Tyr Ser Ser Arg Ile Gln Asn Leu Leu Lys Ile
230                 235                 240                 245

acc aac cta cga gtg aac tta acc cgg ctt cac aca ctg gga gac aac      824
Thr Asn Leu Arg Val Asn Leu Thr Arg Leu His Thr Leu Gly Asp Asn
                250                 255                 260

ttg ctt gac cca cgg agg gag atc cgg gaa aaa tac tat tat gct ctc      872
Leu Leu Asp Pro Arg Arg Glu Ile Arg Glu Lys Tyr Tyr Tyr Ala Leu
            265                 270                 275

tat gaa ctt gtc atc cgt ggc aac tgc ttc tgc tat ggc cac gcc tca      920
Tyr Glu Leu Val Ile Arg Gly Asn Cys Phe Cys Tyr Gly His Ala Ser
        280                 285                 290

cag tgt gcg cct gca cca ggg gcg ccg gcc cat gct gag ggc atg gta      968
Gln Cys Ala Pro Ala Pro Gly Ala Pro Ala His Ala Glu Gly Met Val
    295                 300                 305

cac gga gcc tgt atc tgc aag cac aat act cgt gga ctc aac tgt gag     1016
His Gly Ala Cys Ile Cys Lys His Asn Thr Arg Gly Leu Asn Cys Glu
310                 315                 320                 325

cag tgt cag gat ttc tat cag gac ctt ccc tgg cac cct gca gag gac     1064
Gln Cys Gln Asp Phe Tyr Gln Asp Leu Pro Trp His Pro Ala Glu Asp
                330                 335                 340

ggc cat act cac gcc tgt cgg aag tgt gag tgc aac ggg cat act cat     1112
Gly His Thr His Ala Cys Arg Lys Cys Glu Cys Asn Gly His Thr His
            345                 350                 355

agc tgc cac ttt gac atg gct gtc tac ctg gca tct gga aat gta agt     1160
Ser Cys His Phe Asp Met Ala Val Tyr Leu Ala Ser Gly Asn Val Ser
        360                 365                 370
```

```
gga ggc gta tgc gat ggg tgt cag cac aac aca gct ggg cgc cat tgt      1208
Gly Gly Val Cys Asp Gly Cys Gln His Asn Thr Ala Gly Arg His Cys
    375                 380                 385

gag ttc tgc cgg ccc ttc ttc tac cgt gac ccc acc aag gac atg cgg      1256
Glu Phe Cys Arg Pro Phe Phe Tyr Arg Asp Pro Thr Lys Asp Met Arg
390                 395                 400                     405

gac cca gct gtg tgc cgt cct tgt gac tgt gac cct atg ggt tct caa      1304
Asp Pro Ala Val Cys Arg Pro Cys Asp Cys Asp Pro Met Gly Ser Gln
                410                 415                 420

gat ggt ggt cgc tgt gat tct cat gat gac cct gtg cta gga ctg gtc      1352
Asp Gly Gly Arg Cys Asp Ser His Asp Asp Pro Val Leu Gly Leu Val
            425                 430                 435

tca ggc cag tgt cgc tgc aaa gaa cac gtg gtt ggc act cgc tgc cag      1400
Ser Gly Gln Cys Arg Cys Lys Glu His Val Val Gly Thr Arg Cys Gln
        440                 445                 450

caa tgc cgt gat ggc ttc ttt gga ctt agt gcc agt gac cct cga ggg      1448
Gln Cys Arg Asp Gly Phe Phe Gly Leu Ser Ala Ser Asp Pro Arg Gly
    455                 460                 465

tgc cag cgt tgc cag tgt aat tca cgg ggc aca gtg cct ggg agc tcc      1496
Cys Gln Arg Cys Gln Cys Asn Ser Arg Gly Thr Val Pro Gly Ser Ser
470                 475                 480                     485

cct tgt gac tcc agt agt gga acc tgt ttc tgc aag cgt ctg gtg acc      1544
Pro Cys Asp Ser Ser Ser Gly Thr Cys Phe Cys Lys Arg Leu Val Thr
                490                 495                 500

gga cat ggc tgt gac cgc tgt ctg cct ggc cac tgg ggc ctg agc cat      1592
Gly His Gly Cys Asp Arg Cys Leu Pro Gly His Trp Gly Leu Ser His
            505                 510                 515

gac ctg ctg ggc tgc cgt ccc tgt gac tgt gat gtg ggc ggt gcc ttg      1640
Asp Leu Leu Gly Cys Arg Pro Cys Asp Cys Asp Val Gly Gly Ala Leu
        520                 525                 530

gat cct cag tgt gat gag gcc acc ggt cag tgc cgc tgc cgc caa cac      1688
Asp Pro Gln Cys Asp Glu Ala Thr Gly Gln Cys Arg Cys Arg Gln His
    535                 540                 545

atg att ggg cgg cgc tgc gaa caa gtg cag cct ggc tac ttc cgg cct      1736
Met Ile Gly Arg Arg Cys Glu Gln Val Gln Pro Gly Tyr Phe Arg Pro
550                 555                 560                     565

ttt ctg gac cat tta acc tgg gag gct gag gct gcc caa ggg cag ggg      1784
Phe Leu Asp His Leu Thr Trp Glu Ala Glu Ala Ala Gln Gly Gln Gly
                570                 575                 580

ctt gag gtg gta gag cgg ctg gtg acc aac cga gag act ccg tcc tgg      1832
Leu Glu Val Val Glu Arg Leu Val Thr Asn Arg Glu Thr Pro Ser Trp
            585                 590                 595

act ggc cca ggc ttt gtg cgg ctg cga gaa ggt cag gaa gtg gag ttc      1880
Thr Gly Pro Gly Phe Val Arg Leu Arg Glu Gly Gln Glu Val Glu Phe
        600                 605                 610

ctg gtg acc tct ttg cct agg gcc atg gac tat gac ctg cta ctg cgc      1928
Leu Val Thr Ser Leu Pro Arg Ala Met Asp Tyr Asp Leu Leu Leu Arg
    615                 620                 625

tgg gag ccc cag gtc cct gag caa tgg gca gag ctg gaa ctg atg gtg      1976
Trp Glu Pro Gln Val Pro Glu Gln Trp Ala Glu Leu Glu Leu Met Val
630                 635                 640                     645
```

```
cag cgt ccg ggg cct gtg tct gct cac agt ccg tgc ggg cat gtg ctg      2024
Gln Arg Pro Gly Pro Val Ser Ala His Ser Pro Cys Gly His Val Leu
                650                 655                 660

cct aag gat gac cgc att cag ggg atg ctt cac cca aac acc agg ttt      2072
Pro Lys Asp Asp Arg Ile Gln Gly Met Leu His Pro Asn Thr Arg Phe
            665                 670                 675

ttg gtg ttt ccc aga cct gtc tgc ctt gag cct ggc atc tcc tac aag      2120
Leu Val Phe Pro Arg Pro Val Cys Leu Glu Pro Gly Ile Ser Tyr Lys
        680                 685                 690

ctg aag ctg aaa ctg atc gga aca ggg gga cga gcc cag cct gaa acc      2168
Leu Lys Leu Lys Leu Ile Gly Thr Gly Gly Arg Ala Gln Pro Glu Thr
    695                 700                 705

tcc tac tct gga tta ctc att gac tcg ctg gtc ctg cag ccc cac gtc      2216
Ser Tyr Ser Gly Leu Leu Ile Asp Ser Leu Val Leu Gln Pro His Val
710                 715                 720                 725

ttg gtg cta gag atg ttt agt ggg ggc gat gct gct gct ctg gag cgc      2264
Leu Val Leu Glu Met Phe Ser Gly Gly Asp Ala Ala Ala Leu Glu Arg
                730                 735                 740

cgt acc acc ttt gaa cgc tac cgc tgc cat gag gaa ggt ctg atg ccc      2312
Arg Thr Thr Phe Glu Arg Tyr Arg Cys His Glu Glu Gly Leu Met Pro
            745                 750                 755

agc aag gcc cct cta tct gag acc tgt gcc ccc ctc ctc atc agc gtg      2360
Ser Lys Ala Pro Leu Ser Glu Thr Cys Ala Pro Leu Leu Ile Ser Val
        760                 765                 770

tcc gcc ttg atc tac aat ggc gcc ttg cca tgt cag tgt gac cct caa      2408
Ser Ala Leu Ile Tyr Asn Gly Ala Leu Pro Cys Gln Cys Asp Pro Gln
    775                 780                 785

ggc tca ctg agt tct gaa tgc agt cct cac ggt ggc cag tgc cgg tgc      2456
Gly Ser Leu Ser Ser Glu Cys Ser Pro His Gly Gly Gln Cys Arg Cys
790                 795                 800                 805

aaa cct gga gtg gtt gga cgc cgt tgt gat gtc tgt gct act ggc tac      2504
Lys Pro Gly Val Val Gly Arg Arg Cys Asp Val Cys Ala Thr Gly Tyr
                810                 815                 820

tat ggc ttt ggc cct gca ggc tgt caa gcc tgc cag tgt agt cct gat      2552
Tyr Gly Phe Gly Pro Ala Gly Cys Gln Ala Cys Gln Cys Ser Pro Asp
            825                 830                 835

gga gca ctc agt gcc ctc tgt gaa ggg act agt gga cag tgc ccc tgc      2600
Gly Ala Leu Ser Ala Leu Cys Glu Gly Thr Ser Gly Gln Cys Pro Cys
        840                 845                 850

cga cct ggt gcc ttt ggt ctt cgc tgt gac cac tgt caa cgt ggc cag      2648
Arg Pro Gly Ala Phe Gly Leu Arg Cys Asp His Cys Gln Arg Gly Gln
    855                 860                 865

tgg gga ttc cct aat tgc cgg ccg tgt gtc tgc aat ggg cgt gcg gat      2696
Trp Gly Phe Pro Asn Cys Arg Pro Cys Val Cys Asn Gly Arg Ala Asp
870                 875                 880                 885

gag tgt gat acc cac aca ggc gct tgc ctg ggc tgc cgt gat tac acg      2744
Glu Cys Asp Thr His Thr Gly Ala Cys Leu Gly Cys Arg Asp Tyr Thr
                890                 895                 900

ggg ggc gag cac tgt gaa agg tgc att gct ggt ttt cat ggg gac cca      2792
Gly Gly Glu His Cys Glu Arg Cys Ile Ala Gly Phe His Gly Asp Pro
            905                 910                 915
```

```
cgg ctg cca tat ggg ggc cag tgc cgg cct tgt ccc tgc cct gaa ggc      2840
Arg Leu Pro Tyr Gly Gly Gln Cys Arg Pro Cys Pro Cys Pro Glu Gly
        920                 925                 930

cct ggg agc cag cga cac ttt gct act tct tgc cac cgg gat gga tat      2888
Pro Gly Ser Gln Arg His Phe Ala Thr Ser Cys His Arg Asp Gly Tyr
    935                 940                 945

tcc cag caa att gtg tgc cag tgt cga gaa ggc tac aca ggg ctt cgg      2936
Ser Gln Gln Ile Val Cys Gln Cys Arg Glu Gly Tyr Thr Gly Leu Arg
950                 955                 960                 965

tgt gaa gct tgt gcc ccc ggg cac ttt ggg gac cca tca aag cca ggt      2984
Cys Glu Ala Cys Ala Pro Gly His Phe Gly Asp Pro Ser Lys Pro Gly
                970                 975                 980

ggc agg tgc caa ctg tgt gag tgc agt gga aac att gat ccc atg gac      3032
Gly Arg Cys Gln Leu Cys Glu Cys Ser Gly Asn Ile Asp Pro Met Asp
            985                 990                 995

cct gat gcc  tgt gat ccc cac acg  ggg caa tgc ttg cgt  tgt tta      3077
Pro Asp Ala  Cys Asp Pro His Thr  Gly Gln Cys Leu Arg  Cys Leu
        1000                 1005                 1010

cac aac aca  gag ggg ccc cac tgt  ggc tat tgc aag cct  ggc ttc      3122
His Asn Thr  Glu Gly Pro His Cys  Gly Tyr Cys Lys Pro  Gly Phe
        1015                 1020                 1025

cat ggg caa  gct gcc cga cag agc  tgt cac cgc tgt acc  tgc aac      3167
His Gly Gln  Ala Ala Arg Gln Ser  Cys His Arg Cys Thr  Cys Asn
        1030                 1035                 1040

ctt ctg ggc  aca gat ccc agg cgg  tgc cca tct acc gac  ctg tgc      3212
Leu Leu Gly  Thr Asp Pro Arg Arg  Cys Pro Ser Thr Asp  Leu Cys
        1045                 1050                 1055

cat tgt gac  cca agc act ggg cag  tgc cca tgc ctt ccc  cat gtc      3257
His Cys Asp  Pro Ser Thr Gly Gln  Cys Pro Cys Leu Pro  His Val
        1060                 1065                 1070

caa ggc ctc  aac tgt gac cat tgt  gcc ccc aac ttt tgg  aac ttc      3302
Gln Gly Leu  Asn Cys Asp His Cys  Ala Pro Asn Phe Trp  Asn Phe
        1075                 1080                 1085

acc agt ggc  cgt ggc tgc cag cct  tgt gct tgt cac cca  agc cgg      3347
Thr Ser Gly  Arg Gly Cys Gln Pro  Cys Ala Cys His Pro  Ser Arg
        1090                 1095                 1100

gcc aga ggc  cct acc tgc aat gag  ttc aca ggg cag tgt  cac tgt      3392
Ala Arg Gly  Pro Thr Cys Asn Glu  Phe Thr Gly Gln Cys  His Cys
        1105                 1110                 1115

cat gct ggc  ttt ggt ggg agg act  tgt tct gag tgc caa  gag ctc      3437
His Ala Gly  Phe Gly Gly Arg Thr  Cys Ser Glu Cys Gln  Glu Leu
        1120                 1125                 1130

tac tgg gga  gac cct ggt ctg cag  tgc cgt gcc tgt gac  tgt gat      3482
Tyr Trp Gly  Asp Pro Gly Leu Gln  Cys Arg Ala Cys Asp  Cys Asp
        1135                 1140                 1145

cct aga gga  ata gac aaa cct cag  tgt cat cgt tcc aca  ggc cac      3527
Pro Arg Gly  Ile Asp Lys Pro Gln  Cys His Arg Ser Thr  Gly His
        1150                 1155                 1160

tgt agc tgc  cgc cca ggc gtg tct  ggt gtg cgc tgt gac  cag tgt      3572
Cys Ser Cys  Arg Pro Gly Val Ser  Gly Val Arg Cys Asp  Gln Cys
        1165                 1170                 1175
```

```
gct cgt ggc ttc tca ggg gtt ttt cct gct tgt cac ccc tgc cac        3617
Ala Arg Gly Phe Ser Gly Val Phe Pro Ala Cys His Pro Cys His
        1180                1185                1190

gct tgc ttt gga gac tgg gat cgt gtg gta cag gac ctg gct gct        3662
Ala Cys Phe Gly Asp Trp Asp Arg Val Val Gln Asp Leu Ala Ala
        1195                1200                1205

cgg acg cgg cgc ctg gag cag tgg gct cag gag ttg cag caa aca        3707
Arg Thr Arg Arg Leu Glu Gln Trp Ala Gln Glu Leu Gln Gln Thr
        1210                1215                1220

gga gtg ctg ggt gcc ttt gag agc agc ttt ttg aac atg cag ggg        3752
Gly Val Leu Gly Ala Phe Glu Ser Ser Phe Leu Asn Met Gln Gly
        1225                1230                1235

aag cta ggc atg gtg cag gcc att atg agt gcc cgc aat gcc tca        3797
Lys Leu Gly Met Val Gln Ala Ile Met Ser Ala Arg Asn Ala Ser
        1240                1245                1250

gcc gcc tct acg gcg aag ctt gta gag gcc aca gag gga cta cgt        3842
Ala Ala Ser Thr Ala Lys Leu Val Glu Ala Thr Glu Gly Leu Arg
        1255                1260                1265

cat gaa atc ggg aag acc acc gag cgc ctg act cag tta gaa gca        3887
His Glu Ile Gly Lys Thr Thr Glu Arg Leu Thr Gln Leu Glu Ala
        1270                1275                1280

gag cta aca gct gtg cag gat gag aac ttc aat gcc aac cat gca        3932
Glu Leu Thr Ala Val Gln Asp Glu Asn Phe Asn Ala Asn His Ala
        1285                1290                1295

ctc agt ggt ctg gag aga gac ggg ctt gcg ctt aat ctc acc ctg        3977
Leu Ser Gly Leu Glu Arg Asp Gly Leu Ala Leu Asn Leu Thr Leu
        1300                1305                1310

agg cag ctg gat cag cat ctg gag atc ctc aaa cat tca aat ttc        4022
Arg Gln Leu Asp Gln His Leu Glu Ile Leu Lys His Ser Asn Phe
        1315                1320                1325

tta ggt gcc tat gac agc atc cga cat gcc cac agc cag tcc aca        4067
Leu Gly Ala Tyr Asp Ser Ile Arg His Ala His Ser Gln Ser Thr
        1330                1335                1340

gag gca gag cgc cgt gcc aac gcc tcc acc ttt gca gta ccc agc        4112
Glu Ala Glu Arg Arg Ala Asn Ala Ser Thr Phe Ala Val Pro Ser
        1345                1350                1355

cct gtg agc aac tca gca gat acc cgg cgt cgg acg gaa gtg cta        4157
Pro Val Ser Asn Ser Ala Asp Thr Arg Arg Arg Thr Glu Val Leu
        1360                1365                1370

atg ggt gcc caa aaa gaa aac ttc aac cgc caa cat ttg gcc aac        4202
Met Gly Ala Gln Lys Glu Asn Phe Asn Arg Gln His Leu Ala Asn
        1375                1380                1385

cag cag gca ctg gga cgg ctc tct gca cat gcc cac acc ctg agc        4247
Gln Gln Ala Leu Gly Arg Leu Ser Ala His Ala His Thr Leu Ser
        1390                1395                1400

ctg acg ggc ata aat gag ttg gtg tgt ggg gca cca ggg gac gca        4292
Leu Thr Gly Ile Asn Glu Leu Val Cys Gly Ala Pro Gly Asp Ala
        1405                1410                1415

ccc tgt gcc acc agc cct tgt ggg ggt gcc gga tgt cgg gat gaa        4337
Pro Cys Ala Thr Ser Pro Cys Gly Gly Ala Gly Cys Arg Asp Glu
        1420                1425                1430
```

```
gat ggg cag ccc cgt tgt ggt ggc ctc ggt tgc agt ggg gca gca        4382
Asp Gly Gln Pro Arg Cys Gly Gly Leu Gly Cys Ser Gly Ala Ala
        1435                1440                1445

gcc acg gca gat cta gcg ctg ggc cgg gct cgg cac acg cag gca        4427
Ala Thr Ala Asp Leu Ala Leu Gly Arg Ala Arg His Thr Gln Ala
        1450                1455                1460

gag ctg cag cgg gca ctg gta gaa ggt ggc ggc atc ctc agc cgg        4472
Glu Leu Gln Arg Ala Leu Val Glu Gly Gly Gly Ile Leu Ser Arg
        1465                1470                1475

gtg tct gag act cgt cgg cag gca gaa gag gca cag cag cga gca        4517
Val Ser Glu Thr Arg Arg Gln Ala Glu Glu Ala Gln Gln Arg Ala
        1480                1485                1490

cag gca gcc ctg gac aag gct aat gct tcc agg ggc cag gtg gaa        4562
Gln Ala Ala Leu Asp Lys Ala Asn Ala Ser Arg Gly Gln Val Glu
        1495                1500                1505

cag gcc aat cag gag ctt cga gaa ctt atc cag aat gtg aaa gac        4607
Gln Ala Asn Gln Glu Leu Arg Glu Leu Ile Gln Asn Val Lys Asp
        1510                1515                1520

ttc ctc agc cag gag gga gcc gat cct gac agt att gaa atg gta        4652
Phe Leu Ser Gln Glu Gly Ala Asp Pro Asp Ser Ile Glu Met Val
        1525                1530                1535

gcg act cgg gtg cta gac atc tcc atc ccg gcc tca ccc gag cag        4697
Ala Thr Arg Val Leu Asp Ile Ser Ile Pro Ala Ser Pro Glu Gln
        1540                1545                1550

atc cag cgc cta gcc agt gag att gca gaa cgc gtc cga agc ctg        4742
Ile Gln Arg Leu Ala Ser Glu Ile Ala Glu Arg Val Arg Ser Leu
        1555                1560                1565

gcc gac gtg gac aca atc ctg gcc cat acc atg ggc gac gtg cgt        4787
Ala Asp Val Asp Thr Ile Leu Ala His Thr Met Gly Asp Val Arg
        1570                1575                1580

cgg gct gaa cag cta ctg caa gat gcg cac cgg gca cgg agc cgg        4832
Arg Ala Glu Gln Leu Leu Gln Asp Ala His Arg Ala Arg Ser Arg
        1585                1590                1595

gcc gag ggt gag aga cag aag gca gag aca gtc caa gcg gca ctg        4877
Ala Glu Gly Glu Arg Gln Lys Ala Glu Thr Val Gln Ala Ala Leu
        1600                1605                1610

gag gag gct cag agg gca caa gga gct gct cag ggt gcc atc tgg        4922
Glu Glu Ala Gln Arg Ala Gln Gly Ala Ala Gln Gly Ala Ile Trp
        1615                1620                1625

gga gca gtg gtt gac aca caa aac aca gag cag acc ctg cag cgg        4967
Gly Ala Val Val Asp Thr Gln Asn Thr Glu Gln Thr Leu Gln Arg
        1630                1635                1640

gtc cag gag agg atg gca ggt gca gag aag tct ctg aac tct gcc        5012
Val Gln Glu Arg Met Ala Gly Ala Glu Lys Ser Leu Asn Ser Ala
        1645                1650                1655

ggt gag cgg gct cgg caa tta gac gcc ctc ctg gag gcc ctg aaa        5057
Gly Glu Arg Ala Arg Gln Leu Asp Ala Leu Leu Glu Ala Leu Lys
        1660                1665                1670

ctg aaa cgg gca gga aat agc ctg gca gca tct aca gcg gaa gaa        5102
Leu Lys Arg Ala Gly Asn Ser Leu Ala Ala Ser Thr Ala Glu Glu
        1675                1680                1685
```

```
aca gca ggc  agt gcc cag agc cgt  gcc agg gag gct gag  aaa caa      5147
Thr Ala Gly  Ser Ala Gln Ser Arg  Ala Arg Glu Ala Glu  Lys Gln
        1690                 1695                 1700

cta cgg gaa  caa gta ggt gac caa  tac caa aca gtg agg  gcg ttg      5192
Leu Arg Glu  Gln Val Gly Asp Gln  Tyr Gln Thr Val Arg  Ala Leu
        1705                 1710                 1715

gca gag cgg  aag gct gaa ggt gtt  ctg gct gca caa gcc  agg gca      5237
Ala Glu Arg  Lys Ala Glu Gly Val  Leu Ala Ala Gln Ala  Arg Ala
        1720                 1725                 1730

gaa caa ctg  cgg gat gag gct cgg  gac ctg ttg cag gcc  gct cag      5282
Glu Gln Leu  Arg Asp Glu Ala Arg  Asp Leu Leu Gln Ala  Ala Gln
        1735                 1740                 1745

gat aag ctg  cag cgg cta cag gag  ctg gag ggc aca tat  gag gag      5327
Asp Lys Leu  Gln Arg Leu Gln Glu  Leu Glu Gly Thr Tyr  Glu Glu
        1750                 1755                 1760

aac gag cgt  gca ctg gag ggc aaa  gcg gcc cag ctg gat  ggg ctg      5372
Asn Glu Arg  Ala Leu Glu Gly Lys  Ala Ala Gln Leu Asp  Gly Leu
        1765                 1770                 1775

gaa gcc agg  atg cgc agt gtg ctc  cag gcc atc aac ttg  cag gtc      5417
Glu Ala Arg  Met Arg Ser Val Leu  Gln Ala Ile Asn Leu  Gln Val
        1780                 1785                 1790

cag atc tac  aac acc tgc cag tga ccactcccta gggcctagcc ttgtcgccaa   5471
Gln Ile Tyr  Asn Thr Cys Gln
        1795

gcactgttct gcacacgatc gtccgcacat taaagagctc ctggctagca agagctttca   5531

ataaacctgt gtgaacctca aaaaaaaaaa aaaaaaaaaa aaaaaaaaaa aa           5583
```

```
<210>  8
<211>  1799
<212>  PRT
<213>  Mus musculus

<400>  8

Met Glu Trp Ala Ser Gly Glu Pro Gly Arg Gly Arg Gln Gly Gln Pro
1               5                   10                  15

Leu Pro Trp Glu Leu Arg Leu Gly Leu Leu Leu Ser Val Leu Ala Ala
            20                  25                  30

Thr Leu Ala Gln Ala Pro Ser Leu Asp Val Pro Gly Cys Ser Arg Gly
        35                  40                  45

Ser Cys Tyr Pro Ala Thr Gly Asp Leu Leu Val Gly Arg Ala Asp Arg
    50                  55                  60

Leu Thr Ala Ser Ser Thr Cys Gly Leu His Ser Pro Gln Pro Tyr Cys
65                  70                  75                  80

Ile Val Ser His Leu Gln Asp Glu Lys Lys Cys Phe Leu Cys Asp Ser
                85                  90                  95
```

```
Arg Arg Pro Phe Ser Ala Arg Asp Asn Pro Asn Ser His Arg Ile Gln
            100                 105                 110

Asn Val Val Thr Ser Phe Ala Pro Gln Arg Arg Thr Ala Trp Trp Gln
        115                 120                 125

Ser Glu Asn Gly Val Pro Met Val Thr Ile Gln Leu Asp Leu Glu Ala
    130                 135                 140

Glu Phe His Phe Thr His Leu Ile Met Thr Phe Lys Thr Phe Arg Pro
145                 150                 155                 160

Ala Ala Met Leu Val Glu Arg Ser Ala Asp Phe Gly Arg Thr Trp His
                165                 170                 175

Val Tyr Arg Tyr Phe Ser Tyr Asp Cys Gly Ala Asp Phe Pro Gly Ile
            180                 185                 190

Pro Leu Ala Pro Pro Arg Arg Trp Asp Asp Val Val Cys Glu Ser Arg
        195                 200                 205

Tyr Ser Glu Ile Glu Pro Ser Thr Glu Gly Glu Val Ile Tyr Arg Val
    210                 215                 220

Leu Asp Pro Ala Ile Pro Ile Pro Asp Pro Tyr Ser Ser Arg Ile Gln
225                 230                 235                 240

Asn Leu Leu Lys Ile Thr Asn Leu Arg Val Asn Leu Thr Arg Leu His
                245                 250                 255

Thr Leu Gly Asp Asn Leu Leu Asp Pro Arg Arg Glu Ile Arg Glu Lys
            260                 265                 270

Tyr Tyr Tyr Ala Leu Tyr Glu Leu Val Ile Arg Gly Asn Cys Phe Cys
        275                 280                 285

Tyr Gly His Ala Ser Gln Cys Ala Pro Ala Pro Gly Ala Pro Ala His
    290                 295                 300

Ala Glu Gly Met Val His Gly Ala Cys Ile Cys Lys His Asn Thr Arg
305                 310                 315                 320

Gly Leu Asn Cys Glu Gln Cys Gln Asp Phe Tyr Gln Asp Leu Pro Trp
                325                 330                 335

His Pro Ala Glu Asp Gly His Thr His Ala Cys Arg Lys Cys Glu Cys
            340                 345                 350

Asn Gly His Thr His Ser Cys His Phe Asp Met Ala Val Tyr Leu Ala
        355                 360                 365
```

Ser Gly Asn Val Ser Gly Gly Val Cys Asp Gly Cys Gln His Asn Thr
370 375 380

Ala Gly Arg His Cys Glu Phe Cys Arg Pro Phe Phe Tyr Arg Asp Pro
385 390 395 400

Thr Lys Asp Met Arg Asp Pro Ala Val Cys Arg Pro Cys Asp Cys Asp
405 410 415

Pro Met Gly Ser Gln Asp Gly Gly Arg Cys Asp Ser His Asp Asp Pro
420 425 430

Val Leu Gly Leu Val Ser Gly Gln Cys Arg Cys Lys Glu His Val Val
435 440 445

Gly Thr Arg Cys Gln Gln Cys Arg Asp Gly Phe Phe Gly Leu Ser Ala
450 455 460

Ser Asp Pro Arg Gly Cys Gln Arg Cys Gln Cys Asn Ser Arg Gly Thr
465 470 475 480

Val Pro Gly Ser Ser Pro Cys Asp Ser Ser Ser Gly Thr Cys Phe Cys
485 490 495

Lys Arg Leu Val Thr Gly His Gly Cys Asp Arg Cys Leu Pro Gly His
500 505 510

Trp Gly Leu Ser His Asp Leu Leu Gly Cys Arg Pro Cys Asp Cys Asp
515 520 525

Val Gly Gly Ala Leu Asp Pro Gln Cys Asp Glu Ala Thr Gly Gln Cys
530 535 540

Arg Cys Arg Gln His Met Ile Gly Arg Arg Cys Glu Gln Val Gln Pro
545 550 555 560

Gly Tyr Phe Arg Pro Phe Leu Asp His Leu Thr Trp Glu Ala Glu Ala
565 570 575

Ala Gln Gly Gln Gly Leu Glu Val Val Glu Arg Leu Val Thr Asn Arg
580 585 590

Glu Thr Pro Ser Trp Thr Gly Pro Gly Phe Val Arg Leu Arg Glu Gly
595 600 605

Gln Glu Val Glu Phe Leu Val Thr Ser Leu Pro Arg Ala Met Asp Tyr
610 615 620

Asp Leu Leu Leu Arg Trp Glu Pro Gln Val Pro Glu Gln Trp Ala Glu
625 630 635 640

Leu Glu Leu Met Val Gln Arg Pro Gly Pro Val Ser Ala His Ser Pro
645 650 655

Cys Gly His Val Leu Pro Lys Asp Asp Arg Ile Gln Gly Met Leu His
660 665 670

Pro Asn Thr Arg Phe Leu Val Phe Pro Arg Pro Val Cys Leu Glu Pro
675 680 685

Gly Ile Ser Tyr Lys Leu Lys Leu Lys Leu Ile Gly Thr Gly Gly Arg
690 695 700

Ala Gln Pro Glu Thr Ser Tyr Ser Gly Leu Leu Ile Asp Ser Leu Val
705 710 715 720

Leu Gln Pro His Val Leu Val Leu Glu Met Phe Ser Gly Gly Asp Ala
725 730 735

Ala Ala Leu Glu Arg Arg Thr Thr Phe Glu Arg Tyr Arg Cys His Glu
740 745 750

Glu Gly Leu Met Pro Ser Lys Ala Pro Leu Ser Glu Thr Cys Ala Pro
755 760 765

Leu Leu Ile Ser Val Ser Ala Leu Ile Tyr Asn Gly Ala Leu Pro Cys
770 775 780

Gln Cys Asp Pro Gln Gly Ser Leu Ser Ser Glu Cys Ser Pro His Gly
785 790 795 800

Gly Gln Cys Arg Cys Lys Pro Gly Val Val Gly Arg Arg Cys Asp Val
805 810 815

Cys Ala Thr Gly Tyr Tyr Gly Phe Gly Pro Ala Gly Cys Gln Ala Cys
820 825 830

Gln Cys Ser Pro Asp Gly Ala Leu Ser Ala Leu Cys Glu Gly Thr Ser
835 840 845

Gly Gln Cys Pro Cys Arg Pro Gly Ala Phe Gly Leu Arg Cys Asp His
850 855 860

Cys Gln Arg Gly Gln Trp Gly Phe Pro Asn Cys Arg Pro Cys Val Cys
865 870 875 880

Asn Gly Arg Ala Asp Glu Cys Asp Thr His Thr Gly Ala Cys Leu Gly
885 890 895

Cys Arg Asp Tyr Thr Gly Gly Glu His Cys Glu Arg Cys Ile Ala Gly
900 905 910

Phe His Gly Asp Pro Arg Leu Pro Tyr Gly Gly Gln Cys Arg Pro Cys
915 920 925

Pro Cys Pro Glu Gly Pro Gly Ser Gln Arg His Phe Ala Thr Ser Cys
930 935 940

His Arg Asp Gly Tyr Ser Gln Gln Ile Val Cys Gln Cys Arg Glu Gly
945 950 955 960

Tyr Thr Gly Leu Arg Cys Glu Ala Cys Ala Pro Gly His Phe Gly Asp
965 970 975

Pro Ser Lys Pro Gly Gly Arg Cys Gln Leu Cys Glu Cys Ser Gly Asn
980 985 990

Ile Asp Pro Met Asp Pro Asp Ala Cys Asp Pro His Thr Gly Gln Cys
995 1000 1005

Leu Arg Cys Leu His Asn Thr Glu Gly Pro His Cys Gly Tyr Cys
1010 1015 1020

Lys Pro Gly Phe His Gly Gln Ala Ala Arg Gln Ser Cys His Arg
1025 1030 1035

Cys Thr Cys Asn Leu Leu Gly Thr Asp Pro Arg Arg Cys Pro Ser
1040 1045 1050

Thr Asp Leu Cys His Cys Asp Pro Ser Thr Gly Gln Cys Pro Cys
1055 1060 1065

Leu Pro His Val Gln Gly Leu Asn Cys Asp His Cys Ala Pro Asn
1070 1075 1080

Phe Trp Asn Phe Thr Ser Gly Arg Gly Cys Gln Pro Cys Ala Cys
1085 1090 1095

His Pro Ser Arg Ala Arg Gly Pro Thr Cys Asn Glu Phe Thr Gly
1100 1105 1110

Gln Cys His Cys His Ala Gly Phe Gly Gly Arg Thr Cys Ser Glu
1115 1120 1125

Cys Gln Glu Leu Tyr Trp Gly Asp Pro Gly Leu Gln Cys Arg Ala
1130 1135 1140

Cys Asp Cys Asp Pro Arg Gly Ile Asp Lys Pro Gln Cys His Arg
1145 1150 1155

Ser Thr Gly His Cys Ser Cys Arg Pro Gly Val Ser Gly Val Arg
1160 1165 1170

Cys Asp Gln Cys Ala Arg Gly Phe Ser Gly Val Phe Pro Ala Cys
1175 1180 1185

His Pro Cys His Ala Cys Phe Gly Asp Trp Asp Arg Val Val Gln
1190 1195 1200

Asp Leu Ala Ala Arg Thr Arg Arg Leu Glu Gln Trp Ala Gln Glu
1205 1210 1215

Leu Gln Gln Thr Gly Val Leu Gly Ala Phe Glu Ser Ser Phe Leu
1220 1225 1230

Asn Met Gln Gly Lys Leu Gly Met Val Gln Ala Ile Met Ser Ala
1235 1240 1245

Arg Asn Ala Ser Ala Ala Ser Thr Ala Lys Leu Val Glu Ala Thr
1250 1255 1260

Glu Gly Leu Arg His Glu Ile Gly Lys Thr Thr Glu Arg Leu Thr
1265 1270 1275

Gln Leu Glu Ala Glu Leu Thr Ala Val Gln Asp Glu Asn Phe Asn
1280 1285 1290

Ala Asn His Ala Leu Ser Gly Leu Glu Arg Asp Gly Leu Ala Leu
1295 1300 1305

Asn Leu Thr Leu Arg Gln Leu Asp Gln His Leu Glu Ile Leu Lys
1310 1315 1320

His Ser Asn Phe Leu Gly Ala Tyr Asp Ser Ile Arg His Ala His
1325 1330 1335

Ser Gln Ser Thr Glu Ala Glu Arg Arg Ala Asn Ala Ser Thr Phe
1340 1345 1350

Ala Val Pro Ser Pro Val Ser Asn Ser Ala Asp Thr Arg Arg Arg
1355 1360 1365

Thr Glu Val Leu Met Gly Ala Gln Lys Glu Asn Phe Asn Arg Gln
1370 1375 1380

His Leu Ala Asn Gln Gln Ala Leu Gly Arg Leu Ser Ala His Ala
1385 1390 1395

His Thr Leu Ser Leu Thr Gly Ile Asn Glu Leu Val Cys Gly Ala
1400 1405 1410

Pro Gly Asp Ala Pro Cys Ala Thr Ser Pro Cys Gly Gly Ala Gly
1415 1420 1425

Cys Arg Asp Glu Asp Gly Gln Pro Arg Cys Gly Gly Leu Gly Cys
1430 1435 1440

Ser Gly Ala Ala Ala Thr Ala Asp Leu Ala Leu Gly Arg Ala Arg
1445 1450 1455

His Thr Gln Ala Glu Leu Gln Arg Ala Leu Val Glu Gly Gly Gly
1460 1465 1470

Ile Leu Ser Arg Val Ser Glu Thr Arg Arg Gln Ala Glu Glu Ala
1475 1480 1485

Gln Gln Arg Ala Gln Ala Ala Leu Asp Lys Ala Asn Ala Ser Arg
1490 1495 1500

Gly Gln Val Glu Gln Ala Asn Gln Glu Leu Arg Glu Leu Ile Gln
1505 1510 1515

Asn Val Lys Asp Phe Leu Ser Gln Glu Gly Ala Asp Pro Asp Ser

1520 1525 1530

Ile Glu Met Val Ala Thr Arg Val Leu Asp Ile Ser Ile Pro Ala
1535 1540 1545

Ser Pro Glu Gln Ile Gln Arg Leu Ala Ser Glu Ile Ala Glu Arg
1550 1555 1560

Val Arg Ser Leu Ala Asp Val Asp Thr Ile Leu Ala His Thr Met
1565 1570 1575

Gly Asp Val Arg Arg Ala Glu Gln Leu Leu Gln Asp Ala His Arg
1580 1585 1590

Ala Arg Ser Arg Ala Glu Gly Glu Arg Gln Lys Ala Glu Thr Val
1595 1600 1605

Gln Ala Ala Leu Glu Glu Ala Gln Arg Ala Gln Gly Ala Ala Gln
1610 1615 1620

Gly Ala Ile Trp Gly Ala Val Val Asp Thr Gln Asn Thr Glu Gln
1625 1630 1635

Thr Leu Gln Arg Val Gln Glu Arg Met Ala Gly Ala Glu Lys Ser
1640 1645 1650

Leu Asn Ser Ala Gly Glu Arg Ala Arg Gln Leu Asp Ala Leu Leu
1655 1660 1665

Glu Ala Leu Lys Leu Lys Arg Ala Gly Asn Ser Leu Ala Ala Ser
1670 1675 1680

Thr Ala Glu Glu Thr Ala Gly Ser Ala Gln Ser Arg Ala Arg Glu
1685 1690 1695

Ala Glu Lys Gln Leu Arg Glu Gln Val Gly Asp Gln Tyr Gln Thr
1700 1705 1710

Val Arg Ala Leu Ala Glu Arg Lys Ala Glu Gly Val Leu Ala Ala
1715 1720 1725

Gln Ala Arg Ala Glu Gln Leu Arg Asp Glu Ala Arg Asp Leu Leu
1730 1735 1740

Gln Ala Ala Gln Asp Lys Leu Gln Arg Leu Gln Glu Leu Glu Gly
1745 1750 1755

Thr Tyr Glu Glu Asn Glu Arg Ala Leu Glu Gly Lys Ala Ala Gln
1760 1765 1770

Leu Asp Gly Leu Glu Ala Arg Met Arg Ser Val Leu Gln Ala Ile
1775 1780 1785

Asn Leu Gln Val Gln Ile Tyr Asn Thr Cys Gln
1790 1795

<210> 9
<211> 5153
<212> DNA
<213> Mus musculus

<220>
<221> CDS
<222> (1)..(1476)
<223> laminin 12 gamma 3 chain

<400> 9
atg gct gta tcc agg gtc ctg tcc ctc ctg gca acg gtg gca tcg atg 48
Met Ala Val Ser Arg Val Leu Ser Leu Leu Ala Thr Val Ala Ser Met
1 5 10 15

gcg ctg gtg att cag gag aca cac ttc gcg gca ggc gcg gac atg ggc 96
Ala Leu Val Ile Gln Glu Thr His Phe Ala Ala Gly Ala Asp Met Gly
20 25 30

tct tgc tac gac ggt gtg gga cgc gca cag cgc tgt ctg cct gag ttc 144
Ser Cys Tyr Asp Gly Val Gly Arg Ala Gln Arg Cys Leu Pro Glu Phe
35 40 45

gag aac gcg gcg ttc ggc cga cgc gcc gag gcc tcc cac acg tgc gga 192
Glu Asn Ala Ala Phe Gly Arg Arg Ala Glu Ala Ser His Thr Cys Gly
50 55 60

cgg ccc ccg gag gac ttc tgt cca cac gtg ggg gca cca ggg gct ggg 240
Arg Pro Pro Glu Asp Phe Cys Pro His Val Gly Ala Pro Gly Ala Gly
65 70 75 80

cta cag tgc cag cgc tgc gac gat gct gac ccc gga cga cgc cac gac 288
Leu Gln Cys Gln Arg Cys Asp Asp Ala Asp Pro Gly Arg Arg His Asp

```
                 85                  90                  95
gcc tcc tac ctc aca gac ttc cac agc ccc gat gac agc acc tgg tgg      336
Ala Ser Tyr Leu Thr Asp Phe His Ser Pro Asp Asp Ser Thr Trp Trp
            100                 105                 110

cag agc cca tcc atg gcc ttc ggg gtg cag tac ccc acc tcg gtt aac      384
Gln Ser Pro Ser Met Ala Phe Gly Val Gln Tyr Pro Thr Ser Val Asn
        115                 120                 125

ctg acc ttg agc tta ggg aag gcc tat gag att acc tat gtg agg ctg      432
Leu Thr Leu Ser Leu Gly Lys Ala Tyr Glu Ile Thr Tyr Val Arg Leu
    130                 135                 140

aag ttc cac acc agt cgc cct gag agt ttt gcc atc tac aag cgc acg      480
Lys Phe His Thr Ser Arg Pro Glu Ser Phe Ala Ile Tyr Lys Arg Thr
145                 150                 155                 160

tac gcc agt ggc ccc tgg gag ccc tac caa tac tac agt gcc tcc tgc      528
Tyr Ala Ser Gly Pro Trp Glu Pro Tyr Gln Tyr Tyr Ser Ala Ser Cys
                165                 170                 175

cag aaa acc tat ggc cgt cct gag ggc cac tac ctg cga ccg ggc gag      576
Gln Lys Thr Tyr Gly Arg Pro Glu Gly His Tyr Leu Arg Pro Gly Glu
            180                 185                 190

gat gag agg gtg gcc ttc tgc acc tct gag ttc agt gac atc tcc ccc      624
Asp Glu Arg Val Ala Phe Cys Thr Ser Glu Phe Ser Asp Ile Ser Pro
        195                 200                 205

ttg aac ggg ggc aac gtg gcc ttc tcc acc ctg gaa ggc cgt ccc agt      672
Leu Asn Gly Gly Asn Val Ala Phe Ser Thr Leu Glu Gly Arg Pro Ser
    210                 215                 220

gcc tac aac ttt gag gag agc cct gtg ctg cag gag tgg gtc acc agc      720
Ala Tyr Asn Phe Glu Glu Ser Pro Val Leu Gln Glu Trp Val Thr Ser
225                 230                 235                 240

act gac atc ctg atc tct cta gat cgg ctc aac acg ttt ggg gat gac      768
Thr Asp Ile Leu Ile Ser Leu Asp Arg Leu Asn Thr Phe Gly Asp Asp
                245                 250                 255

atc ttc aag gac ccc aga gtg ctc cag tct tac tac tac gct gtg tct      816
Ile Phe Lys Asp Pro Arg Val Leu Gln Ser Tyr Tyr Tyr Ala Val Ser
            260                 265                 270

gac ttc tct gtg ggt ggc agg tgc aaa tgc aat ggt cac gcc agt gaa      864
Asp Phe Ser Val Gly Gly Arg Cys Lys Cys Asn Gly His Ala Ser Glu
        275                 280                 285

tgc gaa ccc aat gcg gct ggt cag ctg gct tgc cgc tgt cag cac aac      912
Cys Glu Pro Asn Ala Ala Gly Gln Leu Ala Cys Arg Cys Gln His Asn
    290                 295                 300

acc aca gga gtg gac tgc gag cgt tgt ctg ccc ttc ttc cag gac cgt      960
Thr Thr Gly Val Asp Cys Glu Arg Cys Leu Pro Phe Phe Gln Asp Arg
305                 310                 315                 320

ccg tgg gcc cga ggc acc gcc gag gat gcc aac gag tgt ctg ccc tgc     1008
Pro Trp Ala Arg Gly Thr Ala Glu Asp Ala Asn Glu Cys Leu Pro Cys
                325                 330                 335

aac tgc agt ggg cac tct gag gag tgc acg ttt gac agg gag ctc tat     1056
Asn Cys Ser Gly His Ser Glu Glu Cys Thr Phe Asp Arg Glu Leu Tyr
            340                 345                 350

cgg agc aca ggc cat ggt ggg cac tgt cag cgg tgc cgt gac cac aca     1104
Arg Ser Thr Gly His Gly Gly His Cys Gln Arg Cys Arg Asp His Thr
```

```
        355                 360                 365

act ggg cca cac tgt gag cgc tgt gag aag aac tac tac aga tgg tcc     1152
Thr Gly Pro His Cys Glu Arg Cys Glu Lys Asn Tyr Tyr Arg Trp Ser
    370                 375                 380

ccg aag aca cca tgc caa ccc tgt gac tgc cac cca gca ggc tct ctg     1200
Pro Lys Thr Pro Cys Gln Pro Cys Asp Cys His Pro Ala Gly Ser Leu
385                 390                 395                 400

agt ctc cag tgt gac aac tca ggc gtc tgt ccc tgc aag ccc aca gtg     1248
Ser Leu Gln Cys Asp Asn Ser Gly Val Cys Pro Cys Lys Pro Thr Val
                405                 410                 415

act ggc tgg aag tgt gac cgc tgc ctg cct gga ttc cac tca ctc agt     1296
Thr Gly Trp Lys Cys Asp Arg Cys Leu Pro Gly Phe His Ser Leu Ser
            420                 425                 430

gag ggc ggc tgc aga ccc tgt gcc tgc aat gtc gcc ggc agc ttg ggc     1344
Glu Gly Gly Cys Arg Pro Cys Ala Cys Asn Val Ala Gly Ser Leu Gly
        435                 440                 445

acc tgt gac ccc cgc agt ggg aac tgt ccc tgc aaa gag aat gta gaa     1392
Thr Cys Asp Pro Arg Ser Gly Asn Cys Pro Cys Lys Glu Asn Val Glu
    450                 455                 460

ggc agc ctg tgt gac aga tgc cgc cct ggg aca ttt aac ctg cag ccc     1440
Gly Ser Leu Cys Asp Arg Cys Arg Pro Gly Thr Phe Asn Leu Gln Pro
465                 470                 475                 480

cac aat cca gtg ggc tgc agc agc tgc ttc tgt tat ggccactcca          1486
His Asn Pro Val Gly Cys Ser Ser Cys Phe Cys Tyr
                485                 490

aggtgtgttc tcctgctgcc gggttccagg aacaccacat ccgctcagac ttccgccatg   1546
gagctggtgg ctggcagatc agaagcatgg gagtgtccaa gcgtcctctg caatggagcc   1606
agagtgggct cctcctgggc ctgcgaggag gggaggaact ctcagcccca aagaagttcc   1666
tgggagacca gagactcagc tatggacagc cagtcatact gaccctccaa gtacccccctg  1726
gaggctcccc acctcctatt cagctgagac tggagggagc aggcttggct ctgtctctga   1786
ggccctccag tctacccagc cctcaggaca ccaggcagcc aagacgagtt cagctccagt   1846
tcctcttgca ggagacttct gaggaggcag agtccccact gcccaccttc cacttccagc   1906
gcctgctttc caatctgact gctctgagca tctggaccag tggccaagga ccgggccatt   1966
ctggccaagt gctcttgtgt gaagttcagc tcacatcggc ctggccccag cgtgagcttg   2026
cccctccagc ctcttgggtg gagacctgct tatgtcccca gggatacaca ggccagttct   2086
gtgaattctg tgctctggga tacaagagag aaatacctca tgggggtccc tatgccaact   2146
gcattccctg cacctgcaac cagcatggca cctgtgaccc caacacaggg atctgcctgt   2206
gtggccacca caccgagggt ccatcctgtg agcggtgcat gccaggtttc tacggtaacg   2266
ccttctcagg ccgtgctgat gattgccagc cctgtccgtg ccctggccaa tcagcctgtg   2326
caaccatccc agagagtgga gatgtggtgt gcacacactg ccctcctggt cagagaggac   2386
gacgatgcga gagctgcgaa gatggctttt ttggggatcc tctagggctc tctggagctc   2446
cccagccctg ccgccgatgc cagtgcagcg ggaacgtgga tctcaatgct gtgggcaact   2506
```

gtgatcctca ttctggccac tgcttgcgct gtctgtacaa cacgacaggg gcccactgcg 2566
agcactgtcg ggagggtttc tacgggagtg ccgtggccac aaggcccgtg gacaaatgtg 2626
ctccctgcag ctgtgacctg aggggctcag tcagtgagaa gacctgcaac cctgtgactg 2686
gccagtgtgt ctgcctgcct tatgtctccg ggagggactg cagccgctgc agccctggct 2746
tctatgacct ccagtctggg aggggctgcc agagctgcaa atgtcaccca cttggatcct 2806
tggagaataa gtgccacccc aagactggcc agtgtccctg ccgacctggt gtcactggcc 2866
aagcctgtga cagatgccag ctaggtttct ttggcttctc catcaagggc tgccgagact 2926
gtaggtgctc cccattgggt gctgcctcat ctcagtgcca tgagaacagc acctgtgtgt 2986
gccggcccgg ctttgtgggc tataaatgcg accgctgcca ggacaatttc ttcctcgcgg 3046
atggcgacac aggctgccaa gagtgtccca cttgctatgc cctagtgaag gaagaggcag 3106
ccaagctgaa ggccaggttg atgctgatgg aggggtggct tcaaaggtct gactgtggta 3166
gcccctgggg accactagac attctgcagg gagaagcccc tctgggggat gtctaccaag 3226
gtcaccacct acttcaagag acccggggga ccttcctgca gcagatggtg ggcctggagg 3286
attctgtgaa ggccacttgg gagcagttgc aggtgctgag agggcatgta cactgtgccc 3346
aggctggagc tcagaagacc tgcatccagc tggcagagct ggaggagaca ttgcagtcct 3406
cagaggagga ggtccttcgt gcagcctcag ctctctcatt tctggcaagt cttcagaaag 3466
gatccagcac acccaccaat tggagtcacc tggcatcaga ggcccagatc cttgccagaa 3526
gccacaggga cacggccacc aagatcgaag ctacctcgga aagggccctg ctcgcctcca 3586
acgccagcta tgagctcctg aagctgatgg aaggcagagt ggcctcggaa gcccagcagg 3646
aactggagga caggtaccag gaggtgcagg cagctcagac tgccctgggc atagctgtgg 3706
cagaggcgct gcccaaagct gaaaaggcac tggccacggt gaagcaagtc attggtgacg 3766
cagccccaca tctaggcttg ctggtcaccc ctgaagcaat gaacttccaa gccaggggcc 3826
tgagctggaa agtgaaggcc ctggagcaga agctggagca gaaggagccc gaggtgggcc 3886
agtctgtggg agccctgcag gtggaggctg gaagagcctt ggagaagatg gagcccttta 3946
tgcagctacg caataagacc acagctgcct tcacacgggc ttcctcagct gtgcaagctg 4006
ccaaggtgac cgtcatagga gcagagaccc tgctagctga cctagaggga atgaagctga 4066
ggtctcctct acccaaggag caggcagcgc tgaagaagaa agcaggcagc atcaggacca 4126
ggctcctgga ggacacaaag aggaagacca agcatgcaga gaggatgctg ggaaatgctg 4186
cctctctctc ctccagcacc aagaagaaaa gcaaagaagc agaactgatg tctaaggaca 4246
atgccaagct ctccagagct ttgctgaggg aaggcaagca gggctaccgt catgccagcc 4306
gactcgccag ccagacccag gccacactcc gtcgggcctc tcgcctgctg ctgacctcag 4366
aagcacacaa gcaggagctg gaggaagcta aacaggtgac ctctgggctg agcactgtgg 4426
agcgccaggt ccgagagtct cggatctcct tggagaagga caccaaggtc ctgtcagagc 4486
tgcttgtgaa gctggggtcc ctgggtgtcc accaagcccc tgctcagacc ctgaacgaga 4546

cccagcgggc actagaaagc ttgaggctgc agctggattc ccacggagcc ctgcatcaca 4606

aactgaggca gctggaggaa gagtctgctc gacaggagct gcagattcag agctttgagg 4666

acgaccttgc tgagatccgc gctgacaagc acaacttgga gaccattctg agcagtctgc 4726

cagagaactg tgccagctag accctggtac accctcccca ccctgccgtt tcctgtccac 4786

tccctgtagg tgtcccaggt ctgcctgtcg tatgttcacg tgaatgcttg tttgctggtg 4846

catcttcggt ctgagcagga gtgaatacat gctcacacct ccacagatga ccctgtatgt 4906

agtcctcagt gtgtactctc taaacgtgca tcagcataca caccccagta tttgcacata 4966

tgtgtatgtg atgcactgat gtgttaagac cacctgtgtg catgcacaca tatgagagtc 5026

tagagctgtg gagagcagtc ctgagcttgg cacatccaca ttctggtggg ttcctgctat 5086

gaatatcctg caggatgaca catctacacc tcctcagaat cagggccaac aggtgtactc 5146

gagctga 5153

<210> 10
<211> 492
<212> PRT
<213> Mus musculus

<400> 10

Met Ala Val Ser Arg Val Leu Ser Leu Leu Ala Thr Val Ala Ser Met
1 5 10 15

Ala Leu Val Ile Gln Glu Thr His Phe Ala Ala Gly Ala Asp Met Gly
20 25 30

Ser Cys Tyr Asp Gly Val Gly Arg Ala Gln Arg Cys Leu Pro Glu Phe
35 40 45

Glu Asn Ala Ala Phe Gly Arg Arg Ala Glu Ala Ser His Thr Cys Gly
50 55 60

Arg Pro Pro Glu Asp Phe Cys Pro His Val Gly Ala Pro Gly Ala Gly
65 70 75 80

Leu Gln Cys Gln Arg Cys Asp Asp Ala Asp Pro Gly Arg Arg His Asp
85 90 95

Ala Ser Tyr Leu Thr Asp Phe His Ser Pro Asp Asp Ser Thr Trp Trp
100 105 110

Gln Ser Pro Ser Met Ala Phe Gly Val Gln Tyr Pro Thr Ser Val Asn
115 120 125

Leu Thr Leu Ser Leu Gly Lys Ala Tyr Glu Ile Thr Tyr Val Arg Leu
130 135 140

Lys Phe His Thr Ser Arg Pro Glu Ser Phe Ala Ile Tyr Lys Arg Thr

145 150 155 160

Tyr Ala Ser Gly Pro Trp Glu Pro Tyr Gln Tyr Tyr Ser Ala Ser Cys
165 170 175

Gln Lys Thr Tyr Gly Arg Pro Glu Gly His Tyr Leu Arg Pro Gly Glu
180 185 190

Asp Glu Arg Val Ala Phe Cys Thr Ser Glu Phe Ser Asp Ile Ser Pro
195 200 205

Leu Asn Gly Gly Asn Val Ala Phe Ser Thr Leu Glu Gly Arg Pro Ser
210 215 220

Ala Tyr Asn Phe Glu Glu Ser Pro Val Leu Gln Glu Trp Val Thr Ser
225 230 235 240

Thr Asp Ile Leu Ile Ser Leu Asp Arg Leu Asn Thr Phe Gly Asp Asp
245 250 255

Ile Phe Lys Asp Pro Arg Val Leu Gln Ser Tyr Tyr Tyr Ala Val Ser
260 265 270

Asp Phe Ser Val Gly Gly Arg Cys Lys Cys Asn Gly His Ala Ser Glu
275 280 285

Cys Glu Pro Asn Ala Ala Gly Gln Leu Ala Cys Arg Cys Gln His Asn
290 295 300

Thr Thr Gly Val Asp Cys Glu Arg Cys Leu Pro Phe Phe Gln Asp Arg
305 310 315 320

Pro Trp Ala Arg Gly Thr Ala Glu Asp Ala Asn Glu Cys Leu Pro Cys
325 330 335

Asn Cys Ser Gly His Ser Glu Glu Cys Thr Phe Asp Arg Glu Leu Tyr
340 345 350

Arg Ser Thr Gly His Gly Gly His Cys Gln Arg Cys Arg Asp His Thr
355 360 365

Thr Gly Pro His Cys Glu Arg Cys Glu Lys Asn Tyr Tyr Arg Trp Ser
370 375 380

Pro Lys Thr Pro Cys Gln Pro Cys Asp Cys His Pro Ala Gly Ser Leu
385 390 395 400

Ser Leu Gln Cys Asp Asn Ser Gly Val Cys Pro Cys Lys Pro Thr Val
405 410 415

Thr Gly Trp Lys Cys Asp Arg Cys Leu Pro Gly Phe His Ser Leu Ser

420 425 430

Glu Gly Gly Cys Arg Pro Cys Ala Cys Asn Val Ala Gly Ser Leu Gly
435 440 445

Thr Cys Asp Pro Arg Ser Gly Asn Cys Pro Cys Lys Glu Asn Val Glu
450 455 460

Gly Ser Leu Cys Asp Arg Cys Arg Pro Gly Thr Phe Asn Leu Gln Pro
465 470 475 480

His Asn Pro Val Gly Cys Ser Ser Cys Phe Cys Tyr
485 490

<210> 11
<211> 2265
<212> PRT
<213> Bos taurus

<400> 11

Gln Ala Gln Gln Ile Val Gln Pro Gln Ser Pro Leu Thr Val Ser Gln
1 5 10 15

Ser Lys Pro Gly Cys Tyr Asp Asn Gly Lys His Tyr Gln Ile Asn Gln
20 25 30

Gln Trp Glu Arg Thr Tyr Leu Gly Ser Ala Leu Val Cys Thr Cys Tyr
35 40 45

Gly Gly Ser Arg Gly Phe Asn Cys Glu Ser Lys Pro Glu Pro Glu Glu
50 55 60

Thr Cys Phe Asp Lys Tyr Thr Gly Asn Thr Tyr Arg Val Gly Asp Thr
65 70 75 80

Tyr Glu Arg Pro Lys Asp Ser Met Ile Trp Asp Cys Thr Cys Ile Gly
85 90 95

Ala Gly Arg Gly Arg Ile Ser Cys Thr Ile Ala Asn Arg Cys His Glu

100 105 110

Gly Gly Gln Ser Tyr Lys Ile Gly Asp Thr Trp Arg Arg Pro His Glu
115 120 125

Thr Gly Gly Tyr Met Leu Glu Cys Val Cys Leu Gly Asn Gly Lys Gly
130 135 140

Glu Trp Thr Cys Lys Pro Ile Ala Glu Lys Cys Phe Asp Gln Ala Ala
145 150 155 160

Gly Thr Ser Tyr Val Val Gly Glu Thr Trp Glu Lys Pro Tyr Gln Gly

165 170 175

Trp Met Met Val Asp Cys Thr Cys Leu Gly Glu Gly Ser Gly Arg Ile
180 185 190

Thr Cys Thr Ser Arg Asn Arg Cys Asn Asp Gln Asp Thr Arg Thr Ser
195 200 205

Tyr Arg Ile Gly Asp Thr Trp Ser Lys Lys Asp Asn Arg Gly Asn Leu
210 215 220

Leu Gln Cys Ile Cys Thr Gly Asn Gly Arg Gly Glu Trp Lys Cys Glu
225 230 235 240

Arg His Thr Ser Leu Gln Thr Thr Ser Ala Gly Ser Gly Ser Phe Thr
245 250 255

Asp Val Arg Thr Ala Ile Tyr Gln Pro Gln Pro His Pro Gln Pro Pro
260 265 270

Pro Tyr Gly His Cys Val Thr Asp Ser Gly Val Val Tyr Ser Val Gly
275 280 285

Met Gln Trp Leu Lys Thr Gln Gly Asn Lys Gln Met Leu Cys Thr Cys
290 295 300

Leu Gly Asn Gly Val Ser Cys Gln Glu Thr Ala Val Thr Gln Thr Tyr
305 310 315 320

Gly Gly Asn Ser Asn Gly Glu Pro Cys Val Leu Pro Phe Thr Tyr Asn
325 330 335

Gly Lys Thr Phe Tyr Ser Cys Thr Thr Glu Gly Arg Gln Asp Gly His
340 345 350

Leu Trp Cys Ser Thr Thr Ser Asn Tyr Glu Gln Asp Gln Lys Tyr Ser
355 360 365

Phe Cys Thr Asp His Thr Val Leu Val Gln Thr Arg Gly Gly Asn Ser
370 375 380

Asn Gly Ala Leu Cys His Phe Pro Phe Leu Tyr Asn Asn His Asn Tyr
385 390 395 400

Thr Asp Cys Thr Ser Glu Gly Arg Arg Asp Asn Met Lys Trp Cys Gly
405 410 415

Thr Thr Gln Asn Tyr Asp Ala Asp Gln Lys Phe Gly Phe Cys Pro Met
420 425 430

Ala Ala His Glu Glu Ile Cys Thr Thr Asn Glu Gly Val Met Tyr Arg

435 440 445

Ile Gly Asp Gln Trp Asp Lys Gln His Asp Met Gly His Met Met Arg
450 455 460

Cys Thr Cys Val Gly Asn Gly Arg Gly Glu Trp Thr Cys Val Ala Tyr
465 470 475 480

Ser Gln Leu Arg Asp Gln Cys Ile Val Asp Gly Ile Thr Tyr Asn Val
485 490 495

Asn Asp Thr Phe His Lys Arg His Glu Glu Gly His Met Leu Asn Cys
500 505 510

Thr Cys Phe Gly Gln Gly Arg Gly Arg Trp Lys Cys Asp Pro Val Asp
515 520 525

Gln Cys Gln Asp Ser Glu Thr Arg Thr Phe Tyr Gln Ile Gly Asp Ser
530 535 540

Trp Glu Lys Tyr Leu Gln Gly Val Arg Tyr Gln Cys Tyr Cys Tyr Gly
545 550 555 560

Arg Gly Ile Gly Glu Trp Ala Cys Gln Pro Leu Gln Thr Tyr Pro Asp
565 570 575

Thr Ser Gly Pro Val Gln Val Ile Ile Thr Glu Thr Pro Ser Gln Pro
580 585 590

Asn Ser His Pro Ile Gln Trp Ser Ala Pro Glu Ser Ser His Ile Ser
595 600 605

Lys Tyr Ile Leu Arg Trp Lys Pro Lys Asn Ser Pro Asp Arg Trp Lys

610 615 620

Glu Ala Thr Ile Pro Gly His Leu Asn Ser Tyr Thr Ile Lys Gly Leu
625 630 635 640

Arg Pro Gly Val Val Tyr Glu Gly Gln Leu Ile Ser Val Gln His Tyr
645 650 655

Gly Gln Arg Glu Val Thr Arg Phe Asp Phe Thr Thr Thr Ser Thr Ser
660 665 670

Pro Ala Val Thr Ser Asn Thr Val Thr Gly Glu Thr Thr Pro Leu Ser
675 680 685

Pro Val Val Ala Thr Ser Glu Ser Val Thr Glu Ile Thr Ala Ser Ser
690 695 700

Phe Val Val Ser Trp Val Ser Ala Ser Asp Thr Val Ser Gly Phe Arg
705 710 715 720

Val Glu Tyr Glu Leu Ser Glu Glu Gly Asp Glu Pro Gln Tyr Leu Asp
725 730 735

Leu Pro Ser Thr Ala Thr Ser Val Asn Ile Pro Asp Leu Leu Pro Gly
740 745 750

Arg Lys Tyr Thr Val Asn Val Tyr Glu Ile Ser Glu Glu Gly Glu Gln
755 760 765

Asn Leu Ile Leu Ser Thr Ser Gln Thr Thr Ala Pro Asp Ala Pro Pro
770 775 780

Asp Pro Thr Val Asp Gln Val Asp Asp Thr Ser Ile Val Val Arg Trp
785 790 795 800

Ser Arg Pro Arg Ala Pro Ile Thr Gly Tyr Arg Ile Val Tyr Ser Pro
805 810 815

Ser Val Glu Gly Ser Ser Thr Glu Leu Asn Leu Pro Glu Thr Ala Asn
820 825 830

Ser Val Thr Leu Ser Asp Leu Gln Pro Gly Val Gln Tyr Asn Ile Thr
835 840 845

Ile Tyr Ala Val Glu Glu Asn Gln Glu Ser Thr Pro Val Phe Ile Gln
850 855 860

Gln Glu Thr Thr Gly Val Pro Arg Ser Asp Lys Val Pro Pro Pro Arg
865 870 875 880

Asp Leu Gln Phe Val Glu Val Thr Asp Val Lys Ile Thr Ile Met Trp
885 890 895

Thr Pro Pro Glu Ser Pro Val Thr Gly Tyr Arg Val Asp Val Ile Pro
900 905 910

Val Asn Leu Pro Gly Glu His Gly Gln Arg Leu Pro Val Ser Arg Asn
915 920 925

Thr Phe Ala Glu Val Thr Gly Leu Ser Pro Gly Val Thr Tyr His Phe
930 935 940

Lys Val Phe Ala Val Asn Gln Gly Arg Glu Ser Lys Pro Leu Thr Ala
945 950 955 960

Gln Gln Ala Thr Lys Leu Asp Ala Pro Thr Asn Leu Gln Phe Ile Asn
965 970 975

Glu Thr Asp Thr Thr Val Ile Val Thr Trp Thr Pro Pro Arg Ala Arg
980 985 990

Ile Val Gly Tyr Arg Leu Thr Val Gly Leu Thr Arg Gly Gly Gln Pro
995 1000 1005

Lys Gln Tyr Asn Val Gly Pro Ala Ala Ser Gln Tyr Pro Leu Arg
1010 1015 1020

Asn Leu Gln Pro Gly Ser Glu Tyr Ala Val Ser Leu Val Ala Val
1025 1030 1035

Lys Gly Asn Gln Gln Ser Pro Arg Val Thr Gly Val Phe Thr Thr
1040 1045 1050

Leu Gln Pro Leu Gly Ser Ile Pro His Tyr Asn Thr Glu Val Thr
1055 1060 1065

Glu Thr Thr Ile Val Ile Thr Trp Thr Pro Ala Pro Arg Ile Gly
1070 1075 1080

Phe Lys Leu Gly Val Arg Pro Ser Gln Gly Gly Glu Ala Pro Arg
1085 1090 1095

Glu Val Thr Ser Glu Ser Gly Ser Ile Val Val Ser Gly Leu Thr
1100 1105 1110

Pro Gly Val Glu Tyr Val Tyr Thr Ile Ser Val Leu Arg Asp Gly
1115 1120 1125

Gln Glu Arg Asp Ala Pro Ile Val Lys Lys Val Val Thr Pro Leu
1130 1135 1140

Ser Pro Pro Thr Asn Leu His Leu Glu Ala Asn Pro Asp Thr Gly
1145 1150 1155

Val Leu Thr Val Ser Trp Glu Arg Ser Thr Thr Pro Asp Ile Thr
1160 1165 1170

Gly Tyr Arg Ile Thr Thr Thr Pro Thr Asn Gly Gln Gln Gly Tyr
1175 1180 1185

Ser Leu Glu Glu Val Val His Ala Asp Gln Ser Ser Cys Thr Phe
1190 1195 1200

Glu Asn Leu Ser Pro Gly Leu Glu Tyr Asn Val Ser Val Tyr Thr
1205 1210 1215

Val Lys Asp Asp Lys Glu Ser Val Pro Ile Ser Asp Thr Ile Ile
1220 1225 1230

Pro Ala Val Pro Pro Pro Thr Asp Leu Arg Phe Thr Asn Val Gly
1235 1240 1245

Pro Asp Thr Met Arg Val Thr Trp Ala Pro Pro Ser Ser Ile Glu
1250 1255 1260

Leu Thr Asn Leu Leu Val Arg Tyr Ser Pro Val Lys Asn Glu Glu
1265 1270 1275

Asp Val Ala Glu Leu Ser Ile Ser Pro Ser Asp Asn Ala Val Val
1280 1285 1290

Leu Thr Asn Leu Leu Pro Gly Thr Glu Tyr Leu Val Ser Val Ser
1295 1300 1305

Ser Val Tyr Glu Gln His Glu Ser Ile Pro Leu Arg Gly Arg Gln
1310 1315 1320

Lys Thr Ala Leu Asp Ser Pro Ser Gly Ile Asp Phe Ser Asp Ile
1325 1330 1335

Thr Ala Asn Ser Phe Thr Val His Trp Ile Ala Pro Arg Ala Thr
1340 1345 1350

Ile Thr Gly Tyr Arg Ile Arg His His Pro Glu Asn Met Gly Gly
1355 1360 1365

Arg Pro Arg Glu Asp Arg Val Pro Pro Ser Arg Asn Ser Ile Thr
1370 1375 1380

Leu Thr Asn Leu Asn Pro Gly Thr Glu Tyr Val Val Ser Ile Val
1385 1390 1395

Ala Leu Asn Ser Lys Glu Glu Ser Leu Pro Leu Val Gly Gln Gln
1400 1405 1410

Ser Thr Val Ser Asp Val Pro Arg Asp Leu Glu Val Ile Ala Ala
1415 1420 1425

Thr Pro Thr Ser Leu Leu Ile Ser Trp Asp Ala Pro Ala Val Thr
1430 1435 1440

Val Arg Tyr Tyr Arg Ile Thr Tyr Gly Glu Thr Gly Gly Ser Ser
1445 1450 1455

Pro Val Gln Glu Phe Thr Val Pro Gly Ser Lys Ser Thr Ala Thr
1460 1465 1470

Ile Ser Gly Leu Lys Pro Gly Val Asp Tyr Thr Ile Thr Val Tyr
1475 1480 1485

Ala Val Thr Gly Arg Gly Asp Ser Pro Ala Ser Ser Lys Pro Val
1490 1495 1500

Ser Ile Asn Tyr Arg Thr Glu Ile Asp Lys Pro Ser Gln Met Gln
1505 1510 1515

Val Thr Asp Val Gln Asp Asn Ser Ile Ser Val Arg Trp Leu Pro
1520 1525 1530

Ser Ser Ser Pro Val Thr Gly Tyr Arg Val Thr Thr Ala Pro Lys
1535 1540 1545

Asn Gly Pro Gly Pro Ser Lys Thr Lys Thr Val Gly Pro Asp Gln
1550 1555 1560

Thr Glu Met Thr Ile Glu Gly Leu Gln Pro Thr Val Glu Tyr Val
1565 1570 1575

Val Ser Val Tyr Ala Gln Asn Gln Asn Gly Glu Ser Gln Pro Leu
1580 1585 1590

Val Gln Thr Ala Val Thr Thr Ile Pro Ala Pro Thr Asn Leu Lys
1595 1600 1605

Phe Thr Gln Val Thr Pro Thr Ser Leu Thr Ala Gln Trp Thr Ala
1610 1615 1620

Pro Asn Val Gln Leu Thr Gly Tyr Arg Val Arg Val Thr Pro Lys
1625 1630 1635

Glu Lys Thr Gly Pro Met Lys Glu Ile Asn Leu Ala Pro Asp Ser
1640 1645 1650

Ser Ser Val Val Val Ser Gly Leu Met Val Ala Thr Lys Tyr Glu
1655 1660 1665

Val Ser Val Tyr Ala Leu Lys Asp Thr Leu Thr Ser Arg Pro Ala
1670 1675 1680

Gln Gly Val Val Thr Thr Leu Glu Asn Val Ser Pro Pro Arg Arg
1685 1690 1695

Ala Arg Val Thr Asp Ala Thr Glu Thr Thr Ile Thr Ile Ser Trp
1700 1705 1710

Arg Thr Lys Thr Glu Thr Ile Thr Gly Phe Gln Val Asp Ala Ile
1715 1720 1725

Pro Ala Asn Gly Gln Thr Pro Ile Gln Arg Thr Ile Arg Pro Asp
1730 1735 1740

Val Arg Ser Tyr Thr Ile Thr Gly Leu Gln Pro Gly Thr Asp Tyr
1745 1750 1755

Lys Ile His Leu Tyr Thr Leu Asn Asp Asn Ala Arg Ser Ser Pro
1760 1765 1770

Val Val Ile Asp Ala Ser Thr Ala Ile Asp Ala Pro Ser Asn Leu
1775 1780 1785

Arg Phe Leu Ala Thr Thr Pro Asn Ser Leu Leu Val Ser Trp Gln
1790 1795 1800

Pro Pro Arg Ala Arg Ile Thr Gly Tyr Ile Ile Lys Tyr Glu Lys
1805 1810 1815

Pro Gly Ser Pro Pro Arg Glu Val Val Pro Arg Pro Arg Pro Gly
1820 1825 1830

Val Thr Glu Ala Thr Ile Thr Gly Leu Glu Pro Gly Thr Glu Tyr
1835 1840 1845

Thr Ile Gln Val Ile Ala Leu Lys Asn Asn Gln Lys Ser Glu Pro
1850 1855 1860

Leu Ile Gly Arg Lys Lys Thr Asp Glu Leu Pro Gln Leu Val Thr
1865 1870 1875

Leu Pro His Pro Asn Leu His Gly Pro Glu Ile Leu Asp Val Pro
1880 1885 1890

Ser Thr Val Gln Lys Thr Pro Phe Ile Thr Asn Pro Gly Tyr Asp
1895 1900 1905

Thr Gly Asn Gly Ile Gln Leu Pro Gly Thr Ser Gly Gln Gln Pro
1910 1915 1920

Ser Leu Gly Gln Gln Met Ile Phe Glu Glu His Gly Phe Arg Arg
1925 1930 1935

Thr Thr Pro Pro Thr Thr Ala Thr Pro Val Arg His Arg Pro Arg
1940 1945 1950

Pro Tyr Pro Pro Asn Val Asn Glu Glu Ile Gln Ile Gly His Val
1955 1960 1965

Pro Arg Gly Asp Val Asp His His Leu Tyr Pro His Val Val Gly
1970 1975 1980

Leu Asn Pro Asn Ala Ser Thr Gly Gln Glu Ala Leu Ser Gln Thr
1985 1990 1995

Thr Ile Ser Trp Thr Pro Phe Gln Glu Ser Ser Glu Tyr Ile Ile
2000 2005 2010

Ser Cys His Pro Val Gly Ile Asp Glu Glu Pro Leu Gln Phe Arg
2015 2020 2025

Val Pro Gly Thr Ser Ala Ser Ala Thr Leu Thr Gly Leu Thr Arg
2030 2035 2040

Gly Ala Thr Tyr Asn Ile Ile Val Glu Ala Val Lys Asp Gln Gln
2045 2050 2055

Arg Gln Lys Val Arg Glu Glu Val Val Thr Val Gly Asn Ser Val
2060 2065 2070

Asp Gln Gly Leu Ser Gln Pro Thr Asp Asp Ser Cys Phe Asp Pro
2075 2080 2085

Tyr Thr Val Ser His Tyr Ala Ile Gly Glu Glu Trp Glu Arg Leu
2090 2095 2100

Ser Asp Ser Gly Phe Lys Leu Ser Cys Gln Cys Leu Gly Phe Gly
2105 2110 2115

Ser Gly His Phe Arg Cys Asp Ser Ser Lys Trp Cys His Asp Asn
2120 2125 2130

Gly Val Asn Tyr Lys Ile Gly Glu Lys Trp Asp Arg Gln Gly Glu
2135 2140 2145

Asn Gly Gln Met Met Ser Cys Thr Cys Leu Gly Asn Gly Lys Gly
2150 2155 2160

Glu Phe Lys Cys Asp Pro His Glu Ala Thr Cys Tyr Asp Asp Gly
2165 2170 2175

Lys Thr Tyr His Val Gly Glu Gln Trp Gln Lys Glu Tyr Leu Gly
2180 2185 2190

Ala Ile Cys Ser Cys Thr Cys Phe Gly Gly Gln Arg Gly Trp Arg
2195 2200 2205

Cys Asp Asn Cys Arg Arg Pro Gly Ala Glu Pro Gly Asn Glu Gly
2210 2215 2220

Ser Thr Ala His Ser Tyr Asn Gln Tyr Ser Gln Arg Tyr His Gln
2225 2230 2235

Arg Thr Asn Thr Asn Val Asn Cys Pro Ile Glu Cys Phe Met Pro
2240 2245 2250

```
Leu Asp  Val Gln Ala Asp Arg  Glu Asp Ser Arg Glu
    2255                 2260                2265

<210>  12
<211>  21
<212>  RNA
<213>  Artificial

<220>
<223>  siRNA used in the Examples

<400>  12
aagcagcagg acuucuucaa g                                                 21

<210>  13
<211>  984
<212>  DNA
<213>  Mus musculus

<300>
<308>  AF106007
<309>  1999-02-08
<313>  (1)..(984)


<220>
<221>  CDS
<222>  (1)..(984)

<400>  13
atg gag cga agg aac cac act ggg aga gtg agt gaa ttt gtg ttg ctg      48
Met Glu Arg Arg Asn His Thr Gly Arg Val Ser Glu Phe Val Leu Leu
1               5                   10                  15

ggt ttc cca gct cct gcc cca ctg cgg gca cta cta ttt ttc ctt tct      96
Gly Phe Pro Ala Pro Ala Pro Leu Arg Ala Leu Leu Phe Phe Leu Ser
            20                  25                  30

ctg ttg gcc tac gtg ttg gtg ctg act gaa aac ata ctc atc att aca     144
Leu Leu Ala Tyr Val Leu Val Leu Thr Glu Asn Ile Leu Ile Ile Thr
        35                  40                  45

gca att agg aac cac ccc acc ctc cac aaa ccc atg tat ttt ttc ttg     192
Ala Ile Arg Asn His Pro Thr Leu His Lys Pro Met Tyr Phe Phe Leu
    50                  55                  60

gct aat atg tca ttc ctg gag att tgg tat gtc act gtt acg att cct     240
Ala Asn Met Ser Phe Leu Glu Ile Trp Tyr Val Thr Val Thr Ile Pro
65                  70                  75                  80

aag atg ctt gct ggc ttc att ggt tcc gag gag aat cat gga cag ctg     288
Lys Met Leu Ala Gly Phe Ile Gly Ser Glu Glu Asn His Gly Gln Leu
                85                  90                  95

atc tcc ttt gag gca tgc atg aca cag ctc tac ttt ttc cta ggc ttg     336
Ile Ser Phe Glu Ala Cys Met Thr Gln Leu Tyr Phe Phe Leu Gly Leu
            100                 105                 110

ggt tgc aca gag tgt gtc ctt ctt gct gtc atg gcc tat gac cgc tat     384
Gly Cys Thr Glu Cys Val Leu Leu Ala Val Met Ala Tyr Asp Arg Tyr
        115                 120                 125

gtg gcc atc tgt cac cca ctc cac tat cct gtc att gtc agt agc cgg     432
Val Ala Ile Cys His Pro Leu His Tyr Pro Val Ile Val Ser Ser Arg
    130                 135                 140

cta tgt gtg cag atg gca gct gga tcc tgg gct gga ggt ttt ggt atc     480
```

```
Leu Cys Val Gln Met Ala Ala Gly Ser Trp Ala Gly Gly Phe Gly Ile
145                 150                 155                 160

tcc atg gtt aaa gtt ttc ctc att tct cgc ctg tct tac tgt ggc ccc      528
Ser Met Val Lys Val Phe Leu Ile Ser Arg Leu Ser Tyr Cys Gly Pro
                165                 170                 175

aac acc atc aac cac ttt ttc tgt gat gtt tct cca ttg ctc aac ttg      576
Asn Thr Ile Asn His Phe Phe Cys Asp Val Ser Pro Leu Leu Asn Leu
            180                 185                 190

tca tgc act gac atg tcc aca gca gag ctt aca gac ttt atc ctg gcc      624
Ser Cys Thr Asp Met Ser Thr Ala Glu Leu Thr Asp Phe Ile Leu Ala
        195                 200                 205

att ttt att ctg ctg ggg cca ctc tct gtc act ggg gct tcc tat atg      672
Ile Phe Ile Leu Leu Gly Pro Leu Ser Val Thr Gly Ala Ser Tyr Met
    210                 215                 220

gcc atc aca ggt gca gtg atg cgc atc ccc tca gct gct ggc cgc cat      720
Ala Ile Thr Gly Ala Val Met Arg Ile Pro Ser Ala Ala Gly Arg His
225                 230                 235                 240

aag gcc ttt tca acc tgt gcc tcc cac ctc act gtt gtg att atc ttc      768
Lys Ala Phe Ser Thr Cys Ala Ser His Leu Thr Val Val Ile Ile Phe
                245                 250                 255

tat gca gcc agt att ttc atc tat gcc agg cct aag gca ctc tca gct      816
Tyr Ala Ala Ser Ile Phe Ile Tyr Ala Arg Pro Lys Ala Leu Ser Ala
            260                 265                 270

ttt gac acc aac aag ctg gtc tct gta ctc tac gct gtc att gta cca      864
Phe Asp Thr Asn Lys Leu Val Ser Val Leu Tyr Ala Val Ile Val Pro
        275                 280                 285

ttg ctc aat ccc atc atc tac tgc ttg cgc aat caa gaa gtc aaa aaa      912
Leu Leu Asn Pro Ile Ile Tyr Cys Leu Arg Asn Gln Glu Val Lys Lys
    290                 295                 300

gcc cta cgt cgc act ctg cac ctg gcc caa ggc cag gac gcc aat acc      960
Ala Leu Arg Arg Thr Leu His Leu Ala Gln Gly Gln Asp Ala Asn Thr
305                 310                 315                 320

aag aaa tcc agc aga gat ggt tag                                      984
Lys Lys Ser Ser Arg Asp Gly
                325


<210>  14
<211>  327
<212>  PRT
<213>  Mus musculus

<400>  14

Met Glu Arg Arg Asn His Thr Gly Arg Val Ser Glu Phe Val Leu Leu
1               5                   10                  15


Gly Phe Pro Ala Pro Ala Pro Leu Arg Ala Leu Leu Phe Phe Leu Ser
            20                  25                  30


Leu Leu Ala Tyr Val Leu Val Leu Thr Glu Asn Ile Leu Ile Ile Thr
        35                  40                  45


Ala Ile Arg Asn His Pro Thr Leu His Lys Pro Met Tyr Phe Phe Leu
```

50 55 60

Ala Asn Met Ser Phe Leu Glu Ile Trp Tyr Val Thr Val Thr Ile Pro
65 70 75 80

Lys Met Leu Ala Gly Phe Ile Gly Ser Glu Glu Asn His Gly Gln Leu
85 90 95

Ile Ser Phe Glu Ala Cys Met Thr Gln Leu Tyr Phe Phe Leu Gly Leu
100 105 110

Gly Cys Thr Glu Cys Val Leu Leu Ala Val Met Ala Tyr Asp Arg Tyr
115 120 125

Val Ala Ile Cys His Pro Leu His Tyr Pro Val Ile Val Ser Ser Arg
130 135 140

Leu Cys Val Gln Met Ala Ala Gly Ser Trp Ala Gly Gly Phe Gly Ile
145 150 155 160

Ser Met Val Lys Val Phe Leu Ile Ser Arg Leu Ser Tyr Cys Gly Pro
165 170 175

Asn Thr Ile Asn His Phe Phe Cys Asp Val Ser Pro Leu Leu Asn Leu
180 185 190

Ser Cys Thr Asp Met Ser Thr Ala Glu Leu Thr Asp Phe Ile Leu Ala
195 200 205

Ile Phe Ile Leu Leu Gly Pro Leu Ser Val Thr Gly Ala Ser Tyr Met
210 215 220

Ala Ile Thr Gly Ala Val Met Arg Ile Pro Ser Ala Ala Gly Arg His
225 230 235 240

Lys Ala Phe Ser Thr Cys Ala Ser His Leu Thr Val Val Ile Ile Phe
245 250 255

Tyr Ala Ala Ser Ile Phe Ile Tyr Ala Arg Pro Lys Ala Leu Ser Ala
260 265 270

Phe Asp Thr Asn Lys Leu Val Ser Val Leu Tyr Ala Val Ile Val Pro
275 280 285

Leu Leu Asn Pro Ile Ile Tyr Cys Leu Arg Asn Gln Glu Val Lys Lys
290 295 300

Ala Leu Arg Arg Thr Leu His Leu Ala Gln Gly Gln Asp Ala Asn Thr
305 310 315 320

Lys Lys Ser Ser Arg Asp Gly

325

<210> 15
<211> 1325
<212> DNA
<213> Mus musculus

<300>
<308> AF121972
<309> 1999-04-25
<313> (1)..(1325)

<220>
<221> CDS
<222> (138)..(1112)

<400> 15
```
aacacactca aatcaaaata atattggatt ggttccatct ggtttcagaa tactcttgtg      60

tttccttgta gaacttaagt ttgacactca taaaaacctt cagacatatt gaaagtaagg     120

gaattgggat taaactc atg tct ctt ttt ccc caa aga aat tta gat gcc        170
                   Met Ser Leu Phe Pro Gln Arg Asn Leu Asp Ala
                   1               5                   10

atg aac aga tca gca gca cat gta acc gaa ttt gtt ctc ttg gga ttt       218
Met Asn Arg Ser Ala Ala His Val Thr Glu Phe Val Leu Leu Gly Phe
            15                  20                  25

cct ggt tcc tgg aag ata cag att ttc ctc ttc gtg ttg ttt ttg gtg       266
Pro Gly Ser Trp Lys Ile Gln Ile Phe Leu Phe Val Leu Phe Leu Val
        30                  35                  40

ttt tat gtc ttg aca ttg ttg gga aat gga gcc atc atc tgt gca gta       314
Phe Tyr Val Leu Thr Leu Leu Gly Asn Gly Ala Ile Ile Cys Ala Val
    45                  50                  55

aga tgt gac tca cgt cta cat acc ccc atg tac ttc ctc ctg gga aat       362
Arg Cys Asp Ser Arg Leu His Thr Pro Met Tyr Phe Leu Leu Gly Asn
60                  65                  70                  75

ttt tcc ttc ctt gaa atc tgg tat gtt tcc tcc act att cct aac ata       410
Phe Ser Phe Leu Glu Ile Trp Tyr Val Ser Ser Thr Ile Pro Asn Ile
                80                  85                  90

cta gcc aac att ctg tct aag acc aag gcc atc tca ttt tca ggg tgc       458
Leu Ala Asn Ile Leu Ser Lys Thr Lys Ala Ile Ser Phe Ser Gly Cys
            95                  100                 105

ttc ctg cag ttc tat ttc ttc ttt tca ctg ggt aca act gaa tgt ctc       506
Phe Leu Gln Phe Tyr Phe Phe Phe Ser Leu Gly Thr Thr Glu Cys Leu
        110                 115                 120

ttc ctg gca gta atg gct tat gat agg tac ctg gcc att tgc cgc cca       554
Phe Leu Ala Val Met Ala Tyr Asp Arg Tyr Leu Ala Ile Cys Arg Pro
    125                 130                 135

tta cat tac cct act atc atg act agg agg ctg tgt tgc att ctg gta       602
Leu His Tyr Pro Thr Ile Met Thr Arg Arg Leu Cys Cys Ile Leu Val
140                 145                 150                 155

tcc tca tgc tgg ctc att gga ttt ctt ggg tac cca atc cct atc ttc       650
Ser Ser Cys Trp Leu Ile Gly Phe Leu Gly Tyr Pro Ile Pro Ile Phe
                160                 165                 170

tcc att tcc cag ctt ccc ttc tgt ggt tct aat atc att gat cac ttc       698
```

```
Ser Ile Ser Gln Leu Pro Phe Cys Gly Ser Asn Ile Ile Asp His Phe
        175                 180                 185

ctc tgt gac atg gac cca ttg atg gct ttg tcc tgt gcc cca gct cct      746
Leu Cys Asp Met Asp Pro Leu Met Ala Leu Ser Cys Ala Pro Ala Pro
    190                 195                 200

att act gaa ttt att ttt tat gcc caa agt tcc ttt gtc ctc ttt ttc      794
Ile Thr Glu Phe Ile Phe Tyr Ala Gln Ser Ser Phe Val Leu Phe Phe
    205                 210                 215

act att gca tac att ctt cgg tcc tat att ttg ttg ctc agg gct gtt      842
Thr Ile Ala Tyr Ile Leu Arg Ser Tyr Ile Leu Leu Leu Arg Ala Val
220                 225                 230                 235

ttt cag gtt cct tct gca gct ggc cga cga aaa gcc ttc tct acc tgt      890
Phe Gln Val Pro Ser Ala Ala Gly Arg Arg Lys Ala Phe Ser Thr Cys
                240                 245                 250

ggt tcc cat tta gtt gtg gtg tca ctc ttc tat ggt aca gta atg gta      938
Gly Ser His Leu Val Val Val Ser Leu Phe Tyr Gly Thr Val Met Val
            255                 260                 265

atg tat gtg agt cct aca tat ggc att cca att ttg atg cag aag atc      986
Met Tyr Val Ser Pro Thr Tyr Gly Ile Pro Ile Leu Met Gln Lys Ile
        270                 275                 280

ctt aca ctt gta tac tct gta atg act cct ctc ttt aat cct ctg att     1034
Leu Thr Leu Val Tyr Ser Val Met Thr Pro Leu Phe Asn Pro Leu Ile
    285                 290                 295

tat agc ctt cgt aac aag gac atg aaa ctt gct ctg aga aat gtt ttg     1082
Tyr Ser Leu Arg Asn Lys Asp Met Lys Leu Ala Leu Arg Asn Val Leu
300                 305                 310                 315

tta gga atg aga att gtc aaa aat atg taa ttcaaagctg tttcatactc       1132
Leu Gly Met Arg Ile Val Lys Asn Met
                320

acatgttcta ataaagaaaa aactggagat gaatcaattc attcagttgt ctttaccctt   1192

tgttctatgt ttttgagaca ctgtctcatg tggccctggc tagcctcaaa ctcattctct   1252

agccaaggat gaccttgcaa agatcactta tgtatactct catatcatct gccaatagtg   1312

ataccttgac ctc                                                      1325


<210>  16
<211>  324
<212>  PRT
<213>  Mus musculus

<400>  16

Met Ser Leu Phe Pro Gln Arg Asn Leu Asp Ala Met Asn Arg Ser Ala
1               5                   10                  15

Ala His Val Thr Glu Phe Val Leu Leu Gly Phe Pro Gly Ser Trp Lys
            20                  25                  30

Ile Gln Ile Phe Leu Phe Val Leu Phe Leu Val Phe Tyr Val Leu Thr
        35                  40                  45

Leu Leu Gly Asn Gly Ala Ile Ile Cys Ala Val Arg Cys Asp Ser Arg
```

50 55 60

Leu His Thr Pro Met Tyr Phe Leu Leu Gly Asn Phe Ser Phe Leu Glu
65 70 75 80

Ile Trp Tyr Val Ser Ser Thr Ile Pro Asn Ile Leu Ala Asn Ile Leu
85 90 95

Ser Lys Thr Lys Ala Ile Ser Phe Ser Gly Cys Phe Leu Gln Phe Tyr
100 105 110

Phe Phe Phe Ser Leu Gly Thr Thr Glu Cys Leu Phe Leu Ala Val Met
115 120 125

Ala Tyr Asp Arg Tyr Leu Ala Ile Cys Arg Pro Leu His Tyr Pro Thr
130 135 140

Ile Met Thr Arg Arg Leu Cys Cys Ile Leu Val Ser Ser Cys Trp Leu
145 150 155 160

Ile Gly Phe Leu Gly Tyr Pro Ile Pro Ile Phe Ser Ile Ser Gln Leu
165 170 175

Pro Phe Cys Gly Ser Asn Ile Ile Asp His Phe Leu Cys Asp Met Asp
180 185 190

Pro Leu Met Ala Leu Ser Cys Ala Pro Ala Pro Ile Thr Glu Phe Ile
195 200 205

Phe Tyr Ala Gln Ser Ser Phe Val Leu Phe Phe Thr Ile Ala Tyr Ile
210 215 220

Leu Arg Ser Tyr Ile Leu Leu Leu Arg Ala Val Phe Gln Val Pro Ser
225 230 235 240

Ala Ala Gly Arg Arg Lys Ala Phe Ser Thr Cys Gly Ser His Leu Val
245 250 255

Val Val Ser Leu Phe Tyr Gly Thr Val Met Val Met Tyr Val Ser Pro
260 265 270

Thr Tyr Gly Ile Pro Ile Leu Met Gln Lys Ile Leu Thr Leu Val Tyr
275 280 285

Ser Val Met Thr Pro Leu Phe Asn Pro Leu Ile Tyr Ser Leu Arg Asn
290 295 300

Lys Asp Met Lys Leu Ala Leu Arg Asn Val Leu Leu Gly Met Arg Ile
305 310 315 320

Val Lys Asn Met

<210> 17
<211> 1134
<212> DNA
<213> Mus musculus

<300>
<308> AF121980
<309> 1999-04-25
<313> (1)..(1134)

<220>
<221> misc_feature
<222> (99)..(99)
<223> n is a, c, g, or t

<220>
<221> CDS
<222> (106)..(1056)

<400> 17
ccagtccagc ctggtaggct gggcaggtcc tacaggtctt tcagggactg aacccggcat 60

cctgcccctc ccctctccct ggagcctccc tagccctcng gcgtc atg ttg ggt tgg 117
Met Leu Gly Trp
1

agc aat ggc acc tac aat gag tcc tac acc agc ttc ctc ctc atg ggc 165
Ser Asn Gly Thr Tyr Asn Glu Ser Tyr Thr Ser Phe Leu Leu Met Gly
5 10 15 20

ttc cca ggg atg cag gaa gcc aga gcc ctc ctg gtg ctg ccc ttc ctc 213
Phe Pro Gly Met Gln Glu Ala Arg Ala Leu Leu Val Leu Pro Phe Leu
25 30 35

agc ctc tac ctg gtg atc ctc ttc acc aat gcc ctg gtc atc cac acg 261
Ser Leu Tyr Leu Val Ile Leu Phe Thr Asn Ala Leu Val Ile His Thr
40 45 50

gtg gca tcc cag cgc agc ctg cac cag ccc atg tac ctg ctc att gcc 309
Val Ala Ser Gln Arg Ser Leu His Gln Pro Met Tyr Leu Leu Ile Ala
55 60 65

ctg ctc ctg gct gtc aat atc tgt gct gcc acc acg gtg ctg ccc ccc 357
Leu Leu Leu Ala Val Asn Ile Cys Ala Ala Thr Thr Val Leu Pro Pro
70 75 80

atg ctc ttc agc ttc tcc aca cgc ttc aac cgc atc tcc ctc cct cga 405
Met Leu Phe Ser Phe Ser Thr Arg Phe Asn Arg Ile Ser Leu Pro Arg
85 90 95 100

tgc ttg gga cag atg ttc tgc atc tac ttt ctg gtt tct atg gac tgc 453
Cys Leu Gly Gln Met Phe Cys Ile Tyr Phe Leu Val Ser Met Asp Cys
105 110 115

aac atc ctc ctg gtc atg gct cta gat cgc tat gtg gct atc tgc tac 501
Asn Ile Leu Leu Val Met Ala Leu Asp Arg Tyr Val Ala Ile Cys Tyr
120 125 130

cct ctc cgc tac cca gaa ata gtg aca gga cag tta ctg gct ggt ctg 549
Pro Leu Arg Tyr Pro Glu Ile Val Thr Gly Gln Leu Leu Ala Gly Leu
135 140 145

gtg gtg ttg gca gtc acc agg agc aca agc att gtt gct cca gtg gtg 597
Val Val Leu Ala Val Thr Arg Ser Thr Ser Ile Val Ala Pro Val Val

150 155 160

gtg ctg gcc tcg cgg gtt cgc ttc tgc cgc tca gat gtg atc cgc cac 645
Val Leu Ala Ser Arg Val Arg Phe Cys Arg Ser Asp Val Ile Arg His
165 170 175 180

ttt gcc tgt gag cac atg gcc ctg atg aag ctc tcc tgt gga gac atc 693
Phe Ala Cys Glu His Met Ala Leu Met Lys Leu Ser Cys Gly Asp Ile
185 190 195

tcg ctg aat aaa acg gcg gga ctc att att cga acc ttt aat aga gtc 741
Ser Leu Asn Lys Thr Ala Gly Leu Ile Ile Arg Thr Phe Asn Arg Val
200 205 210

ctg gat atg ctc ctt cta ggc acc tcc tac tcc cgc atc atc cat gct 789
Leu Asp Met Leu Leu Leu Gly Thr Ser Tyr Ser Arg Ile Ile His Ala
215 220 225

gcc ttc agg atc tca tca ggt gga gca cgg tcc aaa gcc ctg aac acc 837
Ala Phe Arg Ile Ser Ser Gly Gly Ala Arg Ser Lys Ala Leu Asn Thr
230 235 240

tgt ggt tcc cac ctg ctg gtc atc ttc acc gtc tac tcc tcc acc atg 885
Cys Gly Ser His Leu Leu Val Ile Phe Thr Val Tyr Ser Ser Thr Met
245 250 255 260

tcc tca tcc att gtc tac cgt gtg gct cgc act gcc tcc caa gat gtg 933
Ser Ser Ser Ile Val Tyr Arg Val Ala Arg Thr Ala Ser Gln Asp Val
265 270 275

cac aac ctg ctc agt gct ttc tat ctg ttg ctc ccg tgt ctg gtc aac 981
His Asn Leu Leu Ser Ala Phe Tyr Leu Leu Leu Pro Cys Leu Val Asn
280 285 290

ccc atc atc tac ggg gcc aga acc aag gaa atc agg cag cac ctg gta 1029
Pro Ile Ile Tyr Gly Ala Arg Thr Lys Glu Ile Arg Gln His Leu Val
295 300 305

agg tca ttc ctg agt gca ggc ccc tga ctctcctatg atcagtccgt 1076
Arg Ser Phe Leu Ser Ala Gly Pro
310 315

gttggcccct cagtattcct ggtgaaactg aggaaggaag aaatggagtc agagggac 1134

<210> 18
<211> 316
<212> PRT
<213> Mus musculus

<400> 18

Met Leu Gly Trp Ser Asn Gly Thr Tyr Asn Glu Ser Tyr Thr Ser Phe
1 5 10 15

Leu Leu Met Gly Phe Pro Gly Met Gln Glu Ala Arg Ala Leu Leu Val
20 25 30

Leu Pro Phe Leu Ser Leu Tyr Leu Val Ile Leu Phe Thr Asn Ala Leu
35 40 45

Val Ile His Thr Val Ala Ser Gln Arg Ser Leu His Gln Pro Met Tyr
50 55 60

Leu Leu Ile Ala Leu Leu Leu Ala Val Asn Ile Cys Ala Ala Thr Thr
65 70 75 80

Val Leu Pro Pro Met Leu Phe Ser Phe Ser Thr Arg Phe Asn Arg Ile
85 90 95

Ser Leu Pro Arg Cys Leu Gly Gln Met Phe Cys Ile Tyr Phe Leu Val
100 105 110

Ser Met Asp Cys Asn Ile Leu Leu Val Met Ala Leu Asp Arg Tyr Val
115 120 125

Ala Ile Cys Tyr Pro Leu Arg Tyr Pro Glu Ile Val Thr Gly Gln Leu
130 135 140

Leu Ala Gly Leu Val Val Leu Ala Val Thr Arg Ser Thr Ser Ile Val
145 150 155 160

Ala Pro Val Val Val Leu Ala Ser Arg Val Arg Phe Cys Arg Ser Asp
165 170 175

Val Ile Arg His Phe Ala Cys Glu His Met Ala Leu Met Lys Leu Ser
180 185 190

Cys Gly Asp Ile Ser Leu Asn Lys Thr Ala Gly Leu Ile Ile Arg Thr
195 200 205

Phe Asn Arg Val Leu Asp Met Leu Leu Leu Gly Thr Ser Tyr Ser Arg
210 215 220

Ile Ile His Ala Ala Phe Arg Ile Ser Ser Gly Gly Ala Arg Ser Lys
225 230 235 240

Ala Leu Asn Thr Cys Gly Ser His Leu Leu Val Ile Phe Thr Val Tyr
245 250 255

Ser Ser Thr Met Ser Ser Ser Ile Val Tyr Arg Val Ala Arg Thr Ala
260 265 270

Ser Gln Asp Val His Asn Leu Leu Ser Ala Phe Tyr Leu Leu Leu Pro
275 280 285

Cys Leu Val Asn Pro Ile Ile Tyr Gly Ala Arg Thr Lys Glu Ile Arg
290 295 300

Gln His Leu Val Arg Ser Phe Leu Ser Ala Gly Pro
305 310 315

<210> 19
<211> 1421
<212> DNA
<213> Mus musculus

```
<300>
<308>  AF121976
<309>  1999-12-25
<313>  (1)..(1421)

<220>
<221>  CDS
<222>  (291)..(1310)

<400>  19
agaaagattt caggagtcct taaagacggc acagaaaacc ggtacagact gcaccattca      60

gctgaaagcc agacgtaaca gcaccacggt ggtggtgaac acggtgggct cagagaatcc     120

ggataagcct gctttttat actaagttgg cattataaaa aagcattgct tatcaatttg      180

ttgcaacgaa caggtcacta tcagtcaaaa taaaatcatt atttgatttc aattttgtcc     240

cactccctgc ctctgtcatc acgatactgt gatgccatgg tgtccgactt atg ccc        296
                                                       Met Pro
                                                       1

gag aag atg ttg agc aaa ctt atc gct tat ctg ctt ctc ata gag tct       344
Glu Lys Met Leu Ser Lys Leu Ile Ala Tyr Leu Leu Leu Ile Glu Ser
        5                   10                  15

tgc aga caa act gcg caa ctc gtg aaa ggt agg cgg atc tgg gtc gac       392
Cys Arg Gln Thr Ala Gln Leu Val Lys Gly Arg Arg Ile Trp Val Asp
    20                  25                  30

tct agg cct cac tgg cct aat acg act cac tat agg gag ctc gag gat       440
Ser Arg Pro His Trp Pro Asn Thr Thr His Tyr Arg Glu Leu Glu Asp
35                  40                  45                  50

cag cat gtt tgg att gct att ccc ttc tgc tcc atg tac atc ctt gct       488
Gln His Val Trp Ile Ala Ile Pro Phe Cys Ser Met Tyr Ile Leu Ala
                55                  60                  65

ctg gtt gga aat ggt acc atc ctc tat atc att ata aca gac agg gct       536
Leu Val Gly Asn Gly Thr Ile Leu Tyr Ile Ile Ile Thr Asp Arg Ala
            70                  75                  80

ctc cat gag cca atg tac ctc ttc ttg tgt ctg ctt tct atc act gat       584
Leu His Glu Pro Met Tyr Leu Phe Leu Cys Leu Leu Ser Ile Thr Asp
        85                  90                  95

ctg gtt ctc tgt tca aca aca ttg cct aaa atg ctg gca ata ttc tgg       632
Leu Val Leu Cys Ser Thr Thr Leu Pro Lys Met Leu Ala Ile Phe Trp
    100                 105                 110

ctc aga tcc cat gtc att tcc tac cat ggc tgc ctc act cag atg ttt       680
Leu Arg Ser His Val Ile Ser Tyr His Gly Cys Leu Thr Gln Met Phe
115                 120                 125                 130

ttt gta cat gca gtc ttt gcc aca gag tca gct gtt ctg ctg gcc atg       728
Phe Val His Ala Val Phe Ala Thr Glu Ser Ala Val Leu Leu Ala Met
                135                 140                 145

gct ttt gat cga tat gtt gct atc tgc aga cca ctc cac tat aca tcc       776
Ala Phe Asp Arg Tyr Val Ala Ile Cys Arg Pro Leu His Tyr Thr Ser
            150                 155                 160

atc ctc aat gct gtt gta att ggg aag att ggc ctg gca tgc gtg act       824
Ile Leu Asn Ala Val Val Ile Gly Lys Ile Gly Leu Ala Cys Val Thr
        165                 170                 175
```

```
cgt ggc ctt ctc ttt gtc ttc ccc ttt gtc att ctc att gaa cgt tta        872
Arg Gly Leu Leu Phe Val Phe Pro Phe Val Ile Leu Ile Glu Arg Leu
    180                 185                 190

ccc ttc tgt gga cat cat ata atc cct cac act tac tgt gag cac atg        920
Pro Phe Cys Gly His His Ile Ile Pro His Thr Tyr Cys Glu His Met
195                 200                 205                 210

ggc ata gcc aag ctc gcc tgt gcc agc atc aag cct aac acc atc tat        968
Gly Ile Ala Lys Leu Ala Cys Ala Ser Ile Lys Pro Asn Thr Ile Tyr
                215                 220                 225

ggt ctt act gta gca ctt tca gtc act ggc atg gat gtg gtc ctc att       1016
Gly Leu Thr Val Ala Leu Ser Val Thr Gly Met Asp Val Val Leu Ile
            230                 235                 240

gca acc tcc tac atc ctg att ctg cag gcc gtg ctg cga ctg ccc tca       1064
Ala Thr Ser Tyr Ile Leu Ile Leu Gln Ala Val Leu Arg Leu Pro Ser
        245                 250                 255

aag gat gcc cag ttc cga gca ttc agc aca tgt gga gcc cac att tgt       1112
Lys Asp Ala Gln Phe Arg Ala Phe Ser Thr Cys Gly Ala His Ile Cys
    260                 265                 270

gta att ctt gtc ttc tat atc ccc gca ttc ttt tca ttt ttc act cac       1160
Val Ile Leu Val Phe Tyr Ile Pro Ala Phe Phe Ser Phe Phe Thr His
275                 280                 285                 290

cgc ttt ggt cac cac gtg cct cct cag gta cac atc ata ctt gca aat       1208
Arg Phe Gly His His Val Pro Pro Gln Val His Ile Ile Leu Ala Asn
                295                 300                 305

ctt tat ctc ctt gtg cct cct gtt ctc aac ccc cta gtc tat ggc atc       1256
Leu Tyr Leu Leu Val Pro Pro Val Leu Asn Pro Leu Val Tyr Gly Ile
            310                 315                 320

aat acc aaa caa atc cgc ctg aga ata ctt gac ttt ttt gta aag aga       1304
Asn Thr Lys Gln Ile Arg Leu Arg Ile Leu Asp Phe Phe Val Lys Arg
        325                 330                 335

agg tga caataatctc cacatatacc aaaggctaat gagttcctgg ctttagtttg        1360
Arg

ctgcttctgc tgatctcagt aagtcagtgt atgtacattt aagattttga gatctagagc     1420

a                                                                     1421
```

<210> 20
<211> 339
<212> PRT
<213> Mus musculus

<400> 20

```
Met Pro Glu Lys Met Leu Ser Lys Leu Ile Ala Tyr Leu Leu Leu Ile
1               5                   10                  15

Glu Ser Cys Arg Gln Thr Ala Gln Leu Val Lys Gly Arg Arg Ile Trp
            20                  25                  30

Val Asp Ser Arg Pro His Trp Pro Asn Thr Thr His Tyr Arg Glu Leu
        35                  40                  45
```

Glu Asp Gln His Val Trp Ile Ala Ile Pro Phe Cys Ser Met Tyr Ile
50 55 60

Leu Ala Leu Val Gly Asn Gly Thr Ile Leu Tyr Ile Ile Ile Thr Asp
65 70 75 80

Arg Ala Leu His Glu Pro Met Tyr Leu Phe Leu Cys Leu Leu Ser Ile
85 90 95

Thr Asp Leu Val Leu Cys Ser Thr Thr Leu Pro Lys Met Leu Ala Ile
100 105 110

Phe Trp Leu Arg Ser His Val Ile Ser Tyr His Gly Cys Leu Thr Gln
115 120 125

Met Phe Phe Val His Ala Val Phe Ala Thr Glu Ser Ala Val Leu Leu
130 135 140

Ala Met Ala Phe Asp Arg Tyr Val Ala Ile Cys Arg Pro Leu His Tyr
145 150 155 160

Thr Ser Ile Leu Asn Ala Val Val Ile Gly Lys Ile Gly Leu Ala Cys
165 170 175

Val Thr Arg Gly Leu Leu Phe Val Phe Pro Phe Val Ile Leu Ile Glu
180 185 190

Arg Leu Pro Phe Cys Gly His His Ile Ile Pro His Thr Tyr Cys Glu
195 200 205

His Met Gly Ile Ala Lys Leu Ala Cys Ala Ser Ile Lys Pro Asn Thr
210 215 220

Ile Tyr Gly Leu Thr Val Ala Leu Ser Val Thr Gly Met Asp Val Val
225 230 235 240

Leu Ile Ala Thr Ser Tyr Ile Leu Ile Leu Gln Ala Val Leu Arg Leu
245 250 255

Pro Ser Lys Asp Ala Gln Phe Arg Ala Phe Ser Thr Cys Gly Ala His
260 265 270

Ile Cys Val Ile Leu Val Phe Tyr Ile Pro Ala Phe Phe Ser Phe Phe
275 280 285

Thr His Arg Phe Gly His His Val Pro Pro Gln Val His Ile Ile Leu
290 295 300

Ala Asn Leu Tyr Leu Leu Val Pro Pro Val Leu Asn Pro Leu Val Tyr
305 310 315 320

```
Gly Ile Asn Thr Lys Gln Ile Arg Leu Arg Ile Leu Asp Phe Phe Val
                325                 330                 335

Lys Arg Arg


<210>  21
<211>  930
<212>  DNA
<213>  M.musculus

<300>
<308>  X92969
<309>  1996-07-01
<313>  (1)..(930)


<220>
<221>  CDS
<222>  (1)..(930)

<400>  21
atg cag aga aat aac ttc act gaa gtg ata gag ttc gtc ttc ctg gga      48
Met Gln Arg Asn Asn Phe Thr Glu Val Ile Glu Phe Val Phe Leu Gly
1               5                   10                  15

ttc tcc agc ttt gga aag cat cag ata acc ctc ttt gtg gtt ttc cta      96
Phe Ser Ser Phe Gly Lys His Gln Ile Thr Leu Phe Val Val Phe Leu
            20                  25                  30

acc atc tac att tta act ctg gct ggc aac atc att ata gtg aca atc     144
Thr Ile Tyr Ile Leu Thr Leu Ala Gly Asn Ile Ile Ile Val Thr Ile
        35                  40                  45

aca cac ata gac cac cac ctt cac act ccc atg tac ttc ttt ctg agc     192
Thr His Ile Asp His His Leu His Thr Pro Met Tyr Phe Phe Leu Ser
    50                  55                  60

atg ttg gca agc tca gag act gtg tac aca ctg gtc att gtc cca cga     240
Met Leu Ala Ser Ser Glu Thr Val Tyr Thr Leu Val Ile Val Pro Arg
65                  70                  75                  80

atg ctt tcc agc ctg att ttt tac aac ctt ccc ata tcc ttg gca ggc     288
Met Leu Ser Ser Leu Ile Phe Tyr Asn Leu Pro Ile Ser Leu Ala Gly
                85                  90                  95

tgc gca acc caa atg ttc ttt ttt gtc act ttg gcc acc aac aac tgc     336
Cys Ala Thr Gln Met Phe Phe Phe Val Thr Leu Ala Thr Asn Asn Cys
            100                 105                 110

ttt ctg ctc aca gca atg ggt tat gat cgt tat gtg gct att tgt aat     384
Phe Leu Leu Thr Ala Met Gly Tyr Asp Arg Tyr Val Ala Ile Cys Asn
        115                 120                 125

cct ctg aga tat aca atc atc atg agc aag gga atg tgt gcc ttg ttg     432
Pro Leu Arg Tyr Thr Ile Ile Met Ser Lys Gly Met Cys Ala Leu Leu
    130                 135                 140

gtc tgt ggg tct tta ggc act ggc ctg gtt atg gca gtt ctt cat gtg     480
Val Cys Gly Ser Leu Gly Thr Gly Leu Val Met Ala Val Leu His Val
145                 150                 155                 160

cca gcc atg ttc cat ttg ccc ttt tgt ggc acg gtg gtg gag cac ttt     528
Pro Ala Met Phe His Leu Pro Phe Cys Gly Thr Val Val Glu His Phe
                165                 170                 175
```

```
ttc tgt gac ata tac cca gta atg aag ctt tct tgt gtt gat acc act      576
Phe Cys Asp Ile Tyr Pro Val Met Lys Leu Ser Cys Val Asp Thr Thr
            180                 185                 190

gtc aat gag ata atc aat tat ggt gta agt tca ttt gta att ctt gtg      624
Val Asn Glu Ile Ile Asn Tyr Gly Val Ser Ser Phe Val Ile Leu Val
        195                 200                 205

ccc ata ggg ctg ata ttt atc tcc tat gtg ctc att gtc tct tcc atc      672
Pro Ile Gly Leu Ile Phe Ile Ser Tyr Val Leu Ile Val Ser Ser Ile
    210                 215                 220

ctt aaa att gtg tcc act gaa ggc cag aag aaa gcc ttt gcc acc tgt      720
Leu Lys Ile Val Ser Thr Glu Gly Gln Lys Lys Ala Phe Ala Thr Cys
225                 230                 235                 240

gcc tct cat ctc act gtg gtc att gtc cac tat ggc tgt gcc tcc att      768
Ala Ser His Leu Thr Val Val Ile Val His Tyr Gly Cys Ala Ser Ile
                245                 250                 255

gcc tac ctc aaa ccc aaa tca gaa agt tca gta gaa aaa gac ctt ctt      816
Ala Tyr Leu Lys Pro Lys Ser Glu Ser Ser Val Glu Lys Asp Leu Leu
            260                 265                 270

ctc tct gtg acc tac act atc atc act ccc ttg ctg aac cct gtt gtc      864
Leu Ser Val Thr Tyr Thr Ile Ile Thr Pro Leu Leu Asn Pro Val Val
        275                 280                 285

tac agc ctc agg aac aaa gaa gtc aaa gat gct cta tgc aga gct gtg      912
Tyr Ser Leu Arg Asn Lys Glu Val Lys Asp Ala Leu Cys Arg Ala Val
    290                 295                 300

ggc aga aac act tct taa                                              930
Gly Arg Asn Thr Ser
305


<210>  22
<211>  309
<212>  PRT
<213>  M.musculus

<400>  22

Met Gln Arg Asn Asn Phe Thr Glu Val Ile Glu Phe Val Phe Leu Gly
1               5                   10                  15

Phe Ser Ser Phe Gly Lys His Gln Ile Thr Leu Phe Val Val Phe Leu
            20                  25                  30

Thr Ile Tyr Ile Leu Thr Leu Ala Gly Asn Ile Ile Ile Val Thr Ile
        35                  40                  45

Thr His Ile Asp His His Leu His Thr Pro Met Tyr Phe Phe Leu Ser
    50                  55                  60

Met Leu Ala Ser Ser Glu Thr Val Tyr Thr Leu Val Ile Val Pro Arg
65                  70                  75                  80

Met Leu Ser Ser Leu Ile Phe Tyr Asn Leu Pro Ile Ser Leu Ala Gly
                85                  90                  95
```

Cys Ala Thr Gln Met Phe Phe Phe Val Thr Leu Ala Thr Asn Asn Cys
100 105 110

Phe Leu Leu Thr Ala Met Gly Tyr Asp Arg Tyr Val Ala Ile Cys Asn
115 120 125

Pro Leu Arg Tyr Thr Ile Ile Met Ser Lys Gly Met Cys Ala Leu Leu
130 135 140

Val Cys Gly Ser Leu Gly Thr Gly Leu Val Met Ala Val Leu His Val
145 150 155 160

Pro Ala Met Phe His Leu Pro Phe Cys Gly Thr Val Val Glu His Phe
165 170 175

Phe Cys Asp Ile Tyr Pro Val Met Lys Leu Ser Cys Val Asp Thr Thr
180 185 190

Val Asn Glu Ile Ile Asn Tyr Gly Val Ser Ser Phe Val Ile Leu Val
195 200 205

Pro Ile Gly Leu Ile Phe Ile Ser Tyr Val Leu Ile Val Ser Ser Ile
210 215 220

Leu Lys Ile Val Ser Thr Glu Gly Gln Lys Lys Ala Phe Ala Thr Cys
225 230 235 240

Ala Ser His Leu Thr Val Val Ile Val His Tyr Gly Cys Ala Ser Ile
245 250 255

Ala Tyr Leu Lys Pro Lys Ser Glu Ser Ser Val Glu Lys Asp Leu Leu
260 265 270

Leu Ser Val Thr Tyr Thr Ile Ile Thr Pro Leu Leu Asn Pro Val Val
275 280 285

Tyr Ser Leu Arg Asn Lys Glu Val Lys Asp Ala Leu Cys Arg Ala Val
290 295 300

Gly Arg Asn Thr Ser
305

<210> 23
<211> 957
<212> DNA
<213> Mus musculus

<300>
<308> AB061229
<309> 2001-09-07
<313> (1)..(957)

<220>

<221> CDS
<222> (1)..(957)

<400> 23

```
atg ata ctg tct gaa aaa aac aat agt ggg att att ttc acc ctc ttg      48
Met Ile Leu Ser Glu Lys Asn Asn Ser Gly Ile Ile Phe Thr Leu Leu
1               5                   10                  15

ggc ttc tca gat tat cct gac ctt aaa gtc cct ctc ttc ttg gtg ttt      96
Gly Phe Ser Asp Tyr Pro Asp Leu Lys Val Pro Leu Phe Leu Val Phe
            20                  25                  30

ctc gtc att tac agc atc act gtg gta gga aat att ggt atg atc ctc     144
Leu Val Ile Tyr Ser Ile Thr Val Val Gly Asn Ile Gly Met Ile Leu
        35                  40                  45

gtg atc aga att aat ccc caa ctg cac tcc cct atg tac ttc ttc ctc     192
Val Ile Arg Ile Asn Pro Gln Leu His Ser Pro Met Tyr Phe Phe Leu
    50                  55                  60

agc cac ctc tcc ttt gtg gat ttc tgc tat tct tcg atc att gct ccc     240
Ser His Leu Ser Phe Val Asp Phe Cys Tyr Ser Ser Ile Ile Ala Pro
65                  70                  75                  80

aag atg ctg gtg aac ctt gtt gca aaa gac ata acc att tca ttt gta     288
Lys Met Leu Val Asn Leu Val Ala Lys Asp Ile Thr Ile Ser Phe Val
                85                  90                  95

gaa tgc ata gta caa tat ttt tta ttt tgt gtc ttt gta gta act gaa     336
Glu Cys Ile Val Gln Tyr Phe Leu Phe Cys Val Phe Val Val Thr Glu
            100                 105                 110

gcc ttt tta tta gtg gtt atg gca tat gac cga ttt gtg gct atc tgt     384
Ala Phe Leu Leu Val Val Met Ala Tyr Asp Arg Phe Val Ala Ile Cys
        115                 120                 125

aac cct ctg ctc tac aca gta gcc atg tcc cag aaa ctc tgt atc aca     432
Asn Pro Leu Leu Tyr Thr Val Ala Met Ser Gln Lys Leu Cys Ile Thr
    130                 135                 140

ctg gtg gtg gga tcc tac gca tgg ggg ttc aca tgt tcc ttg aca ctg     480
Leu Val Val Gly Ser Tyr Ala Trp Gly Phe Thr Cys Ser Leu Thr Leu
145                 150                 155                 160

acg tgt tct act gtg caa tta tct ttt cat ggt gtc aat agg atc gat     528
Thr Cys Ser Thr Val Gln Leu Ser Phe His Gly Val Asn Arg Ile Asp
                165                 170                 175

cac ttc ttc tgt gaa ctc tct tca ctg cta gcc ctt tct tcc tct gat     576
His Phe Phe Cys Glu Leu Ser Ser Leu Leu Ala Leu Ser Ser Ser Asp
            180                 185                 190

act ctc atc agt caa tta ctg ctg ttt gtc ttt gcc aca ttt aat gct     624
Thr Leu Ile Ser Gln Leu Leu Leu Phe Val Phe Ala Thr Phe Asn Ala
        195                 200                 205

gtc agc aca tta ctc ctt att ctg ttg tct tac ctg ttc att gtt gtc     672
Val Ser Thr Leu Leu Leu Ile Leu Leu Ser Tyr Leu Phe Ile Val Val
    210                 215                 220

act gtt ctt aag atg cgt tca gcc agt ggg cgt cgt aag gct ttc tcc     720
Thr Val Leu Lys Met Arg Ser Ala Ser Gly Arg Arg Lys Ala Phe Ser
225                 230                 235                 240

acc tgt gca tcc cat ctg gca gcc atc act atc ttc cat ggt acc att     768
Thr Cys Ala Ser His Leu Ala Ala Ile Thr Ile Phe His Gly Thr Ile
                245                 250                 255
```

```
tta ttc ctt ttt tgt gtt ccc aac tct aag aat tcc agg ctc aca gtc      816
Leu Phe Leu Phe Cys Val Pro Asn Ser Lys Asn Ser Arg Leu Thr Val
            260                 265                 270

aaa gtg ggc tct gtg ttt tac aca gtg gtg atc ccc atg ctt aac ccc      864
Lys Val Gly Ser Val Phe Tyr Thr Val Val Ile Pro Met Leu Asn Pro
        275                 280                 285

ata atc tat agt ctg aga aat aag gat gtc caa gat act att aga aaa      912
Ile Ile Tyr Ser Leu Arg Asn Lys Asp Val Gln Asp Thr Ile Arg Lys
    290                 295                 300

ata atg acc ctt atc tca tgt gtt aag aat gat aga cac aat taa          957
Ile Met Thr Leu Ile Ser Cys Val Lys Asn Asp Arg His Asn
305                 310                 315


<210>  24
<211>  318
<212>  PRT
<213>  Mus musculus

<400>  24

Met Ile Leu Ser Glu Lys Asn Asn Ser Gly Ile Ile Phe Thr Leu Leu
1               5                   10                  15


Gly Phe Ser Asp Tyr Pro Asp Leu Lys Val Pro Leu Phe Leu Val Phe
            20                  25                  30


Leu Val Ile Tyr Ser Ile Thr Val Val Gly Asn Ile Gly Met Ile Leu
        35                  40                  45


Val Ile Arg Ile Asn Pro Gln Leu His Ser Pro Met Tyr Phe Phe Leu
    50                  55                  60


Ser His Leu Ser Phe Val Asp Phe Cys Tyr Ser Ser Ile Ile Ala Pro
65                  70                  75                  80


Lys Met Leu Val Asn Leu Val Ala Lys Asp Ile Thr Ile Ser Phe Val
                85                  90                  95


Glu Cys Ile Val Gln Tyr Phe Leu Phe Cys Val Phe Val Val Thr Glu
            100                 105                 110


Ala Phe Leu Leu Val Val Met Ala Tyr Asp Arg Phe Val Ala Ile Cys
        115                 120                 125


Asn Pro Leu Leu Tyr Thr Val Ala Met Ser Gln Lys Leu Cys Ile Thr
    130                 135                 140


Leu Val Val Gly Ser Tyr Ala Trp Gly Phe Thr Cys Ser Leu Thr Leu
145                 150                 155                 160


Thr Cys Ser Thr Val Gln Leu Ser Phe His Gly Val Asn Arg Ile Asp
                165                 170                 175
```

His Phe Phe Cys Glu Leu Ser Ser Leu Leu Ala Leu Ser Ser Ser Asp
180 185 190

Thr Leu Ile Ser Gln Leu Leu Leu Phe Val Phe Ala Thr Phe Asn Ala
195 200 205

Val Ser Thr Leu Leu Leu Ile Leu Leu Ser Tyr Leu Phe Ile Val Val
210 215 220

Thr Val Leu Lys Met Arg Ser Ala Ser Gly Arg Arg Lys Ala Phe Ser
225 230 235 240

Thr Cys Ala Ser His Leu Ala Ala Ile Thr Ile Phe His Gly Thr Ile
245 250 255

Leu Phe Leu Phe Cys Val Pro Asn Ser Lys Asn Ser Arg Leu Thr Val
260 265 270

Lys Val Gly Ser Val Phe Tyr Thr Val Val Ile Pro Met Leu Asn Pro
275 280 285

Ile Ile Tyr Ser Leu Arg Asn Lys Asp Val Gln Asp Thr Ile Arg Lys
290 295 300

Ile Met Thr Leu Ile Ser Cys Val Lys Asn Asp Arg His Asn
305 310 315

<210> 25
<211> 1344
<212> DNA
<213> Mus musculus

<300>
<308> AJ133424
<309> 2003-02-01
<313> (1)..(1344)

<220>
<221> CDS
<222> (61)..(1020)

<400> 25
ggaggaagac aatgttgatg ctgattgctg agttcctgca ggtttcaaac cgaatgtacc 60

atg gac aga tcc aat gag acc gcc ccc ctg tcc ggc ttc att ctc ctg 108
Met Asp Arg Ser Asn Glu Thr Ala Pro Leu Ser Gly Phe Ile Leu Leu
1 5 10 15

ggc ctc tct gcc cac cca aag ctg gag aaa acc ttc ttc gtg ctc atc 156
Gly Leu Ser Ala His Pro Lys Leu Glu Lys Thr Phe Phe Val Leu Ile
20 25 30

ctg atg atg tac ctg gtg atc ctg ctg ggc aac ggc gtc ctc atc ctg 204
Leu Met Met Tyr Leu Val Ile Leu Leu Gly Asn Gly Val Leu Ile Leu
35 40 45

gtg agc atc ctc gac tcc cac ctg cac acg ccc atg tac ttc ttc ctg 252
Val Ser Ile Leu Asp Ser His Leu His Thr Pro Met Tyr Phe Phe Leu

```
                  50                          55                          60

ggg aac ctc tcc ttc ctg gac atc tgc tac act acc tcc tct gtc ccc        300
Gly Asn Leu Ser Phe Leu Asp Ile Cys Tyr Thr Thr Ser Ser Val Pro
65                  70                  75                  80

ctc att ctg gac agc ttt ctg act ccc agg aag acc atc tcc ttc tcg        348
Leu Ile Leu Asp Ser Phe Leu Thr Pro Arg Lys Thr Ile Ser Phe Ser
                85                  90                  95

ggc tgt gcc gtg cag atg ttt ctc tcc ttc gcc atg gga gcc acg gag        396
Gly Cys Ala Val Gln Met Phe Leu Ser Phe Ala Met Gly Ala Thr Glu
            100                 105                 110

tgt gtg ctc ctg agt atg atg gcg ttt gat cgt tat gtg gcc atc tgc        444
Cys Val Leu Leu Ser Met Met Ala Phe Asp Arg Tyr Val Ala Ile Cys
        115                 120                 125

aac ccc ctt aga tat cct gtg gtc atg aac aag gct gcc tat gtg ccc        492
Asn Pro Leu Arg Tyr Pro Val Val Met Asn Lys Ala Ala Tyr Val Pro
    130                 135                 140

atg gct gcc agt tcc tgg gca ggt ggt atc act aat tct gta gtg cag        540
Met Ala Ala Ser Ser Trp Ala Gly Gly Ile Thr Asn Ser Val Val Gln
145                 150                 155                 160

aca tct ttg gca atg cgg ctg ccc ttc tgt ggg gac aat gtc atc aat        588
Thr Ser Leu Ala Met Arg Leu Pro Phe Cys Gly Asp Asn Val Ile Asn
                165                 170                 175

cac ttc acc tgt gag atc ctg gca gtc ctg aaa ctg gcc tgt gct gac        636
His Phe Thr Cys Glu Ile Leu Ala Val Leu Lys Leu Ala Cys Ala Asp
            180                 185                 190

atc tcc atc aat gtc atc agc atg gtt gtg gcc aac atg atc ttc ttg        684
Ile Ser Ile Asn Val Ile Ser Met Val Val Ala Asn Met Ile Phe Leu
        195                 200                 205

gca gtc cca gtc ctc ttc atc ttt gtc tcc tat gtc ttc atc ctt gtg        732
Ala Val Pro Val Leu Phe Ile Phe Val Ser Tyr Val Phe Ile Leu Val
    210                 215                 220

aca atc ctg agg atc ccc tct gct gag ggg agg aag aag gcc ttc tcc        780
Thr Ile Leu Arg Ile Pro Ser Ala Glu Gly Arg Lys Lys Ala Phe Ser
225                 230                 235                 240

acc tgc tct gcc cac ctc acc gtg gta ctt gtc ttc tat gga acc atc        828
Thr Cys Ser Ala His Leu Thr Val Val Leu Val Phe Tyr Gly Thr Ile
                245                 250                 255

ctc ttc atg tac ggg aag ccc aag tcc aag gac cca ctg ggg gca gac        876
Leu Phe Met Tyr Gly Lys Pro Lys Ser Lys Asp Pro Leu Gly Ala Asp
            260                 265                 270

aag cag gac ctt gca gac aag ctc atc tcc ctc ttc tat gga gtg gtg        924
Lys Gln Asp Leu Ala Asp Lys Leu Ile Ser Leu Phe Tyr Gly Val Val
        275                 280                 285

acc ccc atg cta aac ccc atc atc tac agc ttg aga aac aag gac gtg        972
Thr Pro Met Leu Asn Pro Ile Ile Tyr Ser Leu Arg Asn Lys Asp Val
    290                 295                 300

agg gct gct gtg agg aac ctg gtg ggc cag aaa cac cta act gag tga       1020
Arg Ala Ala Val Arg Asn Leu Val Gly Gln Lys His Leu Thr Glu
305                 310                 315

ctgtcacagt gcagaacttc caacctcttc attgtgtttg tgagggaaga gtggtgcaat     1080
```

gaagaggagc cacttcccca aggtccaagt aatgaactca gaactaagac tataaacaaa 1140
ctatcaacgt tccttaagca ccaatgcttc tagttaacag gctggaagga caagccttta 1200
cacctttgga gagaatggct ggttgtcagc tttgtgttca accttagtgg cgtcgtagaa 1260
ctactctttc atgaccagag gctggcacag atctctggaa agatgctgac atgcataact 1320
aggagacaga tgcaaagcct ggtt 1344

<210> 26
<211> 319
<212> PRT
<213> Mus musculus

<400> 26

Met Asp Arg Ser Asn Glu Thr Ala Pro Leu Ser Gly Phe Ile Leu Leu
1 5 10 15

Gly Leu Ser Ala His Pro Lys Leu Glu Lys Thr Phe Phe Val Leu Ile
20 25 30

Leu Met Met Tyr Leu Val Ile Leu Leu Gly Asn Gly Val Leu Ile Leu
35 40 45

Val Ser Ile Leu Asp Ser His Leu His Thr Pro Met Tyr Phe Phe Leu
50 55 60

Gly Asn Leu Ser Phe Leu Asp Ile Cys Tyr Thr Thr Ser Ser Val Pro
65 70 75 80

Leu Ile Leu Asp Ser Phe Leu Thr Pro Arg Lys Thr Ile Ser Phe Ser
85 90 95

Gly Cys Ala Val Gln Met Phe Leu Ser Phe Ala Met Gly Ala Thr Glu
100 105 110

Cys Val Leu Leu Ser Met Met Ala Phe Asp Arg Tyr Val Ala Ile Cys
115 120 125

Asn Pro Leu Arg Tyr Pro Val Val Met Asn Lys Ala Ala Tyr Val Pro
130 135 140

Met Ala Ala Ser Ser Trp Ala Gly Gly Ile Thr Asn Ser Val Val Gln
145 150 155 160

Thr Ser Leu Ala Met Arg Leu Pro Phe Cys Gly Asp Asn Val Ile Asn
165 170 175

His Phe Thr Cys Glu Ile Leu Ala Val Leu Lys Leu Ala Cys Ala Asp
180 185 190

Ile Ser Ile Asn Val Ile Ser Met Val Val Ala Asn Met Ile Phe Leu
195 200 205

Ala Val Pro Val Leu Phe Ile Phe Val Ser Tyr Val Phe Ile Leu Val
210 215 220

Thr Ile Leu Arg Ile Pro Ser Ala Glu Gly Arg Lys Lys Ala Phe Ser
225 230 235 240

Thr Cys Ser Ala His Leu Thr Val Val Leu Val Phe Tyr Gly Thr Ile
245 250 255

Leu Phe Met Tyr Gly Lys Pro Lys Ser Lys Asp Pro Leu Gly Ala Asp
260 265 270

Lys Gln Asp Leu Ala Asp Lys Leu Ile Ser Leu Phe Tyr Gly Val Val
275 280 285

Thr Pro Met Leu Asn Pro Ile Ile Tyr Ser Leu Arg Asn Lys Asp Val
290 295 300

Arg Ala Ala Val Arg Asn Leu Val Gly Gln Lys His Leu Thr Glu
305 310 315

<210> 27
<211> 942
<212> DNA
<213> Mus musculus

<300>
<308> AF102523
<309> 1999-02-08
<313> (1)..(942)

<220>
<221> CDS
<222> (1)..(942)

<400> 27
atg gcg aac agc act act gtt act gag ttt att ttg ctg ggg ctg tca 48
Met Ala Asn Ser Thr Thr Val Thr Glu Phe Ile Leu Leu Gly Leu Ser
1 5 10 15

gat gcc tgt gag ctg cag gtg ctc ata ttc ctg ggc ttt ctc ctg acc 96
Asp Ala Cys Glu Leu Gln Val Leu Ile Phe Leu Gly Phe Leu Leu Thr
20 25 30

tac ttc ctc att ctg ctg gga aac ttc ctc atc atc ttc atc acc ctt 144
Tyr Phe Leu Ile Leu Leu Gly Asn Phe Leu Ile Ile Phe Ile Thr Leu
35 40 45

gtg gac agg cgc ctt tac acc ccc atg tat tac ttc ctc cgc aac ttt 192
Val Asp Arg Arg Leu Tyr Thr Pro Met Tyr Tyr Phe Leu Arg Asn Phe
50 55 60

gcc atg ctg gag atc tgg ttc acc tct gtc atc ttc ccc aag atg cta 240
Ala Met Leu Glu Ile Trp Phe Thr Ser Val Ile Phe Pro Lys Met Leu
65 70 75 80

acc aac atc atc aca gga cat aag acc atc tcc cta cta ggt tgt ttc 288

Thr Asn Ile Ile Thr Gly His Lys Thr Ile Ser Leu Leu Gly Cys Phe
85 90 95

ctc caa gca ttc ctc tat ttc ttc ctt ggc acc act gag ttc ttt cta 336
Leu Gln Ala Phe Leu Tyr Phe Phe Leu Gly Thr Thr Glu Phe Phe Leu
100 105 110

ctg gca gtg atg tcc ttt gac agg tat gtg gcc att tgt aac cct ttg 384
Leu Ala Val Met Ser Phe Asp Arg Tyr Val Ala Ile Cys Asn Pro Leu
115 120 125

cgt tat gcc acc att atg agc aaa aga gtc tgt gtc cag ctt gtg ttt 432
Arg Tyr Ala Thr Ile Met Ser Lys Arg Val Cys Val Gln Leu Val Phe
130 135 140

tgc tca tgg atg tct gga ttg ctt ctc atc ata gtt cct agt tca att 480
Cys Ser Trp Met Ser Gly Leu Leu Leu Ile Ile Val Pro Ser Ser Ile
145 150 155 160

gta ttt cag cag cca ttc tgt ggc cca aac atc att aat cat ttc ttc 528
Val Phe Gln Gln Pro Phe Cys Gly Pro Asn Ile Ile Asn His Phe Phe
165 170 175

tgt gac aac ttt cca ctt atg gaa ctc ata tgt gca gat act agc ctg 576
Cys Asp Asn Phe Pro Leu Met Glu Leu Ile Cys Ala Asp Thr Ser Leu
180 185 190

gta gag ttc ctg ggt ttt gtt att gcc aat ttc agc ctc ctg ggc act 624
Val Glu Phe Leu Gly Phe Val Ile Ala Asn Phe Ser Leu Leu Gly Thr
195 200 205

ctg gct gtg act gcc acc tgc tat ggc cac att ctc tat acc att cta 672
Leu Ala Val Thr Ala Thr Cys Tyr Gly His Ile Leu Tyr Thr Ile Leu
210 215 220

cac att cct tca gcc aag gag agg aag aaa gcc ttc tca act tgc tcc 720
His Ile Pro Ser Ala Lys Glu Arg Lys Lys Ala Phe Ser Thr Cys Ser
225 230 235 240

tct cat att att gtg gtg tct ctc ttc tac ggc agc tgt atc ttc atg 768
Ser His Ile Ile Val Val Ser Leu Phe Tyr Gly Ser Cys Ile Phe Met
245 250 255

tat gtc cgg tct ggc aag aat gga cag ggg gag gat cat aac aag gtg 816
Tyr Val Arg Ser Gly Lys Asn Gly Gln Gly Glu Asp His Asn Lys Val
260 265 270

gtg gca ttg ctc aac act gta gtg aca ccc aca ctc aac ccc ttc atc 864
Val Ala Leu Leu Asn Thr Val Val Thr Pro Thr Leu Asn Pro Phe Ile
275 280 285

tac act ctg agg aac aag cag gtg aag cag gta ttt agg gaa cac gta 912
Tyr Thr Leu Arg Asn Lys Gln Val Lys Gln Val Phe Arg Glu His Val
290 295 300

agc aag ttc caa aag ttc agc cag acg tga 942
Ser Lys Phe Gln Lys Phe Ser Gln Thr
305 310

<210> 28
<211> 313
<212> PRT
<213> Mus musculus

<400> 28

Met Ala Asn Ser Thr Thr Val Thr Glu Phe Ile Leu Leu Gly Leu Ser

1 5 10 15

Asp Ala Cys Glu Leu Gln Val Leu Ile Phe Leu Gly Phe Leu Leu Thr
20 25 30

Tyr Phe Leu Ile Leu Leu Gly Asn Phe Leu Ile Ile Phe Ile Thr Leu
35 40 45

Val Asp Arg Arg Leu Tyr Thr Pro Met Tyr Tyr Phe Leu Arg Asn Phe
50 55 60

Ala Met Leu Glu Ile Trp Phe Thr Ser Val Ile Phe Pro Lys Met Leu
65 70 75 80

Thr Asn Ile Ile Thr Gly His Lys Thr Ile Ser Leu Leu Gly Cys Phe
85 90 95

Leu Gln Ala Phe Leu Tyr Phe Phe Leu Gly Thr Thr Glu Phe Phe Leu
100 105 110

Leu Ala Val Met Ser Phe Asp Arg Tyr Val Ala Ile Cys Asn Pro Leu
115 120 125

Arg Tyr Ala Thr Ile Met Ser Lys Arg Val Cys Val Gln Leu Val Phe
130 135 140

Cys Ser Trp Met Ser Gly Leu Leu Leu Ile Ile Val Pro Ser Ser Ile
145 150 155 160

Val Phe Gln Gln Pro Phe Cys Gly Pro Asn Ile Ile Asn His Phe Phe
165 170 175

Cys Asp Asn Phe Pro Leu Met Glu Leu Ile Cys Ala Asp Thr Ser Leu
180 185 190

Val Glu Phe Leu Gly Phe Val Ile Ala Asn Phe Ser Leu Leu Gly Thr
195 200 205

Leu Ala Val Thr Ala Thr Cys Tyr Gly His Ile Leu Tyr Thr Ile Leu
210 215 220

His Ile Pro Ser Ala Lys Glu Arg Lys Lys Ala Phe Ser Thr Cys Ser
225 230 235 240

Ser His Ile Ile Val Val Ser Leu Phe Tyr Gly Ser Cys Ile Phe Met
245 250 255

Tyr Val Arg Ser Gly Lys Asn Gly Gln Gly Glu Asp His Asn Lys Val
260 265 270

Val Ala Leu Leu Asn Thr Val Val Thr Pro Thr Leu Asn Pro Phe Ile

275 280 285

Tyr Thr Leu Arg Asn Lys Gln Val Lys Gln Val Phe Arg Glu His Val
290 295 300

Ser Lys Phe Gln Lys Phe Ser Gln Thr
305 310

<210> 29
<211> 669
<212> DNA
<213> Mus musculus

<300>
<308> AF102531
<309> 1999-02-08
<313> (1)..(669)

<220>
<221> CDS
<222> (1)..(669)

<400> 29
tgc aac tta gcg acc atg gat att atc tgc acc tcc tct gta ctg ccc 48
Cys Asn Leu Ala Thr Met Asp Ile Ile Cys Thr Ser Ser Val Leu Pro
1 5 10 15

aag gcg ctg gtt ggt cta ctg tct gag gaa aac acc acc tcc ttc aaa 96
Lys Ala Leu Val Gly Leu Leu Ser Glu Glu Asn Thr Thr Ser Phe Lys
20 25 30

ggg tgc atg act cag ctc ttc ttt ctt gtg tgg tct gga tcc tct gag 144
Gly Cys Met Thr Gln Leu Phe Phe Leu Val Trp Ser Gly Ser Ser Glu
35 40 45

ctg ctg ctg ctc aca gtc atg gcc tat gac cgc tat gtg gcc atc tgt 192
Leu Leu Leu Leu Thr Val Met Ala Tyr Asp Arg Tyr Val Ala Ile Cys
50 55 60

ttg ccc ctg cat tac agc tct agg atg agt cca cag ctc tgt ggg acc 240
Leu Pro Leu His Tyr Ser Ser Arg Met Ser Pro Gln Leu Cys Gly Thr
65 70 75 80

ttt gcc gtg ggt gta tgg tcc atc tgc gca cta aat gca tct atc aac 288
Phe Ala Val Gly Val Trp Ser Ile Cys Ala Leu Asn Ala Ser Ile Asn
85 90 95

act ggt ctg atg aca cgg ctg tca ttc tgt ggc ccc aag gtc atc acc 336
Thr Gly Leu Met Thr Arg Leu Ser Phe Cys Gly Pro Lys Val Ile Thr
100 105 110

cac ttc ttc tgt gag att ccc cca ctc ctc ctg ctc tcc tgt agt cct 384
His Phe Phe Cys Glu Ile Pro Pro Leu Leu Leu Leu Ser Cys Ser Pro
115 120 125

aca tat ata aat agc gtt atg act ctt gtg gca gat gcc ttt tat gga 432
Thr Tyr Ile Asn Ser Val Met Thr Leu Val Ala Asp Ala Phe Tyr Gly
130 135 140

ggc atc aat ttt tta ctt acc ttg cta tcc tat ggc tgc atc att gcc 480
Gly Ile Asn Phe Leu Leu Thr Leu Leu Ser Tyr Gly Cys Ile Ile Ala
145 150 155 160

agc atc ctg cgc atg cgt tct gct gag ggc aag agg aag gcc ttt tct 528

Ser Ile Leu Arg Met Arg Ser Ala Glu Gly Lys Arg Lys Ala Phe Ser
165 170 175

acc tgc tca tcc cac ctc att gtg gtc tct gtg tac tac tca tct gtg 576
Thr Cys Ser Ser His Leu Ile Val Val Ser Val Tyr Tyr Ser Ser Val
180 185 190

ttc tgt gcc tat gtc agc cct gct tct agc tac agc cca gaa aga agc 624
Phe Cys Ala Tyr Val Ser Pro Ala Ser Ser Tyr Ser Pro Glu Arg Ser
195 200 205

aaa gtt tcc tca gtg ctg tac tca gtc ctc agc cca acc ctc aac 669
Lys Val Ser Ser Val Leu Tyr Ser Val Leu Ser Pro Thr Leu Asn
210 215 220

<210> 30
<211> 223
<212> PRT
<213> Mus musculus

<400> 30

Cys Asn Leu Ala Thr Met Asp Ile Ile Cys Thr Ser Ser Val Leu Pro
1 5 10 15

Lys Ala Leu Val Gly Leu Leu Ser Glu Glu Asn Thr Thr Ser Phe Lys
20 25 30

Gly Cys Met Thr Gln Leu Phe Phe Leu Val Trp Ser Gly Ser Ser Glu
35 40 45

Leu Leu Leu Leu Thr Val Met Ala Tyr Asp Arg Tyr Val Ala Ile Cys
50 55 60

Leu Pro Leu His Tyr Ser Ser Arg Met Ser Pro Gln Leu Cys Gly Thr
65 70 75 80

Phe Ala Val Gly Val Trp Ser Ile Cys Ala Leu Asn Ala Ser Ile Asn
85 90 95

Thr Gly Leu Met Thr Arg Leu Ser Phe Cys Gly Pro Lys Val Ile Thr
100 105 110

His Phe Phe Cys Glu Ile Pro Pro Leu Leu Leu Leu Ser Cys Ser Pro
115 120 125

Thr Tyr Ile Asn Ser Val Met Thr Leu Val Ala Asp Ala Phe Tyr Gly
130 135 140

Gly Ile Asn Phe Leu Leu Thr Leu Leu Ser Tyr Gly Cys Ile Ile Ala
145 150 155 160

Ser Ile Leu Arg Met Arg Ser Ala Glu Gly Lys Arg Lys Ala Phe Ser
165 170 175

Thr Cys Ser Ser His Leu Ile Val Val Ser Val Tyr Tyr Ser Ser Val

180 185 190

Phe Cys Ala Tyr Val Ser Pro Ala Ser Ser Tyr Ser Pro Glu Arg Ser
195 200 205

Lys Val Ser Ser Val Leu Tyr Ser Val Leu Ser Pro Thr Leu Asn
210 215 220

<210> 31
<211> 1661
<212> DNA
<213> Mus musculus

<300>
<308> AF121974
<309> 1999-04-25
<313> (1)..(1661)

<220>
<221> misc_feature
<222> (3)..(3)
<223> n is a, c, g, or t

<220>
<221> CDS
<222> (303)..(1307)

<400> 31
gtntacatag tgagttcgag gccagccagg gctacacaga caaaccctgt ctcgaaaaac 60

caaaaaaaaa aaaaaaaaaa agaattcatt aatgaaaaag aagggggaaa atggagggcc 120

atggaaagta gctacttcta acatacaact cttcatttcc tccatagaaa tgctgtagtt 180

aatgtctaca cccagtccag cctggtgagg ctggggcagg tcctagcagg gcctttcagg 240

gactgaaccc cggcatcctg cccctcccct ctccctggag cctccccaag ccctcaggcg 300

tc atg tca ggg tgg agc aat ggc acc tac aat gag tcc tac acc agc 347
Met Ser Gly Trp Ser Asn Gly Thr Tyr Asn Glu Ser Tyr Thr Ser
1 5 10 15

ttc ctc ctc atg ggc ttc cca ggg atg cag gaa gcc aga gcc ctc ctg 395
Phe Leu Leu Met Gly Phe Pro Gly Met Gln Glu Ala Arg Ala Leu Leu
20 25 30

gtg ctg ccc ttc ctc agc ctc tac ctg gtg atc ctc ttc acc aat gcc 443
Val Leu Pro Phe Leu Ser Leu Tyr Leu Val Ile Leu Phe Thr Asn Ala
35 40 45

ctg gtc atc cac acg gtg gca tcc cag cgc agc ctg cac cag ccc atg 491
Leu Val Ile His Thr Val Ala Ser Gln Arg Ser Leu His Gln Pro Met
50 55 60

tac ctg ctc att gcc ctg ctc ctg gct gtc aat atc tgc gct gcc acc 539
Tyr Leu Leu Ile Ala Leu Leu Leu Ala Val Asn Ile Cys Ala Ala Thr
65 70 75

acc gtg gtg ccc ccc atg ctc ttc agc ttc tcc aca cgc ttc aac cgc 587
Thr Val Val Pro Pro Met Leu Phe Ser Phe Ser Thr Arg Phe Asn Arg
80 85 90 95

atc tcc ctc cct cga tgc ttg gga caa atg ttc tgc atc tac ttc ctt 635
Ile Ser Leu Pro Arg Cys Leu Gly Gln Met Phe Cys Ile Tyr Phe Leu

```
                        100                           105                          110

att gtc ttt gac tgc aac atc ctc ctg gtc atg gct cta gat cgc tat        683
Ile Val Phe Asp Cys Asn Ile Leu Leu Val Met Ala Leu Asp Arg Tyr
            115                     120                     125

gtg gct atc tgc tac cct ctc cgc tac cca gaa ata gtg aca gga cag        731
Val Ala Ile Cys Tyr Pro Leu Arg Tyr Pro Glu Ile Val Thr Gly Gln
        130                     135                     140

tta ctg gct ggt ctg gtg gtg ctg gca gtc acc agg agc aca agc att        779
Leu Leu Ala Gly Leu Val Val Leu Ala Val Thr Arg Ser Thr Ser Ile
    145                     150                     155

gtt gct cca gtg gtg gtg ctg gcc tcg cgg gtt cgc ttc tgt cgc tca        827
Val Ala Pro Val Val Val Leu Ala Ser Arg Val Arg Phe Cys Arg Ser
160                     165                     170                     175

gat gtg atc cgc cac ttt gcc tgt gag cac atg gcc ctg atg aag ctt        875
Asp Val Ile Arg His Phe Ala Cys Glu His Met Ala Leu Met Lys Leu
                180                     185                     190

tcc tgt ggg gac atc tcg ctg aat aag acg gtg gga ctc act gtt cgc        923
Ser Cys Gly Asp Ile Ser Leu Asn Lys Thr Val Gly Leu Thr Val Arg
            195                     200                     205

atc ttc aac cga gtc ctg gat atg ctc ctg tta ggt gcc tcc tac tcc        971
Ile Phe Asn Arg Val Leu Asp Met Leu Leu Leu Gly Ala Ser Tyr Ser
        210                     215                     220

cgc atc atc cat gct gcc ttc agg atc tca tca ggt gga gca cgg tcc       1019
Arg Ile Ile His Ala Ala Phe Arg Ile Ser Ser Gly Gly Ala Arg Ser
    225                     230                     235

aaa gcc ctg aac acc tgt ggc tcc cac ctg ctg gtc atc ttc acc gtc       1067
Lys Ala Leu Asn Thr Cys Gly Ser His Leu Leu Val Ile Phe Thr Val
240                     245                     250                     255

tac tcc tcc acc atg tcc tca tcc att gtc tac cgt gtg gca cgc act       1115
Tyr Ser Ser Thr Met Ser Ser Ser Ile Val Tyr Arg Val Ala Arg Thr
                260                     265                     270

gcc tcc caa gat gtg cac aac ttg ctt agt gct ttc tat ctg ttg ctc       1163
Ala Ser Gln Asp Val His Asn Leu Leu Ser Ala Phe Tyr Leu Leu Leu
            275                     280                     285

ccc tgt ctg gtc aac ccc atc atc tac ggg gcc aga acc aag gaa atc       1211
Pro Cys Leu Val Asn Pro Ile Ile Tyr Gly Ala Arg Thr Lys Glu Ile
        290                     295                     300

agg cag cac ctg gta gct ctg ttc caa agg act cag caa cag gtc ttc       1259
Arg Gln His Leu Val Ala Leu Phe Gln Arg Thr Gln Gln Gln Val Phe
    305                     310                     315

act gag aag ccc cag tcc ctg ccc tcg aat aga gag ctt cct gga tga       1307
Thr Glu Lys Pro Gln Ser Leu Pro Ser Asn Arg Glu Leu Pro Gly
320                     325                     330

ttgtccagaa tttgtgggtc tcaaaatcac tttcactatt cagtgaagga ggggcattca     1367

agtgggcatt cgtctctggt atattttgtc tcggctattt tagttcagca tcctatttat     1427

gagaagggtc tattctatat ctccagctgt ctagaactcc ttaagtggcc caggatgacc     1487

tggaacccaa acaattctcc tttcttagtt tgccaaatgc tagcattaga ggcatgagtc     1547

acagtgcctg gcttatctgc actcatactg gagagcctca tgtctgcttt ccaaaaagca     1607
```

cctactcact ctgaactagc aactgaaagc aagctctaac cctggcttga agtt 1661

<210> 32
<211> 334
<212> PRT
<213> Mus musculus

<400> 32

Met Ser Gly Trp Ser Asn Gly Thr Tyr Asn Glu Ser Tyr Thr Ser Phe
1 5 10 15

Leu Leu Met Gly Phe Pro Gly Met Gln Glu Ala Arg Ala Leu Leu Val
20 25 30

Leu Pro Phe Leu Ser Leu Tyr Leu Val Ile Leu Phe Thr Asn Ala Leu
35 40 45

Val Ile His Thr Val Ala Ser Gln Arg Ser Leu His Gln Pro Met Tyr
50 55 60

Leu Leu Ile Ala Leu Leu Leu Ala Val Asn Ile Cys Ala Ala Thr Thr
65 70 75 80

Val Val Pro Pro Met Leu Phe Ser Phe Ser Thr Arg Phe Asn Arg Ile
85 90 95

Ser Leu Pro Arg Cys Leu Gly Gln Met Phe Cys Ile Tyr Phe Leu Ile
100 105 110

Val Phe Asp Cys Asn Ile Leu Leu Val Met Ala Leu Asp Arg Tyr Val
115 120 125

Ala Ile Cys Tyr Pro Leu Arg Tyr Pro Glu Ile Val Thr Gly Gln Leu
130 135 140

Leu Ala Gly Leu Val Val Leu Ala Val Thr Arg Ser Thr Ser Ile Val
145 150 155 160

Ala Pro Val Val Val Leu Ala Ser Arg Val Arg Phe Cys Arg Ser Asp
165 170 175

Val Ile Arg His Phe Ala Cys Glu His Met Ala Leu Met Lys Leu Ser
180 185 190

Cys Gly Asp Ile Ser Leu Asn Lys Thr Val Gly Leu Thr Val Arg Ile
195 200 205

Phe Asn Arg Val Leu Asp Met Leu Leu Leu Gly Ala Ser Tyr Ser Arg
210 215 220

Ile Ile His Ala Ala Phe Arg Ile Ser Ser Gly Gly Ala Arg Ser Lys
225 230 235 240

Ala Leu Asn Thr Cys Gly Ser His Leu Leu Val Ile Phe Thr Val Tyr
245 250 255

Ser Ser Thr Met Ser Ser Ser Ile Val Tyr Arg Val Ala Arg Thr Ala
260 265 270

Ser Gln Asp Val His Asn Leu Leu Ser Ala Phe Tyr Leu Leu Leu Pro
275 280 285

Cys Leu Val Asn Pro Ile Ile Tyr Gly Ala Arg Thr Lys Glu Ile Arg
290 295 300

Gln His Leu Val Ala Leu Phe Gln Arg Thr Gln Gln Gln Val Phe Thr
305 310 315 320

Glu Lys Pro Gln Ser Leu Pro Ser Asn Arg Glu Leu Pro Gly
325 330

<210> 33
<211> 1116
<212> DNA
<213> Mus musculus

<300>
<308> AF121975
<309> 1999-04-25
<313> (1)..(1116)

<220>
<221> misc_feature
<222> (15)..(15)
<223> n is a, c, g, or t

<220>
<221> CDS
<222> (50)..(1015)

<400> 33
caagctggct cttcntactg tctctccatt agttttagtc gtcacggga atg aat tca 58
Met Asn Ser
1

aaa gca agc atg ctt gga act aac ttc act atc atc cat cca act gtg 106
Lys Ala Ser Met Leu Gly Thr Asn Phe Thr Ile Ile His Pro Thr Val
5 10 15

ttc atc ctg ctt gga atc cca ggg ctg gag cag tac cac acc tgg ctt 154
Phe Ile Leu Leu Gly Ile Pro Gly Leu Glu Gln Tyr His Thr Trp Leu
20 25 30 35

tct att cct ttt tgt ctt atg tac att gct gca gtc ttg ggg aac gga 202
Ser Ile Pro Phe Cys Leu Met Tyr Ile Ala Ala Val Leu Gly Asn Gly
40 45 50

gcc ctc atc ctt gtt gtc ctg agt gaa cgc acc ctc cat gag ccc atg 250
Ala Leu Ile Leu Val Val Leu Ser Glu Arg Thr Leu His Glu Pro Met
55 60 65

tat gtc ttt ctg tcc atg ctg gct ggc act gat att ctc ctg tca acc 298

```
Tyr Val Phe Leu Ser Met Leu Ala Gly Thr Asp Ile Leu Leu Ser Thr
        70                  75                  80

acc act gtg cct aag acc ttg gct atc ttt tgg ttc cat gct ggg gag      346
Thr Thr Val Pro Lys Thr Leu Ala Ile Phe Trp Phe His Ala Gly Glu
    85                  90                  95

atc ccc ttt gat gcc tgc att gct cag atg ttt ttc atc cac gtt gct      394
Ile Pro Phe Asp Ala Cys Ile Ala Gln Met Phe Phe Ile His Val Ala
100                 105                 110                 115

ttt gtg gct gag tcg gga atc ctt ctg gcc atg gca ttt gac cga tat      442
Phe Val Ala Glu Ser Gly Ile Leu Leu Ala Met Ala Phe Asp Arg Tyr
                120                 125                 130

gtg gct att tgt act cct ctg aga tac tca gcc gtc tta aca cct atg      490
Val Ala Ile Cys Thr Pro Leu Arg Tyr Ser Ala Val Leu Thr Pro Met
            135                 140                 145

gca att gga aaa atg acc ctg gcc atc tgg gga cgg agc att ggg aca      538
Ala Ile Gly Lys Met Thr Leu Ala Ile Trp Gly Arg Ser Ile Gly Thr
        150                 155                 160

att ttc cct atc ata ttt ctg ctg aag agg ctg tca tac tgc agg acc      586
Ile Phe Pro Ile Ile Phe Leu Leu Lys Arg Leu Ser Tyr Cys Arg Thr
    165                 170                 175

aat gtc atc cca cac tca tat tgt gag cat att ggt gta gcc aga ttg      634
Asn Val Ile Pro His Ser Tyr Cys Glu His Ile Gly Val Ala Arg Leu
180                 185                 190                 195

gct tgt gct gac atc act gtc aat atc tgg tat ggc ttc tcg gtg cca      682
Ala Cys Ala Asp Ile Thr Val Asn Ile Trp Tyr Gly Phe Ser Val Pro
                200                 205                 210

atg gct tca gtt ttg gta gat gtt gca ctc att ggt att tct tat acg      730
Met Ala Ser Val Leu Val Asp Val Ala Leu Ile Gly Ile Ser Tyr Thr
            215                 220                 225

ttg atc ctc cag gct gtg ttt aga ctt cct tcc cag gat gct agg cac      778
Leu Ile Leu Gln Ala Val Phe Arg Leu Pro Ser Gln Asp Ala Arg His
        230                 235                 240

aag gcc ctc aat acc tgt ggt tct cac att ggg gtc att ctc ctc ttt      826
Lys Ala Leu Asn Thr Cys Gly Ser His Ile Gly Val Ile Leu Leu Phe
    245                 250                 255

ttc ata cca tca ttt ttt act ttc ctt act cat cgc ttt ggc aag aac      874
Phe Ile Pro Ser Phe Phe Thr Phe Leu Thr His Arg Phe Gly Lys Asn
260                 265                 270                 275

atc ccc cac cat gtg cac att ctt ctg gca aat ctc tat gtg ttg gtt      922
Ile Pro His His Val His Ile Leu Leu Ala Asn Leu Tyr Val Leu Val
                280                 285                 290

ccc ccc atg ctt aac cct atc atc tat ggt gct aag acc aag caa att      970
Pro Pro Met Leu Asn Pro Ile Ile Tyr Gly Ala Lys Thr Lys Gln Ile
            295                 300                 305

agg gac agc atg act cgc atg ttg tct gtt gtg tgg aag tct tga          1015
Arg Asp Ser Met Thr Arg Met Leu Ser Val Val Trp Lys Ser
        310                 315                 320

gagcagtcac agttcacaaa gctgtcttag tttctcttac aaacaggaga gagagagaga    1075

gagagagaga gagagagaga gagagagaga gagagagaga g                        1116
```

<210> 34
<211> 321
<212> PRT
<213> Mus musculus

<400> 34

Met Asn Ser Lys Ala Ser Met Leu Gly Thr Asn Phe Thr Ile Ile His
1               5                   10                  15

Pro Thr Val Phe Ile Leu Leu Gly Ile Pro Gly Leu Glu Gln Tyr His
            20                  25                  30

Thr Trp Leu Ser Ile Pro Phe Cys Leu Met Tyr Ile Ala Ala Val Leu
        35                  40                  45

Gly Asn Gly Ala Leu Ile Leu Val Val Leu Ser Glu Arg Thr Leu His
    50                  55                  60

Glu Pro Met Tyr Val Phe Leu Ser Met Leu Ala Gly Thr Asp Ile Leu
65                  70                  75                  80

Leu Ser Thr Thr Thr Val Pro Lys Thr Leu Ala Ile Phe Trp Phe His
                85                  90                  95

Ala Gly Glu Ile Pro Phe Asp Ala Cys Ile Ala Gln Met Phe Phe Ile
            100                 105                 110

His Val Ala Phe Val Ala Glu Ser Gly Ile Leu Leu Ala Met Ala Phe
        115                 120                 125

Asp Arg Tyr Val Ala Ile Cys Thr Pro Leu Arg Tyr Ser Ala Val Leu
    130                 135                 140

Thr Pro Met Ala Ile Gly Lys Met Thr Leu Ala Ile Trp Gly Arg Ser
145                 150                 155                 160

Ile Gly Thr Ile Phe Pro Ile Ile Phe Leu Leu Lys Arg Leu Ser Tyr
                165                 170                 175

Cys Arg Thr Asn Val Ile Pro His Ser Tyr Cys Glu His Ile Gly Val
            180                 185                 190

Ala Arg Leu Ala Cys Ala Asp Ile Thr Val Asn Ile Trp Tyr Gly Phe
        195                 200                 205

Ser Val Pro Met Ala Ser Val Leu Val Asp Val Ala Leu Ile Gly Ile
    210                 215                 220

Ser Tyr Thr Leu Ile Leu Gln Ala Val Phe Arg Leu Pro Ser Gln Asp
225                 230                 235                 240

Ala Arg His Lys Ala Leu Asn Thr Cys Gly Ser His Ile Gly Val Ile

245 250 255

Leu Leu Phe Phe Ile Pro Ser Phe Phe Thr Phe Leu Thr His Arg Phe
260 265 270

Gly Lys Asn Ile Pro His His Val His Ile Leu Leu Ala Asn Leu Tyr
275 280 285

Val Leu Val Pro Pro Met Leu Asn Pro Ile Ile Tyr Gly Ala Lys Thr
290 295 300

Lys Gln Ile Arg Asp Ser Met Thr Arg Met Leu Ser Val Val Trp Lys
305 310 315 320

Ser

<210> 35
<211> 1267
<212> DNA
<213> Mus musculus

<300>
<308> AF121977
<309> 1999-04-25
<313> (1)..(1267)

<220>
<221> misc_feature
<222> (108)..(108)
<223> n is a, c, g, or t

<220>
<221> CDS
<222> (172)..(1200)

<400> 35
tctattgctc actgaaatat aaactagcaa catgaagaac atatgattga actatatcaa 60

agaaacaaat ttttctaatc ataaatgacc atgaatcatt gaatttcnta agctgaagtt 120

ctttcatgag gtaccacaca acagcatgtt cctgtacaca tgtaactacc t atg ttt 177
Met Phe
1

tgt cat tta tat aat gag aac aat atg caa gtg gca atc ctg gat tcc 225
Cys His Leu Tyr Asn Glu Asn Asn Met Gln Val Ala Ile Leu Asp Ser
5 10 15

att cta ata cct tct tat ttt tct ttc ctg aca gag atg gag cct gga 273
Ile Leu Ile Pro Ser Tyr Phe Ser Phe Leu Thr Glu Met Glu Pro Gly
20 25 30

aac tac aca gtt gta aca gaa ttc att ctt tta ggg tta aca gat gat 321
Asn Tyr Thr Val Val Thr Glu Phe Ile Leu Leu Gly Leu Thr Asp Asp
35 40 45 50

att aca gtc agt gtc att tta ttt gtt atg ttt cta atc gtc tat tct 369
Ile Thr Val Ser Val Ile Leu Phe Val Met Phe Leu Ile Val Tyr Ser
55 60 65

```
gtt act tta atg ggt aac ttg aac ata att gtg cta atc aga acc agc      417
Val Thr Leu Met Gly Asn Leu Asn Ile Ile Val Leu Ile Arg Thr Ser
            70                  75                  80

cct cag ctt cac acc ccc atg tac ctt ttc ctt agc cat ttg gcc ttt      465
Pro Gln Leu His Thr Pro Met Tyr Leu Phe Leu Ser His Leu Ala Phe
        85                  90                  95

cta gac att ggg tac tcc agc tca gtt aca ccc atc atg ctg agg ggc      513
Leu Asp Ile Gly Tyr Ser Ser Ser Val Thr Pro Ile Met Leu Arg Gly
    100                 105                 110

ttt ctc aga aag gga aca ttt atc cct gtg gct ggc tgt gtg gct caa      561
Phe Leu Arg Lys Gly Thr Phe Ile Pro Val Ala Gly Cys Val Ala Gln
115                 120                 125                 130

ctc tgt att gtg gtg gca ttt ggg aca tct gaa tct ttc ttg cta gct      609
Leu Cys Ile Val Val Ala Phe Gly Thr Ser Glu Ser Phe Leu Leu Ala
                135                 140                 145

tcc atg gcc tat gac cgc tat gtg gcc atc tgc tca cct ttg ctc tac      657
Ser Met Ala Tyr Asp Arg Tyr Val Ala Ile Cys Ser Pro Leu Leu Tyr
            150                 155                 160

tca aca cag atg tcc tcc aca gtc tgc atc ctc cta gtt gga act tcc      705
Ser Thr Gln Met Ser Ser Thr Val Cys Ile Leu Leu Val Gly Thr Ser
        165                 170                 175

tac cta ggt gga tgg gtg aat gct tgg ata ttt act ggt tgc tcc tta      753
Tyr Leu Gly Gly Trp Val Asn Ala Trp Ile Phe Thr Gly Cys Ser Leu
    180                 185                 190

aat ctg tca ttt tgt ggg cca aat aaa att aat cac ttt ttc tgt gac      801
Asn Leu Ser Phe Cys Gly Pro Asn Lys Ile Asn His Phe Phe Cys Asp
195                 200                 205                 210

tat tca cca cta ttg aag ctt tct tgt tct cat gac ttt tct ttt gaa      849
Tyr Ser Pro Leu Leu Lys Leu Ser Cys Ser His Asp Phe Ser Phe Glu
                215                 220                 225

gtc att cca gca atc tct tcg gga tcc atc att gtg gtc act gtg ttt      897
Val Ile Pro Ala Ile Ser Ser Gly Ser Ile Ile Val Val Thr Val Phe
            230                 235                 240

atc att gct ctg tct tat gtc tac atc ctt gtg tca atc ctg aag atg      945
Ile Ile Ala Leu Ser Tyr Val Tyr Ile Leu Val Ser Ile Leu Lys Met
        245                 250                 255

cgc tct act gaa ggt cgc cag aag gcc ttc tcc acc tgc act tcc cac      993
Arg Ser Thr Glu Gly Arg Gln Lys Ala Phe Ser Thr Cys Thr Ser His
    260                 265                 270

ctc act gca gtc act ctg ttc ttt ggg acc atc aca ttc att tat gtg     1041
Leu Thr Ala Val Thr Leu Phe Phe Gly Thr Ile Thr Phe Ile Tyr Val
275                 280                 285                 290

atg ccc cag tcc agc tac tcc aca gac cag aac aaa gtg gtg tct gtg     1089
Met Pro Gln Ser Ser Tyr Ser Thr Asp Gln Asn Lys Val Val Ser Val
                295                 300                 305

ttt tac aca gtg gtg atc ccc atg ttg aat ccc ctc atc tac agt ttc     1137
Phe Tyr Thr Val Val Ile Pro Met Leu Asn Pro Leu Ile Tyr Ser Phe
            310                 315                 320

aga aac aaa gag gtt aaa gaa gcc atg aaa aaa ctg att gct aaa aca     1185
Arg Asn Lys Glu Val Lys Glu Ala Met Lys Lys Leu Ile Ala Lys Thr
        325                 330                 335
```

cat tgg tgg tcc tga aatatttgaa tttacaaaca gtaaattctg ctcttacagg 1240
His Trp Trp Ser
340

taaatggcag tatactaagt aaattac 1267

<210> 36
<211> 342
<212> PRT
<213> Mus musculus

<400> 36

Met Phe Cys His Leu Tyr Asn Glu Asn Asn Met Gln Val Ala Ile Leu
1 5 10 15

Asp Ser Ile Leu Ile Pro Ser Tyr Phe Ser Phe Leu Thr Glu Met Glu
20 25 30

Pro Gly Asn Tyr Thr Val Val Thr Glu Phe Ile Leu Leu Gly Leu Thr
35 40 45

Asp Asp Ile Thr Val Ser Val Ile Leu Phe Val Met Phe Leu Ile Val
50 55 60

Tyr Ser Val Thr Leu Met Gly Asn Leu Asn Ile Ile Val Leu Ile Arg
65 70 75 80

Thr Ser Pro Gln Leu His Thr Pro Met Tyr Leu Phe Leu Ser His Leu
85 90 95

Ala Phe Leu Asp Ile Gly Tyr Ser Ser Ser Val Thr Pro Ile Met Leu
100 105 110

Arg Gly Phe Leu Arg Lys Gly Thr Phe Ile Pro Val Ala Gly Cys Val
115 120 125

Ala Gln Leu Cys Ile Val Val Ala Phe Gly Thr Ser Glu Ser Phe Leu
130 135 140

Leu Ala Ser Met Ala Tyr Asp Arg Tyr Val Ala Ile Cys Ser Pro Leu
145 150 155 160

Leu Tyr Ser Thr Gln Met Ser Ser Thr Val Cys Ile Leu Leu Val Gly
165 170 175

Thr Ser Tyr Leu Gly Gly Trp Val Asn Ala Trp Ile Phe Thr Gly Cys
180 185 190

Ser Leu Asn Leu Ser Phe Cys Gly Pro Asn Lys Ile Asn His Phe Phe
195 200 205

Cys Asp Tyr Ser Pro Leu Leu Lys Leu Ser Cys Ser His Asp Phe Ser
210 215 220

Phe Glu Val Ile Pro Ala Ile Ser Ser Gly Ser Ile Ile Val Val Thr
225 230 235 240

Val Phe Ile Ile Ala Leu Ser Tyr Val Tyr Ile Leu Val Ser Ile Leu
245 250 255

Lys Met Arg Ser Thr Glu Gly Arg Gln Lys Ala Phe Ser Thr Cys Thr
260 265 270

Ser His Leu Thr Ala Val Thr Leu Phe Phe Gly Thr Ile Thr Phe Ile
275 280 285

Tyr Val Met Pro Gln Ser Ser Tyr Ser Thr Asp Gln Asn Lys Val Val
290 295 300

Ser Val Phe Tyr Thr Val Val Ile Pro Met Leu Asn Pro Leu Ile Tyr
305 310 315 320

Ser Phe Arg Asn Lys Glu Val Lys Glu Ala Met Lys Lys Leu Ile Ala
325 330 335

Lys Thr His Trp Trp Ser
340

<210> 37
<211> 1120
<212> DNA
<213> Mus musculus

<300>
<308> AF121979
<309> 1999-04-25
<313> (1)..(1120)

<220>
<221> CDS
<222> (84)..(1040)

<220>
<221> misc_feature
<222> (940)..(940)
<223> n is a, c, g, or t

<220>
<221> misc_feature
<222> (1083)..(1083)
<223> n is a, c, g, or t

<400> 37
tgtcattatt agtgctgata aagtgttgtc aagtcctgtg agattccttc aaatgaatat 60

gtccatcaga ggctcctgac aac atg tca cca ggc aac agc tca tgg att cat 113
Met Ser Pro Gly Asn Ser Ser Trp Ile His
1 5 10

cct tct tcc ttc ctg ctc ttg gga atc cca gga ctg gaa gag ttg cag 161
Pro Ser Ser Phe Leu Leu Leu Gly Ile Pro Gly Leu Glu Glu Leu Gln

```
                    15                  20                  25

ttc tgg ctt ggt ttg cca ttt gga aca gtc tat ctt att gct gtc cta      209
Phe Trp Leu Gly Leu Pro Phe Gly Thr Val Tyr Leu Ile Ala Val Leu
            30                  35                  40

ggg aat gtc atc att ctc ttt gta atc tat cta gag cac agc ctt cac      257
Gly Asn Val Ile Ile Leu Phe Val Ile Tyr Leu Glu His Ser Leu His
        45                  50                  55

caa cct atg ttc tac tta ctg gcc ata ctg gct gtt act gac ttg ggt      305
Gln Pro Met Phe Tyr Leu Leu Ala Ile Leu Ala Val Thr Asp Leu Gly
    60                  65                  70

ctg tct aca gca act gtt ccc aga gca ctc ggt ata ttc tgg ttt ggc      353
Leu Ser Thr Ala Thr Val Pro Arg Ala Leu Gly Ile Phe Trp Phe Gly
75                  80                  85                  90

ttc cat aag att gcc ttt agg gac tgt gta gct caa atg ttt ttc ata      401
Phe His Lys Ile Ala Phe Arg Asp Cys Val Ala Gln Met Phe Phe Ile
                95                 100                 105

cat ctg ttt aca ggc atc gaa aca ttc atg ctt gta gct atg gcc ttt      449
His Leu Phe Thr Gly Ile Glu Thr Phe Met Leu Val Ala Met Ala Phe
            110                 115                 120

gat cgc tac att gcc atc tgt aac cct ctc cga tat aac act atc ctc      497
Asp Arg Tyr Ile Ala Ile Cys Asn Pro Leu Arg Tyr Asn Thr Ile Leu
        125                 130                 135

acc aac aga aca atc tgc att att gtt gga gtt gga cta ttt aaa aat      545
Thr Asn Arg Thr Ile Cys Ile Ile Val Gly Val Gly Leu Phe Lys Asn
    140                 145                 150

ttc att ttg gtt ttt cca ctt ata ttt ctc att cta agg ctt tca ttc      593
Phe Ile Leu Val Phe Pro Leu Ile Phe Leu Ile Leu Arg Leu Ser Phe
155                 160                 165                 170

tgt gga cac aat atc ata cca cac aca tac tgt gag cac atg ggc att      641
Cys Gly His Asn Ile Ile Pro His Thr Tyr Cys Glu His Met Gly Ile
                175                 180                 185

gct cga ctg gca tgc gtc agc atc aag gtt aat gta tta ttt gga tta      689
Ala Arg Leu Ala Cys Val Ser Ile Lys Val Asn Val Leu Phe Gly Leu
            190                 195                 200

ata ctc ata tct atg ata ctt ctg gat gtt gtt ttg agt gct ctg tcc      737
Ile Leu Ile Ser Met Ile Leu Leu Asp Val Val Leu Ser Ala Leu Ser
        205                 210                 215

tat gcg aaa att ctt cat gct gta ttt aaa ctc cca tcc tgg gaa gcc      785
Tyr Ala Lys Ile Leu His Ala Val Phe Lys Leu Pro Ser Trp Glu Ala
    220                 225                 230

aga ctc aaa gct ctt aat acc tgt ggt tcc cat gtg tgt gtg atc ttg      833
Arg Leu Lys Ala Leu Asn Thr Cys Gly Ser His Val Cys Val Ile Leu
235                 240                 245                 250

gct ttc ttc act cca gcc ttt ttc tcc ttc ttg act cat cga ttt gga      881
Ala Phe Phe Thr Pro Ala Phe Phe Ser Phe Leu Thr His Arg Phe Gly
                255                 260                 265

cac aat att cca cga tat atc cac atc ctc ctt gct aac tta tat gtg      929
His Asn Ile Pro Arg Tyr Ile His Ile Leu Leu Ala Asn Leu Tyr Val
            270                 275                 280

atc att ccc cng gct ctt aac cct att att tat ggg gtg aga acc aaa      977
Ile Ile Pro Xaa Ala Leu Asn Pro Ile Ile Tyr Gly Val Arg Thr Lys
```

```
        285                 290                 295

cag ata caa gat cgt gcg gtg aca ata ttg tgc aac gag gtt gga cag      1025
Gln Ile Gln Asp Arg Ala Val Thr Ile Leu Cys Asn Glu Val Gly Gln
    300                 305                 310

ctg gca gac gac tag tatgtcttct aatagtctct ttccttccta agaggactac      1080
Leu Ala Asp Asp
315

tgntttgtaa gcttgcatac gtggaacaca ttacacaatg                          1120
```

<210> 38
<211> 318
<212> PRT
<213> Mus musculus

<220>
<221> misc_feature
<222> (286)..(286)
<223> The 'Xaa' at location 286 stands for Gln, Arg, Pro, or Leu.

<400> 38

```
Met Ser Pro Gly Asn Ser Ser Trp Ile His Pro Ser Ser Phe Leu Leu
1               5                   10                  15

Leu Gly Ile Pro Gly Leu Glu Glu Leu Gln Phe Trp Leu Gly Leu Pro
            20                  25                  30

Phe Gly Thr Val Tyr Leu Ile Ala Val Leu Gly Asn Val Ile Ile Leu
        35                  40                  45

Phe Val Ile Tyr Leu Glu His Ser Leu His Gln Pro Met Phe Tyr Leu
    50                  55                  60

Leu Ala Ile Leu Ala Val Thr Asp Leu Gly Leu Ser Thr Ala Thr Val
65                  70                  75                  80

Pro Arg Ala Leu Gly Ile Phe Trp Phe Gly Phe His Lys Ile Ala Phe
                85                  90                  95

Arg Asp Cys Val Ala Gln Met Phe Phe Ile His Leu Phe Thr Gly Ile
            100                 105                 110

Glu Thr Phe Met Leu Val Ala Met Ala Phe Asp Arg Tyr Ile Ala Ile
        115                 120                 125

Cys Asn Pro Leu Arg Tyr Asn Thr Ile Leu Thr Asn Arg Thr Ile Cys
    130                 135                 140

Ile Ile Val Gly Val Gly Leu Phe Lys Asn Phe Ile Leu Val Phe Pro
145                 150                 155                 160

Leu Ile Phe Leu Ile Leu Arg Leu Ser Phe Cys Gly His Asn Ile Ile
                165                 170                 175
```

Pro His Thr Tyr Cys Glu His Met Gly Ile Ala Arg Leu Ala Cys Val
180 185 190

Ser Ile Lys Val Asn Val Leu Phe Gly Leu Ile Leu Ile Ser Met Ile
195 200 205

Leu Leu Asp Val Val Leu Ser Ala Leu Ser Tyr Ala Lys Ile Leu His
210 215 220

Ala Val Phe Lys Leu Pro Ser Trp Glu Ala Arg Leu Lys Ala Leu Asn
225 230 235 240

Thr Cys Gly Ser His Val Cys Val Ile Leu Ala Phe Phe Thr Pro Ala
245 250 255

Phe Phe Ser Phe Leu Thr His Arg Phe Gly His Asn Ile Pro Arg Tyr
260 265 270

Ile His Ile Leu Leu Ala Asn Leu Tyr Val Ile Ile Pro Xaa Ala Leu
275 280 285

Asn Pro Ile Ile Tyr Gly Val Arg Thr Lys Gln Ile Gln Asp Arg Ala
290 295 300

Val Thr Ile Leu Cys Asn Glu Val Gly Gln Leu Ala Asp Asp
305 310 315

<210> 39
<211> 2333
<212> DNA
<213> Mus musculus

<300>
<308> M36778
<309> 1995-08-22
<313> (1)..(2333)

<220>
<221> CDS
<222> (24)..(1088)

<400> 39
gctgtggcag ggaaggggcc acc atg gga tgt acg ctg agc gca gag gag aga 53
Met Gly Cys Thr Leu Ser Ala Glu Glu Arg
1 5 10

gcc gcc ctc gag cgg agc aag gcg att gag aaa aac ctc aaa gaa gat 101
Ala Ala Leu Glu Arg Ser Lys Ala Ile Glu Lys Asn Leu Lys Glu Asp
15 20 25

ggc atc agc gcc gcc aaa gac gtg aaa tta ctc ctg ctg ggg gct gga 149
Gly Ile Ser Ala Ala Lys Asp Val Lys Leu Leu Leu Leu Gly Ala Gly
30 35 40

gaa tca gga aaa agc acc att gtg aag cag atg aag atc atc cat gaa 197
Glu Ser Gly Lys Ser Thr Ile Val Lys Gln Met Lys Ile Ile His Glu
45 50 55

```
gat ggc ttc tct ggg gaa gac gtg aag cag tac aag cct gtg gtc tac        245
Asp Gly Phe Ser Gly Glu Asp Val Lys Gln Tyr Lys Pro Val Val Tyr
    60                  65                  70

agc aac acc atc cag tct ctg gcg gcc att gtc cgg gcc atg gac act        293
Ser Asn Thr Ile Gln Ser Leu Ala Ala Ile Val Arg Ala Met Asp Thr
75                  80                  85                  90

ttg ggc gtg gag tat ggt gac aag gag agg aag acg gac tcc aag atg        341
Leu Gly Val Glu Tyr Gly Asp Lys Glu Arg Lys Thr Asp Ser Lys Met
                95                  100                 105

gtg tgt gac gtg gtg agt cgt atg gaa gac act gaa ccg ttc tct gca        389
Val Cys Asp Val Val Ser Arg Met Glu Asp Thr Glu Pro Phe Ser Ala
            110                 115                 120

gaa ctt ctt tct gcc atg atg cga ctc tgg ggc gac tcg ggg atc cag        437
Glu Leu Leu Ser Ala Met Met Arg Leu Trp Gly Asp Ser Gly Ile Gln
        125                 130                 135

gag tgc ttc aac cga tct cgg gag tat cag ctc aat gac tct gcc aaa        485
Glu Cys Phe Asn Arg Ser Arg Glu Tyr Gln Leu Asn Asp Ser Ala Lys
    140                 145                 150

tac tac ctg gac agc ctg gat cgg att gga gcc ggt gac tac cag ccc        533
Tyr Tyr Leu Asp Ser Leu Asp Arg Ile Gly Ala Gly Asp Tyr Gln Pro
155                 160                 165                 170

act gag cag gac atc ctc cga acc aga gtc aaa aca act ggc atc gta        581
Thr Glu Gln Asp Ile Leu Arg Thr Arg Val Lys Thr Thr Gly Ile Val
                175                 180                 185

gaa acc cac ttc acc ttc aag aac ctc cac ttc agg ctg ttt gac gtc        629
Glu Thr His Phe Thr Phe Lys Asn Leu His Phe Arg Leu Phe Asp Val
            190                 195                 200

ggg ggc cag cga tct gaa cgc aag aag tgg atc cac tgc ttt gag gat        677
Gly Gly Gln Arg Ser Glu Arg Lys Lys Trp Ile His Cys Phe Glu Asp
        205                 210                 215

gtc acg gcc atc atc ttc tgt gtc gca ctc agc ggc tat gac cag gtg        725
Val Thr Ala Ile Ile Phe Cys Val Ala Leu Ser Gly Tyr Asp Gln Val
    220                 225                 230

ctc cac gag gac gaa acc acg aac cgc atg cac gaa tcc ctg aag ctc        773
Leu His Glu Asp Glu Thr Thr Asn Arg Met His Glu Ser Leu Lys Leu
235                 240                 245                 250

ttc gac agc atc tgc aac aac aag tgg ttc aca gac aca tct att atc        821
Phe Asp Ser Ile Cys Asn Asn Lys Trp Phe Thr Asp Thr Ser Ile Ile
                255                 260                 265

ctg ttt ctc aac aag aag gac ata ttt gag gag aag atc aag aag tcc        869
Leu Phe Leu Asn Lys Lys Asp Ile Phe Glu Glu Lys Ile Lys Lys Ser
            270                 275                 280

cca ctc acc atc tgc ttt cct gaa tac aca ggc ccc agt gcc ttc aca        917
Pro Leu Thr Ile Cys Phe Pro Glu Tyr Thr Gly Pro Ser Ala Phe Thr
        285                 290                 295

gaa gct gtg gct cac atc caa ggg cag tat gag agt aag aat aag tca        965
Glu Ala Val Ala His Ile Gln Gly Gln Tyr Glu Ser Lys Asn Lys Ser
    300                 305                 310

gct cac aag gaa gtc tac agc cat gtc acc tgt gcc acg gac acc aac       1013
Ala His Lys Glu Val Tyr Ser His Val Thr Cys Ala Thr Asp Thr Asn
315                 320                 325                 330
```

aac atc caa ttc gtc ttt gat gcc gtg aca gat gtc atc atc gcc aaa 1061
Asn Ile Gln Phe Val Phe Asp Ala Val Thr Asp Val Ile Ile Ala Lys
335 340 345

aac cta cgg ggc tgt gga ctc tac tga gccctggcct cctacccagc 1108
Asn Leu Arg Gly Cys Gly Leu Tyr
350

ctgccactca ctcctcccct ggacccagag ctctgtcact gctcagatgc cctgttaact 1168

gaagaaaacc tggaggctag ccttgggggc aggaggaggc atcctttgag catccccacc 1228

ccacccaact tcagcctcgt gacacgtggg aacagggttg ggcagaggtg tggaacagca 1288

caaggccaga gaccacggca tgccacttgg gtgctgctca ctggtcagct gtgtgtctta 1348

cacagaggcc gagtgggcaa cactgccatc tgattcagaa tgggcatgcc ctgtcctctg 1408

tacctcttgt tcagtgtcct ggtttctctt ccaccttggt gataggatgg ctggcaggaa 1468

ggccccatgg aaggtgctgc ttgattaggg gatagtcgat ggcatctctc agcagtcctc 1528

agggtctgtt tggtagaggg tggtttcgtc gacaaaagcc aacatggaat caggccactt 1588

ttggggcgca aagactcaga ctttggggac gggttccctc ctccttcact ttggatcttg 1648

gcccctctct ggtcatcttc ccttgccctt gggctcccca ggatactcag ccctgactcc 1708

catggggttg ggaatattcc ttaagactgg ctgactgcaa aggtcaccga tggagaaaca 1768

tccctgtgct acagaattgg gggtgggaca gctgaggggg caggcggctc tttcctgata 1828

gttgatgaca agccctgaga atgccatctg ctggctccac tcacacgggc tcaactgtcc 1888

tgggtgatag tgacttgcca ggccacaggc tgcaggtcac agacagagca ggcaagcagc 1948

cttgcaactg cagattactt agggagaagc atcggggcct cgtgagccag gccccgtagc 2008

cagtgccctg ctttactcca gccttggtca ggaagtcgaa agcccttggt gtattcctgg 2068

tctcggagca aataatgagc cagcaccctg aagggtgggc tccaactcag acatgcagcc 2128

agccccctag gtgggtaaac gccctaggga cctagggaga gcctttgctg cagagattcc 2188

taagcaaaac ggcgtggtgg agctttggca accctagccc cagctaactt tggacagtca 2248

gcatatgtcc ctgccatccc tagacatctc cagtcagctg gtatcacagc cagtggttca 2308

gacaggtttg aatgctcatg tggca 2333

<210> 40
<211> 354
<212> PRT
<213> Mus musculus

<400> 40

Met Gly Cys Thr Leu Ser Ala Glu Glu Arg Ala Ala Leu Glu Arg Ser
1 5 10 15

Lys Ala Ile Glu Lys Asn Leu Lys Glu Asp Gly Ile Ser Ala Ala Lys
20 25 30

Asp Val Lys Leu Leu Leu Leu Gly Ala Gly Glu Ser Gly Lys Ser Thr
35 40 45

Ile Val Lys Gln Met Lys Ile Ile His Glu Asp Gly Phe Ser Gly Glu
50 55 60

Asp Val Lys Gln Tyr Lys Pro Val Val Tyr Ser Asn Thr Ile Gln Ser
65 70 75 80

Leu Ala Ala Ile Val Arg Ala Met Asp Thr Leu Gly Val Glu Tyr Gly
85 90 95

Asp Lys Glu Arg Lys Thr Asp Ser Lys Met Val Cys Asp Val Val Ser
100 105 110

Arg Met Glu Asp Thr Glu Pro Phe Ser Ala Glu Leu Leu Ser Ala Met
115 120 125

Met Arg Leu Trp Gly Asp Ser Gly Ile Gln Glu Cys Phe Asn Arg Ser
130 135 140

Arg Glu Tyr Gln Leu Asn Asp Ser Ala Lys Tyr Tyr Leu Asp Ser Leu
145 150 155 160

Asp Arg Ile Gly Ala Gly Asp Tyr Gln Pro Thr Glu Gln Asp Ile Leu
165 170 175

Arg Thr Arg Val Lys Thr Thr Gly Ile Val Glu Thr His Phe Thr Phe
180 185 190

Lys Asn Leu His Phe Arg Leu Phe Asp Val Gly Gly Gln Arg Ser Glu
195 200 205

Arg Lys Lys Trp Ile His Cys Phe Glu Asp Val Thr Ala Ile Ile Phe
210 215 220

Cys Val Ala Leu Ser Gly Tyr Asp Gln Val Leu His Glu Asp Glu Thr
225 230 235 240

Thr Asn Arg Met His Glu Ser Leu Lys Leu Phe Asp Ser Ile Cys Asn
245 250 255

Asn Lys Trp Phe Thr Asp Thr Ser Ile Ile Leu Phe Leu Asn Lys Lys
260 265 270

Asp Ile Phe Glu Glu Lys Ile Lys Lys Ser Pro Leu Thr Ile Cys Phe
275 280 285

Pro Glu Tyr Thr Gly Pro Ser Ala Phe Thr Glu Ala Val Ala His Ile
290 295 300

Gln Gly Gln Tyr Glu Ser Lys Asn Lys Ser Ala His Lys Glu Val Tyr
305 310 315 320

Ser His Val Thr Cys Ala Thr Asp Thr Asn Asn Ile Gln Phe Val Phe
325 330 335

Asp Ala Val Thr Asp Val Ile Ile Ala Lys Asn Leu Arg Gly Cys Gly
340 345 350

Leu Tyr

<210> 41
<211> 1135
<212> DNA
<213> Mus musculus

<300>
<308> M87286
<309> 1993-04-27
<313> (1)..(1135)

<220>
<221> CDS
<222> (41)..(1063)

<400> 41
gctcttcact tgagacgcct gagggaaacc accaggcagg atg agc gag ctg gag 55
Met Ser Glu Leu Glu
1 5

cag ctg agg cag gag gct gaa cag ctt cgg aat cag atc cag gat gct 103
Gln Leu Arg Gln Glu Ala Glu Gln Leu Arg Asn Gln Ile Gln Asp Ala
10 15 20

cgg aag gcc tgc aac gat gcc acg ctg gtt cag atc acg tct aat atg 151
Arg Lys Ala Cys Asn Asp Ala Thr Leu Val Gln Ile Thr Ser Asn Met
25 30 35

gac tcc gtg ggc cga ata caa atg cga aca agg cgc acg ctg cgt ggc 199
Asp Ser Val Gly Arg Ile Gln Met Arg Thr Arg Arg Thr Leu Arg Gly
40 45 50

cac ctc gct aag atc tac gcc atg cac tgg gga tat gat tcc agg cta 247
His Leu Ala Lys Ile Tyr Ala Met His Trp Gly Tyr Asp Ser Arg Leu
55 60 65

cta gtc agt gct tcg caa gat gga aaa tta att att tgg gat agc tat 295
Leu Val Ser Ala Ser Gln Asp Gly Lys Leu Ile Ile Trp Asp Ser Tyr
70 75 80 85

acg aca aat aag atg cac gcc atc cct ctg agg tcc tcc tgg gtg atg 343
Thr Thr Asn Lys Met His Ala Ile Pro Leu Arg Ser Ser Trp Val Met
90 95 100

acc tgt gcc tac gcc ccg tcc ggg aac tac gtt gcc tgt gga ggc ttg 391
Thr Cys Ala Tyr Ala Pro Ser Gly Asn Tyr Val Ala Cys Gly Gly Leu
105 110 115

gat aac atc tgc tcc ata tac aac cta aag acc cga gag gga gat gtg 439
Asp Asn Ile Cys Ser Ile Tyr Asn Leu Lys Thr Arg Glu Gly Asp Val
120 125 130

cgg gtg agc cga gaa ttg gca gga cac acg ggc tac ttg tcc tgc tgc 487
Arg Val Ser Arg Glu Leu Ala Gly His Thr Gly Tyr Leu Ser Cys Cys

```
    135                 140                 145

cga ttc tta gat gat gga caa atc att aca agt tcg gga gac acg act      535
Arg Phe Leu Asp Asp Gly Gln Ile Ile Thr Ser Ser Gly Asp Thr Thr
150                 155                 160                 165

tgt gct ttg tgg gac att gag acc gga cag cag act acg acc ttc aca      583
Cys Ala Leu Trp Asp Ile Glu Thr Gly Gln Gln Thr Thr Thr Phe Thr
                170                 175                 180

gga cac tcg ggt gac gtg atg agc ctc tca ctg agt cct gac ttg aag      631
Gly His Ser Gly Asp Val Met Ser Leu Ser Leu Ser Pro Asp Leu Lys
            185                 190                 195

acg ttt gtg tct ggt gct tgt gat gca tcc tca aag ctg tgg gat atc      679
Thr Phe Val Ser Gly Ala Cys Asp Ala Ser Ser Lys Leu Trp Asp Ile
        200                 205                 210

cga gat ggg atg tgt aga cag tct ttc acc gga cac atc tca gac atc      727
Arg Asp Gly Met Cys Arg Gln Ser Phe Thr Gly His Ile Ser Asp Ile
    215                 220                 225

aac gct gtc agt ttc ttc ccg agt gga tat gcc ttt gcc act ggt tct      775
Asn Ala Val Ser Phe Phe Pro Ser Gly Tyr Ala Phe Ala Thr Gly Ser
230                 235                 240                 245

gat gat gcc aca tgc cga ctc ttt gac ctc cgt gca gac cag gag ctc      823
Asp Asp Ala Thr Cys Arg Leu Phe Asp Leu Arg Ala Asp Gln Glu Leu
                250                 255                 260

ctg cta tac tct cat gac aat atc atc tgt ggc att act tct gtg gcc      871
Leu Leu Tyr Ser His Asp Asn Ile Ile Cys Gly Ile Thr Ser Val Ala
            265                 270                 275

ttc tca aag agt ggg cgc ctc ctg tta gcc ggc tat gac gac ttc aac      919
Phe Ser Lys Ser Gly Arg Leu Leu Leu Ala Gly Tyr Asp Asp Phe Asn
        280                 285                 290

tgc agt gtg tgg gac gct ctg aaa gga ggc cgg tca ggt gtc ctt gct      967
Cys Ser Val Trp Asp Ala Leu Lys Gly Gly Arg Ser Gly Val Leu Ala
    295                 300                 305

ggt cat gac aac cgt gtt agc tgc tta ggt gtg act gat gac ggc atg     1015
Gly His Asp Asn Arg Val Ser Cys Leu Gly Val Thr Asp Asp Gly Met
310                 315                 320                 325

gct gtg gcc act ggc tcc tgg gac agt ttt ctt aga atc tgg aat tga     1063
Ala Val Ala Thr Gly Ser Trp Asp Ser Phe Leu Arg Ile Trp Asn
                330                 335                 340

gtgccatatt ttctgttctc caatgatacc tggagaaatc cgtgttacag cctatagctg   1123

tgaggaaaaa aa                                                       1135
```

<210> 42
<211> 340
<212> PRT
<213> Mus musculus

<400> 42

```
Met Ser Glu Leu Glu Gln Leu Arg Gln Glu Ala Glu Gln Leu Arg Asn
1               5                   10                  15

Gln Ile Gln Asp Ala Arg Lys Ala Cys Asn Asp Ala Thr Leu Val Gln
            20                  25                  30
```

```
Ile Thr Ser Asn Met Asp Ser Val Gly Arg Ile Gln Met Arg Thr Arg
        35                  40                      45

Arg Thr Leu Arg Gly His Leu Ala Lys Ile Tyr Ala Met His Trp Gly
    50                  55                  60

Tyr Asp Ser Arg Leu Leu Val Ser Ala Ser Gln Asp Gly Lys Leu Ile
65                  70                  75                  80

Ile Trp Asp Ser Tyr Thr Thr Asn Lys Met His Ala Ile Pro Leu Arg
                85                  90                  95

Ser Ser Trp Val Met Thr Cys Ala Tyr Ala Pro Ser Gly Asn Tyr Val
            100                 105                 110

Ala Cys Gly Gly Leu Asp Asn Ile Cys Ser Ile Tyr Asn Leu Lys Thr
        115                 120                 125

Arg Glu Gly Asp Val Arg Val Ser Arg Glu Leu Ala Gly His Thr Gly
    130                 135                 140

Tyr Leu Ser Cys Cys Arg Phe Leu Asp Asp Gly Gln Ile Ile Thr Ser
145                 150                 155                 160

Ser Gly Asp Thr Thr Cys Ala Leu Trp Asp Ile Glu Thr Gly Gln Gln
                165                 170                 175

Thr Thr Thr Phe Thr Gly His Ser Gly Asp Val Met Ser Leu Ser Leu
            180                 185                 190

Ser Pro Asp Leu Lys Thr Phe Val Ser Gly Ala Cys Asp Ala Ser Ser
        195                 200                 205

Lys Leu Trp Asp Ile Arg Asp Gly Met Cys Arg Gln Ser Phe Thr Gly
    210                 215                 220

His Ile Ser Asp Ile Asn Ala Val Ser Phe Phe Pro Ser Gly Tyr Ala
225                 230                 235                 240

Phe Ala Thr Gly Ser Asp Asp Ala Thr Cys Arg Leu Phe Asp Leu Arg
                245                 250                 255

Ala Asp Gln Glu Leu Leu Leu Tyr Ser His Asp Asn Ile Ile Cys Gly
            260                 265                 270

Ile Thr Ser Val Ala Phe Ser Lys Ser Gly Arg Leu Leu Leu Ala Gly
        275                 280                 285

Tyr Asp Asp Phe Asn Cys Ser Val Trp Asp Ala Leu Lys Gly Gly Arg
    290                 295                 300
```

Ser Gly Val Leu Ala Gly His Asp Asn Arg Val Ser Cys Leu Gly Val
305 310 315 320

Thr Asp Asp Gly Met Ala Val Ala Thr Gly Ser Trp Asp Ser Phe Leu
325 330 335

Arg Ile Trp Asn
340

<210> 43
<211> 307
<212> DNA
<213> Mus musculus

<300>
<308> U37527
<309> 1997-12-30
<313> (1)..(307)

<220>
<221> CDS
<222> (40)..(267)

<400> 43
tccaagctgc tgtaccacct ctcagcaggg agtgcagga atg aag gaa ggc atg 54
Met Lys Glu Gly Met
1 5

tct aat aac agc acc acc agc atc tcc cag gcc agg aaa gcc gtg gag 102
Ser Asn Asn Ser Thr Thr Ser Ile Ser Gln Ala Arg Lys Ala Val Glu
10 15 20

cag ctg aag atg gaa gcc tgc atg gac agg gtg aag gtc tcc cag gct 150
Gln Leu Lys Met Glu Ala Cys Met Asp Arg Val Lys Val Ser Gln Ala
25 30 35

gcc tca gac ctc ctg gcc tac tgt gaa gcc cac gtg cgg gag gac ccc 198
Ala Ser Asp Leu Leu Ala Tyr Cys Glu Ala His Val Arg Glu Asp Pro
40 45 50

ctc atc atc cca gtg cct gcc tca gaa aac ccc ttc cgg gag aag aag 246
Leu Ile Ile Pro Val Pro Ala Ser Glu Asn Pro Phe Arg Glu Lys Lys
55 60 65

ttc ttc tgc acc atc ctc taa cacccatggc gatgaagcgg gccctttcct 297
Phe Phe Cys Thr Ile Leu
70 75

gctgtaacag 307

<210> 44
<211> 75
<212> PRT
<213> Mus musculus

<400> 44

Met Lys Glu Gly Met Ser Asn Asn Ser Thr Thr Ser Ile Ser Gln Ala
1 5 10 15

```
Arg Lys Ala Val Glu Gln Leu Lys Met Glu Ala Cys Met Asp Arg Val
            20                  25                  30

Lys Val Ser Gln Ala Ala Ser Asp Leu Leu Ala Tyr Cys Glu Ala His
        35                  40                  45

Val Arg Glu Asp Pro Leu Ile Ile Pro Val Pro Ala Ser Glu Asn Pro
    50                  55                  60

Phe Arg Glu Lys Lys Phe Phe Cys Thr Ile Leu
65                  70                  75


<210>  45
<211>  2666
<212>  DNA
<213>  Mus musculus

<300>
<308>  BC023729
<309>  2003-04-16
<313>  (1)..(2666)


<220>
<221>  CDS
<222>  (252)..(2219)

<400>  45
ccacgcgtcc ggccccagcg caacgcgcag cagcctccct cctcttcttc ccgcactgtg      60

cgctcctcct gggctagggc gtctggatcg agtcccggag gctaccgcct cccagacaga     120

cgacaggtca cctggacgcg agcctgtgtc cgggtctcgt cgttgccggc gcagtcactg     180

ggcacaaccg tgggactccg tctgtctcgg attaatcccg gagagccaga gccaacctct     240

cccggtcaga g atg cga ccc tca ggg acc gcg aga acc aca ctg ctg gtg      290
             Met Arg Pro Ser Gly Thr Ala Arg Thr Thr Leu Leu Val
             1               5                   10

ttg ctg acc gcg ctc tgc gcc gca ggt ggg gcg ttg gag gaa aag aaa       338
Leu Leu Thr Ala Leu Cys Ala Ala Gly Gly Ala Leu Glu Glu Lys Lys
    15                  20                  25

gtc tgc caa ggc aca agt aac agg ctc acc caa ctg ggc act ttt gaa       386
Val Cys Gln Gly Thr Ser Asn Arg Leu Thr Gln Leu Gly Thr Phe Glu
30                  35                  40                  45

gac cac ttt ctg agc ctg cag agg atg tac aac aac tgt gaa gtg gtc       434
Asp His Phe Leu Ser Leu Gln Arg Met Tyr Asn Asn Cys Glu Val Val
                50                  55                  60

ctt ggg aac ttg gaa att acc tat gtg caa agg aat tac gac ctt tcc       482
Leu Gly Asn Leu Glu Ile Thr Tyr Val Gln Arg Asn Tyr Asp Leu Ser
            65                  70                  75

ttc tta aag acc atc cag gag gtg gcc ggc tat gtc ctc att gcc ctc       530
Phe Leu Lys Thr Ile Gln Glu Val Ala Gly Tyr Val Leu Ile Ala Leu
        80                  85                  90

aac acc gtg gag aga atc cct ttg gag aac ctg cag atc atc agg gga       578
Asn Thr Val Glu Arg Ile Pro Leu Glu Asn Leu Gln Ile Ile Arg Gly
    95                  100                 105
```

```
aat gct ctt tat gaa aac acc tat gcc tta gcc atc ctg tcc aac tat        626
Asn Ala Leu Tyr Glu Asn Thr Tyr Ala Leu Ala Ile Leu Ser Asn Tyr
110                 115                 120                 125

ggg aca aac aga act ggg ctt agg gaa ctg ccc atg cgg aac tta cag        674
Gly Thr Asn Arg Thr Gly Leu Arg Glu Leu Pro Met Arg Asn Leu Gln
                130                 135                 140

gaa atc ctg att ggt gct gtg cga ttc agc aac aac ccc atc ctc tgc        722
Glu Ile Leu Ile Gly Ala Val Arg Phe Ser Asn Asn Pro Ile Leu Cys
            145                 150                 155

aat atg gat act atc cag tgg agg gac atc gtc caa aac gtc ttt atg        770
Asn Met Asp Thr Ile Gln Trp Arg Asp Ile Val Gln Asn Val Phe Met
        160                 165                 170

agc aac atg tca atg gac tta cag agc cat ccg agc agt tgc ccc aaa        818
Ser Asn Met Ser Met Asp Leu Gln Ser His Pro Ser Ser Cys Pro Lys
    175                 180                 185

tgt gat cca agc tgt ccc aat gga agc tgc tgg gga gga gga gag gag        866
Cys Asp Pro Ser Cys Pro Asn Gly Ser Cys Trp Gly Gly Gly Glu Glu
190                 195                 200                 205

aac tgc cag aaa ttg acc aaa atc atc tgt gcc cag caa tgt tcc cat        914
Asn Cys Gln Lys Leu Thr Lys Ile Ile Cys Ala Gln Gln Cys Ser His
                210                 215                 220

cgc tgt cgt ggc agg tcc ccc agt gac tgc tgc cac aac caa tgt gct        962
Arg Cys Arg Gly Arg Ser Pro Ser Asp Cys Cys His Asn Gln Cys Ala
            225                 230                 235

gcg ggg tgt aca ggg ccc cga gag agt gac tgt ctg gtc tgc caa aag       1010
Ala Gly Cys Thr Gly Pro Arg Glu Ser Asp Cys Leu Val Cys Gln Lys
        240                 245                 250

ttc caa gat gag gcc aca tgc aaa gac acc tgc cca cca ctc atg ctg       1058
Phe Gln Asp Glu Ala Thr Cys Lys Asp Thr Cys Pro Pro Leu Met Leu
    255                 260                 265

tac aac ccc acc acc tat cag atg gat gtc aac cct gaa ggg aag tac       1106
Tyr Asn Pro Thr Thr Tyr Gln Met Asp Val Asn Pro Glu Gly Lys Tyr
270                 275                 280                 285

agc ttt ggt gcc acc tgt gtg aag aag tgc ccc cga aac tac gtg gtg       1154
Ser Phe Gly Ala Thr Cys Val Lys Lys Cys Pro Arg Asn Tyr Val Val
                290                 295                 300

aca gat cat ggc tca tgt gtc cga gcc tgt ggg cct gac tac tac gaa       1202
Thr Asp His Gly Ser Cys Val Arg Ala Cys Gly Pro Asp Tyr Tyr Glu
            305                 310                 315

gtg gaa gaa gat ggc atc cgc aag tgt aaa aaa tgt gat ggg ccc tgt       1250
Val Glu Glu Asp Gly Ile Arg Lys Cys Lys Lys Cys Asp Gly Pro Cys
        320                 325                 330

cgc aaa gtt tgt aat ggc ata ggc att ggt gaa ttt aaa gac aca ctc       1298
Arg Lys Val Cys Asn Gly Ile Gly Ile Gly Glu Phe Lys Asp Thr Leu
    335                 340                 345

tcc ata aat gct aca aac atc aaa cac ttc aaa tac tgc act gcc atc       1346
Ser Ile Asn Ala Thr Asn Ile Lys His Phe Lys Tyr Cys Thr Ala Ile
350                 355                 360                 365

agc ggg gac ctt cac atc ctg cca gtg gcc ttt aag ggg gat tct ttc       1394
Ser Gly Asp Leu His Ile Leu Pro Val Ala Phe Lys Gly Asp Ser Phe
                370                 375                 380
```

```
acg cgc act cct cct cta gac cca cga gaa cta gaa att cta aaa acc      1442
Thr Arg Thr Pro Pro Leu Asp Pro Arg Glu Leu Glu Ile Leu Lys Thr
            385                 390                 395

gta aag gaa ata aca ggc ttt ttg ctg att cag gct tgg cct gat aac      1490
Val Lys Glu Ile Thr Gly Phe Leu Leu Ile Gln Ala Trp Pro Asp Asn
        400                 405                 410

tgg act gac ctc cat gct ttc gag aac cta gaa ata ata cgt ggc aga      1538
Trp Thr Asp Leu His Ala Phe Glu Asn Leu Glu Ile Ile Arg Gly Arg
    415                 420                 425

aca aag caa cat ggt cag ttt tct ttg gcg gtc gtt ggc ctg aac atc      1586
Thr Lys Gln His Gly Gln Phe Ser Leu Ala Val Val Gly Leu Asn Ile
430                 435                 440                     445

aca tca ctg ggg ctg cgt tcc ctc aag gag atc agt gat ggg gat gtg      1634
Thr Ser Leu Gly Leu Arg Ser Leu Lys Glu Ile Ser Asp Gly Asp Val
                450                 455                 460

atc att tct gga aac cga aat ttg tgc tac gca aac aca ata aac tgg      1682
Ile Ile Ser Gly Asn Arg Asn Leu Cys Tyr Ala Asn Thr Ile Asn Trp
            465                 470                 475

aaa aaa ctc ttc ggg aca ccc aat cag aaa acc aaa atc atg aac aac      1730
Lys Lys Leu Phe Gly Thr Pro Asn Gln Lys Thr Lys Ile Met Asn Asn
        480                 485                 490

aga gct gag aaa gac tgc aag gcc gtg aac cac gtc tgc aat cct tta      1778
Arg Ala Glu Lys Asp Cys Lys Ala Val Asn His Val Cys Asn Pro Leu
    495                 500                 505

tgc tcc tcg gaa ggc tgc tgg ggc cct gag ccc agg gac tgt gtc tcc      1826

Cys Ser Ser Glu Gly Cys Trp Gly Pro Glu Pro Arg Asp Cys Val Ser
510                 515                 520                     525

tgc cag aat gtg agc aga ggc agg gag tgc gtg gag aaa tgc aac atc      1874
Cys Gln Asn Val Ser Arg Gly Arg Glu Cys Val Glu Lys Cys Asn Ile
                530                 535                 540

ctg gag ggg gaa cca agg gag ttt gtg gaa aat tct gaa tgc atc cag      1922
Leu Glu Gly Glu Pro Arg Glu Phe Val Glu Asn Ser Glu Cys Ile Gln
            545                 550                 555

tgc cat cca gaa tgt ctg ccc cag gcc atg aac atc acc tgt aca ggc      1970
Cys His Pro Glu Cys Leu Pro Gln Ala Met Asn Ile Thr Cys Thr Gly
        560                 565                 570

agg gga cca gac aac tgc atc cag tgt gcc cac tac att gat ggc cca      2018
Arg Gly Pro Asp Asn Cys Ile Gln Cys Ala His Tyr Ile Asp Gly Pro
    575                 580                 585

cac tgt gtc aag acc tgc cca gct ggc atc atg gga gag aac aac act      2066
His Cys Val Lys Thr Cys Pro Ala Gly Ile Met Gly Glu Asn Asn Thr
590                 595                 600                     605

ctg gtc tgg aag tat gca gat gcc aat aat gtc tgc cac cta tgc cac      2114
Leu Val Trp Lys Tyr Ala Asp Ala Asn Asn Val Cys His Leu Cys His
                610                 615                 620

gcc aac tgt acc tat gga tgt gct ggg cca ggt ctt caa gga tgt gaa      2162
Ala Asn Cys Thr Tyr Gly Cys Ala Gly Pro Gly Leu Gln Gly Cys Glu
            625                 630                 635

gtg tgg cca tct ggg tac gtt caa tgg cag tgg atc tta aag acc ttt      2210
Val Trp Pro Ser Gly Tyr Val Gln Trp Gln Trp Ile Leu Lys Thr Phe
        640                 645                 650
```

```
tgg atc taa gaccagaagc catctctgac tcccctctca ccttccagtt           2259
Trp Ile
    655

tcttccaaat cctctgggcc agccagaggt ctcagattct gccctcttgc cctgtgccca 2319

ccttgttgac cactggacag catatgtgat ggctactgct agtgccagct tcacaagagg 2379

ttaacactac ggactagcca ttcttcctat gtatctgttt ctgcaaatac agccgcttta 2439

cttaagtctc agcacttctt agtctcctct tttcctctca gtagcccaag gggtcatgtc 2499

acaaacatgg tgtgaagggc tactttgtca aatgaaaagg tctatcttgg ggggcatttt 2559

tttcttttct ttttttcttg aaacacattg cccagcaaag ccaataaatt tctctcatca 2619

ttttgtttct gataaattct tactattgat aaaaaaaaaa aaaaaaa               2666


<210>  46
<211>  655
<212>  PRT
<213>  Mus musculus

<400>  46

Met Arg Pro Ser Gly Thr Ala Arg Thr Thr Leu Leu Val Leu Leu Thr
1               5                   10                  15


Ala Leu Cys Ala Ala Gly Gly Ala Leu Glu Glu Lys Lys Val Cys Gln
            20                  25                  30


Gly Thr Ser Asn Arg Leu Thr Gln Leu Gly Thr Phe Glu Asp His Phe
        35                  40                  45


Leu Ser Leu Gln Arg Met Tyr Asn Asn Cys Glu Val Val Leu Gly Asn
    50                  55                  60


Leu Glu Ile Thr Tyr Val Gln Arg Asn Tyr Asp Leu Ser Phe Leu Lys
65                  70                  75                  80


Thr Ile Gln Glu Val Ala Gly Tyr Val Leu Ile Ala Leu Asn Thr Val
                85                  90                  95


Glu Arg Ile Pro Leu Glu Asn Leu Gln Ile Ile Arg Gly Asn Ala Leu
            100                 105                 110


Tyr Glu Asn Thr Tyr Ala Leu Ala Ile Leu Ser Asn Tyr Gly Thr Asn
        115                 120                 125


Arg Thr Gly Leu Arg Glu Leu Pro Met Arg Asn Leu Gln Glu Ile Leu
    130                 135                 140


Ile Gly Ala Val Arg Phe Ser Asn Asn Pro Ile Leu Cys Asn Met Asp
145                 150                 155                 160


Thr Ile Gln Trp Arg Asp Ile Val Gln Asn Val Phe Met Ser Asn Met
```

165 170 175

Ser Met Asp Leu Gln Ser His Pro Ser Ser Cys Pro Lys Cys Asp Pro
180 185 190

Ser Cys Pro Asn Gly Ser Cys Trp Gly Gly Gly Glu Glu Asn Cys Gln
195 200 205

Lys Leu Thr Lys Ile Ile Cys Ala Gln Gln Cys Ser His Arg Cys Arg
210 215 220

Gly Arg Ser Pro Ser Asp Cys Cys His Asn Gln Cys Ala Ala Gly Cys
225 230 235 240

Thr Gly Pro Arg Glu Ser Asp Cys Leu Val Cys Gln Lys Phe Gln Asp
245 250 255

Glu Ala Thr Cys Lys Asp Thr Cys Pro Pro Leu Met Leu Tyr Asn Pro
260 265 270

Thr Thr Tyr Gln Met Asp Val Asn Pro Glu Gly Lys Tyr Ser Phe Gly
275 280 285

Ala Thr Cys Val Lys Lys Cys Pro Arg Asn Tyr Val Val Thr Asp His
290 295 300

Gly Ser Cys Val Arg Ala Cys Gly Pro Asp Tyr Tyr Glu Val Glu Glu
305 310 315 320

Asp Gly Ile Arg Lys Cys Lys Lys Cys Asp Gly Pro Cys Arg Lys Val
325 330 335

Cys Asn Gly Ile Gly Ile Gly Glu Phe Lys Asp Thr Leu Ser Ile Asn
340 345 350

Ala Thr Asn Ile Lys His Phe Lys Tyr Cys Thr Ala Ile Ser Gly Asp
355 360 365

Leu His Ile Leu Pro Val Ala Phe Lys Gly Asp Ser Phe Thr Arg Thr
370 375 380

Pro Pro Leu Asp Pro Arg Glu Leu Glu Ile Leu Lys Thr Val Lys Glu
385 390 395 400

Ile Thr Gly Phe Leu Leu Ile Gln Ala Trp Pro Asp Asn Trp Thr Asp
405 410 415

Leu His Ala Phe Glu Asn Leu Glu Ile Ile Arg Gly Arg Thr Lys Gln
420 425 430

His Gly Gln Phe Ser Leu Ala Val Val Gly Leu Asn Ile Thr Ser Leu

435 440 445

Gly Leu Arg Ser Leu Lys Glu Ile Ser Asp Gly Asp Val Ile Ile Ser
450 455 460

Gly Asn Arg Asn Leu Cys Tyr Ala Asn Thr Ile Asn Trp Lys Lys Leu
465 470 475 480

Phe Gly Thr Pro Asn Gln Lys Thr Lys Ile Met Asn Asn Arg Ala Glu
485 490 495

Lys Asp Cys Lys Ala Val Asn His Val Cys Asn Pro Leu Cys Ser Ser
500 505 510

Glu Gly Cys Trp Gly Pro Glu Pro Arg Asp Cys Val Ser Cys Gln Asn
515 520 525

Val Ser Arg Gly Arg Glu Cys Val Glu Lys Cys Asn Ile Leu Glu Gly
530 535 540

Glu Pro Arg Glu Phe Val Glu Asn Ser Glu Cys Ile Gln Cys His Pro
545 550 555 560

Glu Cys Leu Pro Gln Ala Met Asn Ile Thr Cys Thr Gly Arg Gly Pro
565 570 575

Asp Asn Cys Ile Gln Cys Ala His Tyr Ile Asp Gly Pro His Cys Val
580 585 590

Lys Thr Cys Pro Ala Gly Ile Met Gly Glu Asn Asn Thr Leu Val Trp
595 600 605

Lys Tyr Ala Asp Ala Asn Asn Val Cys His Leu Cys His Ala Asn Cys
610 615 620

Thr Tyr Gly Cys Ala Gly Pro Gly Leu Gln Gly Cys Glu Val Trp Pro
625 630 635 640

Ser Gly Tyr Val Gln Trp Gln Trp Ile Leu Lys Thr Phe Trp Ile
645 650 655

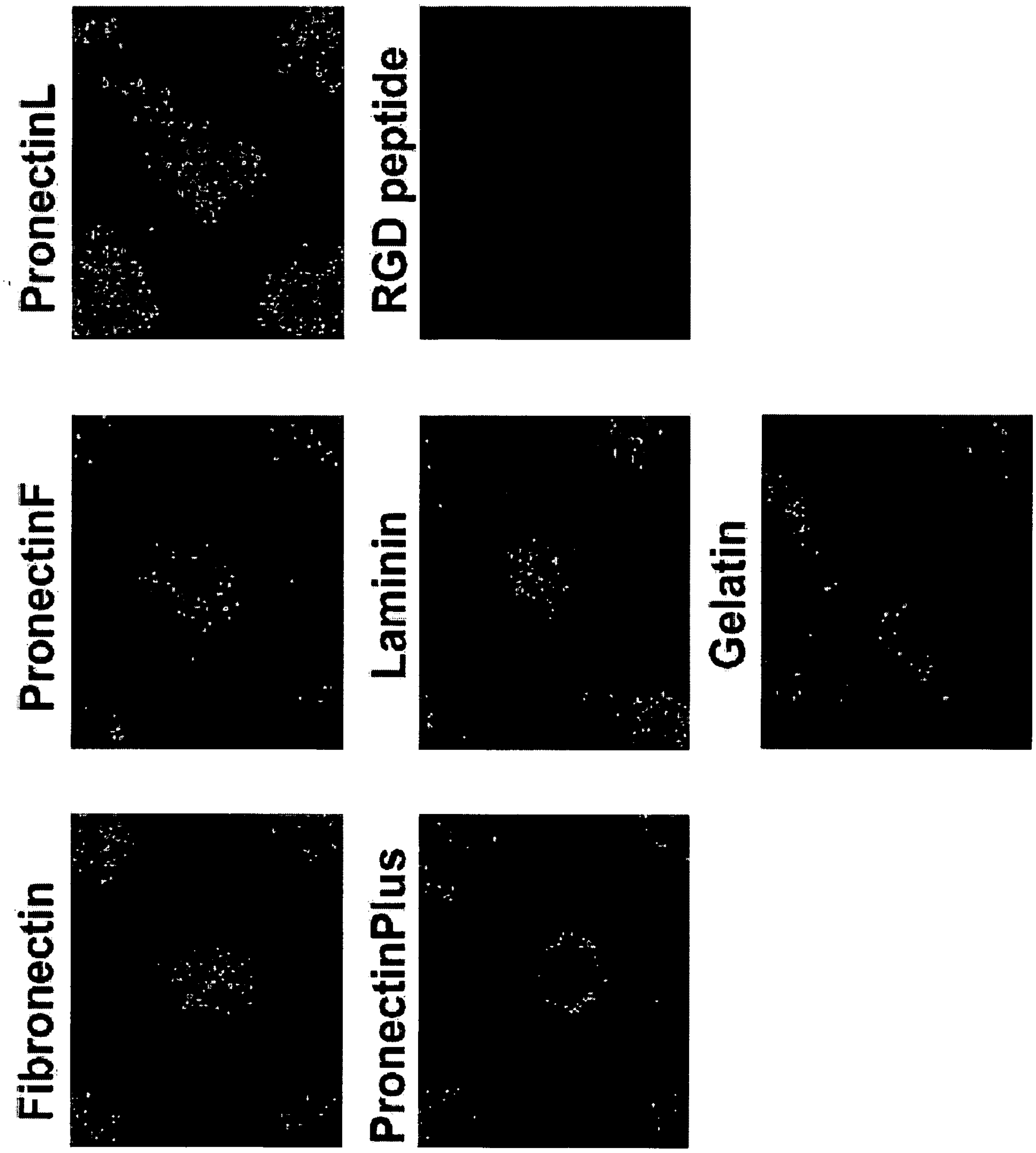
PronectinL
RGD peptide
PronectinF
Laminin
Gelatin
Fibronectin
PronectinPlus

FIG.1

FIG. 2

Fibronectin (43kDafragment)

Fibronectin (72kDafragment)

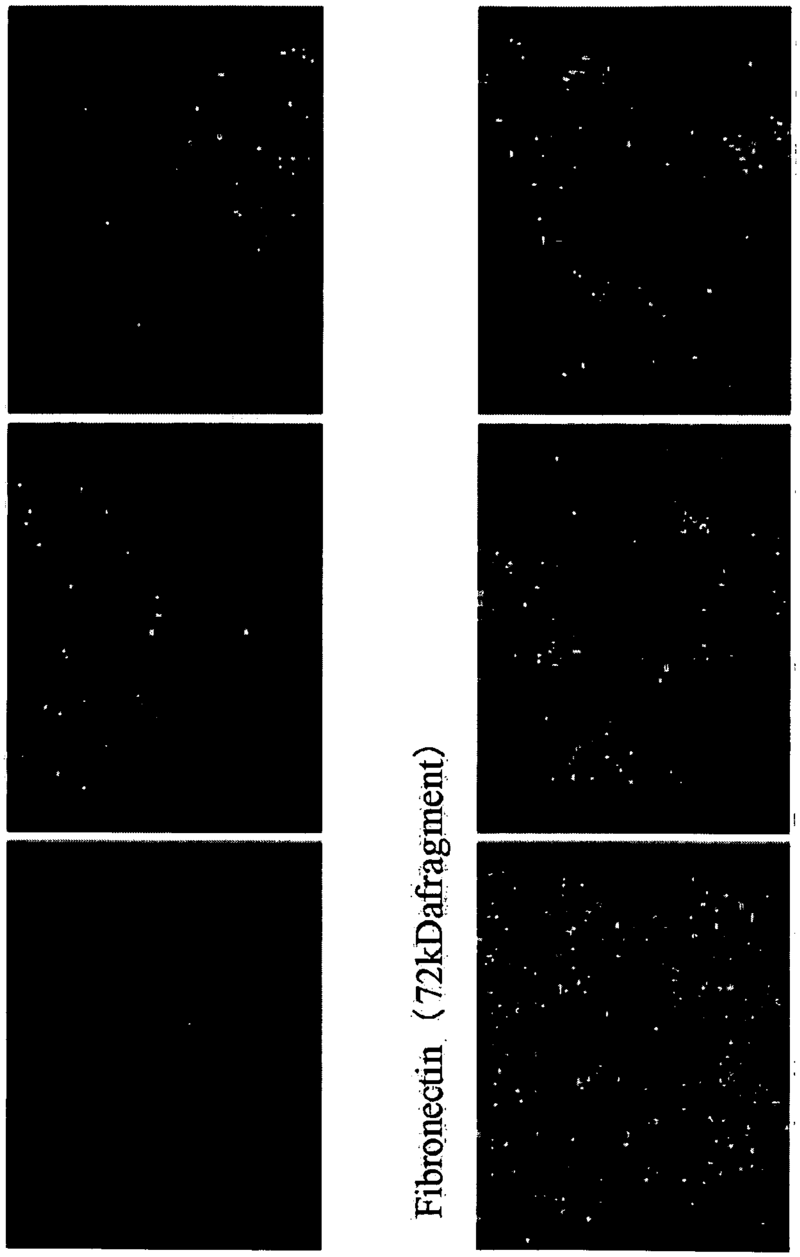

FIG. 3

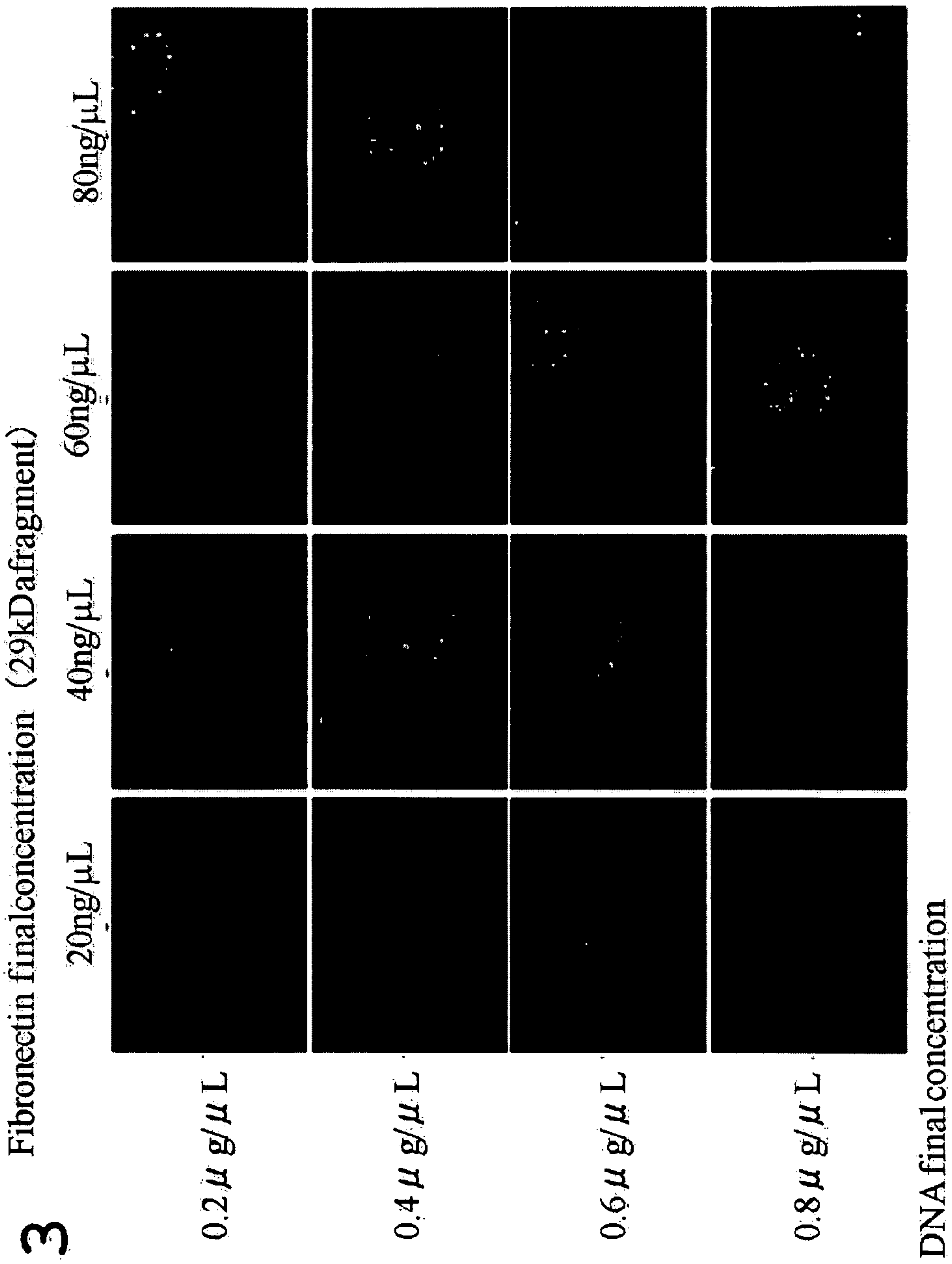
Fibronectin finalconcentration (29kDafragment)
20ng/µL
40ng/µL
60ng/µL
80ng/µL
0.2 µ g/ µ L
0.4 µ g/ µ L
0.6 µ g/ µ L
0.8 µ g/ µ L
DNA final concentration

FIG. 4

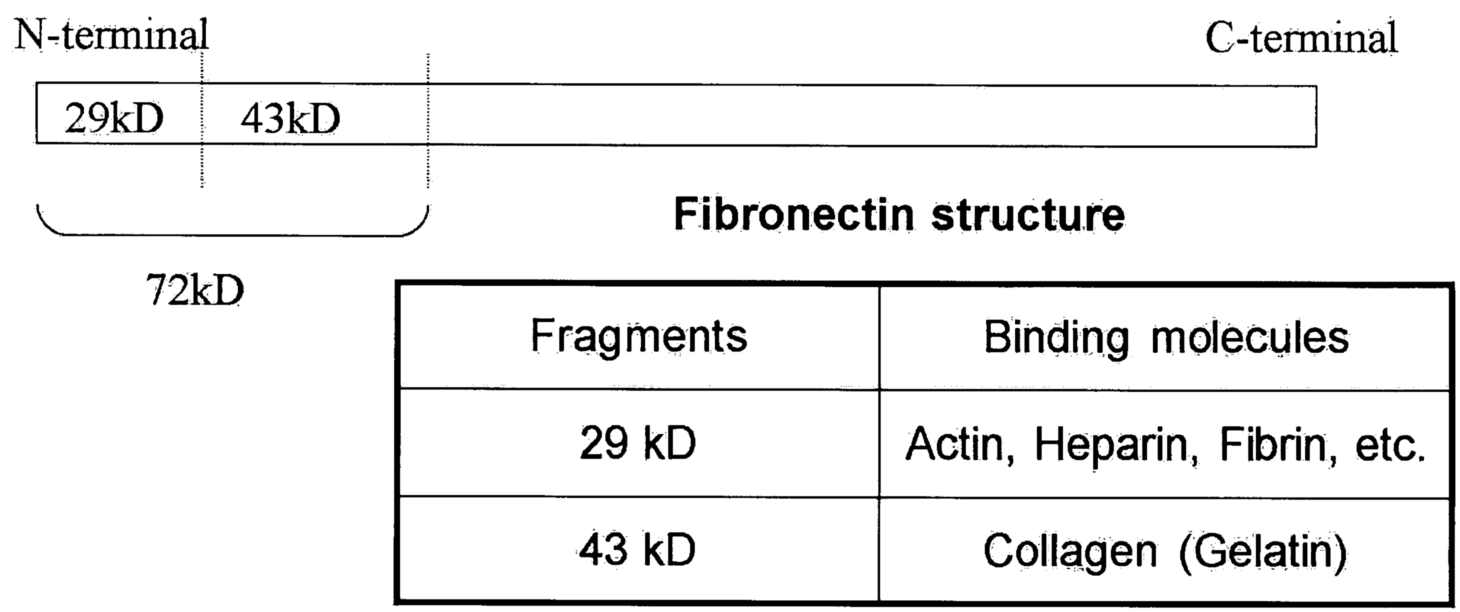


**Fibronectin structure**

| Fragments | Binding molecules |
| --- | --- |
| 29 kD | Actin, Heparin, Fibrin, etc. |
| 43 kD | Collagen (Gelatin) |

| | 29 kD | 43 kD | 72 kD |
| --- | --- | --- | --- |
| TF efficiency | ◎ | ○ | ◎ |
| Cross-contamination | **none** | **some** | **some** |

FIG. 5

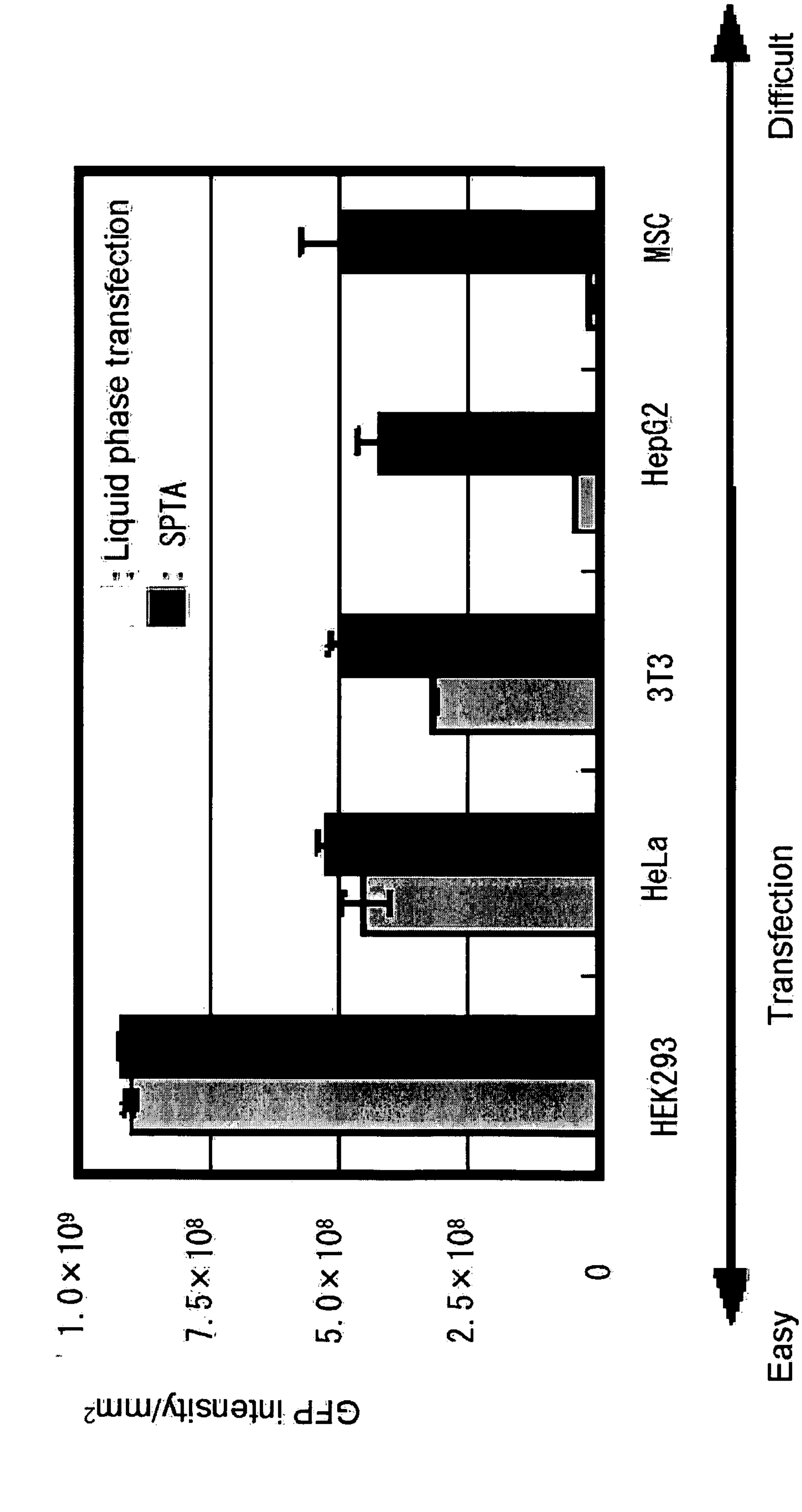

GFP intensity/mm²
1.0×10⁹
7.5×10⁸
5.0×10⁸
2.5×10⁸
0
Liquid phase transfection
SPTA
HEK293
HeLa
3T3
HepG2
MSC
Easy
Transfection
Difficult

FIG. 6

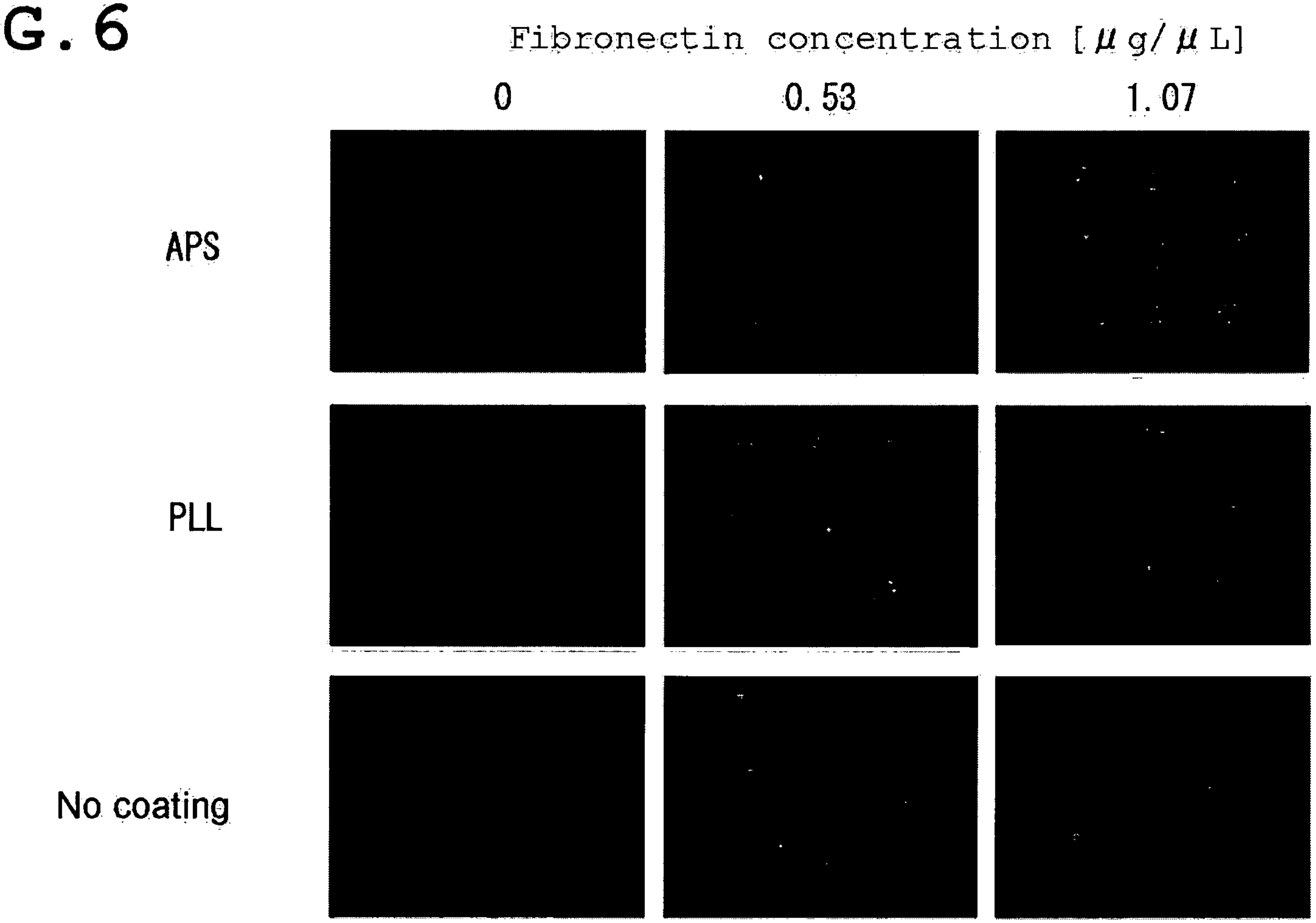
Fibronectin concentration [μg/μL]
0
0.53
1.07
APS
PLL
No coating

Fig. 7

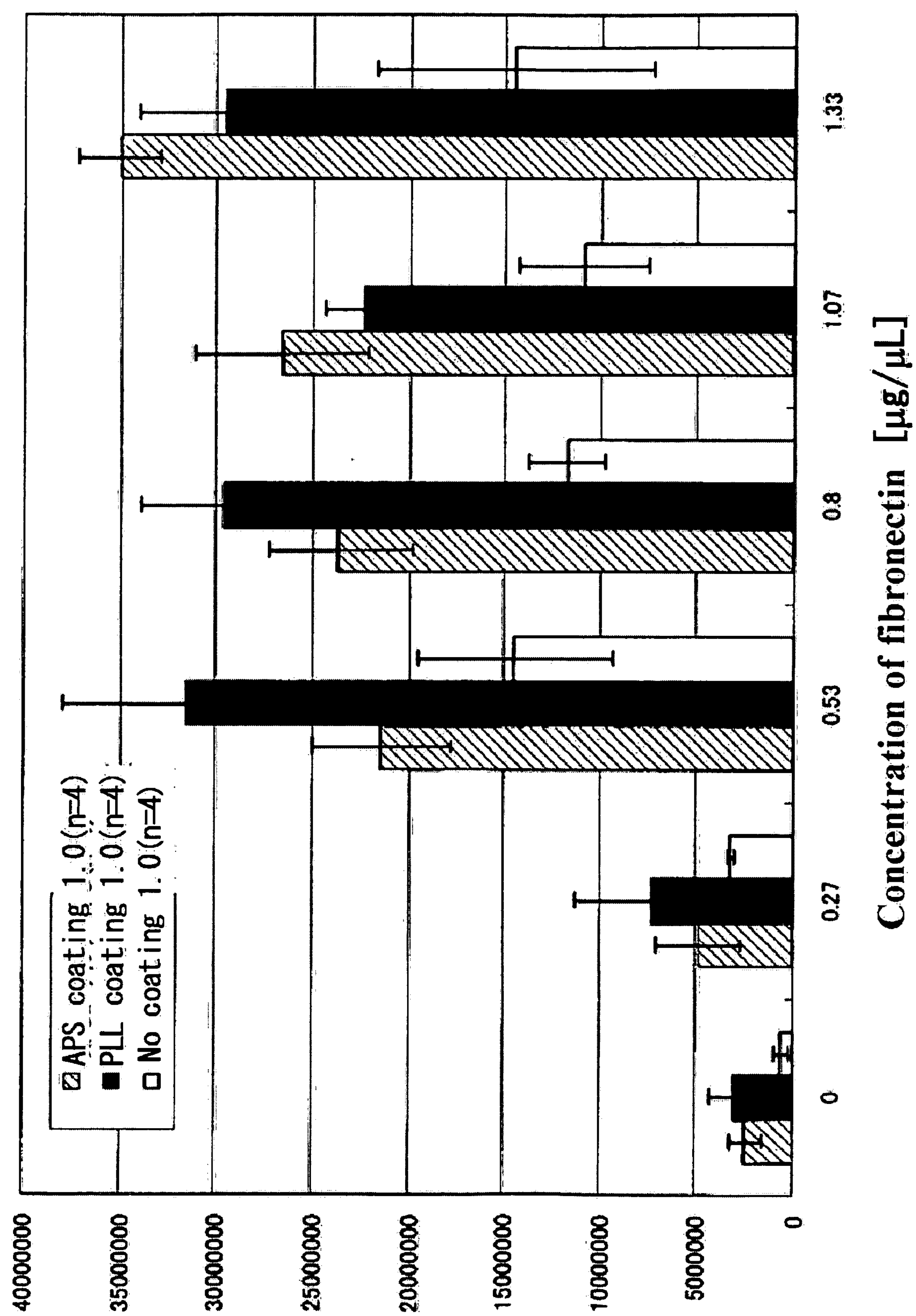
40000000
35000000
30000000
25000000
20000000
15000000
10000000
5000000
0
APS coating 1.0(n=4)
PLL coating 1.0(n=4)
No coating 1.0(n=4)
0
0.27
0.53
0.8
1.07
1.33
Concentration of fibronectin [μg/μL]

FIG. 8

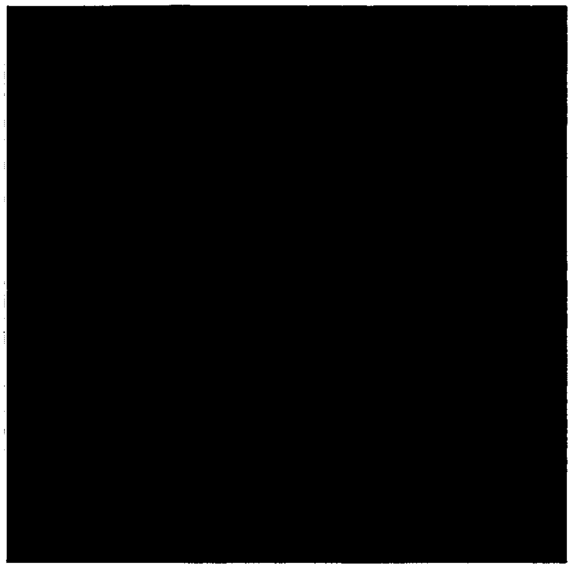

Fibronectin(-)

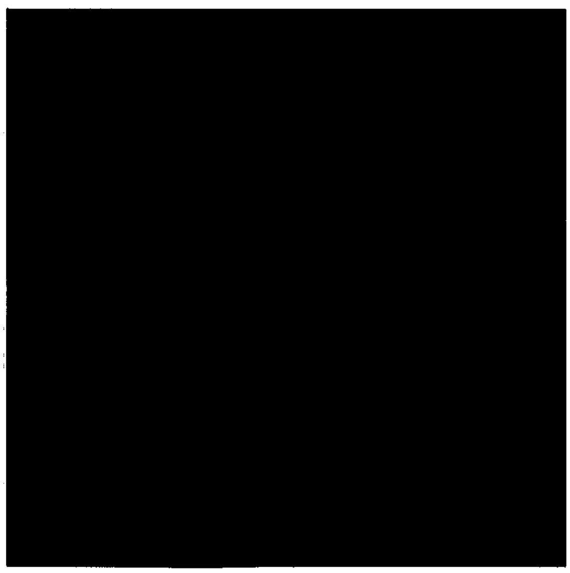

Fibronectin(+)

FIG. 9

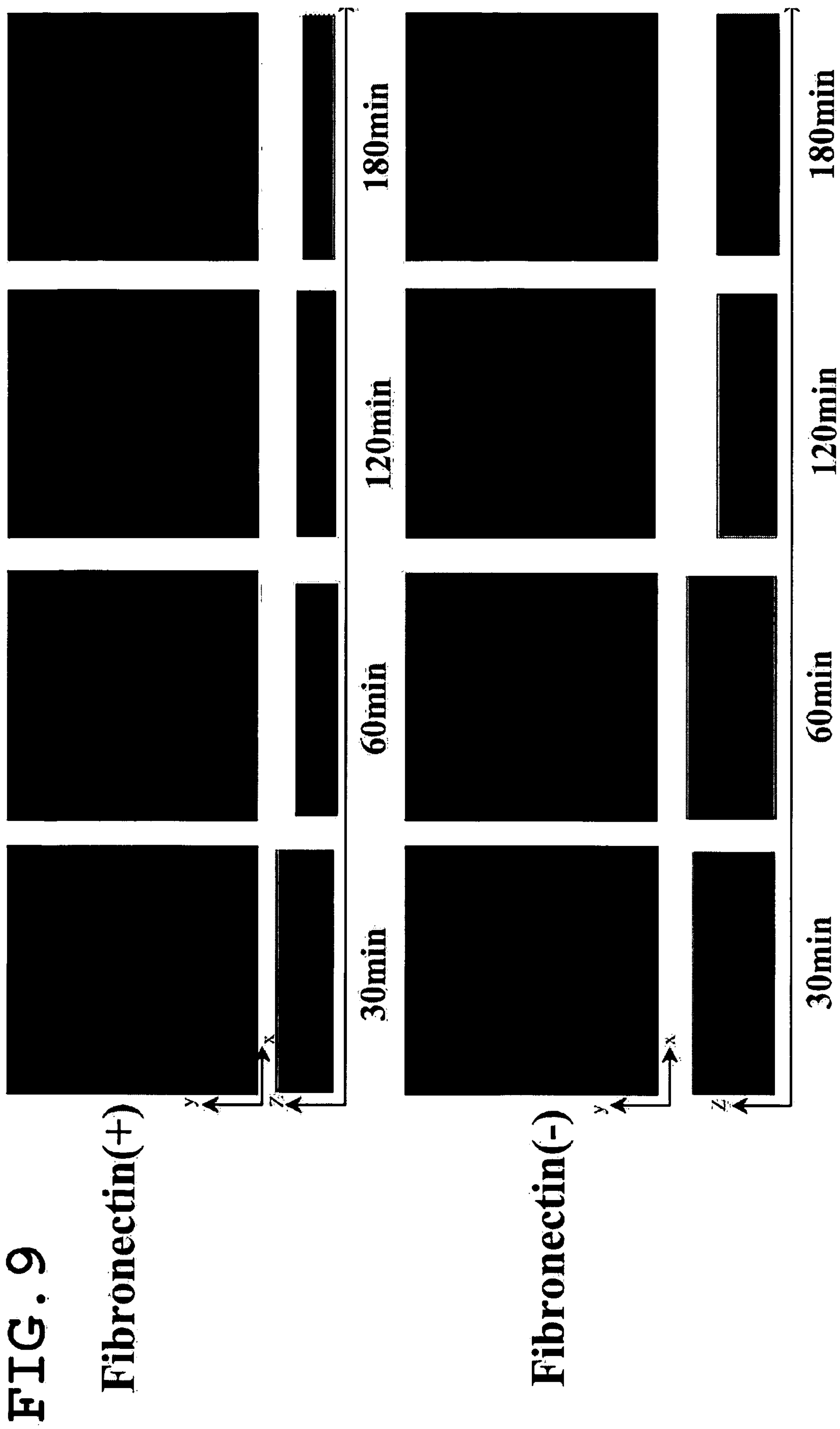

FIG.10

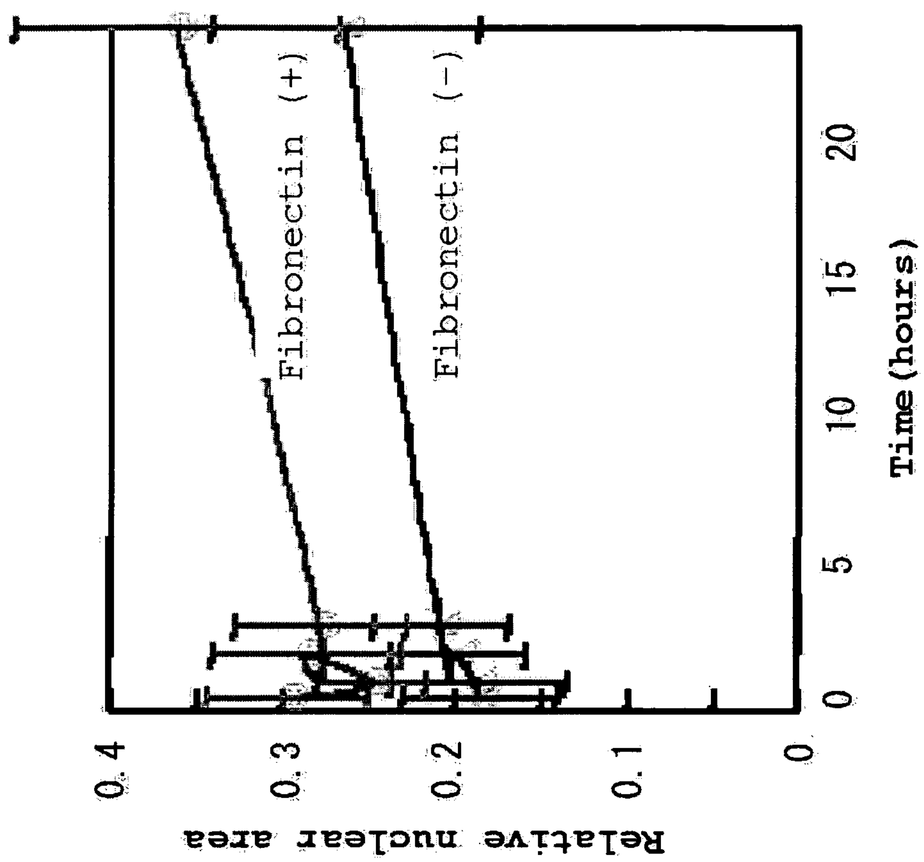
Relative nuclear area
0.4
0.3
0.2
0.1
0
Fibronectin (+)
Fibronectin (−)
0
5
10
15
20
Time (hours)

FIG.11

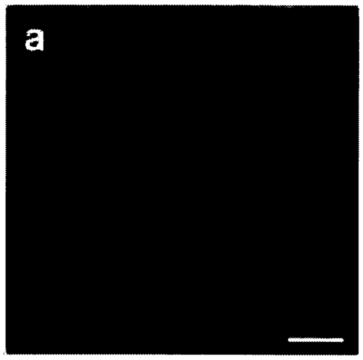


FIG.12

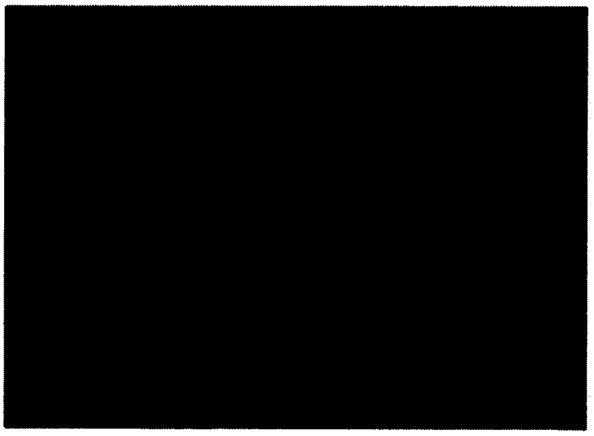

FIG. 13

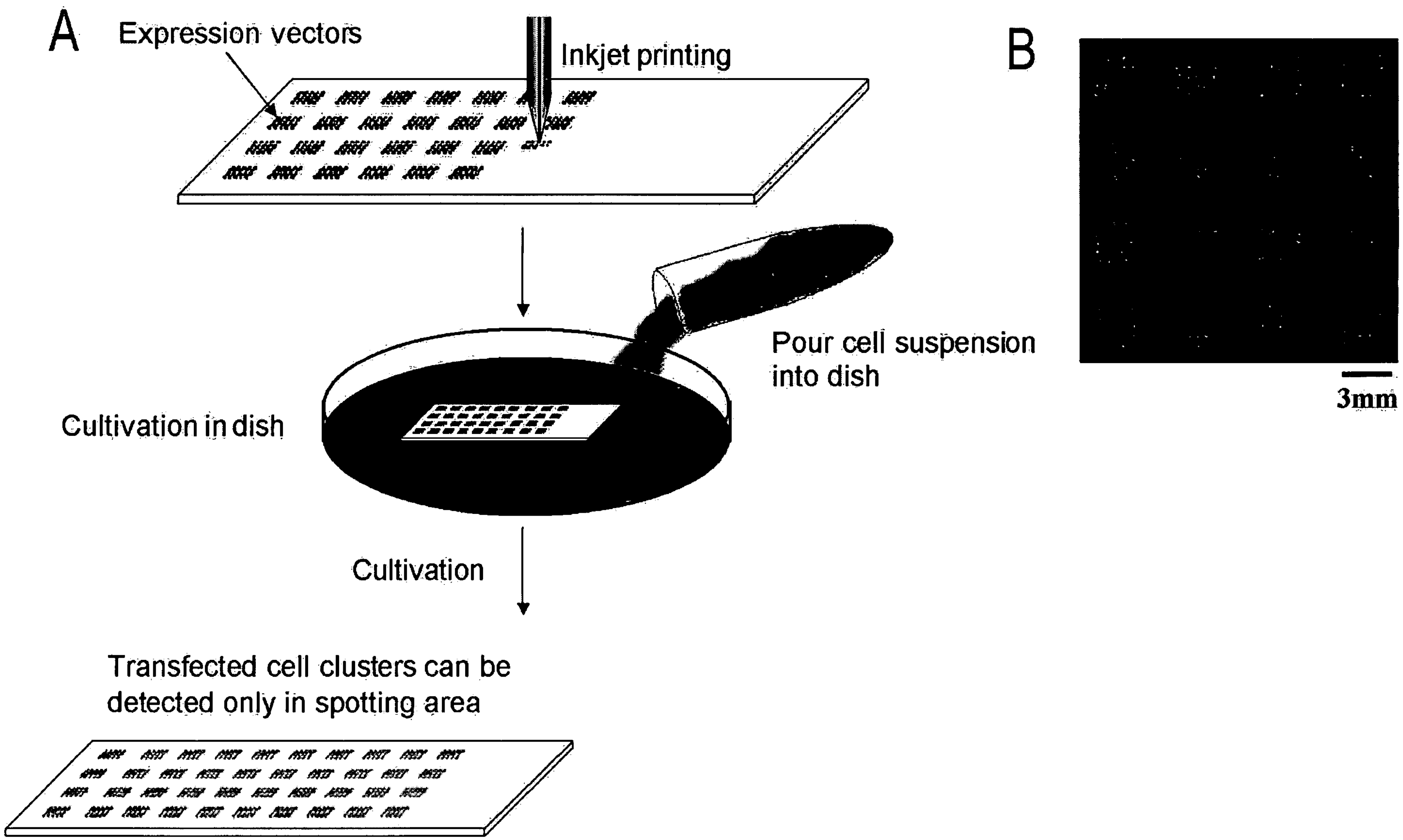
A
Expression vectors
Inkjet printing
Pour cell suspension into dish
Cultivation in dish
Cultivation
Transfected cell clusters can be detected only in spotting area
B
3mm

FIG.13C

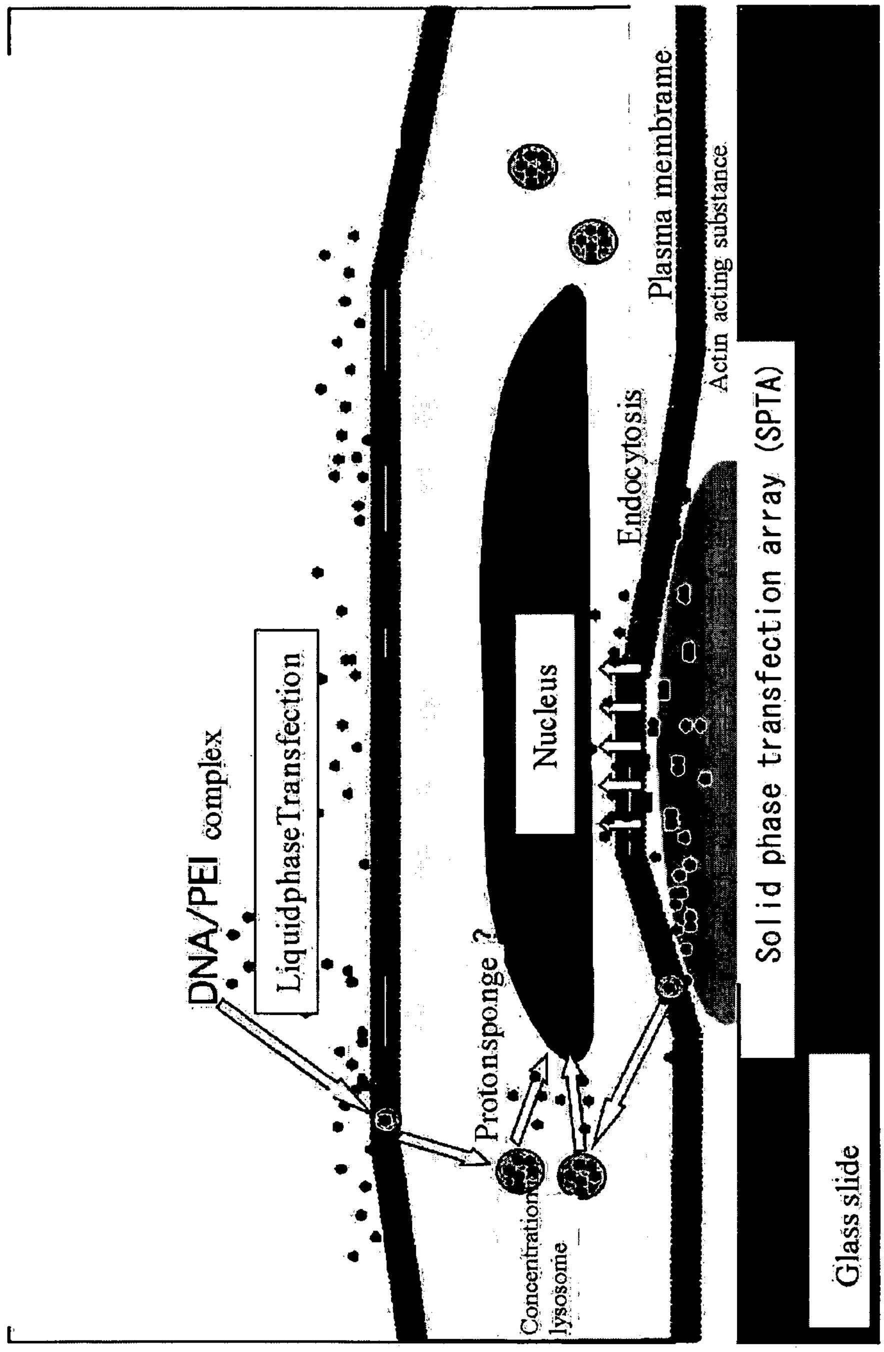
DNA/PEI complex
LiquidphaseTransfection
Nucleus
Protonsponge ?
Concentration lysosome
Endocytosis
Plasma membrane
Actin acting substance
Solid phase transfection array (SPTA)
Glass slide

FIG. 14

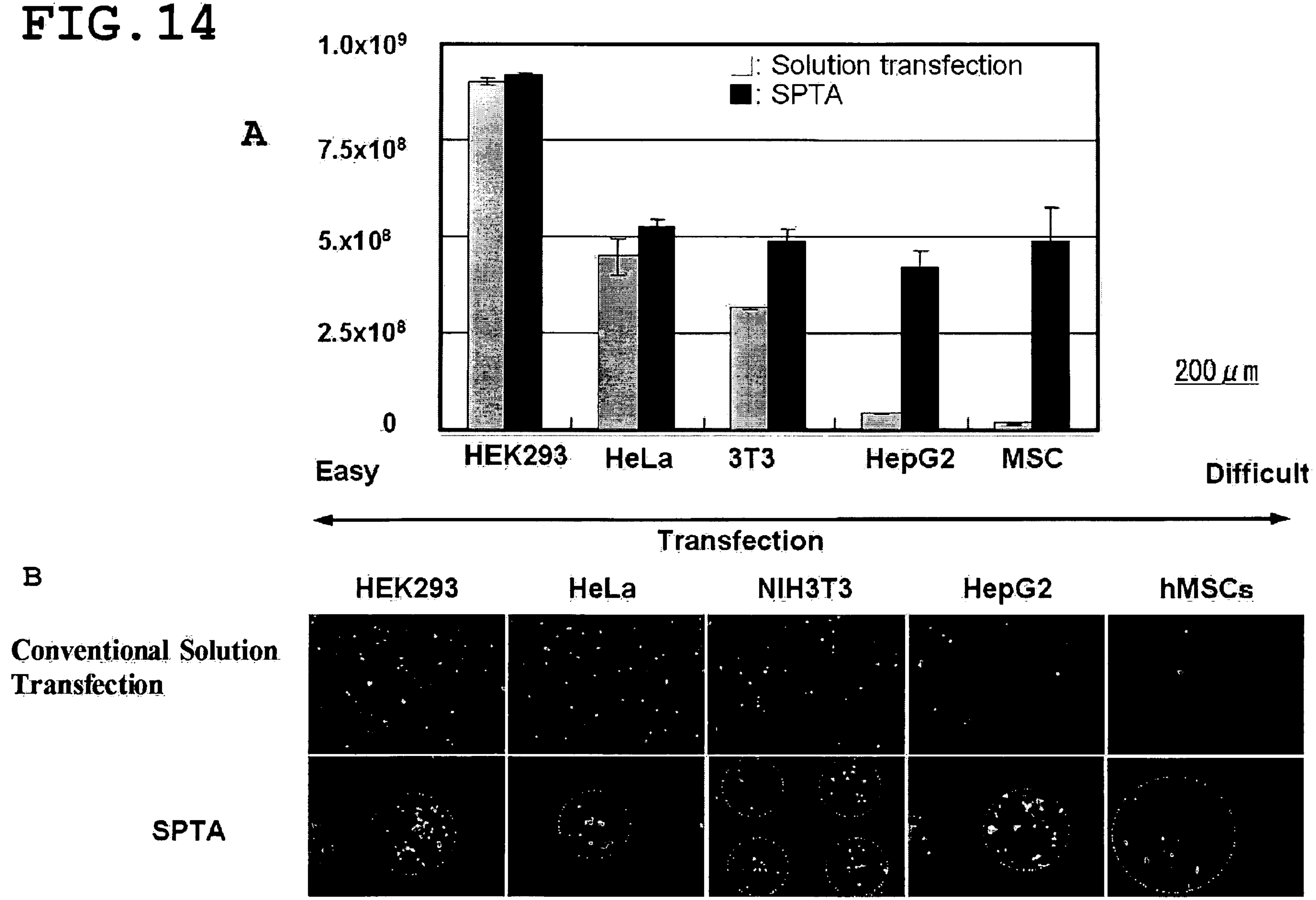
A
1.0x10⁹
7.5x10⁸
5x10⁸
2.5x10⁸
0
: Solution transfection
: SPTA
HEK293
HeLa
3T3
HepG2
MSC
Easy
Difficult
200 μm
Transfection
B
HEK293
HeLa
NIH3T3
HepG2
hMSCs
Conventional Solution Transfection
SPTA

FIG. 14C

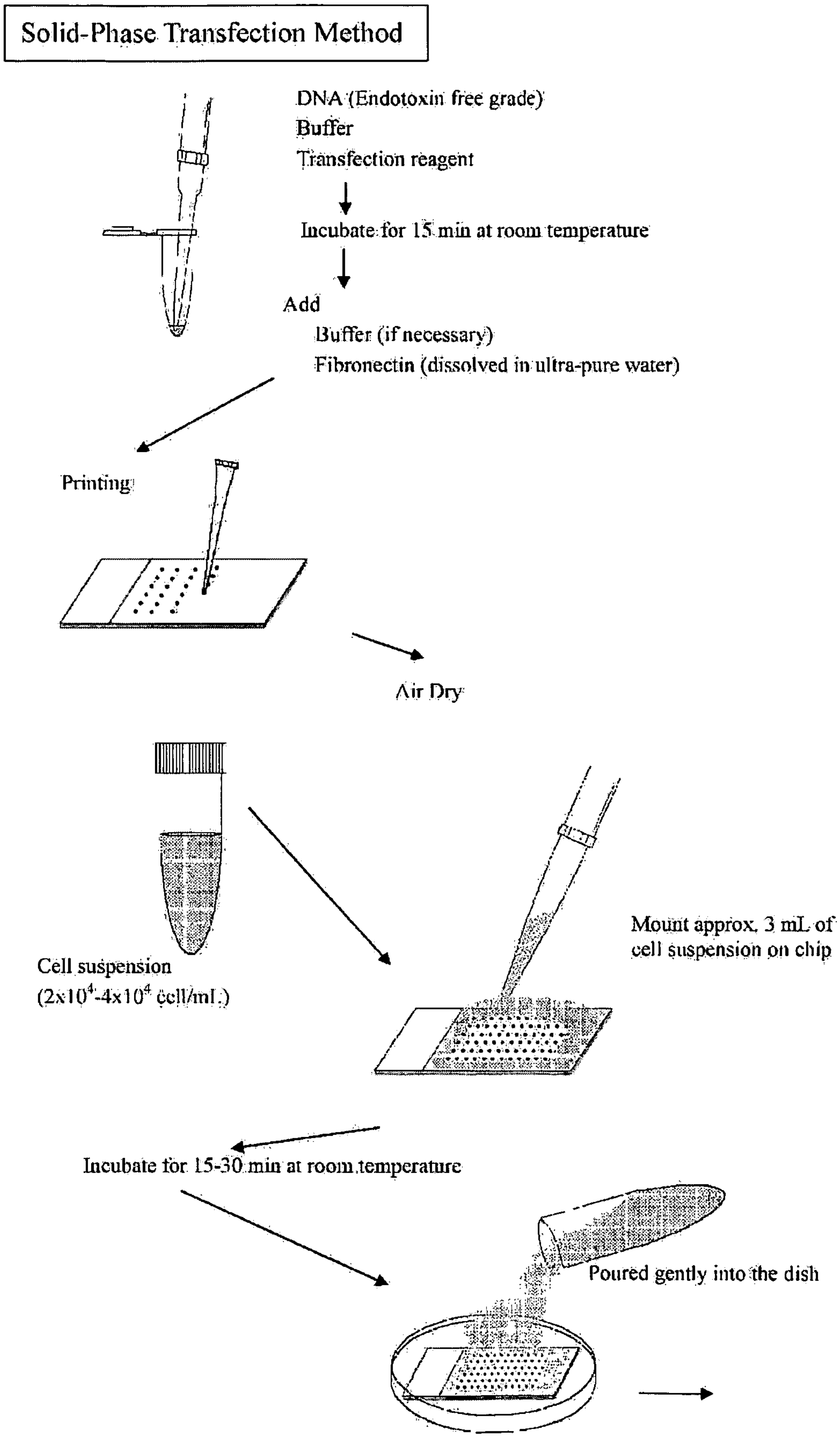
Solid-Phase Transfection Method
DNA (Endotoxin free grade)
Buffer
Transfection reagent
Incubate for 15 min at room temperature
Add
Buffer (if necessary)
Fibronectin (dissolved in ultra-pure water)
Printing
Air Dry
Cell suspension
(2x10^4-4x10^4 cell/mL)
Mount approx. 3 mL of
cell suspension on chip
Incubate for 15-30 min at room temperature
Poured gently into the dish

# FIG. 14D

| For HEK293 | | |
|---|---|---|
| DMEM (serum free) | 9.5 | uL |
| Plasmid DNA (1mg/mL) | 1.5 | uL |
| TransFast (1mg/mL) | 9.0 | uL |
| DMEM (serum free) | 5.0 | uL |
| Fibronectin (4mg/mL) | 5.0 | uL |
| Final volume | 30.0 | uL |

Scheme for HEK293

1.5mL micro-tube

↓ ←DMEM

↓ ←Plasmid DNA

mix

↓ ←TransFast

Incubate for 2-3 days at 37℃ in 5% $CO_2$

mix completely and incubate for 15 min at RT

↓ ←DMEM

↓ ←Fibronectin

mix completely

↓

ready to print

| For HeLa, NIH3T3-3, HepG2 | | |
|---|---|---|
| DMEM (serum free) | 14.5 | uL |
| Plasmid DNA (1mg/mL) | 1.5 | uL |
| Lipofectamine2000 | 4.5 | uL |
| DMEM (serum free) | 5.0 | uL |
| Fibronectin (4mg/mL) | 5.0 | uL |
| Final volume | 30.0 | uL |

Scheme for HeLa, NIH3T3-3, and HepG2

1.5mL micro-tube

↓ ←DMEM

↓ ←Plasmid DNA

mix

↓ ←Lipofectamine2000

mix completely and incubate for 15 min at RT

↓ ←DMEM

↓ ←Fibronectin

mix completely

↓

ready to print

For hMSCs

| | N/P=5 | N/P=10 | N/P=20 | |
|---|---|---|---|---|
| DMEM (serum free) | 12.75 | 12.0 | 10.5 | uL |
| Plasmid DNA (1mg/mL) | 1.5 | 1.5 | 1.5 | uL |
| JetPEI (x4) conc. | 0.75 | 1.5 | 3.0 | uL |
| Fibronectin (4mg/mL) | 5.0 | 5.0 | 5.0 | uL |
| Final volume | 20.0 | 20.0 | 20.0 | uL |

Scheme for hMSCs

1.5mL micro-tube

↓ ←DMEM

↓ ←Plasmid DNA

mix

↓ ←jetPEI

mix completely and incubate for 15 min at RT

↓ ←Fibronectin

mix completely

↓

ready to print

FIG. 15

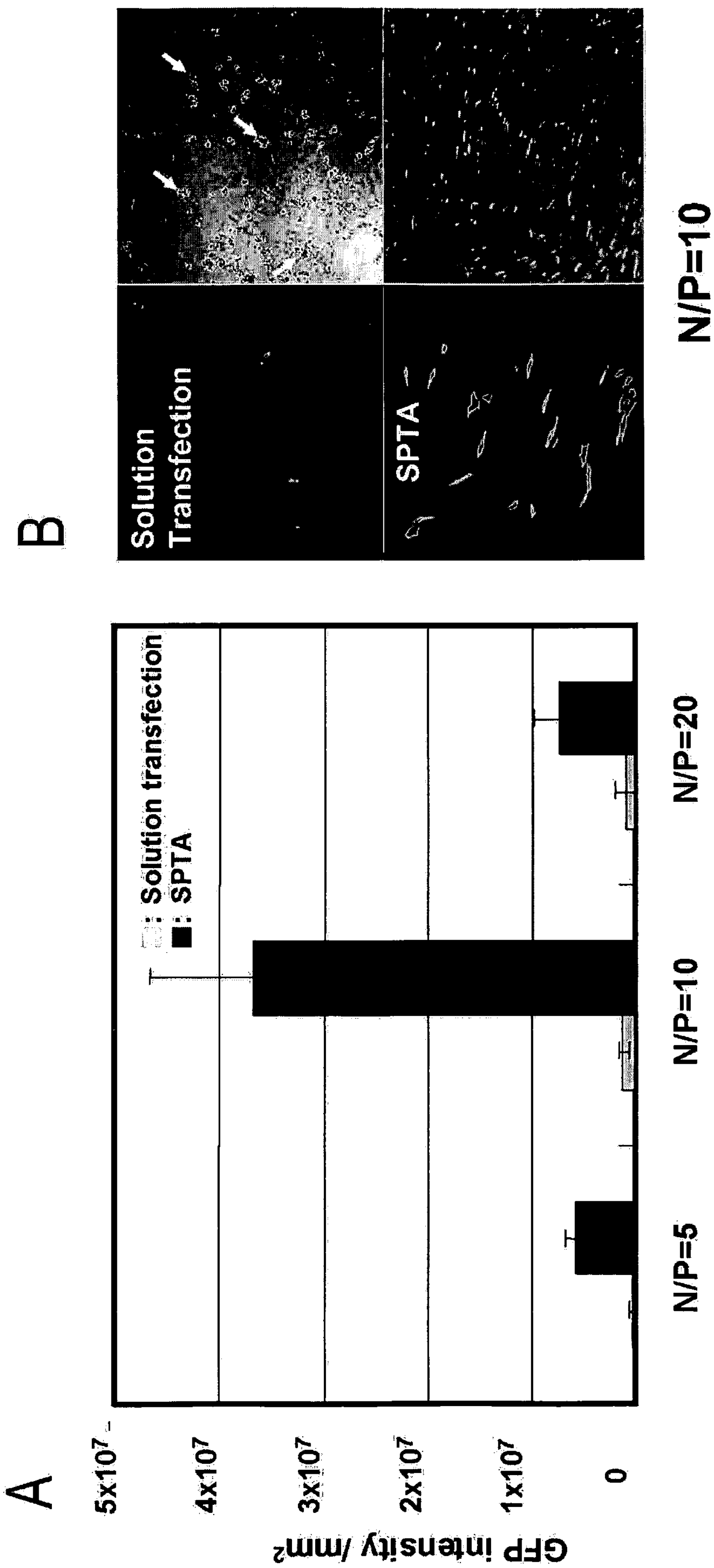
A
B
GFP intensity /mm²
5x10⁷
4x10⁷
3x10⁷
2x10⁷
1x10⁷
0
: Solution transfection
: SPTA
N/P=5
N/P=10
N/P=20
Solution Transfection
SPTA
N/P=10

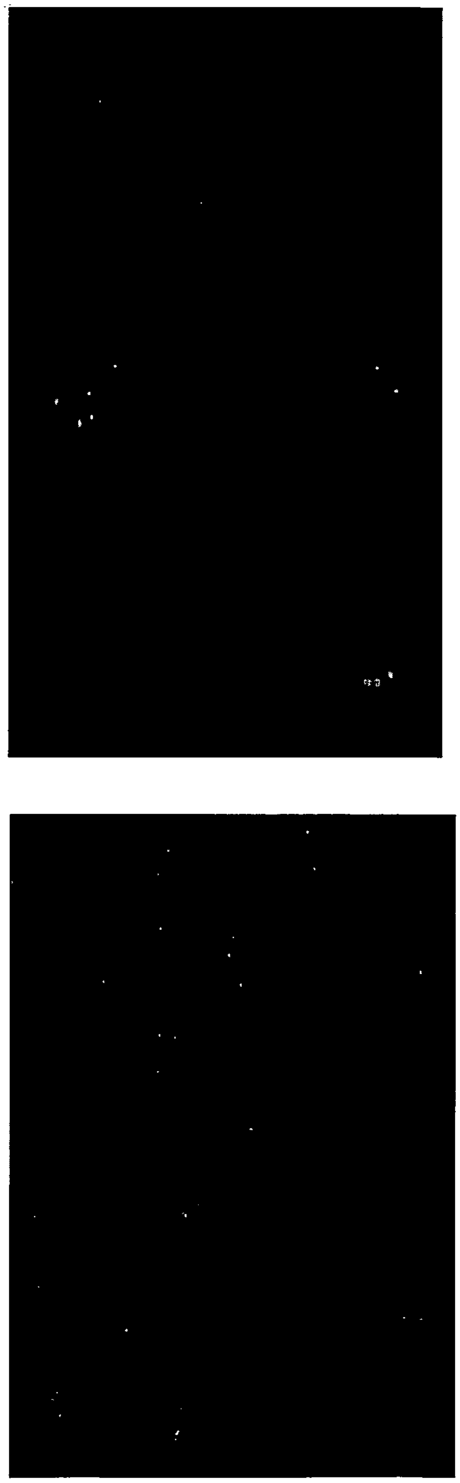

FIG.16

A

B

# FIG.16C

Number of adherent cells

| | Time(min) | | | | | |
|---|---|---|---|---|---|---|
| | 0 | 5 | 10 | 15 | 20 | 30 |
| APS | 235 | 220 | 202 | 157 | 170 | 162 |
| APS+gelatin | 212 | 206 | 184 | 145 | 156 | 183 |
| APS+fibronectin | 229 | 198 | 183 | 132 | 100 | 85 |
| APS+pronectin L | 257 | 170 | 126 | 94 | 71 | 47 |
| | | | | | | |
| PLL | 231 | 221 | 205 | 162 | 168 | 159 |
| PLL+gelatin | 218 | 208 | 186 | 151 | 146 | 156 |
| PLL+fibronectin | 225 | 174 | 162 | 129 | 98 | 79 |
| PLL+pronectin L | 214 | 151 | 132 | 90 | 76 | 50 |
| | | | | | | |
| MAS | 231 | 222 | 216 | 182 | 176 | 169 |
| MAS+gelatin | 224 | 198 | 182 | 163 | 159 | 162 |
| MAS+fibronectin | 218 | 182 | 169 | 143 | 112 | 86 |
| MAS+pronectin L | 220 | 176 | 152 | 124 | 101 | 66 |
| | | | | | | |
| No coating | 226 | 216 | 208 | 192 | 183 | 164 |

Cell adhesion rate (proportion of adherent cells (%))

| | Time(min) | | | | | |
|---|---|---|---|---|---|---|
| | 0 | 5 | 10 | 15 | 20 | 30 |
| APS | 0 | 6.382979 | 14.04255 | 33.19149 | 27.65957 | 31.06383 |
| APS+gelatin | 0 | 2.830189 | 13.20755 | 31.60377 | 26.41509 | 13.67925 |
| APS+fibronectin | 0 | 13.53712 | 20.08734 | 42.35808 | 56.33188 | 62.8821 |
| APS+pronectin L | 0 | 33.85214 | 50.97276 | 63.42412 | 72.37354 | 81.71206 |
| | | | | | | |
| PLL | 0 | 4.329004 | 11.25541 | 29.87013 | 27.27273 | 31.16883 |
| PLL+gelatin | 0 | 4.587156 | 14.6789 | 30.73394 | 33.02752 | 28.44037 |
| PLL+fibronectin | 0 | 22.66667 | 28 | 42.66667 | 56.44444 | 64.88889 |
| PLL+pronectin L | 0 | 29.43925 | 38.31776 | 57.94393 | 64.48598 | 76.63551 |
| | | | | | | |
| MAS | 0 | 3.896104 | 6.493506 | 21.21212 | 23.80952 | 26.83983 |
| MAS+gelatin | 0 | 11.60714 | 18.75 | 27.23214 | 29.01786 | 27.67857 |
| MAS+fibronectin | 0 | 16.51376 | 22.47706 | 34.40367 | 48.62385 | 60.55046 |
| MAS+pronectin L | 0 | 20 | 30.90909 | 43.63636 | 54.09091 | 70 |
| | | | | | | |
| No coating | 0 | 4.424779 | 7.964602 | 15.04425 | 19.02655 | 27.43363 |

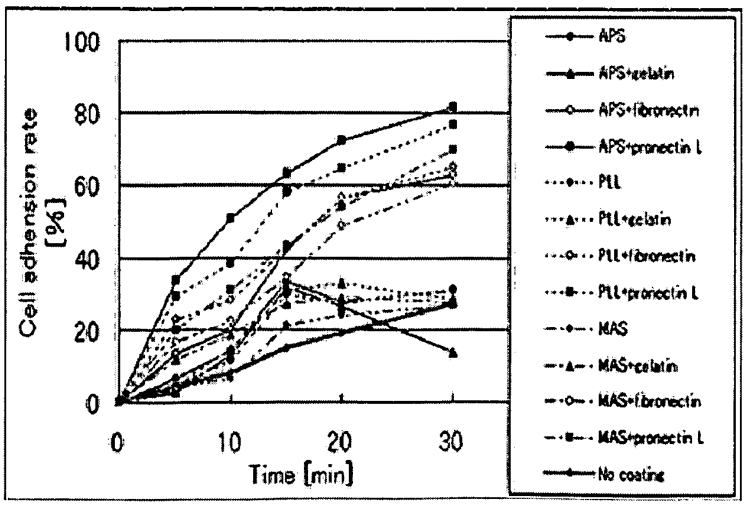

FIG. 16D

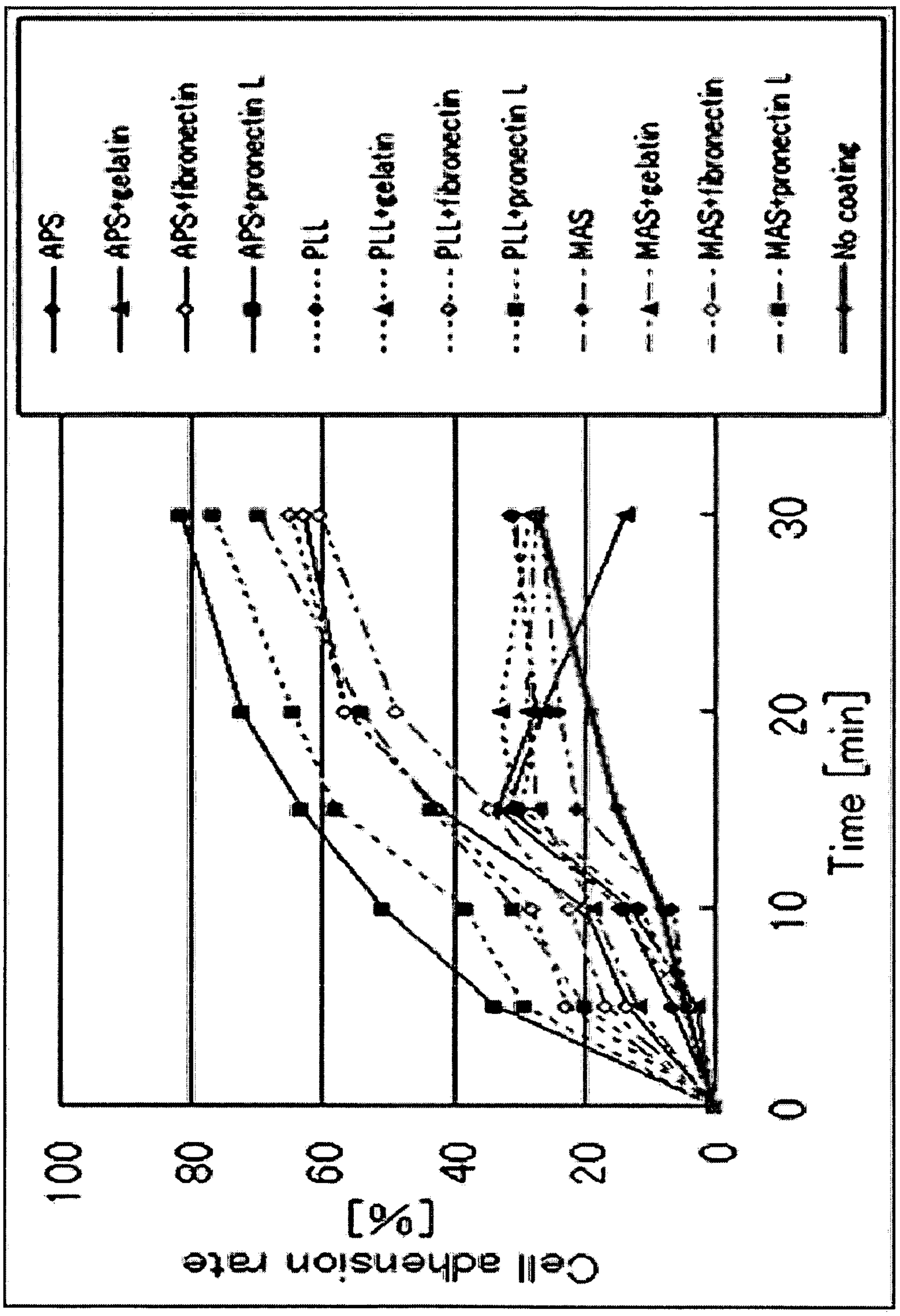
Cell adhesion rate [%]
100
80
60
40
20
0
0
10
20
30
Time [min]
APS
APS+gelatin
APS+fibronectin
APS+pronectin L
PLL
PLL+gelatin
PLL+fibronectin
PLL+pronectin L
MAS
MAS+gelatin
MAS+fibronectin
MAS+pronectin L
No coating

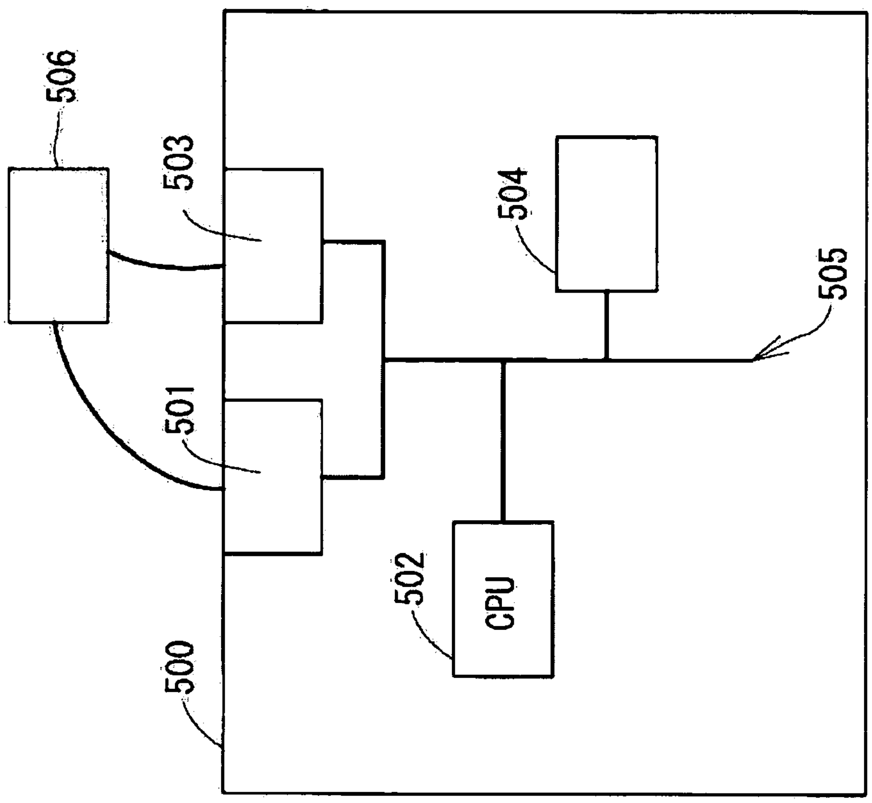


FIG.17

FIG. 18A

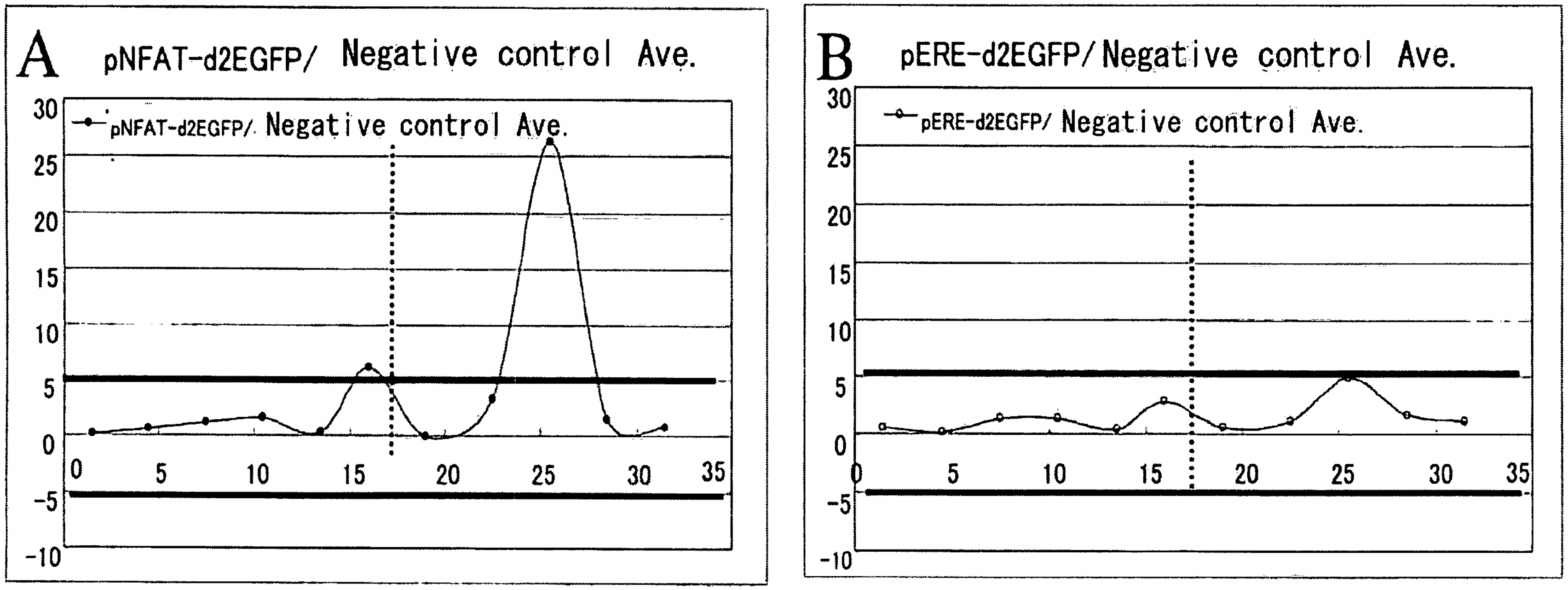


| | 0-31.5 hr | 17.5-31.5 hr | 0-17.5 hr |
|---|---|---|---|
| A | + | + | + |
| B | + | + | - |

FIG.18B

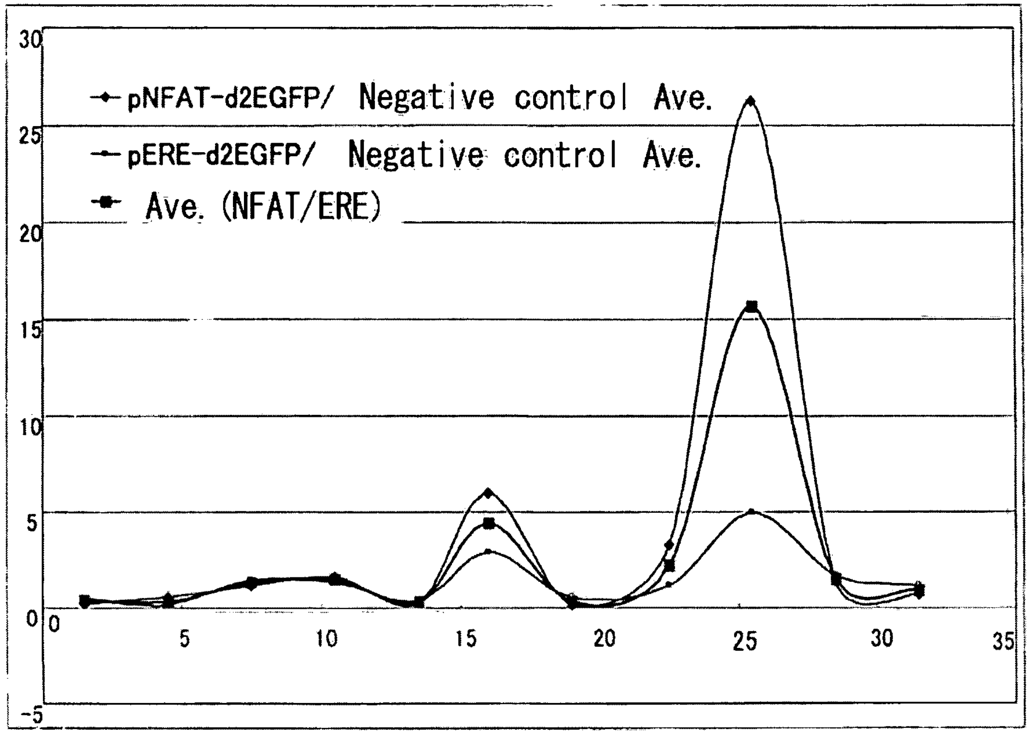


| | 0-31.5 hr | 17.5-31.5 hr | 0-17.5 hr |
|---|---|---|---|
| NFAT | + | + | + |
| ERE | + | - | - |
| NFAT/ERE | + | + | - |

FIG. 19

| |
|---|
| pNFAT-d2EGFP |
| pMyc-d2EGFP |
| pAP1-d2EGFP |
| pSRE-d2EGFP |
| pGRE-d2EGFP |
| pCRE-d2EGFP |
| pNFkB-d2EGFP |
| pAP1(PMA)-d2EGFP |
| pERE-d2EGFP |
| pRARE-d2EGFP |
| pTRE-d2EGFP |
| pE2F-d2EGFP |
| pp53-d2EGFP |
| pRb-d2EGFP |
| pGAS-d2EGFP |
| pISRE-d2EGFP |

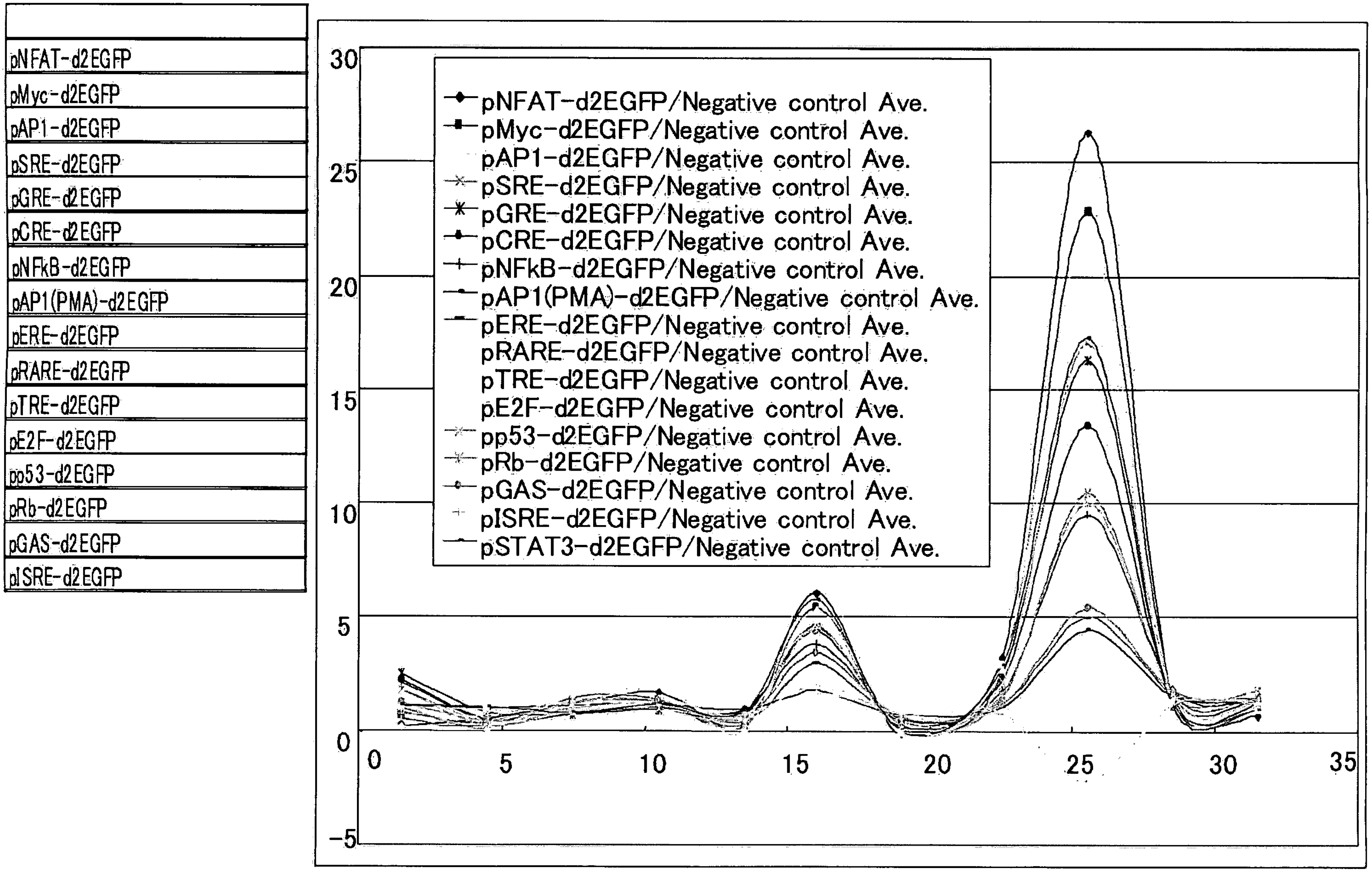

# FIG.20

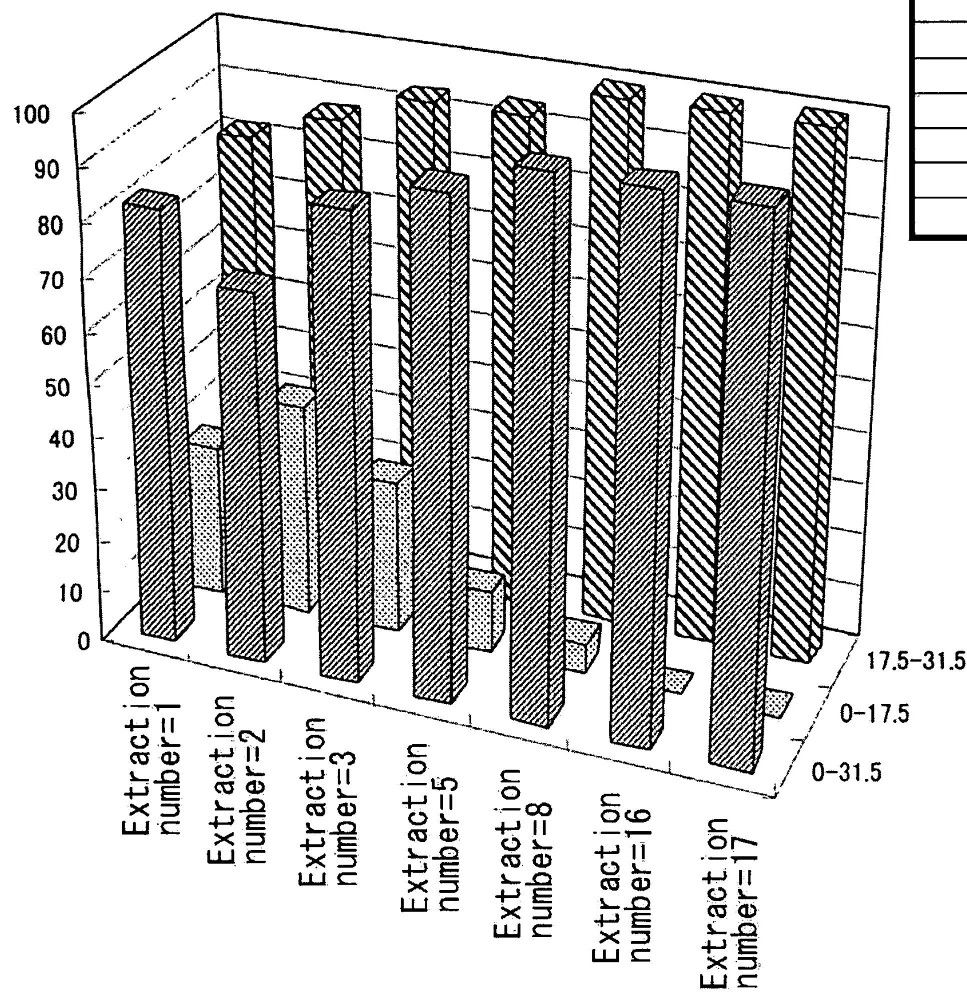


| TH=5 | Day0-1 | | | |
|---|---|---|---|---|
| | Induction of differentiation | 0-31.5 | 0-17.5 | 17.5-31.5 |
| | Extractionnumber=1 | 82.35294 | 29.41176 | 82.35294 |
| | Extractionnumber=2 | 70.58824 | 41.17647 | 88.23529 |
| | Extractionnumber=3 | 88.23529 | 29.41176 | 94.11765 |
| | Extractionnumber=5 | 94.11765 | 11.76471 | 94.11765 |
| | Extractionnumber=8 | 100 | 5.882353 | 100 |
| | Extractionnumber=16 | 100 | 0 | 100 |
| | Extractionnumber=17 | 100 | 0 | 100 |

# FIG. 21

| No induction of differentiation | 0-31.5 | 0-17.5 | 17.5-31.5 |
|---|---|---|---|
| Extraction number=1 | 5.882353 | 5.882353 | 0 |
| Extraction number=2 | 0 | 0 | 0 |
| Extraction number=3 | 0 | 0 | 0 |
| Extraction number=5 | 0 | 0 | 0 |
| Extraction number=8 | 0 | 0 | 0 |
| Extraction number=16 | 0 | 0 | 0 |
| Extraction number=17 | 0 | 0 | 0 |

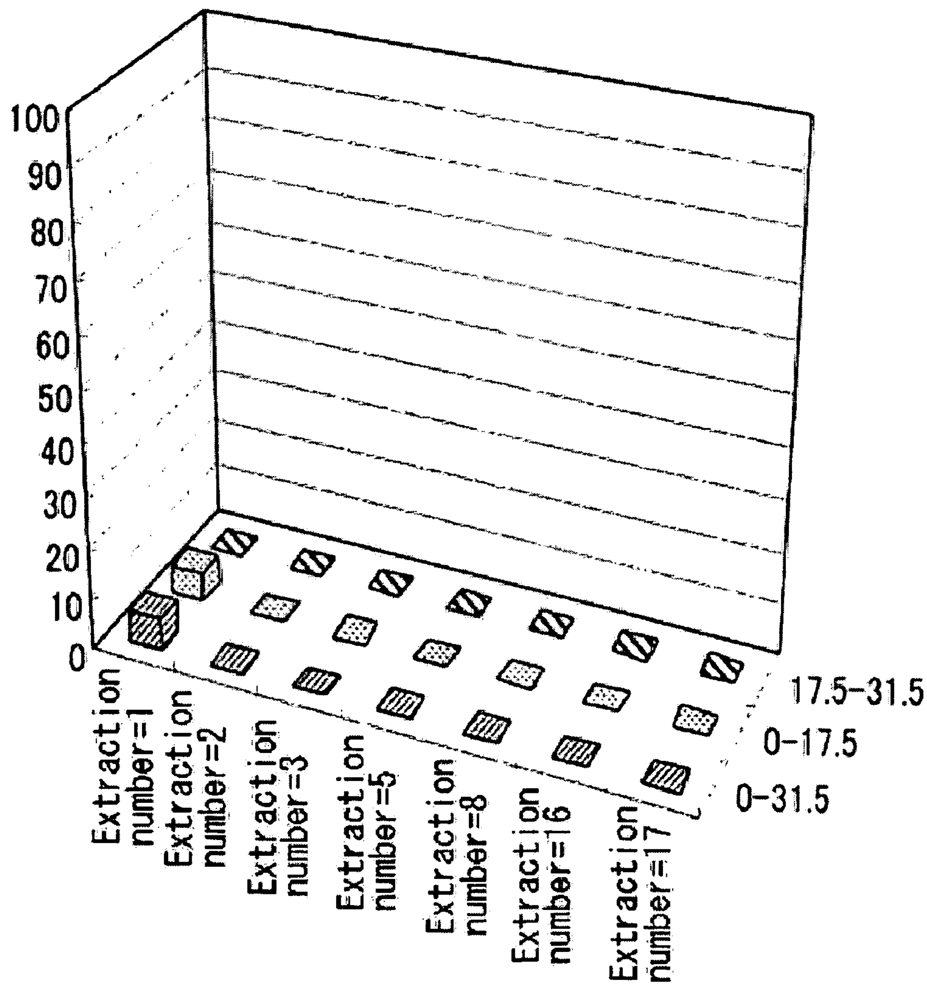

FIG. 22

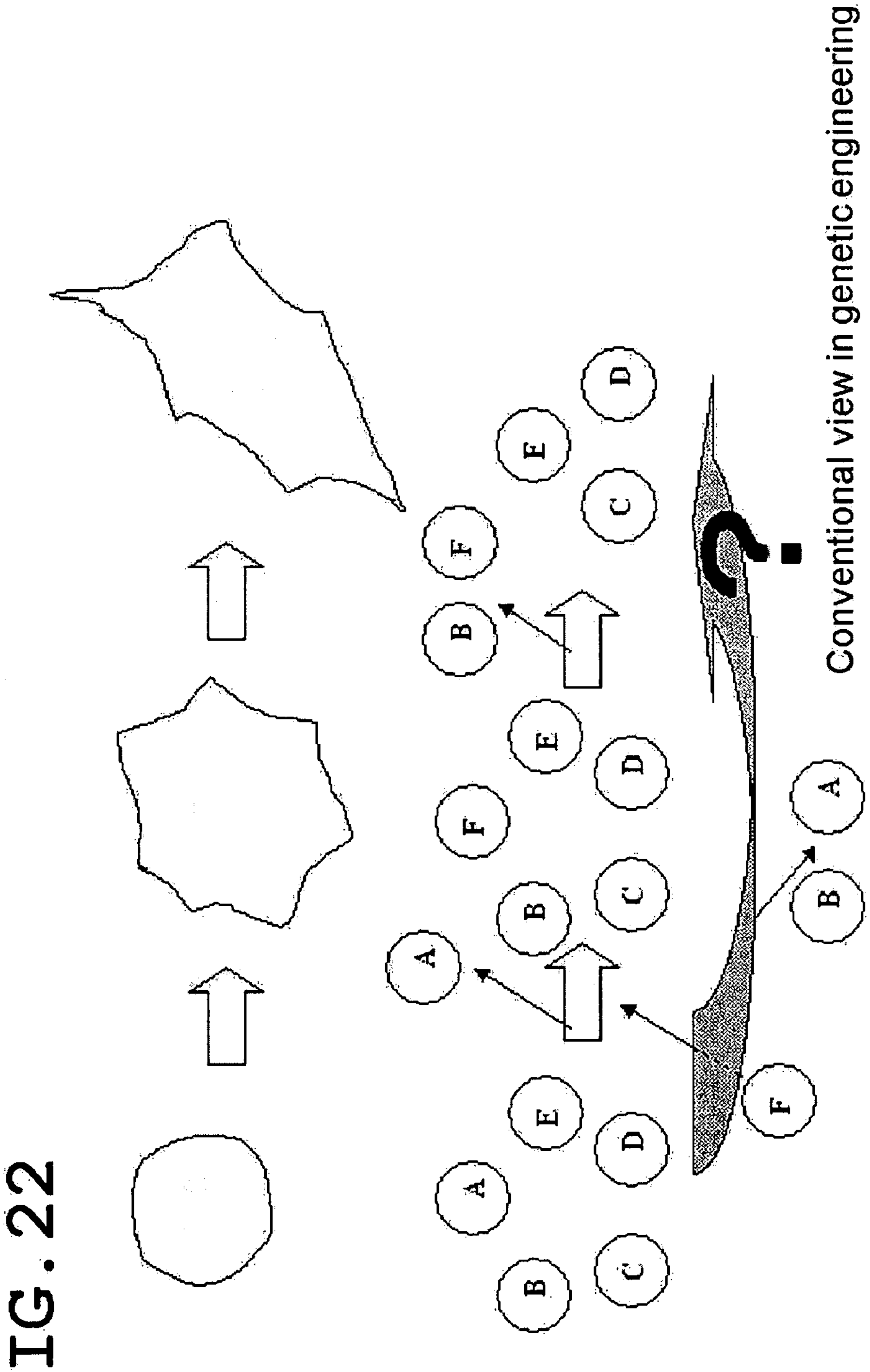

FIG. 23

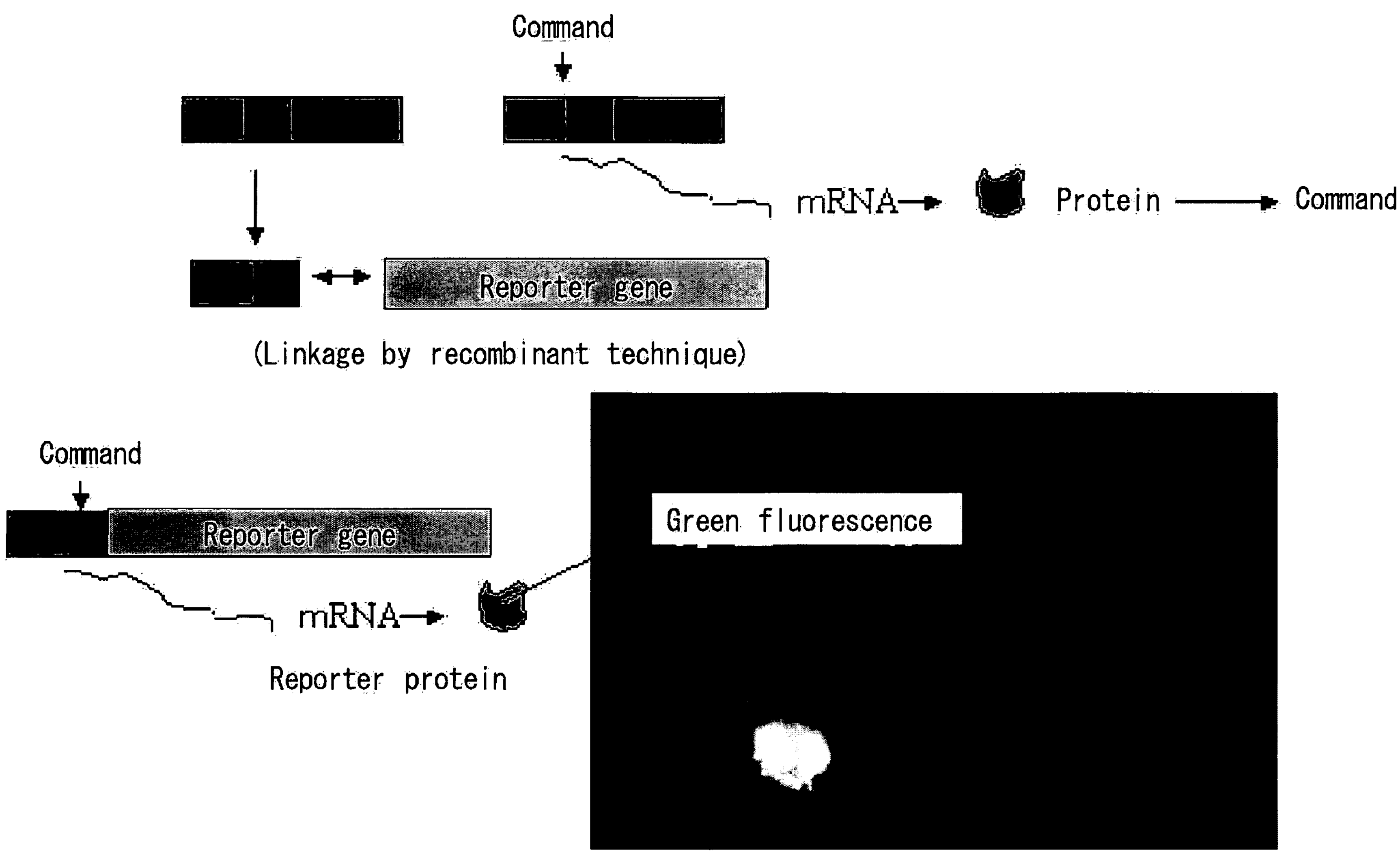
Command
mRNA
Protein
Command
Reporter gene
(Linkage by recombinant technique)
Command
Reporter gene
mRNA
Reporter protein
Green fluorescence

FIG.24

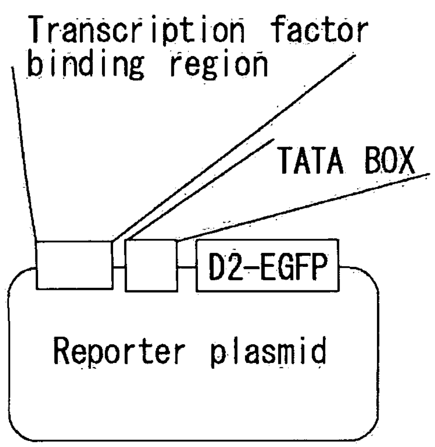


## Construction of transcription factor reporter

| Vector | Pathway | Transcription factor | Cis-acting enhancer element |
|---|---|---|---|
| pNFkB-d2FGFP | IKK/NFkB | NFkB | kB |
| pAP1-d2FGFP | SAPK/JNK | c−Jun, c-Fos | AP1 |
| pSRF-d2FGFP | MAPK/JNK, MAPK/FRK | Flk-1,STAT, TCF,SRF | SRF |
| pGRF-d2FGFP | Glicocorticoide (HXP90 mediation) | GR | GRF |
| pCRF-d2FGFP | PKA/CRFB,JNK/p38 PKA | ATF2/CRFB | CRF |
| pMpc-TA-d2FGFP, pMYC-,d2FGFP | Cell cycle | c-myc | F-box |
| pHSF-d2FGFP | HSF | HSF | HSF |
| pNFAT-d2FGFP | NFAT/Calcineurin/PKC | NFAT | NFAT |
| pAP1(PMA)-TA-d2FGFP | PKC | | AP1(PMA) |
| pRb-TA-d2FGFP | Cell cycle | | Rb |
| pF2F-TA-d2FGFP | Cell cycle | | F2F |
| pp53-TA-d2FGFP | Cell cycle apoptosis | | P53 |
| pGAN-TA-d2FGFP | JAK/STAT | STAT1/STAT1 | GAS |
| pISRF-TA-d2FGFP | JAK/STAT | STAT2/STAT1 | ISRF |
| pSTAT3-TA-d2FGFP | JAK/STAT | STAT3/STAT3 | STAT3 |
| pFRF-TA-d2FGFP | Estrogen receptor | | FRF |
| pRARF-TA-d2FGFP | Retinoic acid | | RARF |
| pTRF-TA-d2FGFP | Thyroid receptor | | TRF |

FIG.25

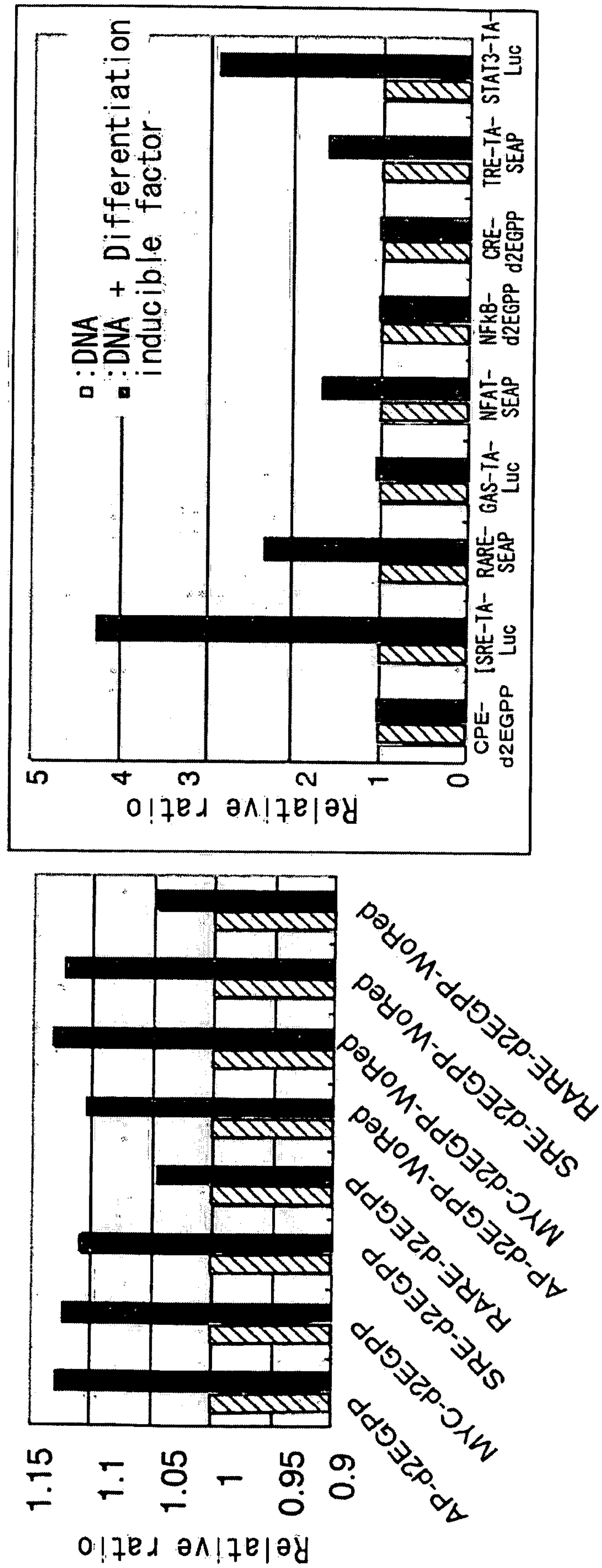
Relative ratio
1.15
1.1
1.05
1
0.95
0.9
AP-d2EGPP
MYC-d2EGPP
SRE-d2EGPP
RARE-d2EGPP
AP-d2EGPP-WoRed
MYC-d2EGPP-WoRed
SRE-d2EGPP-WoRed
RARE-d2EGPP-WoRed
□ : DNA
■ : DNA + Differentiation inducible factor
Relative ratio
5
4
3
2
1
0
CPE-d2EGPP
ISRE-TA-Luc
RARE-SEAP
GAS-TA-Luc
NFAT-SEAP
NFkB-d2EGPP
CRE-d2EGPP
TRE-TA-SEAP
STAT3-TA-Luc

FIG.26

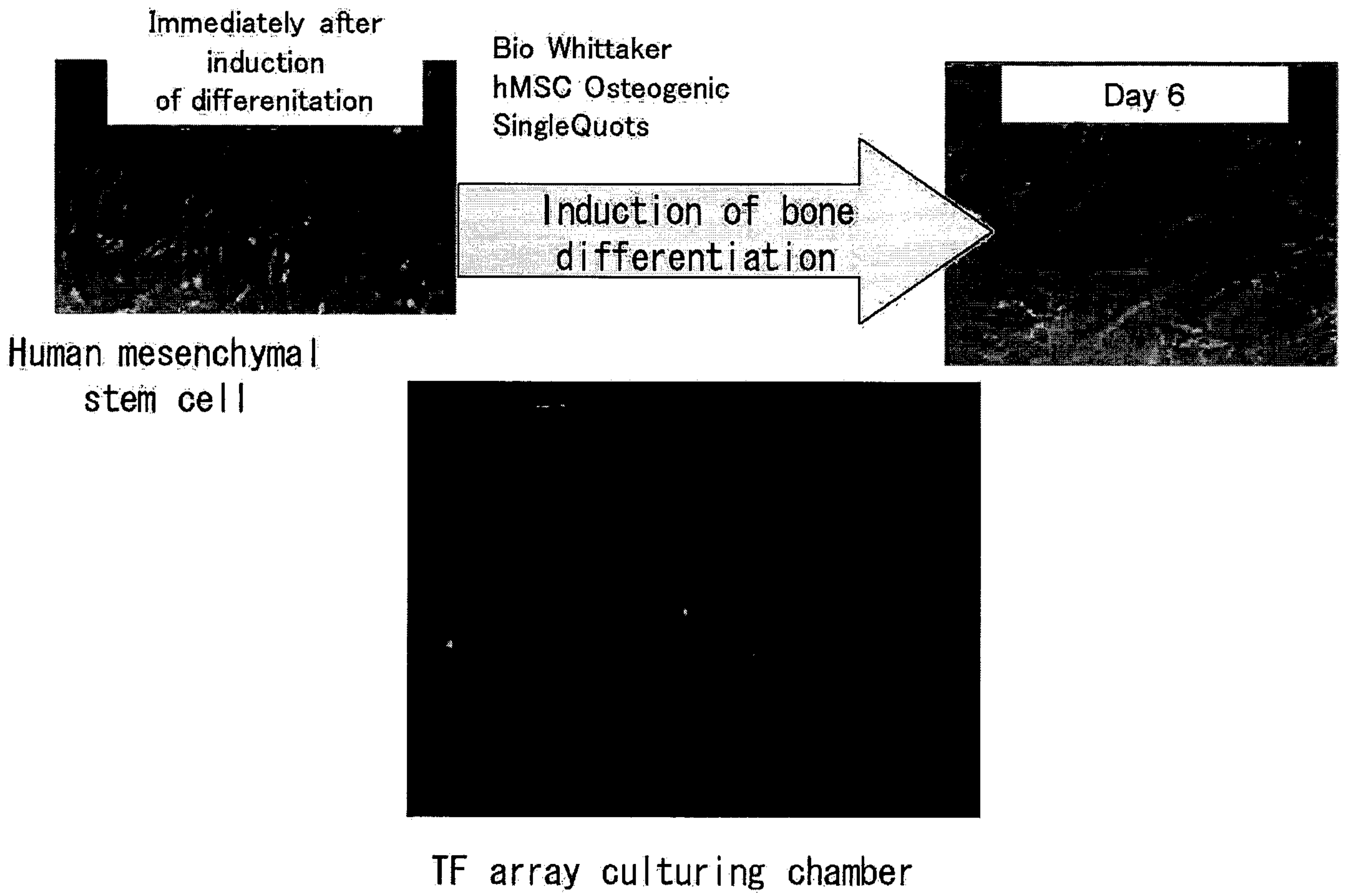
Immediately after
induction
of differenitation
Human mesenchymal
stem cell
Bio Whittaker
hMSC Osteogenic
SingleQuots
Induction of bone
differentiation
Day 6
TF array culturing chamber

# FIG.27

$$\frac{V_P - V_{Degfp}}{V_{PCMV} - V_{Degfp}}$$

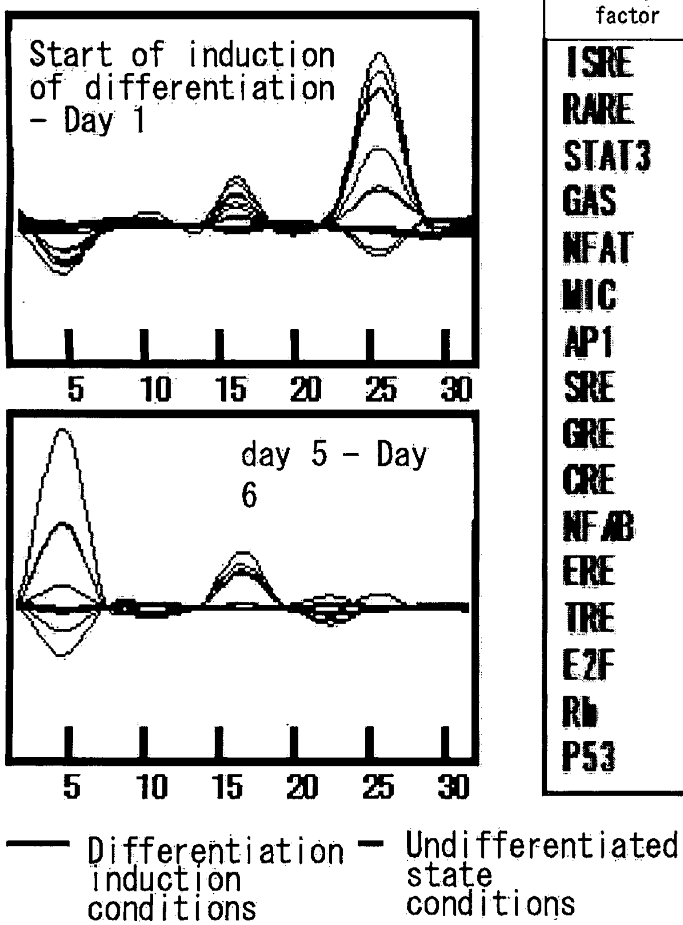


Phases of transcription activity oscillation by induction of differentiation

| Transcription factor | Undifferentiated state | | Differentiated state | | | | |
|---|---|---|---|---|---|---|---|
| ISRE | c | c | - | + | + | ○ | ← STAT1/STAT2 |
| RARE | c | c | - | + | + | ○ | ← Retinoic acid |
| STAT3 | c | c | + | - | + | ○ | ← Control of differentiation |
| GAS | c | c | + | + | + | | |
| NFAT | c | c | + | + | + | | |
| MIC | c | c | + | + | + | | |
| AP1 | c | c | + | + | + | | |
| SRE | c | c | + | + | + | | |
| GRE | c | c | + | - | + | ○ | ← Glucocorticoid |
| CRE | c | c | + | - | + | ○ | ← cAMP |
| NF AB | c | c | + | + | + | | |
| ERE | c | c | + | + | + | | |
| TRE | c | c | + | + | c | ○ | ← Thyroid hormone |
| E2F | c | c | + | c | + | ○ | ← Cell cycle |
| Rb | c | c | + | + | + | | |
| P53 | c | c | + | - | + | ○ | ← G1 check point |

FIG.28

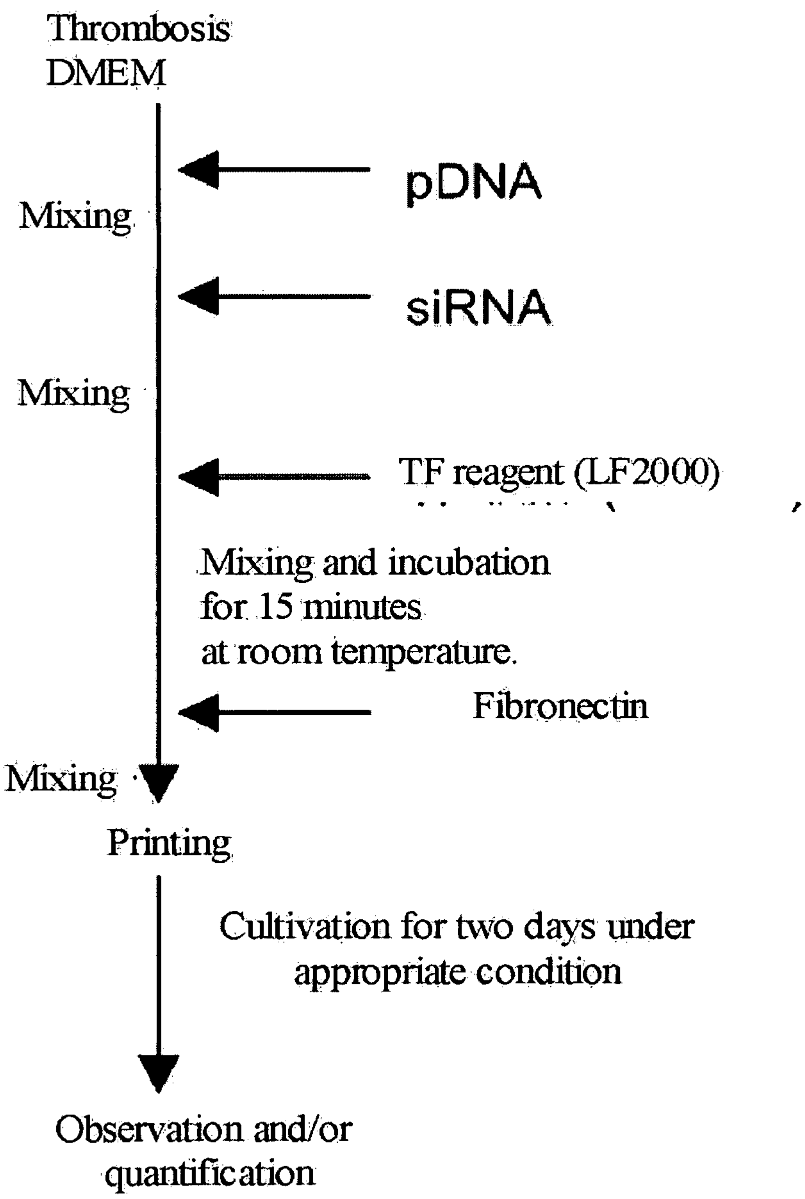
Thrombosis
DMEM
Mixing
pDNA
siRNA
Mixing
TF reagent (LF2000)
Mixing and incubation
for 15 minutes
at room temperature.
Fibronectin
Mixing
Printing
Cultivation for two days under
appropriate condition
Observation and/or
quantification

Fig. 29

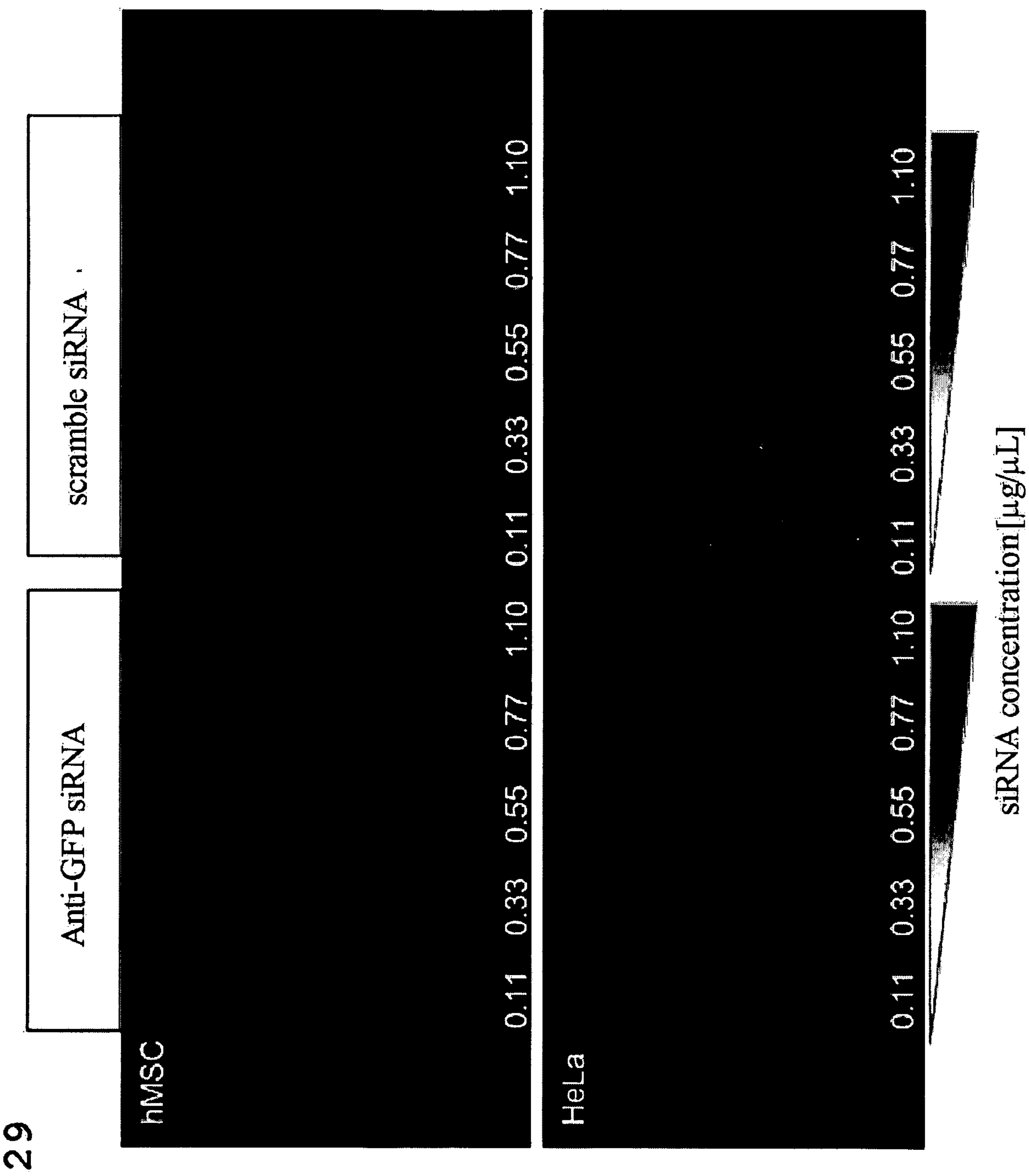
Anti-GFP siRNA
scramble siRNA
hMSC
HeLa
0.11 0.33 0.55 0.77 1.10 0.11 0.33 0.55 0.77 1.10
0.11 0.33 0.55 0.77 1.10 0.11 0.33 0.55 0.77 1.10
siRNA concentration[μg/μL]

FIG. 30

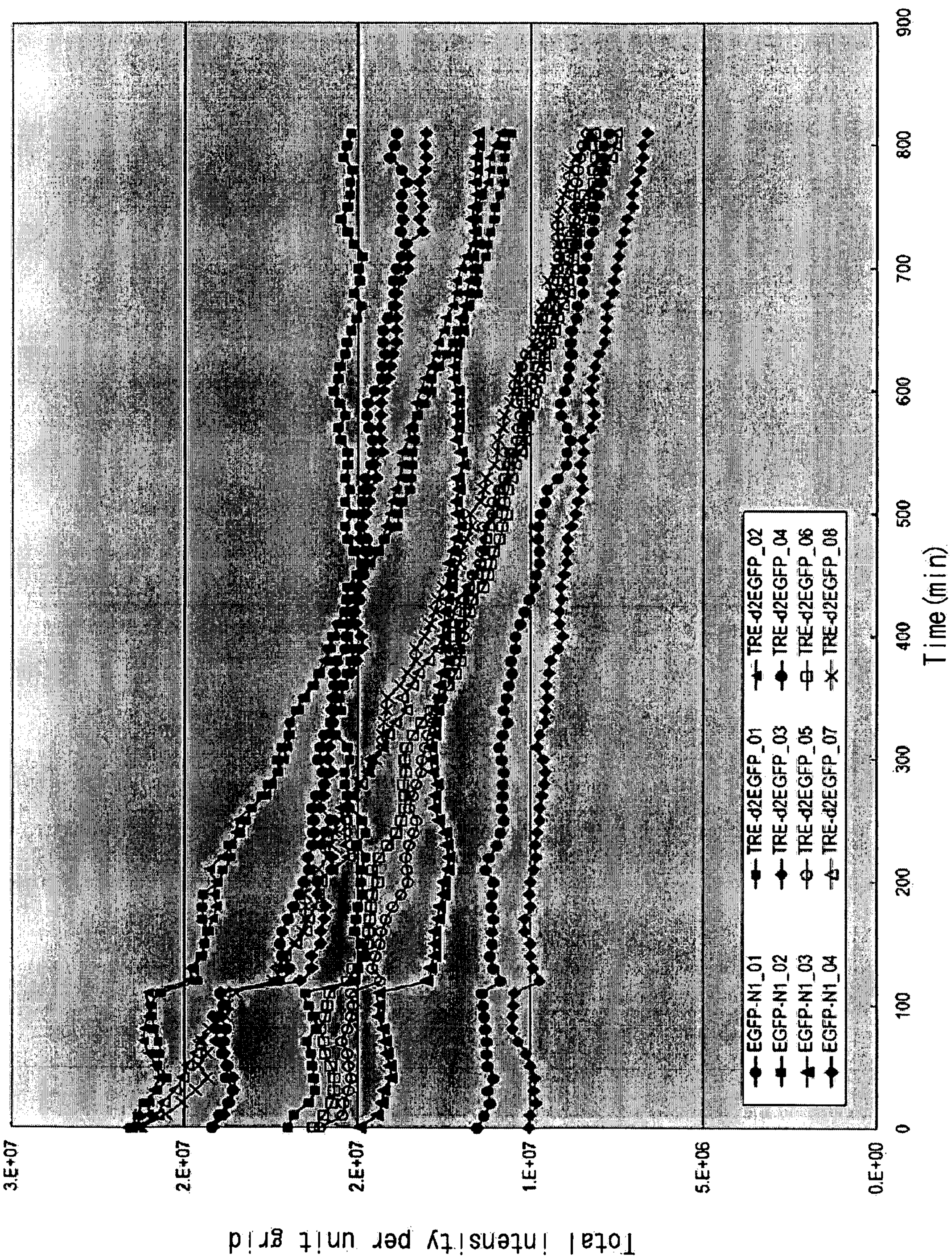
Total intensity per unit grid
3.E+07
2.E+07
2.E+07
1.E+07
5.E+06
0.E+00
0
100
200
300
400
500
600
700
800
900
Time (min)
EGFP-N1_01
EGFP-N1_02
EGFP-N1_03
EGFP-N1_04
TRE-d2EGFP_01
TRE-d2EGFP_02
TRE-d2EGFP_03
TRE-d2EGFP_04
TRE-d2EGFP_05
TRE-d2EGFP_06
TRE-d2EGFP_07
TRE-d2EGFP_08

Fig. 31

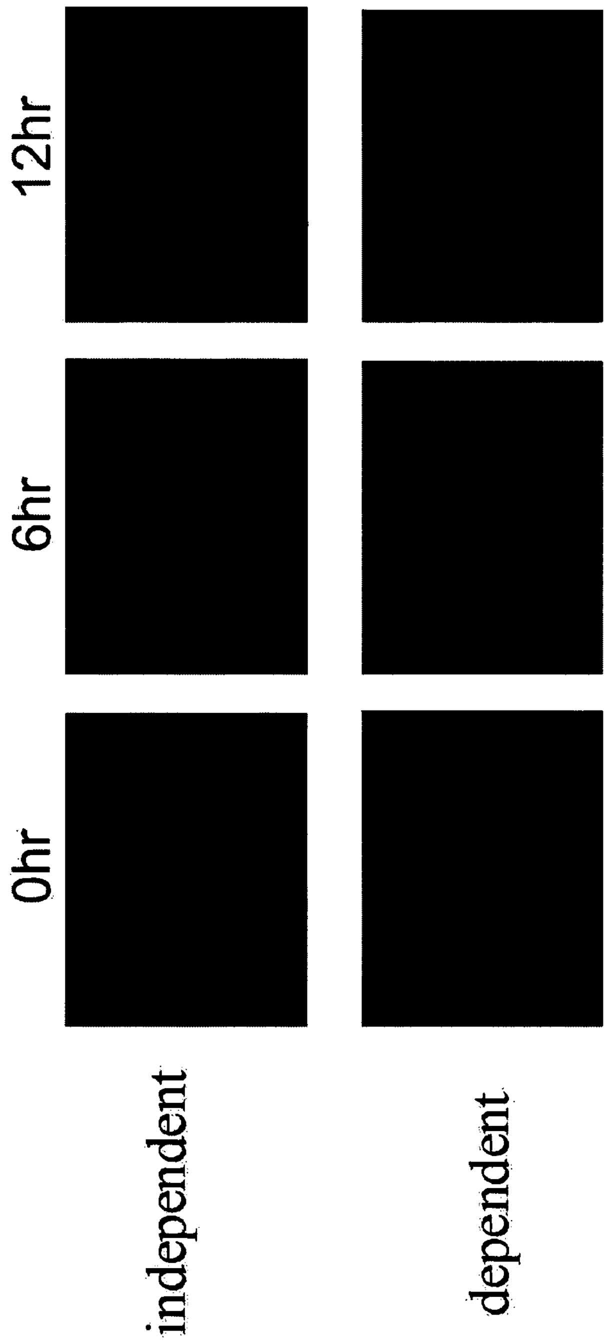

Fig. 32

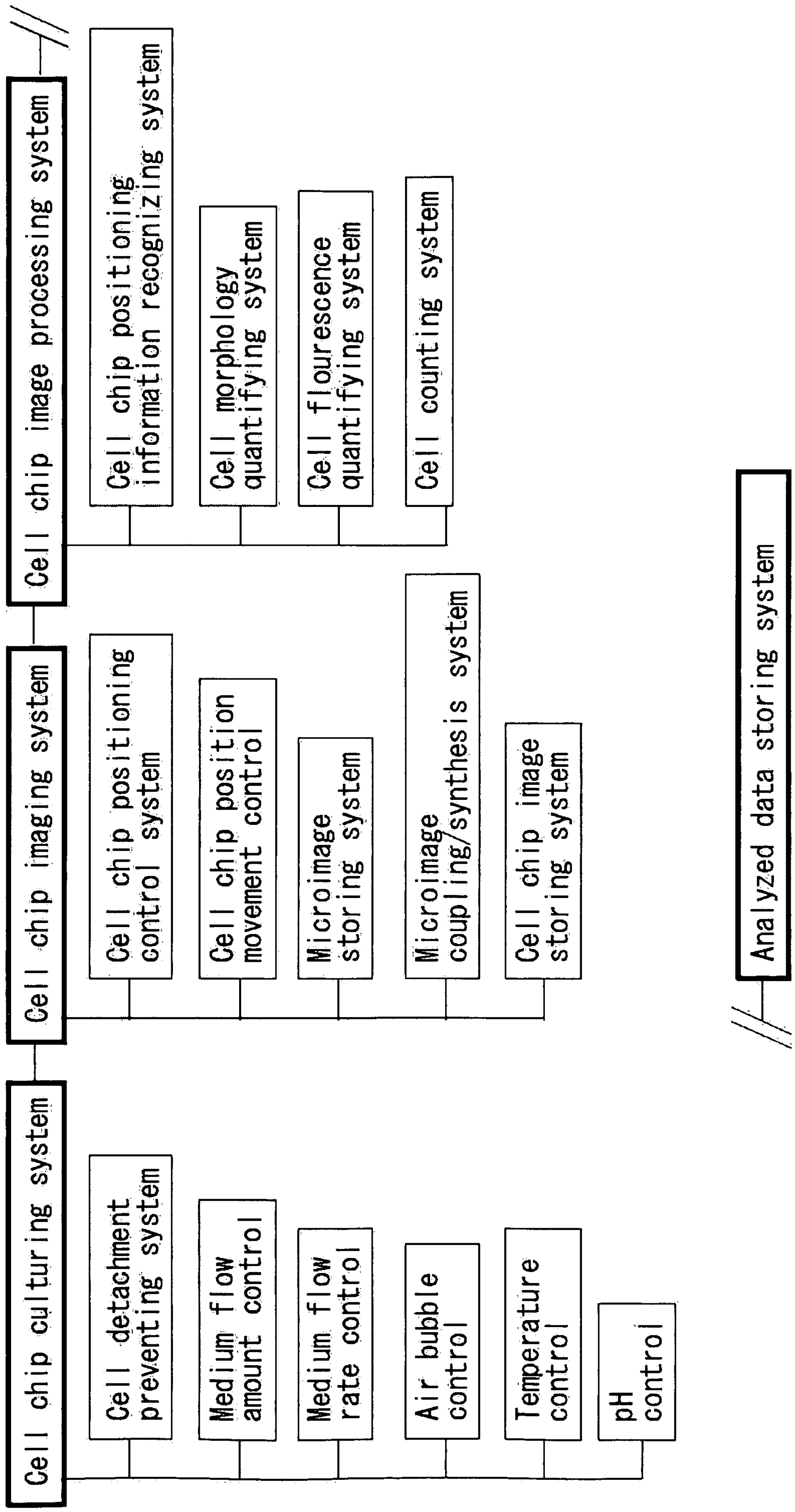
Cell chip culturing system
Cell detachment preventing system
Medium flow amount control
Medium flow rate control
Air bubble control
Temperature control
pH control
Cell chip imaging system
Cell chip positioning control system
Cell chip position movement control
Microimage storing system
Microimage coupling/synthesis system
Cell chip image storing system
Cell chip image processing system
Cell chip positioning information recognizing system
Cell morphology quantifying system
Cell flourescence quantifying system
Cell counting system
Analyzed data storing system

Fig. 33

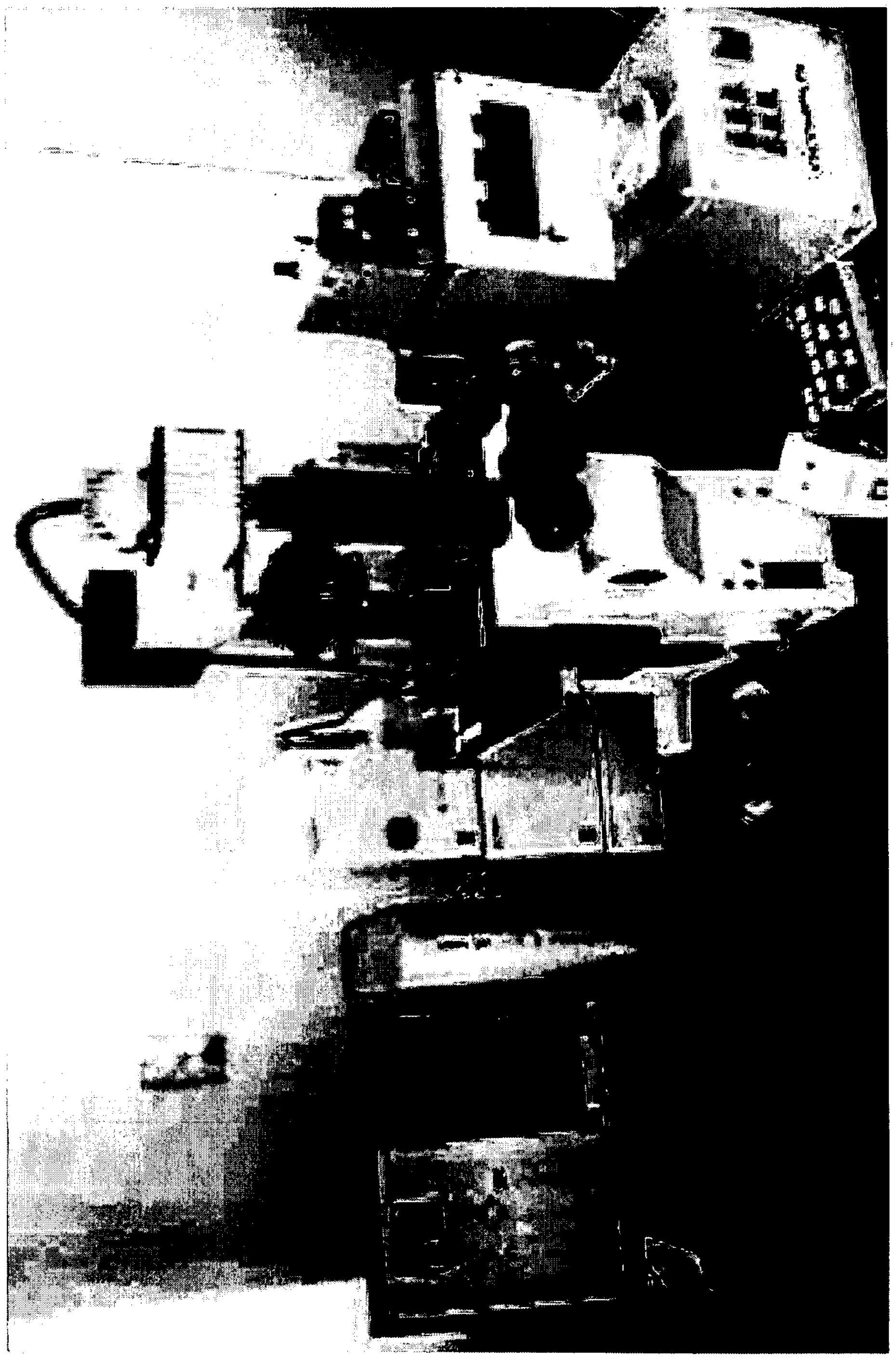

Fig. 34

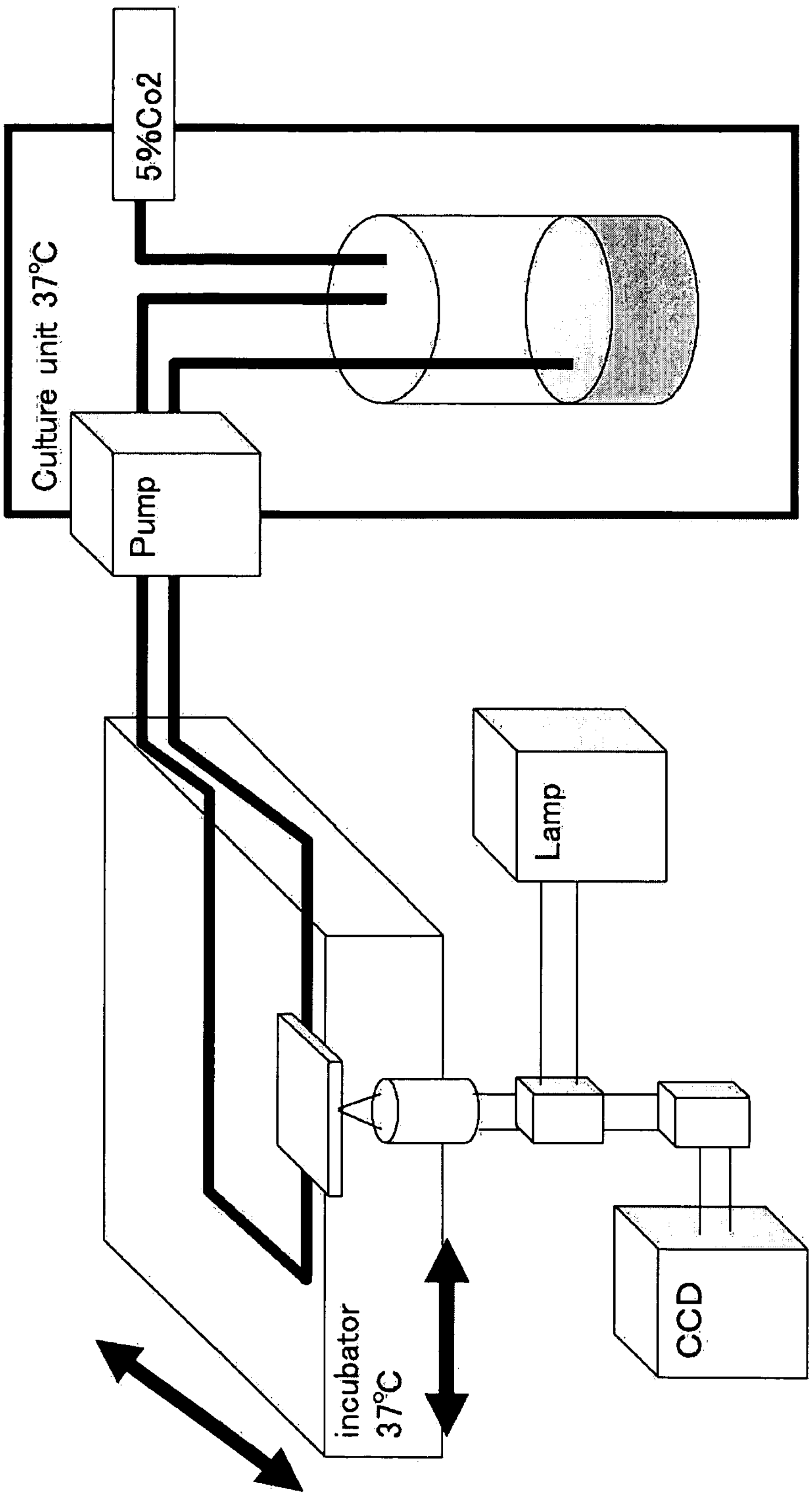

Fig. 35

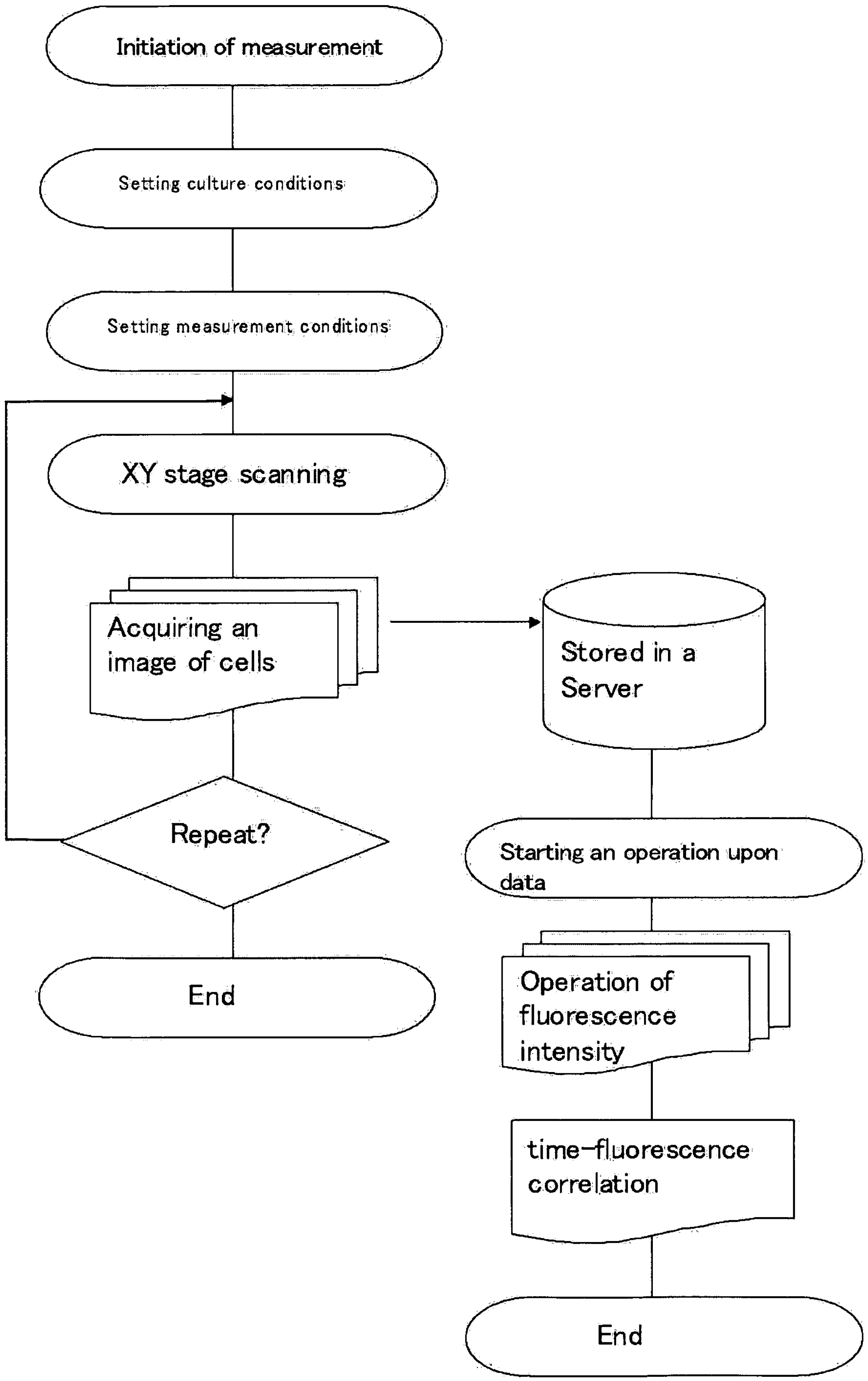
Initiation of measurement
Setting culture conditions
Setting measurement conditions
XY stage scanning
Acquiring an image of cells
Stored in a Server
Repeat?
End
Starting an operation upon data
Operation of fluorescence intensity
time-fluorescence correlation
End

Fig. 36

Format of Experiments

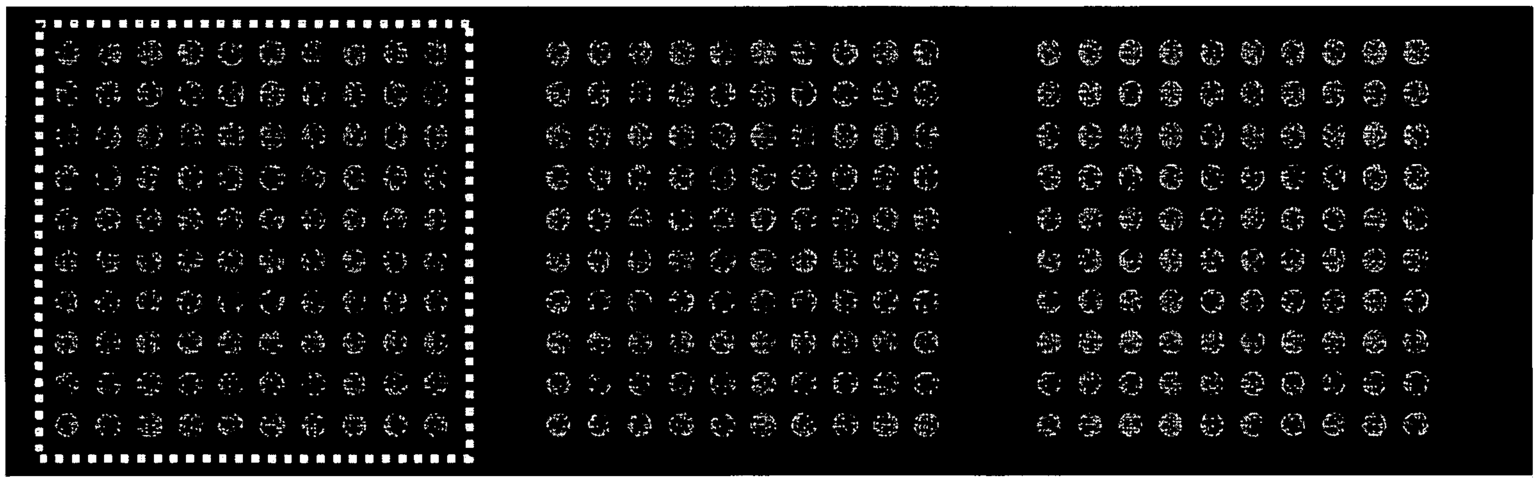

570 grid slide

Fig. 37

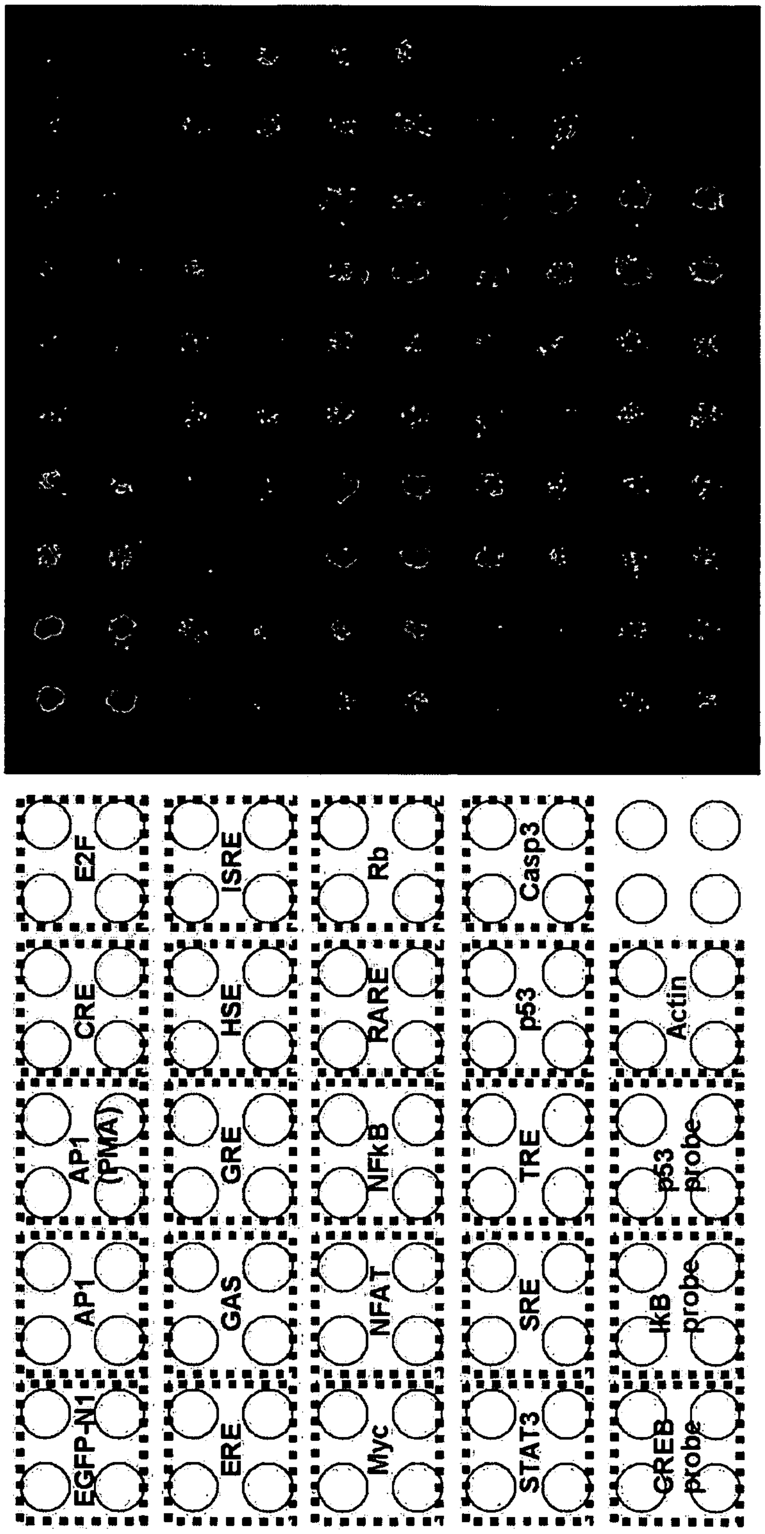
E2F
ISRE
Rb
Casp3
CRE
HSE
RARE
p53
Actin
AP1 (PMA)
GRE
NFkB
TRE
p53 probe
AP1
GAS
NFAT
SRE
IkB probe
EGFP-N1
ERE
Myc
STAT3
CREB probe

Fig. 38A Results (HeLa Cell strain)

**Change of Culture medium (10%FBS → Serum Free)**

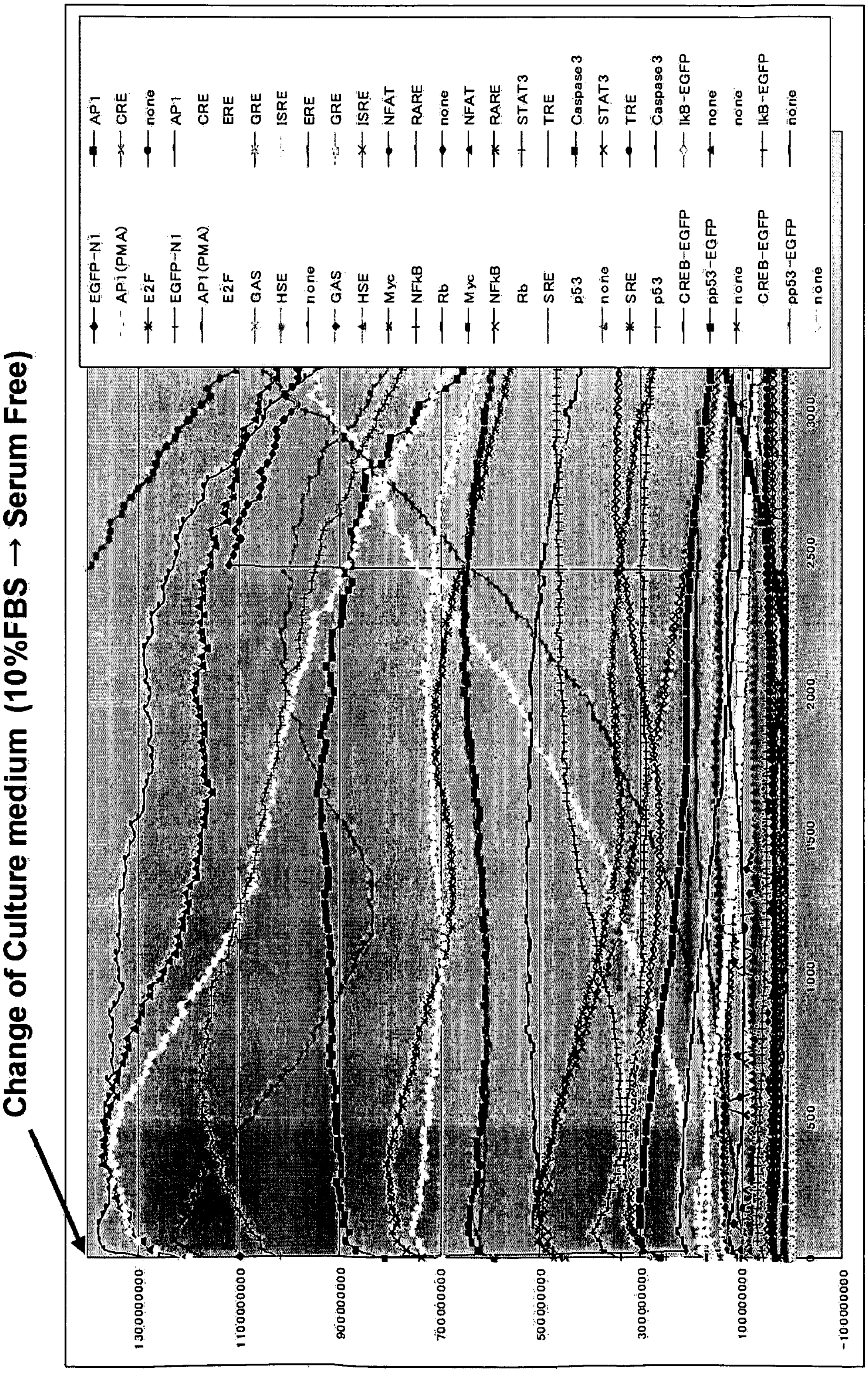

Fig. 38B

| t | #1 | #2 | #3 | #4 | #5 | #6 | #7 | #8 | t | #1 | #2 | #3 | #4 | #5 | #6 | #7 | #8 |
|---|---|---|---|---|---|---|---|---|---|---|---|---|---|---|---|---|---|
| 0 | 131846971 | 107021053 | 119151278 | 115001222 | 60181788 | 72578952 | 24282714 | 103114488 | 1770 | 105322910 | 64948202 | 95113647 | 84294954 | 52418689 | 65019400 | 23145557 | 67732656 |
| 30 | 134451985 | 107894767 | 122857933 | 122720386 | 68838381 | 80973487 | 28939215 | 118973499 | 1800 | 104508583 | 65502649 | 95499933 | 84745230 | 52088396 | 65147189 | 23490877 | 68340593 |
| 60 | 140354047 | 113097806 | 128086766 | 122937261 | 70679354 | 81007496 | 29413561 | 117546392 | 1830 | 100457983 | 65924657 | 96145135 | 87223611 | 52000027 | 65248036 | 23786561 | 68448770 |
| 90 | 135127114 | 110683443 | 124428392 | 114849994 | 72795014 | 85346853 | 31632821 | 121715629 | 1860 | 100869178 | 66346743 | 95770910 | 86640901 | 51365574 | 64934118 | 24381842 | 68228044 |
| 120 | 139989937 | 111820990 | 126071631 | 121370714 | 69284700 | 82412690 | 30124200 | 118642262 | 1890 | 100699983 | 66868156 | 96439888 | 87743949 | 51381145 | 64491699 | 24616221 | 67987812 |
| 150 | 139231204 | 111510471 | 129776876 | 122180796 | 68744394 | 83797070 | 30032617 | 117512581 | 1920 | 99776591 | 66739357 | 97319114 | 88407893 | 51642720 | 62469426 | 25077546 | 68750853 |
| 180 | 139382051 | 111678196 | 131330883 | 123718176 | 68349785 | 84728536 | 30433108 | 117683460 | 1950 | 101366546 | 67166966 | 95366923 | 88015728 | 50410209 | 62384228 | 24924564 | 69334820 |
| 210 | 139391269 | 111072765 | 131442707 | 124048095 | 67783148 | 84584143 | 30145205 | 117950409 | 1980 | 101495008 | 67318419 | 96199166 | 88489672 | 51253664 | 62832515 | 24904899 | 68642956 |
| 240 | 137815611 | 109339612 | 131631733 | 122103264 | 67554325 | 84918465 | 30395891 | 116678188 | 2010 | 102297162 | 67719495 | 97144172 | 90516943 | 50674681 | 62786025 | 25198400 | 68774799 |
| 270 | 137950180 | 108815023 | 131301086 | 120422706 | 66310409 | 84722404 | 30378410 | 115181667 | 2040 | 102856981 | 68330783 | 97405421 | 90272600 | 50675313 | 63264232 | 25398343 | 69520617 |
| 300 | 136070787 | 105767037 | 131061594 | 118120615 | 65653080 | 84236500 | 30190912 | 113655290 | 2070 | 103964829 | 68349822 | 97493770 | 90541068 | 50821998 | 63747553 | 25707582 | 69362122 |
| 330 | 135868692 | 104686932 | 129864920 | 117626536 | 65582066 | 85905002 | 29954900 | 115402355 | 2100 | 103603280 | 68738298 | 96998261 | 87424808 | 50906841 | 62839807 | 25236523 | 69386052 |
| 360 | 135790350 | 103722384 | 129839038 | 116783082 | 65349430 | 85976876 | 29779244 | 114873250 | 2130 | 102699521 | 69264374 | 96190028 | 88818843 | 49802662 | 62446835 | 24946255 | 69632944 |
| 390 | 135270416 | 101862108 | 128508503 | 115832507 | 64254234 | 85962538 | 29209193 | 114513652 | 2160 | 103400704 | 69725979 | 94552320 | 88540037 | 50628141 | 62595780 | 25145669 | 69740934 |
| 420 | 135154900 | 99496833 | 127144112 | 114731896 | 63281454 | 84893882 | 29017565 | 111973268 | 2190 | 103727077 | 70420359 | 94473758 | 88388638 | 50427011 | 62362626 | 25167625 | 70188667 |
| 450 | 134348428 | 97506154 | 126472490 | 113945948 | 63383715 | 84983620 | 29278131 | 112634585 | 2220 | 101854014 | 69806097 | 92491372 | 88433384 | 50464098 | 61557266 | 25155570 | 70396049 |
| 480 | 134488233 | 98045189 | 125952544 | 112932584 | 62460643 | 86022494 | 28800906 | 111598194 | 2250 | 102028092 | 70360627 | 92692076 | 89582488 | 50199331 | 61602318 | 25218991 | 70360574 |
| 510 | 134828916 | 96601977 | 125741989 | 111068793 | 61644282 | 84821638 | 28190380 | 110427198 | 2280 | 102154875 | 70393537 | 91913008 | 88804552 | 50369943 | 62049361 | 25290410 | 70408935 |
| 540 | 133349371 | 96358917 | 126274332 | 108369862 | 61396669 | 85631764 | 27741874 | 108064077 | 2310 | 100659775 | 69845449 | 90988570 | 87894515 | 47543813 | 61775859 | 25193905 | 70981622 |
| 570 | 129625398 | 95140023 | 126679700 | 107770289 | 60527134 | 84186661 | 28164319 | 107556086 | 2340 | 100624095 | 65330456 | 90739489 | 88749169 | 47566142 | 62901005 | 25270120 | 70093019 |
| 600 | 129766103 | 93314758 | 125411482 | 104323800 | 60681496 | 84141929 | 27600351 | 106741665 | 2370 | 100707159 | 65040667 | 91263725 | 89712153 | 46615016 | 62521334 | 25063903 | 69411561 |
| 630 | 129977116 | 92430983 | 125047544 | 104772646 | 59049329 | 83202321 | 27306758 | 105614103 | 2400 | 100050318 | 65537842 | 90322183 | 89526685 | 45872070 | 63252882 | 25150789 | 69103213 |
| 660 | 128163514 | 90032772 | 124870358 | 100972677 | 59673914 | 83978923 | 27217770 | 103993748 | 2430 | 102482171 | 66973986 | 90476617 | 89693643 | 45060215 | 63958898 | 25586520 | 70203632 |
| 690 | 125801897 | 88904991 | 123570533 | 101712740 | 57687685 | 84150812 | 26928592 | 103170500 | 2460 | 100958146 | 65160183 | 88505939 | 86410517 | 43848201 | 63071497 | 25605133 | 67234853 |
| 720 | 125925415 | 87908198 | 123064384 | 100244137 | 58864582 | 83836341 | 27008980 | 103722366 | 2490 | 101139338 | 64734324 | 88491643 | 86669978 | 43639965 | 63347304 | 25324382 | 67741821 |
| 750 | 125367660 | 87057412 | 120719703 | 98212103 | 56207749 | 82990816 | 27009884 | 101632337 | 2520 | 100274865 | 65277633 | 88252591 | 86380010 | 43637465 | 63225341 | 25160808 | 67652523 |
| 780 | 124187804 | 84808149 | 121110019 | 96045385 | 55741066 | 81534786 | 27043023 | 101452289 | 2550 | 98877249 | 64598928 | 87124517 | 85690599 | 43419603 | 63663380 | 25166222 | 68038842 |
| 810 | 125624336 | 84694968 | 121178380 | 94346438 | 57072256 | 79976718 | 26902374 | 99411088 | 2580 | 99777785 | 64894956 | 86723267 | 86502580 | 37776213 | 64072719 | 25316786 | 68207253 |
| 840 | 124512900 | 83987566 | 120066053 | 93562312 | 55392563 | 80602389 | 26227324 | 98674126 | 2610 | 97455822 | 64371955 | 84530365 | 85594440 | 38366987 | 63738070 | 24740382 | 67169360 |
| 870 | 123482642 | 82265507 | 119449961 | 91848781 | 54608975 | 80205625 | 26406085 | 98445125 | 2640 | 97114881 | 62737861 | 83525514 | 84129796 | 38345830 | 63010342 | 24768881 | 67903630 |
| 900 | 121881387 | 80320204 | 117653941 | 90175926 | 55596195 | 78570864 | 26249152 | 95557166 | 2670 | 96298850 | 62619278 | 82692172 | 84303073 | 38589094 | 63015466 | 24326059 | 66699483 |
| 930 | 121000814 | 78935834 | 117857470 | 87894345 | 55204079 | 77613609 | 25969646 | 94884861 | 2700 | 96790138 | 61938875 | 83084975 | 84157516 | 38492352 | 61971793 | 24380118 | 66199817 |
| 960 | 121217439 | 78721664 | 117498607 | 86174510 | 54890776 | 77950176 | 25884231 | 92473467 | 2730 | 96427294 | 62032720 | 82973219 | 83970747 | 35822973 | 61773658 | 24109968 | 63896612 |
| 990 | 119689559 | 78367429 | 115712520 | 84011164 | 52725908 | 75918983 | 25837336 | 91872351 | 2760 | 95160617 | 61348596 | 82696864 | 83815693 | 35662484 | 61181782 | 22506697 | 63444596 |
| 1020 | 118828214 | 77245641 | 112878856 | 81128461 | 53202010 | 76080480 | 25335940 | 89714563 | 2790 | 94289850 | 60963400 | 82153786 | 83389363 | 35660585 | 60573812 | 22166569 | 63947493 |
| 1050 | 120428518 | 76724895 | 112303381 | 80564403 | 52301600 | 73901401 | 24803954 | 89282217 | 2820 | 93315858 | 60873501 | 80815892 | 82051319 | 36021177 | 60428169 | 22068989 | 63436228 |
| 1080 | 117339882 | 75455791 | 111254118 | 79884027 | 52137789 | 74234664 | 24743484 | 88199600 | 2850 | 92786669 | 61023543 | 80110815 | 81908528 | 36058633 | 60542073 | 22181822 | 62931729 |
| 1110 | 115288544 | 74456514 | 109978423 | 78142627 | 51877076 | 73940594 | 24440233 | 86410805 | 2880 | 91942511 | 61649178 | 80214430 | 79209180 | 35516431 | 59853555 | 22047843 | 61920688 |
| 1140 | 113373592 | 75449009 | 109136081 | 77557038 | 51919351 | 73183376 | 24515029 | 85587808 | 2910 | 90996637 | 61440342 | 78170560 | 78783029 | 35476953 | 60291853 | 21461294 | 61847408 |
| 1170 | 114665693 | 74072956 | 109000816 | 76606585 | 51022260 | 73357973 | 24387934 | 85170827 | 2940 | 90203892 | 61236413 | 78621644 | 79259773 | 35292931 | 59491344 | 21242055 | 61783759 |
| 1200 | 114132828 | 73690460 | 107263956 | 76621755 | 51799575 | 73558888 | 24379387 | 84648706 | 2970 | 88967317 | 59522577 | 77093589 | 77679630 | 31896587 | 59132020 | 20063381 | 61054815 |
| 1230 | 112602759 | 72698494 | 105640142 | 76396837 | 51796705 | 71745699 | 24434965 | 83411094 | 3000 | 89291717 | 59373037 | 76814793 | 77710615 | 32059088 | 58437220 | 19139287 | 61260020 |
| 1260 | 111009652 | 71888671 | 103149178 | 74847542 | 52490882 | 72609640 | 24344763 | 81657191 | 3030 | 85905320 | 57138722 | 74497772 | 76801355 | 31903665 | 58227651 | 18884521 | 60029287 |
| 1290 | 110047755 | 71383154 | 101648986 | 74726667 | 52261727 | 70890831 | 23751970 | 80024916 | 3060 | 83868139 | 56445199 | 73730258 | 72049347 | 31416138 | 58160390 | 18212490 | 59628851 |
| 1320 | 109932718 | 70061682 | 101464739 | 74165877 | 51682935 | 70305211 | 24171020 | 80773835 | 3090 | 84509915 | 56203798 | 73352672 | 72663856 | 30509968 | 57238110 | 17955753 | 59050836 |
| 1350 | 109827085 | 68975824 | 100462007 | 75088530 | 52489112 | 69917340 | 23795008 | 79150373 | 3120 | 83289744 | 56064765 | 73214703 | 70988969 | 30276813 | 56454083 | 18143794 | 58444721 |
| 1380 | 108724271 | 67640615 | 98977468 | 74444528 | 51344046 | 70253547 | 23877421 | 77528308 | 3150 | 82387342 | 55380177 | 72158575 | 70766817 | 30484186 | 57133900 | 17538418 | 57936605 |
| 1410 | 106915024 | 68018893 | 96497904 | 75099037 | 52188667 | 69605083 | 23618713 | 75858150 | 3180 | 83372063 | 55051640 | 72237427 | 69883759 | 30496310 | 55048572 | 17445909 | 57482810 |
| 1440 | 105940641 | 67429779 | 95653785 | 75126295 | 52726840 | 68949316 | 23300886 | 74726905 | 3210 | 81361665 | 53905736 | 71459808 | 68578035 | 29221828 | 54364114 | 16820894 | 56896238 |
| 1470 | 104860405 | 66253385 | 95107743 | 76469640 | 52610902 | 69104073 | 23414560 | 74806369 | 3240 | 79401154 | 54075372 | 71002908 | 67853166 | 28678942 | 52914379 | 15896562 | 55596994 |
| 1500 | 104424521 | 65958943 | 94511582 | 76305188 | 51608752 | 68453682 | 23527429 | 74917093 | 3270 | 78124334 | 53814753 | 70726638 | 67532930 | 28257104 | 52748508 | 16098944 | 50705707 |
| 1530 | 104959292 | 65605726 | 94201167 | 76514580 | 52138960 | 67873825 | 22724363 | 74352733 | 3300 | 77913928 | 54325196 | 70929795 | 66194462 | 28003773 | 51884736 | 16125926 | 50067433 |
| 1560 | 104776748 | 65268092 | 94016753 | 77374669 | 52482829 | 68892619 | 22849883 | 73932156 | 3330 | 77248420 | 54031138 | 72262709 | 64752752 | 27911259 | 52312550 | 15962597 | 49621129 |
| 1590 | 103261499 | 64878730 | 92709198 | 78357502 | 51781084 | 67276825 | 23074659 | 73498933 | 3360 | 77019465 | 53996105 | 70077476 | 63440200 | 28027324 | 51981829 | 16051163 | 49386940 |
| 1620 | 105470523 | 65464767 | 93195444 | 80351759 | 52451967 | 66418851 | 22999513 | 72602128 | 3390 | 76017107 | 52732984 | 70286428 | 63659108 | 27282791 | 51164093 | 15928955 | 47123766 |
| 1650 | 103477466 | 64903465 | 93459882 | 79643691 | 52208404 | 65381025 | 23101788 | 71122562 | 3420 | 75050043 | 52686537 | 66847930 | 62836112 | 26788805 | 50287342 | 14257277 | 46700987 |
| 1680 | 103899492 | 64828164 | 93515113 | 80828986 | 52196717 | 66048909 | 23423746 | 70827774 | 3450 | 75002886 | 52771400 | 64338066 | 62415088 | 26402025 | 48725603 | 12215883 | 46687448 |
| 1710 | 103584921 | 64873250 | 95310568 | 83049717 | 52352457 | 65898325 | 22891952 | 70110913 | 3480 | 72914273 | 52786206 | 62942485 | 61293955 | 26206084 | 49391510 | 12059247 | 46012364 |
| 1740 | 104426757 | 65204624 | 95617227 | 83702546 | 52430985 | 65370529 | 22965109 | 69051817 | 3510 | 72474115 | 53315973 | 62755715 | 59233688 | 25394483 | 49141615 | 12005823 | 46268059 |

Fig. 38C

| t | #1 | #2 | #3 | #4 | #5 | #6 | #7 | #8 |
|---|---|---|---|---|---|---|---|---|
| 0 | 133336219 | 108191971 | 121331814 | 119456098 | 65650027 | 76613685 | 26347195 | 111362893 |
| 30 | 135226201 | 109690390 | 123405608 | 119575883 | 66922317 | 78535716 | 27765405 | 113689929 |
| 60 | 136634878 | 110642831 | 125098136 | 119812134 | 67830100 | 80176951 | 28834431 | 115430681 |
| 90 | 137634525 | 111130735 | 126458159 | 120098092 | 68423253 | 81564703 | 29610833 | 116670156 |
| 120 | 138288691 | 111226226 | 127538850 | 120370442 | 68746627 | 82724060 | 30144292 | 117483351 |
| 150 | 138652938 | 110992932 | 128378600 | 120588224 | 68840441 | 83677984 | 30478227 | 117938144 |
| 180 | 138775833 | 110488748 | 129011778 | 120711824 | 68740659 | 84447498 | 30650372 | 118086135 |
| 210 | 138699141 | 109758534 | 129487424 | 120712010 | 68479337 | 85051795 | 30693311 | 117983426 |
| 240 | 138458971 | 108844889 | 129769896 | 120567066 | 68084955 | 85508366 | 30634986 | 117671359 |
| 270 | 138088132 | 107788369 | 129939464 | 120261898 | 67582722 | 85833124 | 30499164 | 117187199 |
| 300 | 137606743 | 106619276 | 129992851 | 119787300 | 66994868 | 86040516 | 30305879 | 116562787 |
| 330 | 137042757 | 105364096 | 129943736 | 119139215 | 66340913 | 86143631 | 30071838 | 115825039 |
| 360 | 136412442 | 104048021 | 129803204 | 118318040 | 65637913 | 86154308 | 29810797 | 114996691 |
| 390 | 135730829 | 102684413 | 129580158 | 117328032 | 64900701 | 86083228 | 29533914 | 114096606 |
| 420 | 135010122 | 101295433 | 129281693 | 116176667 | 64142098 | 85940010 | 29250070 | 113140344 |
| 450 | 134260078 | 99892432.4 | 128913430 | 114874162 | 63373115 | 85733299 | 28968164 | 112140574 |
| 480 | 133488350 | 98486317.1 | 128479815 | 113432951 | 62603143 | 85470847 | 28687390 | 111107464 |
| 510 | 132700805 | 97085874.9 | 127984382 | 111867239 | 61840119 | 85159591 | 28417482 | 110049047 |
| 540 | 131901809 | 95698077.6 | 127429994 | 110192588 | 61090688 | 84805729 | 28158941 | 108971545 |
| 570 | 131094489 | 94328353 | 126819038 | 108425539 | 60360344 | 84414787 | 27913243 | 107879669 |
| 600 | 130280963 | 92980831.6 | 126153610 | 106583274 | 59653565 | 83991683 | 27681023 | 106776889 |
| 630 | 129462550 | 91658567.5 | 125435662 | 104683309 | 58973935 | 83540787 | 27462243 | 105666669 |
| 660 | 128639959 | 90303738.5 | 124667131 | 102743217 | 58324252 | 83065983 | 27256341 | 104547688 |
| 690 | 127813447 | 89097811.8 | 123850039 | 100780390 | 57708627 | 82570713 | 27062361 | 103424028 |
| 720 | 126982971 | 87861718.7 | 122986585 | 98811816.4 | 57122576 | 82058033 | 26879074 | 102295340 |
| 750 | 126148305 | 86655970.6 | 122079203 | 96853899 | 56573101 | 81530854 | 26705076 | 101161993 |
| 780 | 125309149 | 85480785.8 | 121130611 | 94922290.4 | 56058761 | 80990985 | 26538877 | 100024197 |
| 810 | 124465222 | 84336188.2 | 120143847 | 93031755.5 | 55579736 | 80441173 | 26378978 | 98882111.5 |
| 840 | 123616332 | 83222092.1 | 119122281 | 91196050.2 | 55135886 | 79883135 | 26223932 | 97735933.1 |
| 870 | 122762441 | 82138372.7 | 118069621 | 89427857.5 | 54726800 | 79318593 | 26072397 | 96585970.9 |
| 900 | 121903707 | 81084822.6 | 116989907 | 87738653 | 54351842 | 78749099 | 25923176 | 95432703.2 |
| 930 | 121040522 | 80061696.6 | 115887492 | 86138709.3 | 54010186 | 78176068 | 25775251 | 94276822.6 |
| 960 | 120173536 | 79068745.3 | 114767016 | 84637026.8 | 53700849 | 77600784 | 25627808 | 93119267.3 |
| 990 | 119303669 | 78106238.4 | 113633372 | 83241317.3 | 53422722 | 77024476 | 25480248 | 91961241.0 |
| 1020 | 118432113 | 77174478.7 | 112491664 | 81957995.1 | 53174591 | 76448236 | 25332202 | 90804226.4 |
| 1050 | 117560337 | 76273908.1 | 111347162 | 80792182.1 | 52955157 | 75873133 | 25183527 | 89649979.2 |
| 1080 | 116690065 | 75405106.5 | 110205248 | 79747724.5 | 52763051 | 75300178 | 25034306 | 88500526.7 |
| 1110 | 115823268 | 74568783.9 | 109071369 | 78827221.2 | 52596847 | 74730347 | 24884839 | 87358150 |
| 1140 | 114962134 | 73765767 | 107950970 | 78032060.9 | 52455075 | 74164589 | 24735630 | 86225363.1 |
| 1170 | 114109046 | 72996981.9 | 106849447 | 77362469.1 | 52336222 | 73603836 | 24587367 | 85104885.7 |
| 1200 | 113266548 | 72263431.7 | 105772079 | 76817562.0 | 52238745 | 73049006 | 24440909 | 83999612.3 |
| 1230 | 112437308 | 71566171.3 | 104723977 | 76395409.9 | 52161067 | 72501013 | 24297256 | 82912576.6 |
| 1260 | 111624086 | 70906280.6 | 103710023 | 76093098.8 | 52101584 | 71960763 | 24157528 | 81846913 |
| 1290 | 110829689 | 70284834.6 | 102734814 | 75906807.6 | 52058665 | 71429163 | 24022939 | 80805816.4 |
| 1320 | 110056935 | 69702872.7 | 101802610 | 75831681.1 | 52030653 | 70907115 | 23894766 | 79792499.7 |
| 1350 | 109308610 | 69161368.4 | 100917284 | 75862909.5 | 52015859 | 70395519 | 23774325 | 78810151 |
| 1380 | 108587428 | 68861197.6 | 100082276 | 75993809.5 | 52012570 | 69895269 | 23662940 | 77861890.2 |
| 1410 | 107895990 | 68203108 | 99300549.1 | 76217907.3 | 52019039 | 69407253 | 23561915 | 76950726.2 |
| 1440 | 107230744 | 67787690 | 98574552.6 | 76528022.1 | 52033480 | 68932343 | 23472509 | 76078514.5 |
| 1470 | 106611948 | 67415348.2 | 97906189 | 76916550.2 | 52054115 | 68471393 | 23395903 | 75250917 |
| 1500 | 106023634 | 67086275.5 | 97296786.7 | 77375547.9 | 52079072 | 68025252 | 23333182 | 74467363 |
| 1530 | 105473573 | 66800429 | 96747078.3 | 77896814.2 | 52106491 | 67594712 | 23285304 | 73731013 |
| 1560 | 104963244 | 66557508.5 | 96257185 | 78471971.4 | 52134466 | 67180548 | 23253083 | 73043725 |
| 1590 | 104493808 | 66356838.6 | 95826600.7 | 79082542.9 | 52161064 | 66783489 | 23237165 | 72407023.8 |
| 1620 | 104066081 | 66197853.1 | 95454219.2 | 79750029.3 | 52184326 | 66404216 | 23238014 | 71822074.6 |
| 1650 | 103680513 | 66079083.5 | 95138277.5 | 80435980.8 | 52202269 | 66043351 | 23255893 | 71289658.7 |
| 1680 | 103337173 | 65999150.8 | 94876425.3 | 81142060 | 52212891 | 65701452 | 23290851 | 70810155.2 |
| 1710 | 103035732 | 65956261.2 | 94665712.1 | 81860136.4 | 52214179 | 65379004 | 23342716 | 70383525 |
| 1740 | 102775455 | 65948305.6 | 94502615.4 | 82582287.5 | 52204115 | 65078412 | 23411086 | 70009301 |
| 1770 | 102555197 | 65972883.7 | 94383070.5 | 83300914.2 | 52180685 | 64793992 | 23495323 | 69686581.1 |
| 1800 | 102373404 | 66027211.4 | 94302606.2 | 84008762 | 52141886 | 64531966 | 23594557 | 69414027.2 |
| 1830 | 102228111 | 66108332.7 | 94255886.6 | 84698972.0 | 52085741 | 64290452 | 23707680 | 69189868 |
| 1850 | 102116958 | 66212936 | 94237758.5 | 85365124.3 | 52010309 | 64069459 | 23833361 | 69011907 |

| t | #1 | #2 | #3 | #4 | #5 | #6 | #7 | #8 |
|---|---|---|---|---|---|---|---|---|
| 1890 | 102037194 | 66337473.8 | 94242305.3 | 86001267.2 | 51913697 | 63868877 | 23970045 | 68877535.2 |
| 1920 | 101985704 | 66478166.8 | 94263405 | 86601952.2 | 51794076 | 63688478 | 24115968 | 68783748.1 |
| 1950 | 101959022 | 66631031.7 | 94294693.9 | 87162254.4 | 51649697 | 63527902 | 24269173 | 68727167.4 |
| 1980 | 101953362 | 66791912.9 | 94329634.5 | 87677791.5 | 51478904 | 63386661 | 24427525 | 68704067.6 |
| 2010 | 101964646 | 66956517.1 | 94361587.1 | 88144735.5 | 51280154 | 63264126 | 24588728 | 68710406.2 |
| 2040 | 101988537 | 67120452.2 | 94383885.4 | 88559818.8 | 51052032 | 63159532 | 24750353 | 68741858.3 |
| 2070 | 102020480 | 67279267.6 | 94389914.3 | 88920335.4 | 50793271 | 63071969 | 24909861 | 68793855 |
| 2100 | 102055736 | 67428499.2 | 94373189.8 | 89224135.6 | 50502770 | 63000386 | 25064626 | 68861625.4 |
| 2130 | 102089436 | 67563714.6 | 94327441.6 | 89469616 | 50179610 | 62943585 | 25211970 | 68940241.8 |
| 2160 | 102116617 | 67680562.1 | 94246694.7 | 89655703.8 | 49823078 | 62900226 | 25349192 | 69024667.4 |
| 2190 | 102132281 | 67774820.2 | 94125352.1 | 89781836.3 | 49432679 | 62868827 | 25473597 | 69109807.5 |
| 2220 | 102131441 | 67842448.5 | 93958276.5 | 89847936.5 | 49008155 | 62847768 | 25582536 | 69190561.3 |
| 2250 | 102109176 | 67879639 | 93740869.8 | 89854381.9 | 48549506 | 62835292 | 25673434 | 69261876.6 |
| 2280 | 102060685 | 67882868.3 | 93469150.2 | 89801972 | 48057000 | 62829515 | 25743832 | 69318804.9 |
| 2310 | 101981345 | 67848948.1 | 93139825.6 | 89691890.5 | 47531191 | 62828427 | 25791415 | 69356557.1 |
| 2340 | 101866762 | 67775075.6 | 92750362 | 89525664.6 | 46972931 | 62829907 | 25814052 | 69370559.2 |
| 2370 | 101712830 | 67658882.1 | 92299046.2 | 89305121.3 | 46383379 | 62831726 | 25809828 | 69356507.1 |
| 2400 | 101515780 | 67498478.8 | 91785041.7 | 89032341.3 | 45764013 | 62831564 | 25777081 | 69310421 |
| 2430 | 101272260 | 67292499.9 | 91208435.6 | 88709611.5 | 45116634 | 62827017 | 25714425 | 69228696.2 |
| 2460 | 100979327 | 67040142.2 | 90570277.5 | 88339375.6 | 44443365 | 62815617 | 25620789 | 69108152.3 |
| 2490 | 100634556 | 66741199.5 | 89872606.1 | 87924184.7 | 43748658 | 62794843 | 25495439 | 68940078.9 |
| 2520 | 100236055 | 66396092 | 89118464.6 | 87466647.2 | 43029284 | 62762143 | 25338000 | 68740275.8 |
| 2550 | 99782508.7 | 66005889.6 | 88311903.2 | 86969379.9 | 42284325 | 62714949 | 25148478 | 68489089.3 |
| 2580 | 99273216 | 65572327.9 | 87457964.1 | 86434960.3 | 41545163 | 62650703 | 24927276 | 68191441.9 |
| 2610 | 98708118 | 65097816.2 | 86562653.6 | 85865880.7 | 40785457 | 62566875 | 24675200 | 67846854.5 |
| 2640 | 98087820.8 | 64585436.8 | 85632894 | 85264505.8 | 40019124 | 62460993 | 24393470 | 67455461.4 |
| 2670 | 97413610 | 64038933.8 | 84676456 | 84633003.6 | 39250305 | 62330666 | 24083700 | 67018015.6 |
| 2700 | 96687457.3 | 63462691.9 | 83701870.6 | 83973461.4 | 38483329 | 62173615 | 23747943 | 66535884.8 |
| 2730 | 95912016.5 | 62861701.8 | 82718317.8 | 83287556.7 | 37722668 | 61987702 | 23388575 | 66011035.7 |
| 2760 | 95090610.3 | 62241513.8 | 81735490.6 | 82576835.1 | 36972869 | 61770965 | 23008306 | 65446006.2 |
| 2790 | 94227204 | 61608176.2 | 80763432.2 | 81842545.4 | 36238587 | 61521651 | 22610396 | 64843864.3 |
| 2820 | 93326368 | 60968158.9 | 79812345.4 | 81085663.3 | 35524322 | 61238253 | 22198019 | 64208152.5 |
| 2850 | 92393225.7 | 60328259.7 | 78892371.5 | 80306894.9 | 34834537 | 60919551 | 21774805 | 63542816.2 |
| 2880 | 91433386.7 | 59695403.2 | 78013330.9 | 79506690.2 | 34173473 | 60564651 | 21344476 | 62852115 |
| 2910 | 90452864.3 | 59076960.7 | 77184465.9 | 78685268.9 | 33545065 | 60173030 | 20910815 | 62140516.2 |
| 2940 | 89457975.3 | 58479698.6 | 76414056.7 | 77842659.3 | 32952835 | 59744580 | 20477579 | 61412567.4 |
| 2970 | 88455221.8 | 57910506.4 | 75709119.5 | 76978750.8 | 32399767 | 59279656 | 20048385 | 60672749.1 |
| 3000 | 87451152.5 | 57375750.3 | 75074973.7 | 76093362.2 | 31888176 | 58779127 | 19626581 | 59925304.4 |
| 3030 | 86452205.1 | 56881142.7 | 74514603.2 | 75186326.7 | 31419553 | 58244429 | 19215107 | 59174044.4 |
| 3060 | 85464522.9 | 56431495.6 | 74029165.4 | 74257595.2 | 30994407 | 57677615 | 18816332 | 58422128.5 |
| 3090 | 84493751.1 | 56030446.2 | 73615454.1 | 73307358.1 | 30612087 | 57081417 | 18431873 | 57671817.4 |
| 3120 | 83544805.6 | 55680153.2 | 73267312.4 | 72336189.1 | 30270585 | 56459304 | 18062398 | 56924197.8 |
| 3150 | 82621616.7 | 55380963.1 | 72973993.3 | 71345210.4 | 29966331 | 55815540 | 17707401 | 56178876.6 |
| 3180 | 81726844.2 | 55131043.4 | 72719660.1 | 70336282.2 | 29693965 | 55155252 | 17364962 | 55433643.2 |
| 3210 | 80361562.7 | 54925982.4 | 72482665.6 | 69312216.6 | 29446089 | 54484494 | 17031478 | 54684098.8 |
| 3240 | 80024916.8 | 54758352.9 | 72234681.9 | 68277019.8 | 29213009 | 53810314 | 16701373 | 53923249.3 |
| 3270 | 79213741.7 | 54617238.9 | 71939888.2 | 67236160.5 | 28982447 | 53140828 | 16366779 | 53141062.8 |
| 3300 | 78422153.3 | 54487723.5 | 71554003.1 | 66196871.3 | 28739242 | 52485292 | 16017183 | 52323986.5 |
| 3330 | 77641097.5 | 54350334.9 | 71023286 | 65168478.8 | 28465022 | 51854174 | 15639107 | 51454424.1 |
| 3360 | 76857866.1 | 54180451 | 70283462.1 | 64162768.8 | 28137857 | 51259240 | 15215599 | 50510170.3 |
| 3390 | 76055571.6 | 53947658.4 | 69258573.9 | 63194386.7 | 27731891 | 50713624 | 14725905 | 49463800.5 |
| 3420 | 75212580.6 | 53015065.6 | 67859757.1 | 62281273.8 | 27216946 | 50231918 | 14144955 | 48282014.9 |
| 3450 | 74301905.9 | 53138567.3 | 65983937.6 | 61445143.8 | 26558104 | 49830255 | 13442866 | 46924933 |
| 3480 | 73290553 | 52466059.1 | 63512446.3 | 60711998.6 | 25715259 | 49526396 | 12584417 | 45345338.5 |
| 3510 | 72138820.3 | 51536599.2 | 60309549.7 | 60112687.1 | 24642652 | 49339816 | 11526473 | 43487871.8 |

Fig. 38d1

| t | #1 | first order diff. | second order diff. | #2 | first order diff. | second order diff. | #3 | first order diff. | second order diff. |
|---|---|---|---|---|---|---|---|---|---|
| 0 | 133336219 | | | 108191970.6 | | | 121331814.2 | | |
| 30 | 135226201.4 | 3779964.709 | | 109690389.5 | 2996837.838 | | 123405608.5 | 4147588.535 | |
| 60 | 136634878.2 | 2817353.632 | -1925222.153 | 110642831.3 | 1904883.526 | -2183908.623 | 125096135.7 | 3381054.489 | -1533068.092 |
| 90 | 137634524.9 | 1999293.419 | -1636120.428 | 111130735.2 | 975807.8942 | -1858151.264 | 126458159 | 2724046.631 | -1314015.716 |
| 120 | 138288690.5 | 1308331.181 | -1381924.475 | 111226226.3 | 190982.2501 | -1569651.288 | 127538849.8 | 2161381.438 | -1125330.386 |
| 150 | 138652987.6 | 728594.0902 | -1159474.182 | 110992932 | -466588.7574 | -1315142.015 | 128378600.2 | 1679500.965 | -963760.946 |
| 180 | 138775832.9 | 245690.6882 | -965806.8039 | 110486747.7 | -1012368.623 | -1091559.731 | 129011778 | 1266355.545 | -826290.8394 |
| 210 | 138699140.9 | -153384.0006 | -798149.3777 | 109756554.4 | -1460386.522 | -896035.7984 | 129467423.8 | 911291.5695 | -710127.9508 |
| 240 | 138458971.1 | -480339.6401 | -653911.279 | 108844888.9 | -1823330.974 | -725888.9045 | 129769895.9 | 604944.2487 | -612694.6415 |
| 270 | 138086132 | -745678.0961 | -530676.9119 | 107788569.1 | -2112639.705 | -578617.4621 | 129939463.5 | 339135.254 | -531617.9896 |
| 300 | 137606743.4 | -958777.3632 | -426198.5343 | 106619276.2 | -2338585.78 | -451892.1498 | 129992851.1 | 106775.1379 | -464720.232 |
| 330 | 137042757.4 | -1127971.974 | -338389.2208 | 105364096.1 | -2510360.079 | -343548.5982 | 129943736.3 | -98229.56606 | -410009.408 |
| 360 | 136412442.4 | -1260629.954 | -265315.9602 | 104046021 | -2636150.19 | -251580.221 | 129803204 | -281064.6686 | -365670.2051 |
| 390 | 135730829.2 | -1363226.398 | -205192.8895 | 102684413.1 | -2723215.785 | -174131.19 | 129580157.9 | -446092.1719 | -330055.0066 |
| 420 | 135010122.3 | -1441413.73 | -156374.6638 | 101295432.9 | -2777960.563 | -109489.5554 | 129281693 | -596929.7427 | -301675.1415 |
| 450 | 134260078 | -1500088.711 | -117349.9621 | 99892432.44 | -2806000.818 | -56080.50967 | 128913430.1 | -736525.9107 | -279192.3362 |
| 480 | 133488349.8 | -1543456.276 | -86735.12887 | 98486317.08 | -2812230.716 | -12459.79672 | 128479814.5 | -867231.0946 | -261410.3678 |
| 510 | 132700804.7 | -1575090.252 | -63267.95176 | 97085874.91 | -2800884.349 | 22692.7346 | 127984382.3 | -990864.5547 | -247266.9202 |
| 540 | 131901809.2 | -1597991.039 | -45801.57523 | 95698077.59 | -2775594.632 | 50579.43261 | 127429993.6 | -1108777.375 | -235825.6415 |
| 570 | 131094489 | -1614640.314 | -33298.54999 | 94328353.03 | -2739449.132 | 72291.00021 | 126819037.8 | -1221911.577 | -226268.4036 |
| 600 | 130280962.6 | -1627052.824 | -24825.01844 | 92980831.59 | -2695042.878 | 88812.50787 | 126153610.1 | -1330855.459 | -217887.7638 |
| 630 | 129462549.9 | -1636825.342 | -19545.03602 | 91658567.47 | -2644528.247 | 101029.2619 | 125435662.4 | -1435895.273 | -210079.6284 |
| 660 | 128639958.5 | -1645182.856 | -16715.02856 | 90363736.47 | -2589661.983 | 109732.5286 | 124667130.8 | -1537063.332 | -202336.1182 |
| 690 | 127813447.5 | -1653022.049 | -15678.38544 | 89097811.76 | -2531849.426 | 115625.1131 | 123850039.4 | -1634182.65 | -194238.6361 |
| 720 | 126982971.4 | -1660952.143 | -15860.18883 | 87861718.75 | -2472186.029 | 119326.7946 | 122986585.3 | -1726908.219 | -185451.1366 |
| 750 | 126148304.8 | -1669333.182 | -16762.07872 | 86655970.64 | -2411496.221 | 121379.6165 | 122079202.8 | -1814765.017 | -175713.597 |
| 780 | 125309148.9 | -1678311.809 | -17957.25398 | 85480785.78 | -2350369.705 | 122253.0317 | 121130611.4 | -1897182.863 | -164835.6914 |
| 810 | 124465221.6 | -1687854.614 | -19085.60932 | 84336188.16 | -2289195.253 | 122348.9046 | 120143847.3 | -1973528.196 | -152690.6656 |
| 840 | 123616332.1 | -1697779.118 | -19849.00815 | 83222092.12 | -2228192.069 | 122006.3669 | 119122280.8 | -2043132.903 | -139209.4149 |
| 870 | 122762440.8 | -1707782.464 | -20006.69144 | 82138372.72 | -2167438.804 | 121506.5302 | 118069620.7 | -2105320.285 | -124374.7634 |
| 900 | 121903706.9 | -1717467.875 | -19370.82247 | 81084922.58 | -2106900.277 | 121077.0533 | 116989906.6 | -2159428.258 | -108215.9453 |
| 930 | 121040522.4 | -1726368.959 | -17802.16748 | 80061696.58 | -2046451.995 | 120896.5656 | 115887491.6 | -2204829.902 | -90803.28823 |
| 960 | 120173536.5 | -1733971.915 | -15205.91235 | 79068745.32 | -1985902.522 | 121098.9448 | 114767015.9 | -2240951.451 | -72243.09891 |
| 990 | 119303668.6 | -1739735.723 | -11527.61509 | 78106238.43 | -1925013.796 | 121777.452 | 113633372 | -2267287.826 | -52672.75009 |
| 1020 | 118432113.4 | -1743110.37 | -6749.294368 | 77174478.71 | -1863519.436 | 122988.7201 | 112491664.1 | -2283415.811 | -32255.97009 |
| 1050 | 117560336.8 | -1743553.197 | -885.653907 | 76273908.14 | -1801141.137 | 124756.599 | 111347161.6 | -2289004.978 | -11178.33398 |
| 1080 | 116690065.1 | -1740543.418 | 6019.557164 | 75405106.53 | -1737603.208 | 127075.8563 | 110205248.3 | -2283826.457 | 10357.04309 |
| 1110 | 115823267.7 | -1733594.894 | 13897.04804 | 74568783.86 | -1672645.342 | 129915.7326 | 109071368.5 | -2267759.651 | 32133.6107 |
| 1140 | 114962134 | -1722267.217 | 22655.35399 | 73765767.03 | -1606033.666 | 133223.3529 | 107950970 | -2240797.008 | 53925.28589 |
| 1170 | 114109046.5 | -1706175.183 | 32184.06827 | 72996981.95 | -1537570.169 | 136926.9935 | 106849446.5 | -2203046.931 | 75500.15398 |
| 1200 | 113266548.1 | -1684996.714 | 42356.93801 | 72263431.66 | -1467100.567 | 140939.2041 | 105772079.1 | -2154734.948 | 96623.96736 |
| 1230 | 112437308.4 | -1658479.302 | 53034.82421 | 71566171.33 | -1394520.674 | 145159.7854 | 104723977.5 | -2096203.227 | 117063.4424 |
| 1260 | 111624085.9 | -1626445.039 | 64068.52579 | 70906280.64 | -1319781.363 | 149478.622 | 103710023.2 | -2027908.549 | 136589.3541 |
| 1290 | 110829688.8 | -1588794.305 | 75301.46775 | 70284834.56 | -1242892.177 | 153778.3717 | 102734813.8 | -1950418.835 | 154979.4293 |
| 1320 | 110056934.7 | -1545508.178 | 86572.25334 | 69702872.72 | -1163923.673 | 157937.0086 | 101802609.6 | -1864408.316 | 172021.0372 |
| 1350 | 109308609.9 | -1496649.638 | 97717.08031 | 69161368.44 | -1083008.561 | 161830.2233 | 100917283.9 | -1770651.477 | 187513.6787 |
| 1380 | 108587428.1 | -1442363.628 | 108572.0213 | 68661197.58 | -1000341.723 | 165333.6776 | 100082276 | -1670015.84 | 201271.2728 |

Fig. 38d2

| t | #1 | first order diff. | second order diff. | #2 | first order diff. | second order diff. | #3 | first order diff. | second order diff. |
|---|---|---|---|---|---|---|---|---|---|
| 1410 | 107895990 | -1382876.044 | 118975.1681 | 68203107.99 | -916179.1651 | 168325.115 | 99300549.09 | -1563453.72 | 213124.242 |
| 1440 | 107236744.2 | -1318491.723 | 128768.6404 | 67787689.99 | -830836.0019 | 170686.3264 | 98574552.58 | -1451993.022 | 222921.3951 |
| 1470 | 106611948.4 | -1249591.494 | 137800.4579 | 67415348.23 | -744683.5161 | 172304.9716 | 97906188.97 | -1336727.218 | 230531.6082 |
| 1500 | 106023634.2 | -1176628.356 | 145926.2774 | 67086275.54 | -658145.3879 | 173076.2565 | 97296786.69 | -1218804.566 | 235845.3038 |
| 1530 | 105473572.8 | -1100122.859 | 153010.993 | 66800428.96 | -571693.1556 | 172904.4644 | 96747078.34 | -1099416.702 | 238775.7276 |
| 1560 | 104963243.9 | -1020657.759 | 158930.2011 | 66557508.47 | -485840.9832 | 171704.3449 | 96257184.99 | -979786.6902 | 239260.0239 |
| 1590 | 104493807.9 | -938871.9939 | 163571.5294 | 66356938.57 | -401139.8051 | 169402.3562 | 95826606.68 | -861156.6361 | 237260.1083 |
| 1620 | 104066080.9 | -855454.0793 | 166835.8293 | 66197853.11 | -318170.9229 | 165937.7644 | 95454219.19 | -744774.9666 | 232763.3391 |
| 1650 | 103680513.4 | -771134.9628 | 168638.233 | 66079083.55 | -237539.1244 | 161263.5969 | 95138277.46 | -631883.4735 | 225782.9862 |
| 1680 | 103337173.2 | -686680.4253 | 168909.075 | 65999150.85 | -159865.3979 | 155347.453 | 94876425.34 | -523704.2243 | 216358.4984 |
| 1710 | 103035731.7 | -602883.0872 | 167594.6763 | 65956261.19 | -85779.31383 | 148172.1681 | 94665712.12 | -421426.44 | 204555.5685 |
| 1740 | 102775454.6 | -520554.0901 | 164657.9942 | 65948305.62 | -15911.14655 | 139736.3346 | 94502615.4 | -326193.4418 | 190465.9964 |
| 1770 | 102555197.4 | -440514.5223 | 160079.1355 | 65972863.71 | 49116.19228 | 130054.6777 | 94383070.52 | -239089.7667 | 174207.3502 |
| 1800 | 102373404 | -363586.6556 | 153855.7334 | 66027211.38 | 108695.3358 | 119158.287 | 94302506.24 | -161128.5537 | 155922.4259 |
| 1830 | 102228111.5 | -290585.061 | 146003.1891 | 66108332.72 | 162242.6876 | 107094.7036 | 94255886.59 | -93239.30177 | 135778.504 |
| 1860 | 102116957.7 | -222307.6724 | 136554.7773 | 66212936.03 | 209206.6186 | 93927.86201 | 94237758.54 | -36256.09927 | 113966.405 |
| 1890 | 102037194.2 | -159526.8648 | 125561.6153 | 66337473.81 | 249075.5627 | 79737.88817 | 94242305.33 | 9093.572214 | 90699.34297 |
| 1920 | 101985703.9 | -102980.6163 | 113092.497 | 66478166.78 | 281385.9392 | 64620.75292 | 94263405.01 | 42199.36041 | 66211.57638 |
| 1950 | 101959022 | -53363.8211 | 99233.59035 | 66631031.7 | 305729.8295 | 48687.7806 | 94294693.9 | 62577.78927 | 40756.85773 |
| 1980 | 101953362.1 | -11319.8216 | 84087.99899 | 66791912.87 | 321762.3361 | 32065.01333 | 94329634.47 | 69881.12961 | 14606.68067 |
| 2010 | 101964646 | 22567.77242 | 67775.18804 | 66956517.14 | 329208.5514 | 14892.43054 | 94361587.11 | 63905.29236 | -11951.67449 |
| 2040 | 101988537.4 | 47782.90936 | 50430.27388 | 67120452.17 | 327870.0636 | -2676.975533 | 94383885.44 | 44596.64363 | -38617.29746 |
| 2070 | 102020479.7 | 63884.49843 | 32203.17814 | 67279267.64 | 317630.9288 | -20478.26966 | 94389914.26 | 12057.64031 | -65078.00665 |
| 2100 | 102055736.3 | 70513.32123 | 13257.64559 | 67428499.16 | 298463.0352 | -38335.78719 | 94373189.85 | -33448.8145 | -91012.90962 |
| 2130 | 102089435.5 | 67398.3843 | -6229.87385 | 67563714.55 | 270430.7888 | -56064.49273 | 94327441.65 | -91496.3974 | -116095.1658 |
| 2160 | 102116616.9 | 54362.64486 | -26071.47888 | 67680562.07 | 233695.0473 | -73471.483 | 94246694.71 | -161493.8728 | -139994.9508 |
| 2190 | 102132280.9 | 31328.04143 | -46069.20687 | 67774820.19 | 188516.23 | -90357.63459 | 94125352.12 | -242685.1845 | -162382.6234 |
| 2220 | 102131440.8 | -1680.238304 | -66016.55947 | 67842448.46 | 135256.5322 | -106519.3957 | 93958276.5 | -334151.2313 | -182932.0935 |
| 2250 | 102109175.6 | -44530.32045 | -85700.16429 | 67879639.04 | 74381.17071 | -121750.723 | 93740869.79 | -434813.4277 | -201324.3929 |
| 2280 | 102060685.1 | -96981.10681 | -104901.5727 | 67882868.34 | 6458.589321 | -135845.1628 | 93469150.22 | -543439.1517 | -217251.4479 |
| 2310 | 101981344.7 | -158680.7034 | -123399.1932 | 67848948.11 | -67840.44894 | -148598.0765 | 93139825.63 | -658649.1783 | -230420.0532 |
| 2340 | 101866761.8 | -229165.884 | -140970.3612 | 67775075.63 | -147744.9545 | -159809.0111 | 92750362.02 | -778927.2029 | -240556.0493 |
| 2370 | 101712830.4 | -307862.656 | -157393.5439 | 67658882.1 | -232387.0614 | -169284.2138 | 92299046.25 | -902631.5529 | -247408.6999 |
| 2400 | 101515786.4 | -394087.9971 | -172450.6823 | 67498478.75 | -320806.707 | -176839.2911 | 91785041.65 | -1028009.189 | -250755.2727 |
| 2430 | 101272260 | -487052.8312 | -185929.6681 | 67292499.89 | -411957.7136 | -182302.0132 | 91208435.6 | -1153212.1 | -250405.822 |
| 2460 | 100979326.9 | -585866.3098 | -197626.9572 | 67040142.22 | -504715.3445 | -185515.2618 | 90570277.51 | -1276316.187 | -246208.1728 |
| 2490 | 100634556.1 | -689541.4689 | -207350.3183 | 66741199.52 | -597885.4061 | -186340.1232 | 89872606.14 | -1395342.74 | -238053.1069 |
| 2520 | 100236055 | -797002.3282 | -214921.7186 | 66396092.03 | -690214.9691 | -184659.126 | 89118464.83 | -1508282.616 | -225879.7518 |
| 2550 | 99782508.71 | -907092.5006 | -220180.3448 | 66005889.64 | -780404.7803 | -180379.6224 | 88311903.23 | -1613123.201 | -209681.1705 |
| 2580 | 99273216.02 | -1018585.38 | -222985.7595 | 65572327.92 | -867123.4382 | -173437.3158 | 87457964.09 | -1707878.278 | -189510.1539 |
| 2610 | 98708118.03 | -1130195.978 | -223221.1945 | 65097816.22 | -949023.4036 | -163799.9307 | 86562653.65 | -1790620.886 | -165485.2152 |
| 2640 | 98087820.8 | -1240594.467 | -220796.9798 | 64585436.76 | -1024758.918 | -151471.0292 | 85632894.01 | -1859519.279 | -137796.7858 |
| 2670 | 97413610.04 | -1348421.521 | -215654.1078 | 64038933.81 | -1093005.903 | -136493.9704 | 84676455.97 | -1912876.086 | -106713.6141 |
| 2700 | 96687457.29 | -1452305.489 | -207767.9345 | 63462691.86 | -1152483.911 | -118956.0158 | 83701870.58 | -1949170.768 | -72589.36477 |
| 2730 | 95912016.55 | -1550881.496 | -197152.0156 | 62861701.76 | -1201980.2 | -98992.5777 | 82718317.84 | -1967105.479 | -35869.4218 |
| 2760 | 95090610.28 | -1642812.536 | -183862.0799 | 62241513.75 | -1240376.007 | -76791.6139 | 81735490.63 | -1965654.425 | 2902.107452 |
| 2790 | 94227203.97 | -1726812.605 | -168000.1364 | 61608176.21 | -1266675.09 | -52598.165 | 80763432.22 | -1944116.832 | 43075.18718 |

Fig. 38d3

| t | #1 | first order diff. | second order diff. | #2 | first order diff. | second order diff. | #3 | first order diff. | second order diff. |
|---|---|---|---|---|---|---|---|---|---|
| 2820 | 93326367. 99 | -1801671. 965 | -149718. 7205 | 60968158. 9 | -1280034. 608 | -26719. 03767 | 79812345. 41 | -1902173. 61 | 83886. 44313 |
| 2850 | 92393225. 69 | -1866284. 601 | -129225. 2726 | 60328259. 69 | -1279798. 425 | 472. 3673973 | 78892371. 49 | -1839947. 833 | 124451. 5545 |
| 2880 | 91433386. 73 | -1919677. 929 | -106786. 6554 | 59695493. 25 | -1265532. 882 | 28531. 08512 | 78013336. 94 | -1758069. 113 | 163757. 4407 |
| 2910 | 90452864. 31 | -1961044. 832 | -82733. 80613 | 59076960. 68 | -1237065. 148 | 56935. 4679 | 77184465. 94 | -1657741. 989 | 200654. 2472 |
| 2940 | 89457975. 26 | -1989778. 094 | -57466. 52381 | 58479698. 58 | -1194524. 185 | 85081. 92588 | 76414056. 73 | -1540818. 424 | 233847. 1301 |
| 2970 | 88455221. 62 | -2005507. 291 | -31458. 39476 | 57910506. 37 | -1138384. 425 | 112279. 5205 | 75709119. 48 | -1409874. 505 | 261887. 8384 |
| 3000 | 87451152. 51 | -2008138. 217 | -5261. 850876 | 57375750. 26 | -1069512. 217 | 137744. 4161 | 75074973. 75 | -1268291. 459 | 283166. 0922 |
| 3030 | 86452205. 06 | -1997894. 9 | 20486. 63301 | 56881142. 7 | -989215. 1257 | 160594. 1825 | 74514803. 21 | -1120341. 077 | 295900. 7636 |
| 3060 | 85464522. 91 | -1975364. 296 | 45061. 20765 | 56431495. 63 | -899294. 1463 | 179841. 9589 | 74029165. 38 | -971275. 6518 | 298130. 8499 |
| 3090 | 84493751. 06 | -1941543. 705 | 67641. 18218 | 56030446. 17 | -802098. 9119 | 194390. 4687 | 73615454. 12 | -827422. 527 | 287706. 2497 |
| 3120 | 83544805. 56 | -1897890. 995 | 87305. 41976 | 55680153. 19 | -700585. 9665 | 203025. 8909 | 73267312. 44 | -696283. 3602 | 262278. 3336 |
| 3150 | 82621616. 71 | -1846377. 696 | 103026. 598 | 55380963. 1 | -598380. 1722 | 204411. 5886 | 72973993. 34 | -586638. 2023 | 219290. 3158 |
| 3180 | 81726844. 2 | -1789545. 03 | 113665. 3332 | 55131043. 44 | -499839. 3267 | 197081. 691 | 72719666. 09 | -508654. 4922 | 155967. 4201 |
| 3210 | 80861562. 73 | -1730562. 945 | 117964. 1698 | 54925982. 41 | -410122. 0608 | 179434. 5318 | 72482665. 56 | -474001. 0678 | 69306. 84886 |
| 3240 | 80024916. 61 | -1673292. 229 | 114541. 4316 | 54758352. 86 | -335259. 0896 | 149725. 9423 | 72234681. 91 | -495967. 2957 | -43932. 45587 |
| 3270 | 79213741. 73 | -1622349. 76 | 101884. 9388 | 54617238. 92 | -282227. 8886 | 106062. 4021 | 71939888. 2 | -589587. 4194 | -187240. 2474 |
| 3300 | 78422153. 25 | -1583176. 965 | 78345. 58955 | 54487723. 48 | -259030. 8679 | 46394. 04139 | 71554003. 09 | -771770. 2278 | -364365. 6167 |
| 3330 | 77641097. 47 | -1562111. 563 | 42130. 80318 | 54350334. 93 | -274777. 1166 | -31492. 49741 | 71023286. 02 | -1061434. 144 | -579327. 8317 |
| 3360 | 76857866. 14 | -1566462. 648 | -8702. 169968 | 54180451. 03 | -339767. 7884 | -129981. 3438 | 70283462. 1 | -1479647. 835 | -836427. 3836 |
| 3390 | 76055571. 55 | -1604589. 185 | -76253. 07448 | 53947658. 43 | -465585. 2029 | -251634. 8289 | 69258573. 87 | -2049776. 45 | -1140257. 229 |
| 3420 | 75212580. 55 | -1685981. 994 | -162785. 6177 | 53615065. 56 | -665185. 7334 | -399201. 0609 | 67859757. 09 | -2797633. 572 | -1495714. 244 |
| 3450 | 74301905. 92 | -1821349. 275 | -270734. 5615 | 53138567. 29 | -952996. 553 | -575621. 6393 | 65983937. 59 | -3751639. 004 | -1908010. 865 |
| 3480 | 73290553. 04 | -2022705. 757 | -402712. 9635 | 52466059. 13 | -1345016. 313 | -784039. 5198 | 63512446. 35 | -4942982. 478 | -2382686. 948 |
| 3510 | 72138820. 27 | -2303465. 529 | -561519. 5448 | 51536599. 22 | -1858919. 824 | -1027807. 022 | 60309549. 65 | -6405793. 388 | -2925621. 819 |

Fig. 38d4

| t | #4 | first order diff. | second order diff. | #5 | first order diff. | second order diff. | #6 | first order diff. | second order diff. |
|---|---|---|---|---|---|---|---|---|---|
| 0 | 119456098.4 | | | 65650027.48 | | | 76613685.07 | | |
| 30 | 119575882.8 | 239568.7252 | | 66922317.31 | 2544579.665 | | 78535715.5 | 3844060.868 | |
| 60 | 119812133.6 | 472501.6323 | 465865.8141 | 67830100.02 | 1815565.42 | -1458028.491 | 80176950.84 | 3282470.685 | -1123180.367 |
| 90 | 120096092.3 | 567917.5286 | 190831.7926 | 68423253.11 | 1186306.188 | -1258518.463 | 81564707.52 | 2775513.358 | -1013914.653 |
| 120 | 120370441.6 | 548698.4435 | -38438.17017 | 68746626.92 | 646747.6077 | -1079117.161 | 82724059.57 | 2318704.102 | -913618.5127 |
| 150 | 120588223.6 | 435564.1422 | -226268.6028 | 68840441.37 | 187628.9028 | -918237.4097 | 83677983.78 | 1907848.404 | -821711.3958 |
| 180 | 120711823.9 | 247200.6316 | -376727.0211 | 68740659.33 | -199564.069 | -774385.9437 | 84447498.33 | 1539029.098 | -737638.6117 |
| 210 | 120712016 | 384.0870195 | -493633.0891 | 68479337.36 | -522643.9475 | -646159.757 | 85051795.21 | 1208593.76 | -660870.6755 |
| 240 | 120567066.1 | -289899.7267 | -580567.6274 | 68084954.76 | -788765.211 | -532242.5271 | 85508366.42 | 913142.4307 | -590902.6595 |
| 270 | 120261895.9 | -610340.4611 | -640881.4688 | 67582721.87 | -1004465.764 | -431401.1059 | 85833124.25 | 649515.6555 | -527253.5503 |
| 300 | 119787299.6 | -949192.5425 | -677704.1628 | 66994868.47 | -1175706.803 | -342482.0787 | 86040515.67 | 414782.8492 | -469465.6126 |
| 330 | 119139215.2 | -1296168.806 | -693952.5265 | 66340912.97 | -1307911 | -264408.3928 | 86143631.16 | 206230.9696 | -417103.7591 |
| 360 | 118318046.1 | -1642338.328 | -692339.0437 | 65637913.46 | -1405999.026 | -196176.0533 | 86154307.91 | 21353.5066 | -369754.9261 |
| 390 | 117328031.9 | -1980028.383 | -675380.1118 | 64900701.22 | -1474424.471 | -136850.8893 | 86083227.8 | -142160.2211 | -327027.4555 |
| 420 | 116176666.6 | -2302730.452 | -645404.1363 | 64142097.64 | -1517207.164 | -85565.38634 | 85940010.07 | -286435.4627 | -288550.4831 |
| 450 | 114874161.5 | -2605010.188 | -604559.4729 | 63373115.16 | -1537964.959 | -41515.5896 | 85733299 | -413422.129 | -253973.3327 |
| 480 | 113432950.9 | -2882421.297 | -554822.217 | 62603143.16 | -1539943.996 | -3958.074053 | 85470846.71 | -524904.5872 | -222964.9163 |
| 510 | 111867239.3 | -3131423.217 | -498003.8417 | 61840119.42 | -1526047.488 | 27793.01605 | 85159591.13 | -622511.1577 | -195213.1411 |
| 540 | 110192588 | -3349302.559 | -435758.6825 | 61090687.89 | -1498863.058 | 54368.85941 | 84805729.47 | -707723.3183 | -170424.3212 |
| 570 | 108425538.9 | -3534098.194 | -369591.2704 | 60360343.56 | -1460688.668 | 76348.78129 | 84414787.16 | -781884.617 | -148322.5973 |
| 600 | 106583273.9 | -3684529.95 | -300863.512 | 59653564.97 | -1413557.163 | 94263.00864 | 83991682.52 | -846209.2973 | -128649.3607 |
| 630 | 104683308.5 | -3799930.809 | -230801.7184 | 58973935.23 | -1359259.485 | 108595.3566 | 83540787.2 | -901790.6398 | -111162.6849 |
| 660 | 102743217.3 | -3880182.549 | -160503.4804 | 58324251.95 | -1299366.562 | 119785.8467 | 83065982.69 | -949609.0209 | -95636.76227 |
| 690 | 100780389.9 | -3925654.745 | -90944.39221 | 57706626.98 | -1235249.934 | 128233.256 | 82570712.84 | -990539.6946 | -81861.3473 |
| 720 | 98811816.36 | -3937147.057 | -22984.62282 | 57122576.42 | -1168101.135 | 134297.5983 | 82058032.69 | -1025360.298 | -69641.20613 |
| 750 | 96853898.99 | -3915834.724 | 42624.66525 | 56573101.48 | -1098949.866 | 138302.5364 | 81530653.65 | -1054758.084 | -58795.57183 |
| 780 | 94922290.39 | -3863217.2 | 105235.0494 | 56058760.98 | -1028681.003 | 140537.7261 | 80990985.2 | -1079336.887 | -49157.606 |
| 810 | 93031755.48 | -3781069.833 | 164294.7327 | 55579735.75 | -958050.4576 | 141261.0913 | 80441173.29 | -1099623.82 | -40573.86646 |
| 840 | 91196056.21 | -3671398.542 | 219342.5819 | 55135885.78 | -887699.9422 | 140701.0309 | 79883135.44 | -1116075.71 | -32903.78108 |
| 870 | 89427857.51 | -3536397.383 | 270002.3179 | 54726800.45 | -818170.6637 | 139058.5569 | 79318592.8 | -1129085.274 | -26019.12768 |
| 900 | 87738653.04 | -3378408.954 | 315976.8585 | 54351842.46 | -749915.9816 | 136509.3643 | 78749099.29 | -1138987.034 | -19803.52012 |
| 930 | 86138709.26 | -3199887.547 | 357042.8136 | 54010185.93 | -683313.0656 | 133205.832 | 78176067.79 | -1146062.984 | -14151.90046 |
| 960 | 84637026.77 | -3003364.981 | 393045.1322 | 53700849.13 | -618673.5878 | 129278.9556 | 77600793.79 | -1150548.003 | -8970.037247 |
| 990 | 83241317.26 | -2791419.03 | 423891.9024 | 53422722.39 | -556253.4821 | 124840.2113 | 77024476.28 | -1152635.018 | -4174.029967 |
| 1020 | 81957995.07 | -2566644.378 | 449549.3035 | 53174591.49 | -496261.8064 | 119983.3514 | 76448236.32 | -1152479.928 | 310.1804402 |
| 1050 | 80792182.06 | -2331626.023 | 470036.71 | 52955157.12 | -438868.7407 | 114786.1315 | 75873133.18 | -1150206.28 | 4547.294897 |
| 1080 | 79747724.53 | -2088915.049 | 485421.9488 | 52763050.74 | -384212.7563 | 109311.9688 | 75300178.32 | -1145909.714 | 8593.133414 |
| 1110 | 78827221.19 | -1841006.695 | 495816.7075 | 52596847.25 | -332406.9904 | 103611.5318 | 74730347.24 | -1139662.161 | 12495.10617 |
| 1140 | 78032060.86 | -1590320.647 | 501372.0965 | 52455074.82 | -283544.8593 | 97724.26202 | 74164589.33 | -1131515.821 | 16292.67848 |
| 1170 | 77362469.13 | -1339183.466 | 502274.3621 | 52336222.34 | -237704.9461 | 91679.82644 | 73603835.88 | -1121506.907 | 20017.82965 |
| 1200 | 76817562.58 | -1089813.089 | 498740.7526 | 52238744.74 | -194955.1951 | 85499.50205 | 73049006.3 | -1109659.154 | 23695.5057 |
| 1230 | 76395409.92 | -844305.3211 | 491015.5369 | 52161066.52 | -155356.4494 | 79197.49143 | 72501012.74 | -1095987.121 | 27344.06597 |
| 1260 | 76093098.81 | -604622.2338 | 479366.1744 | 52101583.84 | -118965.3644 | 72782.16999 | 71960763.11 | -1080499.259 | 30975.72358 |
| 1290 | 75906807.6 | -372582.4143 | 464079.639 | 52058665.47 | -85836.73212 | 66257.26455 | 71429162.73 | -1063200.769 | 34596.97981 |
| 1320 | 75831881.12 | -149852.9677 | 445458.8933 | 52030652.85 | -56025.25037 | 59622.96351 | 70907114.6 | -1044096.243 | 38209.05233 |
| 1350 | 75862909.51 | 62056.79075 | 423819.5169 | 52015859.46 | -29586.77119 | 52876.95835 | 70395518.56 | -1023192.094 | 41808.2973 |
| 1380 | 75993809.53 | 261800.0337 | 399486.486 | 52012569.93 | -6579.062845 | 46015.41669 | 69895269.17 | -1000498.781 | 45386.6254 |

Fig. 38d5

| t | #4 | first order diff. | second order diff. | #5 | first order diff. | second order diff. | #6 | first order diff. | second order diff. |
|---|---|---|---|---|---|---|---|---|---|
| 1410 | 76217907.32 | 448195.5867 | 372791.1058 | 52019038.87 | 12937.88053 | 39033.88675 | 69407252.75 | -976032.8257 | 48931.91166 |
| 1440 | 76528022.14 | 620229.6342 | 344068.095 | 52033489.84 | 28901.94717 | 31928.13327 | 68932343.44 | -949818.6261 | 52428.39921 |
| 1470 | 76916550.16 | 777056.0455 | 313652.8226 | 52054114.54 | 41249.39962 | 24694.90491 | 68471398.4 | -921890.0776 | 55857.09696 |
| 1500 | 77375547.86 | 917995.3941 | 281878.6972 | 52079072.4 | 49915.71618 | 17332.63312 | 68025252.41 | -892291.9921 | 59196.17102 |
| 1530 | 77896814.23 | 1042532.748 | 249074.7086 | 52106490.78 | 54836.74739 | 9842.062417 | 67594711.74 | -861081.327 | 62421.33016 |
| 1560 | 78471971.39 | 1150314.309 | 215563.1215 | 52134465.85 | 55950.15348 | 2226.812166 | 67180547.63 | -828328.2245 | 65506.20501 |
| 1590 | 79092542.87 | 1241142.97 | 181657.3218 | 52161064.4 | 53197.08838 | -5506.130182 | 66783489.2 | -794116.8639 | 68422.72125 |
| 1620 | 79750029.31 | 1314972.878 | 147659.8151 | 52184326.44 | 46524.09617 | -13345.98443 | 66404216.13 | -758546.1306 | 71141.46657 |
| 1650 | 80435980.84 | 1371903.066 | 113860.3775 | 52202269.04 | 35885.18544 | -21277.82147 | 66043351.08 | -721730.1048 | 73632.05163 |
| 1680 | 81142065.97 | 1412170.246 | 80534.3587 | 52212891.06 | 21244.04759 | -29282.2757 | 65701451.89 | -683798.3724 | 75863.46475 |
| 1710 | 81860136.37 | 1436140.814 | 47941.13753 | 52214179.25 | 2576.384611 | -37335.32595 | 65379003.81 | -644896.1621 | 77804.42066 |
| 1740 | 82582287.46 | 1444302.179 | 16322.72986 | 52204115.41 | -20127.68789 | -45408.145 | 65076411.66 | -605184.3106 | 79423.70294 |
| 1770 | 83300914.19 | 1437253.454 | -14097.45151 | 52180684.81 | -46861.19665 | -53467.01752 | 64793992.13 | -564839.0604 | 80690.50045 |
| 1800 | 84008762 | 1415695.612 | -43115.68417 | 52141885.88 | -77597.86002 | -61473.32674 | 64531966.28 | -524051.6915 | 81574.73766 |
| 1830 | 84698972.58 | 1380421.176 | -70548.87146 | 52085741.05 | -112289.6648 | -69383.60951 | 64290452.29 | -483027.9922 | 82047.39875 |
| 1860 | 85365124.34 | 1332303.513 | -96235.32539 | 52010308.8 | -150864.5047 | -77149.67991 | 64069458.5 | -441987.5693 | 82080.84567 |
| 1890 | 86001267.25 | 1272285.815 | -120035.3971 | 51913696.84 | -193223.9155 | -84718.8215 | 63868877 | -401163.0043 | 81649.13009 |
| 1920 | 86601952.17 | 1201369.837 | -141831.9552 | 51794076.37 | -239240.9395 | -92034.04805 | 63688477.57 | -360798.8547 | 80728.29913 |
| 1950 | 87162254.41 | 1120604.481 | -161530.7117 | 51649697.29 | -288758.1559 | -99034.43275 | 63527902.32 | -321150.5072 | 79296.69511 |
| 1980 | 87677791.55 | 1031074.283 | -179060.3957 | 51478904.34 | -341585.9089 | -105655.5061 | 63386660.88 | -282482.8826 | 77335.24905 |
| 2010 | 88144735.5 | 933887.8961 | -194372.7744 | 51280153.95 | -397500.7701 | -111829.7222 | 63264126.38 | -245068.9986 | 74827.76811 |
| 2040 | 88559818.81 | 830166.6349 | -207442.5224 | 51052031.82 | -456244.267 | -117486.9938 | 63159532.18 | -209188.3901 | 71761.21688 |
| 2070 | 88920335.4 | 721033.1659 | -218266.938 | 50793270.86 | -517521.9147 | -122555.2955 | 63071969.49 | -175125.3938 | 68125.99263 |
| 2100 | 89224135.6 | 607600.4119 | -226865.5079 | 50502769.57 | -581002.5826 | -126961.3358 | 63000385.84 | -143167.2967 | 63916.19431 |
| 2130 | 89469615.98 | 490960.7524 | -233279.319 | 50179610.45 | -646318.2315 | -130631.2978 | 62943584.66 | -113602.3539 | 59129.88551 |
| 2160 | 89655703.78 | 372175.5936 | -237570.3177 | 49823078.43 | -713064.0556 | -133491.6482 | 62900225.82 | -86717.67828 | 53769.35125 |
| 2190 | 89781836.47 | 252265.3846 | -239820.4179 | 49432678.89 | -780799.0629 | -135470.0146 | 62868827.32 | -62797.00389 | 47841.34878 |
| 2220 | 89847936.55 | 132200.1567 | -240130.4558 | 49008155.33 | -849047.129 | -136496.1323 | 62847768.16 | -42118.32788 | 41357.35202 |
| 2250 | 89854381.88 | 12890.66069 | -238618.9921 | 48549506.05 | -917298.5581 | -136502.8581 | 62835292.44 | -24951.43282 | 34333.79011 |
| 2280 | 89801971.97 | -104819.8209 | -235420.9631 | 48056999.96 | -985012.1852 | -135427.2544 | 62829514.79 | -11555.29297 | 26792.27969 |
| 2310 | 89691890.51 | -220162.9101 | -230686.1784 | 47531190.93 | -1051618.055 | -133211.7403 | 62828427.11 | -2175.367402 | 18759.85115 |
| 2340 | 89525664.64 | -332451.7433 | -224577.6664 | 46972930.57 | -1116520.712 | -129805.3125 | 62829906.72 | 2959.216926 | 10269.16866 |
| 2370 | 89305121.3 | -441086.6774 | -217269.8682 | 46383379.01 | -1179103.128 | -125164.8337 | 62831726.01 | 3638.589026 | 1358.7442 |
| 2400 | 89032341.29 | -545560.0166 | -208946.6784 | 45764013.35 | -1238731.324 | -119256.3901 | 62831563.59 | -324.8382867 | -7926.854626 |
| 2430 | 88709611.45 | -645459.6834 | -199799.3336 | 45116633.51 | -1294759.682 | -112056.7174 | 62827016.97 | -9093.239737 | -17536.8029 |
| 2460 | 88339375.57 | -740471.7582 | -190024.1495 | 44443364.99 | -1346537.03 | -103554.6956 | 62815616.9 | -22800.15109 | -27413.82271 |
| 2490 | 87924184.67 | -830381.8102 | -179820.1041 | 43746658.25 | -1393413.486 | -93752.91114 | 62794843.32 | -41547.14798 | -37493.99378 |
| 2520 | 87466647.19 | -915074.9455 | -169386.2705 | 43029284.18 | -1434748.13 | -82669.28945 | 62762143.11 | -65400.43298 | -47706.57001 |
| 2550 | 86969379.95 | -994534.4932 | -158919.0954 | 42294325.42 | -1469917.528 | -70338.79454 | 62714949.44 | -94387.33415 | -57973.80232 |
| 2580 | 86434960.32 | -1068839.257 | -148609.527 | 41545162.86 | -1498325.126 | -56815.19757 | 62650703.08 | -128492.7179 | -68210.76751 |
| 2610 | 85865880.69 | -1138159.251 | -138639.9893 | 40785457.07 | -1519411.584 | -42172.91446 | 62566875.42 | -167655.3195 | -78325.20325 |
| 2640 | 85264505.77 | -1202749.854 | -129181.2047 | 40019124.05 | -1532666.039 | -26508.91109 | 62460993.42 | -211763.9942 | -88217.34925 |
| 2670 | 84633033.62 | -1262944.286 | -120388.8643 | 39250304.86 | -1537638.378 | -9944.678082 | 62330666.48 | -260653.8914 | -97779.7945 |
| 2700 | 83973461.44 | -1319144.359 | -112400.146 | 38483328.6 | -1533952.515 | 7371.726751 | 62173615.2 | -314102.5567 | -106897.3306 |
| 2730 | 83287556.75 | -1371809.398 | -105330.0789 | 37722668.23 | -1521320.731 | 25263.56702 | 61987702.22 | -371825.9625 | -115446.8115 |
| 2760 | 82576835.11 | -1421443.277 | -99267.75795 | 36972888.68 | -1499559.108 | 43523.24741 | 61770964.98 | -433474.4717 | -123297.0186 |
| 2790 | 81842545.37 | -1468579.479 | -94272.40371 | 36238586.64 | -1468604.075 | 61910.06609 | 61521650.61 | -498628.7382 | -130308.533 |

Fig. 38d6

| t | #4 | first order diff. | second order diff. | #5 | first order diff. | second order diff. | #6 | first order diff. | second order diff. |
|---|---|---|---|---|---|---|---|---|---|
| 2820 | 81085663.31 | -1513764.115 | -90369.27182 | 35524321.58 | -1428530.126 | 80147.89684 | 61238252.84 | -566795.5449 | -136333.6133 |
| 2850 | 80306894.9 | -1557536.819 | -87545.40793 | 34834537.22 | -1379568.724 | 97922.80415 | 60919551.05 | -637403.5845 | -141216.0792 |
| 2880 | 79506690.18 | -1600409.446 | -85745.25316 | 34173473 | -1322128.43 | 114880.5888 | 60564651.46 | -709799.1856 | -144791.2022 |
| 2910 | 78685268.93 | -1642842.493 | -84866.09441 | 33545064.85 | -1256816.297 | 130624.2656 | 60173030.46 | -783241.9862 | -146885.6012 |
| 2940 | 77842659.34 | -1685219.175 | -84753.36417 | 32952834.57 | -1184460.561 | 144711.4719 | 59744580.18 | -856900.5589 | -147317.1454 |
| 2970 | 76978750.81 | -1727817.069 | -85195.78747 | 32399767.24 | -1106134.657 | 156651.8078 | 59279656.19 | -929847.9901 | -145894.8625 |
| 3000 | 76093362.18 | -1770777.257 | -85920.37616 | 31888175.94 | -1023182.603 | 165904.1088 | 58779127.48 | -1001057.417 | -142418.8531 |
| 3030 | 75186326.73 | -1814070.893 | -86587.27193 | 31419553.05 | -937245.7787 | 171873.6478 | 58244428.72 | -1069397.523 | -136680.212 |
| 3060 | 74257595.18 | -1857463.11 | -86784.43438 | 30994407.48 | -850291.1434 | 173909.2707 | 57677614.72 | -1133628 | -128460.9546 |
| 3090 | 73307358.08 | -1900474.201 | -86022.18224 | 30612087.03 | -764640.9125 | 171300.4619 | 57081417.23 | -1192394.975 | -117533.9497 |
| 3120 | 72336189.09 | -1942337.988 | -83727.57379 | 30270585.16 | -683003.7411 | 163274.3427 | 56459304.03 | -1244226.404 | -103662.8592 |
| 3150 | 71345210.43 | -1981957.31 | -79238.64491 | 29966331.44 | -608507.4413 | 148992.5996 | 55815540.31 | -1287527.446 | -86602.08289 |
| 3180 | 70336282.15 | -2017856.553 | -71798.48508 | 29693964.8 | -544733.2688 | 127548.345 | 55155252.41 | -1320575.801 | -66096.70971 |
| 3210 | 69312216.59 | -2048131.138 | -60549.17002 | 29446088.89 | -495751.8139 | 97962.9098 | 54484493.89 | -1341517.038 | -41882.47531 |
| 3240 | 68277019.63 | -2070393.905 | -44525.53387 | 29213008.63 | -466160.5311 | 59182.56562 | 53810313.94 | -1348359.901 | -13685.72584 |
| 3270 | 67236160.48 | -2081718.303 | -22648.79622 | 28982447.16 | -461122.9405 | 10075.18106 | 53140828.14 | -1338971.595 | 18776.61263 |
| 3300 | 66196871.32 | -2078578.318 | 6279.969925 | 28739242.39 | -486409.5369 | -50573.19281 | 52485291.61 | -1311073.066 | 55797.05828 |
| 3330 | 65168478.79 | -2056785.06 | 43586.51607 | 28465022.17 | -548440.4395 | -124061.8051 | 51854174.47 | -1262234.27 | 97677.59138 |
| 3360 | 64162768.83 | -2011419.933 | 90730.25293 | 28137857.26 | -654329.8163 | -211778.7536 | 51259239.75 | -1189869.437 | 144729.6665 |
| 3390 | 63194386.67 | -1936764.316 | 149311.2351 | 27731891.2 | -811932.1198 | -315204.6071 | 50713623.59 | -1091232.328 | 197274.2182 |
| 3420 | 62281273.84 | -1826225.664 | 221077.3031 | 27216946.12 | -1029890.165 | -435916.0909 | 50231917.84 | -963411.4974 | 255641.6606 |
| 3450 | 61445143.84 | -1672259.982 | 307931.3651 | 26558103.58 | -1317685.087 | -575589.8445 | 49830255.06 | -803325.5568 | 320171.8813 |
| 3480 | 60711998.57 | -1466290.557 | 411938.8492 | 25715259.47 | -1685688.21 | -736006.245 | 49526395.84 | -607718.4427 | 391214.2282 |
| 3510 | 60112687.11 | -1198622.912 | 535335.2911 | 24642652.04 | -2145214.861 | -919053.3029 | 49339818.49 | -373154.6969 | 469127.4917 |

Fig. 38d7

| t | #7 | first order diff. | second order diff. | #8 | first order diff. | second order diff. |
|---|---|---|---|---|---|---|
| 0 | 26347194.64 | | | 111362893.4 | | |
| 30 | 27765404.61 | 2836419.936 | | 113689929 | 4654071.202 | |
| 60 | 28834431.2 | 2138053.186 | -1396733.5 | 115430680.9 | 3481503.76 | -2345134.885 |
| 90 | 29610832.79 | 1552803.175 | -1170500.023 | 116670156.1 | 2478950.352 | -2005106.817 |
| 120 | 30144291.6 | 1066917.619 | -971771.1106 | 117483350.9 | 1626389.694 | -1705121.315 |
| 150 | 30478227.23 | 667871.2533 | -798092.7325 | 117936144.2 | 905586.5522 | -1441606.284 |
| 180 | 30650372.26 | 344290.0661 | -647162.3744 | 118086134.8 | 299981.1009 | -1211210.903 |
| 210 | 30693311.49 | 85878.44957 | -516823.233 | 117983426 | -205417.4753 | -1010797.152 |
| 240 | 30634986.08 | -116650.8082 | -405058.5156 | 117671359.4 | -624133.22 | -837431.4893 |
| 270 | 30499164.22 | -271643.7307 | -309985.845 | 117187198.6 | -968321.5571 | -688376.6743 |
| 300 | 30305879.41 | -386569.6152 | -229851.769 | 116562766.9 | -1248863.434 | -561083.754 |
| 330 | 30071838.01 | -468082.8022 | -163026.374 | 115825039.2 | -1475455.531 | -453184.1931 |
| 360 | 29810797.11 | -522081.8041 | -107998.0038 | 114996690.9 | -1656696.611 | -362482.1605 |
| 390 | 29533914.18 | -553765.8456 | -63368.08303 | 114096605.8 | -1800170.094 | -286946.9662 |
| 420 | 29250069.75 | -567688.8679 | -27846.04453 | 113140344.3 | -1912522.92 | -224705.6516 |
| 450 | 28966164.22 | -567811.049 | -244.3622363 | 112140574 | -1999540.786 | -174035.7315 |
| 480 | 28687390.28 | -557547.8931 | 20526.31184 | 111107464 | -2066219.83 | -133358.0889 |
| 510 | 28417481.81 | -539816.9402 | 35461.9058 | 110049046.6 | -2116834.841 | -101230.021 |
| 540 | 28158940.73 | -517082.1495 | 45469.58145 | 108971544.6 | -2155004.06 | -76338.43921 |
| 570 | 27913242.73 | -491396.0078 | 51372.28326 | 107879669.3 | -2183750.67 | -57493.21948 |
| 600 | 27681023.02 | -464439.4165 | 53913.18268 | 106776888.7 | -2205561.023 | -43620.70657 |
| 630 | 27462243.32 | -437559.4076 | 53760.01782 | 105665668.9 | -2222439.708 | -33757.36959 |
| 660 | 27256340.95 | -411804.7433 | 51509.32848 | 104547688.1 | -2235961.513 | -27043.61022 |
| 690 | 27062361.22 | -387959.45 | 47690.5866 | 103424027.9 | -2247320.375 | -22717.72313 |
| 720 | 26879074.05 | -366574.339 | 42770.22196 | 102295340.3 | -2257375.379 | -20110.00868 |
| 750 | 26705075.77 | -347996.5673 | 37155.5434 | 101161993.3 | -2266693.898 | -18637.03792 |
| 780 | 26538877.12 | -332397.2897 | 31198.55523 | 100024197.3 | -2275591.933 | -17796.06982 |
| 810 | 26378978.39 | -319797.4552 | 25199.66916 | 98882111.47 | -2284171.743 | -17159.62087 |
| 840 | 26223932.49 | -310091.7994 | 19411.31149 | 97735933.05 | -2292356.837 | -16370.18687 |
| 870 | 26072396.95 | -303071.0865 | 14041.42572 | 96585970.85 | -2299924.395 | -15135.11702 |
| 900 | 25923175.63 | -298442.6513 | 9256.870451 | 95432703.25 | -2306535.215 | -13221.64038 |
| 930 | 25775250.98 | -295849.2949 | 5186.712774 | 94276822.63 | -2311761.238 | -10452.04448 |
| 960 | 25627807.69 | -294886.5865 | 1925.41689 | 93119267.26 | -2315110.741 | -6699.006295 |
| 990 | 25480248.37 | -295118.6224 | -464.0718222 | 91961241.62 | -2316051.279 | -1881.075475 |
| 1020 | 25332202.23 | -296092.2961 | -1947.347408 | 90804226.4 | -2314030.433 | 4041.690137 |
| 1050 | 25183527.16 | -297350.1304 | -2515.668568 | 89649979.17 | -2308494.465 | 11071.93672 |
| 1080 | 25034306.3 | -298441.7246 | -2183.188524 | 88500526.7 | -2298904.928 | 19179.07437 |
| 1110 | 24884839.36 | -298933.8694 | -984.2895252 | 87358150.04 | -2284753.336 | 28303.18478 |
| 1140 | 24735629.67 | -298419.3804 | 1028.978007 | 86225363.06 | -2265573.947 | 38358.77659 |
| 1170 | 24587367.32 | -296524.7045 | 3789.351718 | 85104885.69 | -2240954.753 | 49238.38844 |
| 1200 | 24440909.15 | -292916.3501 | 7216.708875 | 83999612.32 | -2210546.733 | 60816.0398 |
| 1230 | 24297256.05 | -287306.1934 | 11220.31335 | 82912576.59 | -2174071.468 | 72950.5295 |
| 1260 | 24157528.19 | -279455.7145 | 15700.9579 | 81846913 | -2131327.177 | 85488.58191 |
| 1290 | 24022938.59 | -269179.2135 | 20553.00189 | 80805816.37 | -2082193.257 | 98267.84095 |
| 1320 | 23894765.55 | -256346.0614 | 25666.30426 | 79792499.67 | -2026633.401 | 111119.7118 |
| 1350 | 23774324.54 | -240882.0354 | 30928.05201 | 78810150.98 | -1964697.376 | 123872.0501 |
| 1380 | 23662939.64 | -222769.7934 | 36224.48391 | 77861890.22 | -1896521.526 | 136351.6993 |

Fig. 38d8

| t | #7 | first order diff. | second order diff. | #8 | first order diff. | second order diff. |
|---|---|---|---|---|---|---|
| 1410 | 23561915.37 | -202048.5386 | 41442.50964 | 76950726.17 | -1822328.089 | 148386.8754 |
| 1440 | 23472508.91 | -178812.9265 | 46471.2243 | 76079514.48 | -1742423.389 | 159809.3996 |
| 1470 | 23395903.27 | -153211.2674 | 51203.31825 | 75250916.98 | -1657195 | 170456.7782 |
| 1500 | 23333181.74 | -125443.0762 | 55536.38235 | 74467363.01 | -1567107.934 | 180174.1312 |
| 1530 | 23285303.73 | -95756.02192 | 59374.10852 | 73731013.04 | -1472699.951 | 188815.9672 |
| 1560 | 23253082.56 | -64442.32906 | 62627.38571 | 73043725.01 | -1374576.047 | 196247.8078 |
| 1590 | 23237165.22 | -31834.68348 | 65215.29117 | 72407023.9 | -1273402.218 | 202347.658 |
| 1620 | 23238014.37 | 1698.305109 | 67065.97718 | 71822074.63 | -1169898.555 | 207007.3251 |
| 1650 | 23255892.89 | 35757.03164 | 68117.45307 | 71289658.74 | -1064831.762 | 210133.5859 |
| 1680 | 23290850.97 | 69916.16292 | 68318.26256 | 70810155.16 | -959007.162 | 211649.2003 |
| 1710 | 23342716.06 | 103730.1912 | 67628.05664 | 70383525.03 | -853260.2752 | 211493.7737 |
| 1740 | 23411085.68 | 136739.222 | 66018.06158 | 70009301 | -748448.0419 | 209624.4666 |
| 1770 | 23495323.15 | 168474.9433 | 63471.4425 | 69686581.12 | -645439.7658 | 206016.5522 |
| 1800 | 23594556.51 | 198466.7244 | 59983.56221 | 69414027.19 | -545107.8551 | 200663.8214 |
| 1830 | 23707680.41 | 226247.7921 | 55562.13539 | 69189867.98 | -448318.4371 | 193578.8359 |
| 1860 | 23833361.12 | 251361.4312 | 50227.27821 | 69011907.01 | -355921.9229 | 184793.0285 |
| 1890 | 23970044.7 | 273367.1578 | 44011.45327 | 68877535.22 | -268743.5969 | 174356.6519 |
| 1920 | 24115968.11 | 291846.8128 | 36959.30989 | 68783748.06 | -187574.3098 | 162338.5743 |
| 1950 | 24269173.37 | 306410.5227 | 29127.41981 | 68727167.39 | -113161.3481 | 148825.9234 |
| 1980 | 24427524.61 | 316702.4768 | 20583.9082 | 68704067.61 | -46199.5592 | 133923.5778 |
| 2010 | 24588727.84 | 322406.4668 | 11407.98006 | 68710406.2 | 12677.19406 | 117753.5065 |
| 2040 | 24750353.41 | 323251.1378 | 1689.342003 | 68741858.29 | 62904.17178 | 100453.9554 |
| 2070 | 24909860.86 | 319014.8975 | -8472.480626 | 68793855 | 103993.4129 | 82178.48218 |
| 2100 | 25064626.07 | 309530.4318 | -18968.93131 | 68861625.41 | 135540.8322 | 63094.83863 |
| 2130 | 25211970.46 | 294688.7747 | -29683.31435 | 68940241.75 | 157232.6826 | 43383.7008 |
| 2160 | 25349191.9 | 274442.8787 | -40491.79196 | 69024667.41 | 168851.3059 | 23237.24671 |
| 2190 | 25473597.22 | 248810.6361 | -51264.48525 | 69109807.46 | 170280.0969 | 2857.581865 |
| 2220 | 25582535.87 | 217877.2957 | -61866.68077 | 69190561.26 | 161507.6032 | -17544.98732 |
| 2250 | 25673434.48 | 181797.2253 | -72160.14073 | 69261876.6 | 142630.6868 | -37753.83281 |
| 2280 | 25743831.96 | 140794.9659 | -82004.51873 | 69318804.94 | 113856.6688 | -57548.0361 |
| 2310 | 25791414.72 | 95165.52622 | -91258.87944 | 69356557.13 | 75504.38282 | -76704.57187 |
| 2340 | 25814051.66 | 45273.86452 | -99783.32338 | 69370559.16 | 28004.0609 | -95000.64384 |
| 2370 | 25809828.41 | -8446.493543 | -107440.7161 | 69356507.14 | -28104.02555 | -112216.1729 |
| 2400 | 25777080.53 | -65495.75457 | -114098.5221 | 69310421.02 | -92172.24445 | -128136.4378 |
| 2430 | 25714424.97 | -125311.1261 | -119630.7431 | 69228696.18 | -163449.6781 | -142554.8674 |
| 2460 | 25620789.42 | -187271.1069 | -123919.9615 | 69108152.35 | -241087.6712 | -155275.9861 |
| 2490 | 25495438.99 | -250700.851 | -126859.4882 | 68946078.88 | -324146.9267 | -166118.511 |
| 2520 | 25337999.66 | -314878.6583 | -128355.6147 | 68740275.77 | -411606.2274 | -174918.6014 |
| 2550 | 25148477.84 | -379043.6435 | -128329.9704 | 68489089.34 | -502372.8576 | -181533.2605 |
| 2580 | 24927275.52 | -442404.6356 | -126721.9841 | 68191441.94 | -595294.8027 | -185843.8901 |
| 2610 | 24675200.34 | -504150.361 | -123491.4508 | 67846854.54 | -689174.8009 | -187759.9964 |
| 2640 | 24393469.86 | -563460.962 | -118621.2019 | 67455461.38 | -782786.3253 | -187223.0488 |
| 2670 | 24083709.41 | -619520.9027 | -112119.8815 | 67018015.59 | -874891.5709 | -184210.4912 |
| 2700 | 23747942.75 | -671533.3159 | -104024.8263 | 66535884.83 | -964261.5234 | -178739.9049 |
| 2730 | 23388574.83 | -718735.8412 | -94405.05058 | 66011035.74 | -1049698.186 | -170873.3244 |
| 2760 | 23008365.83 | -760418.0091 | -83364.33581 | 65446006.22 | -1130059.038 | -160721.705 |
| 2790 | 22610395.71 | -795940.2212 | -71044.42433 | 64843864.31 | -1204283.81 | -148449.5429 |

Fig. 38d9

| t | #7 | first order diff. | second order diff. | #8 | first order diff. | second order diff. |
|---|---|---|---|---|---|---|
| 2820 | 22198018. 52 | -824754. 3808 | -57628. 31903 | 64208152. 5 | -1271423. 633 | -134279. 6479 |
| 2850 | 21774805. 41 | -846426. 2236 | -43343. 68562 | 63542816. 16 | -1330672. 667 | -118498. 0677 |
| 2880 | 21344475. 71 | -860659. 4044 | -28466. 36177 | 62852115. 04 | -1381402. 25 | -101459. 1647 |
| 2910 | 20910815. 02 | -867321. 3891 | -13323. 96928 | 62140516. 2 | -1423197. 673 | -83590. 846 |
| 2940 | 20477579. 41 | -866471. 2047 | 1700. 36882 | 61412567. 38 | -1455897. 645 | -65399. 94375 |
| 2970 | 20048384. 86 | -858389. 1019 | 16164. 20554 | 60672749. 12 | -1479636. 52 | -47477. 75031 |
| 3000 | 19626580. 77 | -843608. 1798 | 29561. 84425 | 59925304. 43 | -1494889. 371 | -30505. 70296 |
| 3030 | 19215106. 76 | -822948. 0265 | 41320. 3066 | 59174044. 44 | -1502519. 983 | -15261. 22271 |
| 3060 | 18816331. 55 | -797550. 428 | 50795. 19704 | 58422128. 52 | -1503831. 836 | -2623. 706169 |
| 3090 | 18431872. 95 | -768917. 1966 | 57266. 46275 | 57671817. 44 | -1500622. 168 | 6419. 334312 |
| 3120 | 18062397. 86 | -738950. 1729 | 59934. 04729 | 56924197. 85 | -1495239. 182 | 10765. 97224 |
| 3150 | 17707401. 13 | -709993. 452 | 57913. 44191 | 56178876. 61 | -1490642. 471 | 9193. 422241 |
| 3180 | 17364962. 19 | -684877. 8866 | 50231. 13086 | 55433643. 24 | -1490466. 752 | 351. 4381943 |
| 3210 | 17031478. 23 | -666967. 9206 | 35819. 93188 | 54684098. 75 | -1499088. 971 | -17244. 43729 |
| 3240 | 16701372. 83 | -660210. 804 | 13514. 23318 | 53923249. 32 | -1521698. 859 | -45219. 77724 |
| 3270 | 16366778. 71 | -669188. 242 | -17954. 87591 | 53141062. 81 | -1564373. 024 | -85348. 32935 |
| 3300 | 16017193. 44 | -699170. 5299 | -59964. 5759 | 52323986. 49 | -1634152. 634 | -139559. 2197 |
| 3330 | 15639106. 83 | -756173. 2291 | -114005. 3983 | 51454424. 1 | -1739124. 795 | -209944. 3214 |
| 3360 | 15215598. 61 | -847016. 4306 | -181686. 403 | 50510170. 26 | -1888507. 674 | -298765. 7596 |
| 3390 | 14725905. 28 | -979386. 6656 | -264740. 4699 | 49463800. 53 | -2092739. 466 | -408463. 5837 |
| 3420 | 14144954. 53 | -1161901. 51 | -365029. 6879 | 48282014. 9 | -2363571. 258 | -541663. 5827 |
| 3450 | 13442866. 06 | -1404176. 935 | -484550. 8506 | 46924932. 95 | -2714163. 885 | -701185. 2554 |
| 3480 | 12584417. 33 | -1716897. 464 | -625441. 0591 | 45345338. 53 | -3159188. 852 | -890049. 9338 |
| 3510 | 11528472. 74 | -2111889. 177 | -789983. 4256 | 43487871. 84 | -3714933. 381 | -1111489. 057 |

Fig. 39-1

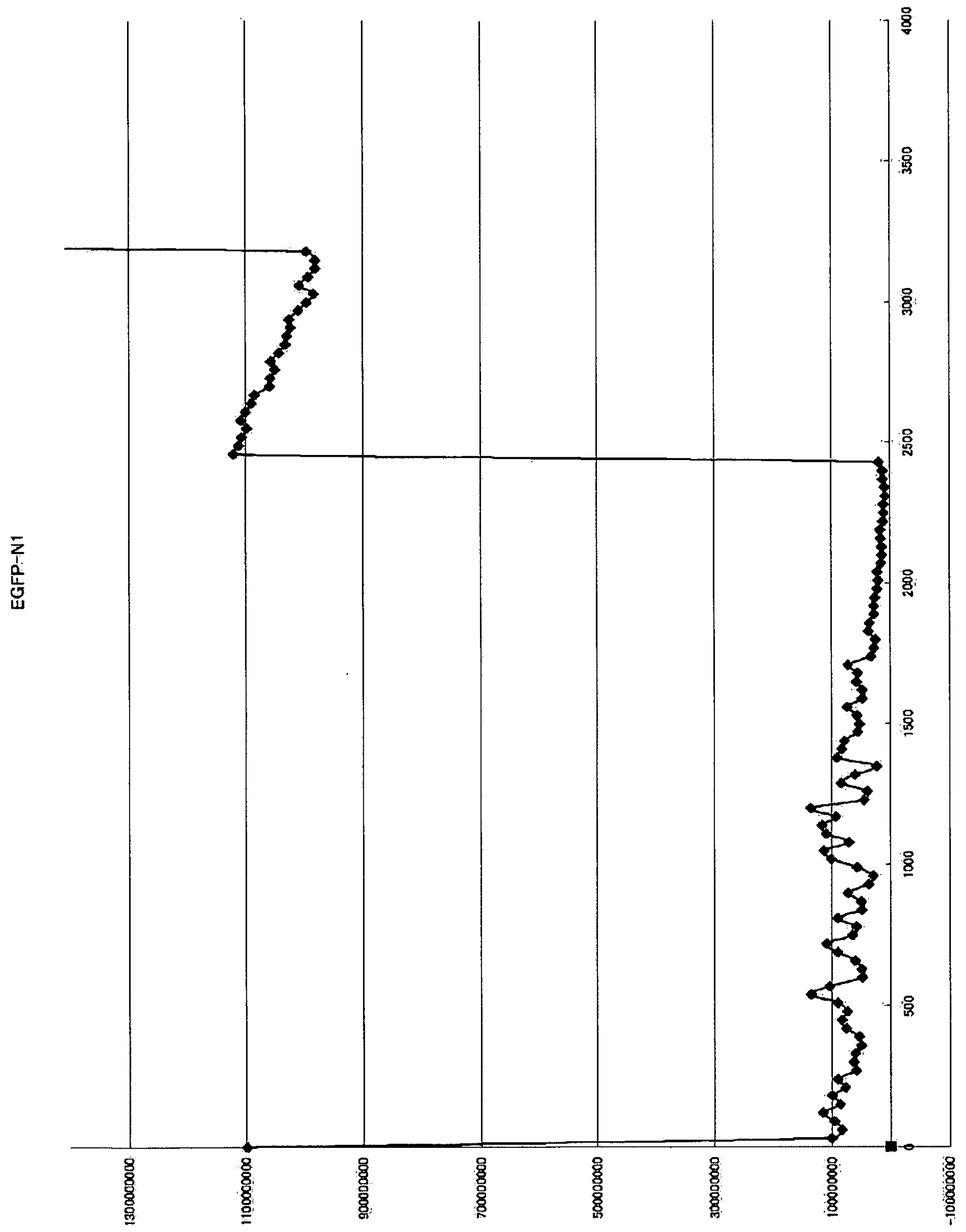
EGFP-N1
1300000000
1100000000
900000000
700000000
500000000
300000000
100000000
-100000000
0
500
1000
1500
2000
2500
3000
3500
4000

Fig. 39-2

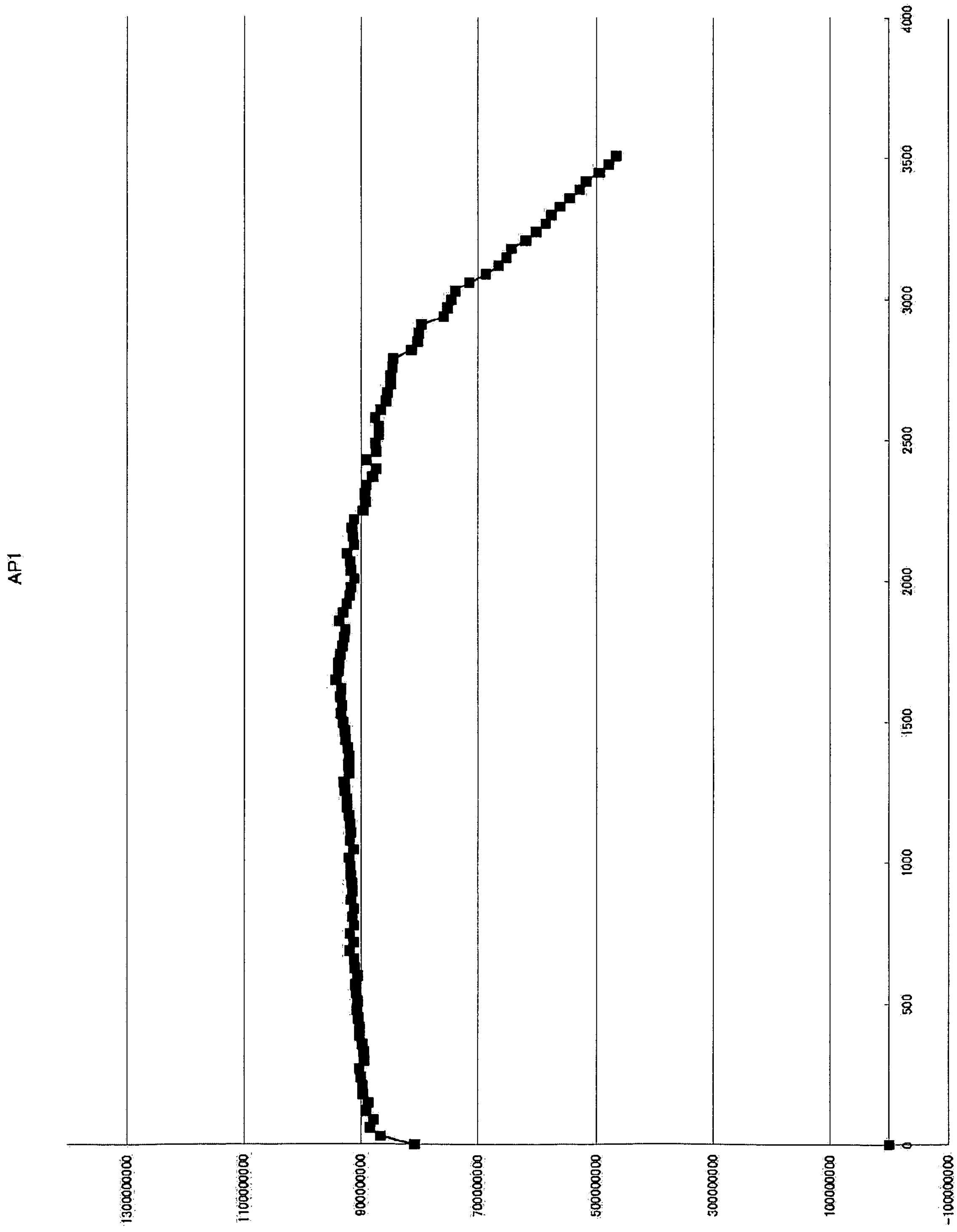
AP1
1300000000
1100000000
900000000
700000000
500000000
300000000
100000000
-100000000
0
500
1000
1500
2000
2500
3000
3500
4000

Fig. 39-3

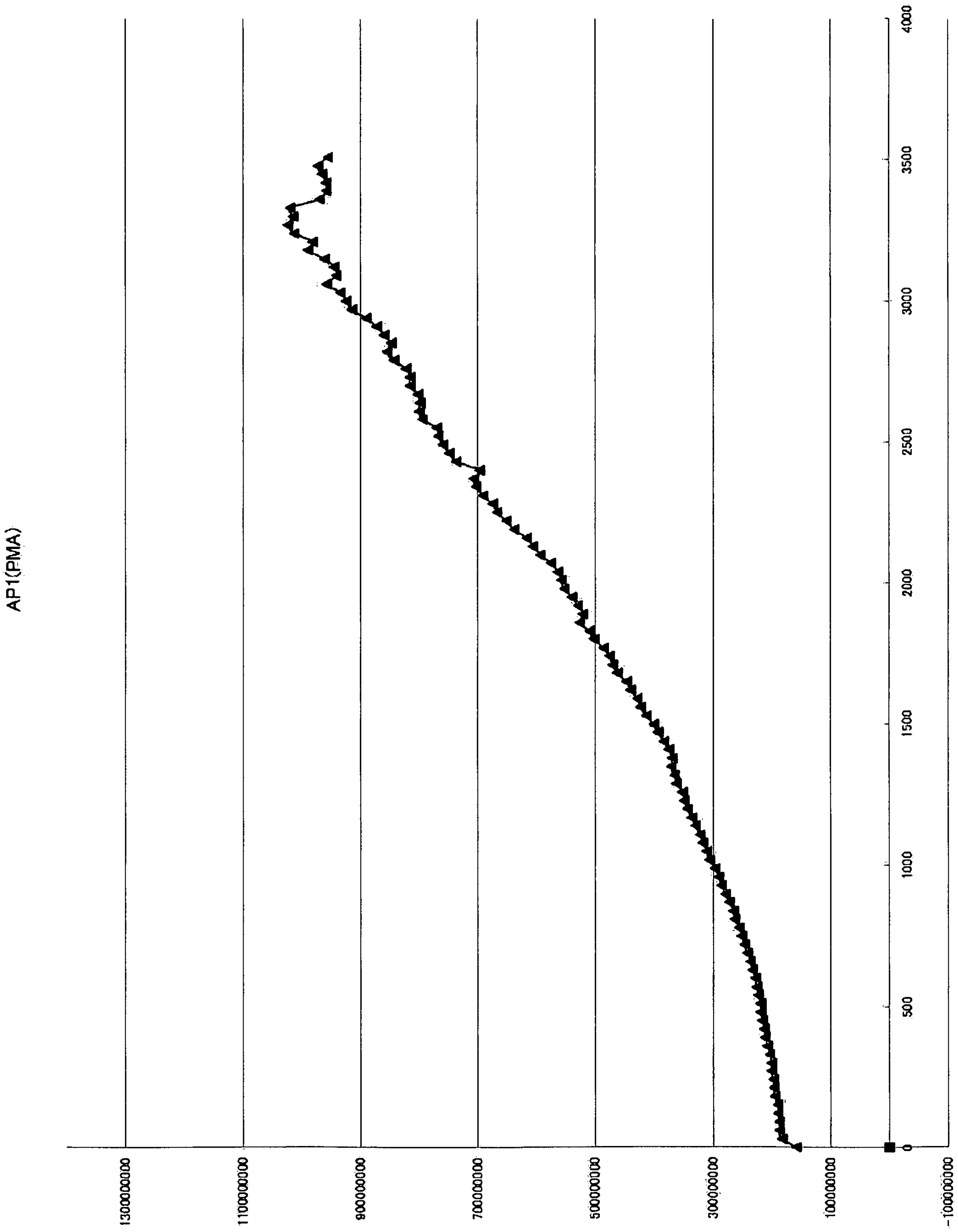
AP1(PMA)
1300000000
1100000000
900000000
700000000
500000000
300000000
100000000
-100000000
0
500
1000
1500
2000
2500
3000
3500
4000

Fig. 39-4

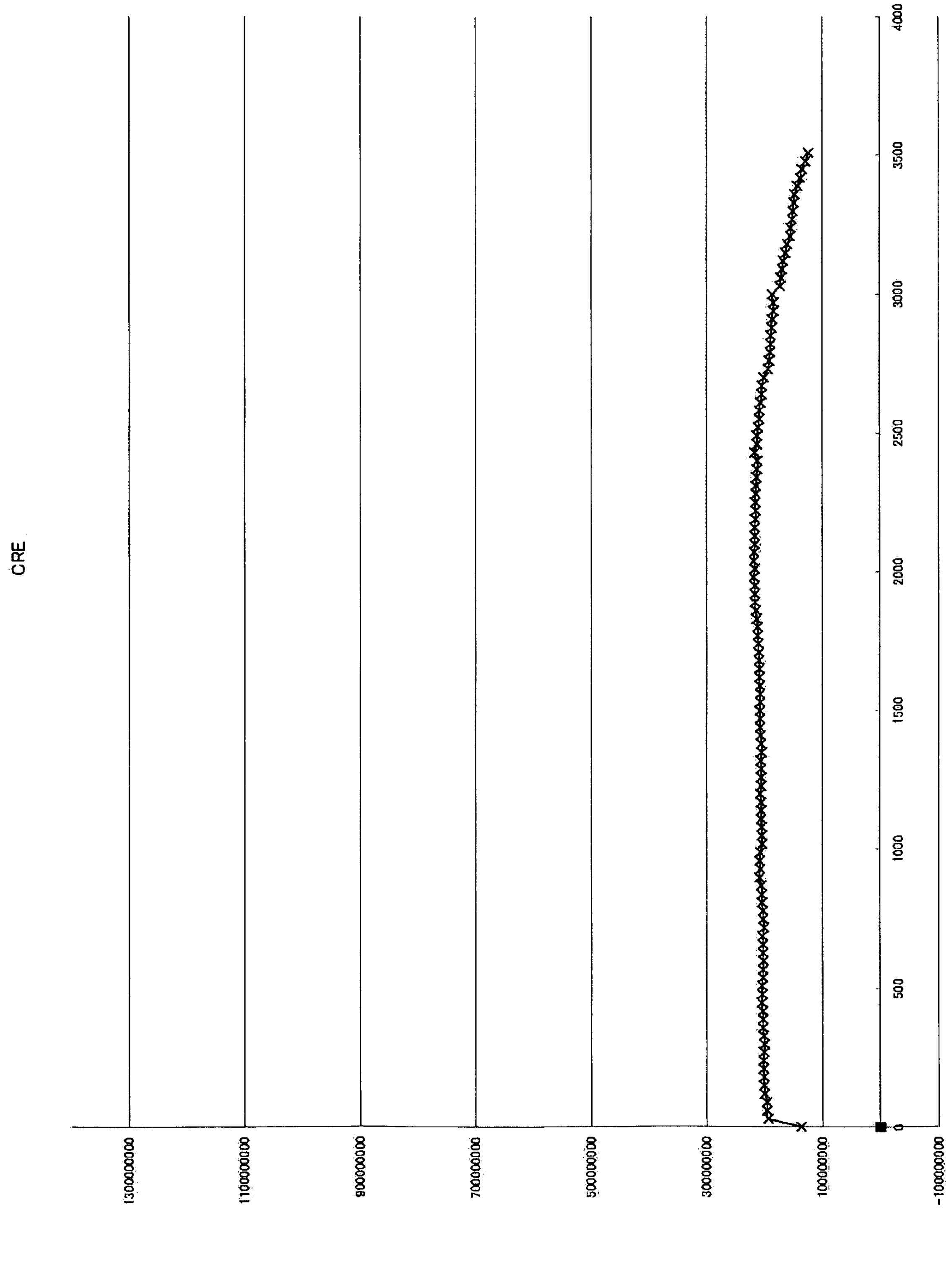
CRE
1300000000
1100000000
900000000
700000000
500000000
300000000
100000000
-100000000
0
500
1000
1500
2000
2500
3000
3500
4000

Fig. 39-5

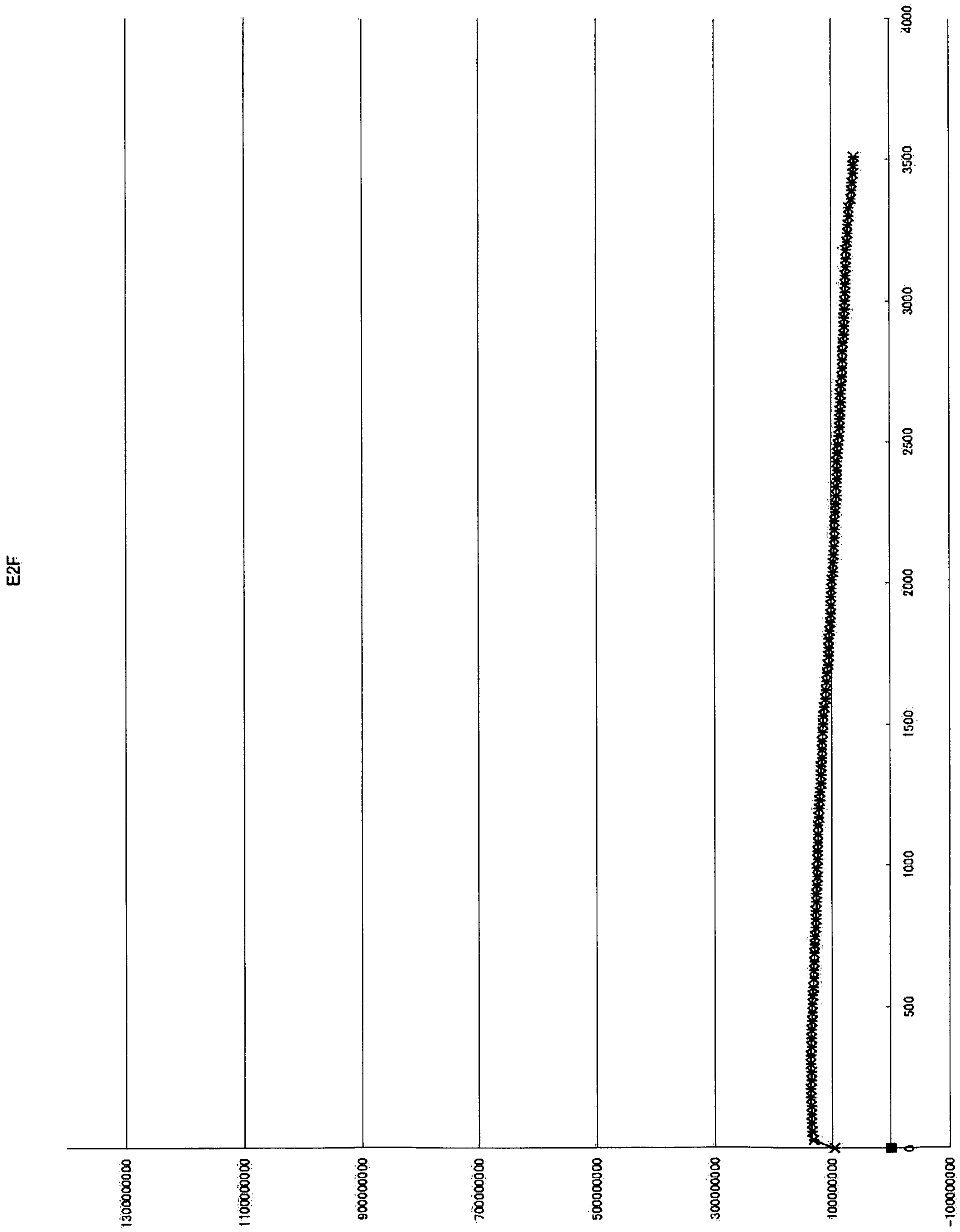
E2F
1300000000
1100000000
900000000
700000000
500000000
300000000
100000000
-100000000
0
500
1000
1500
2000
2500
3000
3500
4000

Fig. 39-6

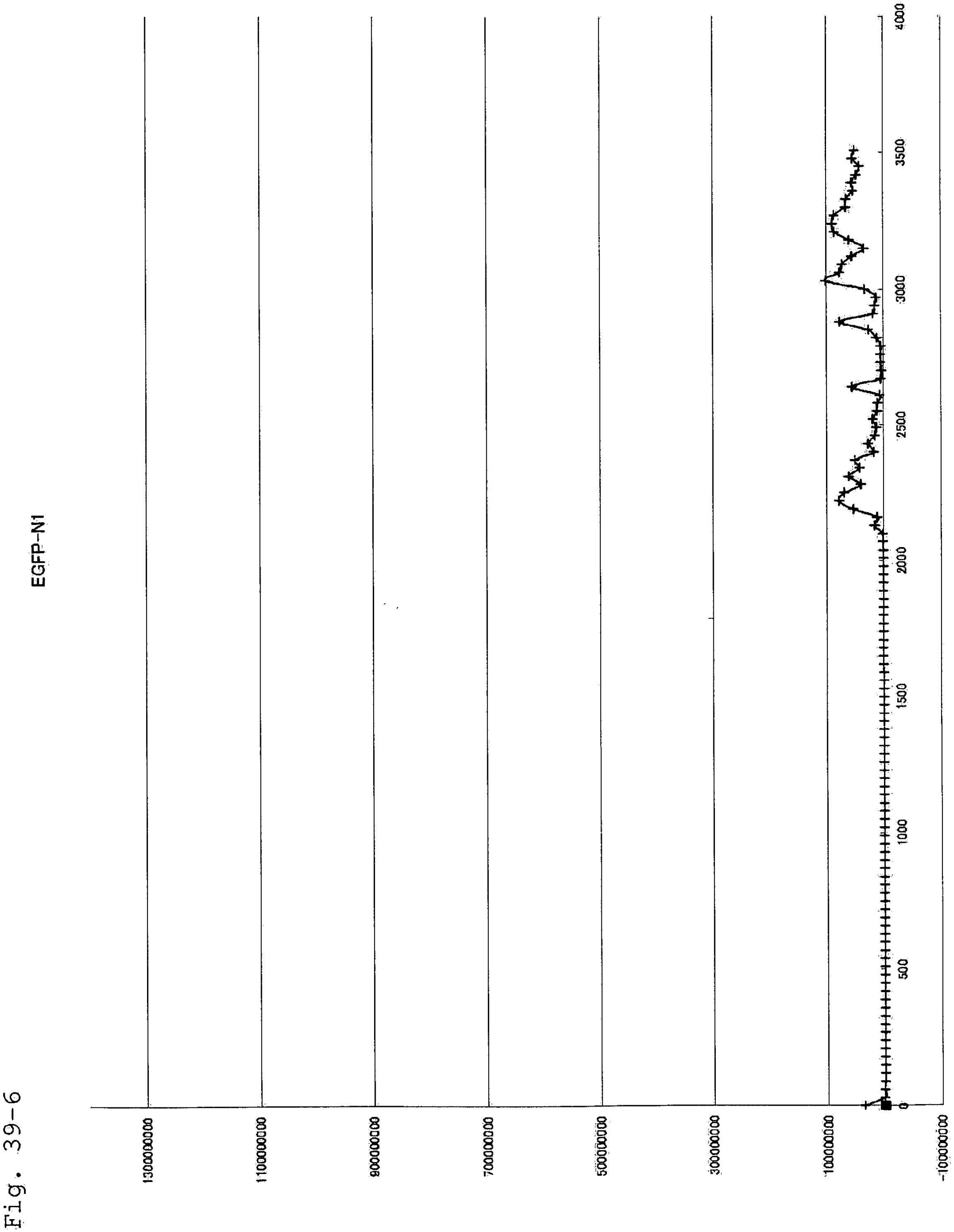
EGFP-N1
1300000000
1100000000
900000000
700000000
500000000
300000000
100000000
-100000000
0
500
1000
1500
2000
2500
3000
3500
4000

Fig. 39-7

none

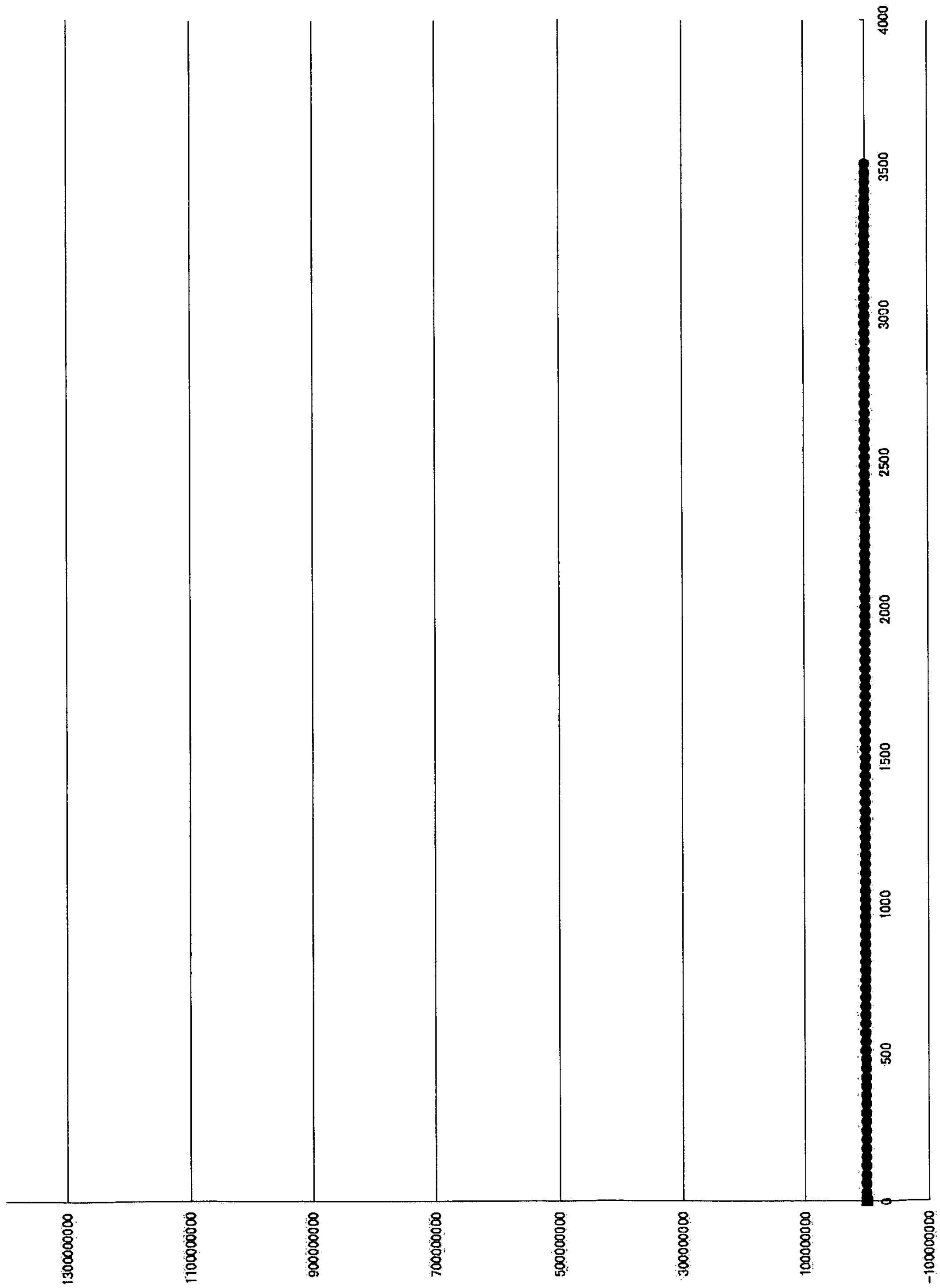
1300000000
1100000000
900000000
700000000
500000000
300000000
100000000
-100000000
0
500
1000
1500
2000
2500
3000
3500
4000

Fig. 39-8

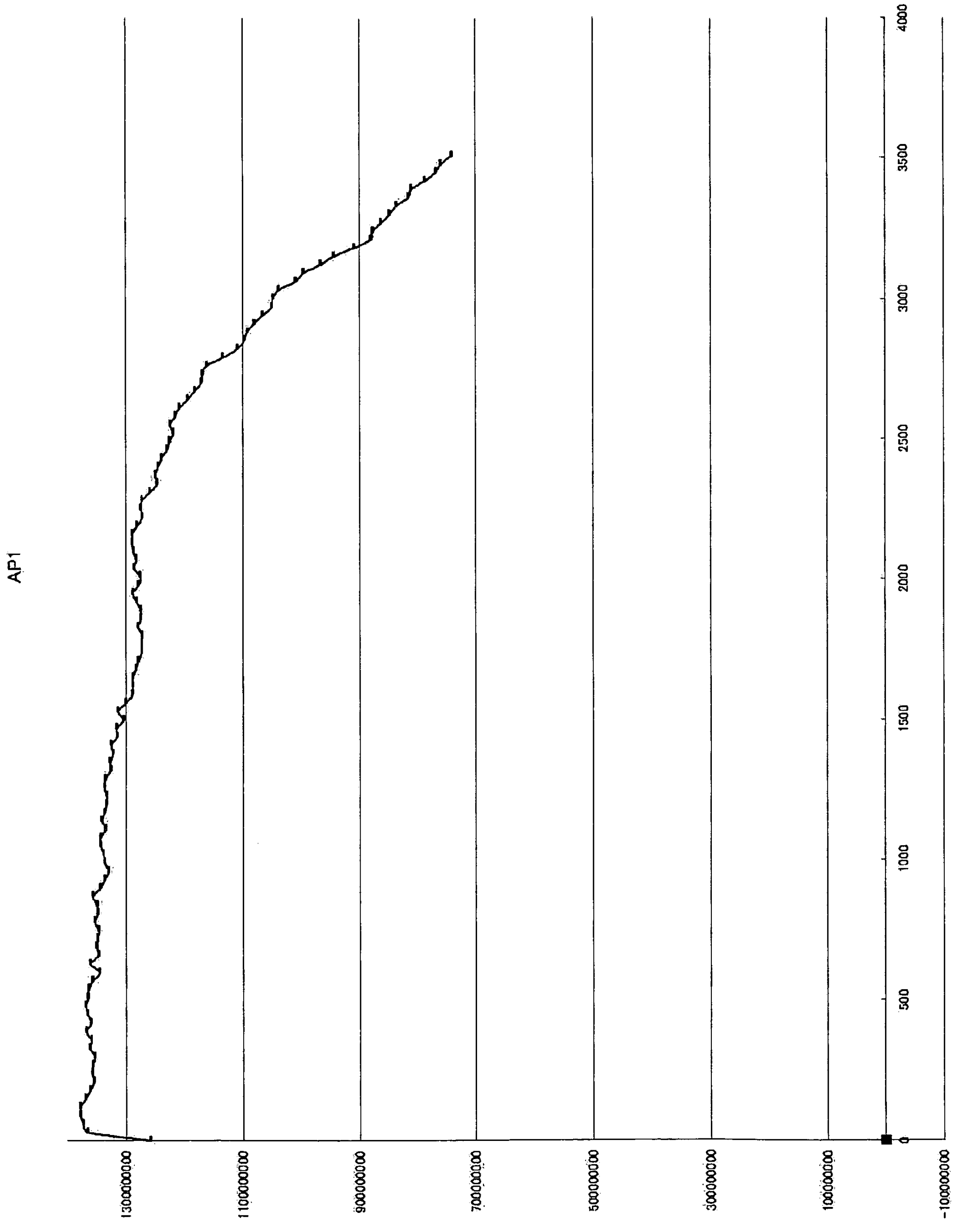
AP1
1300000000
1100000000
900000000
700000000
500000000
300000000
100000000
-100000000
0
500
1000
1500
2000
2500
3000
3500
4000

Fig. 39-9

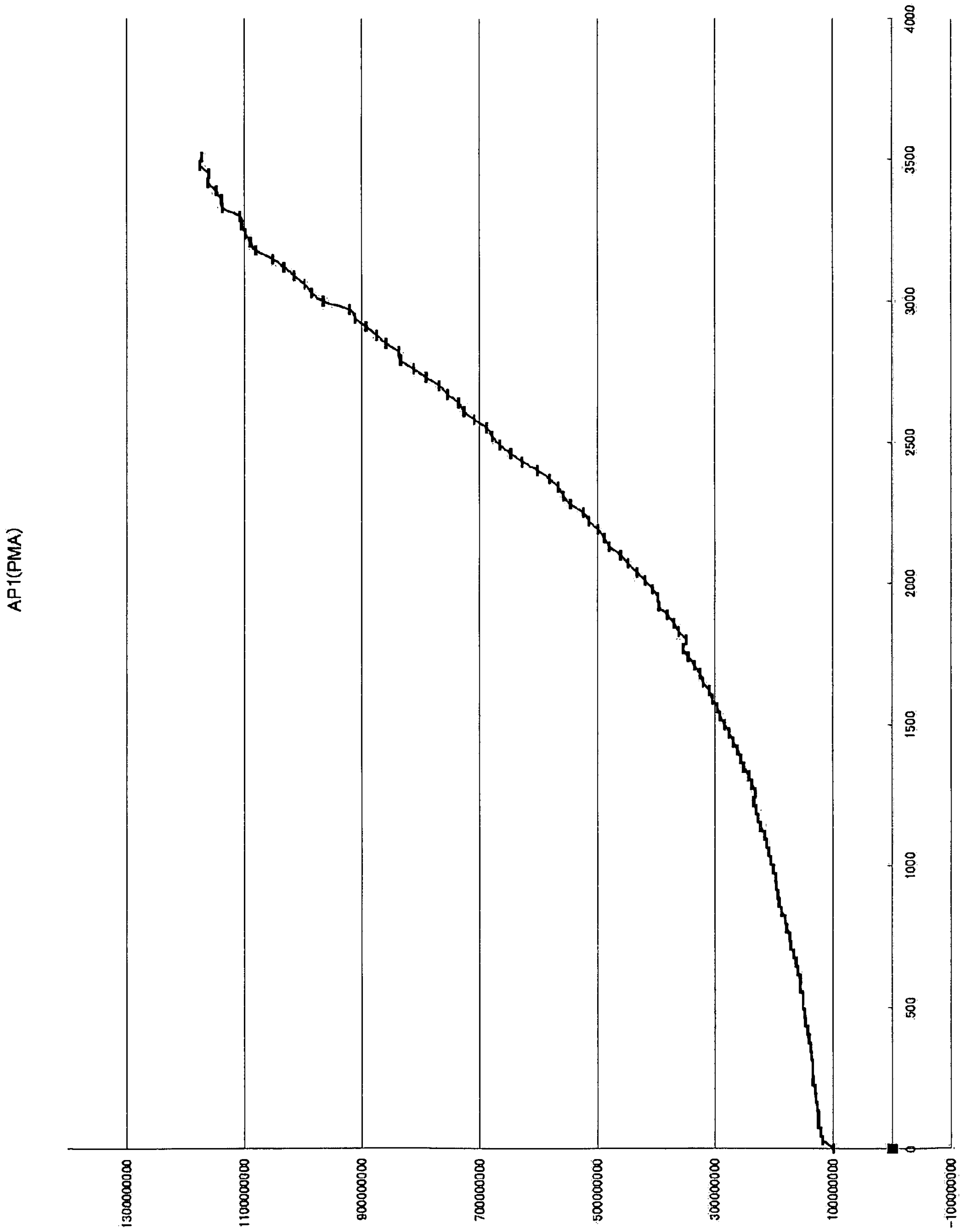
AP1(PMA)
1300000000
1100000000
900000000
700000000
500000000
300000000
100000000
-100000000
0
500
1000
1500
2000
2500
3000
3500
4000

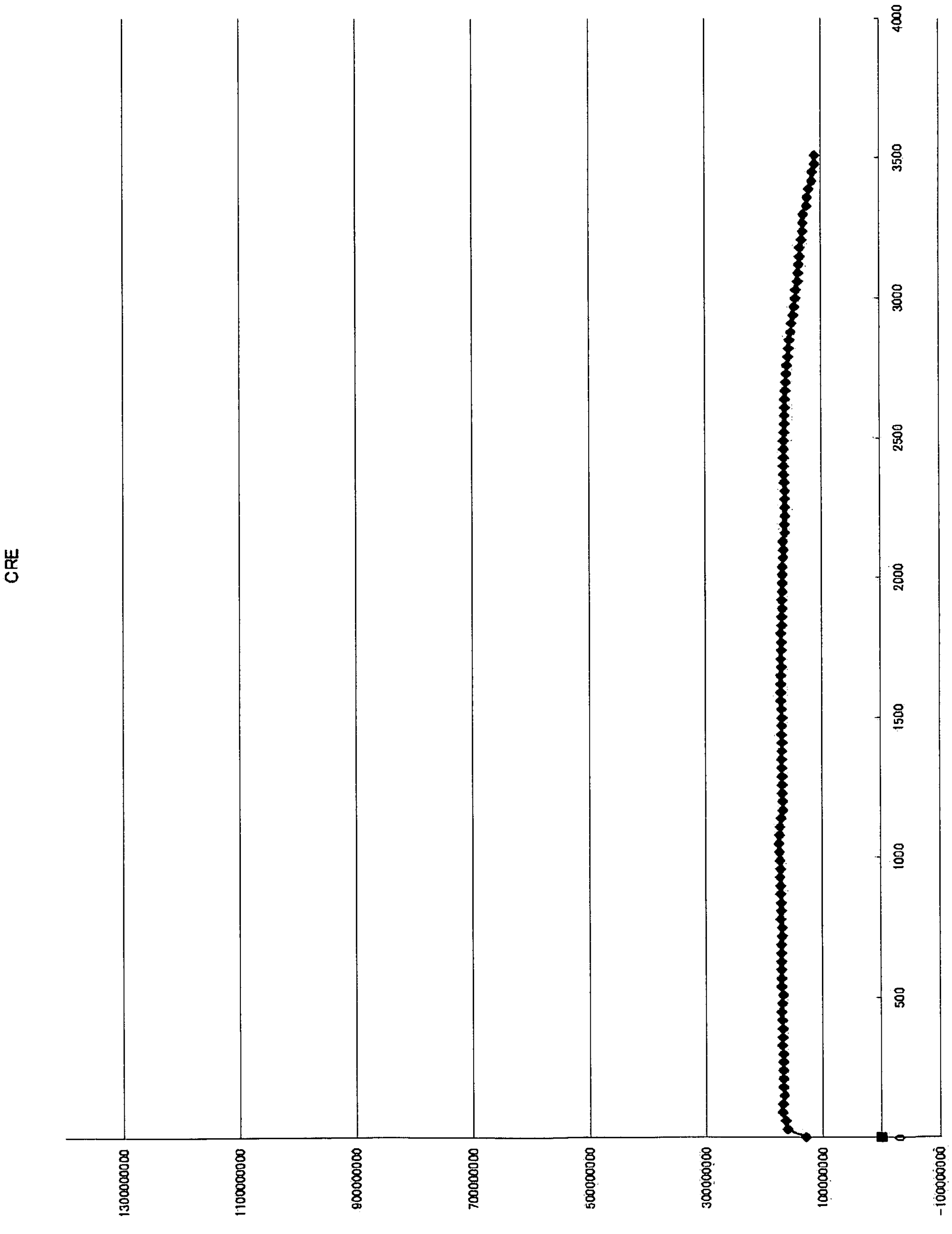
CRE
1300000000
1100000000
900000000
700000000
500000000
300000000
100000000
-100000000
0
500
1000
1500
2000
2500
3000
3500
4000

Fig. 39-10

Fig. 39-11

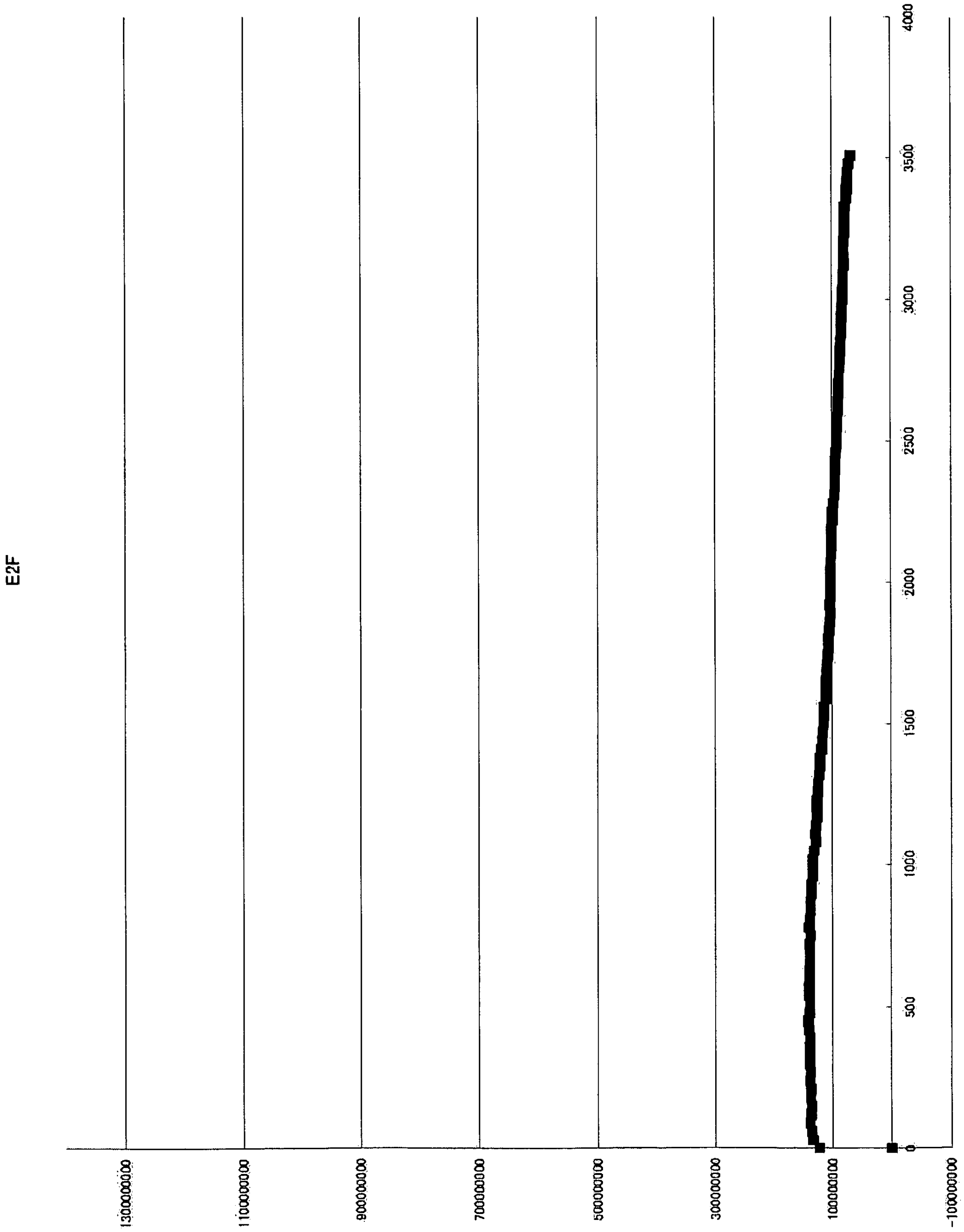
E2F
1300000000
1100000000
900000000
700000000
500000000
300000000
100000000
-100000000
0
500
1000
1500
2000
2500
3000
3500
4000

Fig. 39-12

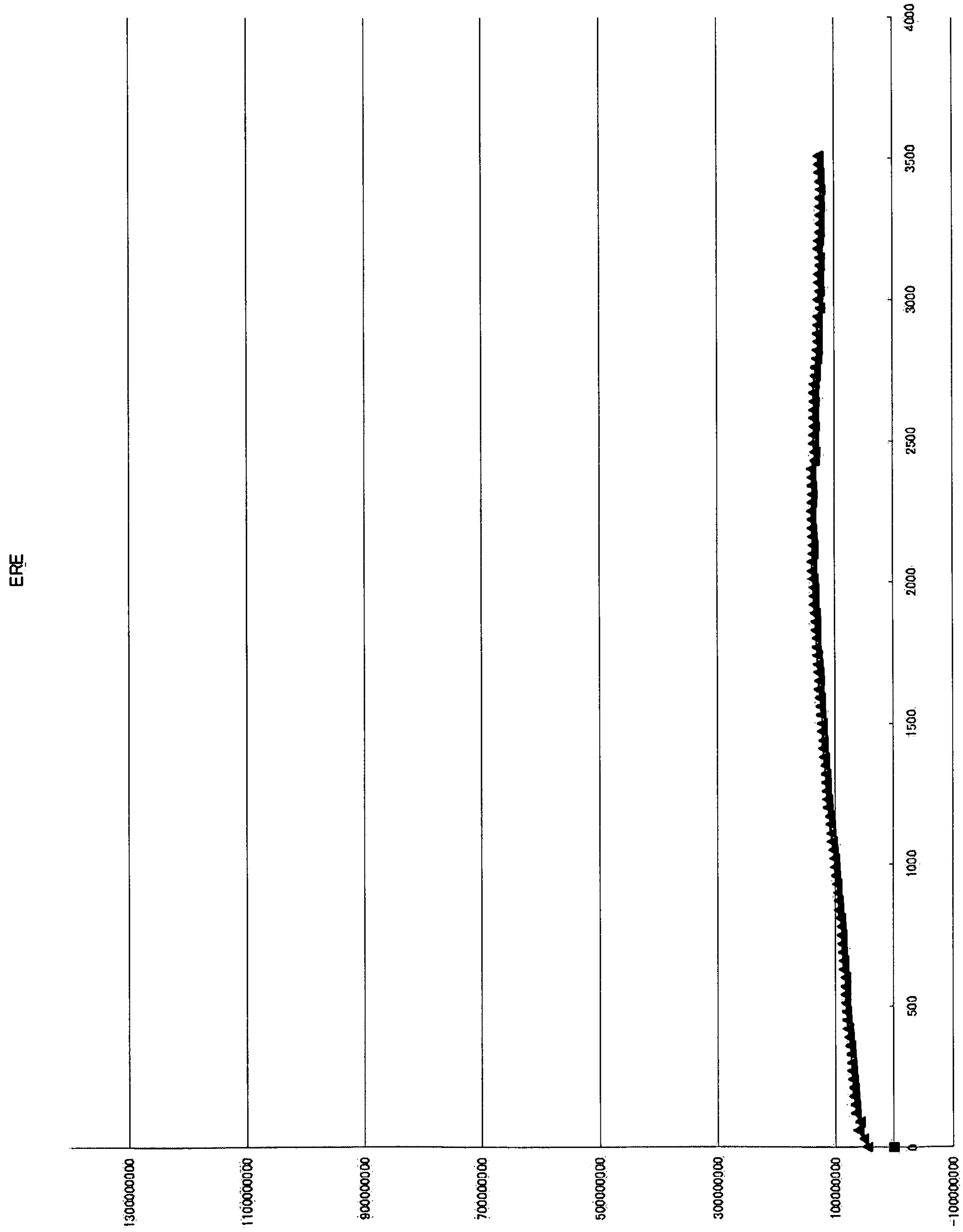
ERE
1300000000
1100000000
900000000
700000000
500000000
300000000
100000000
-100000000
0
500
1000
1500
2000
2500
3000
3500
4000

Fig. 39-13

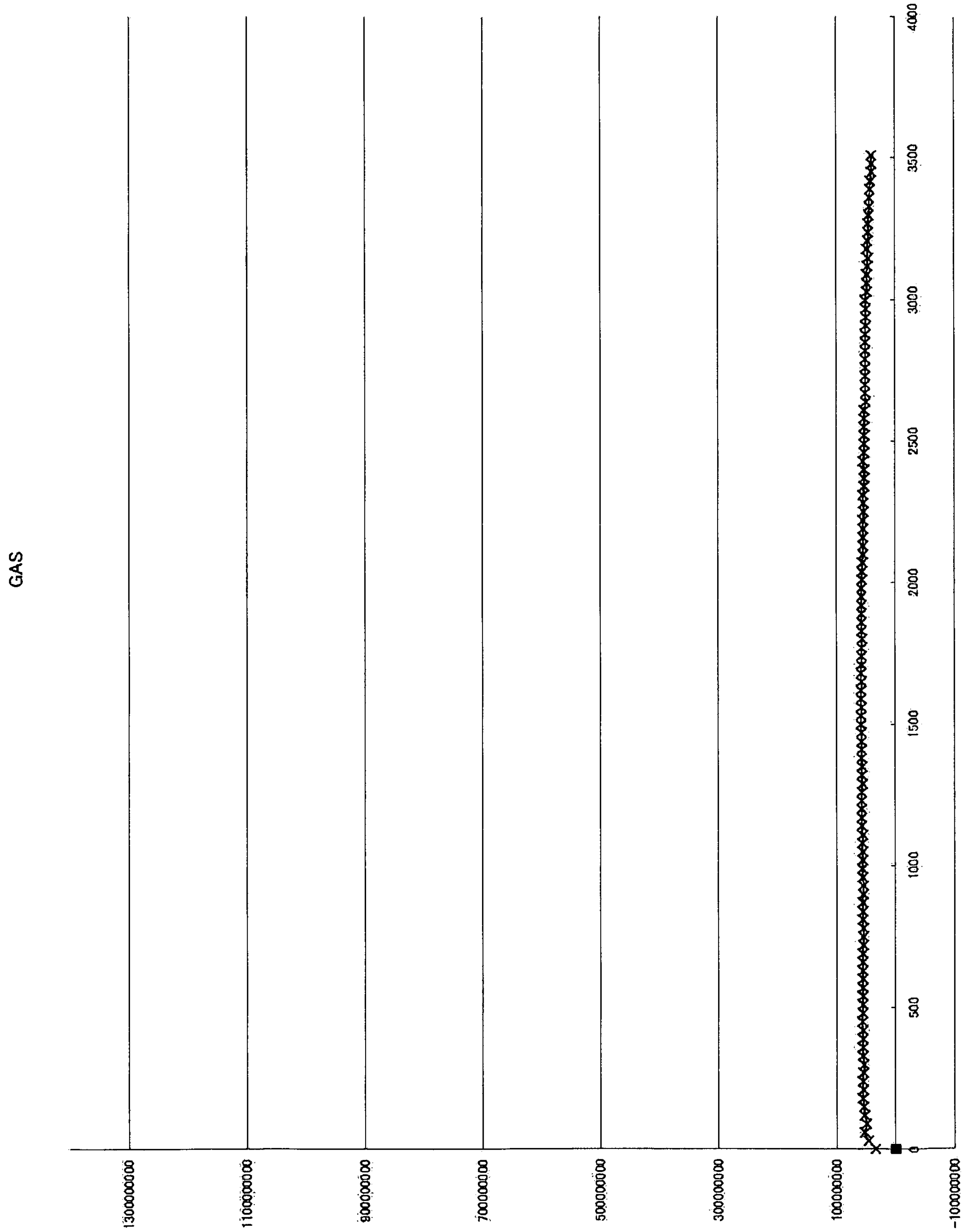
GAS
1300000000
1100000000
900000000
700000000
500000000
300000000
100000000
-100000000
0
500
1000
1500
2000
2500
3000
3500
4000

Fig. 39-14

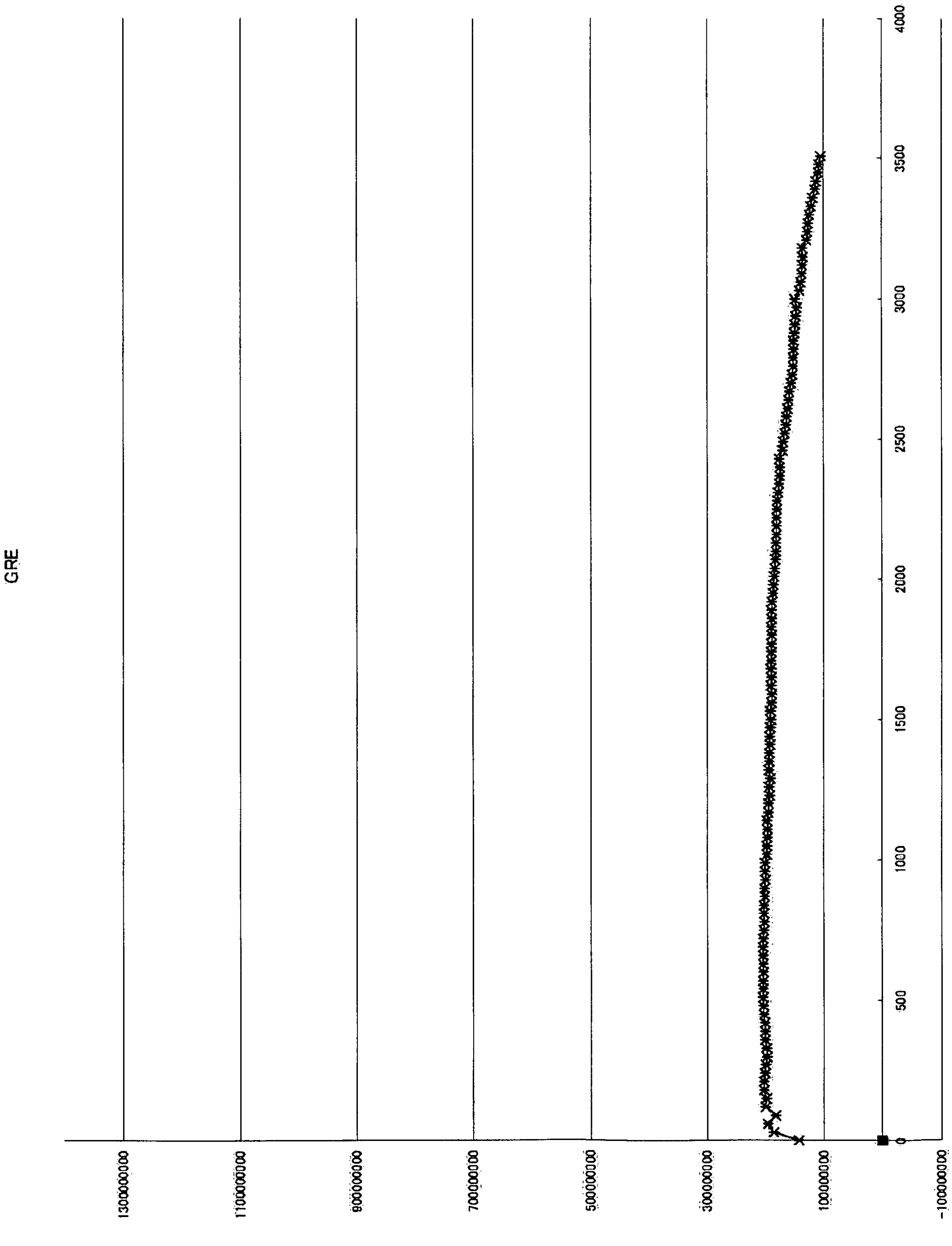
GRE
1300000000
1100000000
900000000
700000000
500000000
300000000
100000000
-100000000
0
500
1000
1500
2000
2500
3000
3500
4000

Fig. 39-15

HSE

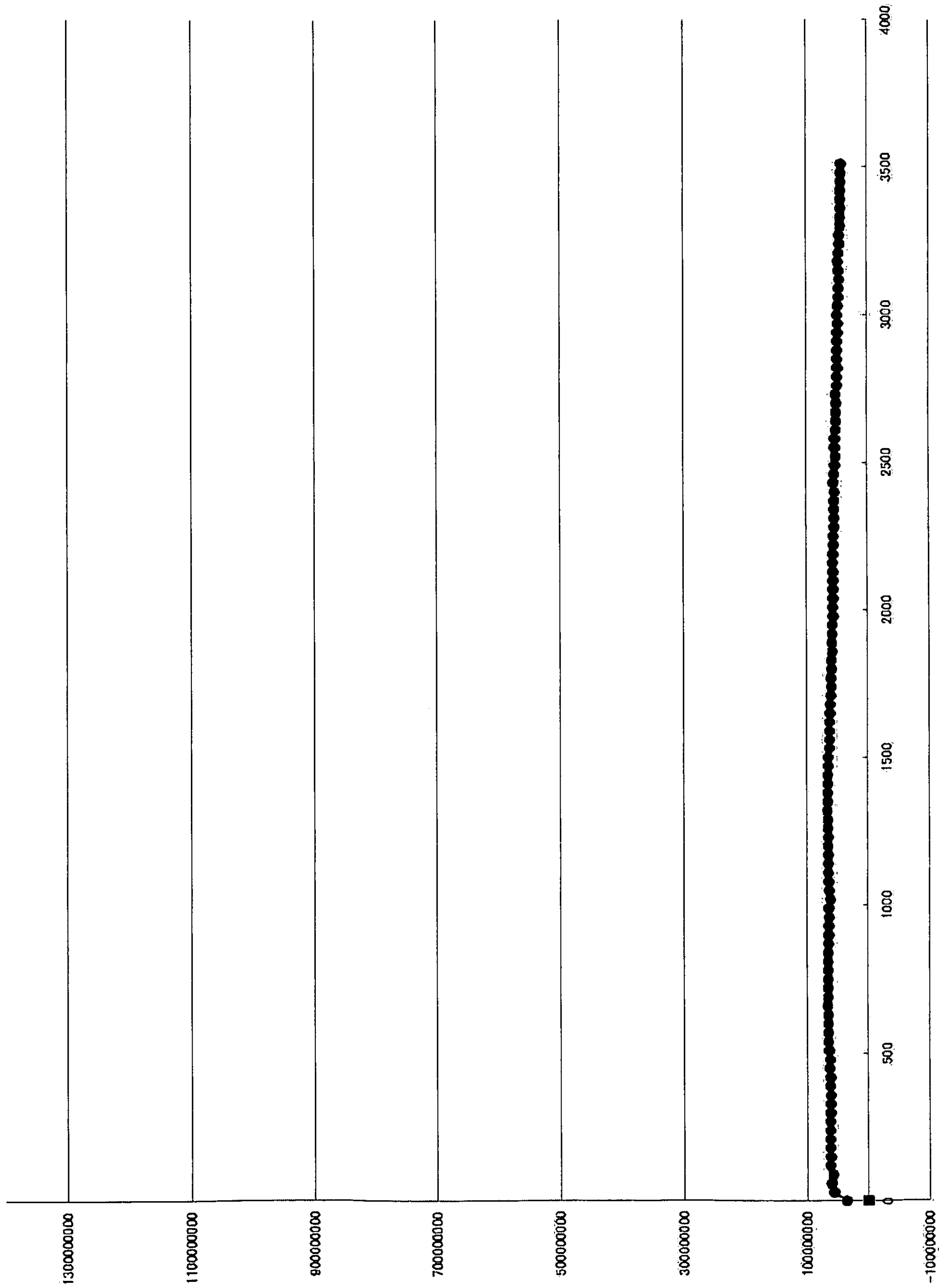
1300000000
1100000000
900000000
700000000
500000000
300000000
100000000
-100000000
0
500
1000
1500
2000
2500
3000
3500
4000

Fig. 39-16

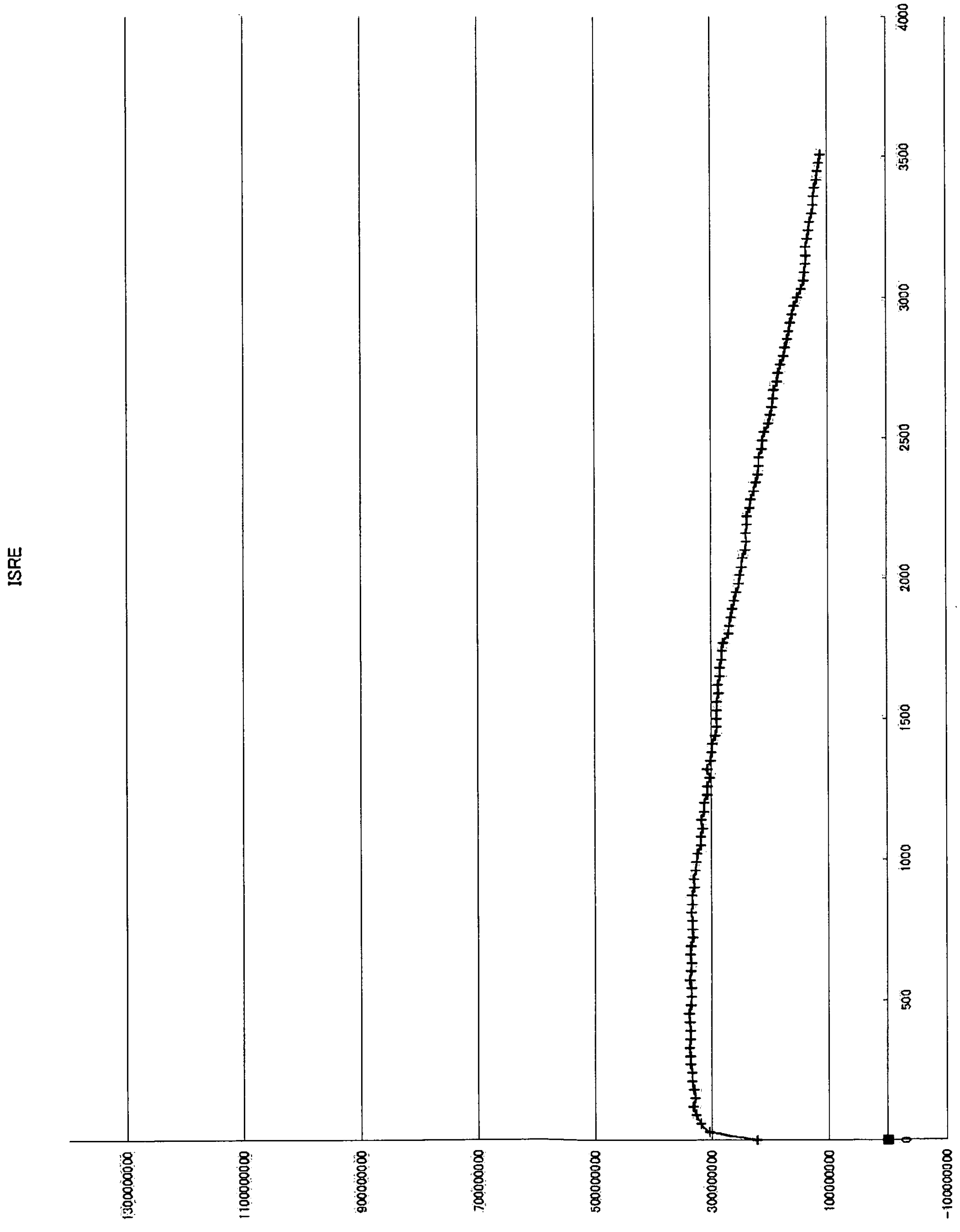
ISRE
1300000000
1100000000
900000000
700000000
500000000
300000000
100000000
-100000000
0
500
1000
1500
2000
2500
3000
3500
4000

Fig. 39-17

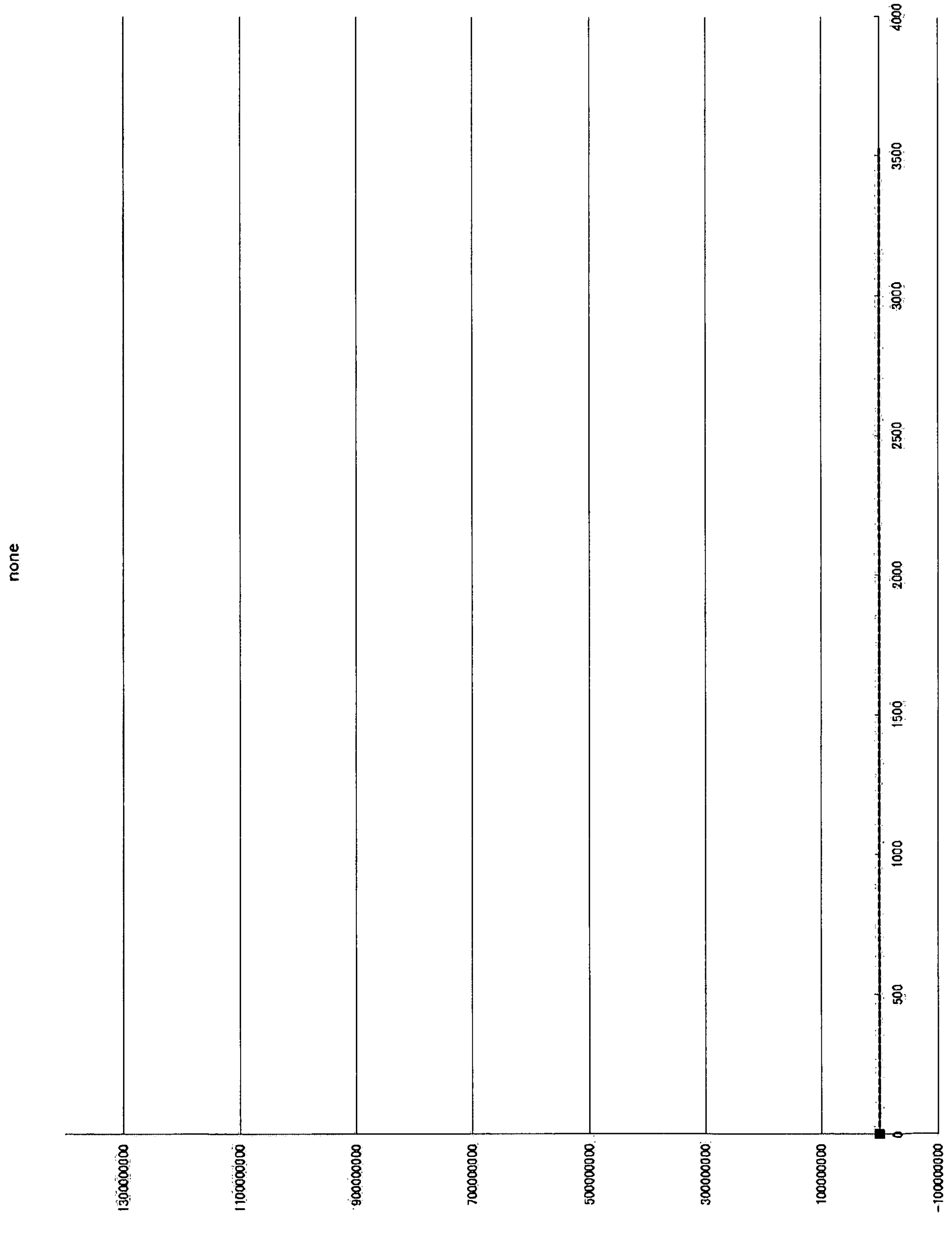
none
1300000000
1100000000
900000000
700000000
500000000
300000000
100000000
-100000000
0
500
1000
1500
2000
2500
3000
3500
4000

Fig. 39-18

ERE

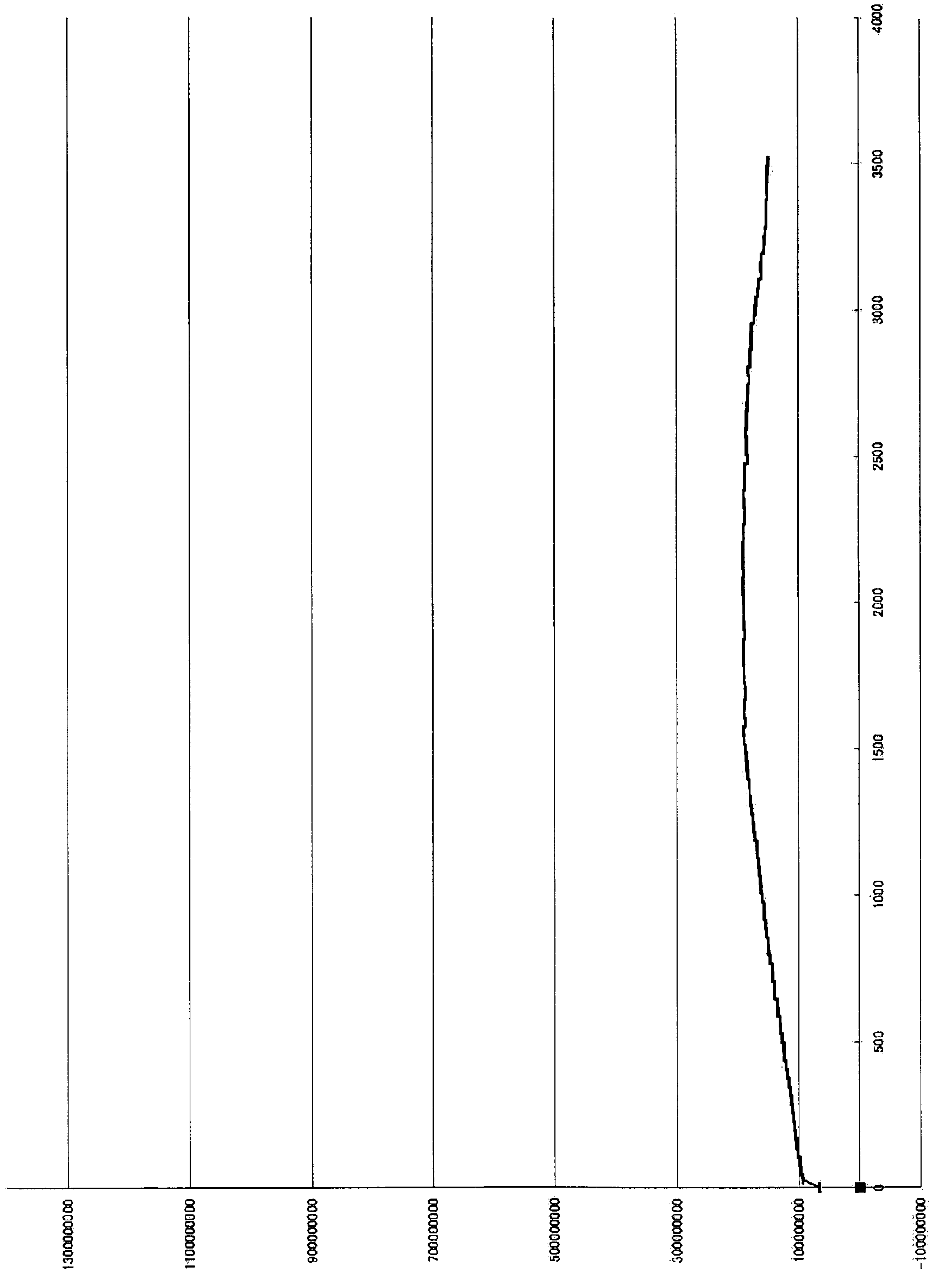
1300000000
1100000000
900000000
700000000
500000000
300000000
100000000
-100000000
0
500
1000
1500
2000
2500
3000
3500
4000

Fig. 39-19

GAS

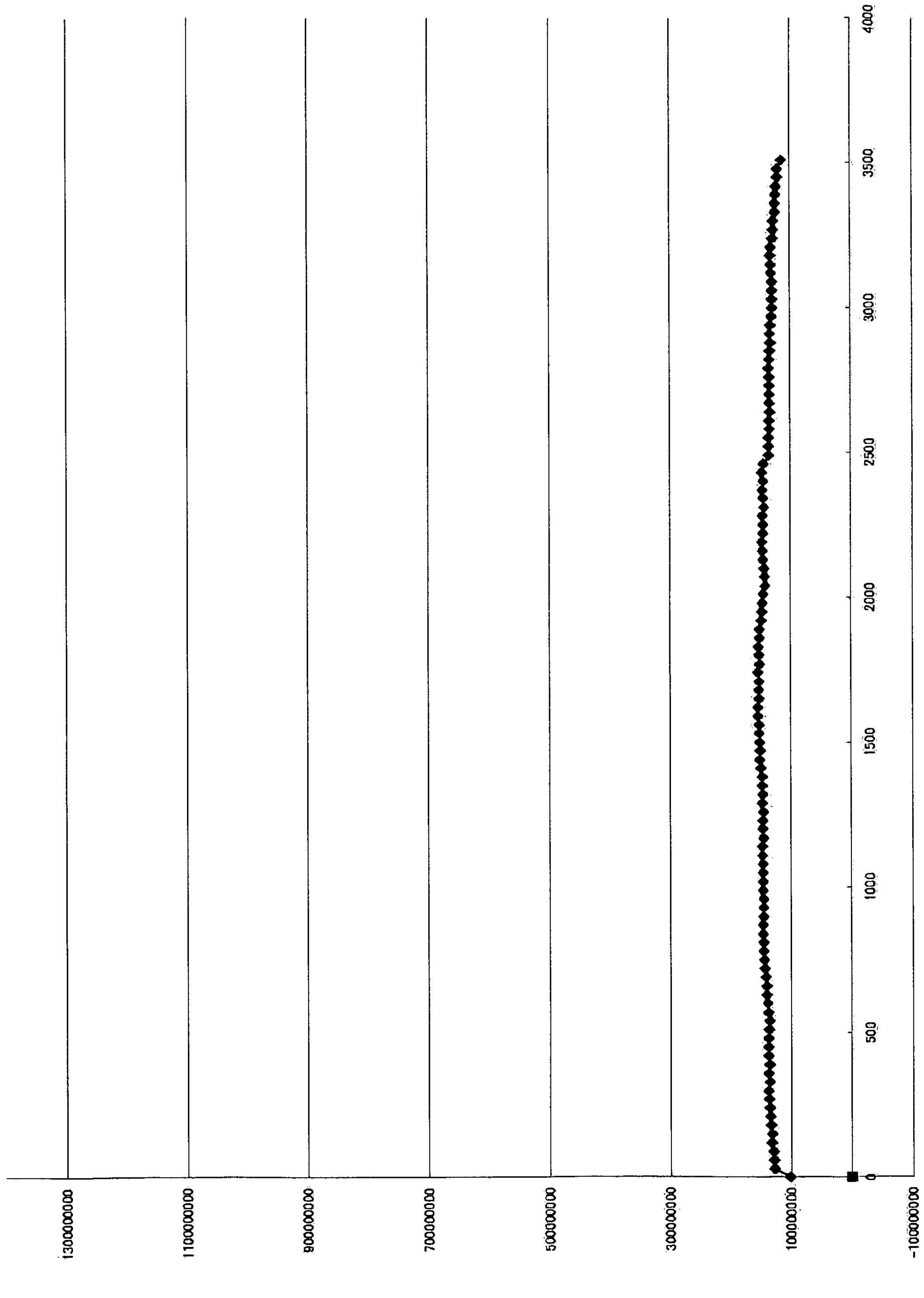
1300000000
1100000000
900000000
700000000
500000000
300000000
100000000
-100000000
0
500
1000
1500
2000
2500
3000
3500
4000

Fig. 39-20

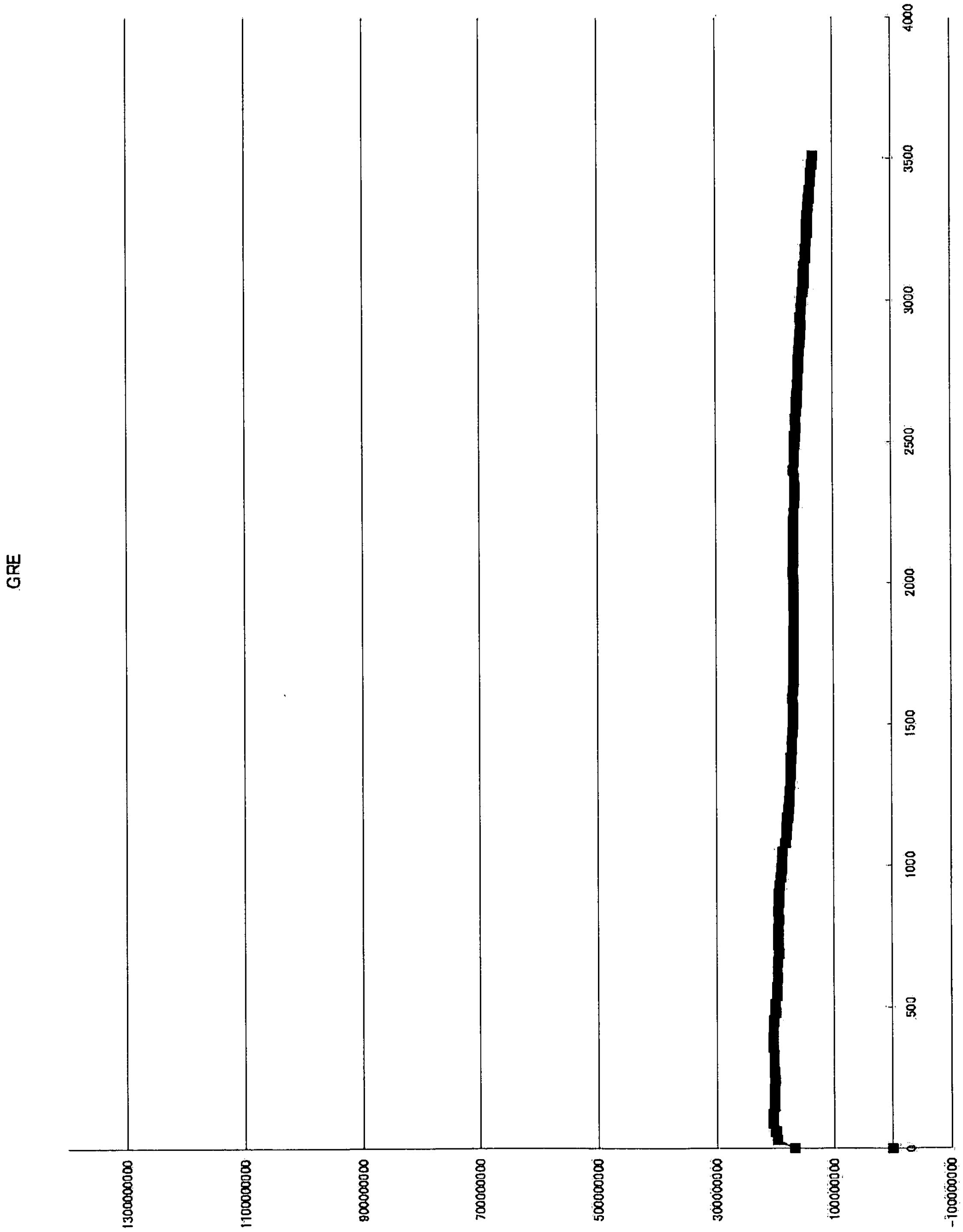
GRE
1300000000
1100000000
900000000
700000000
500000000
300000000
100000000
-100000000
0
500
1000
1500
2000
2500
3000
3500
4000

Fig. 39-21

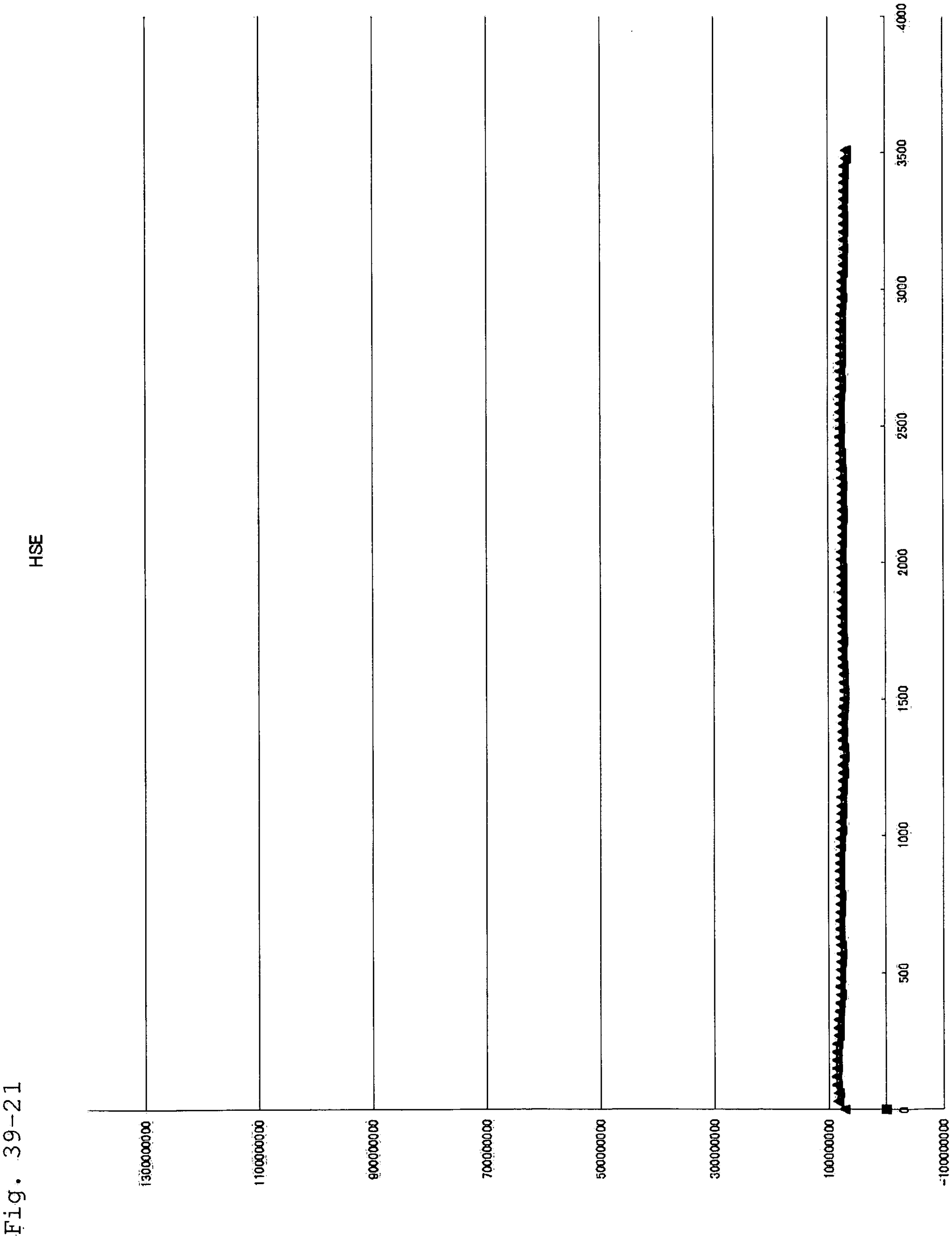
HSE
1300000000
1100000000
900000000
700000000
500000000
300000000
100000000
-100000000
0
500
1000
1500
2000
2500
3000
3500
4000

Fig. 39-22

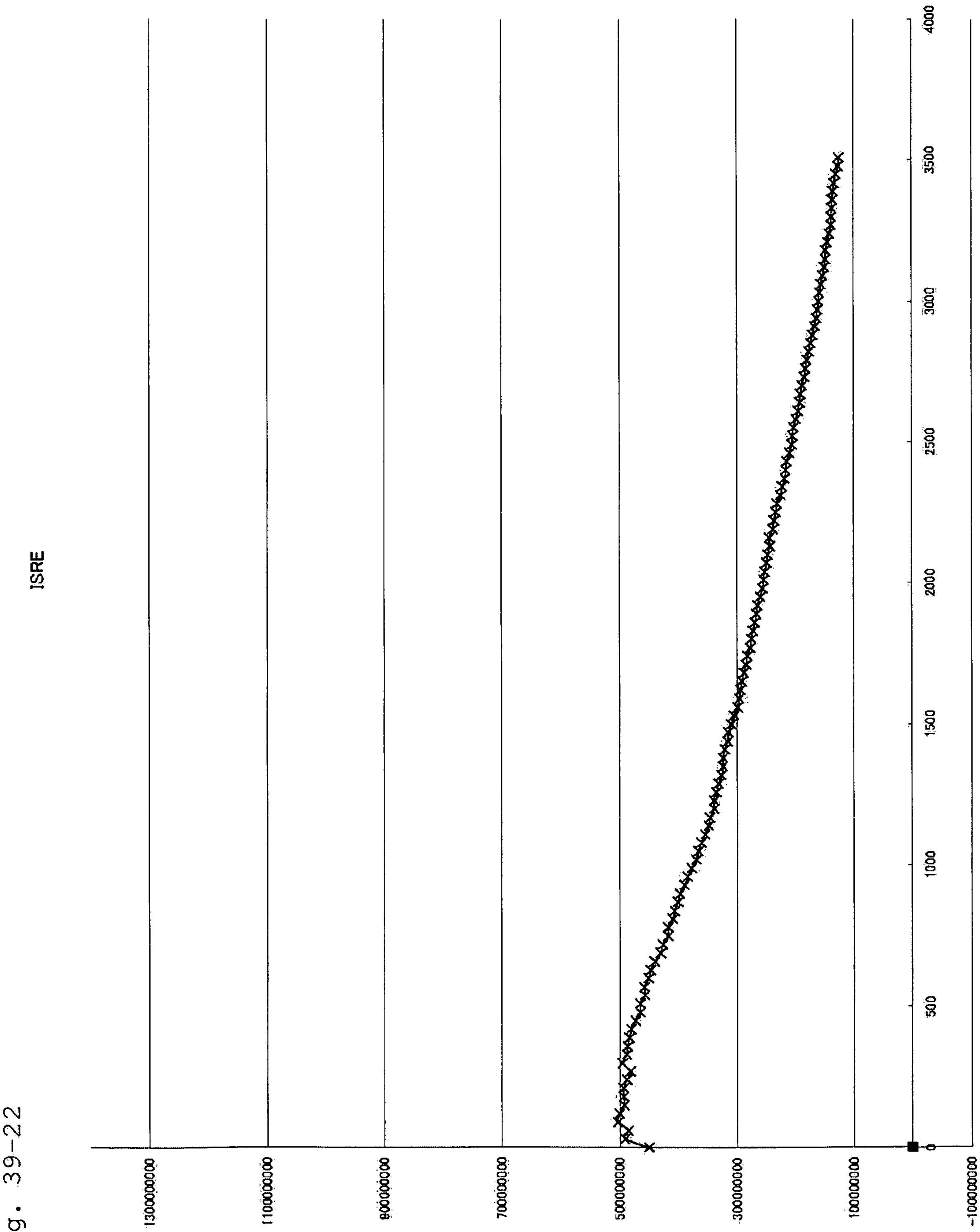
ISRE
1300000000
1100000000
900000000
700000000
500000000
300000000
100000000
-100000000
0
500
1000
1500
2000
2500
3000
3500
4000

Fig. 39-23

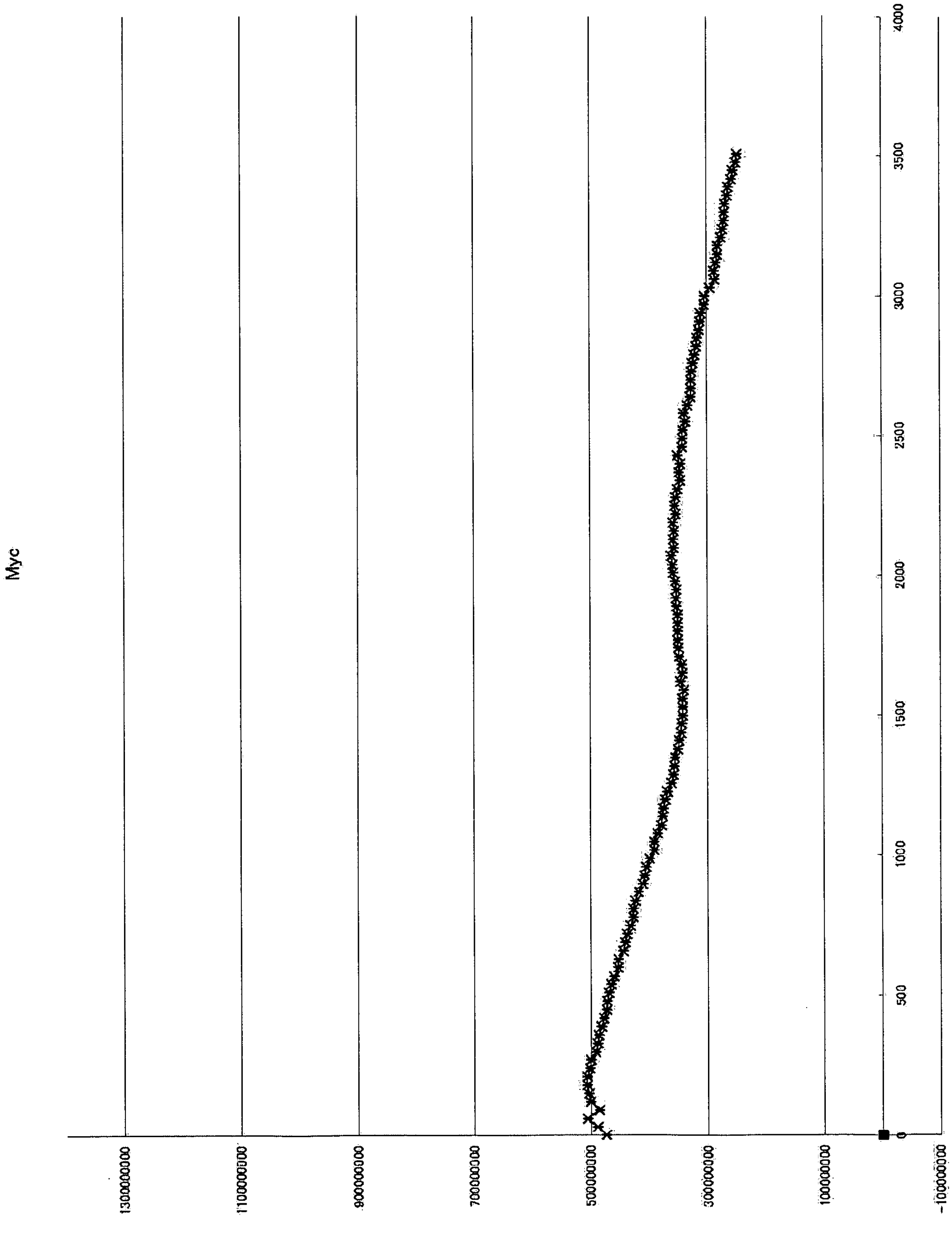
Myc
1300000000
1100000000
900000000
700000000
500000000
300000000
100000000
-100000000
0
500
1000
1500
2000
2500
3000
3500
4000

Fig. 39-24

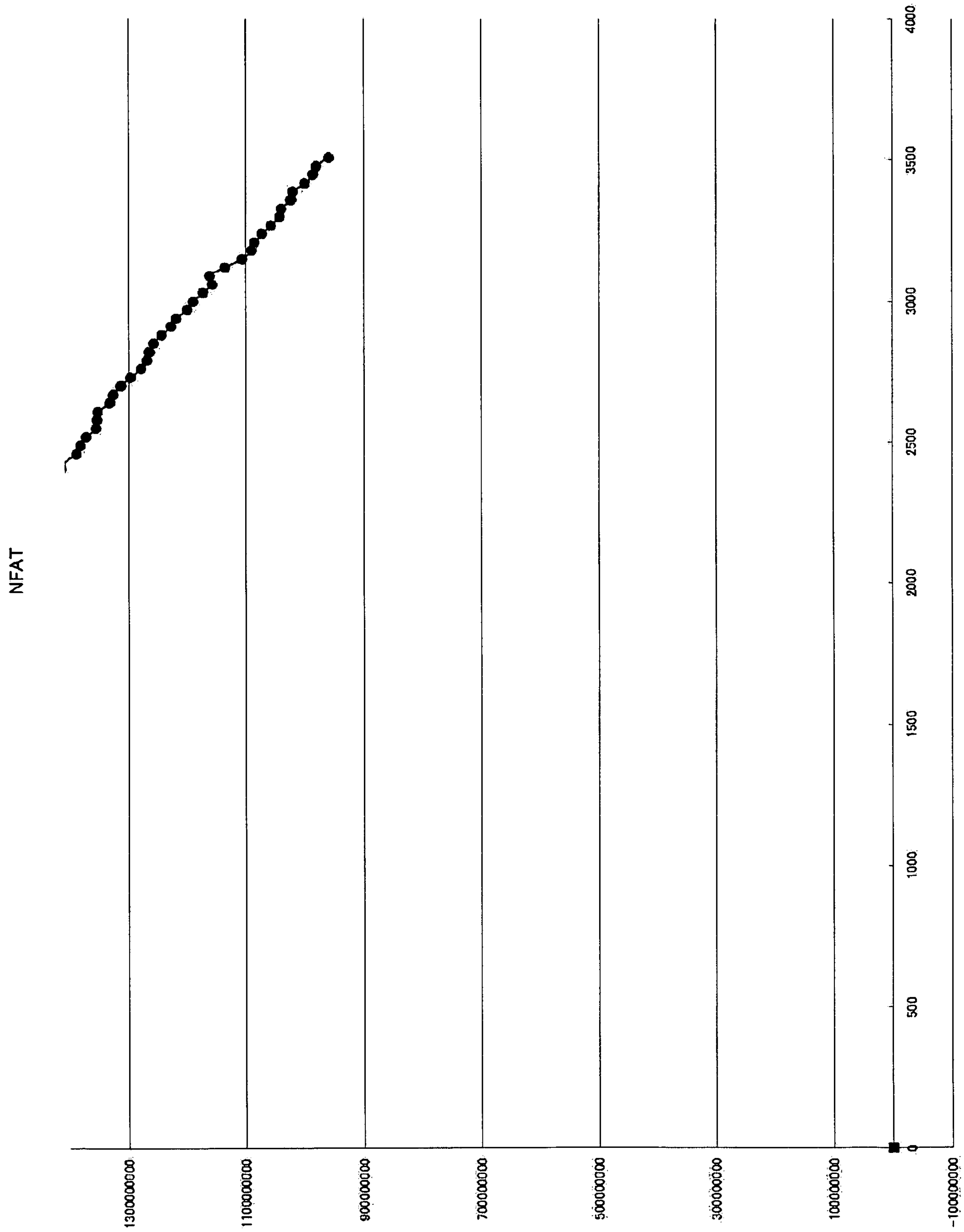
NFAT
1300000000
1100000000
900000000
700000000
500000000
300000000
100000000
-100000000
0
500
1000
1500
2000
2500
3000
3500
4000

Fig. 39-25

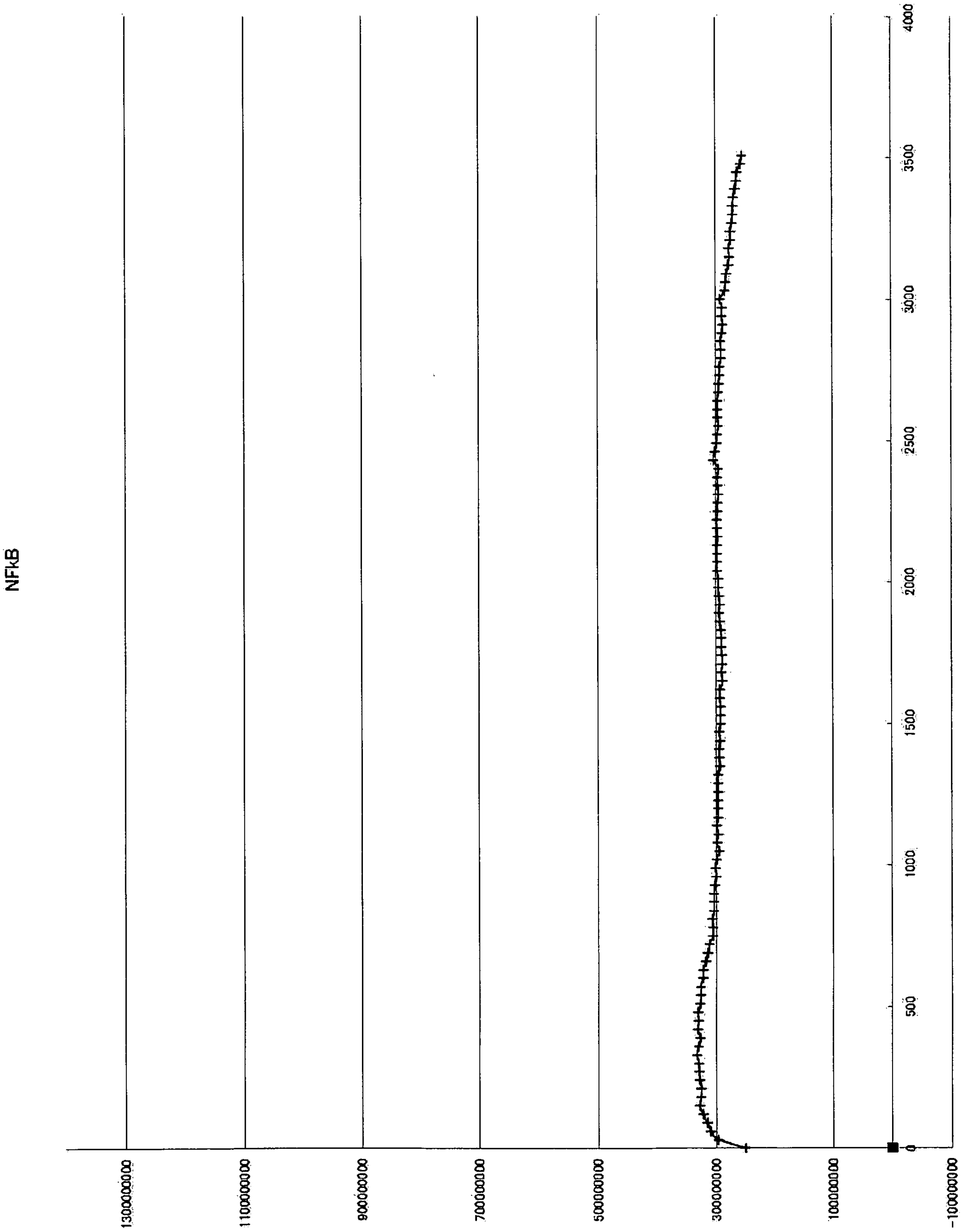
NFkB
1300000000
1100000000
900000000
700000000
500000000
300000000
100000000
-100000000
0
500
1000
1500
2000
2500
3000
3500
4000

Fig. 39-26

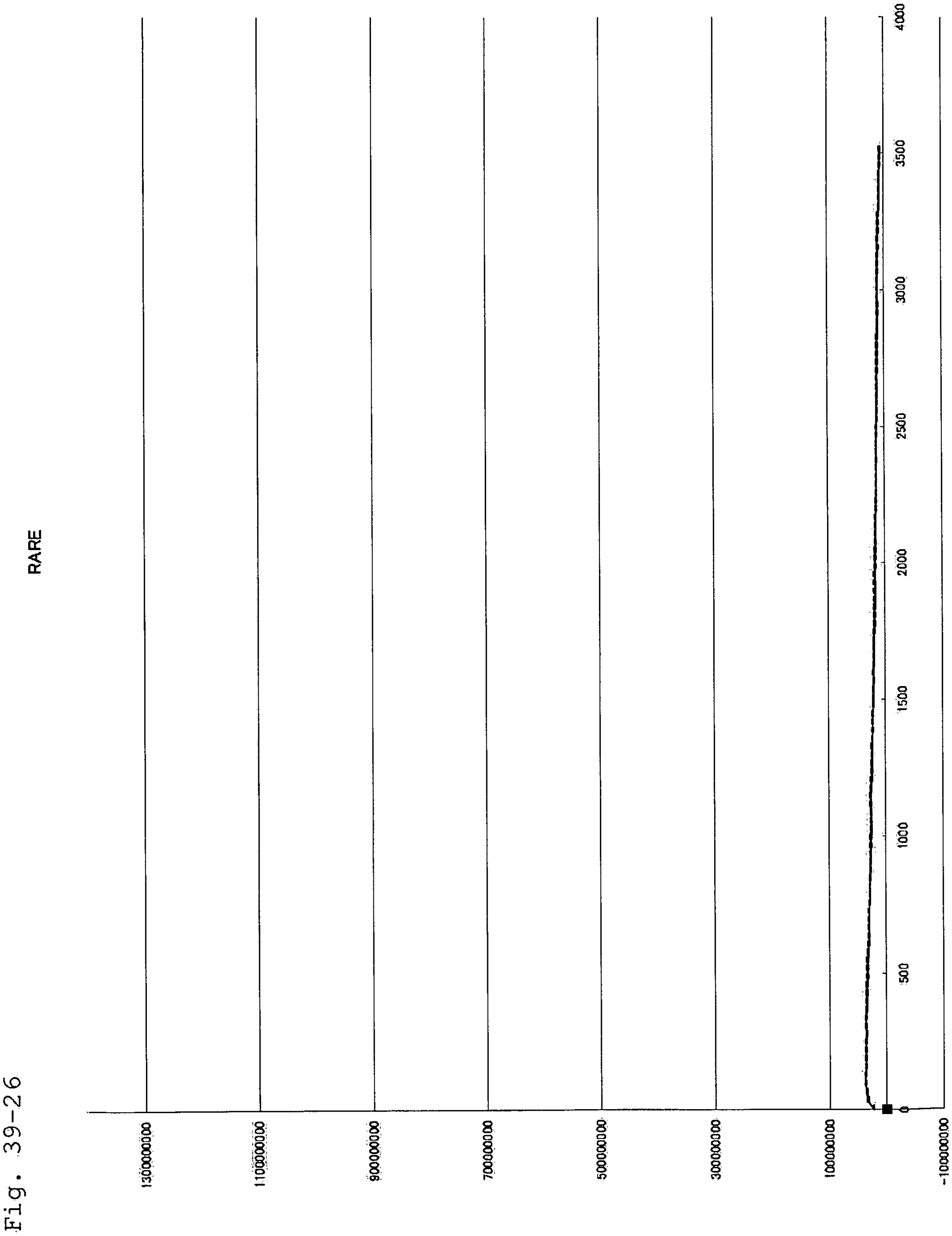
RARE
1300000000
1100000000
900000000
700000000
500000000
300000000
100000000
-100000000
0
500
1000
1500
2000
2500
3000
3500
4000

Fig. 39-27

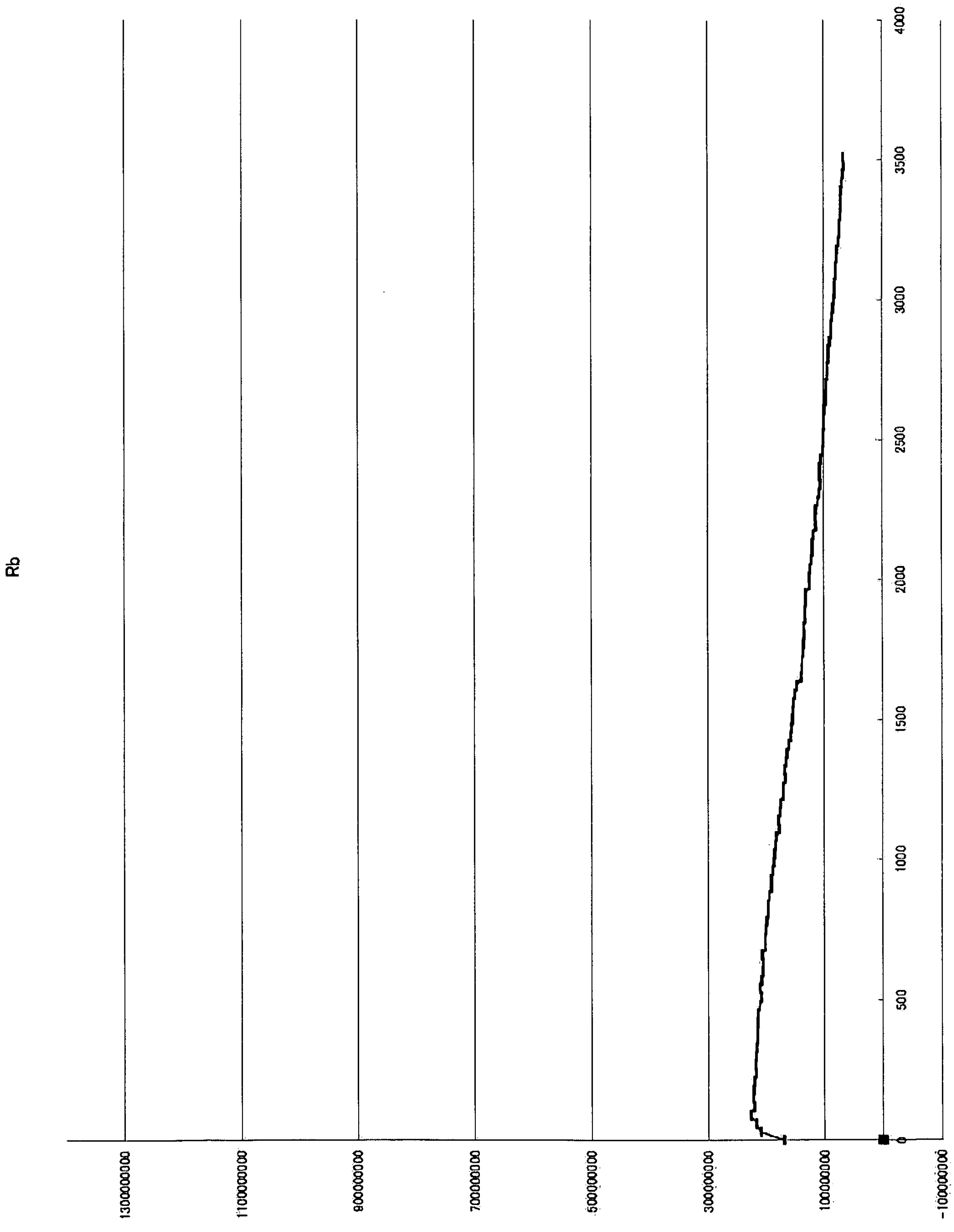

Rb
1300000000
1100000000
900000000
700000000
500000000
300000000
100000000
-100000000
0
500
1000
1500
2000
2500
3000
3500
4000

Fig. 39-28

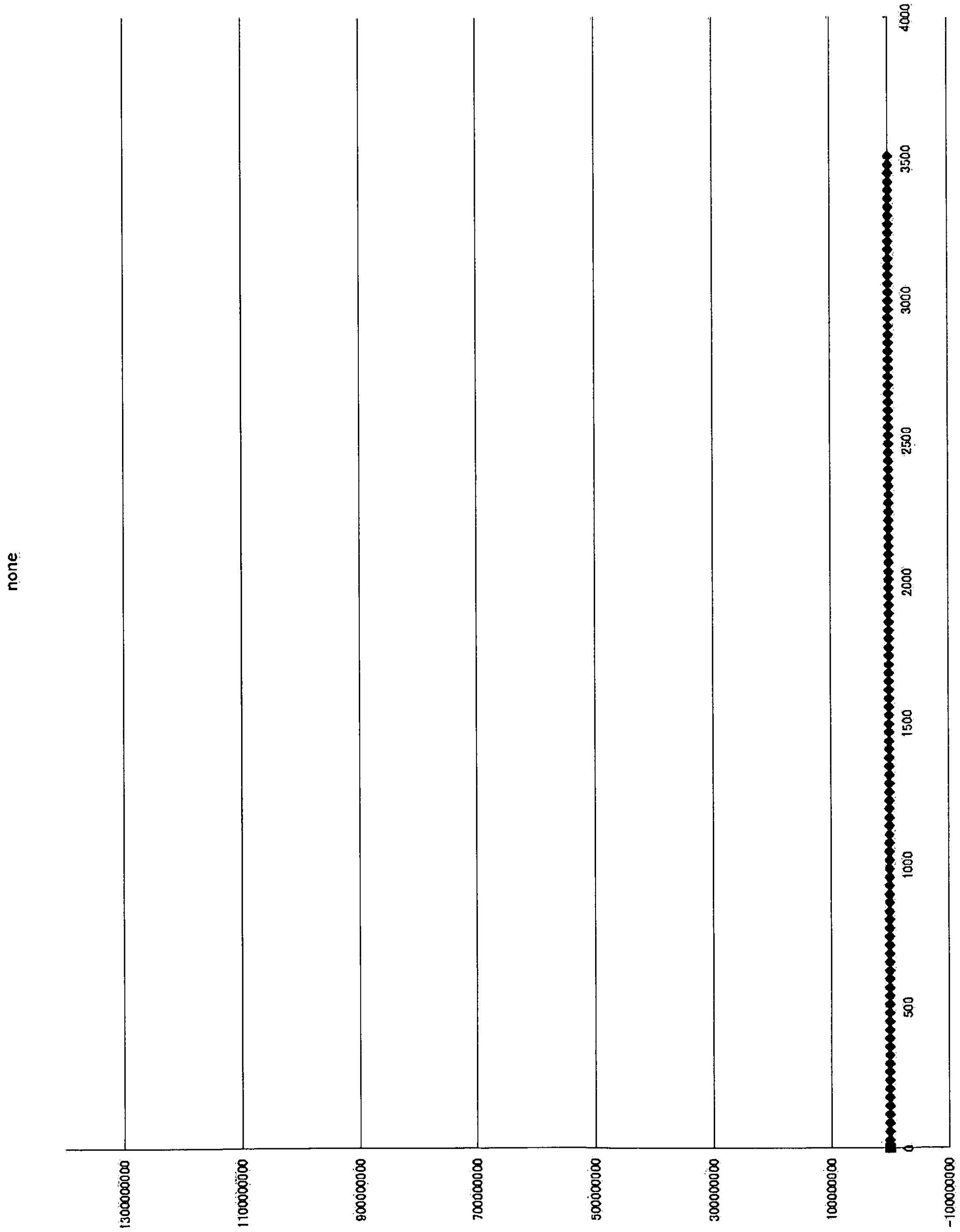
none
1300000000
1100000000
900000000
700000000
500000000
300000000
100000000
-100000000
0
500
1000
1500
2000
2500
3000
3500
4000

Fig. 39-29

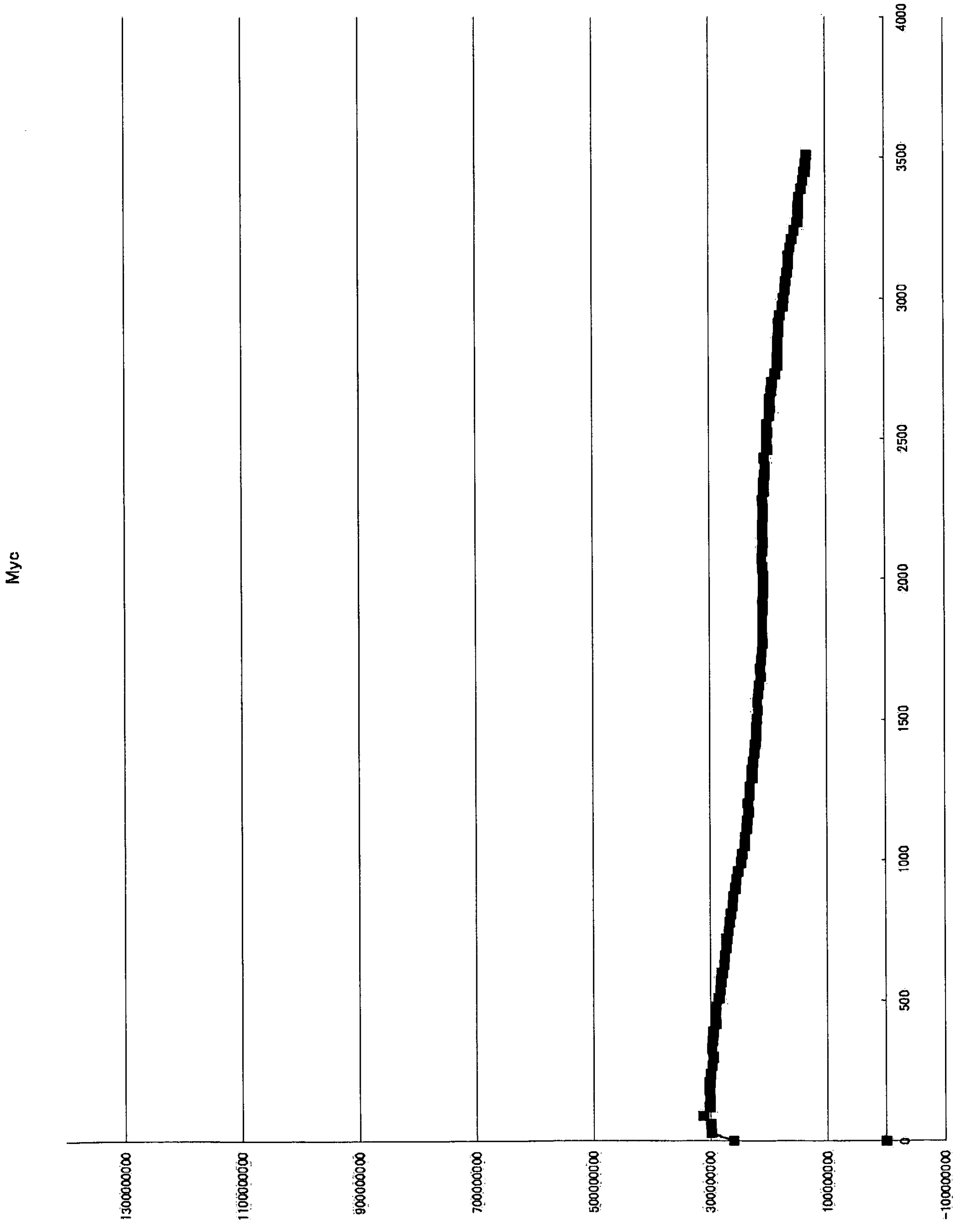
Myc
1300000000
1100000000
900000000
700000000
500000000
300000000
100000000
-100000000
0
500
1000
1500
2000
2500
3000
3500
4000

Fig. 39-30

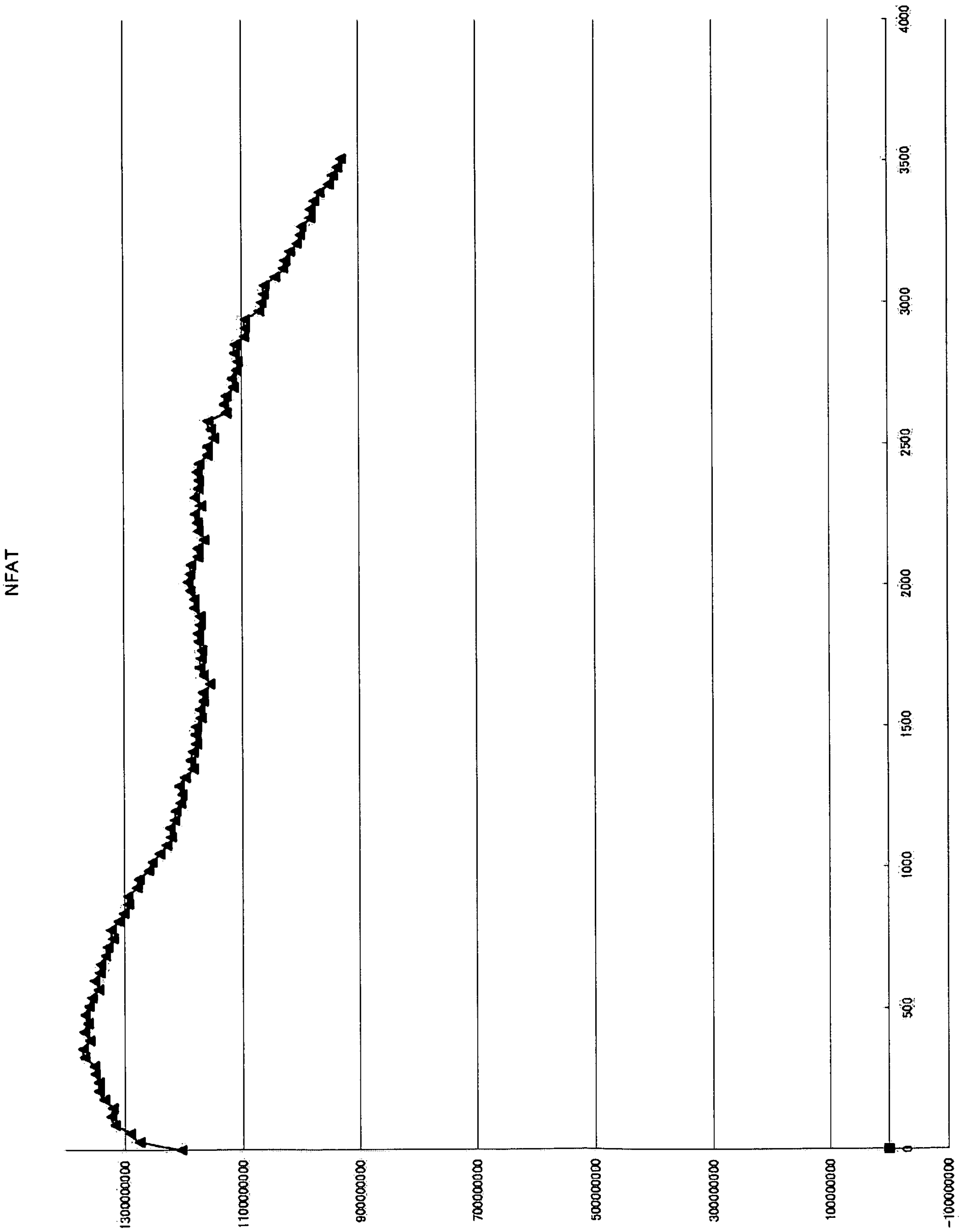
NFAT
1300000000
1100000000
900000000
700000000
500000000
300000000
100000000
-100000000
0
500
1000
1500
2000
2500
3000
3500
4000

Fig. 39-31

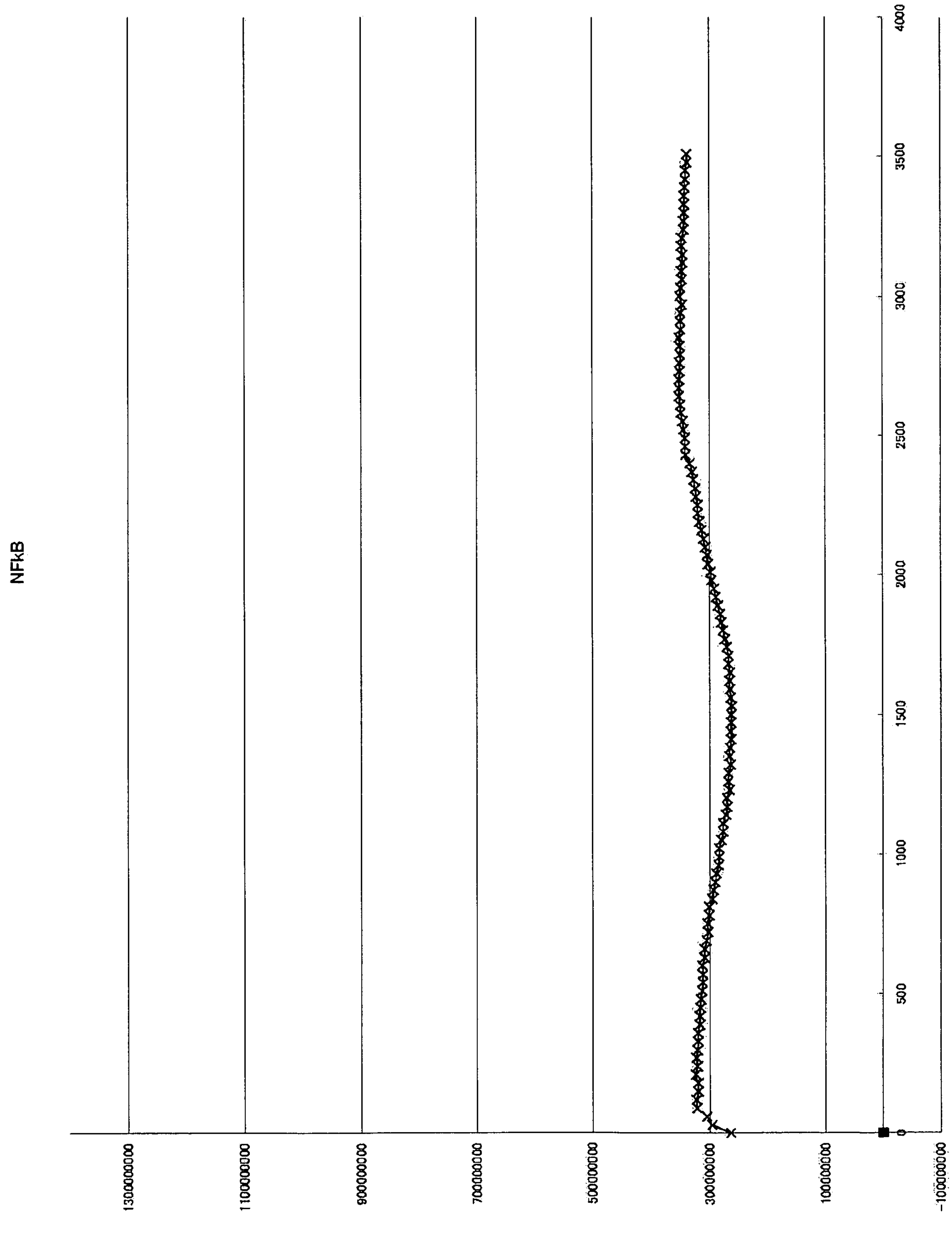
NFkB
1300000000
1100000000
900000000
700000000
500000000
300000000
100000000
-100000000
0
500
1000
1500
2000
2500
3000
3500
4000

Fig. 39-32

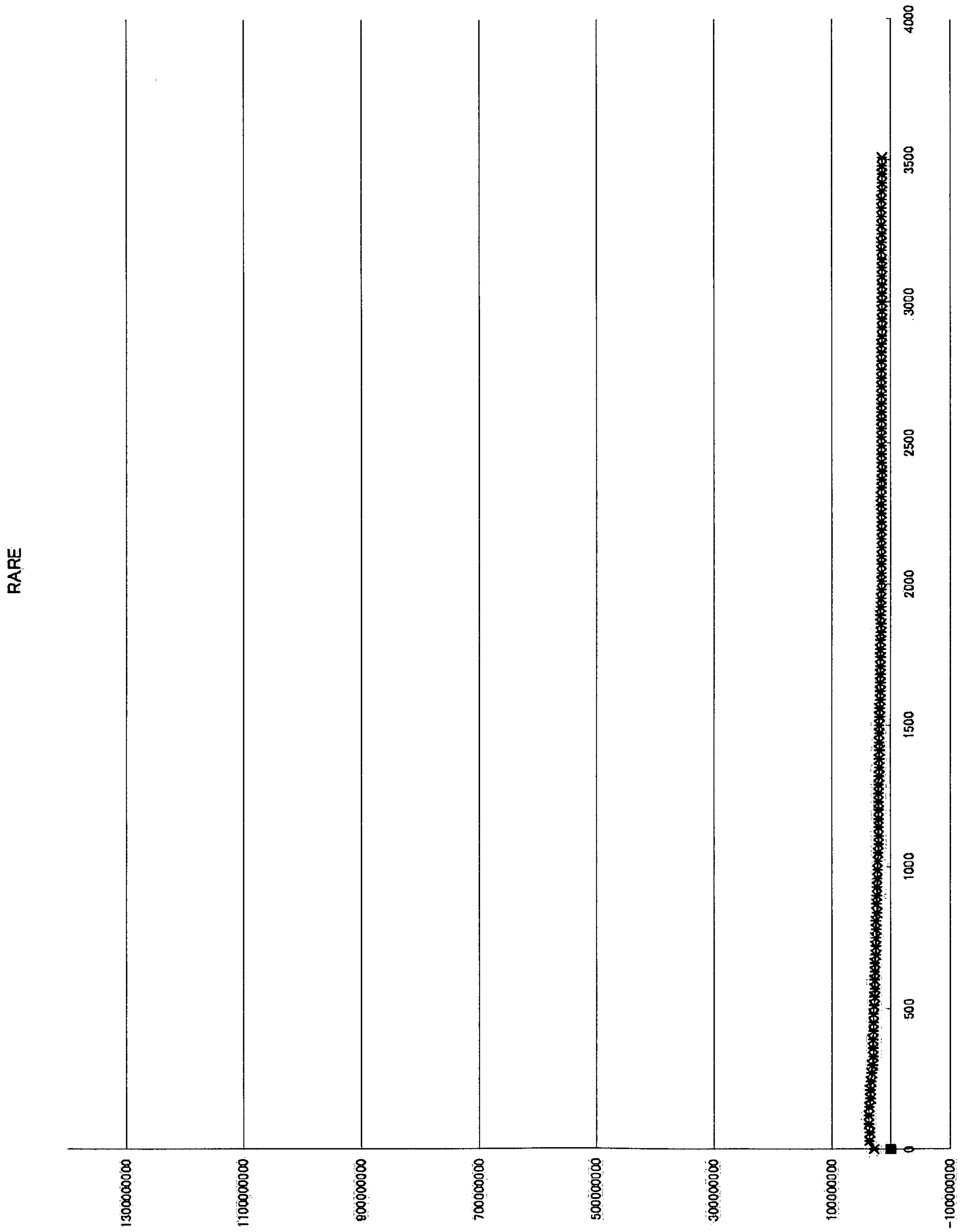
RARE
1300000000
1100000000
900000000
700000000
500000000
300000000
100000000
-100000000
0
500
1000
1500
2000
2500
3000
3500
4000

Fig. 39-33

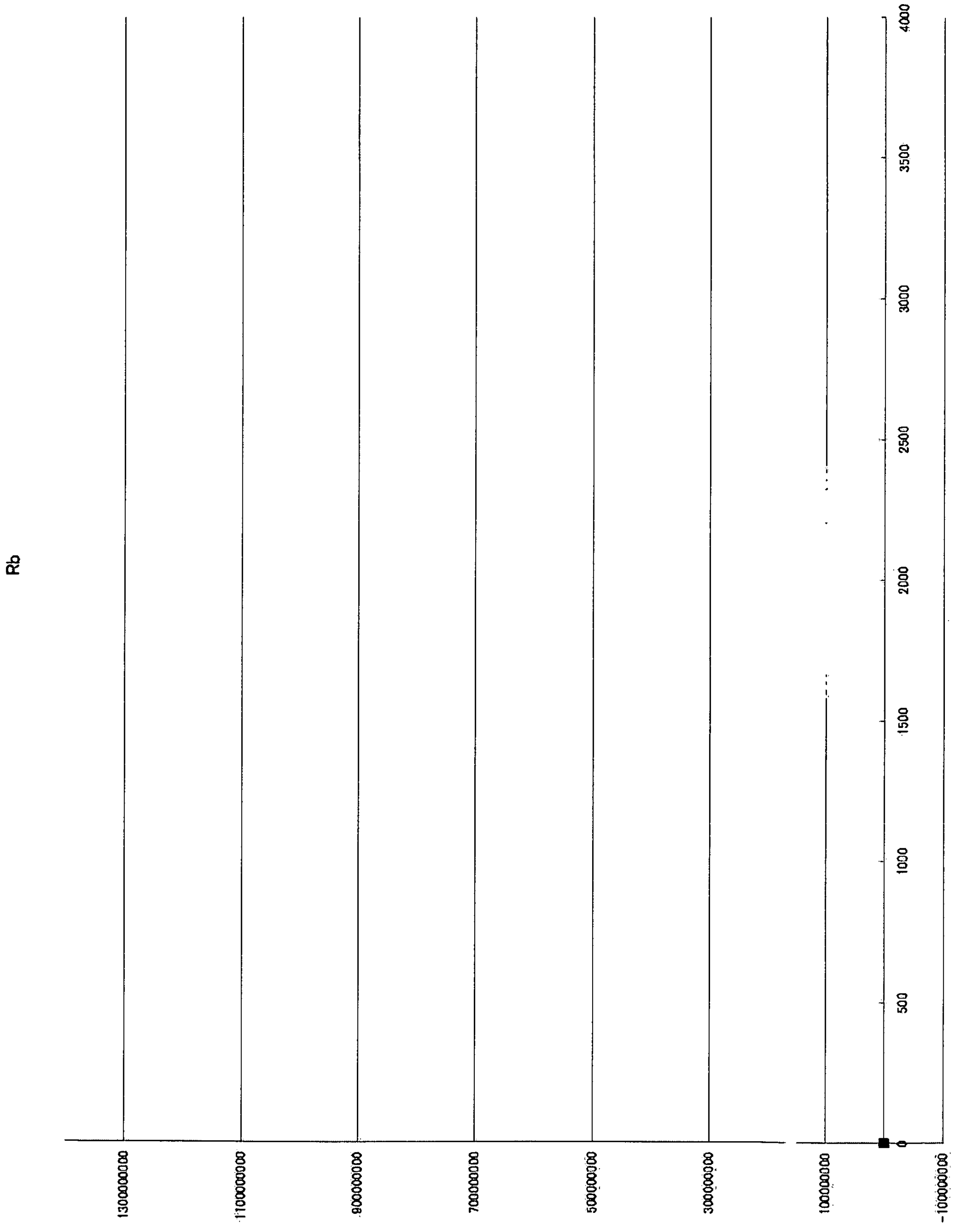
Rb
1300000000
1100000000
900000000
700000000
500000000
300000000
100000000
-100000000
0
500
1000
1500
2000
2500
3000
3500
4000

Fig. 39-34

STAT3

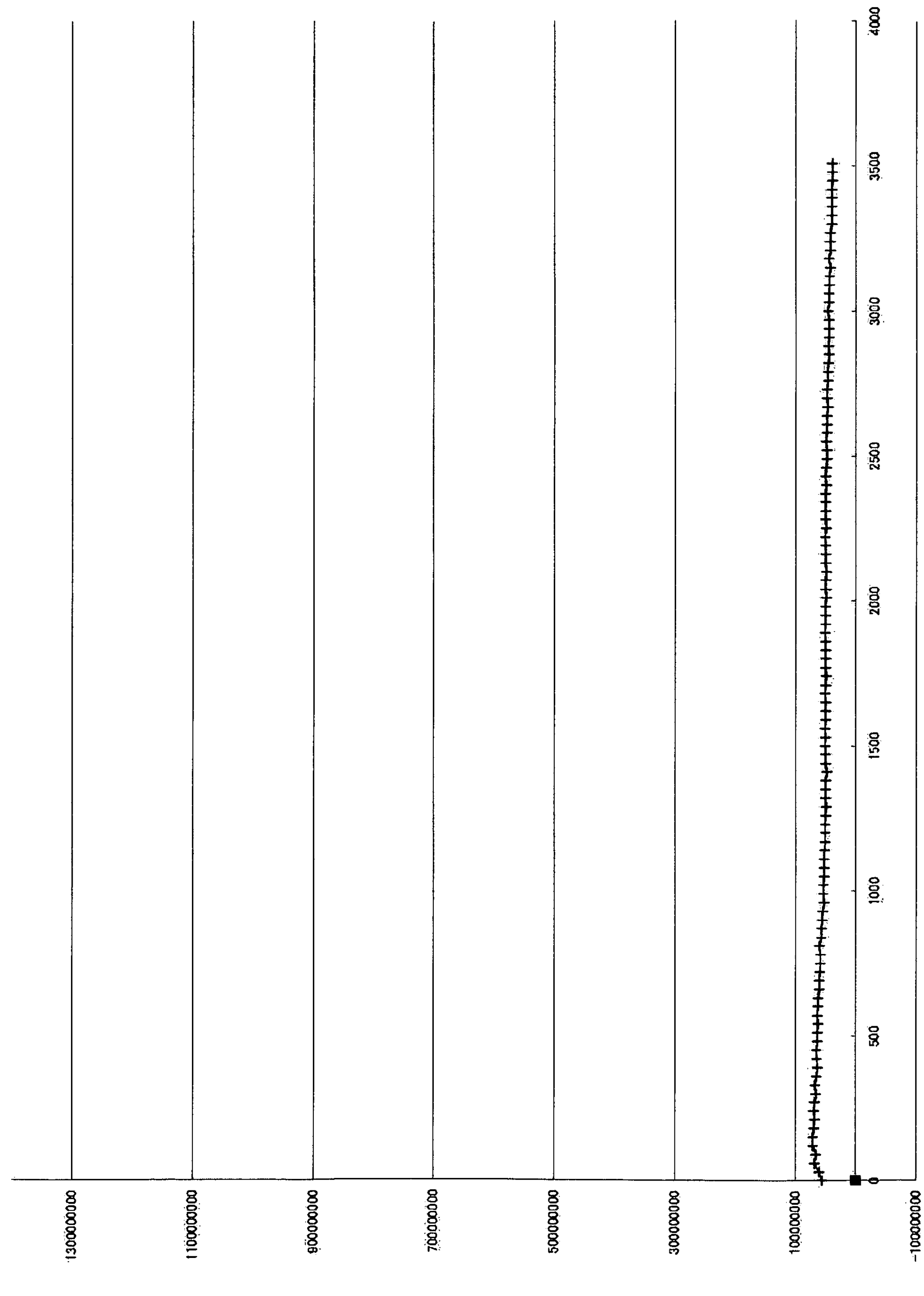

Fig. 39-35

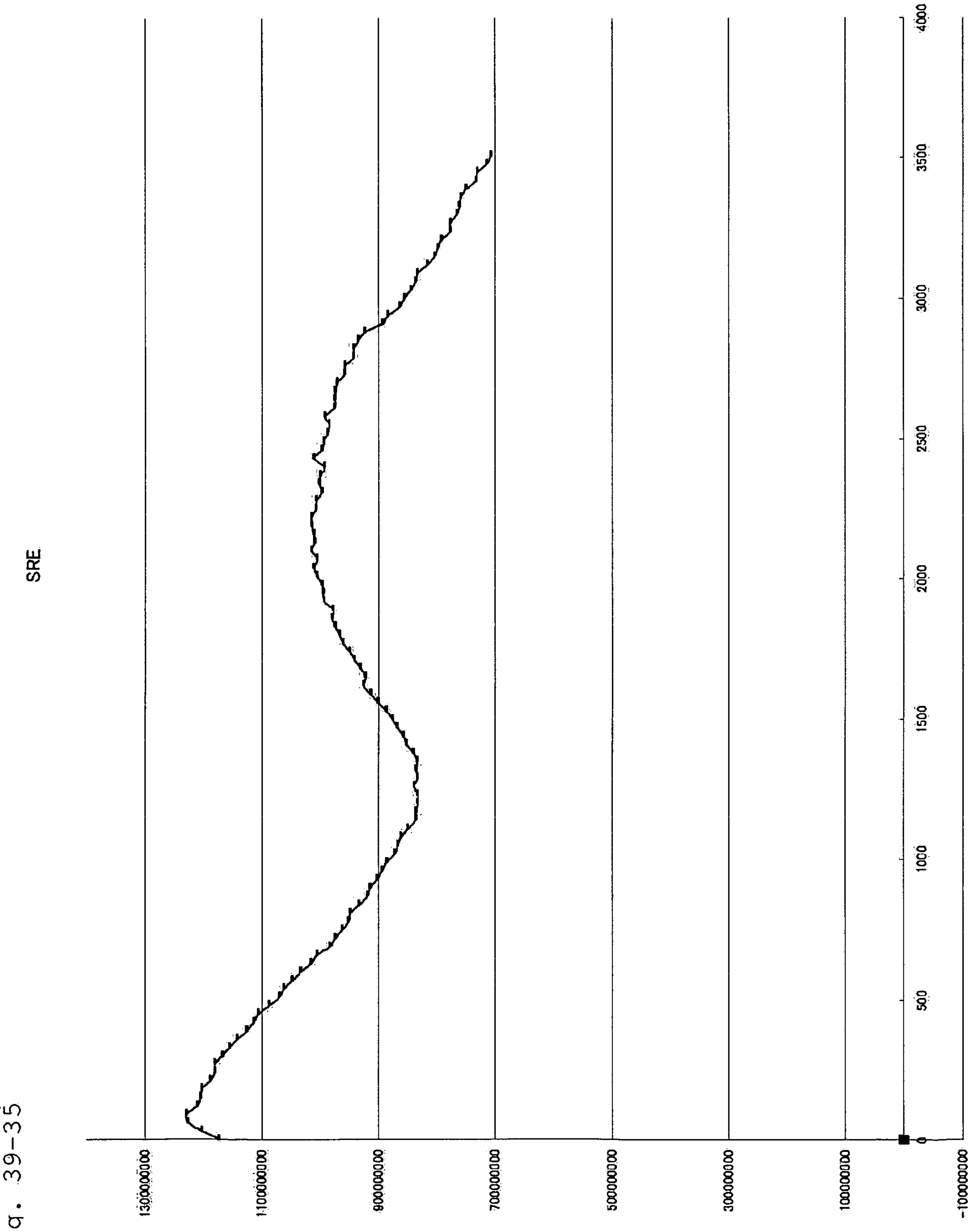
SRE
1300000000
1100000000
900000000
700000000
500000000
300000000
100000000
-100000000
0
500
1000
1500
2000
2500
3000
3500
4000

Fig. 39-36

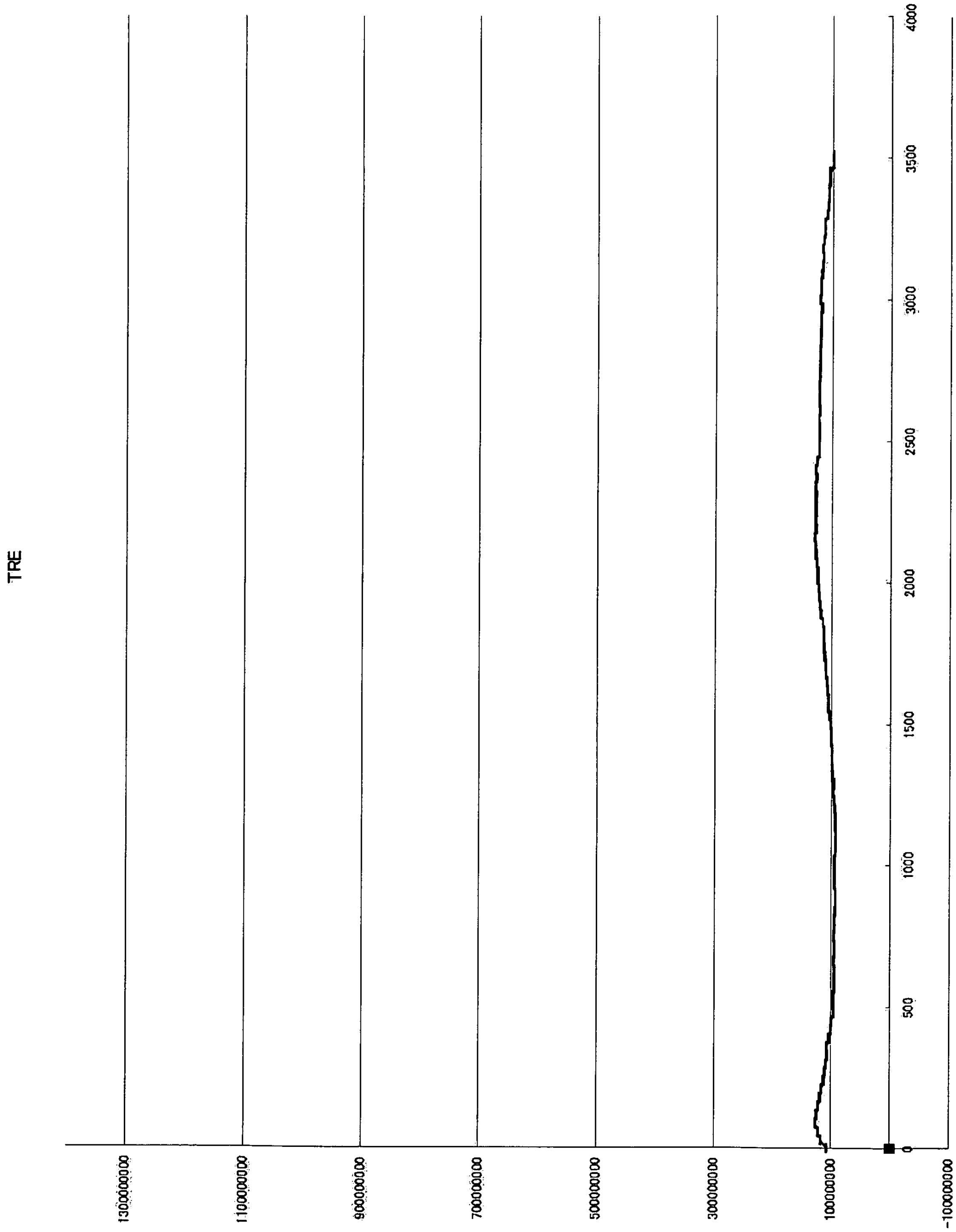
TRE
1300000000
1100000000
900000000
700000000
500000000
300000000
100000000
-100000000
0
500
1000
1500
2000
2500
3000
3500
4000

Fig. 39-37

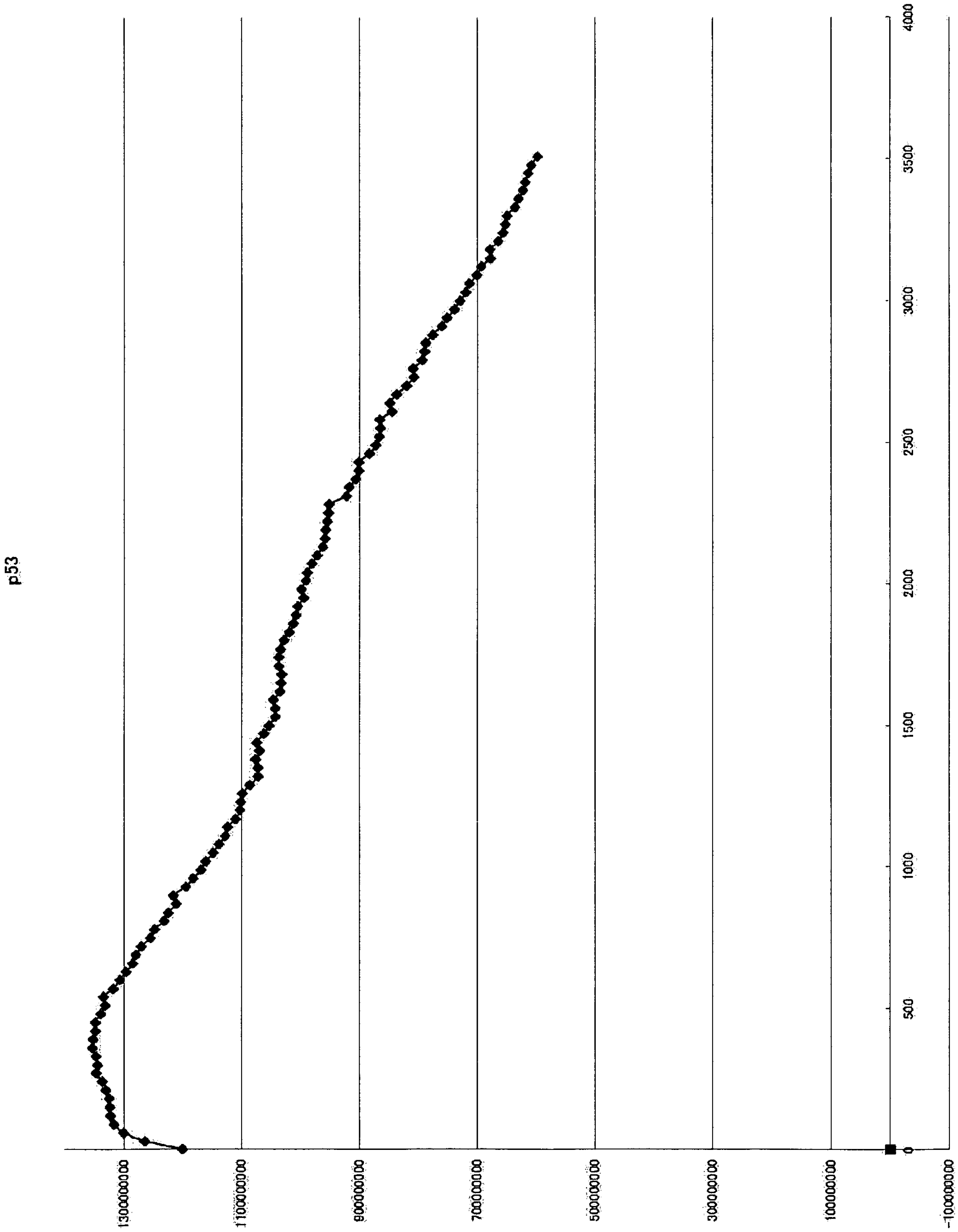
p53
130000000
110000000
90000000
70000000
50000000
30000000
10000000
-10000000
0
500
1000
1500
2000
2500
3000
3500
4000

Fig. 39-38

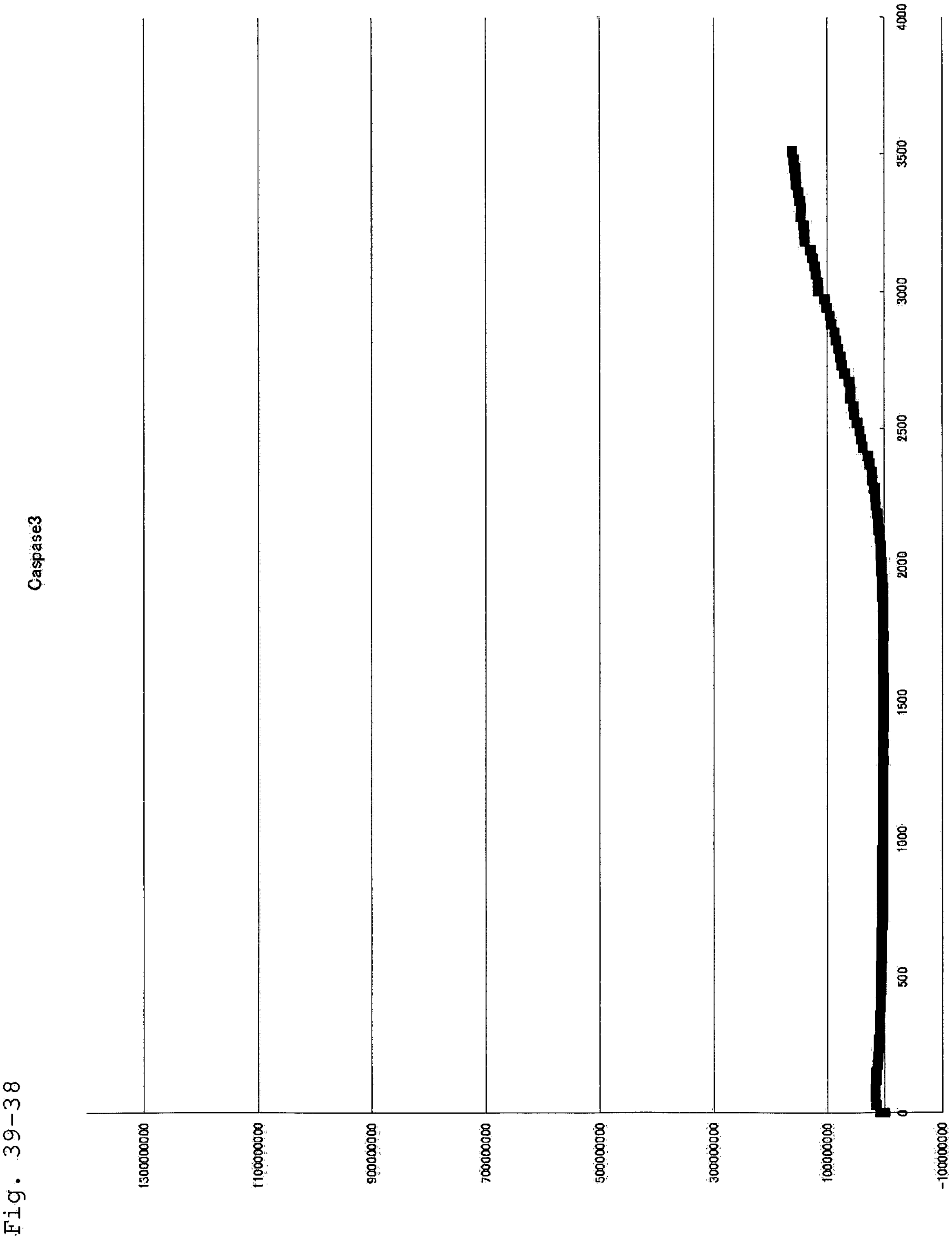

Caspase3
130000000
110000000
90000000
70000000
50000000
30000000
10000000
-10000000
0
500
1000
1500
2000
2500
3000
3500
4000

Fig. 39-39

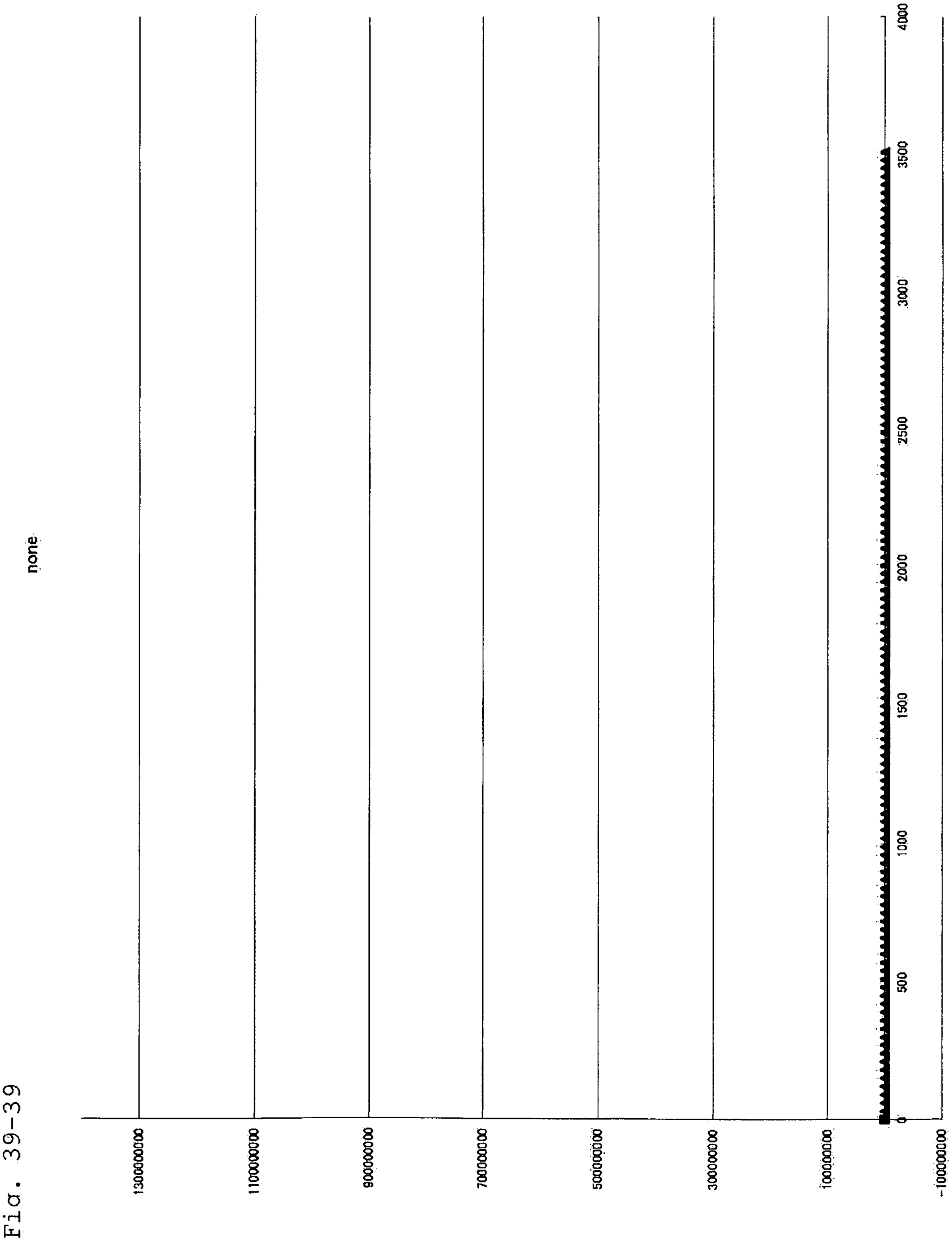
none
1300000000
1100000000
900000000
700000000
500000000
300000000
100000000
-100000000
0
500
1000
1500
2000
2500
3000
3500
4000

Fig. 39-40

STAT3

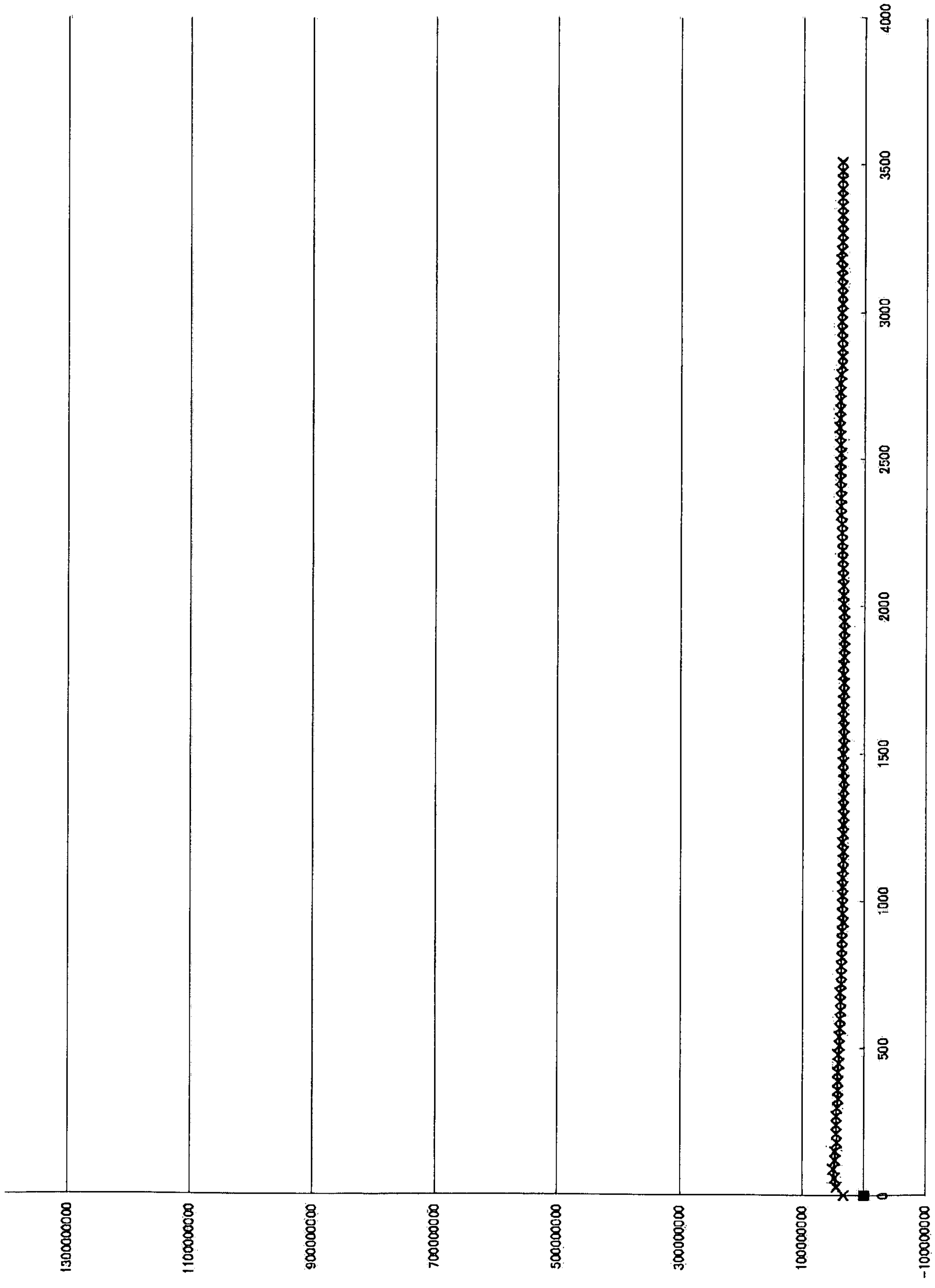

Fig. 39-41

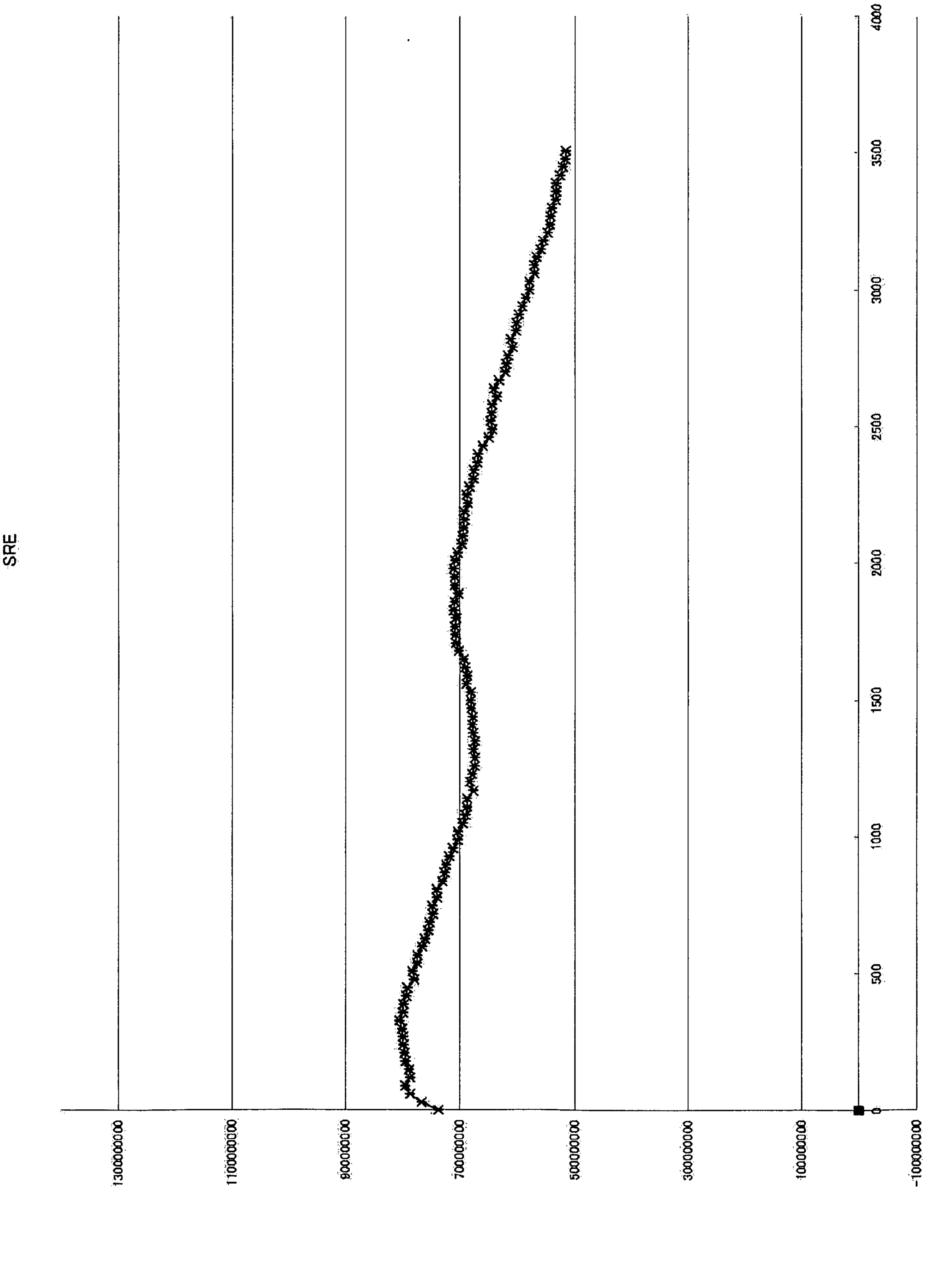
SRE
1300000000
1100000000
900000000
700000000
500000000
300000000
100000000
-100000000
0
500
1000
1500
2000
2500
3000
3500
4000

Fig. 39-42

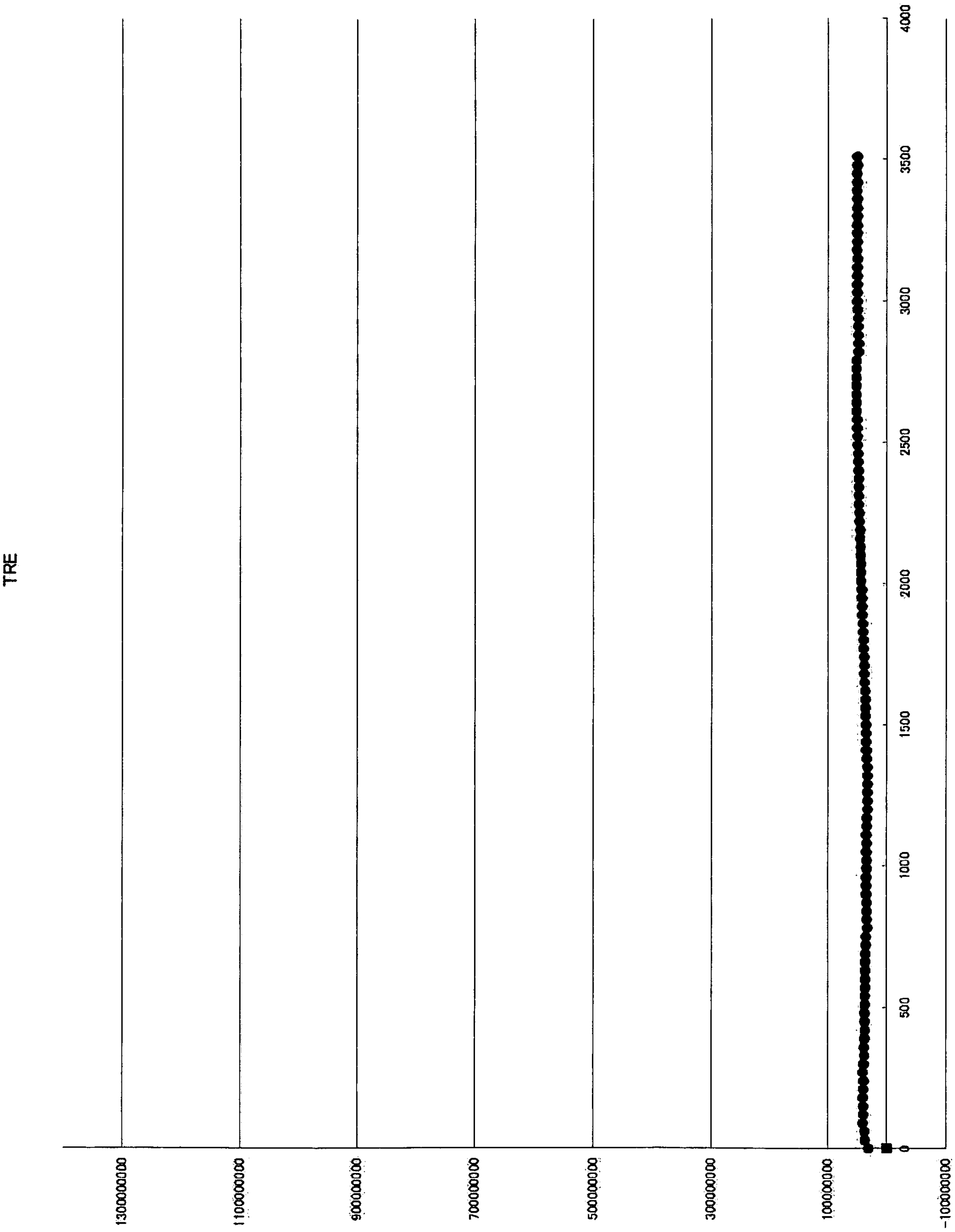
TRE
130000000
110000000
90000000
70000000
50000000
30000000
10000000
-10000000
0
500
1000
1500
2000
2500
3000
3500
4000

Fig. 39-43

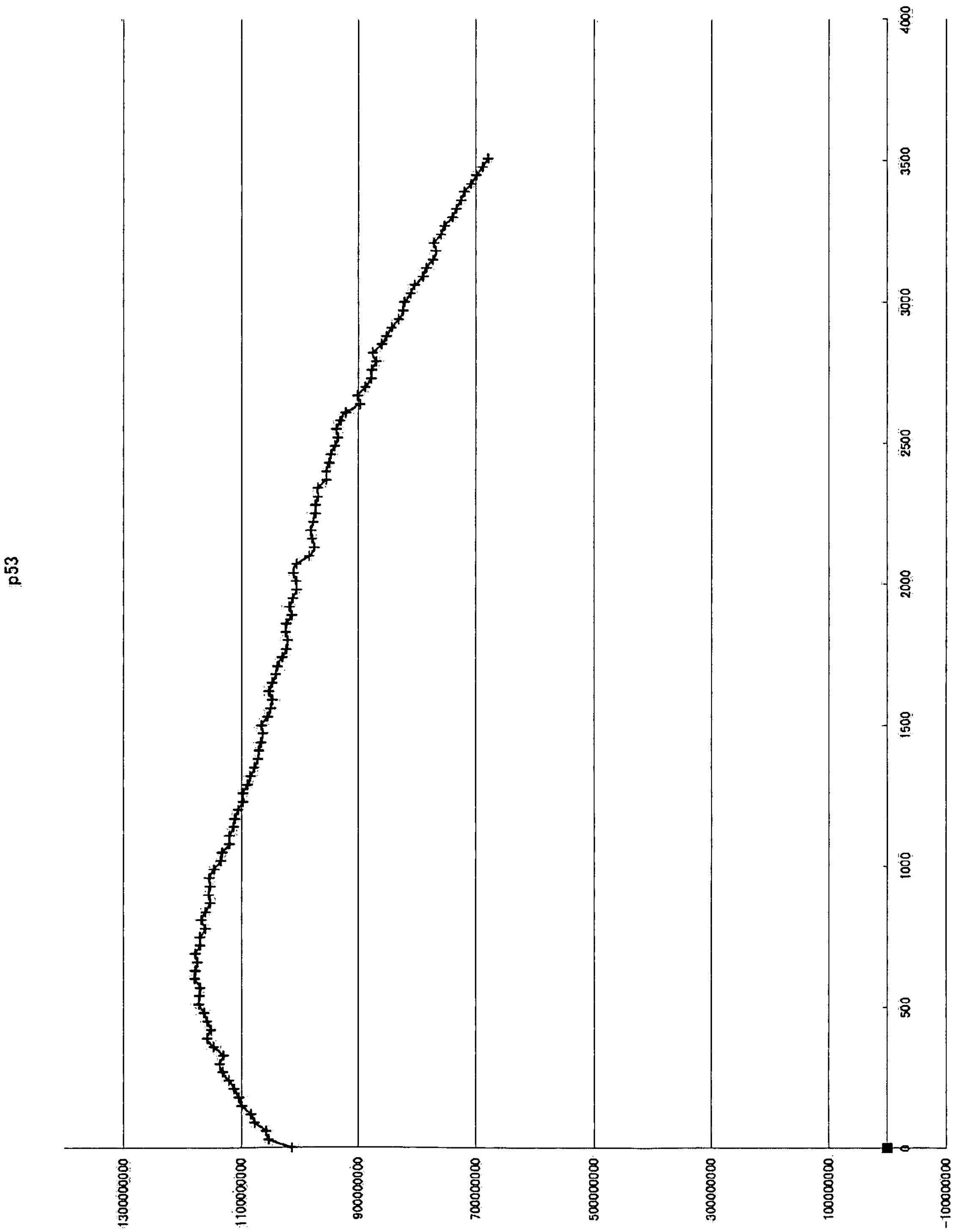
p53
1300000000
1100000000
900000000
700000000
500000000
300000000
100000000
-100000000
0
500
1000
1500
2000
2500
3000
3500
4000

Fig. 39-44

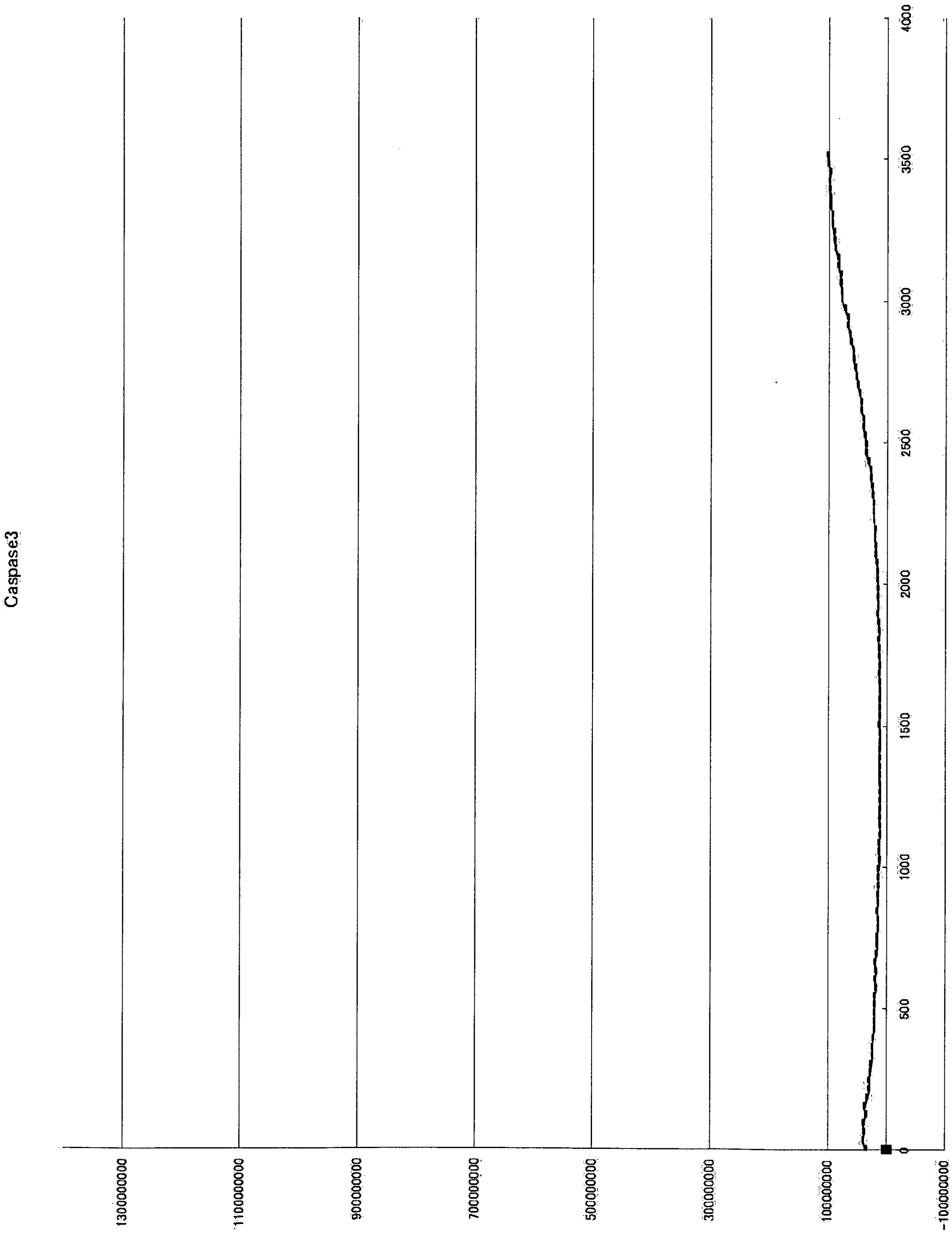
Caspase3
1300000000
1100000000
900000000
700000000
500000000
300000000
100000000
-100000000
0
500
1000
1500
2000
2500
3000
3500
4000

Fig. 39-45

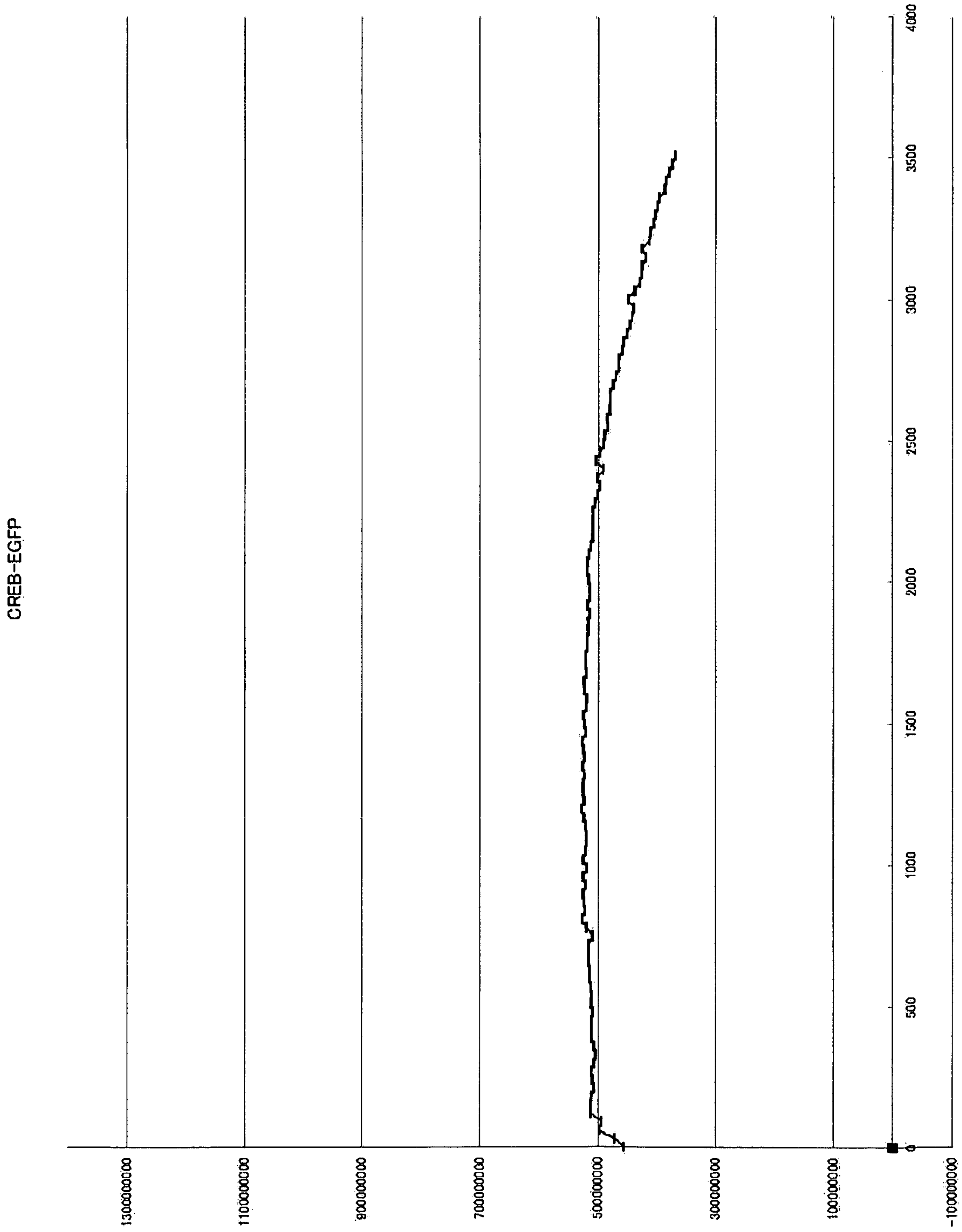
CREB-EGFP
1300000000
1100000000
900000000
700000000
500000000
300000000
100000000
-100000000
0
500
1000
1500
2000
2500
3000
3500
4000

Fig. 39-46

IkB-EGFP

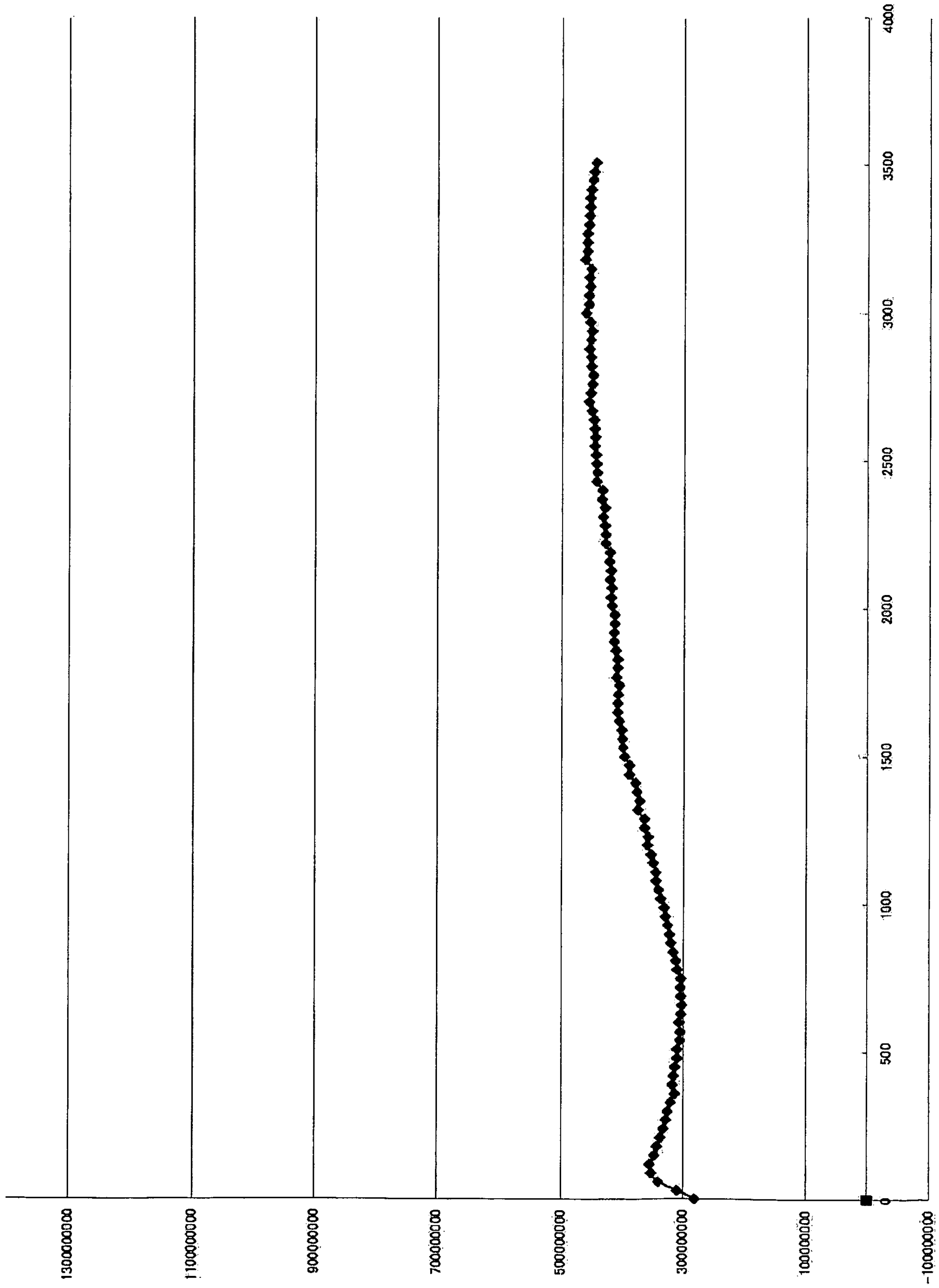

Fig. 39-47

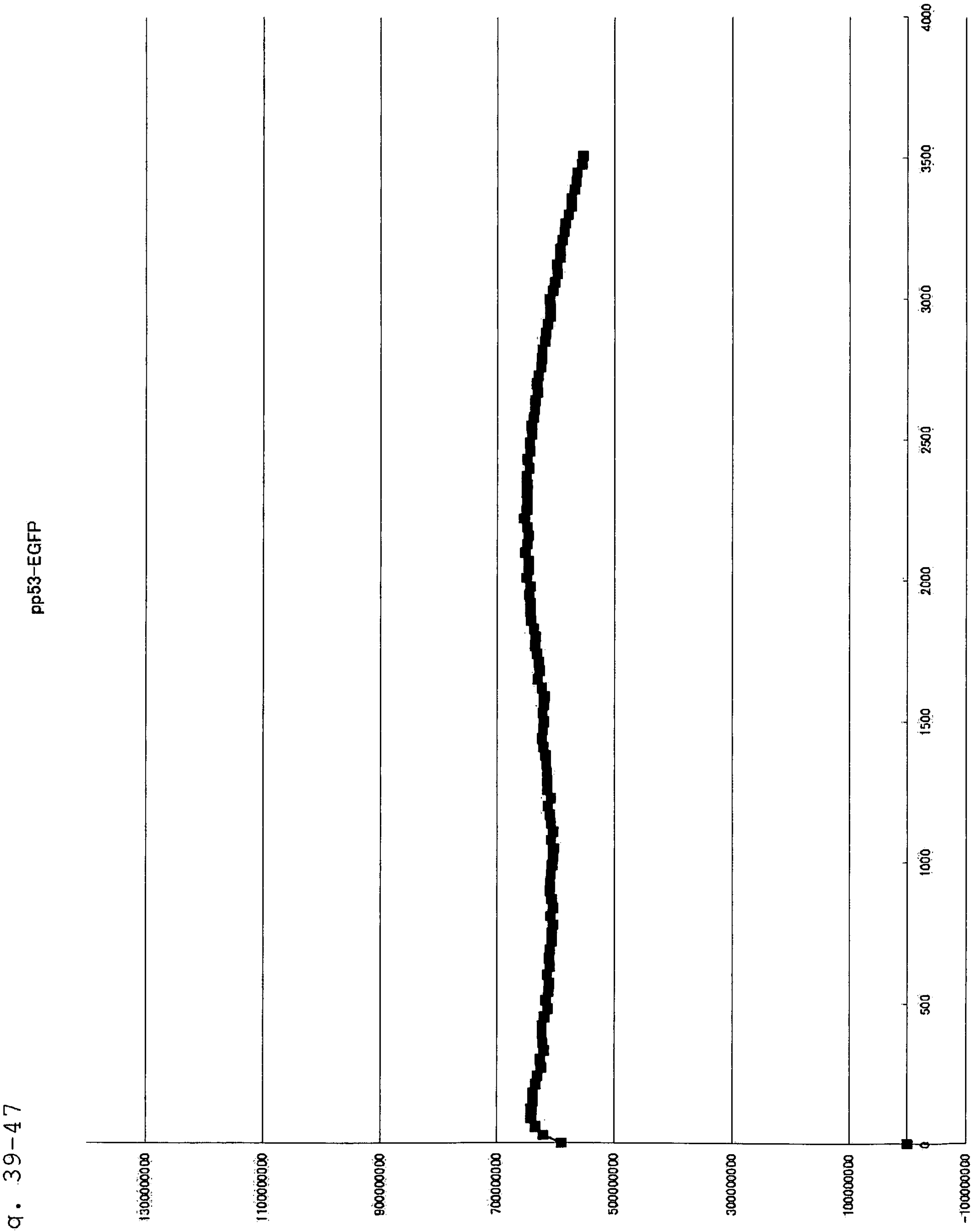
pp53-EGFP
1300000000
1100000000
900000000
700000000
500000000
300000000
100000000
-100000000
0
500
1000
1500
2000
2500
3000
3500
4000

Fig. 39-48

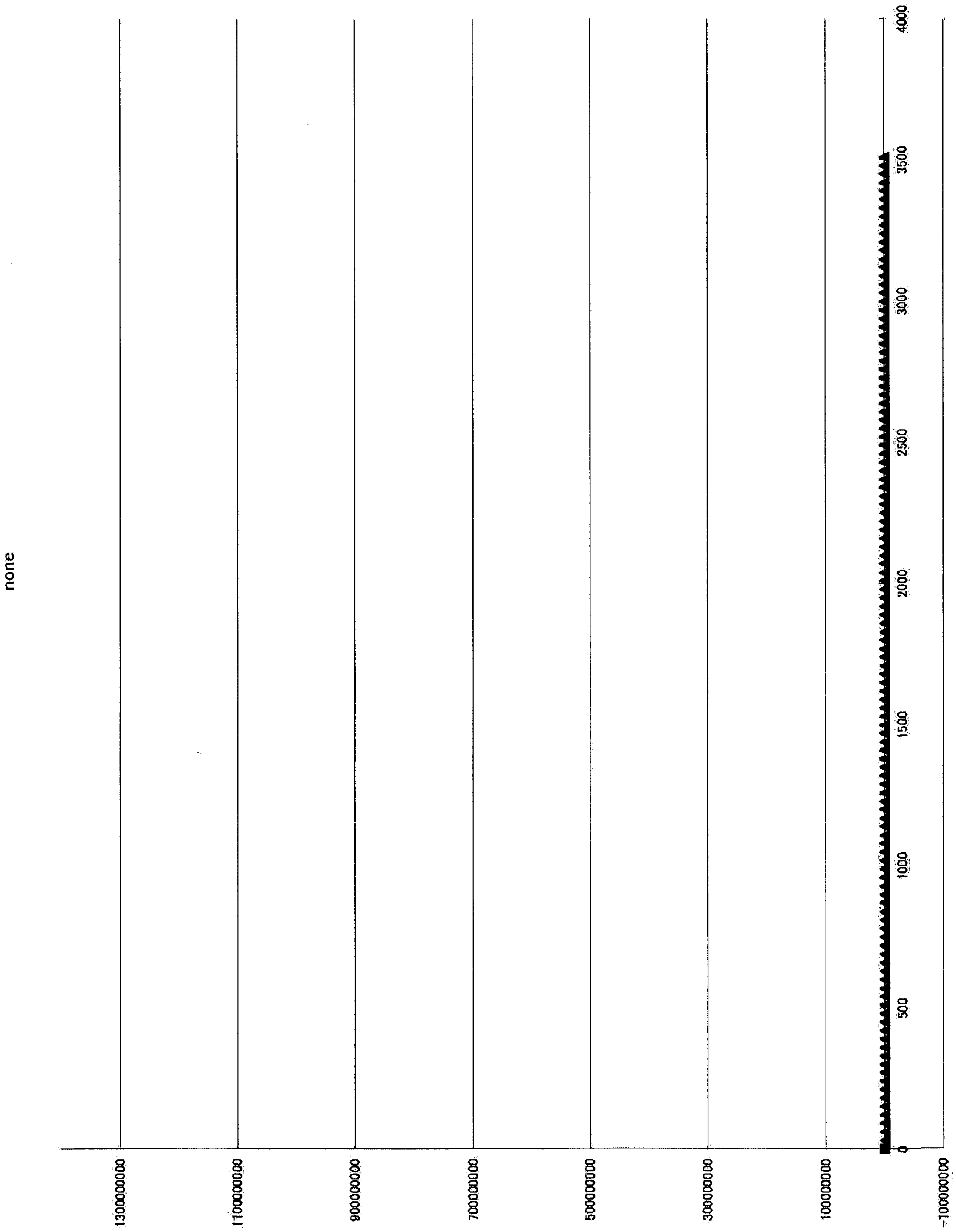
none
1300000000
1100000000
900000000
700000000
500000000
300000000
100000000
-100000000
0
500
1000
1500
2000
2500
3000
3500
4000

Fig. 39-49

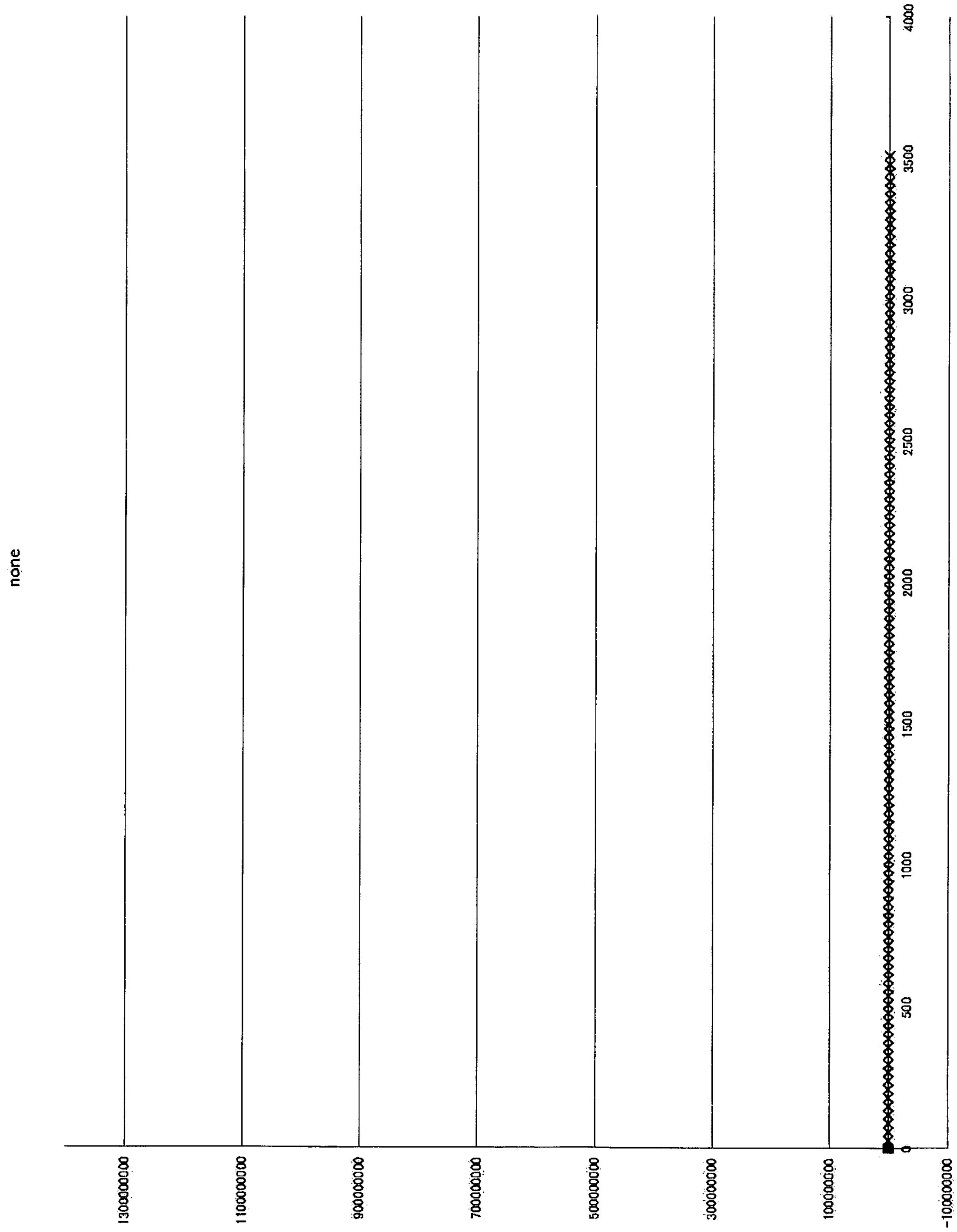
none
1300000000
1100000000
900000000
700000000
500000000
300000000
100000000
-100000000
0
500
1000
1500
2000
2500
3000
3500
4000

Fig. 39-50

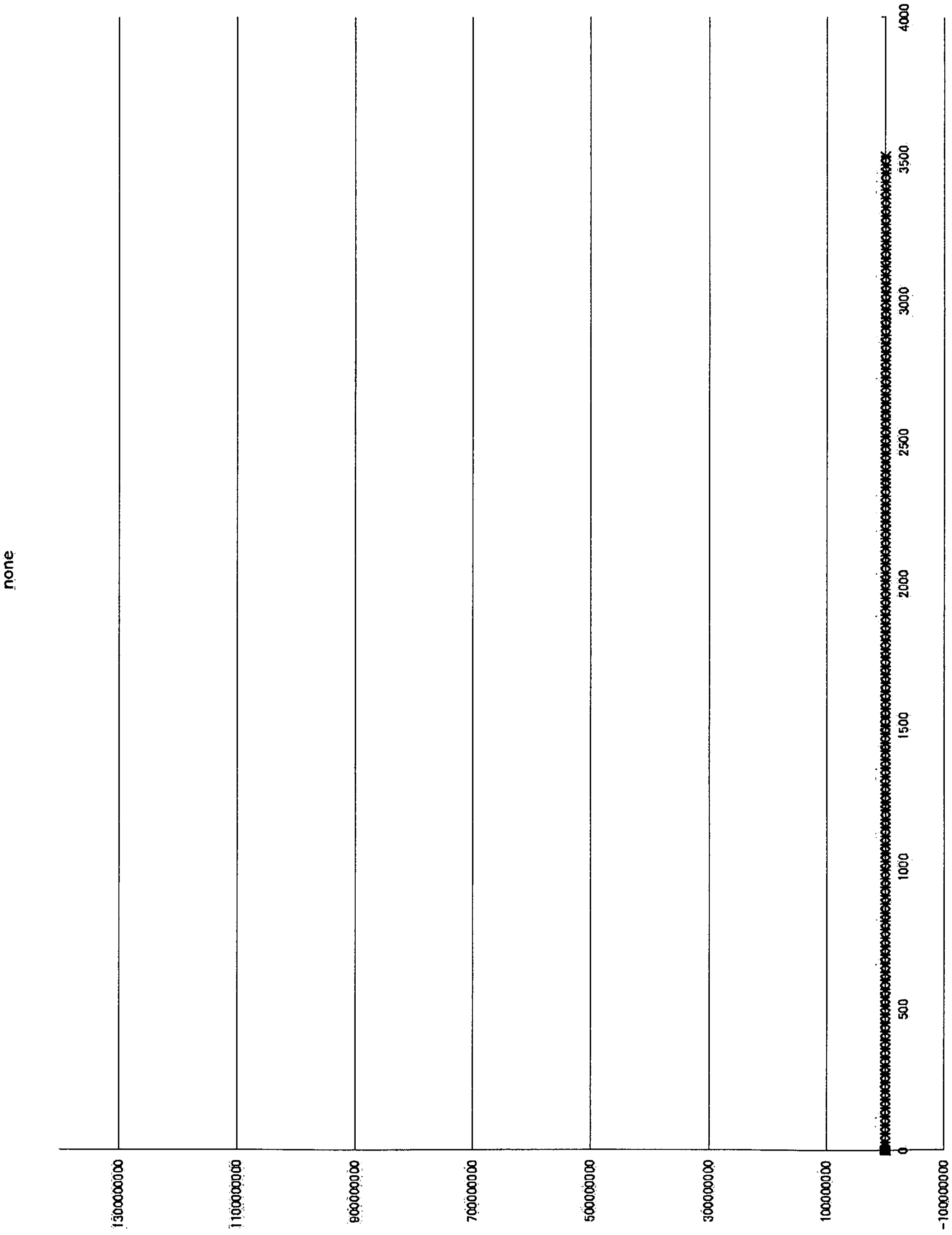
none
1300000000
1100000000
900000000
700000000
500000000
300000000
100000000
-100000000
0
500
1000
1500
2000
2500
3000
3500
4000

Fig. 39-51

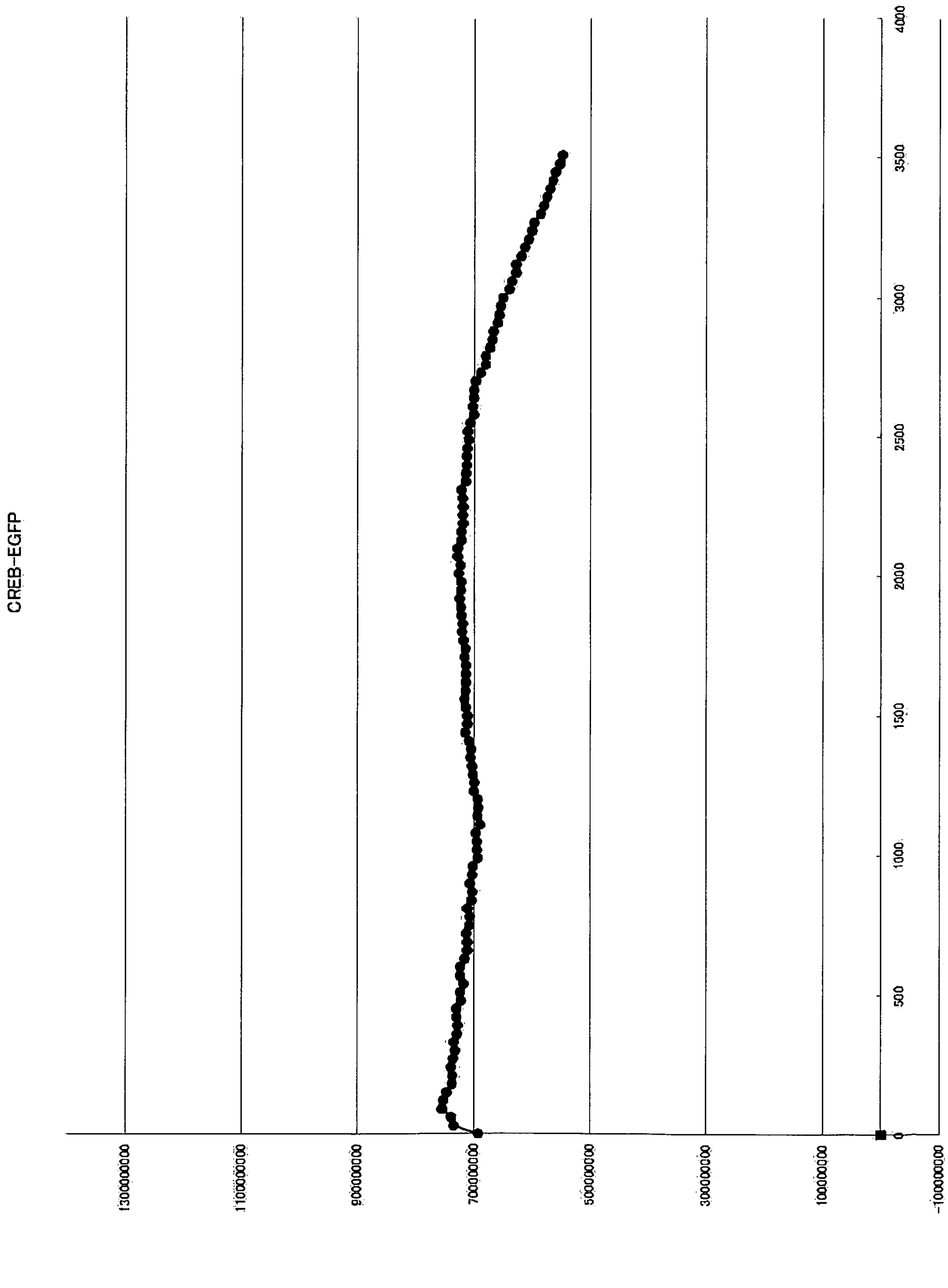
CREB-EGFP
130000000
110000000
90000000
70000000
50000000
30000000
10000000
-10000000
0
500
1000
1500
2000
2500
3000
3500
4000

Fig. 39-52

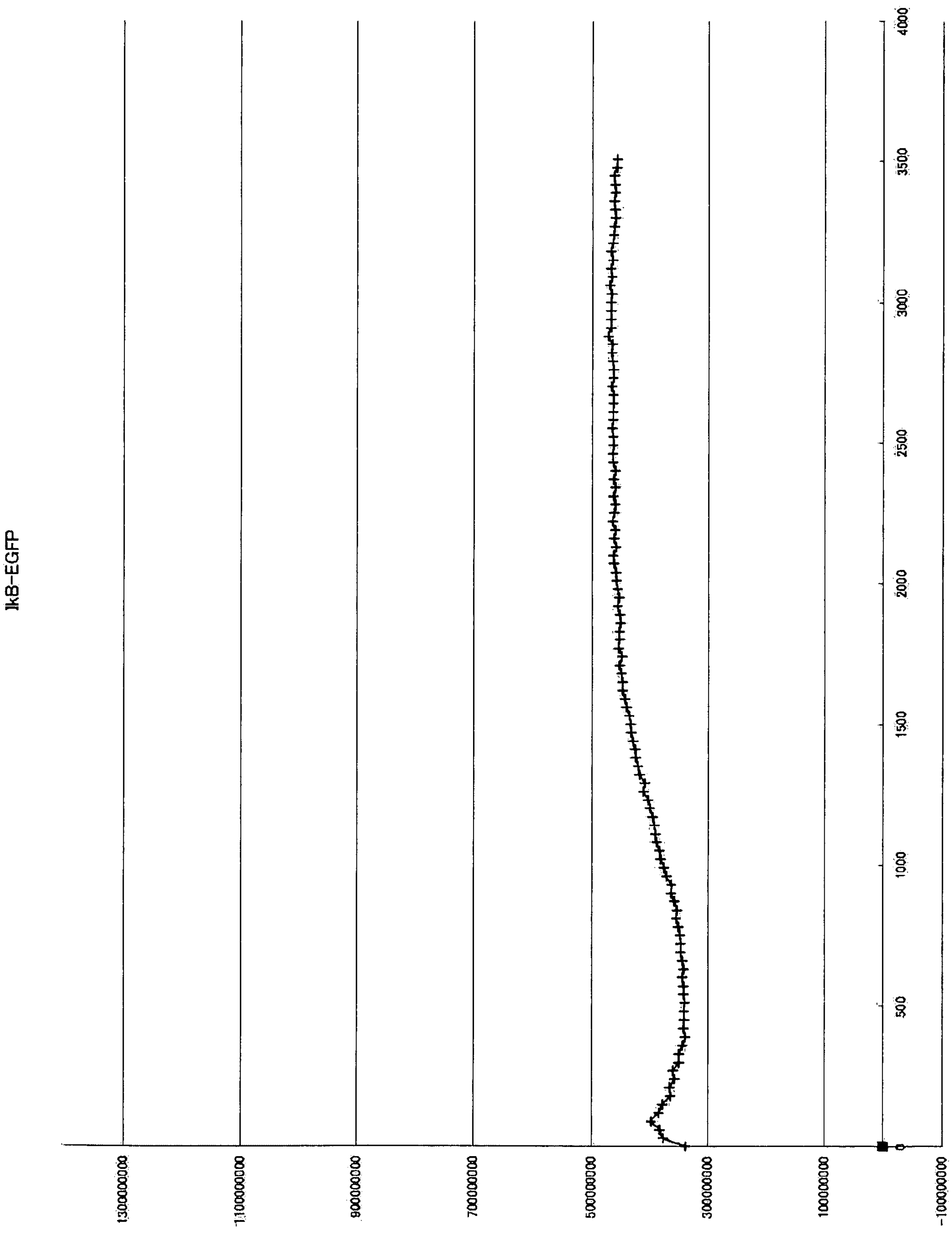
IkB-EGFP
1300000000
1100000000
900000000
700000000
500000000
300000000
100000000
-100000000
0
500
1000
1500
2000
2500
3000
3500
4000

Fig. 39-53

pp53-EGFP

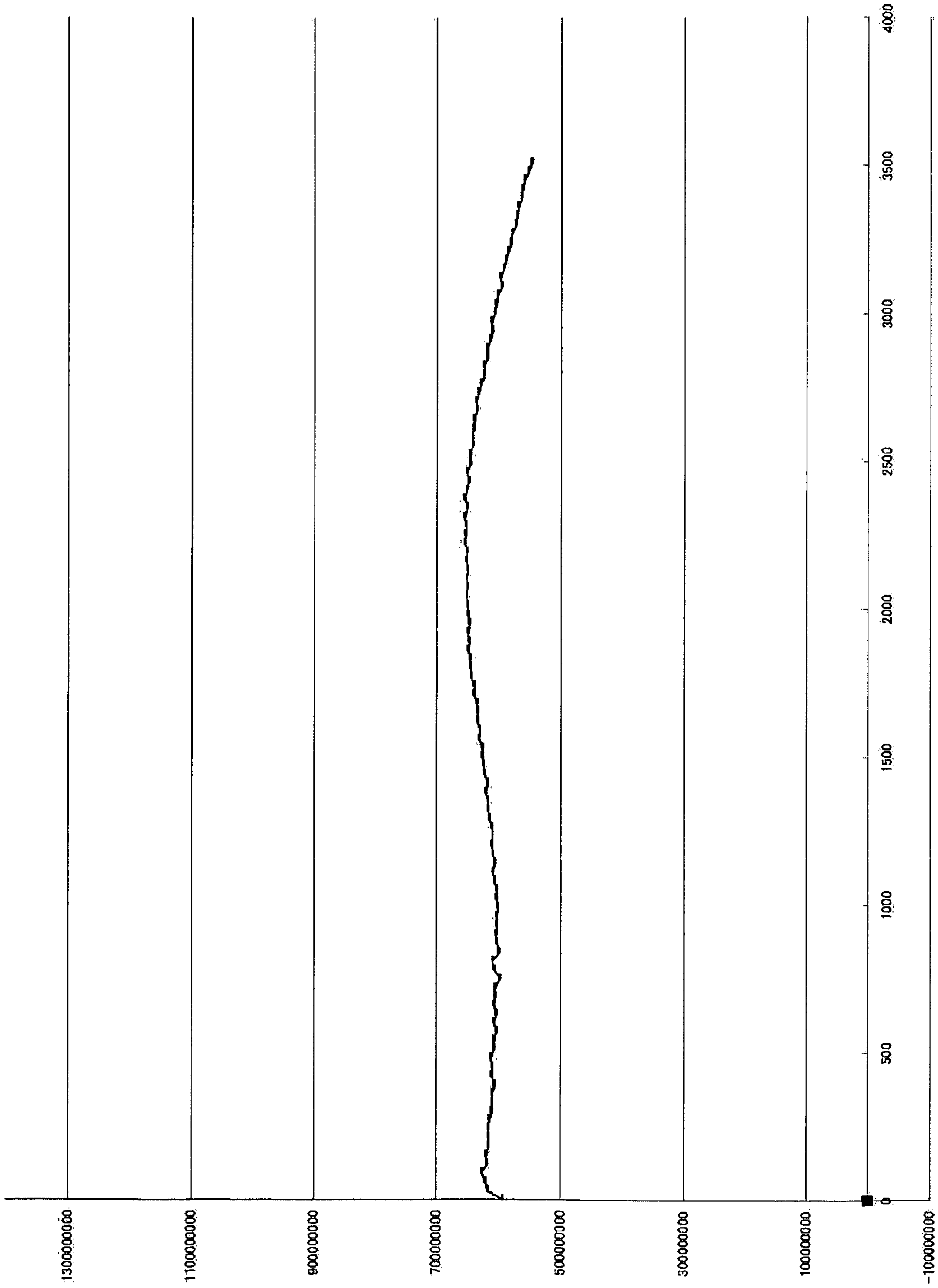

Fig. 39-54

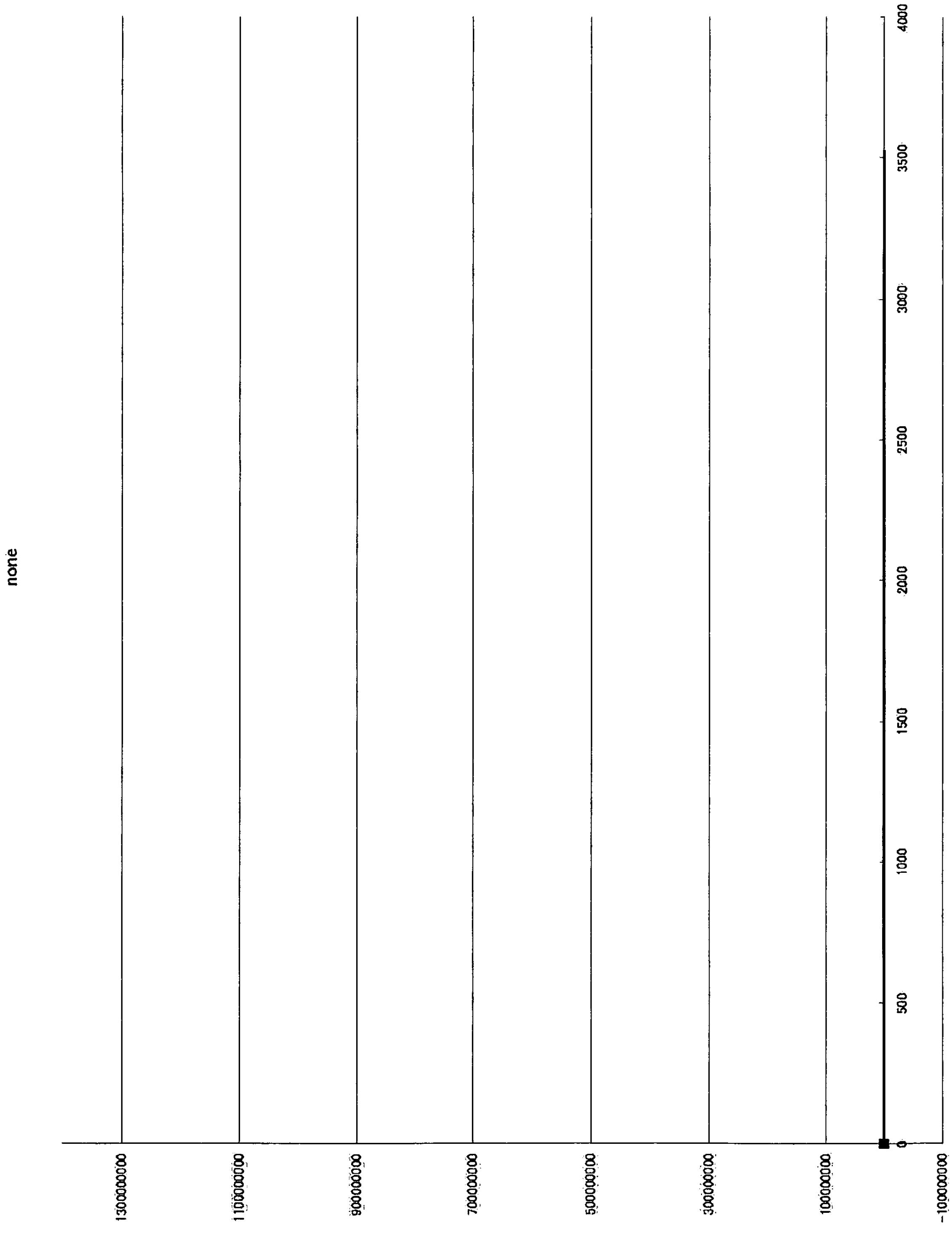
none
1300000000
1100000000
900000000
700000000
500000000
300000000
100000000
-100000000
0
500
1000
1500
2000
2500
3000
3500
4000

Fig. 39-55

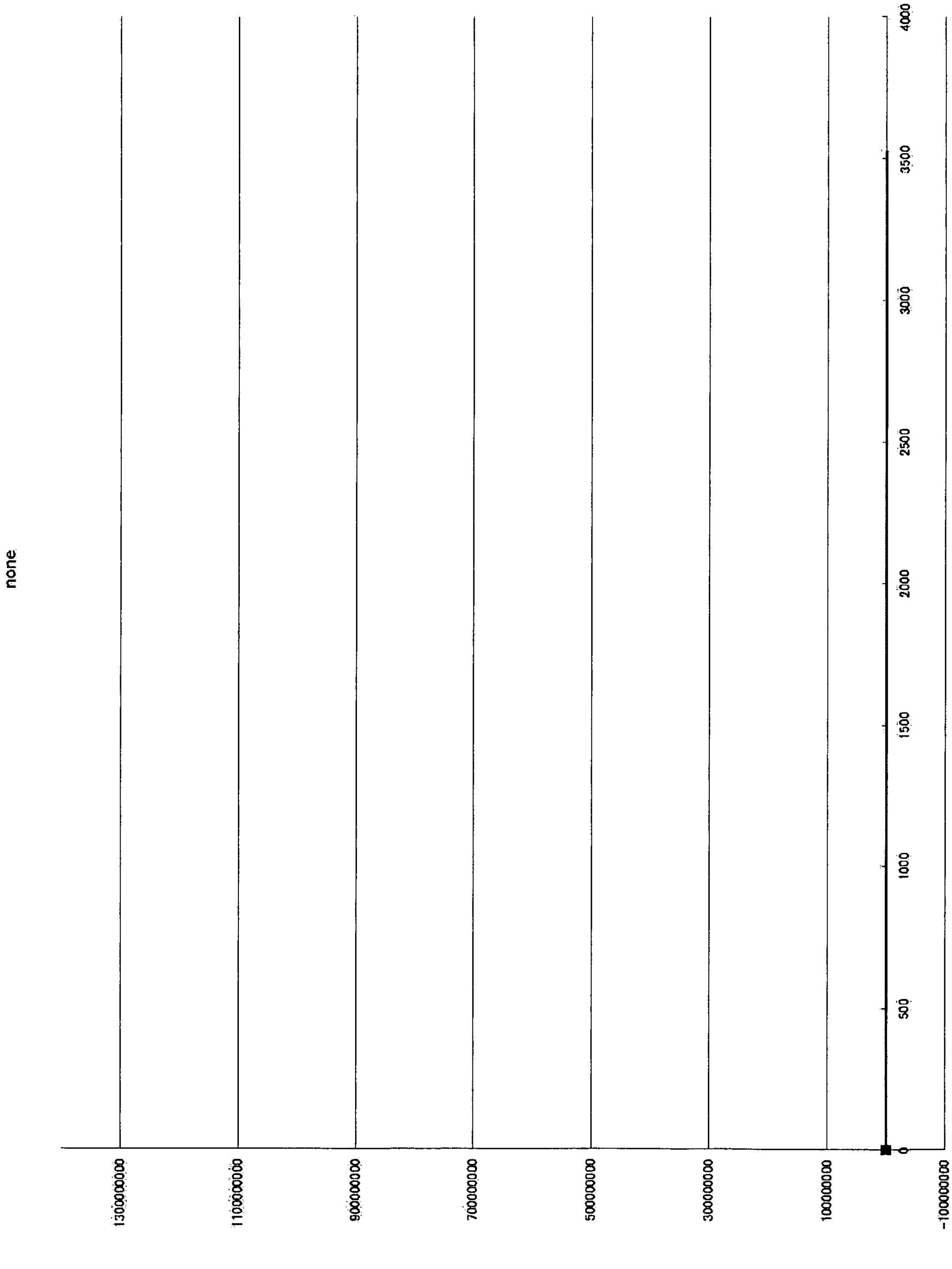

none
1300000000
1100000000
900000000
700000000
500000000
300000000
100000000
-100000000
0
500
1000
1500
2000
2500
3000
3500
4000

Fig. 40

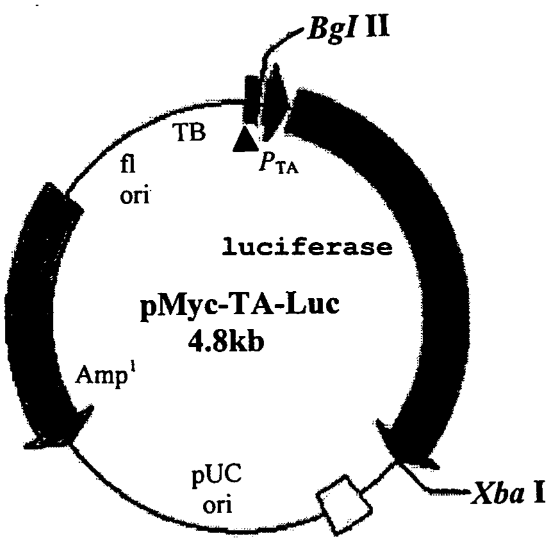


▲=cMyc *cis*-acting enhancer element
TB =Transcription Blocker

Fig. 41

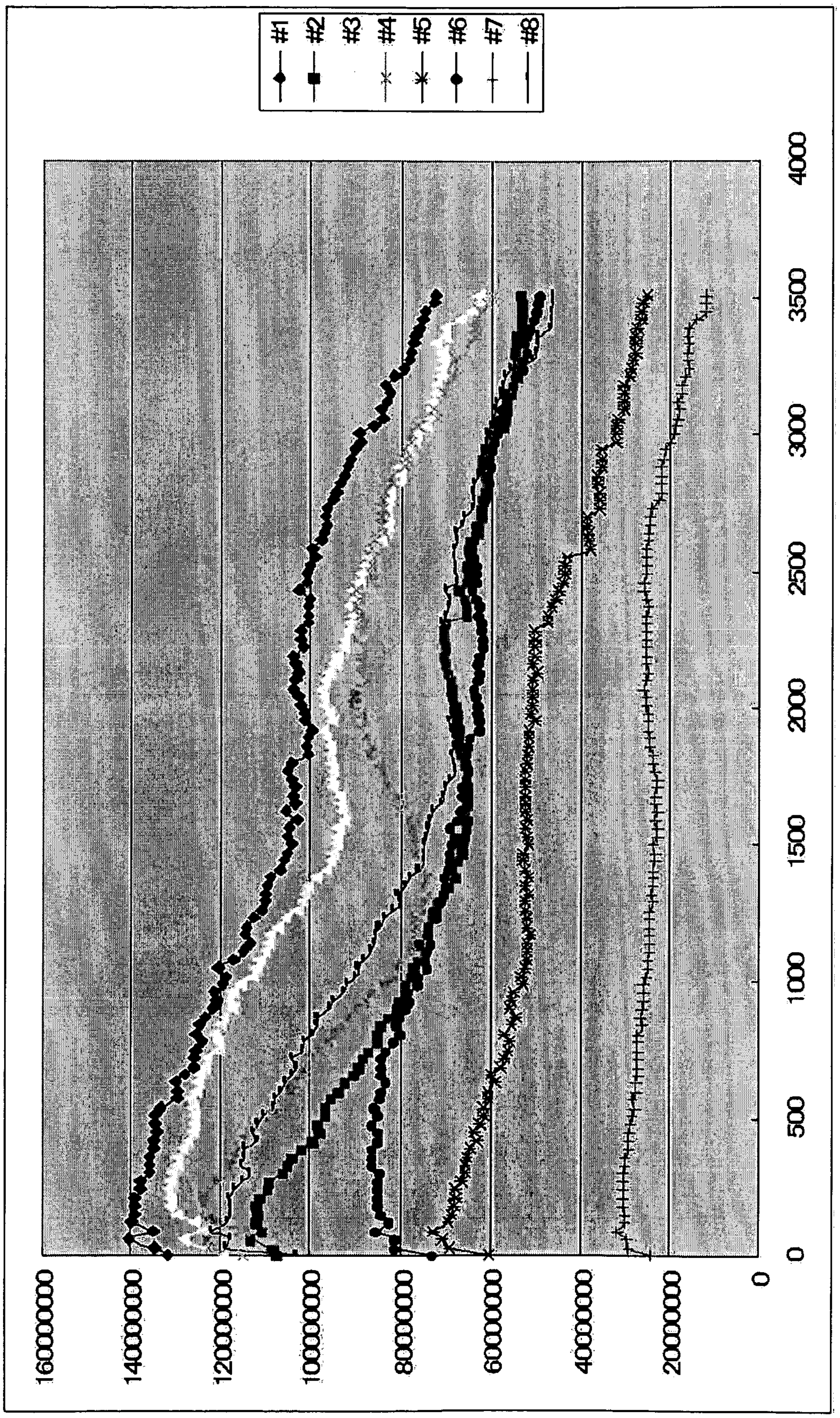

#1
#2
#3
#4
#5
#6
#7
#8
0
500
1000
1500
2000
2500
3000
3500
4000
0
20000000
40000000
60000000
80000000
100000000
120000000
140000000
160000000

Fig. 42

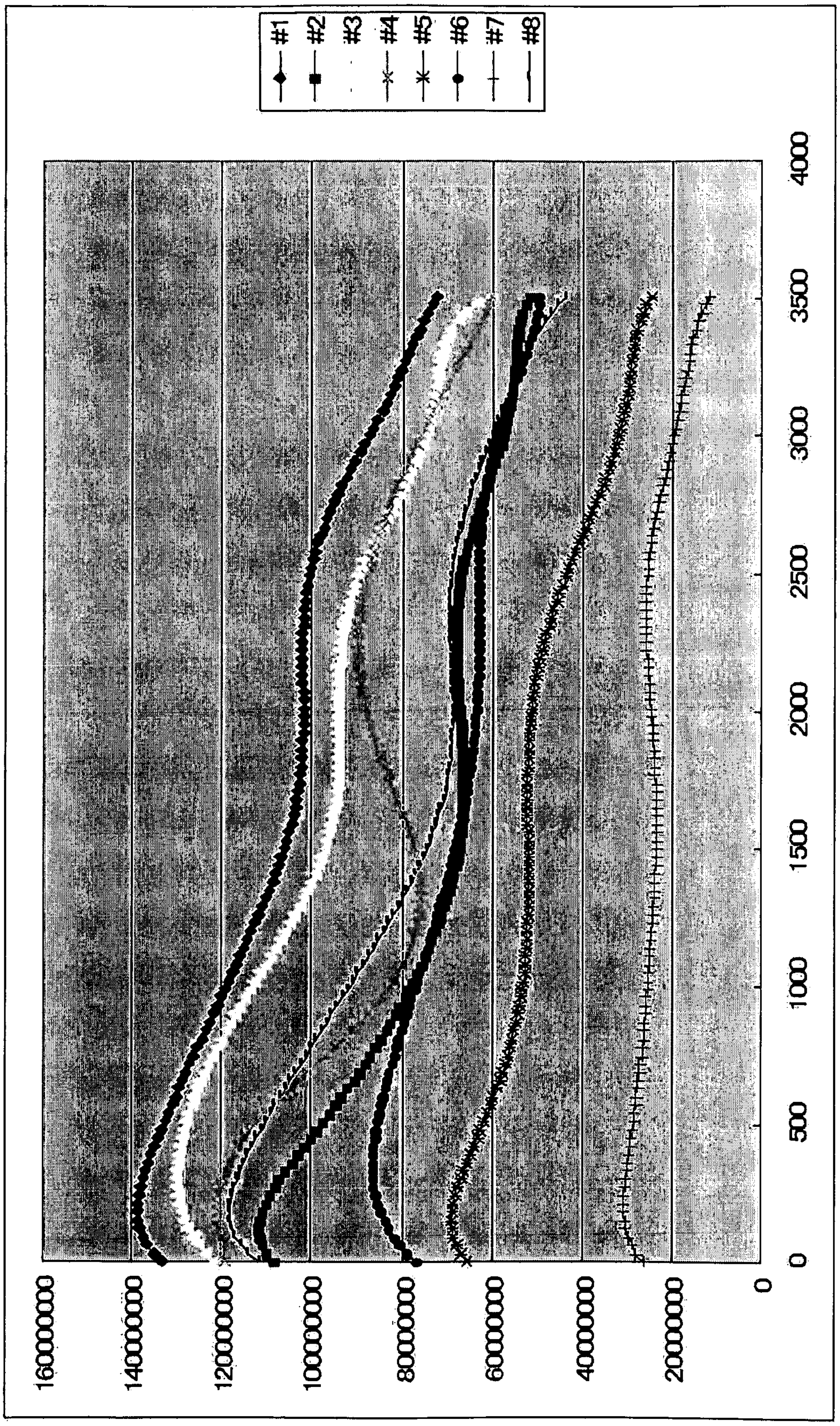

#1
#2
#3
#4
#5
#6
#7
#8
0
2000000
4000000
6000000
8000000
10000000
12000000
14000000
16000000
0
500
1000
1500
2000
2500
3000
3500
4000

Fig. 43

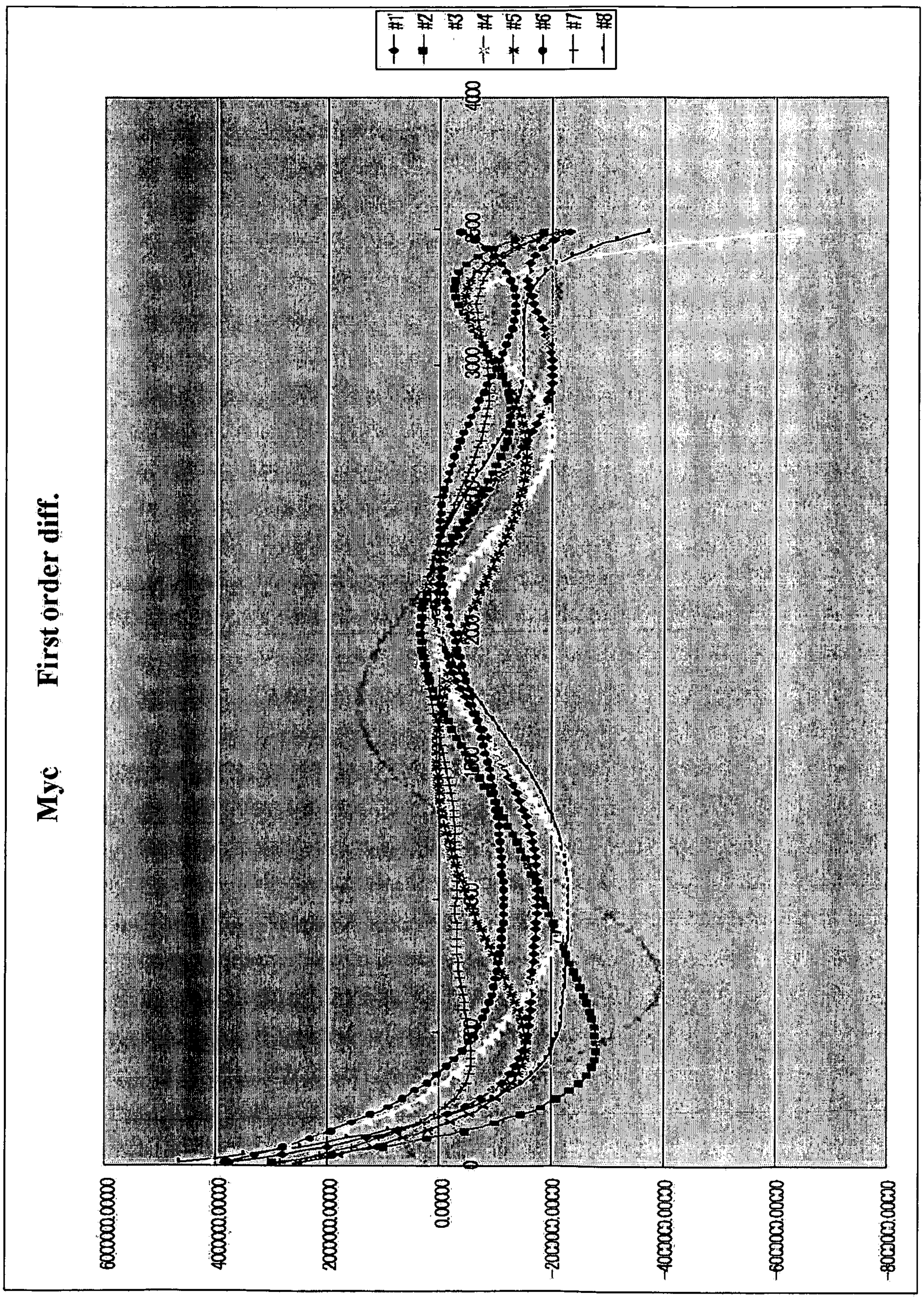

Myc
First order diff.
#1
#2
#3
#4
#5
#6
#7
#8
600000.0000
400000.0000
200000.0000
0.0000
-200000.0000
-400000.0000
-600000.0000
-800000.0000
0
3000
4000

Fig. 44

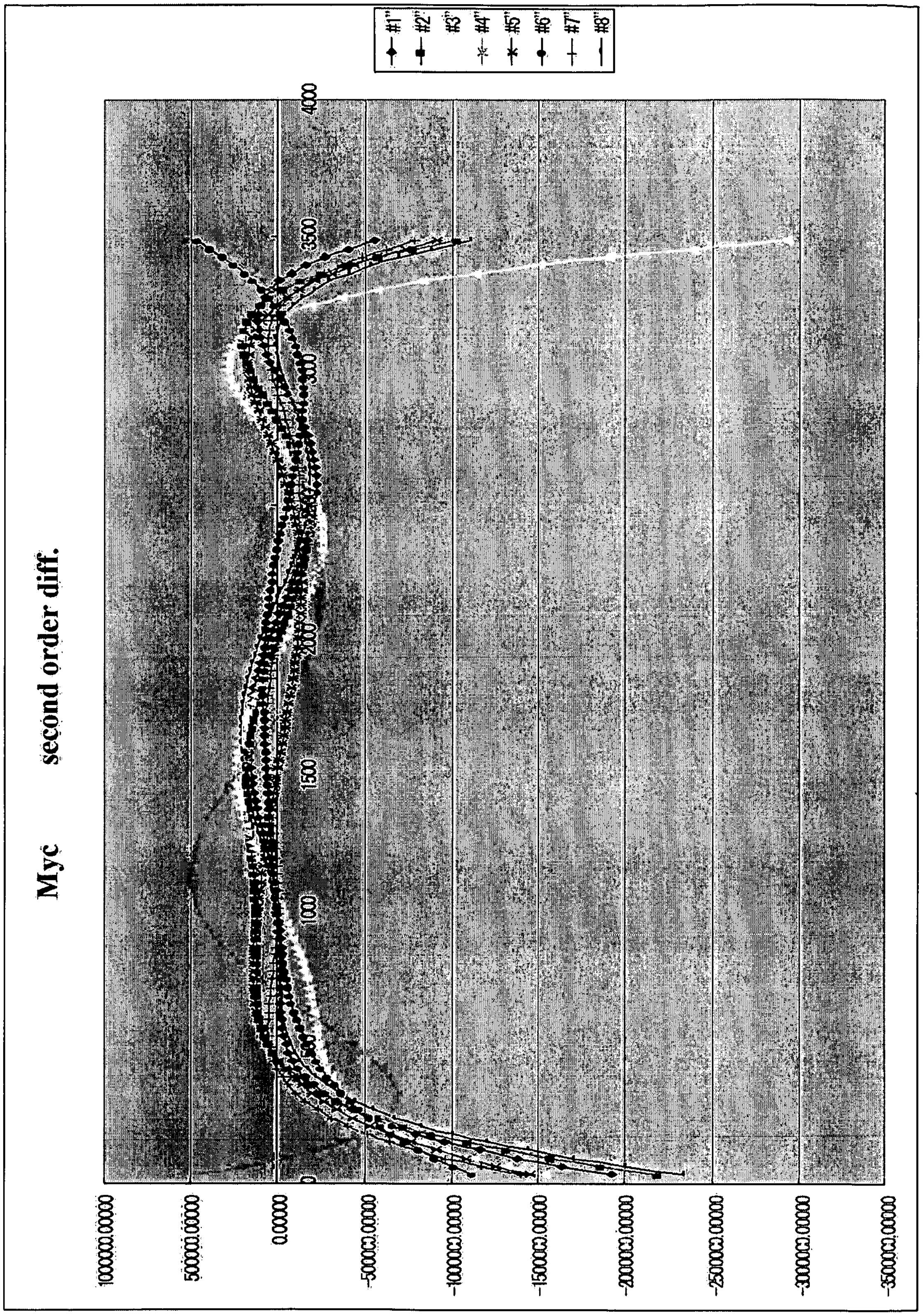
Myc second order diff.
#1''
#2''
#3''
#4''
#5''
#6''
#7''
#8''
1000000.00000
500000.00000
0.00000
-500000.00000
-1000000.00000
-1500000.00000
-2000000.00000
-2500000.00000
-3000000.00000
-3500000.00000
0
1000
1500
2000
3000
3500
4000

Fig. 45

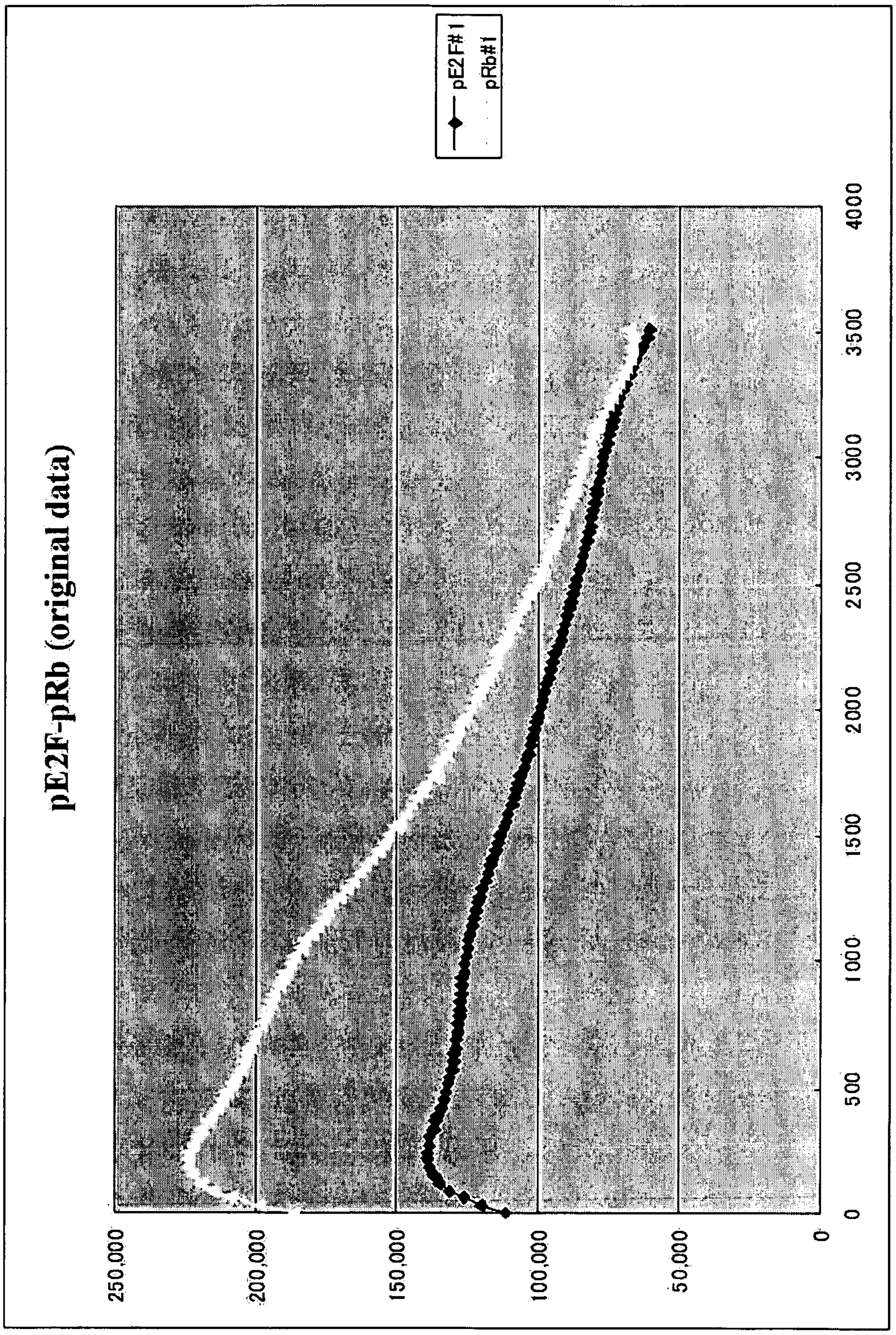
pE2F-pRb (original data)
pE2F#1
pRb#1
250,000
200,000
150,000
100,000
50,000
0
0
500
1000
1500
2000
2500
3000
3500
4000

Fig. 46

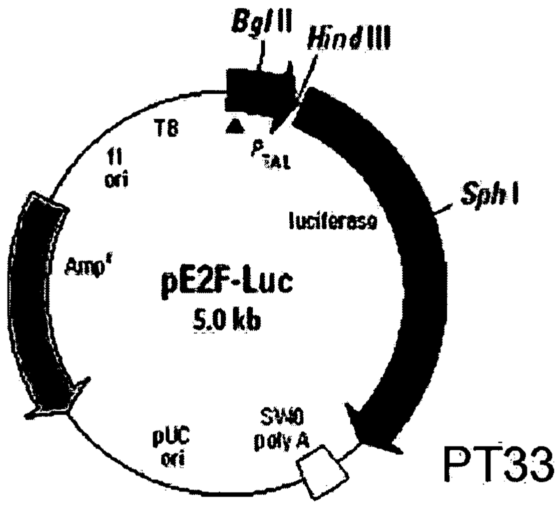


PT3373-5

▲ = E2F *cis*-acting enhancer element
TB = Transcription Blocker

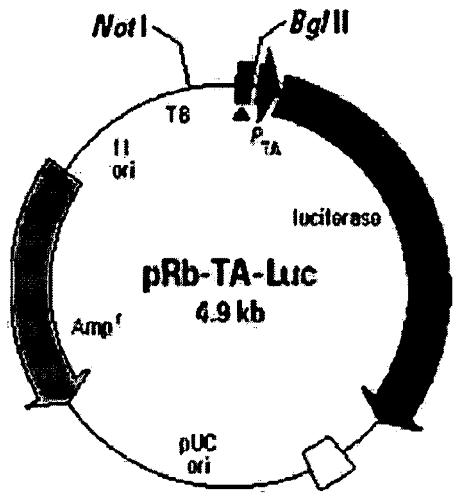


PT3512-5

▲ = Rb *cis*-acting enhancer element
TB = Transcription Blocker

Fig. 47

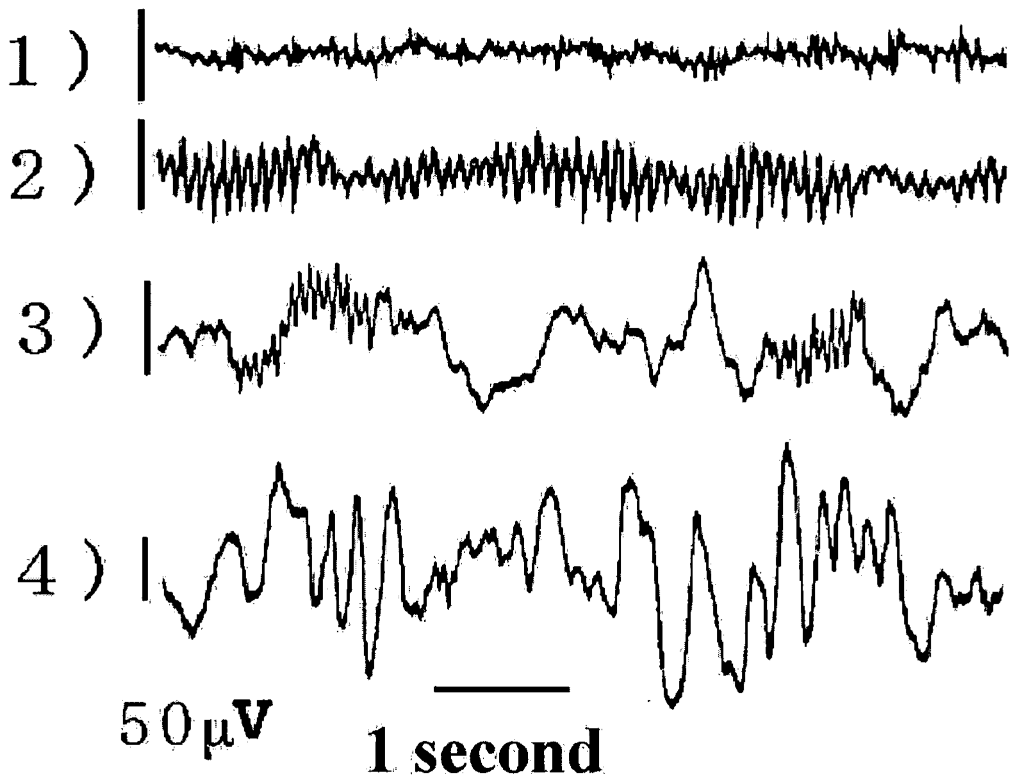

Fig. 48

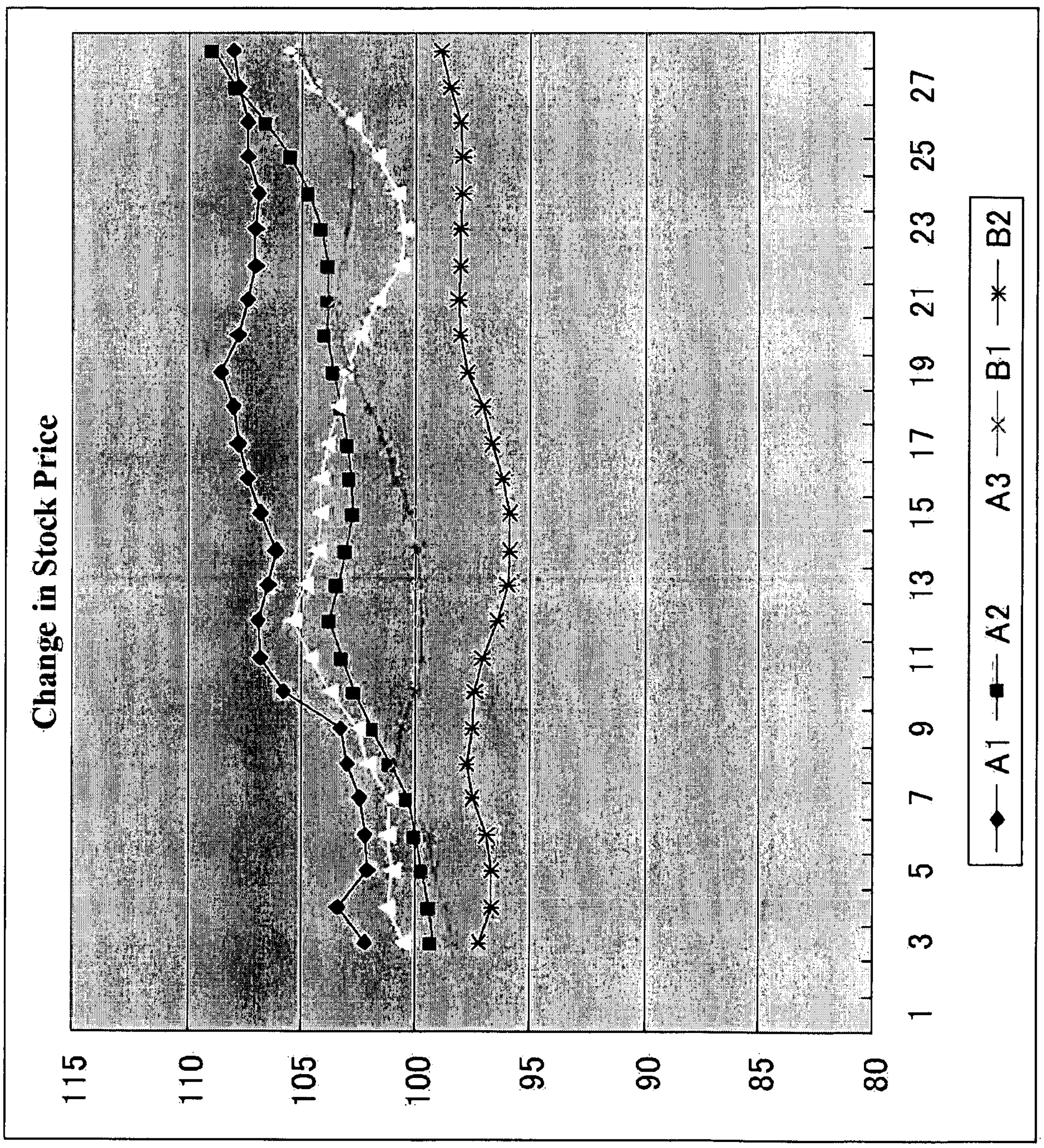
Change in Stock Price
115
110
105
100
95
90
85
80
1
3
5
7
9
11
13
15
17
19
21
23
25
27
A1
A2
A3
B1
B2

**INTERNATIONAL SEARCH REPORT**

International application No.
PCT/JP2005/001151

A. CLASSIFICATION OF SUBJECT MATTER
Int.Cl$^7$ G06F19/00

According to International Patent Classification (IPC) or to both national classification and IPC

B. FIELDS SEARCHED

Minimum documentation searched (classification system followed by classification symbols)
Int.Cl$^7$ G06F19/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2005 |
| Kokai Jitsuyo Shinan Koho | 1971-2005 | Jitsuyo Shinan Toroku Koho | 1996-2005 |

Electronic data base consulted during the international search (name of data base and, where practicable, search terms used)
JICST FILE(JOIS), WPI, INSPEC(DIALOG)

C. DOCUMENTS CONSIDERED TO BE RELEVANT

| Category* | Citation of document, with indication, where appropriate, of the relevant passages | Relevant to claim No. |
|---|---|---|
| X | Kengo KOARA, "Ugoki no Henka ni Chakumoku shita Pattern no 1-Jigen Fugoka ni Motozuku Miburi Ninshiki", Transactions of Information Processing Society of Japan, Information Processing Society of Japan, 15 February, 2003 (15.02.03), Vol.44, No.2, pages 466 to 477 | 1-86 |
| A | JP 10-3464 A (Fuji Facom Corp.), 06 January, 1998 (06.01.98), Full text; Figs. 1 to 17 (Family: none) | 1-86 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 March, 2005 (30.03.05) | Date of mailing of the international search report<br>12 April, 2005 (12.04.05) |
| Name and mailing address of the ISA/<br>Japanese Patent Office<br><br>Facsimile No. | Authorized officer<br><br>Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 01006013 A
- WO 01005935 A
- WO 003933 A
- WO 0039338 A
- WO 0039337 A
- WO 0024936 A
- WO 0017402 A
- WO 9966067 A
- WO 9966024 A
- WO 9958720 A
- WO 9959037 A
- US 5777888 A
- JP 2003505038 W
- JP 2003505022 W
- JP 2002533701 W
- JP 2002533700 W
- JP 2002533699 W
- JP 2002528095 W
- JP 2002526757 W
- JP 2002518021 W
- JP 2002518003 W
- JP 2002514804 W
- JP 2002514773 W
- JP 2002514437 W
- US 5569588 A
- US 4708871 A
- US 4554101 A
- JP 3000229 A
- US 5985660 A

### Non-patent literature cited in the description


- **SCHENA et al.** Quantitative monitoring of gene expression patterns with a complementary DNA microarray. *Science,* 1995, vol. 270, 467-470
- **LOCKHART et al.** Expression monitoring by hybridization to high-density oligonucleotide arrays. *Nature Biotechnology,* 1996, vol. 14, 1675-1680
- **BLANCHARD et al.** Sequence to array: Probing the genome's secrets. *Nature Biotechnology,* 1996, vol. 14, 1649
- **MARTON et al.** Drug target validation and identification of second-order drug target effects using Microarrays. *Nat. Med.,* November 1998, vol. 4 (11), 1293-301
- **GRAY et al.** Exploiting chemical libraries, structure, and genomics in the search for kinase inhibitors. *Science,* 1998, vol. 281, 533-538
- **SCHENA et al.** Quantitative monitoring of gene expression patterns with a complementary DNA micro-array. *Science,* 1995, vol. 270, 467-470
- **MARTON et al.** Drug target validation and identification of second-order drug target effects using Microarrays. *Nat Med.,* November 1998, vol. 4 (11), 1293-301
- **GRAY et al.** Exploiting chemical libralies, structure, and genomics in the search for kinase inhibitors. *Science,* 1998, vol. 281, 533-538
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- Current Protocols in Molecular Biology
- DNA Cloning 1: Core Techniques, A Practical Approach. Oxford University Press, 1995
- **ALTSCHUL et al.** *J. Mol. Biol.,* 1990, vol. 215, 403-410
- **PEARSON ; LIPMAN.** *Proc. Natl. Acad. Sci.,* 1988, vol. 85, 2444-2448
- **SMITH ; WATERMAN.** *J. Mol. Biol.,* 1981, vol. 147, 195-197
- **NEEDLEMAN ; WUNSCH.** *J. Mol. Biol.,* 1970, vol. 48, 443-453
- **GEYSEN et al.** *Proc. Natl. Acad. Sci. USA,* 1984, vol. 81, 3998
- **GEYSEN et al.** *Molecular immunology,* 1986, vol. 23, 709
- **KOHLER ; MILSTEIN.** *Nature,* 1975, vol. 256, 495
- **BUCK et al.** *In Vitro,* 1982, vol. 18, 377
- **KYTE, J. ; DOOLITTLE, R.F.** *J. Mol. Biol.,* 1982, vol. 157 (1), 105-132
- **ERMOLAEVA O. et al.** *Nat. Genet.,* 1998, vol. 20, 19-23
- **CAMPBELL, S.A.** The Science and Engineering of Microelectronic Fabrication. Oxford University Press, 1996

- **ZAUT, P.V.** Micromicroarray Fabrication: a Practical Guide to Semiconductor Processing. 1996
- **MADOU, M.J.** Fundamentals of Microfabrication. CRC1 5 Press, 1997
- **RAI-CHOUDHURY, P.** Handbook of Microlithography, Micromachining, & Microfabrication: Microlithography. 1997
- **SLUDER ; WOLF ; SALMON.** A High-Resolution Multimode Digital Microscope System. *METHODS IN CELL BIOLOGY,* 1998, vol. 56, 185-215
- **INOUE ; SPRING.** *Video Microscopy,* 1997
- Current Protocols in Molecular Biology. Wiley, 1988
- **SAMBROOK J. et al.** Molecular Cloning: A Laboratory Manual. Cold Spring Harbor Laboratory Press, 1987
- Experimental Methods for Gene introduction & Expression Analysis. Yodo-sha, 1997
- *Biochem.,* 1987, vol. 101, 1307
- *J. Biol. Chem.,* 1993, vol. 268, 22782-22787
- *Proc. Natl. Acad. Sci. USA,* 1978, vol. 84, 1929
- *J. Bacteriol.,* 1983, vol. 153, 163
- *Proc. Natl. Acad. Sci. USA,* 1978, vol. 75, 1929
- DNA Microarray and Up-to-date PCR Method
- *Nat Genet.,* December 2002, vol. 32, 526-32
- Genome Analysis Experimental Method, Yusuke Nakamura's Lab-Manual. Yodo-sha, 2002
- **MORRISON et al.** *Cell,* 1999, vol. 96, 737-49
- Kogan zai Manuaru. Lippincott Williams & Wilkins, Inc,
- Kogan zai Manuaru [Manual of Anticancer agents. Lippincott Williams & Wilkins, Inc,
- **SHO KEI ZEN.** Hoshasenkensa to Chiryo no Kiso: Hoshasen Chiryo to Shugakuteki Chiryo [Basics of Radiation Examination and Therapies: Radiation Therapy and Incentive Therapy. *Shiga Medical School, Radiation): Total digestive system care,* February 2002, vol. 6 (6), 79-89
- Dobutsu Baiyo Saibo Manuaru [Animal Culture Cell Manual. Kyoritsu Shuppan, 1993
- **GOLDSPIEL et al.** *Clinical Pharmacy,* 1993, vol. 12, 488-505
- **WU AND WU.** *Biotherapy,* 1991, vol. 3, 87-95
- **TOLSTOSHEV.** *Ann. Rev. Pharmacol. Toxicol.,* 1993, vol. 32, 573-596
- **MULLIGAN.** *Science,* 1993, vol. 260, 926-932
- **ANDERSON.** *Ann. Rev. Biochem.,* 1993, vol. 62, 191-217
- **MAY.** *TIBTECH,* 1993, vol. 11 (5), 155-215
- Current Protocols in Molecular Biology. John Wiley & Sons, 1993
- **KRIEGLER.** Gene Transfer and Expression, A Laboratory Manual. Stockton Press, 1990
- **SAMBROOK J. et al.** Molecular Cloning: A Laboratory Manual. Cold Spring Harbor, 1989
- **AUSUBEL, F.M.** Current Protocols in Molecular Biology. Greene Pub. Associates and Wiley-Interscience, 1987
- **AUSUBEL, F.M.** Short Protocols in Molecular Biology: A Compendium of Methods from Current Protocols in Molecular Biology. Greene Pub. Associates and Wiley-Interscience, 1989
- **INNIS, M.A.** PCR Protocols: A Guide to Methods and Applications. Academic Press, 1990
- **AUSUBEL, F.M.** Short Protocols in Molecular Biology: A Compendium of Methods from Current Protocols in Molecular Biology. Greene Pub. Associates, 1992
- **AUSUBEL, F.M.** Short Protocols in Molecular Biology: A Compendium of Methods from Current Protocols in Molecular Biology. Greene Pub. Associates, 1995
- **INNIS, M.A. et al.** PCR Strategies. Academic Press, 1995
- **AUSUBEL, F.M.** Short Protocols in Molecular Biology: A Compendium of Methods from Current Protocols in Molecular Biology. Wiley, 1999
- **SNINSKY, J.J. et al.** PCR Applications: Protocols for Functional Genomics. Academic Press, 1999
- Idenshi Donyu & Hatsugenkaiseki Jikkenho [Experimental Method for Gene introduction & Expression Analysis. Jikken Igaku [Experimental Medicine. Yodo-sha, 1997
- **FUKUNAGA.** Statistical Pattern Recognition. Academic Press, 1990
- **ANDERBERG.** Cluster Analysis for Applications. Academic Press, 1973
- **EVERITT.** Cluster Analysis. Heinemann Educ. Books, 1974
- **HARTIGAN.** Clustering Algorithms. Wiley, 1975
- **SNEATH ; SOKAL.** Numerical Taxonomy. 1973
- **STORMO ; HARTZELL.** Identifying protein binding sites from unaligned DNA fragments. *Proc. Natl. Acad. Sci.,* 1989, vol. 86, 1183-1187
- Identification of consensus patterns in unaligned DNA and protein sequences: a large-deviation statistical basis for penalizing gaps. **HERTZ ; STORMO.** Proc. of 3rd Intl. Conf. on Bioinformatics and Genome Research. World Scientific Publishing Co., Ltd, 1995, 201-216
- **SNEATH ; SOKAL.** Numerical taxonomy. W.H. Freeman & Co, 1973
- **WARD.** *J. Am. Stat. Assn.,* 1963, vol. 58, 236
- **HARTIGAN.** Clustering algorithms. Wiley, 1975
- Seimei Sisutemu Kaiseki notameno Sugaku [Mathematics for analyzing life systems. Corona sha, 1999

- Rinsho Igaku notameno Ueburetto Kaiseki [Wavelet analysis for clinical medicine. Igaku Shuppan
- **MIYAWAKI A.** Visualization of the spatial and temporal dynamics of intracellular signaling. *Dev. Cell,* March 2003, vol. 4 (3), 295-305
- **BOUTE N.** The use of resonance energy transfer in high-throughput screening: BRET versus FRET. *Trends Pharmacol Sci.,* August 2002, vol. 23 (8), 351-4
- **J. ZIAUDDIN ; D. M. SABATINI.** *Nature,* 2001, vol. 411, 107
- **R.W. ZU ; S.N. BAILEY ; D.M. SABATINI.** *Trends in Cell Biology,* vol. 12 (10), 485
- **LOCKER.** Transcription Factors: Essential Data. Wiley&Sons, 1996
- **BOUCHARD, C. et al.** *Gene,* 1988, vol. 66, 1-10
- *Proc. Natl. Acad. Sci. USA,* 1999, vol. 96, 4040-4045
- *Nature,* 2003, vol. 423, 838-842
- *YAKUGAKU ZASSHI [Journal of Phamacology,* 2003, vol. 123 (5), 305-313